# EUROPEAN PATENT APPLICATION

(11) **EP 1 790 725 A2**
(43) Date of publication of application: **30.05.2007**
(21) Application number: 06025271.5
(22) Date of filing: 05.03.2001
(51) Int. Cl.: C12N 15/12, C12N 15/24, C07K 14/47, C07K 14/705, C07K 16/28, C07K 16/18, C07K 16/24, G01N 33/68, C12Q 1/68, A61K 38/17, A61K 38/20, A61K 49/00, A61K 31/70, A61K 39/395

(54) **Proteins named after FCTRX and nucleic acids encoding same**

(30) Priority: 03.03.2000 US 186592 P; 03.03.2000 US 186718 P; 06.03.2000 US 187293 P; 06.03.2000 US 187294 P; 17.03.2000 US 190400 P; 07.04.2000 US 196018 P; 03.01.2001 US 259548 P
(62) Divisional of application: 01920218.3
(71) Applicant: Curagen Corporation, New Haven, CT 06511 (US)
(72) Inventor: Vernet, Corine A.M., Branford, CO 06471 (US); Fernandes, Elma, Bridgewater, NJ 08807 (US); Shimkets, Richard A., Commerce, GA 30529 (US); Herrmann, John L., Quincy, MA 01269 (US); Majumder, Kumud, Stamford,CT 06903 (US); MacDougall, John, Hingham, MA 02043 (US); Mishra, Vishnu, Gainesville, Florida 32608 (US); Mezes, Peter S., Old Lyme, CT 06371 (US); Rastelli, Luca, Guilford, CT 06437 (US)
(74) Representative: MacLean, Martin Robert

(57) **Abstract**

Disclosed herein are novel human nucleic acid sequences which encode polypeptides. Also disclosed are polypeptides encoded by these nucleic acid sequences, and antibodies which immunospecifically-bind to the polypeptide, as well as derivatives, variants, mutants, or fragments of the aforementioned polypeptide, polynucleotide, or antibody. The invention further discloses therapeutic, diagnostic and research methods for diagnosis, treatment and prevention of disorders involving any one of these novel human nucleic acids and proteins.

## Description

### BACKGROUND OF THE INVENTION

The invention relates generally to polynucleotides and polypeptides, as well as vectors, host cells, antibodies, and recombinant methods for producing these nucleic acids and polypeptides.

### SUMMARY OF THE INVENTION

The invention is based in part upon the discovery of novel nucleic acid sequences encoding novel polypeptides. The disclosed FCTR1, FCTR2, FCTR3, FCTR4, FCTR5, FCTR6 and FCTR7 nucleic acids and polypeptides encoded therefrom, as well as derivatives, homologs, analogs and fragments thereof, will hereinafter be collectively designated as "FCTRX" nucleic acid or polypeptide sequences.

In one aspect, the invention provides an isolated FCTRX nucleic acid molecule encoding a FCTRX polypeptide that includes a nucleic acid sequence that has identity to the nucleic acids disclosed in SEQ ID NOS:1, 3, 5, 7,9,10,11,12,14,16,18,20,22, and 24. In some embodiments, the FCTRX nucleic acid molecule will hybridize under stringent conditions to a nucleic acid sequence complementary to a nucleic acid molecule that includes a protein-coding sequence of a FCTRX nucleic acid sequence. The invention also includes an isolated nucleic acid that encodes a FCTRX polypeptide, or a fragment, homolog, analog or derivative thereof. For example, the nucleic acid can encode a polypeptide at least 80% identical to a polypeptide comprising the amino acid sequences of SEQ ID NOS:2, 4, 6, 8, 13, 15, 17, 19, 21, 23, and 25. The nucleic acid can be, for example, a genomic DNA fragment or a cDNA molecule that includes the nucleic acid sequence of any of SEQ ID NOS: 1, 3, 5, 7, 9, 10, 11, 12, 14, 16, 18, 20, 22, and 24.

Also included in the invention is an oligonucleotide, *e.g.,* an oligonucleotide which includes at least 6 contiguous nucleotides of a FCTRX nucleic acid (*e.g*., SEQ ID NOS: 1, 3, 5, 7, 9, 10,11, 12, 14, 16, 18, 20, 22, and 24) or a complement of said oligonucleotide.

Also included in the invention are substantially purified FCTRX polypeptides (SEQ ID NO: 2, 4, 6, 8, 13, 15, 17, 19, 21, 23, and 25). In certain embodiments, the FCTRX polypeptides include an amino acid sequence that is substantially identical to the amino acid sequence of a human FCTRX polypeptide.

The invention also features antibodies that immunoselectively-binds to FCTRX polypeptides, or fragments, homologs, analogs or derivatives thereof.

In another aspect, the invention includes pharmaceutical compositions that include therapeutically- or prophylactically-effective amounts of a therapeutic and a pharmaceutically-acceptable carrier. The therapeutic can be, *e.g.,* a FCTRX nucleic acid, a FCTRX polypeptide, or an antibody specific for a FCTRX polypeptide. In a further aspect, the invention includes, in one or more containers, a therapeutically- or prophylactically-effective amount of this pharmaceutical composition.

In a further aspect, the invention includes a method of producing a polypeptide by culturing a cell that includes a FCTRX nucleic acid, under conditions allowing for expression of the FCTRX polypeptide encoded by the DNA. If desired, the FCTRX polypeptide can then be recovered.

In another aspect, the invention includes a method of detecting the presence of a FCTRX polypeptide in a sample. In the method, a sample is contacted with a compound that selectively binds to the polypeptide under conditions allowing for formation of a complex between the polypeptide and the compound. The complex is detected, if present, thereby identifying the FCTRX polypeptide within the sample.

The invention also includes methods to identify specific cell or tissue types based on their expression of a FCTRX.

Also included in the invention is a method of detecting the presence of a FCTRX nucleic acid molecule in a sample by contacting the sample with a FCTRX nucleic acid probe or primer, and detecting whether the nucleic acid probe or primer bound to a FCTRX nucleic acid molecule in the sample.

In a further aspect, the invention provides a method for modulating the activity of a FCTRX polypeptide by contacting a cell sample that includes the FCTRX polypeptide with a compound that binds to the FCTRX polypeptide in an amount sufficient to modulate the activity of said polypeptide. The compound can be, *e.g.,* a small molecule, such as a nucleic acid, peptide, polypeptide, peptidomimetic, carbohydrate, lipid or other organic (carbon containing) or inorganic molecule, as further described herein.

Also within the scope of the invention is the use of a Therapeutic in the manufacture of a medicament for treating or preventing disorders or syndromes including, *e.g*., Colorectal cancer, adenomatous polyposis coli, myelogenous leukemia, congenital ceonatal alloimmune thrombocytopenia, multiple human solid malignancies, malignant ovarian tumours particularly at the interface between epithelia and stroma, malignant brain tumors, mammary tumors, human gliomas, astrocytomas, mixed glioma/astrocytomas, renal cells carcinoma, breast adenocarcinoma, ovarian cancer, melanomas, renal cell carcinoma, clear cell and granular cell carcinomas, autocrine/paracrine stimulation of tumor cell proliferation, autocrine/paracrine stimulation of tumor cell survival and tumor cell resistance to cytotoxic therapy, paranechmal and basement membrane invasion and motility of tumor cells thereby contributing to metastasis, tumor-mediated immunosuppression of T-cell mediated immune effector cells and pathways resulting in tumor escape from immune surveilance, neurological disorders, neurodegenerative disorders, nerve trauma, familial myelodysplastic syndrome, Charcot-Marie-Tooth neuropathy, demyelinating Gardner syndrome, familial myelodysplastic syndrome; mental health conditions, immunological disorders, allergy and infection, asthma, bronchial asthma, Avellino type eosinophilia, lung diseases, reproductive disorders, male infertility, female reproductive system disorders, male and female reproductive diseases, hemangioma, deafness, glycoprotein Ia deficiency, desmoid disease, turcot syndrome, liver cirrhosis, hepatitis C, gastric disorders, pancreatic diseases like diabetes, Schistosoma mansoni infection, Spinocerebellar ataxia, Plasmodium falciparum parasitemia, Corneal dystrophy - Groenouw type I, Corneal dystrophy - lattice type I, and Reis-Bucklers corneal dystrophy. The Therapeutic can be, *e.g.,* a FCTRX nucleic acid, a FCTRX polypeptide, or a FCTRX-specific antibody, or biologically-active derivatives or fragments thereof.

The invention further includes a method for screening for a modulator of disorders or syndromes including, *e.g*., Also within the scope of the invention is the use of a Therapeutic in the manufacture of a medicament for treating or preventing disorders or syndromes including, *e.g*., Colorectal cancer, adenomatous polyposis coli, myelogenous leukemia, congenital ceonatal alloimmune thrombocytopenia, multiple human solid malignancies, malignant ovarian tumours particularly at the interface between epithelia and stroma, malignant brain tumors, mammary tumors, human gliomas, astrocytomas, mixed glioma/astrocytomas, renal cells carcinoma, breast adenocarcinoma, ovarian cancer, melanomas, renal cell carcinoma, clear cell and granular cell carcinomas, autocrine/paracrine stimulation of tumor cell proliferation, autocrine/paracrine stimulation of tumor cell survival and tumor cell resistance to cytotoxic therapy, paranechmal and basement membrane invasion and motility of tumor cells thereby contributing to metastasis, tumor-mediated immunosuppression of T-cell mediated immune effector cells and pathways resulting in tumor escape from immune surveilance, neurological disorders, neurodegenerative disorders, nerve trauma, familial myelodysplastic syndrome, Charcot-Marie-Tooth neuropathy, demyelinating Gardner syndrome, familial myelodysplastic syndrome; mental health conditions, immunological disorders, allergy and infection, asthma, bronchial asthma, Avellino type eosinophilia, lung diseases, reproductive disorders, male infertility, female reproductive system disorders, male and female reproductive diseases, hemangioma, deafness, glycoprotein Ia deficiency, desmoid disease, turcot syndrome, liver cirrhosis, hepatitis C, gastric disorders, pancreatic diseases like diabetes, Schistosoma mansoni infection, Spinocerebellar ataxia, Plasmodium falciparum parasitemia, Corneal dystrophy - Groenouw type I, Corneal dystrophy - lattice type I, and Reis-Bucklers corneal dystrophy. The method includes contacting a test compound with a FCTRX polypeptide and determining if the test compound binds to said FCTRX polypeptide. Binding of the test compound to the FCTRX polypeptide indicates the test compound is a modulator of activity, or of latency or predisposition to the aforementioned disorders or syndromes.

Also within the scope of the invention is a method for screening for a modulator of activity, or of latency or predisposition to an disorders or syndromes including, *e.g.,* Also within the scope of the invention is the use of a Therapeutic in the manufacture of a medicament for treating or preventing disorders or syndromes including, *e.g*., Colorectal cancer, adenomatous polyposis coli, myelogenous leukemia, congenital ceonatal alloimmune thrombocytopenia, multiple human solid malignancies, malignant ovarian tumours particularly at the interface between epithelia and stroma, malignant brain tumors, mammary tumors, human gliomas, astrocytomas, mixed glioma/astrocytomas, renal cells carcinoma, breast adenocarcinoma, ovarian cancer, melanomas, renal cell carcinoma, clear cell and granular cell carcinomas, autocrine/paracrine stimulation of tumor cell proliferation, autocrine/paracrine stimulation of tumor cell survival and tumor cell resistance to cytotoxic therapy, paranechmal and basement membrane invasion and motility of tumor cells thereby contributing to metastasis, tumor-mediated immunosuppression of T-cell mediated immune effector cells and pathways resulting in tumor escape from immune surveilance, neurological disorders, neurodegenerative disorders, nerve trauma, familial myelodysplastic syndrome, Charcot-Marie-Tooth neuropathy, demyelinating Gardner syndrome, familial myelodysplastic syndrome; mental health conditions, immunological disorders, allergy and infection, asthma, bronchial asthma, Avellino type eosinophilia, lung diseases, reproductive disorders, male infertility, female reproductive system disorders, male and female reproductive diseases, hemangioma, deafness, glycoprotein Ia deficiency, desmoid disease, turcot syndrome, liver cirrhosis, hepatitis C, gastric disorders, pancreatic diseases like diabetes, Schistosoma mansoni infection, Spinocerebellar ataxia, Plasmodium falciparum parasitemia, Corneal dystrophy - Groenouw type I, Corneal dystrophy - lattice type I, and Reis-Bucklers corneal dystrophy by administering a test compound to a test animal at increased risk for the aforementioned disorders or syndromes. The test animal expresses a recombinant polypeptide encoded by a FCTRX nucleic acid. Expression or activity of FCTRX polypeptide is then measured in the test animal, as is expression or activity of the protein in a control animal which recombinantly-expresses FCTRX polypeptide and is not at increased risk for the disorder or syndrome. Next, the expression of FCTRX polypeptide in both the test animal and the control animal is compared. A change in the activity of FCTRX polypeptide in the test animal relative to the control animal indicates the test compound is a modulator of latency of the disorder or syndrome.

In yet another aspect, the invention includes a method for determining the presence of or predisposition to a disease associated with altered levels of a FCTRX polypeptide, a FCTRX nucleic acid, or both, in a subject (*e.g.,* a human subject). The method includes measuring the amount of the FCTRX polypeptide in a test sample from the subject and comparing the amount of the polypeptide in the test sample to the amount of the FCTRX polypeptide present in a control sample. An alteration in the level of the FCTRX polypeptide in the test sample as compared to the control sample indicates the presence of or predisposition to a disease in the subject. Preferably, the predisposition includes, *e.g.,* Also within the scope of the invention is the use of a Therapeutic in the manufacture of a medicament for treating or preventing disorders or syndromes including, *e.g.,* Colorectal cancer, adenomatous polyposis coli, myelogenous leukemia, congenital ceonatal alloimmune thrombocytopenia, multiple human solid malignancies, malignant ovarian tumours particularly at the interface between epithelia and stroma, malignant brain tumors, mammary tumors, human gliomas, astrocytomas, mixed glioma/astrocytomas, renal cells carcinoma, breast adenocarcinoma, ovarian cancer, melanomas, renal cell carcinoma, clear cell and granular cell carcinomas, autocrine/paracrine stimulation of tumor cell proliferation, autocrine/paracrine stimulation of tumor cell survival and tumor cell resistance to cytotoxic therapy, paranechmal and basement membrane invasion and motility of tumor cells thereby contributing to metastasis, tumor-mediated immunosuppression of T-cell mediated immune effector cells and pathways resulting in tumor escape from immune surveilance, neurological disorders, neurodegenerative disorders, nerve trauma, familial myelodysplastic syndrome, Charcot-Marie-Tooth neuropathy, demyelinating Gardner syndrome, familial myelodysplastic syndrome; mental health conditions, immunological disorders, allergy and infection, asthma, bronchial asthma, Avellino type eosinophilia, lung diseases, reproductive disorders, male infertility, female reproductive system disorders, male and female reproductive diseases, hemangioma, deafness, glycoprotein Ia deficiency, desmoid disease, turcot syndrome, liver cirrhosis, hepatitis C, gastric disorders, pancreatic diseases like diabetes, Schistosoma mansoni infection, Spinocerebellar ataxia, Plasmodium falciparum parasitemia, Corneal dystrophy - Groenouw type I, Corneal dystrophy - lattice type I, and Reis-Bucklers corneal dystrophy. Also, the expression levels of the new polypeptides of the invention can be used in a method to screen for various cancers as well as to determine the stage of cancers.

In a further aspect, the invention includes a method of treating or preventing a pathological condition associated with a disorder in a mammal by administering to the subject a FCTRX polypeptide, a FCTRX nucleic acid, or a FCTRX-specific antibody to a subject (*e.g*., a human subject), in an amount sufficient to alleviate or prevent the pathological condition. In preferred embodiments, the disorder, includes, *e.g*., Also within the scope of the invention is the use of a Therapeutic in the manufacture of a medicament for treating or preventing disorders or syndromes including, *e.g.,* Colorectal cancer, adenomatous polyposis coli, myelogenous leukemia, congenital ceonatal alloimmune thrombocytopenia, multiple human solid malignancies, malignant ovarian tumours particularly at the interface between epithelia and stroma, malignant brain tumors, mammary tumors, human gliomas, astrocytomas, mixed glioma/astrocytomas, renal cells carcinoma, breast adenocarcinoma, ovarian cancer, melanomas, renal cell carcinoma, clear cell and granular cell carcinomas, autocrine/paracrine stimulation of tumor cell proliferation, autocrine/paracrine stimulation of tumor cell survival and tumor cell resistance to cytotoxic therapy, paranechmal and basement membrane invasion and motility of tumor cells thereby contributing to metastasis, tumor-mediated immunosuppression of T-cell mediated immune effector cells and pathways resulting in tumor escape from immune surveilance, neurological disorders, neurodegenerative disorders, nerve trauma, familial myelodysplastic syndrome, Charcot-Marie-Tooth neuropathy, demyelinating Gardner syndrome, familial myelodysplastic syndrome; mental health conditions, immunological disorders, allergy and infection, asthma, bronchial asthma, Avellino type eosinophilia, lung diseases, reproductive disorders, male infertility, female reproductive system disorders, male and female reproductive diseases, hemangioma, deafness, glycoprotein Ia deficiency, desmoid disease, turcot syndrome, liver cirrhosis, hepatitis C, gastric disorders, pancreatic diseases like diabetes, Schistosoma mansoni infection, Spinocerebellar ataxia, Plasmodium falciparum parasitemia, Corneal dystrophy - Groenouw type I, Corneal dystrophy - lattice type I, and Reis-Bucklers corneal dystrophy.

In yet another aspect, the invention can be used in a method to identity the cellular receptors and downstream effectors of the invention by any one of a number of techniques commonly employed in the art. These include but are not limited to the two-hybrid system, affinity purification, co-precipitation with antibodies or other specific-interacting molecules.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. All publications, patent applications, patents, and other references mentioned herein are incorporated by reference in their entirety. In the case of conflict, the present specification, including definitions, will control. In addition, the materials, methods, and examples are illustrative only and not intended to be limiting.

Other features and advantages of the invention will be apparent from the following detailed description and claims.

### DETAILED DESCRIPTION

The invention is based, in part, upon the discovery of novel nucleic acid sequences that encode novel polypeptides. The novel nucleic acids and their encoded polypeptides are referred to individually as FCTR1, FCTR2, FCTR3, FCTR4, FCTR5, FCTR6, and FCTR7. The nucleic acids, and their encoded polypeptides, are collectively designated herein as "FCTRX".

The novel FCTRX nucleic acids of the invention include the nucleic acids whose sequences are provided in Tables 1A, 2A, 3A, 3C, 3E, 3F, 3G, 3H, 4A, 5A, 5C, 5E, 6A, 6C, and 7A inclusive ("Tables 1A - 7A"), or a fragment, derivative, analog or homolog thereof. The novel FCTRX proteins of the invention include the protein fragments whose sequences are provided in Tables 1B, 2B, 3B, 3I, 4B, 5B, 5D, 6B, 6D, and 7B inclusive ("Tables 1B - 7B"). The individual FCTRX nucleic acids and proteins are described below. Within the scope of this invention is a method of using these nucleic acids and peptides in the treatment or prevention of a disorder related to cell signaling or metabolic pathway modulation.

### FCTR1

Novel FCTR1 is a growth factor ("FCTR") protein related to follistatin-like gene, and mac25. FCTR1 (also referred to by proprietary accession number 58092213.0.36) is a full-length clone of 771 nucleotides, including the entire coding sequence of a 105 amino acid protein from nucleotides 438 to 753. The clone was originally obtained from thyroid gland, kidney, fetal kidney, and spleen tissues.

The nucleotide sequence of FCTR1 as presently determined is reported in Table 1A. The start and stop codons are bolded and the 5' and 3' untranslated regions are underlined.

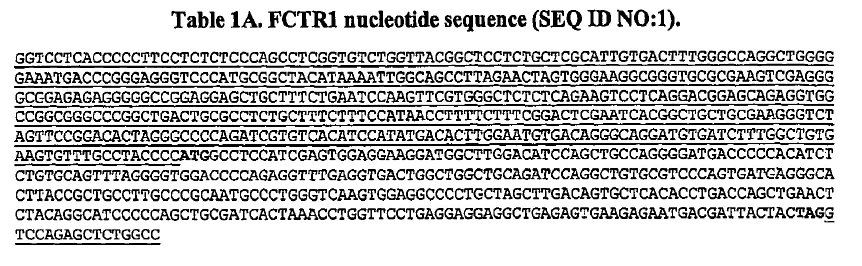

The predicted amino acid sequence of FCTR1 protein corresponding to the foregoing nucleotide sequence is reported in Table 1B. FCTR1 was searched against other databases using SignalPep and PSort search protocols. The protein is most likely located in the cytoplasm (certainty=0.6500) and seems to have no N-terminal signal sequence. The predicted molecular weight of FCTR1 protein is 11711.8 daltons.

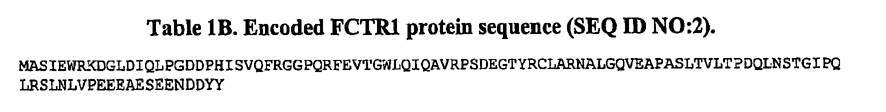

FCTR1 was initially identified with a TblastN analysis of a proprietary sequence file for a follistatin-like probe or homolog which was run against the Genomic Daily Files made available by GenBank. A proprietary software program (Genscan^{™}) was used to further predict the nucleic acid sequence and the selection of exons. The resulting sequences were further modified by means of similarities using BLAST searches. The sequences were then manually corrected for apparent inconsistencies, thereby obtaining the sequences encoding the full-length protein.

In an analysis of sequence databases, it was found, for example, that the FCTR1 nucleic acid sequence has 31/71 bases (43%) identical and 46/71 bases positively alike to a *Mus Musculus* IGFBP-like protein (TREMBL Accession Number:BAA21725) shown in Table 1C. In all BLAST alignments herein, the "E-value" or "Expect" value is a numeric indication of the probability that the aligned sequences could have achieved their similarity to the BLAST query sequence by chance alone, within the database that was searched. For example, as shown in Table 1C, the probability that the subject ("Sbjct") retrieved from the FCTR1 BLAST analysis, in this case the *Mus Musculus* IGFBP-like protein, matched the Query FCTR1 sequence purely by chance is 1.2x10⁻¹¹.

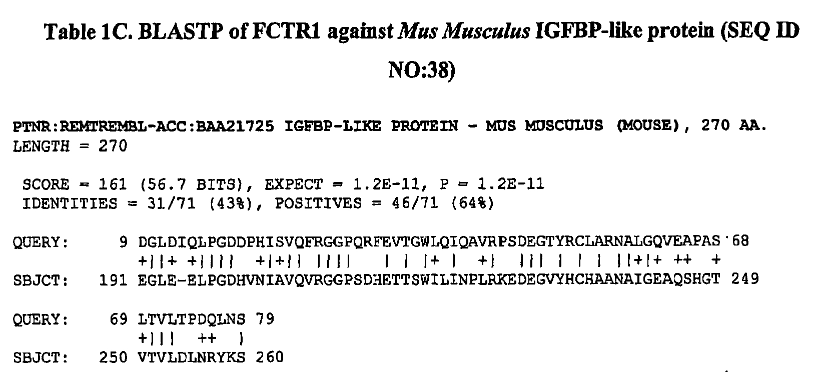

The amino acid sequence of FCTR1 also had 26/58 bases (44%) identical, and 38/58 bases (65%) positive for *Mus Musculus* Follistatin-like Protein shown in Table 1D.

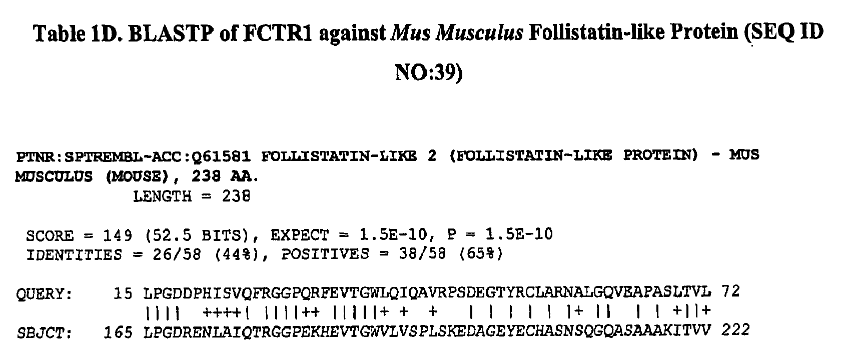

The amino acid sequence of FCTR1 also had 26/58 bases (44%) identical, and 38/58 bases (65%) positive for *Homo sapiens* MAC25 protein shown in Table 1E.

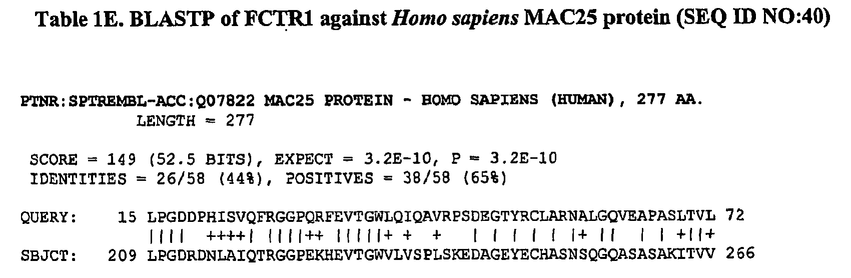

The amino acid sequence of FCTR1 also had 26/58 bases (44%) identical, and 38/58 bases (65%) positive for *Mus musculus* MAC25 protein shown in Table 1F.

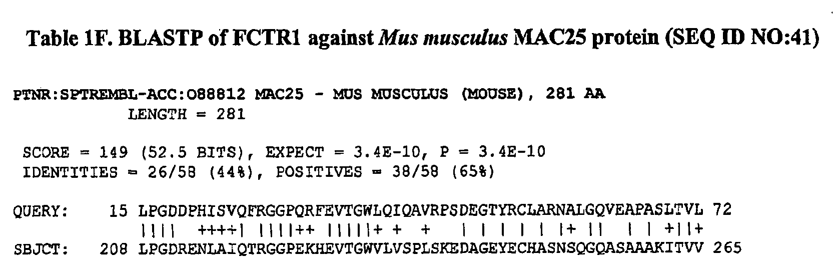

The amino acid sequence of FCTR1 also had 26/58 bases (44%) identical, and 38/58 bases (65%) positive for *Homo sapiens* Prostacyclin-stimulating factor shown in Table 1G.

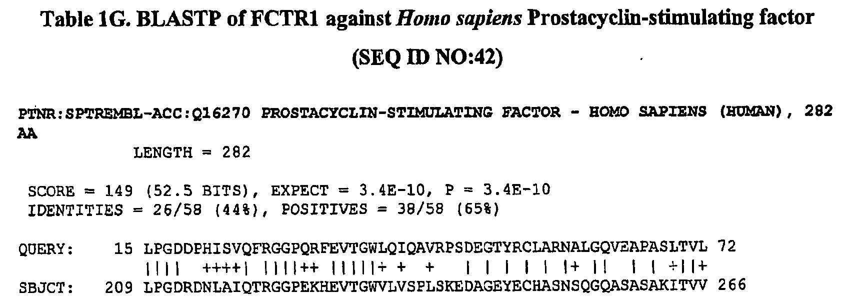

The amino acid sequence of FCTR1 also had 18/44 bases (40%) identical, and 25/44 bases (56%) positive for rat Colorectal cancer suppressor shown in Table 1H.

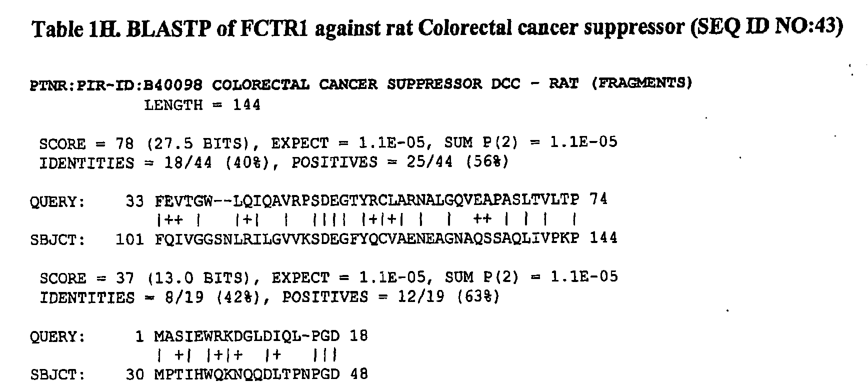

The amino acid sequence of FCTR1 also had 32/83 bases (38%) identical, and 45/83 bases (54%) positive to bases 55-137, and 24/68 bases (35%) identical, and 37/68 bases (54%) positive to bases 166-225 of *Homo sapiens* PTPsigma-(Brain) Precursor shown in Table 1I.

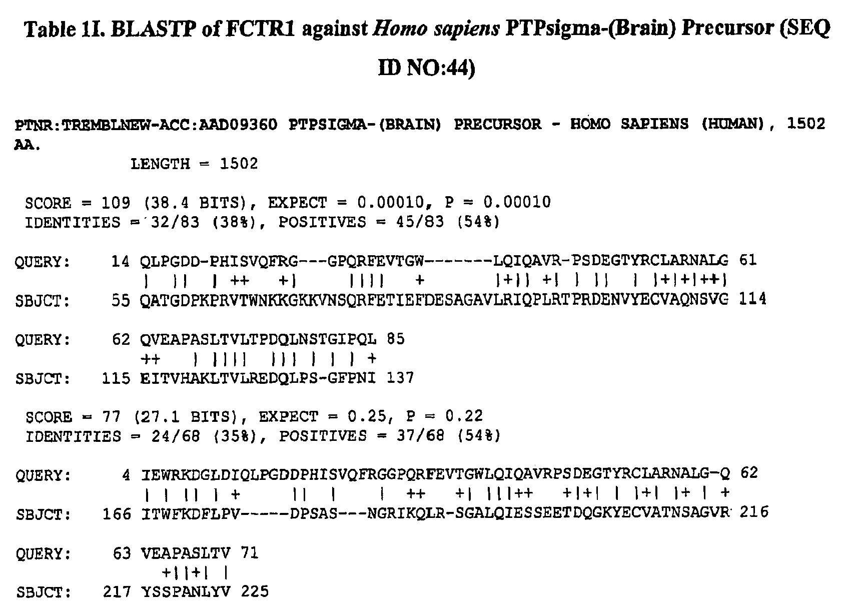

The amino acid sequence of FCTR1 also had 32/83 bases (38%) identical, and 45/83 bases (54%) positive for amino acids 55-137 and 26/69 bases (37%) identical, and 38/69 (54%) positive for amino acids 166-234 of *Homo sapiens* Protein-Tyrosine Phosphatase Sigma shown in Table 1J.

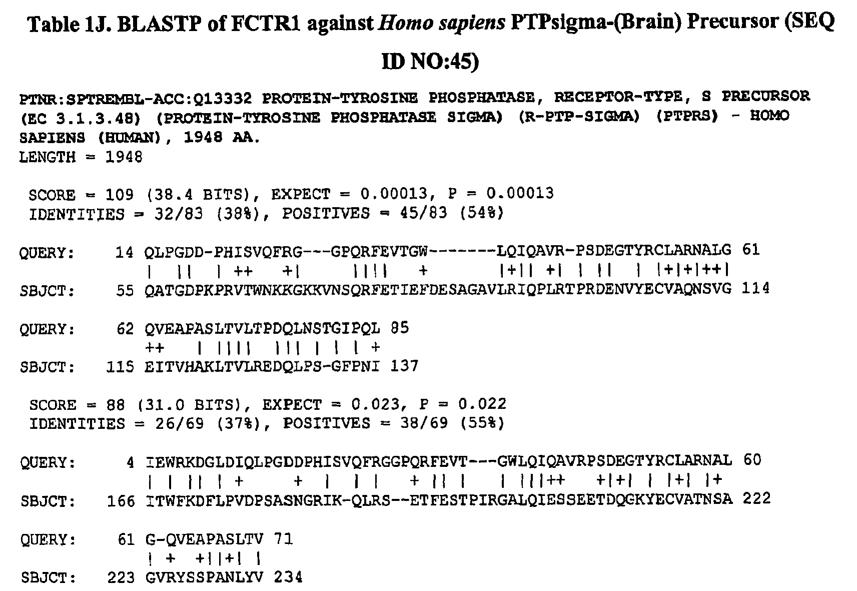

A ClustalW analysis comparing the protein of the invention with related protein sequences is given in Table 1K, with FCTR1 shown on line 2. In the ClustalW alignment of the FCTR1 protein, as well as all other ClustalW analyses herein, the black outlined amino acid residues indicate regions of conserved sequence (*i.e*., regions that may be required to preserve structural or functional properties), whereas non-highlighted amino acid residues are less conserved and can potentially be mutated to a much broader extent without altering protein structure or function.

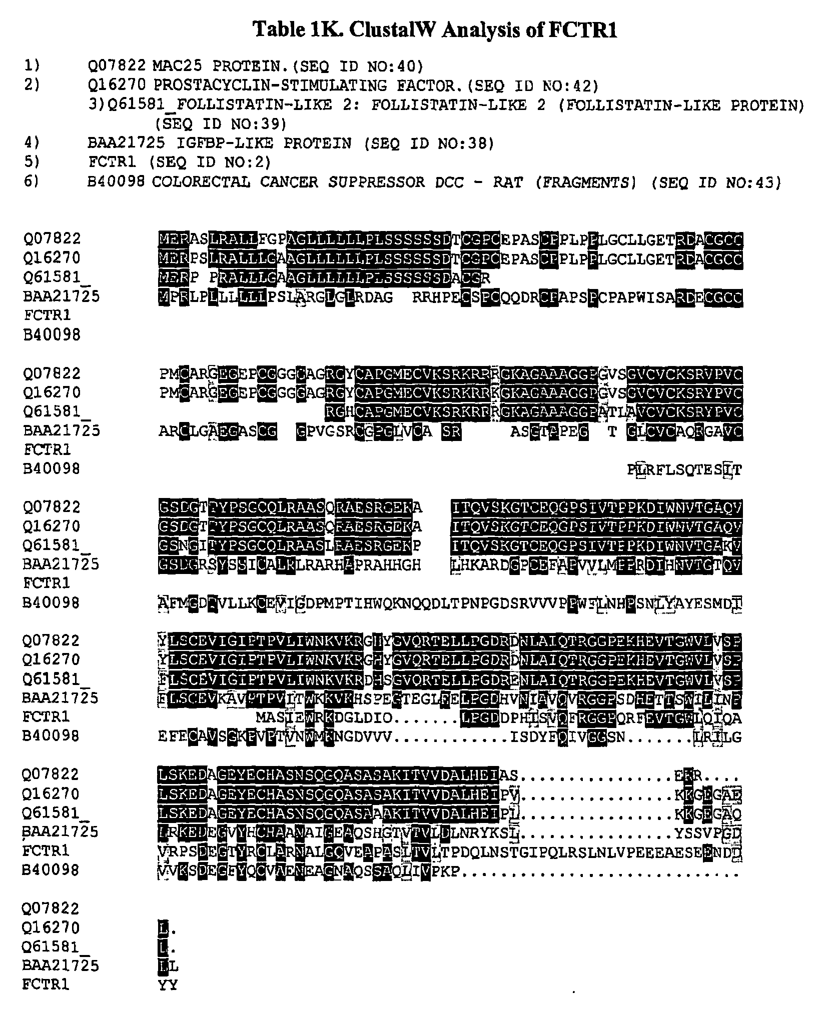

IGFBP is expressed in neurostem cell and developing central nervous system. MAC-25, a follistatin like protein is a growth suppressor of osteosarcoma cells, and meningiomas. DCC is expressed in most normal tissues especially in colonic mucosa, but is deleted in colorectal cancers.

Since FCTR1 has similarity to these proteins (shown in BlastP, Tables 1C-1J, and in clustalW, Table 1K) it is likely that it has similar function. Therefore FCTR1 could function as on or more of the following: a tumor suppressor geneor regulator of neurological system development.

Based on the protein similarity and tissue expression, FCTR1 may be useful in the following diseases and uses:
(i) Tissue regeneration in vitro and in vivo
(ii) Neurological disorders, neurodegenerative disorders, nerve trauma
(iii) Reproductive health
(iv) Immunological disorders, allergy and infection
(v) In cancer as a diagnostic and prognostic marker, as well as a protein therapeutic

### FCTR2

FCTR2 (alternatively referred to herein as AC012614_1.0.123), is a growth factor bearing sequence similarity to human KIAA1061 protein and to genes involved in neuronal development and reproductive physiology (e.g., cell adhesion molecules, follistatin, roundabout and frazzled). FCTR2 is a full-length clone of 5502 nucleotides, including the entire coding sequence of a 815 amino acid protein. This sequence is expressed in glioma, osteoblast, other cancer cells, lung carcinoma, small intestine (This sequence maps to Unigene Hs.123420 which is expressed in brain, breast, kidney, pancreas, pooled tissue).

A FCTR2 ORF begins with an ATG initiation codon at nucleotides 420-422 and ends with a TGA codon at nucleotides 2865-2867. Putative untranslated regions upstream from the initiation codon and downstream from the termination codon are underlined in Table 2A, and the start and stop codons are in bold letters.

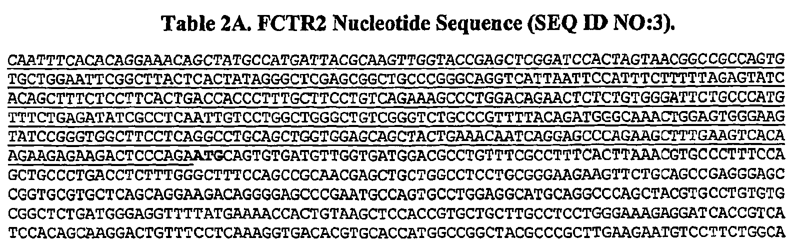

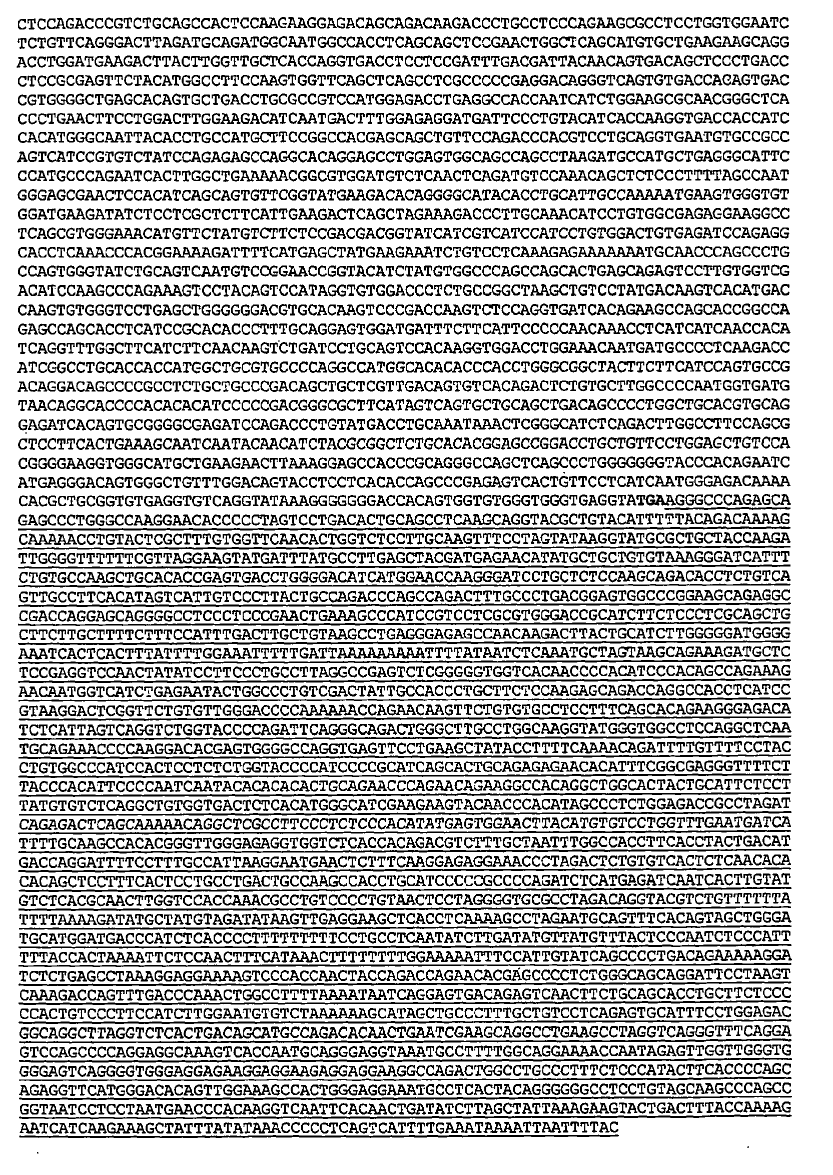

The predicted amino acid sequence of FCTR2 protein corresponding to the foregoing nucleotide sequence is reported in Table 2B. FCTR2 was searched against other databases using SignalPep and PSort search protocols. The protein is most likely located in the mitochondrial matrix space (certainty=0.4718) and seems to have no N-terminal signal sequence. The predicted molecular weight is 90346.9 Daltons.

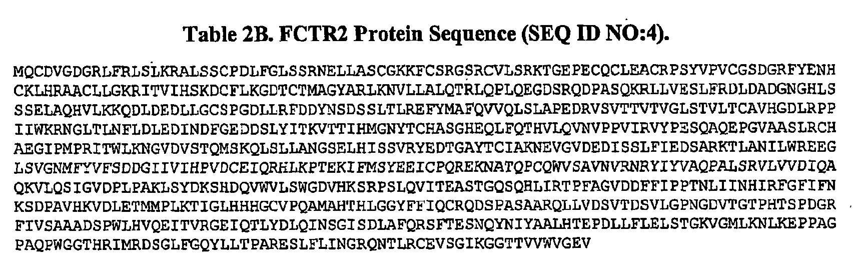

In a BLASTN search it was also found that nucleotides 784-5502 of FCTR2 nucleic acid had 4672 of 4719 bases (99%) identical to *Homo sapiens* mRNA for KIAA1061 protein, partial cds (GenBank Acc:AB028984) (Table 2C).

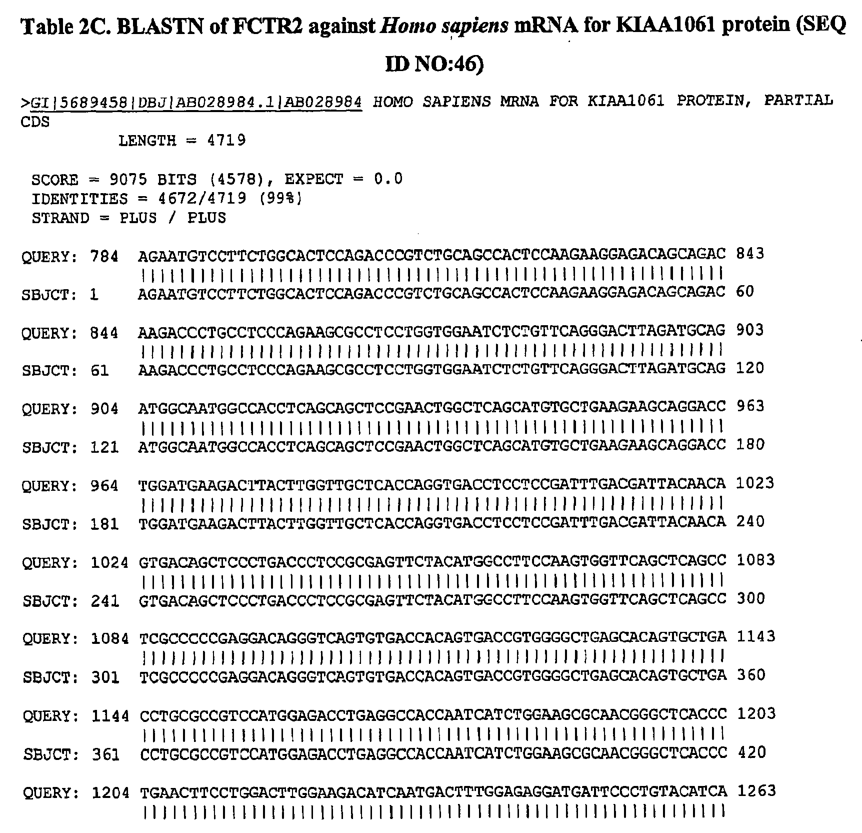

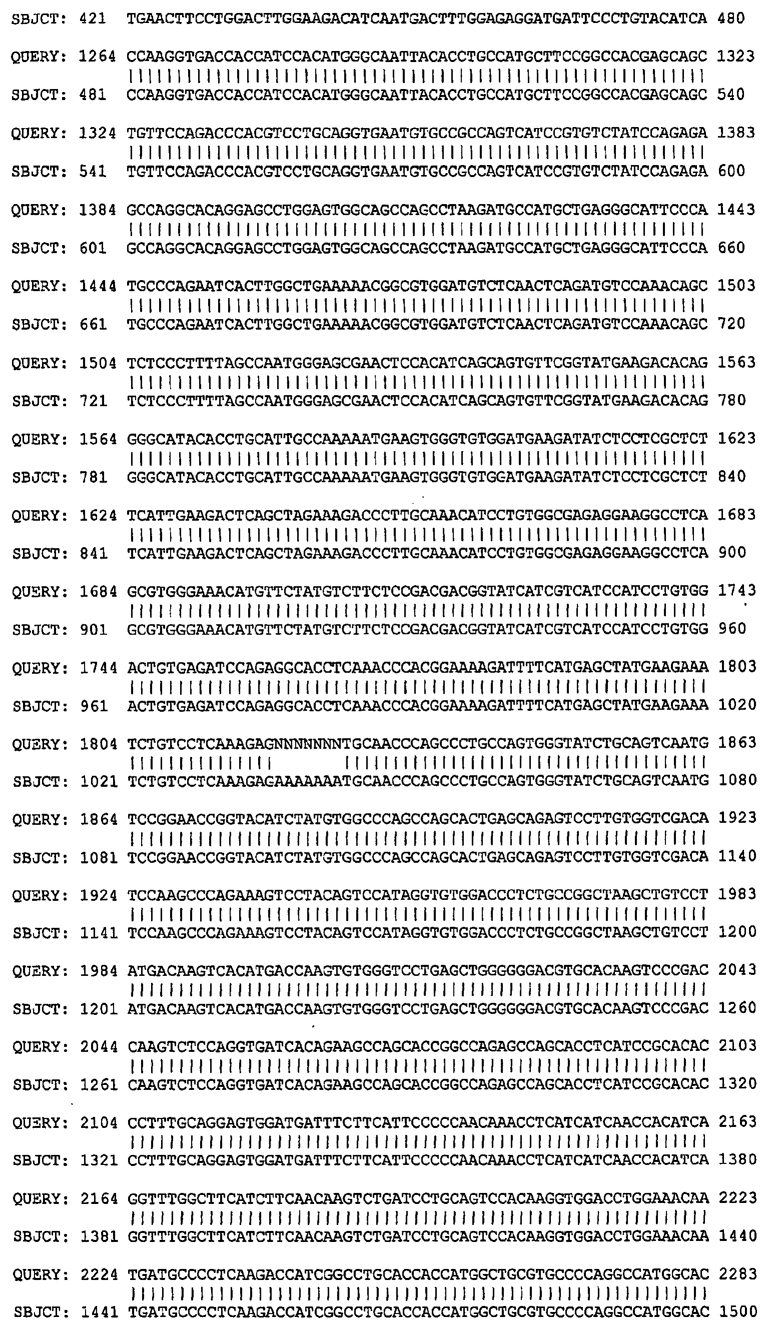

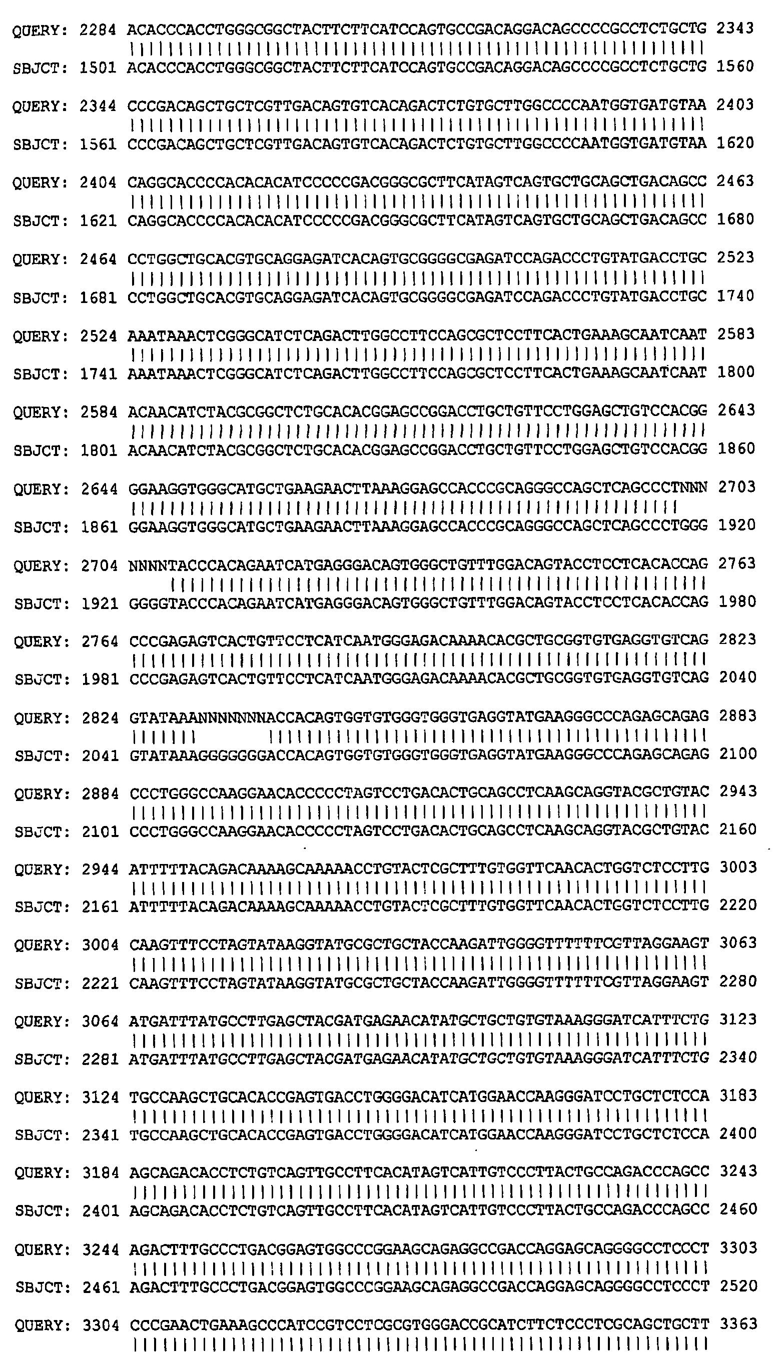

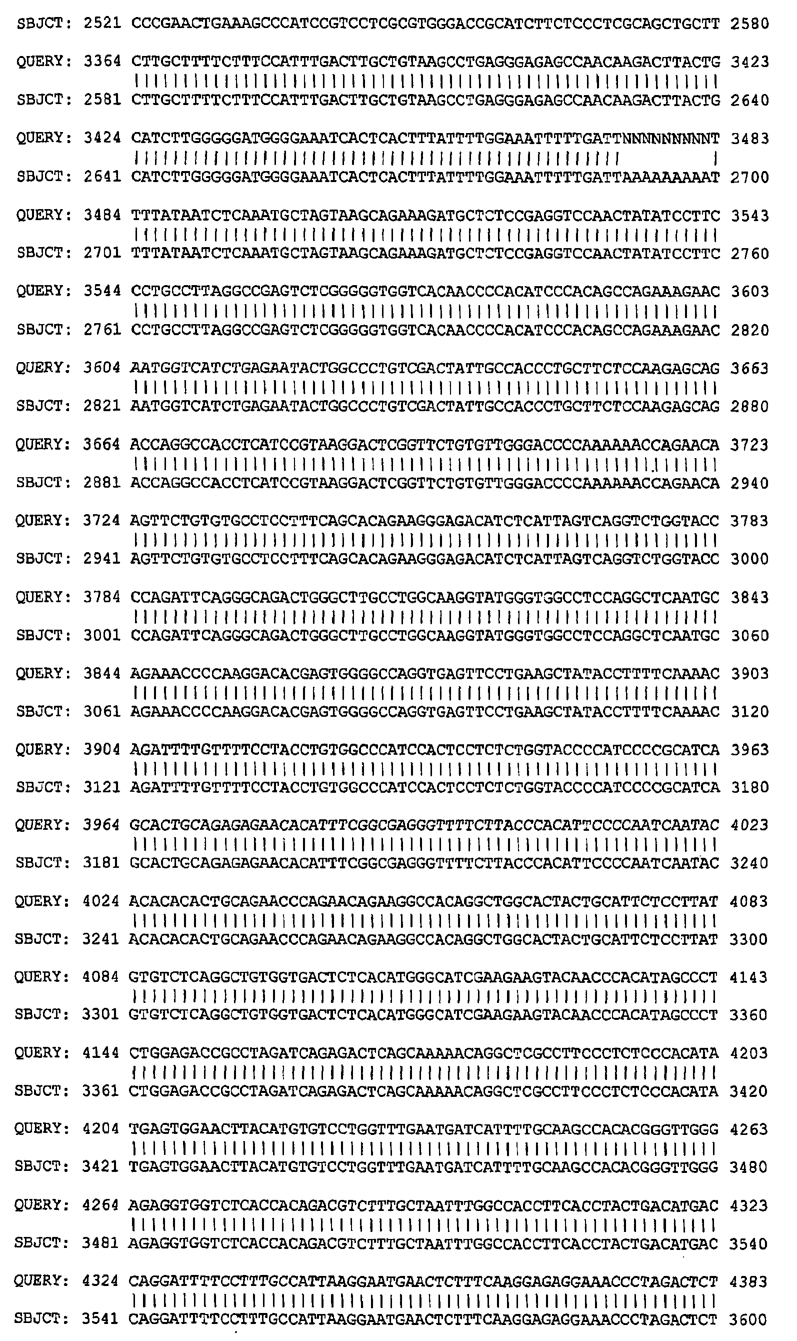

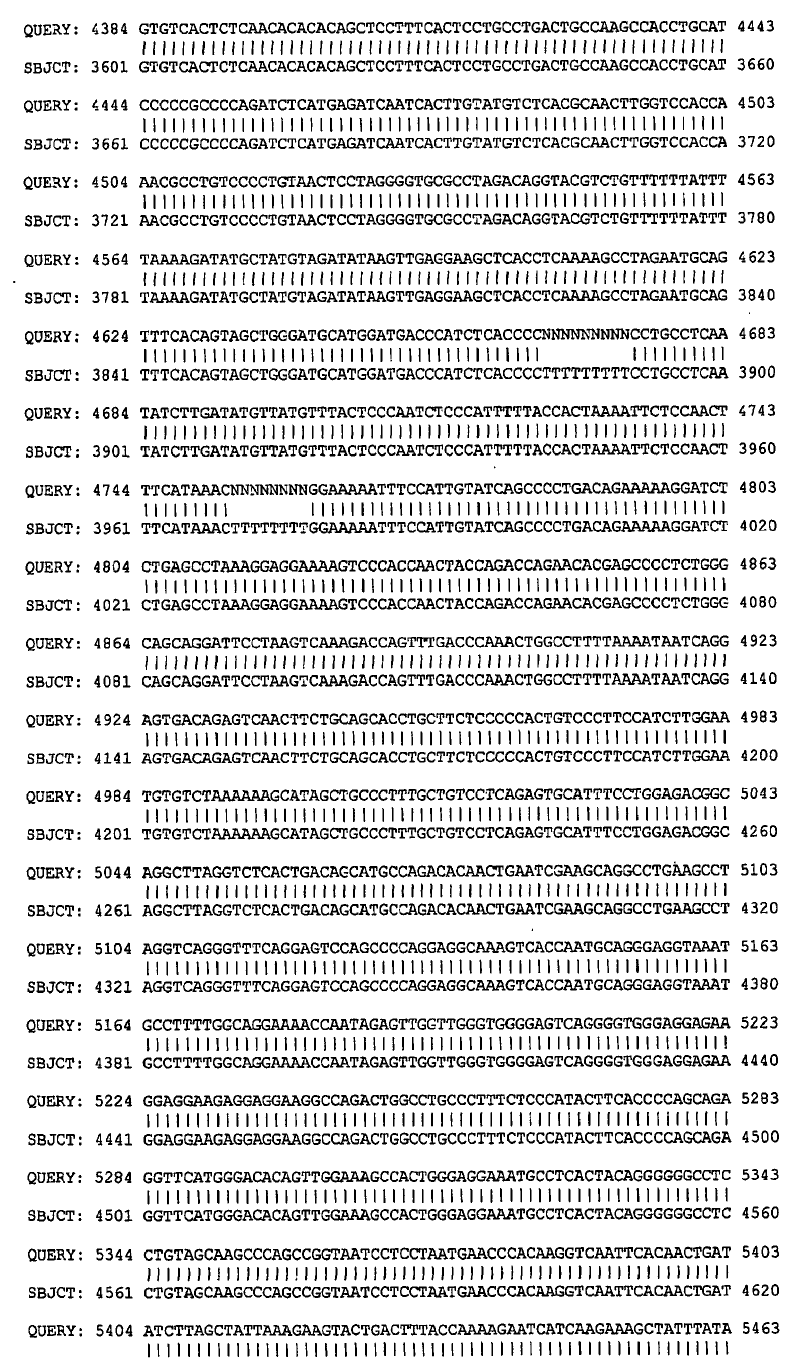

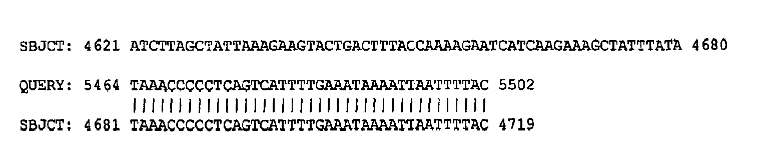

The FCTR2 amino acid sequence has 473 of 810 amino acid residues (58%) identical to, and 616 of 810 residues (76%) positive with, the 850 amino acid residue proteins from *Homo sapiens* KIAA1263 Protein fragment (ptnr: TREMBLNEW-ACC:BAA86577) (SEQ ID NO:47) (Table 2D).

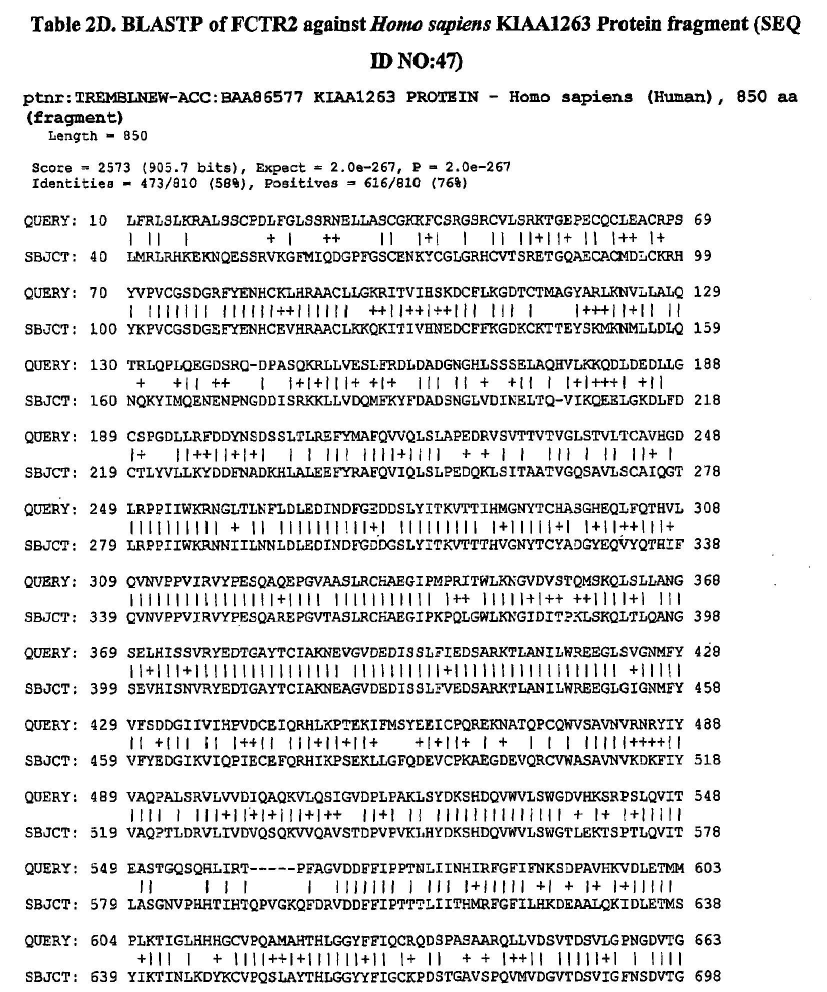

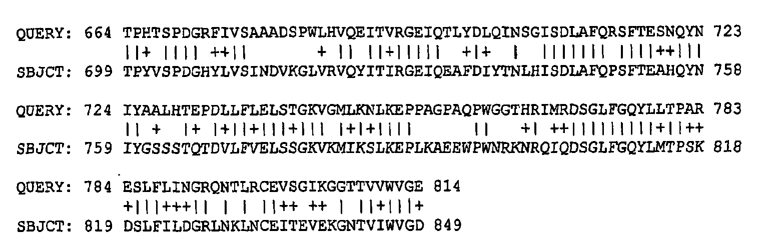

Amino acids 123-815 of FCTR2 also have 693 of 693 amino acid residues (100%) identical to,the 693 amino acid residue protein fragment of KIAA1061 Protein from *Homo sapiens* (ptnr: TREMBLNEW-ACC: BAA83013) (SEQ ID NO:48) (Table 2E).

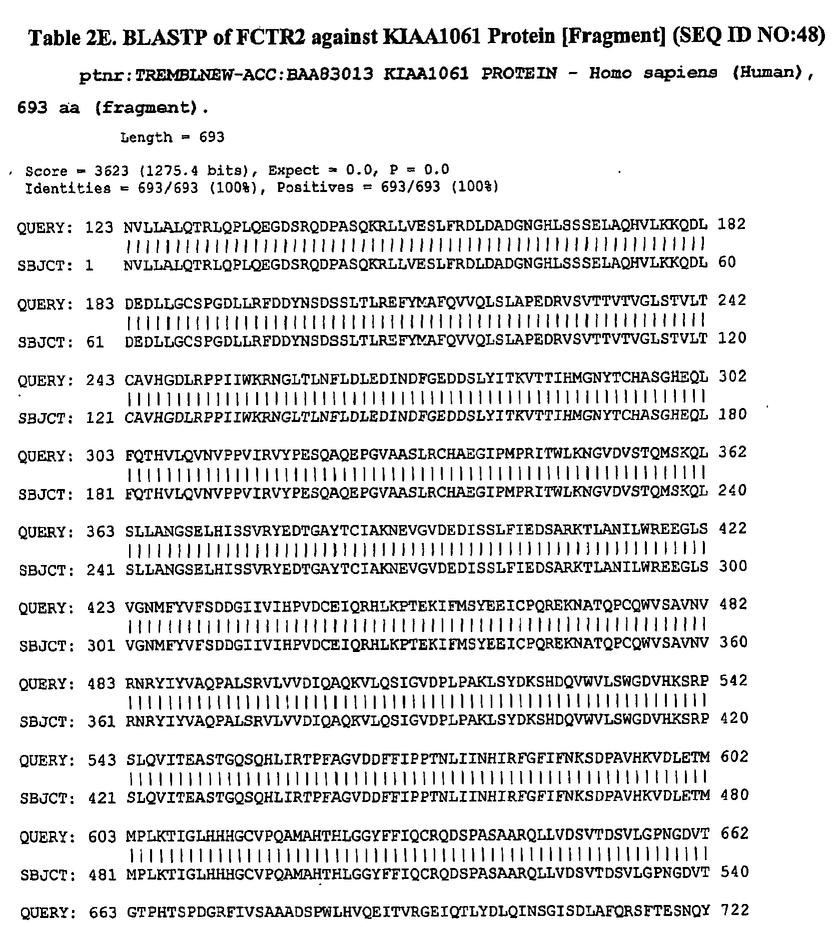

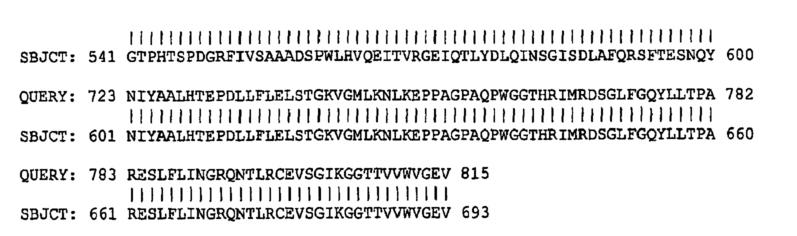

The amino acid sequence of the FCTR2 protein has 451 of 772 amino acid residues (58%) identical to, and 586 of 772 residues (75%) positive with, the 773 amino acid residue proteins hypothetical protein DKFZp566D234.1 from *Homo sapiens* (fragments) (ptnr: SPTREMBL-ACC: CAB70877.1) (SEQ ID NO:49) (Table 2F).

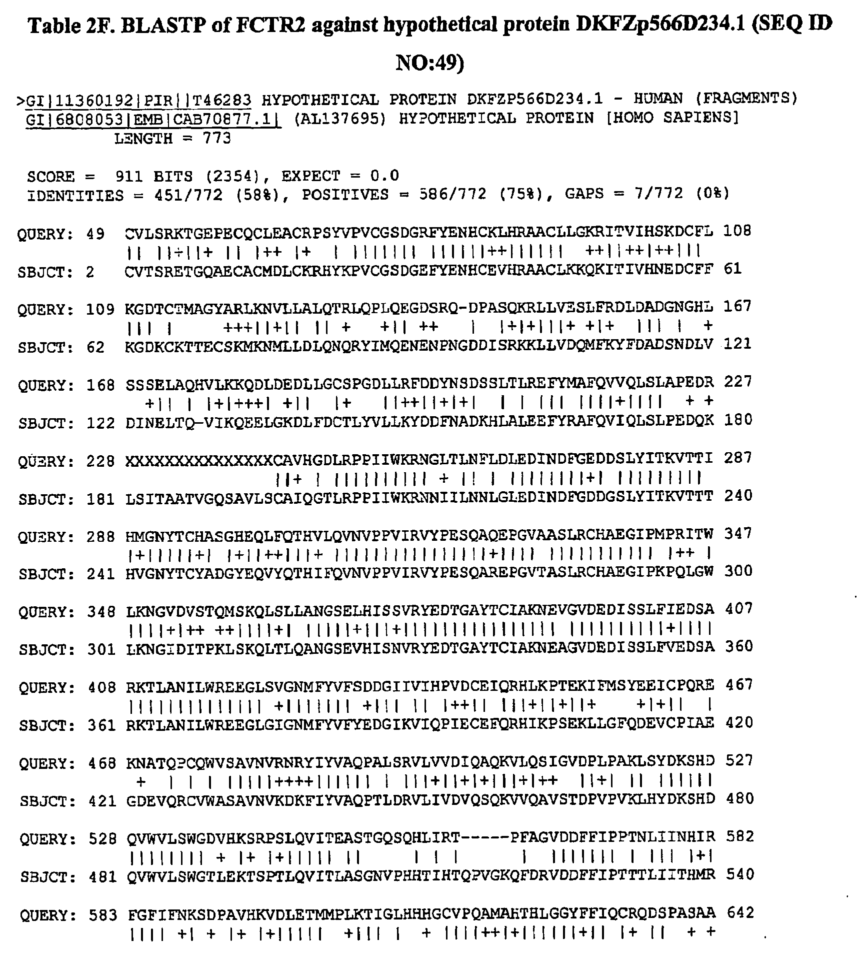

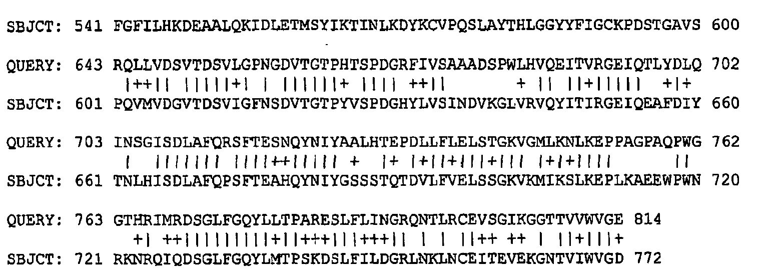

The amino acid sequence of the FCTR2 protein has 61 of 194 amino acid residues (31%) identical to, and 90 of 194 residues (45%) positive with, the 306 amino acid residue protein Follastin-Related Protein 1 Precursor from *Rattus Norvegicus* (ptnr: GenBank Acc:Q62632) (SEQ ID NO:50) (Table 2G).

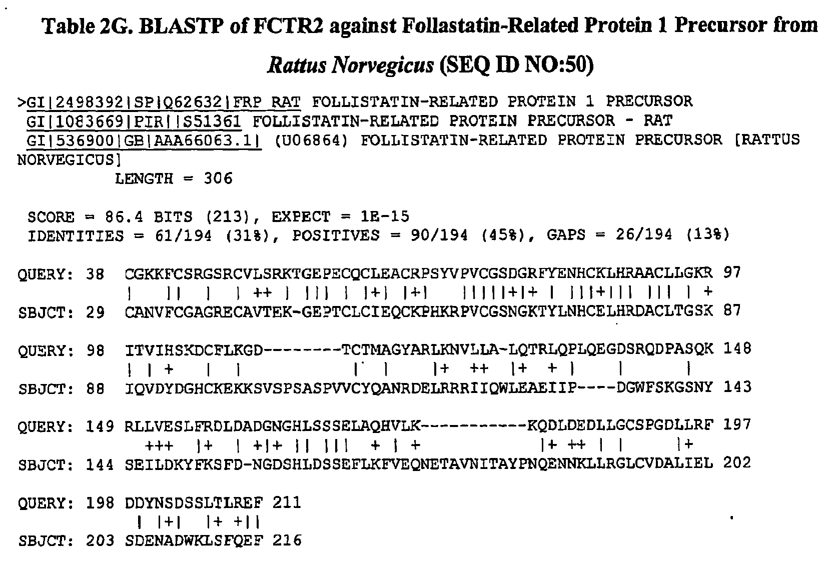

The amino acid sequence of the FCTR2 protein has 61 of 194 amino acid residues (31 %) identical to, and 89 of 194 residues (45%) positive with, the 306 amino acid residue protein Follastin-Related Protein 1 Precursor from *Mus musculus* (GenBank Acc:Q62356) (SEQ ID NO:51) (Table 2H).

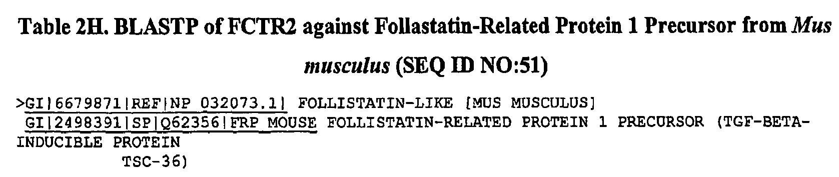

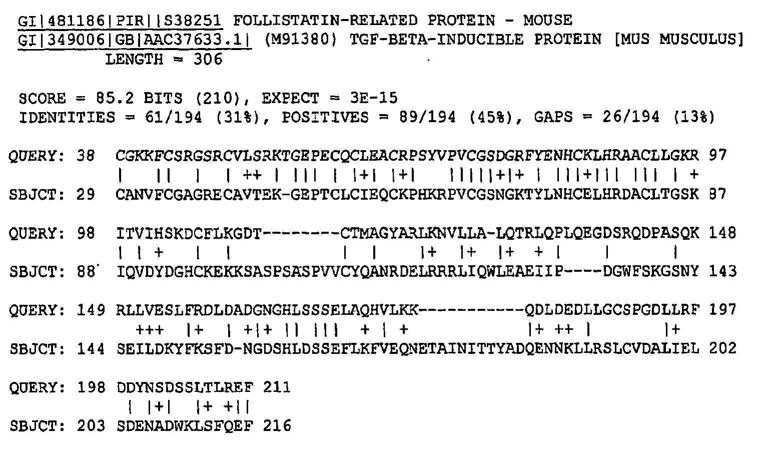

The amino acid sequence of the FCTR2 protein has 63 of 193 amino acid residues (32%) identical to, and 89 of 193 residues (45%) positive with, the 299 amino acid residue protein Follastatin-Related Protein from the African Clawed Frog (GenBank Acc:JG0187) (SEQ ID NO:52) (Table 2I).

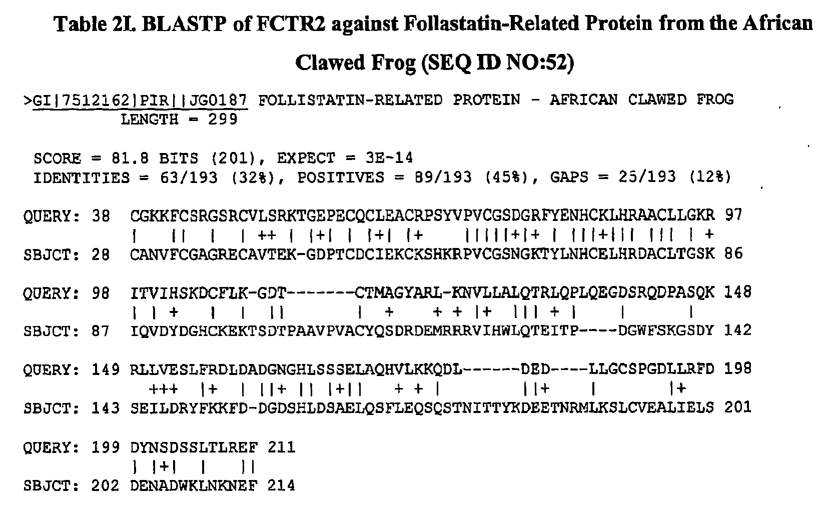

The amino acid sequence of the FCTR2 protein has 59 of 194 amino acid residues (30%) identical to, and 90 of 194 residues (45%) positive with, the 308 amino acid residue protein Follistatin-Related Protein 1 Precursor from *Homo sapiens* (GenBank Acc:Q12841) (SEQ ID NO:53) (Table 2J).

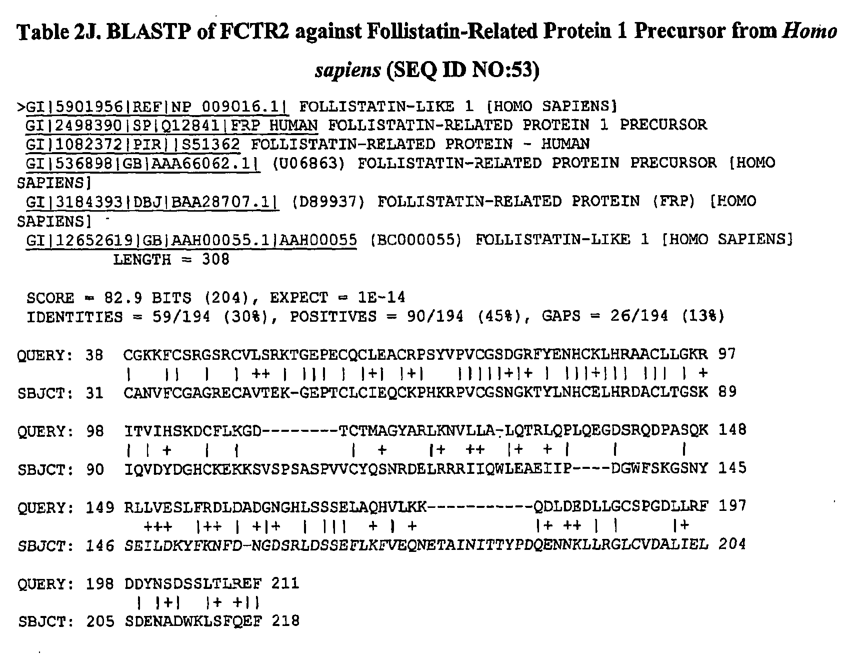

The amino acid sequence of the FCTR2 protein has 35 of 69 amino acid residues (50%) identical to, and 45 of 69 residues (64%) positive with, the 315 amino acid residue Flik protein [*Gallus gallus*] (EMBL Acc:CAB42968.1) (SEQ ID NO:54) (Table 2K).

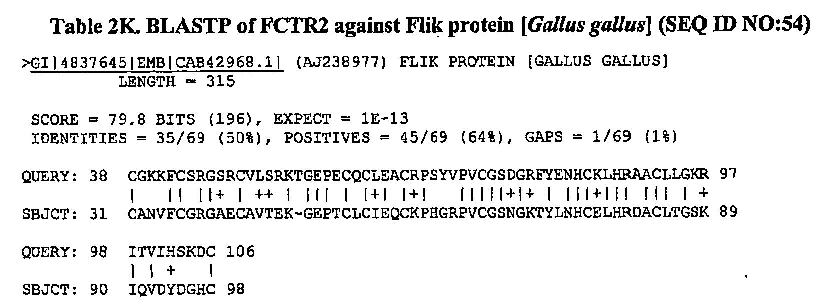

The amino acid sequence of the FCTR2 protein has 49 of 152 amino acid residues (32%) identical to, and 65 of 152 residues (42%) positive with a 272-420 amino acid fragment and, 31 of 83 residues (37%) identical to and 44 of 83 residues (52%) positive with a 248-329 amino acid fragment, both of the 1375 amino acid residue Frazzled gene protein [*Drosophila melanogaster*] (GenBankAcc:T13822) (SEQ ID NO:55) (Table 2L).

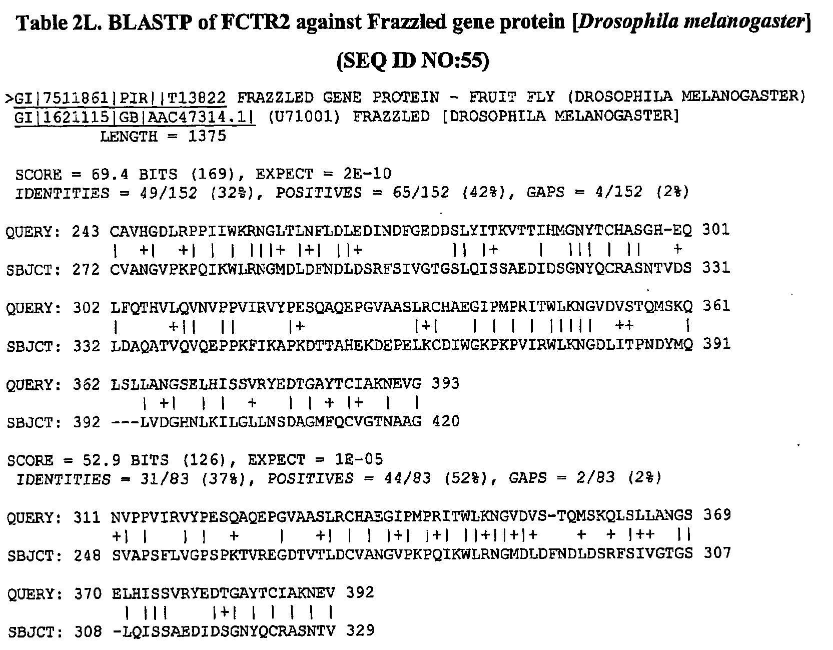

The amino acid sequence of the FCTR2 protein has 53 of 177 amino acid residues (29%) identical to, and 78 of 177 residues (43%) positive with a 366-539 amino acid fragment, 51 of 170 residues (30%) identical to and 74 of 170 residues (43%) positive with a 276-438 amino acid fragment, 46 of 165 amino acid residues (27%) identical to, and 74 of 165 amino acid residues positive with a 185-341 amino acid fragment, 48 of 167 amino acid residues (28%) identical to and 70 of 167 amino acid residues (41%) positive with a 77-243 amino acid fragment, and 28 of 84 amino acid residues (33%) and 37 of 84 amino acid residues positive with a 56-139 amino acid fragment all of the protein 1395 residue Roundabout 1 protein [*Drosophila melanogaster*] (GenBankAcc:AAC38849.1) (SEQ ID NO:56) (Table 2M).

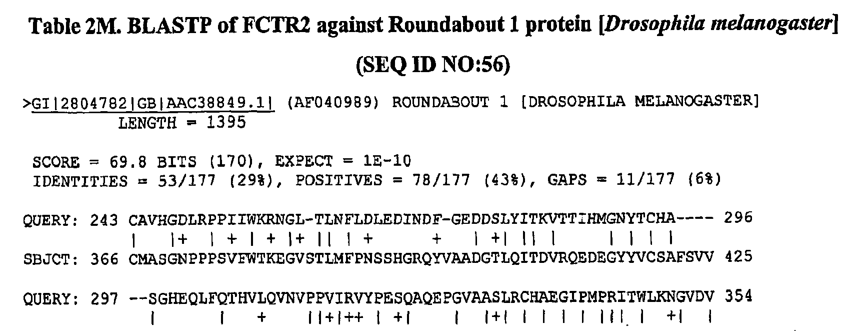

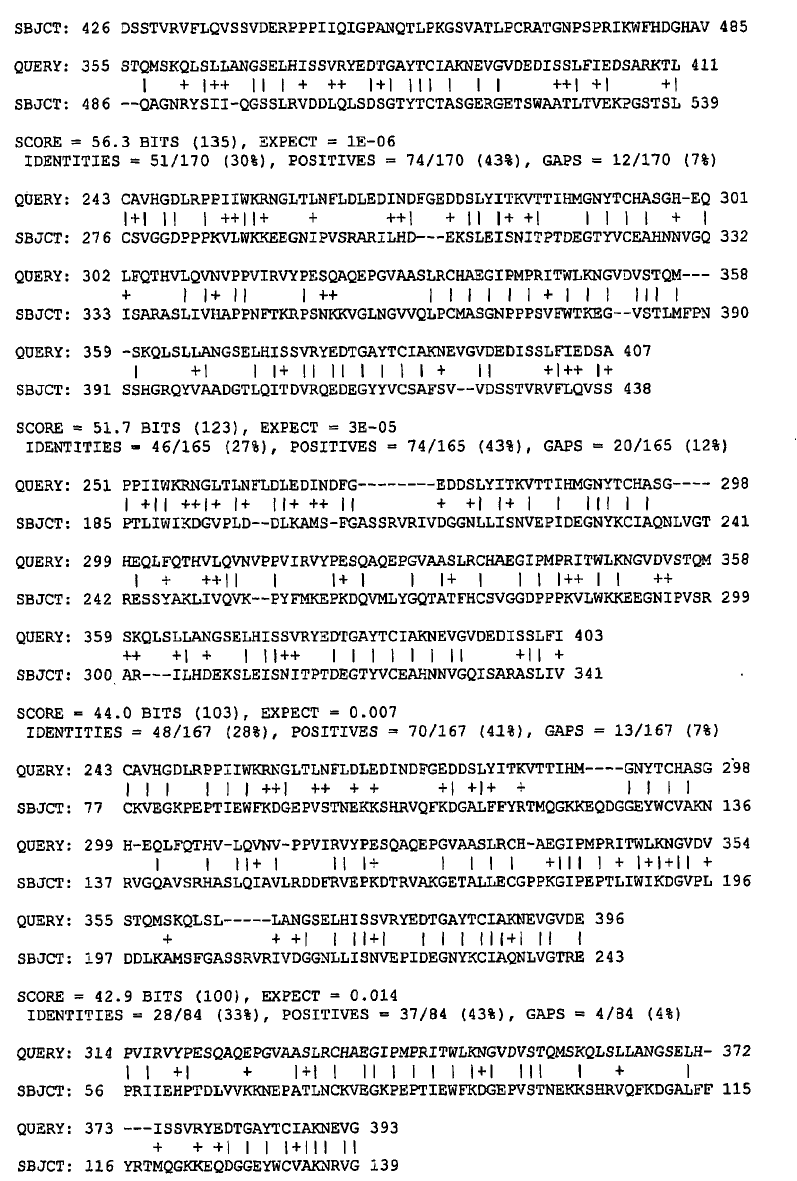

The amino acid sequence of the FCTR2 protein has 55 of 157 amino acid residues (35%) identical to, and 75 of 157 residues (47%) positive with a 620-775 amino acid fragment, 49 of 163 residues (30%) identical to and 71 of 163 residues (43%) positive with a 335-492 amino acid fragment, 32 of 85 amino acid residues (37%) identical to, and 48 of 85 amino acid residues (55%) positive with a 1305-1388 amino acid fragment, 37 of 143 amino acid residues (25%) identical to and 60 of 143 amino acid residues (41%) positive with a 183-319 amino acid fragment, 43 of 174 amino acid residues (24%) and 70 of 174 amino acid residues (39%) positive with a 711-884 amino acid fragment, and 46 of 165 residues (27%) identical to and 69 of 165 residues positive with a 831-884 amino acid fragment all of the protein 1395 residue Down Syndrome Cell Adhesion Molecule Precursor (CHD2) from *Homo Sapiens* (GenBankAcc:060469) (SEQ ID NO:57) (Table 2N).

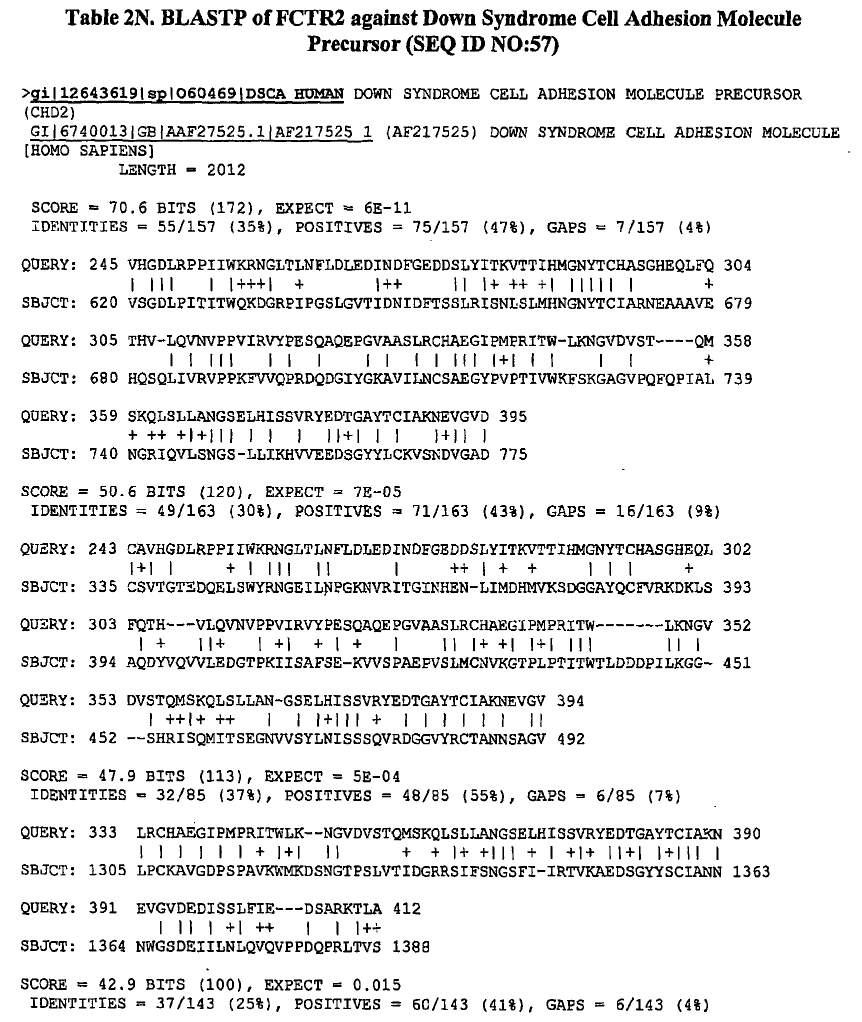

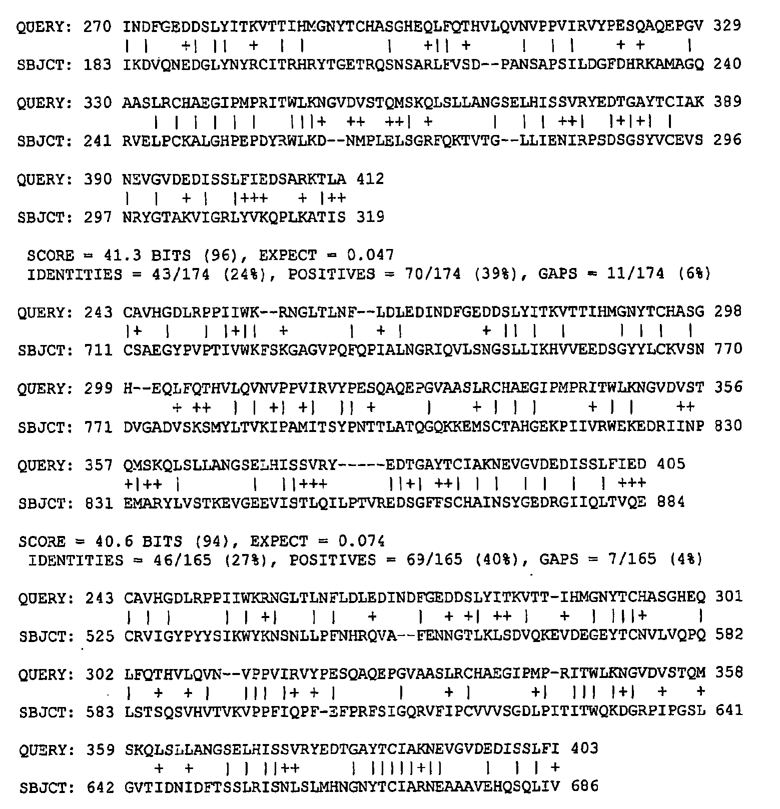

The amino acid sequence of the FCTR2 protein has 55 of 194 amino acid residues (28%) identical to, and 86 of 194 residues (44%) positive with Limbic System-Associated Membrane Protein Precursor (LSAMP) from *Homo sapiens* (SWISSPROT Acc:Q13449) (SEQ ID NO:58) (Table 20).

**Table 2O. BLASTP of FCTR2 against Limbic System-Associated Membrane Protein Precursor (SEQ ID NO:58)**

| |
|---|
| PTNR:SWISSPROT-ACC:Q13449 LIMBIC SYSTEM-ASSOCIATED MEMBRANE PROTEIN PRECURSOR (LSAMP) - HOMO SAPIENS (HUMAN), 338 AA. |
| LENGTH = 338 |
| SCORE = 191 (67.2 BITS), EXPECT = 6.7E-12, P = 6.7E-12 |
| IDENTITIES = 55/199 (28%), POSITIVES - 86/194 (44%) |

The amino acid sequence of the FCTR2 protein has 68 of 190 amino acid residues (35%) identical to, and 92 of 190 residues (48%) positive with Putative Neuronal Cell Adhesion Molecule, Short Form from *Mus musculus* (SPTREMBL Acc:O70246) (SEQ ID NO:59) (Table 2P).

**Table 2P. BLASTP of FCTR2 against Putative Neuronal Cell Adhesion Molecule, Short Form from Mus musculus (SEQ ID NO:59)**

| |
|---|
| PTNR:SPTREMBL-ACC:O70246 PUTATIVE NEURONAL CELL ADHESION MOLECULE (PUNC) (PUTATIVE NEURONAL CELL ADHESION MOLECULE, SHORT FORM) - MUS MUSCULUS (MOUSE), 793 AA |
| LENGTH = 793 |
| SCORE = 203 (71.5 BITS), EXPECT = 7.0E-12, SUM P(2) = 7.0E-12 |
| IDENTITIES = 68/190 (35%), POSITIVES = 92/190 (48%) |

The amino acid sequence of the FCTR2 protein has 58 of 199 amino acid residues (29%) identical to, and 91 of 199 residues (45%) positive with CHLAMP, G11-Isoform Precursor from *Gallus gallus* (SPTREMBL Acc: 002869) (SEQ ID NO:60) (Table 2Q).

**Table 2Q. BLASTP of FCTR2 against CHLAMP, G11-Isoform Precursor from Gallus gallus (SEQ ID NO:60)**

| |
|---|
| PTNR:SPTREMBL-ACC:002869 CHLAMP, G11-ISOFORM PRECURSOR - GALLUS GALLUS (CHICKEN), 350 AA. |
| LENGTH = 350 |
| SCORE = 191 (67.2 BITS), EXPECT = 7.7E-12, P = 7.7E-12 |
| IDENTITIES = 58/199 (29%), POSITIVES = 91/199 (45%) |

The amino acid sequence of the FCTR2 protein has 55 of 194 amino acid residues (28%) identical to, and 86 of 194 residues (44%) positive with Limbic System-Associated Membrane Protein Precursor (LSAMP) from *Rattus norvegicus* (SWISSPROT Acc:Q62813) (SEQ ID NO:61) (Table 2R).

**Table 2R. BLASTP of FCTR2 against Limbic System-Associated Membrane Protein Precursor (LSAMP) from Rattus norvegicus (SEQ ID NO:61)**

| |
|---|
| PTNR:SWISSPROT-ACC:Q62813 LIMBIC SYSTEM-ASSOCIATED MEMBRANE PROTEIN PRECURSOR (LSAMP) - RATTUS NORVEGICUS (RAT), 338 AA. |
| LENGTH = 338 |
| SCORE - 188 (66.2 BITS), EXPECT = 1.5E-11, P = 1.5E-11 |
| IDENTITIES = 55/194 (28%), POSITIVES = 86/194 (44%) |

FCTR2 protein has similarity to cell adhesion molecules, follistatin, roundabout and frazzled (see BlastP results). These genes are involved in neuronal development and reproductive physiology. Frazzled encodes a Drosophila member of the DCC immunoglobulin subfamily and is required for CNS and motor axon guidance (Cell 87:197-204(1996)). Characterization of a rat C6 glioma-secreted follistatin-related protein (FRP) and cloning and sequence of the human homologue is described in Eur. J. Biochem. 225:937-946(1994). This protein may modulate the action of some growth factors on cell proliferation and differentiation. FRP binds heparin. The follistatin-related protein is a secreted protein and has one follistatin-like domain. The cloning and early dorsal axial expression of Flik, a chick follistatin-related gene and evidence for involvement in dorsalization/neural induction is presented in Dev. Biol. 178:327-342(1996). Roundabout controls axon crossing of the CNS midline and defines a novel subfamily of evolutionarily conserved guidance receptors, as shown in Cell 92:205-215(1998). cDNA cloning and structural analysis of the human limbic-system- associated membrane protein (LAMP) is described in Gene 170:189-195(1996). LAMP, a protein of the OBCAM family that contains three immunoglobulin-like C2-type domains, mediates selective neuronal growth and axon targeting. LAMP contributes to the guidance of developing axons and remodeling of mature circuits in the limbic system. This protein is essential for normal growth of the hippocampal mossy fiber projection. LAMP is attached to the membrane by a GPI-Anchor. It is expressed on limbic neurons and fiber tracts as well as in single layers of the superior colliculus, spinal chord and cerbellum. Characterization of the human full-length PTK7 cDNA encoding a receptor protein tyrosine kinase-like molecule closely related to chick KLG is disclosed in J. Biochem. 119:235-239(1996). Based upon homology, FCTR2 proteins and each homologous protein or peptide may share at least some activity.

### Functions and therapeutic uses:

The OMIM gene map has identified this region which the invention maps to (5q21-5q31) as associated with susceptibility to the following diseases (OMIM Ids are underlined):
- Allergy and asthma
- Hemangioma,
- capillary infantile Schistosoma mansoni infection, susceptibility/resistance to Spinocerebellar ataxia
- Bronchial asthma
- Plasmodium falciparum parasitemia,
- intensity of Corneal dystrophy, Groenouw type I, 121900; Corneal dystrophy,lattice type I, 122200;
- Reis-Bucklers corneal dystrophy;Corneal dystrophy, Avellino type Eosinophilia, familial Myelodysplastic syndrome;
- Myelogenous leukemia, Acute Cutis laxa, recessive, type I, Deafness, autosomal dominant nonsyndromic sensorineural, 1 Contractural arachnodactyly, Congenital Neonatal alloimmune thrombocytopenia;
- Glycoprotein Ia deficiency Male infertility;
- Charcot-Marie-Tooth neuropathy, Demyelinating Gardner syndrome;
- Adenomatous polyposis coli;
- Colorectal cancer;
- Desmoid disease, hereditary, 135290;
- Turcot syndrome,276300;
- Adenomatous polyposis coli, attenuated
- Colorectal cancer

Therefore the invention is implicated in at least all of the above mentioned diseases and may have therapeutic uses for these diseases.

This sequence has similarity to cell adhesion molecules, follistatin, roundabout and frazzled (see BlastP results). These genes are involved in neuronal development and reproductive physiology. Therefore the invention is also implicated in disorders such as or therapeutic uses for:
- Neurodegenerative disorders, nerve trauma, epilepsy, mental health conditions
- Tissue regeneration in vivo and in vitro
Female reproductive system disorders and pregnancy

### FCTR3

FCTR3, is an amino acid type II membrane, neurestin-like protein. The FCTR3a nucleic acid of 1430 nucleotides (also designated 10129612.0.118) is shown in Table 3A. An ORF was identified beginning with an ATG initiation codon at nucleotides 69-71 and ending with a TAG codon at nucleotides 1212-1214. A putative untranslated region upstream from the initiation codon and downstream from the termination codon is underlined in Table 3A, and the start and stop codons are in bold letters.

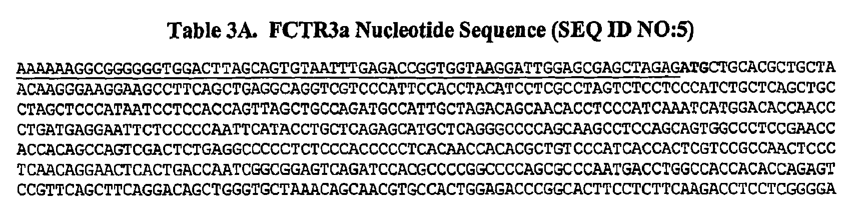

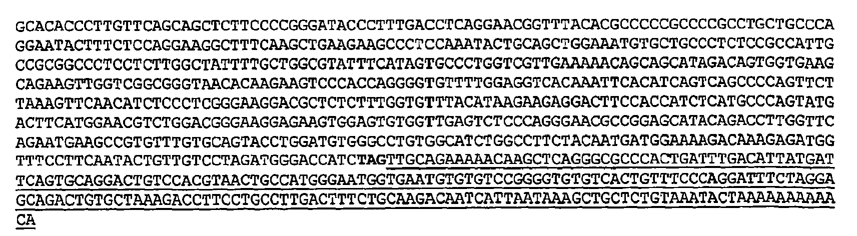

The FCTR3 polypeptide (SEQ ID NO:5) encoded by SEQ ID NO:5 is 381 amino acid residues and is presented using the one-letter code in Table 3B.

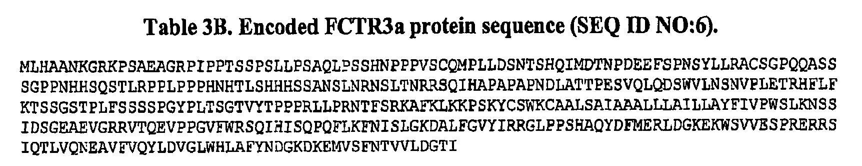

In an alternative embodiment, the 5' end of the FCTR3a nucleic acid could be extended as it is in the 9826bp FCTR3b (also referred to herein as 10129612.0.405) shown in Table 3C. An ORF was identified beginning with an ATG initiation codon at nucleotides 280-282 and ending with a TAA codon at nucleotides 8479-8481. A putative untranslated region upstream from the initiation codon and downstream from the termination codon is underlined in Table 3C, and the start and stop codons are in bold letters. Italicized bases 1-201 refer to a variable 5' region that will be further discussed below.

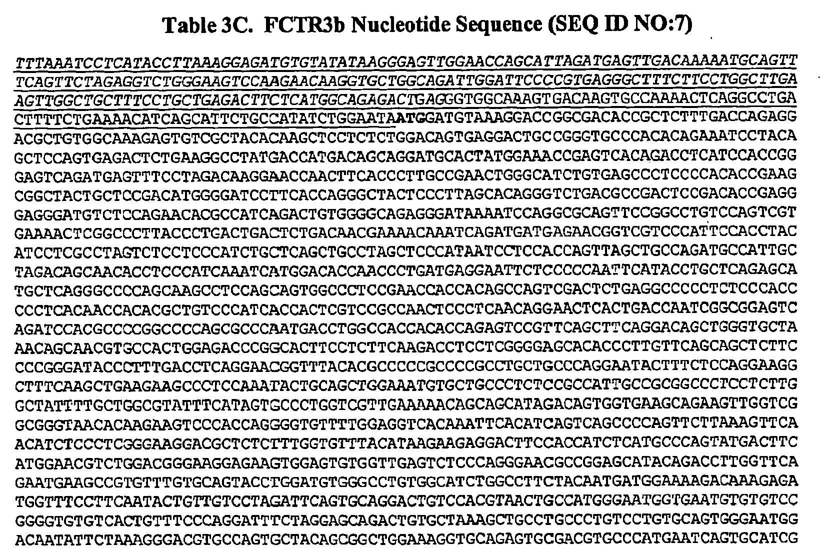

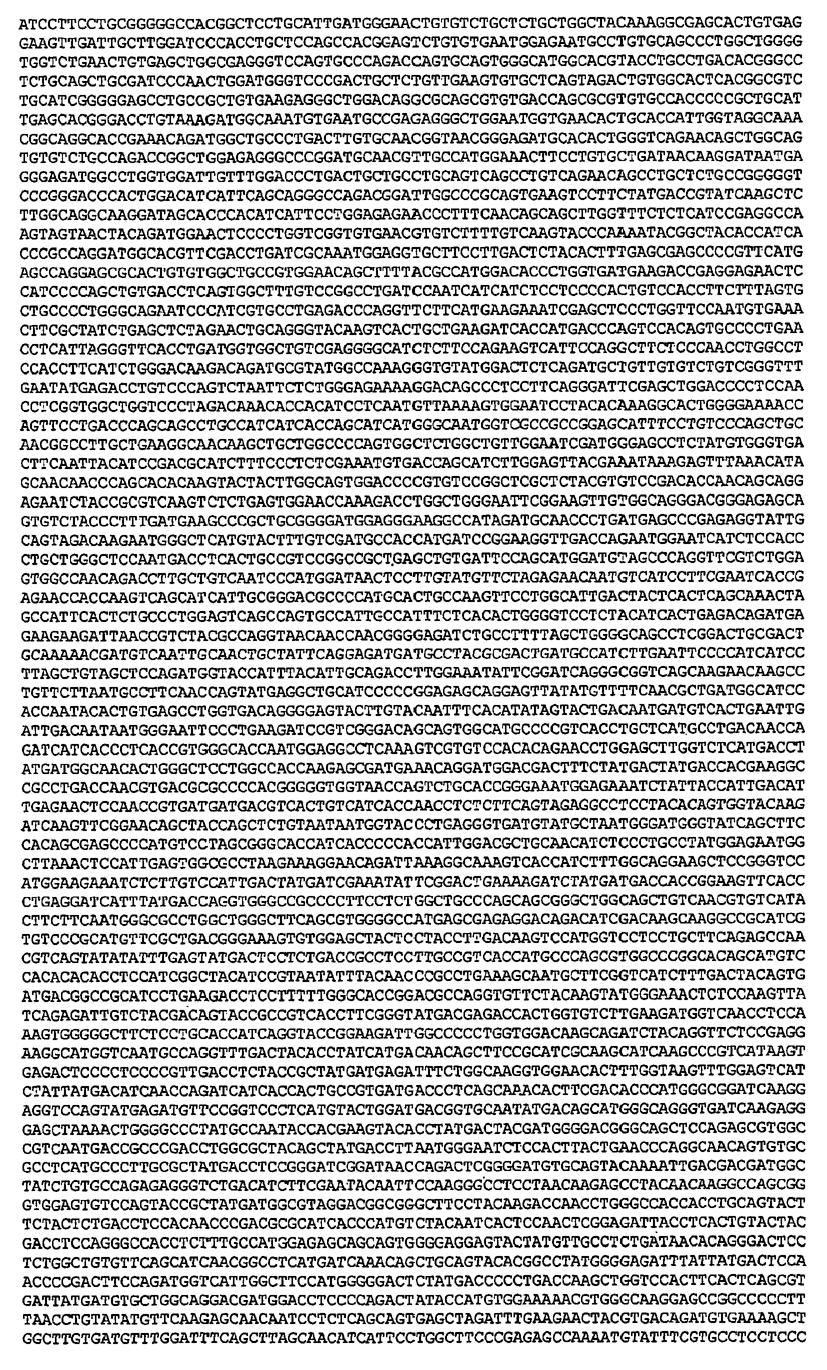

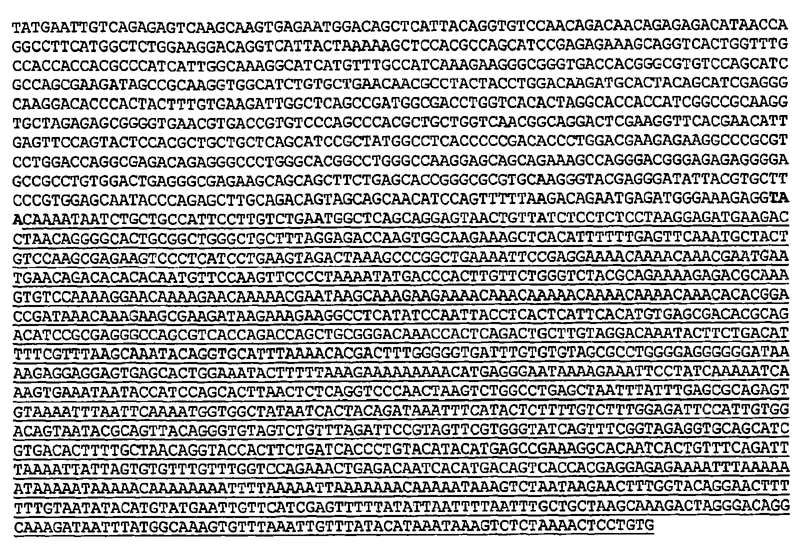

The FCTR3b polypeptide (SEQ ID NO:8) encoded by SEQ ID NO:7 is 2733 amino acid residues and is presented using the one-letter code in Table 3D. The protein has a predicted molecular weight of 303424.3 daltons.

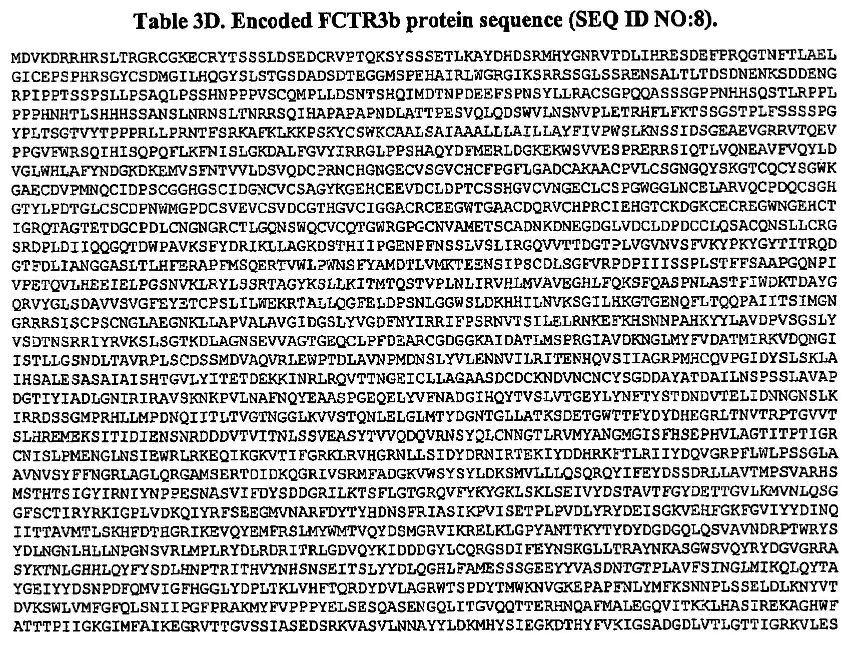

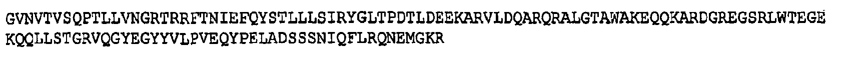

In further alternative embodiments the italicized bases in the 5' end of the FCTR3b sequence in table 3C is a variable region. This region can be substituted for in other embodiments of FCTR3. The nucleotide sequence for 9823bp FCTR3c (also referred to herein as 10129612.0.154) has the same nucleotide sequence as FCTR3b except that the italicized region is replaced with the 201 base sequence shown in Table 3E. An ORF for the total FCTR3c nucleotide sequence was identified beginning with an ATG initiation codon at nucleotides 277-280 and ending with a TAG codon at nucleotides 8473-8475. This is the same open reading frame that is shown in Table 3C, with the corresponding base numbers for FCTR3c. This open reading frame will translate the same amino acid sequence as shown in Table 3C for FCTR3b.

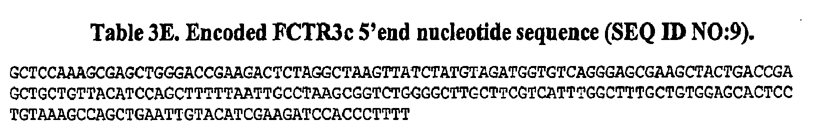

In yet another embodiment, the italicized region shown in the 5' end of the sequence in Table 3C can be replaced with the sequence shown in Table 3F to form 9823bp FCTR3d (also referred to herein as 10129612.0.67). An ORF was identified beginning with an ATG initiation codon at nucleotides 277-280 and ending with a TAG codon at nucleotides 8473-8475. This is the same open reading frame that is shown in Table 3C, with the corresponding base numbers for FCTR3d. This open reading frame will translate the same amino acid sequence as shown in Table 3D for FCTR3b.

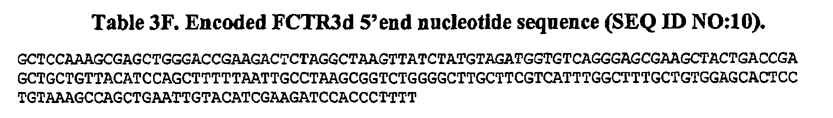

In yet another embodiment, the italicized region shown in the 5' end of the sequence in Table 3C can be replaced with the sequence shown in Table 3G to form 9765 bp FCTR3e (also referred to as 10129612.0.258). An ORF was identified beginning with an ATG initiation codon at nucleotides 210-212 and ending with a TAG codon at nucleotides 8408-8410. This is the same open reading frame that is shown in Table 3C, with the corresponding base numbers for FCTR3e. This open reading frame will translate the same amino acid sequence as shown in Table 3D for FCTR3b.

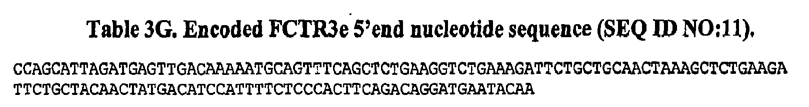

In yet another embodiment another FCTR3a homolog, FCTR3f (also referred to as 10129612.0.352) was found having the 9729bp sequence shown in Table 3H. An ORF was identified beginning with an ATG initiation codon at nucleotides 210-212 and ending with a TAG codon at nucleotides 8382-8384. A putative untranslated region upstream from the initiation codon and downstream from the termination codon is underlined in Table 3G, and the start and stop codons are in bold letters.

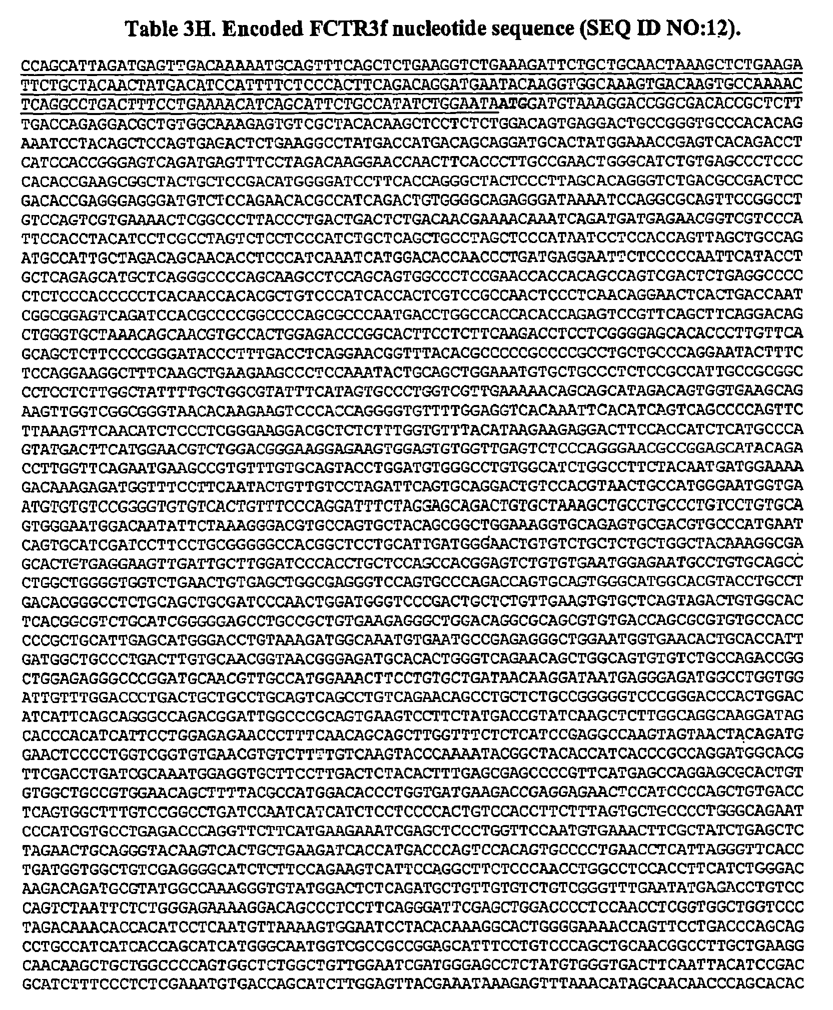

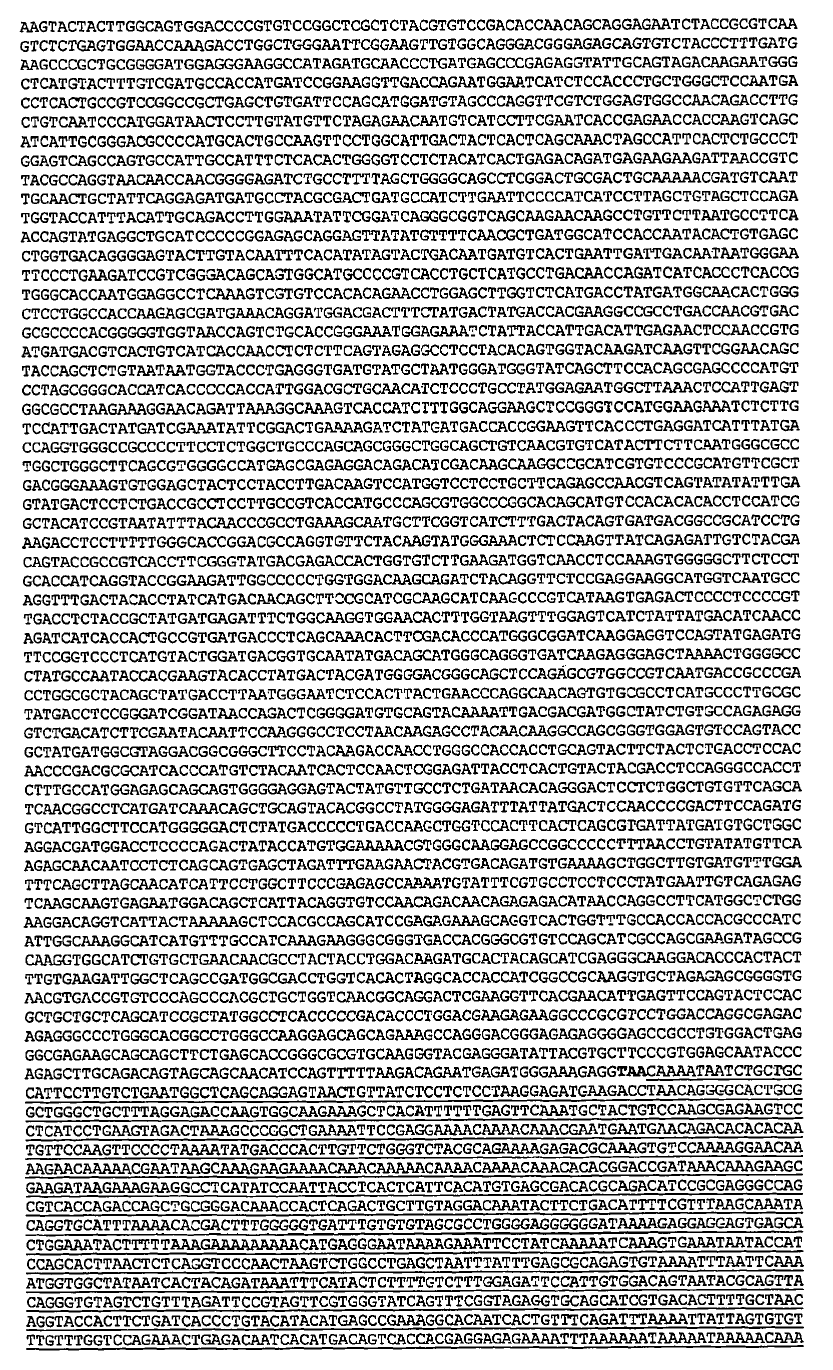

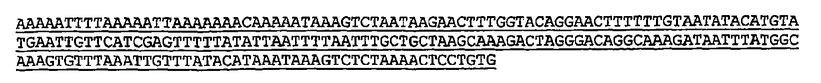

The FCTR3f polypeptide (SEQ ID NO:13) encoded by SEQ ID NO:12 is 2724 amino acid residues long and is presented using the one-letter code in Table 3I. This sequence differs from FCTR3b in that it is missing amino acids 758-766 from that polypeptide.

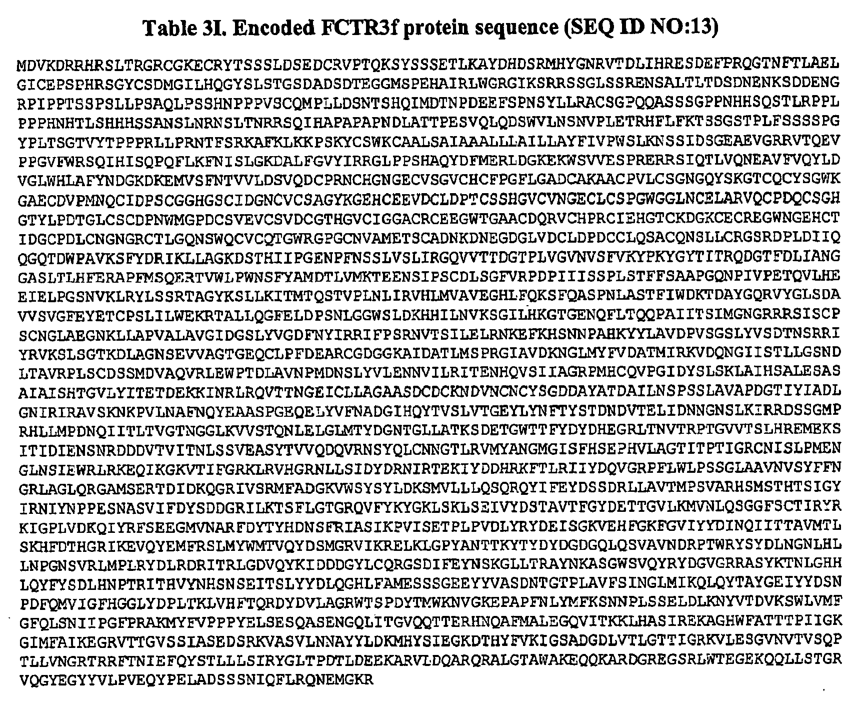

In a BLASTN search it was found that the FCTR3a nucleic acid has homology to three fragments of *Mus musculus* odd Oz/ten-m homolog 2. It has 634 of 685 bases (92%) identical to bases 614-1298, 365 of 406 bases (89%) identical to bases 1420-1825, and 93 of 103 bases (90%) identical to bases 1823-1925 of *Mus musculus* odd Oz/ten-m homolog 2 (GenBank Acc: NM_011856.2) (Table 3J).

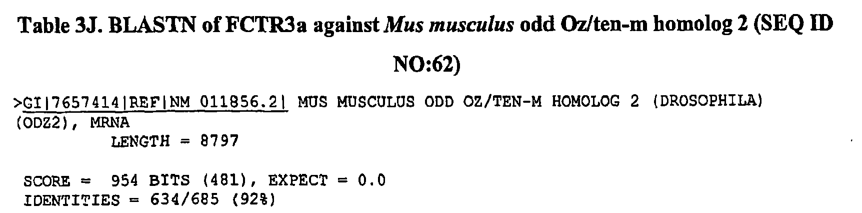

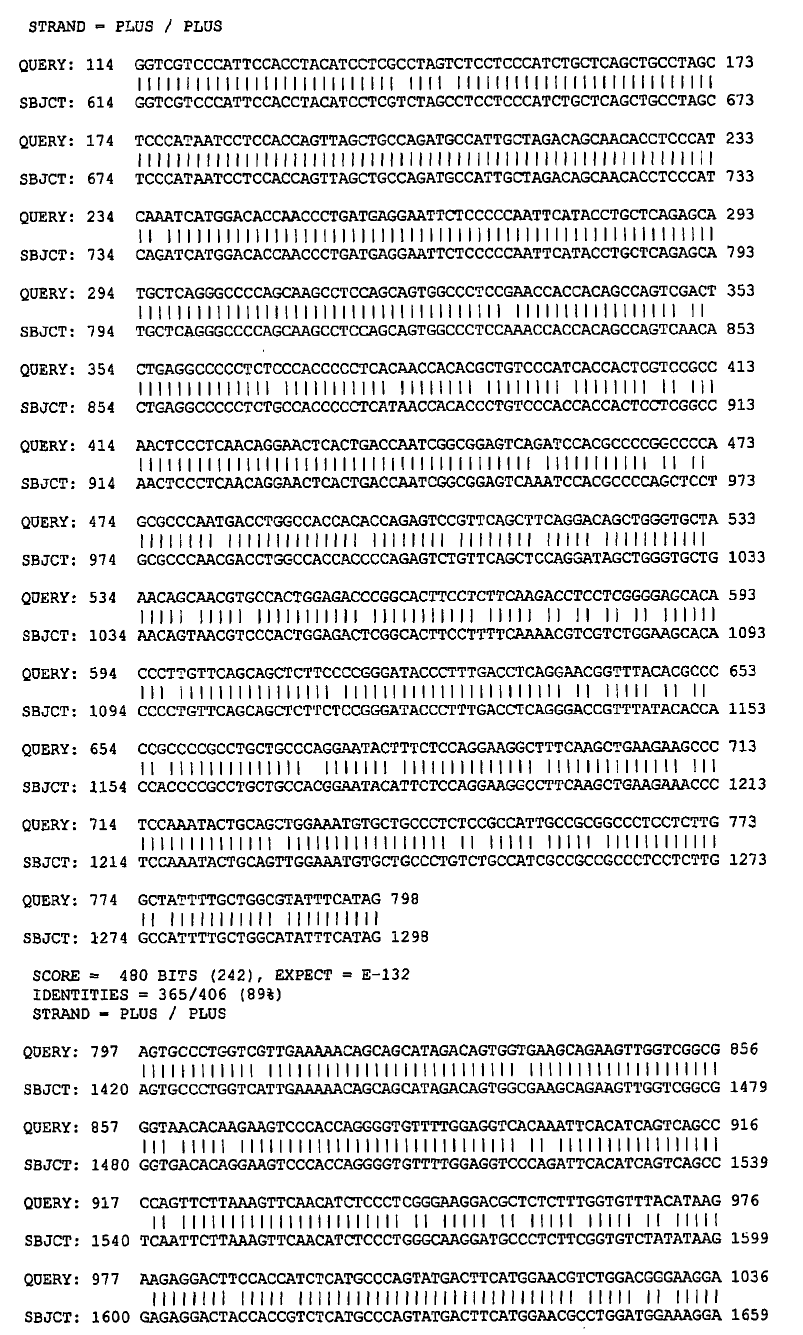

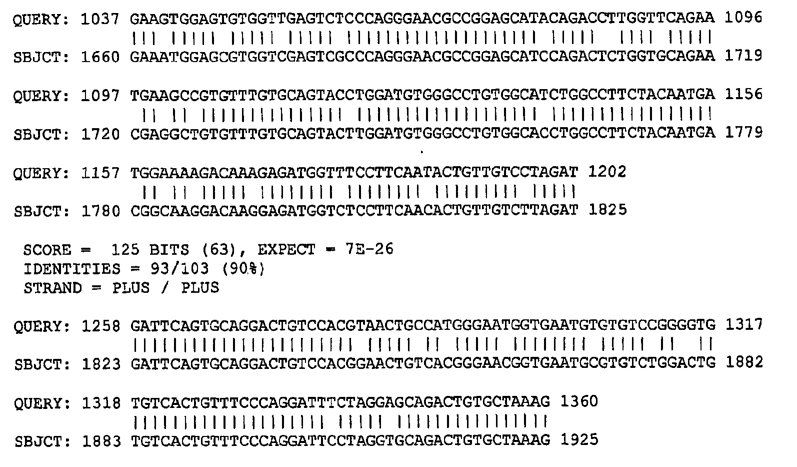

In another BLASTN search it was found that the FCTR3a nucleic acid has homology to three fragments of *Gallus gallus* mRNA for teneurin-2. It has 541 of 629 bases (86%) identical to bases 502-1130, 302 of 367 bases (82%) identical to bases 1330-1696, and 87 of 103 bases (84%) identical to bases 1711-1813 of *Gallus gallus* mRNA for teneurin-2 (EMBL Acc: AJ245711.1) (Table 3K).

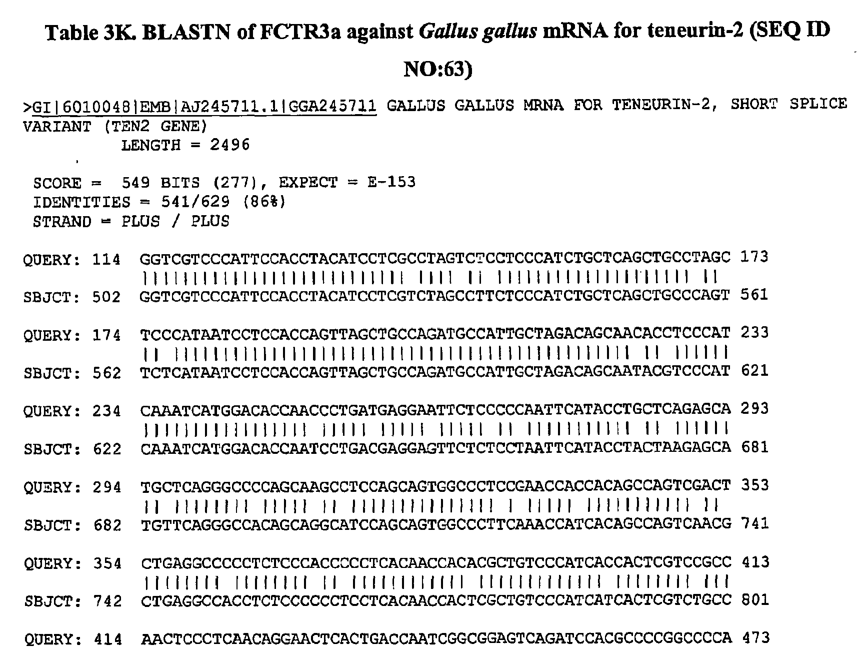

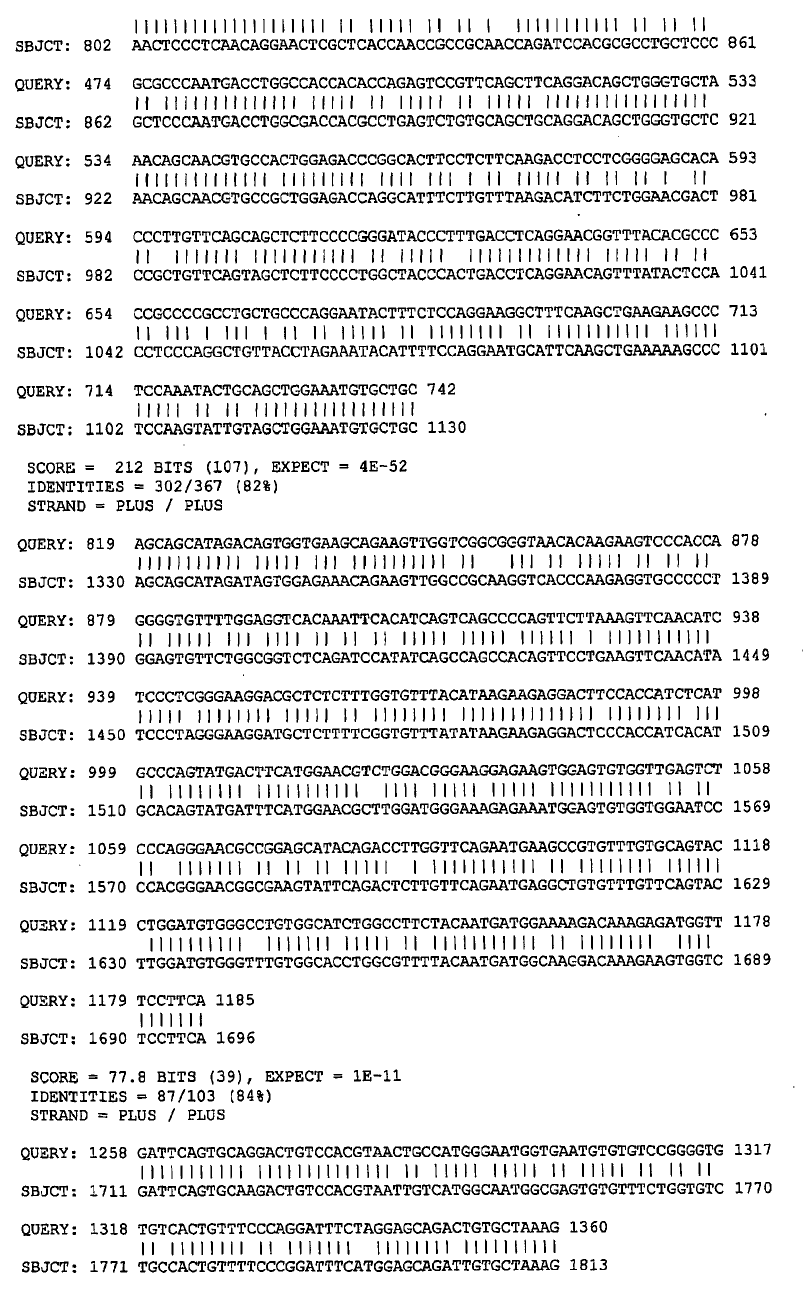

In this search it was also found that the fragments of FCTR3bcd and e nucleic acids had homology to three fragments of *Homo sapiens* mRNA for KIAA1127 protein. It has 5537 of 5538 bases (99%) identical to bases 1-5538, 705 of 714 bases (98%) identical to bases 5609-6322, and 176 of 176 bases (100%) identical to bases 6385-6560 of *Homo sapiens* mRNA for KIAA1127 protein (GenBank Acc: AB032953) (Table 3L).

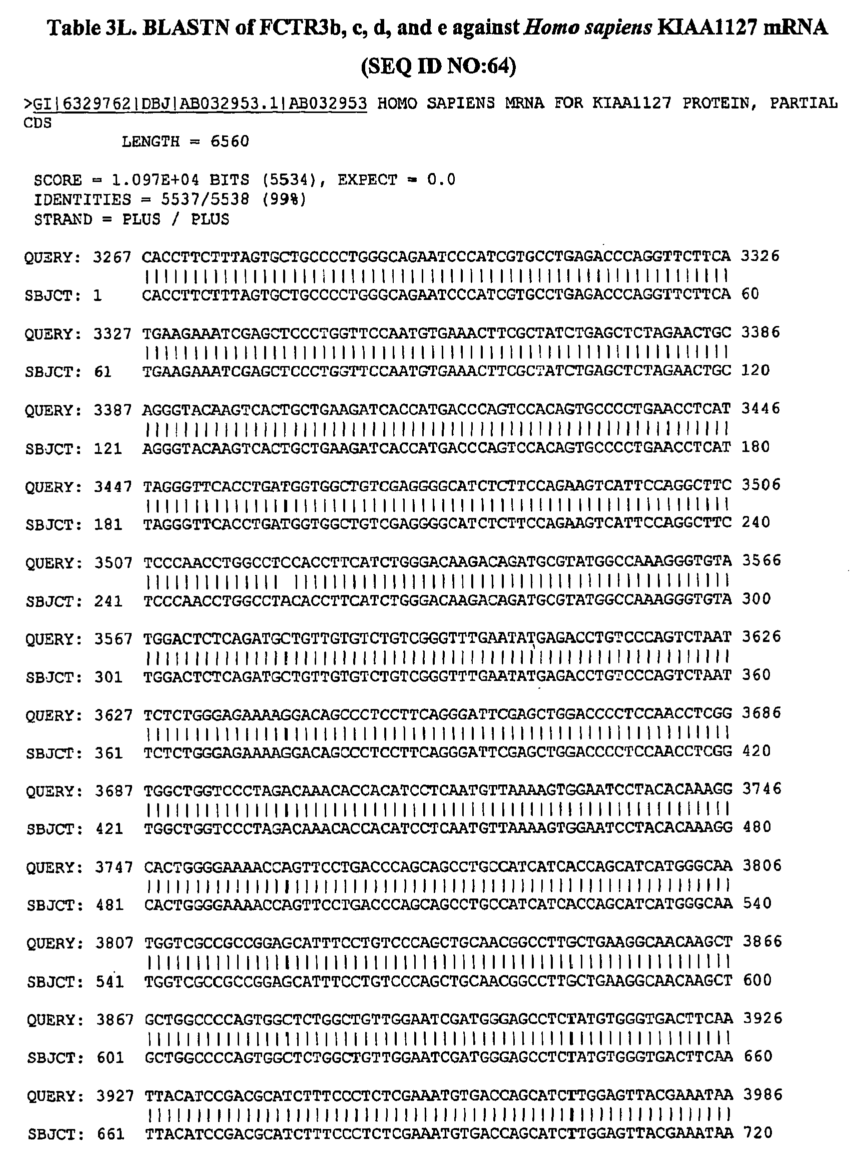

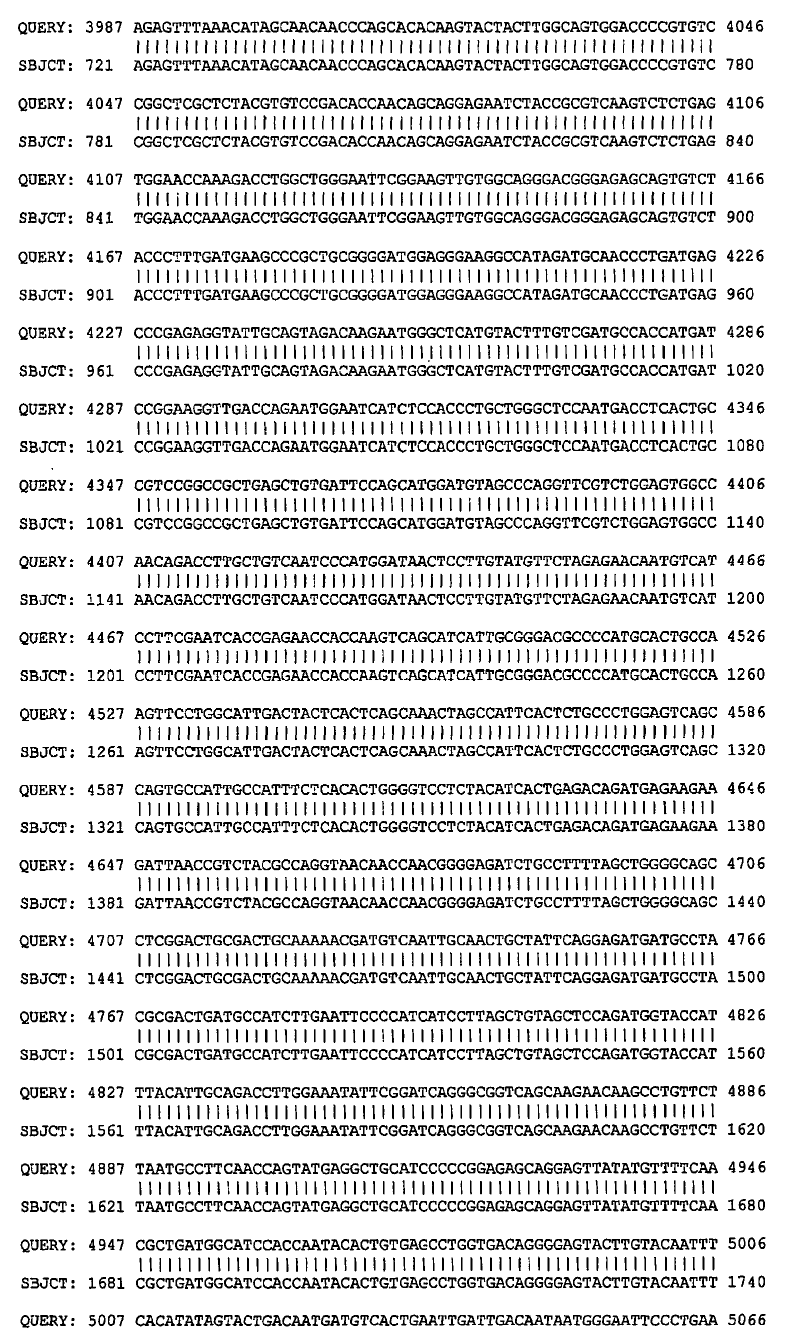

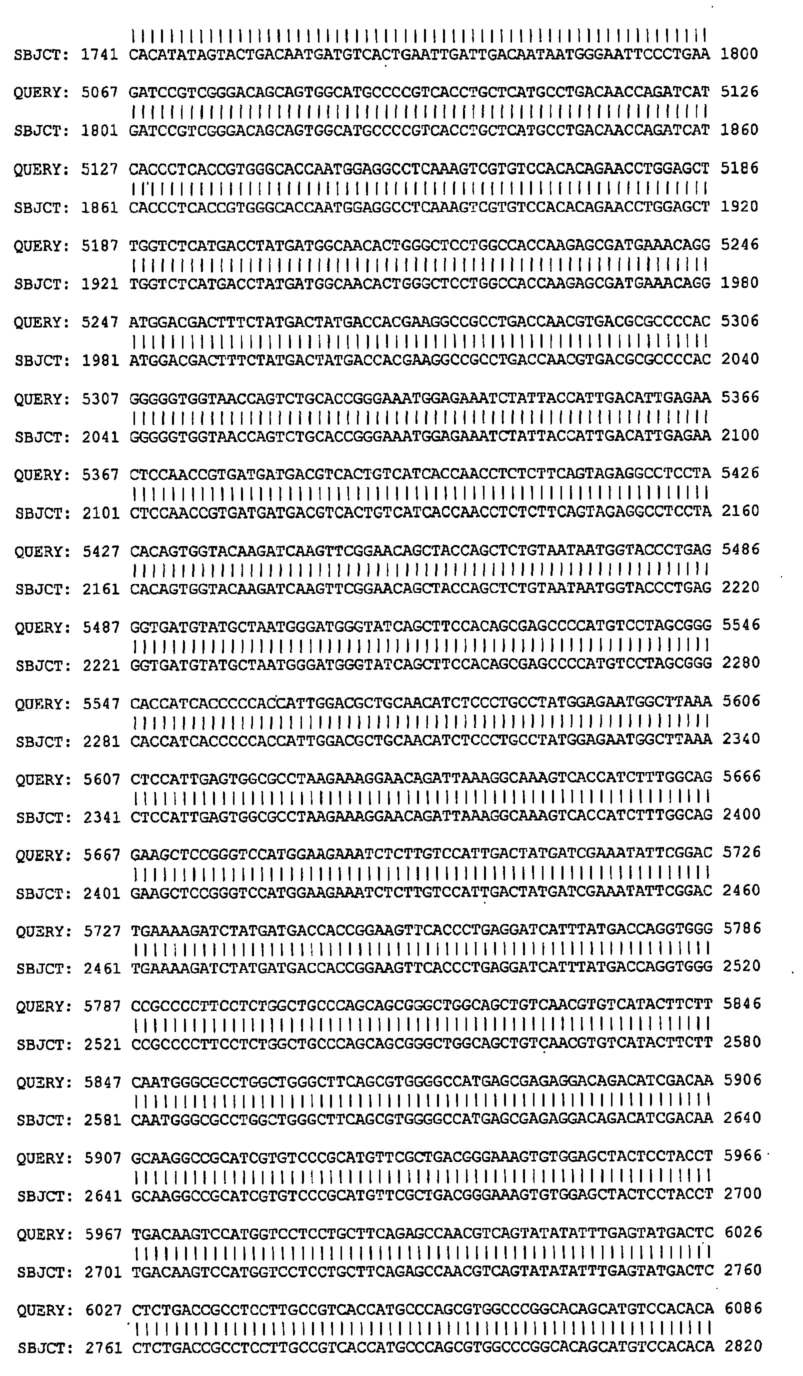

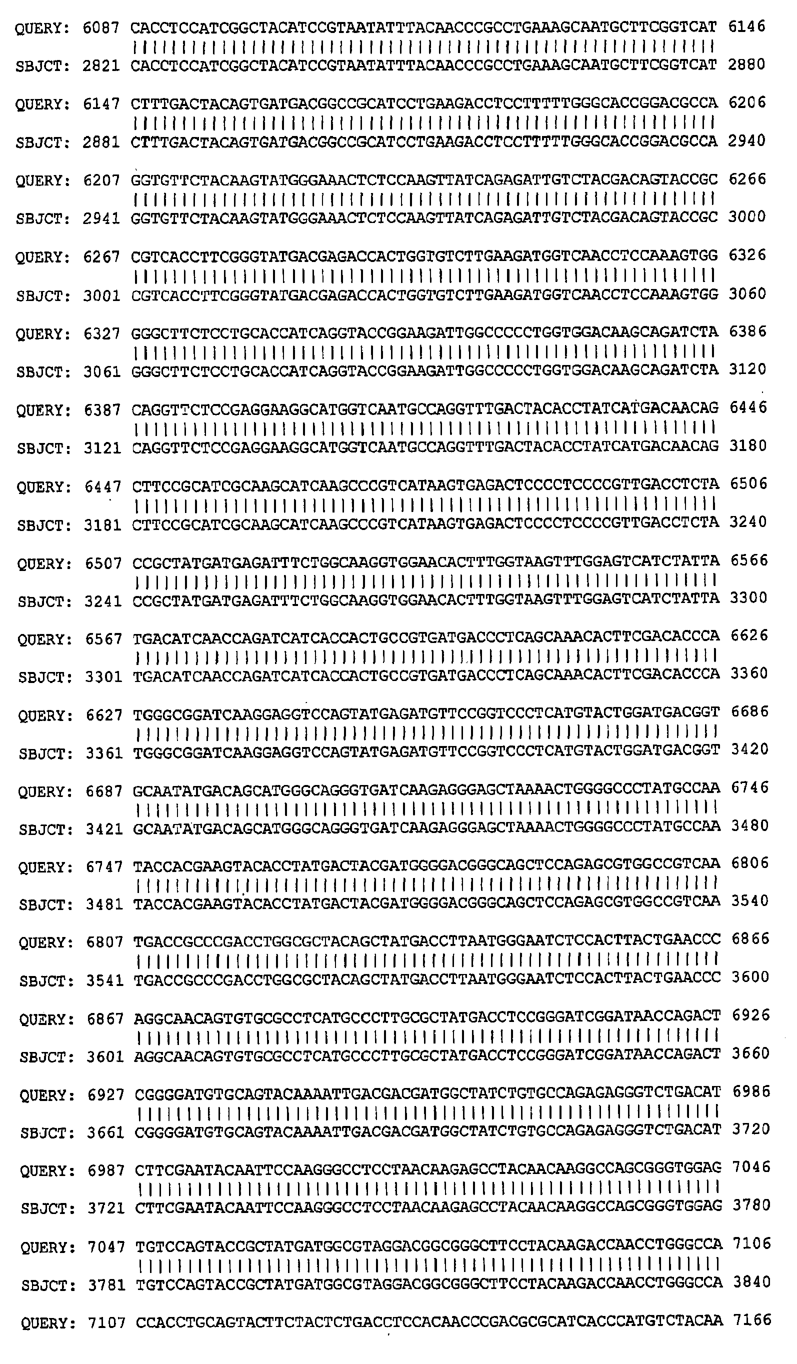

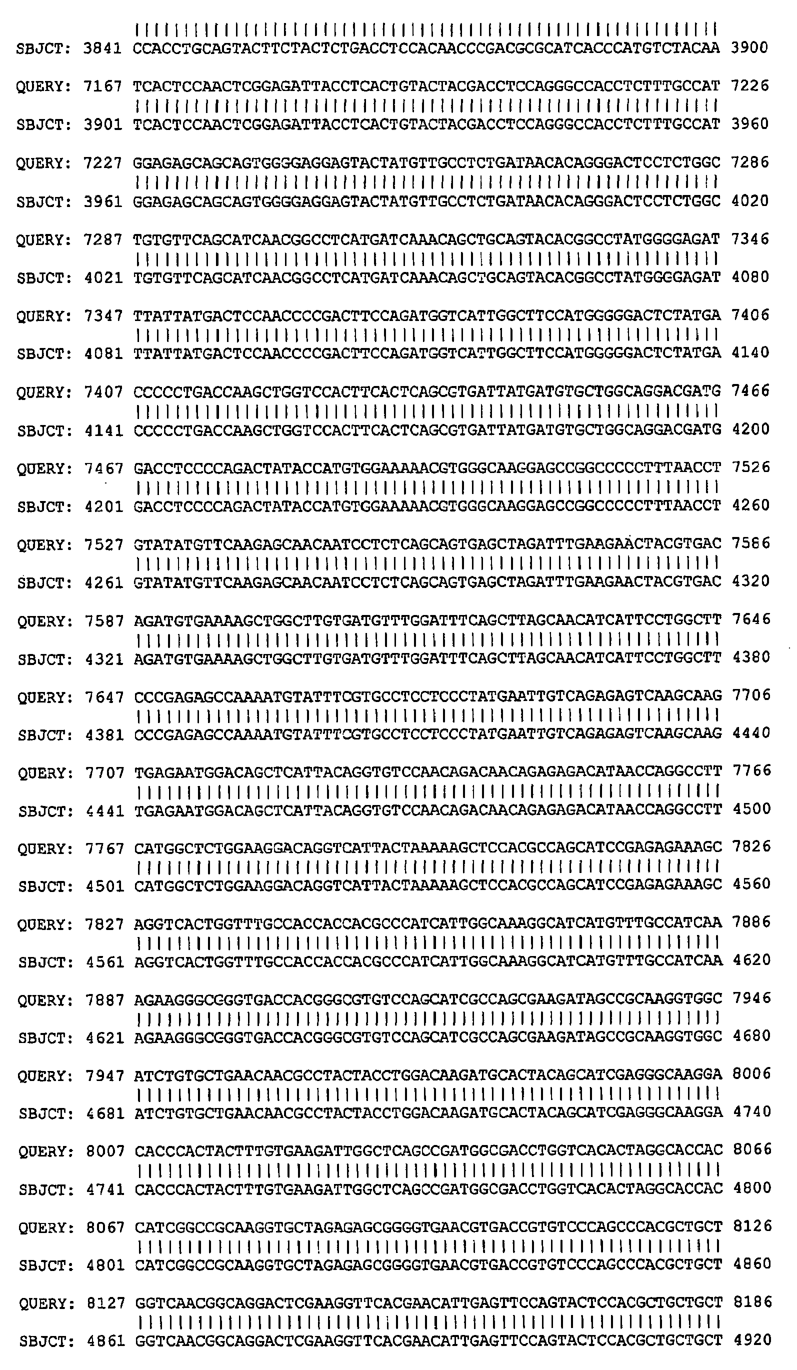

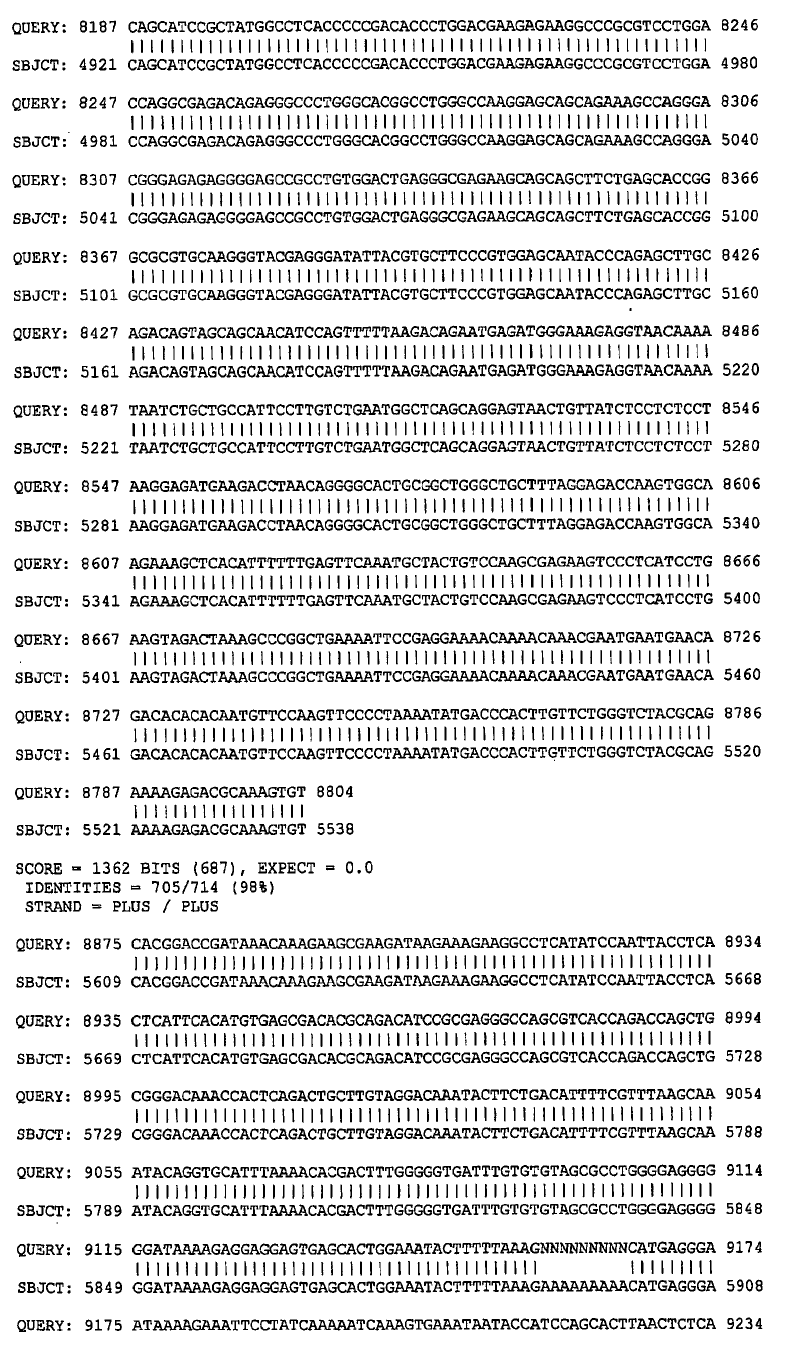

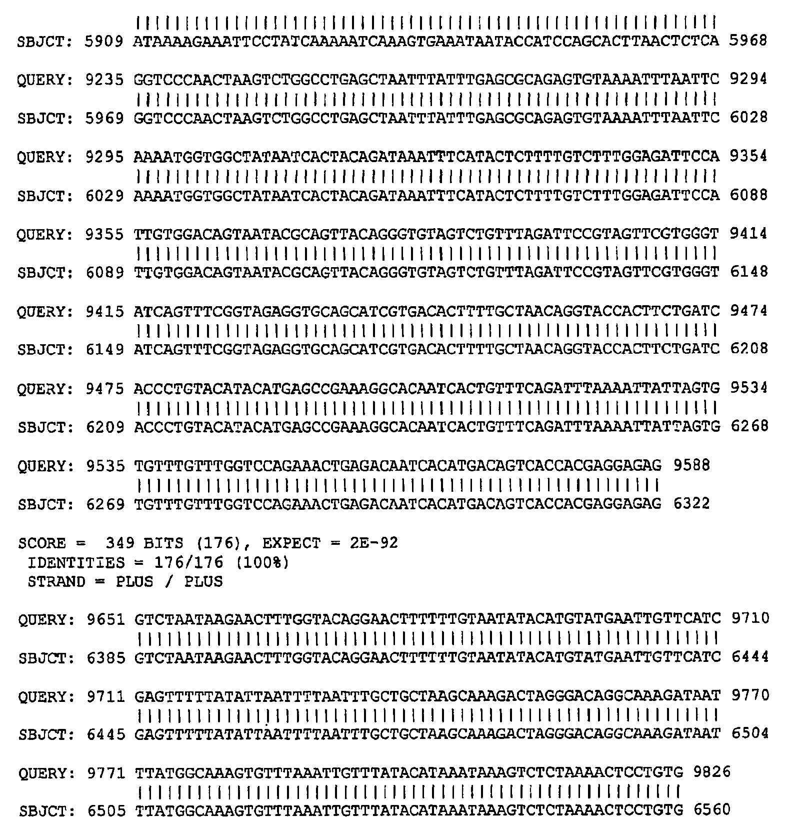

In this search it was also found that the FCTR3bcd and e nucleic acids had homology to five fragments of *Mus musculus* mRNA for Ten-m2. It has 5498 of 6108 bases (90%) identical to bases 2504-8610, 1095 of 1196 bases (91%) identical to bases 103-1298, 1000 of 1088 bases (91%) identical to bases 1420-2540, 81 of 89 bases (91%) identical to bases 8655-8743, and 30 of 32 bases (93%) identical to bases 7-38 of *Mus musculus* mRNA for Ten-m2 (Table 3M).

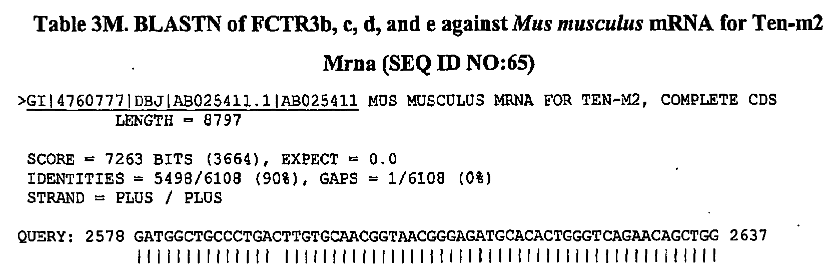

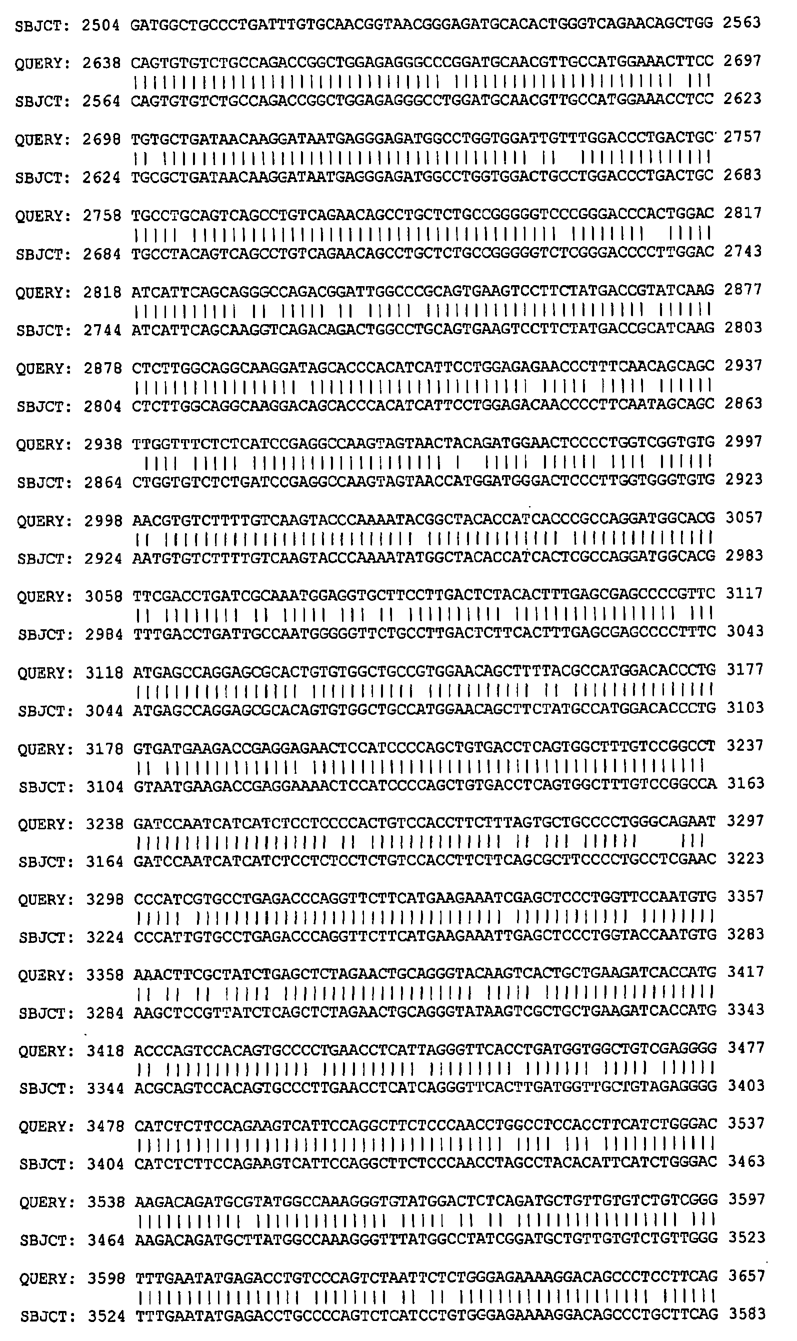

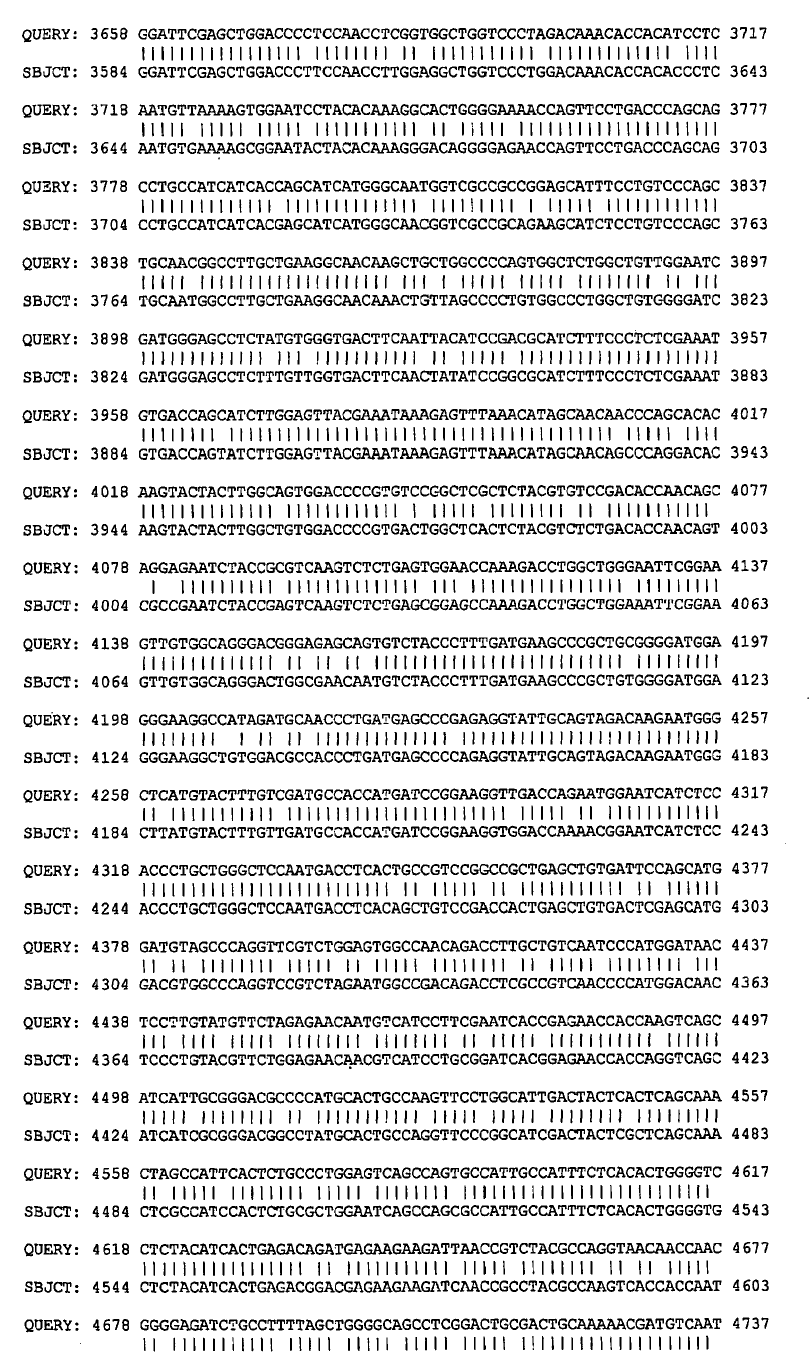

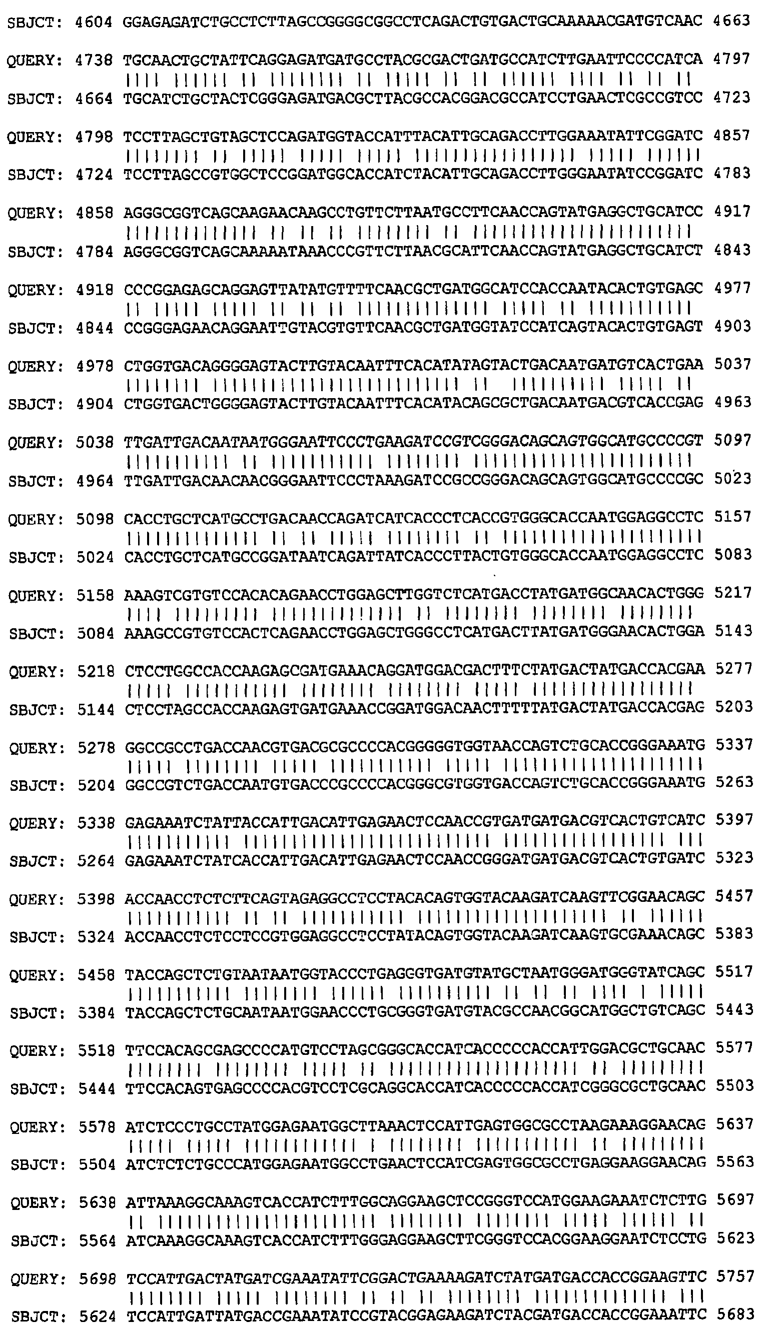

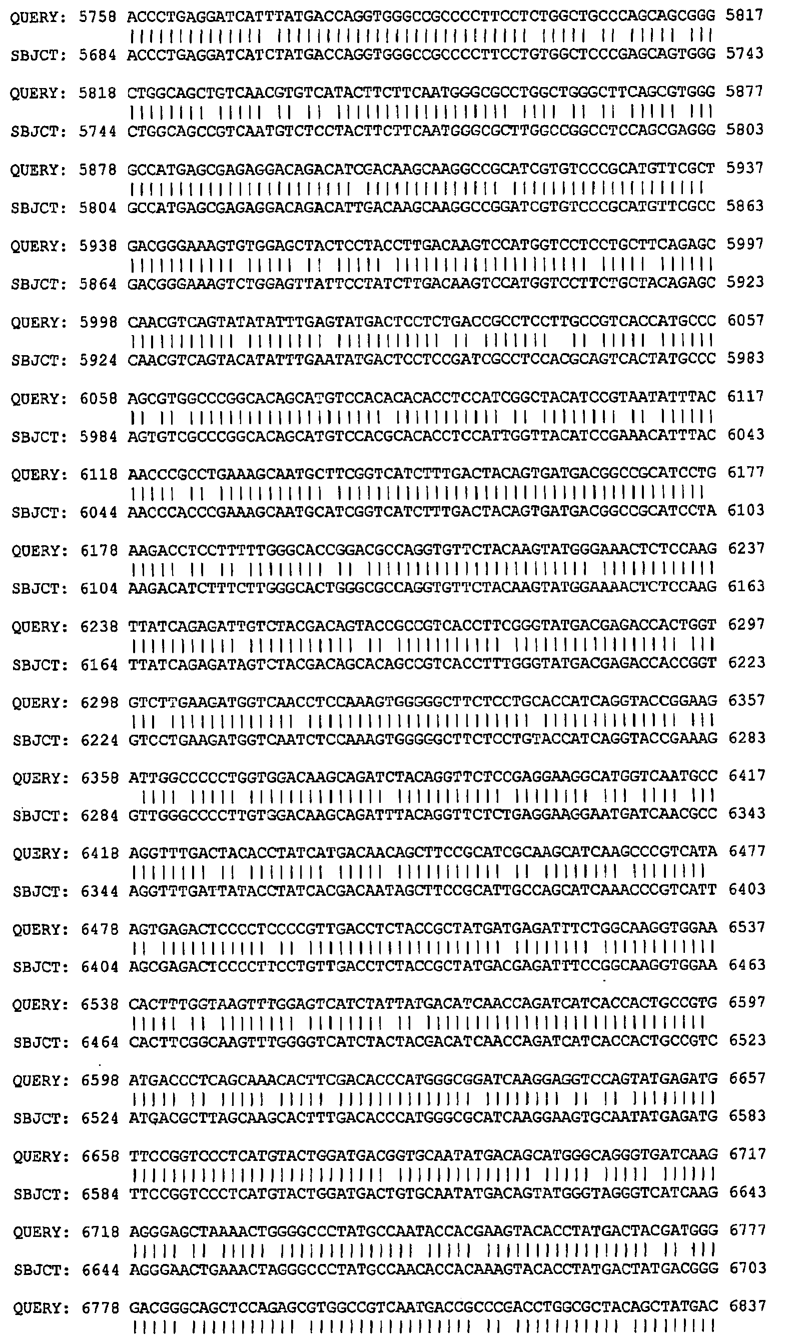

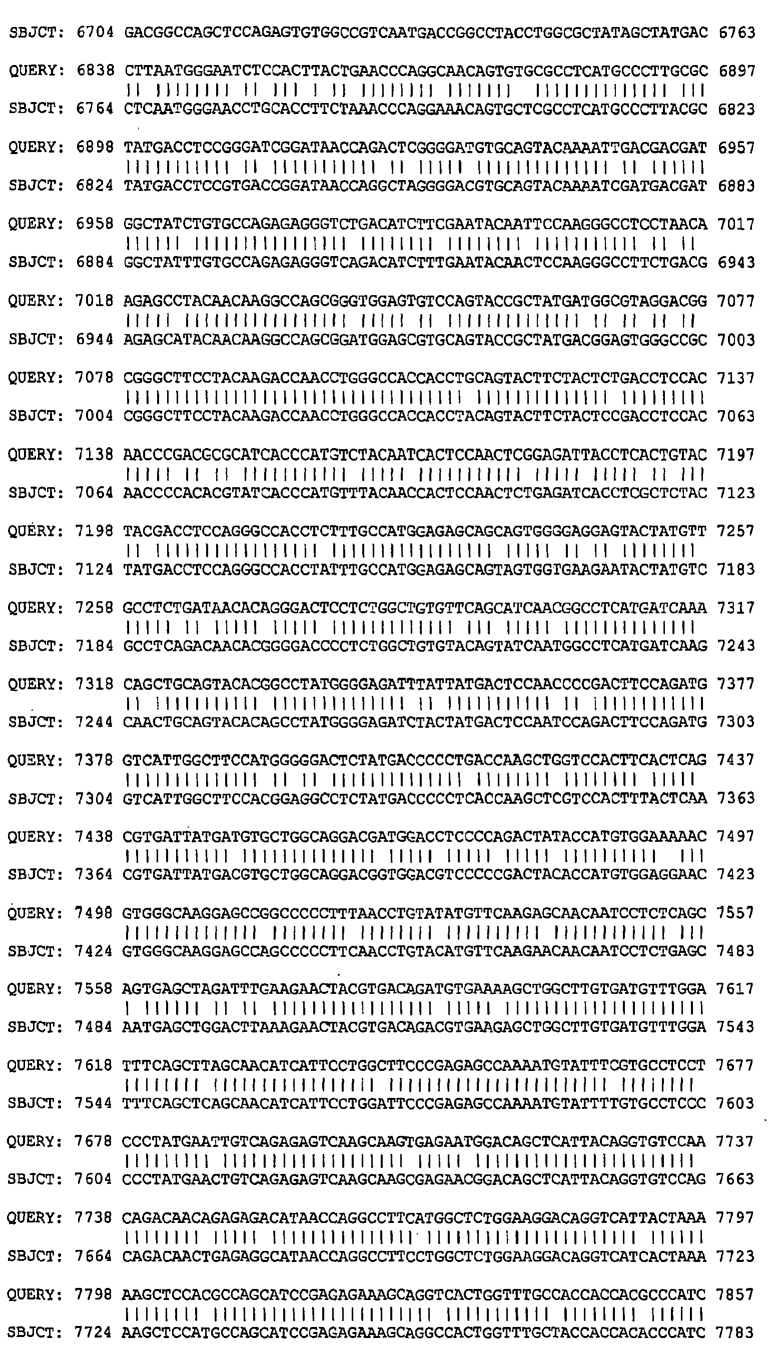

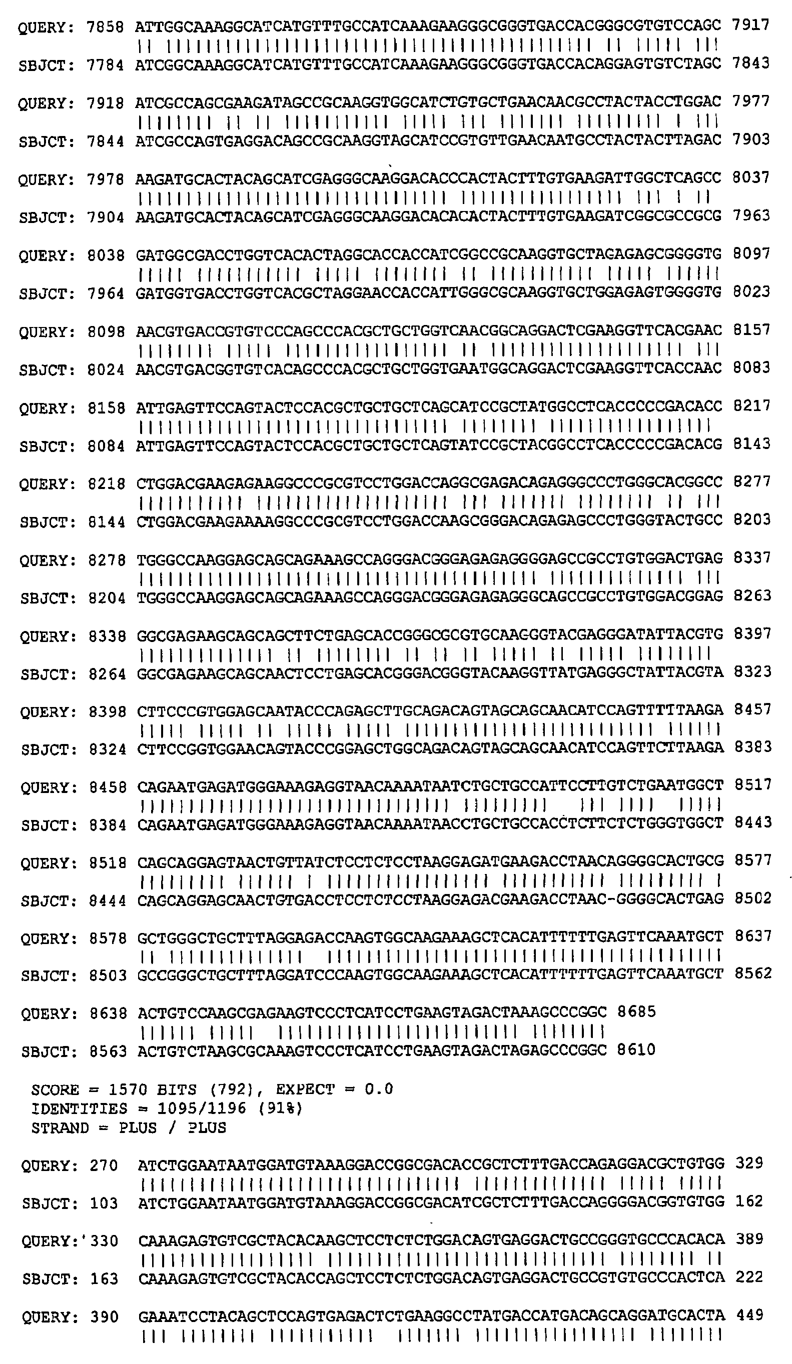

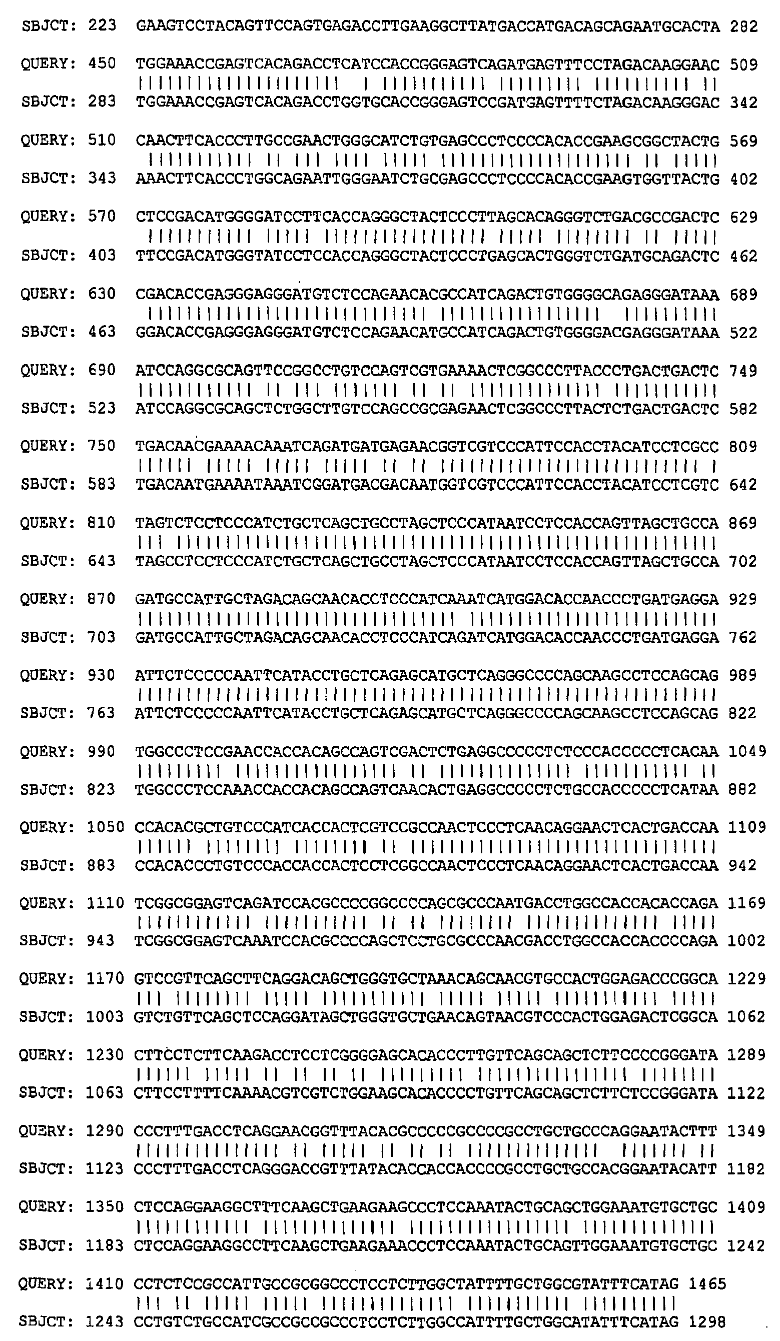

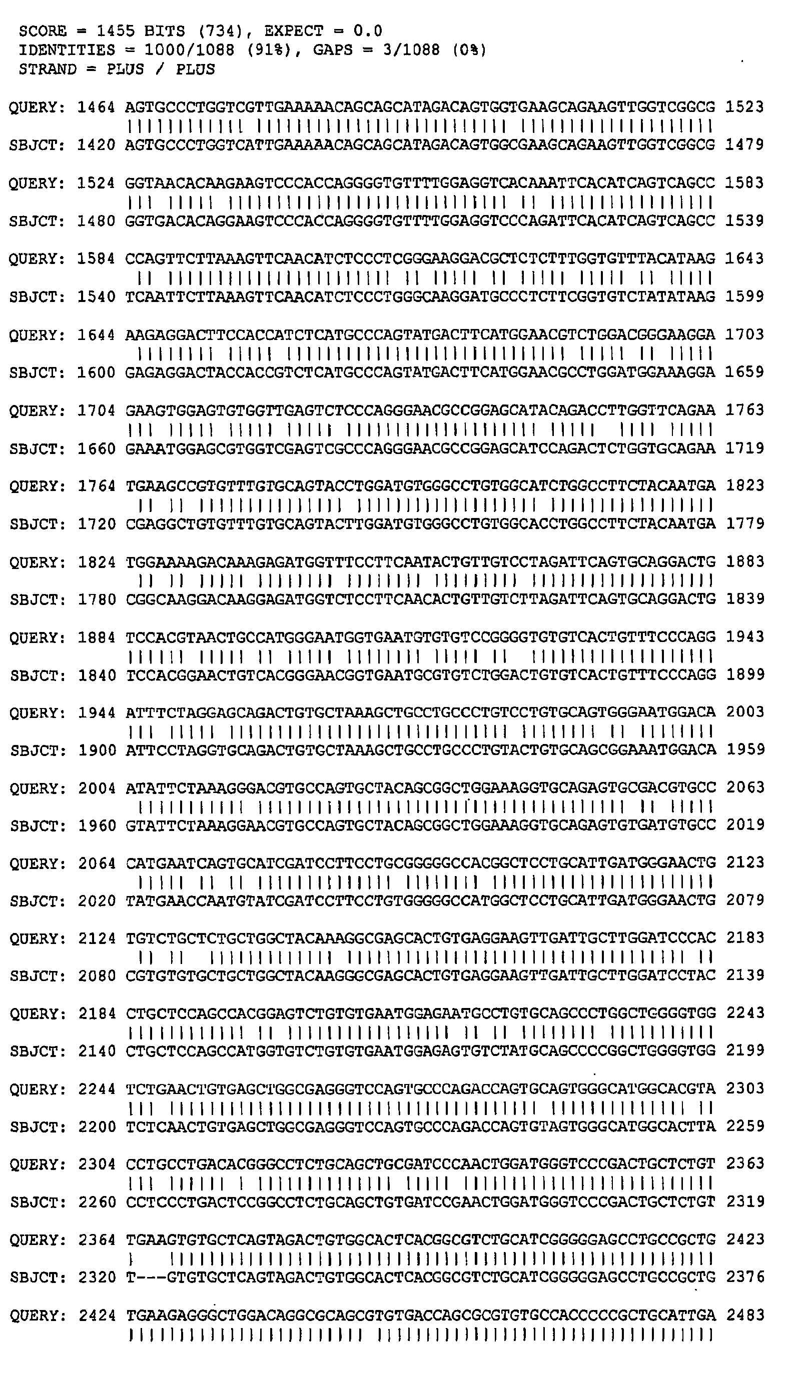

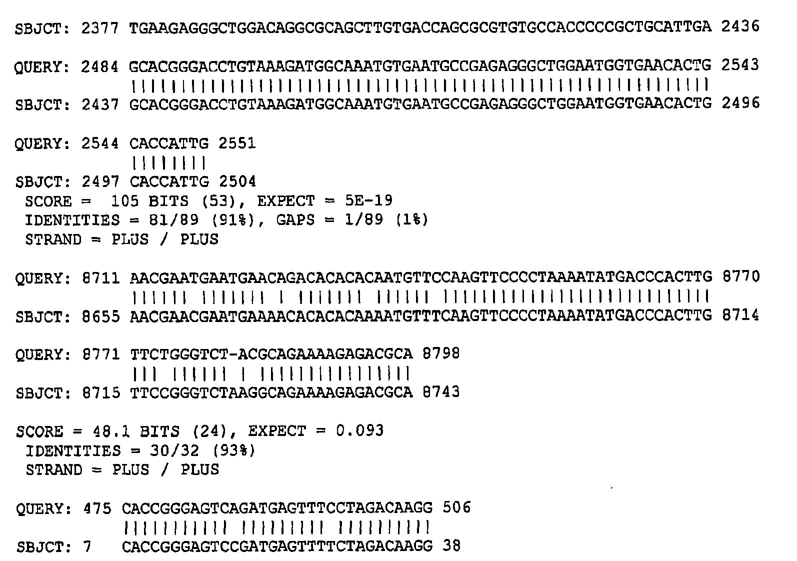

In this search it was also found that the FCTR3bcd and e nucleic acids had homology to three fragments of *Rattus norvegicus* neurestin alpha. It has 5498 of 6132 bases (89%) identical to bases 2527-8658,1081 of 1196 bases (90%) identical to bases 123-1318, 996 of 1088 bases (91%) identical to bases 1440-2527 of *Rattus norvegicus* neurestin alpha (GenBank Acc:NM_020088.1) (Table 3N).

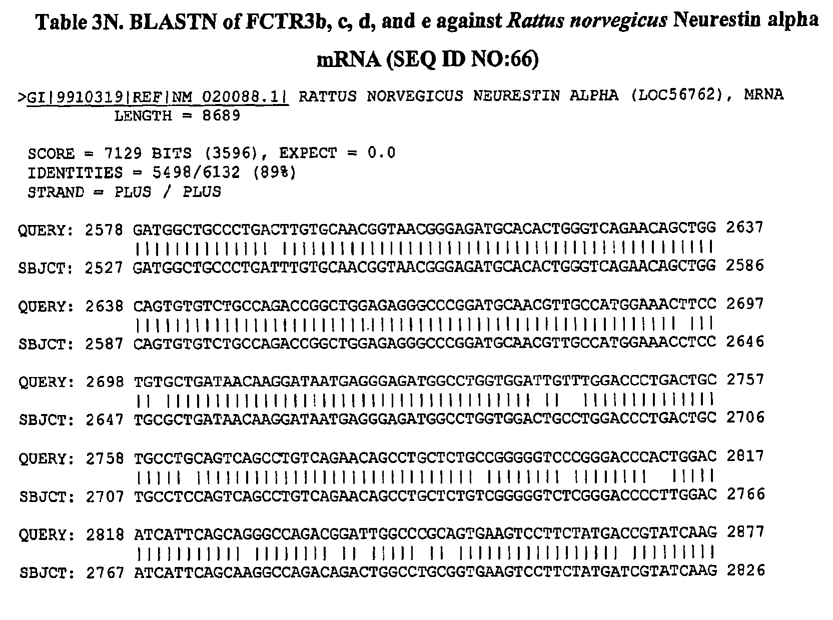

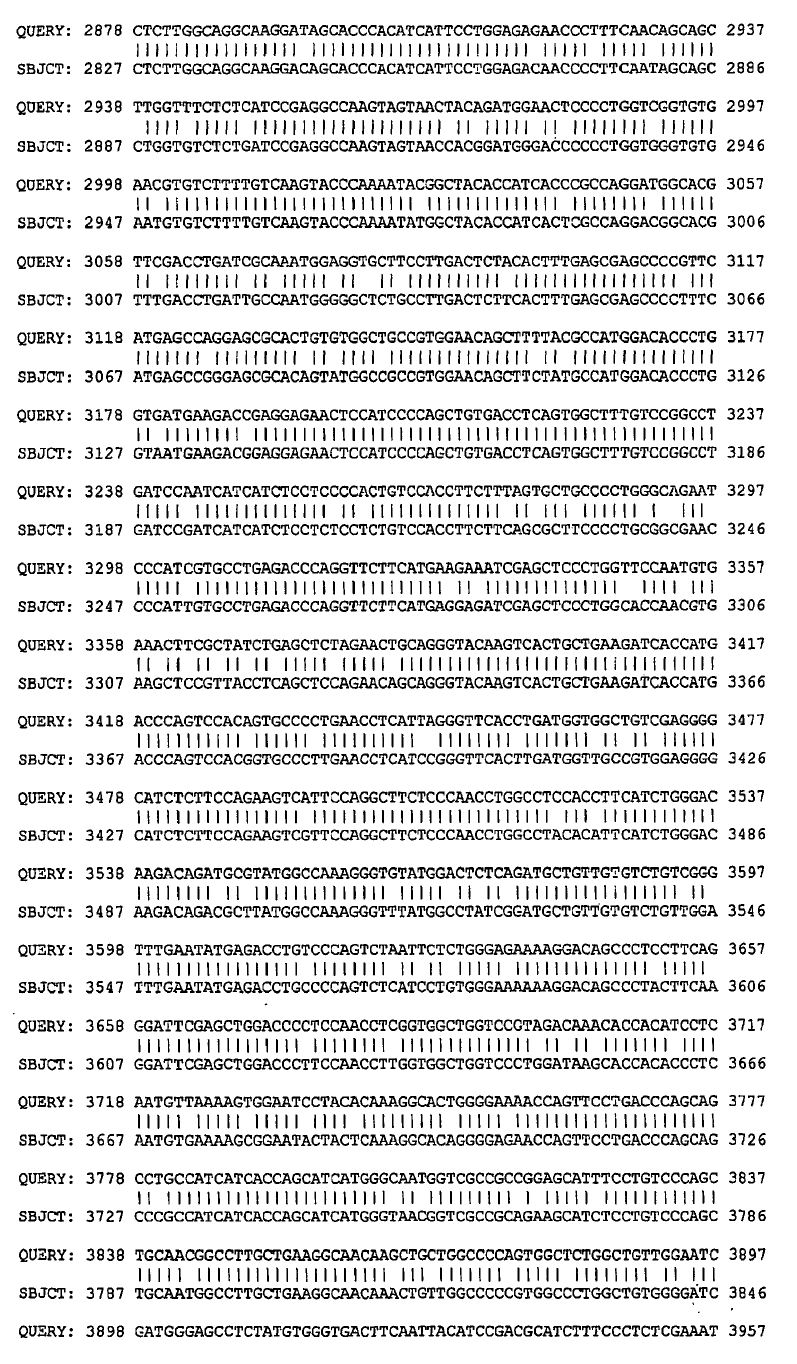

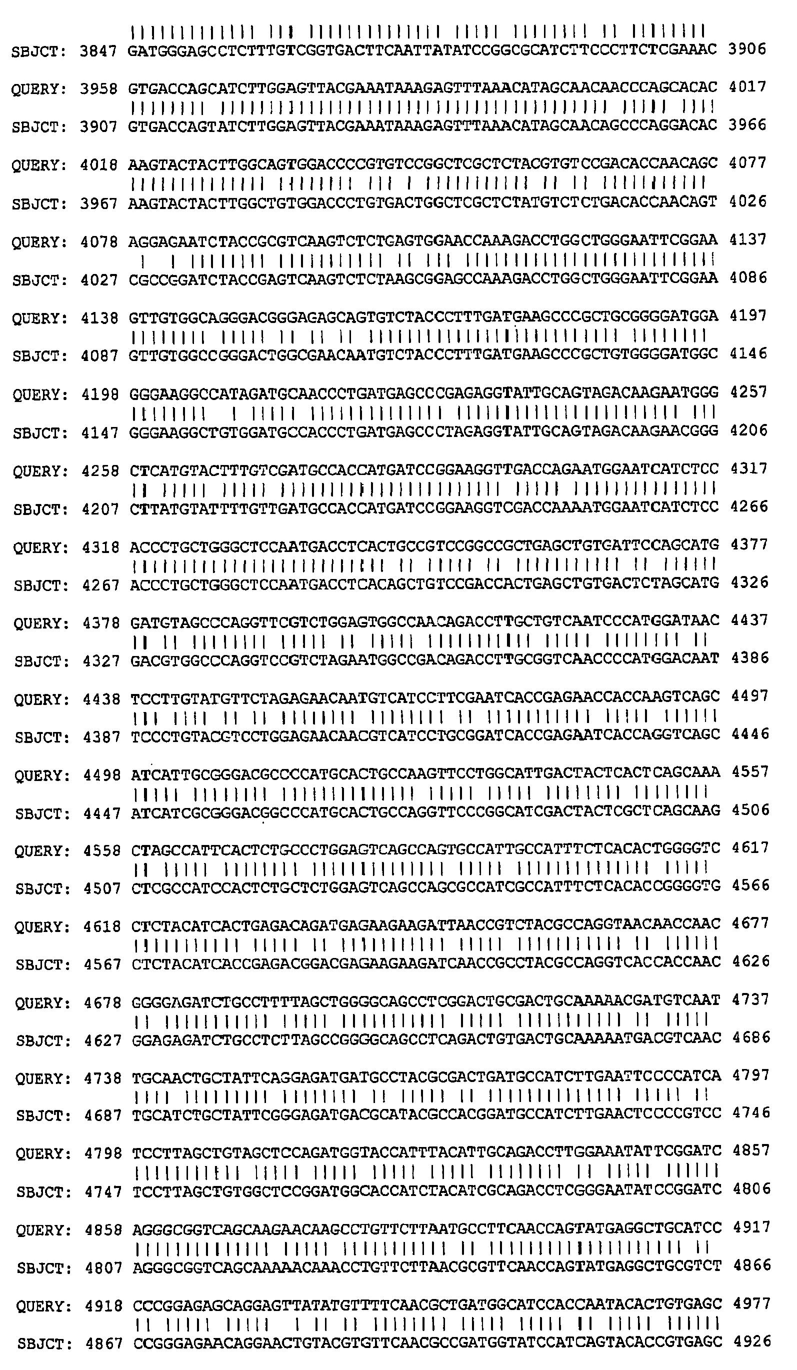

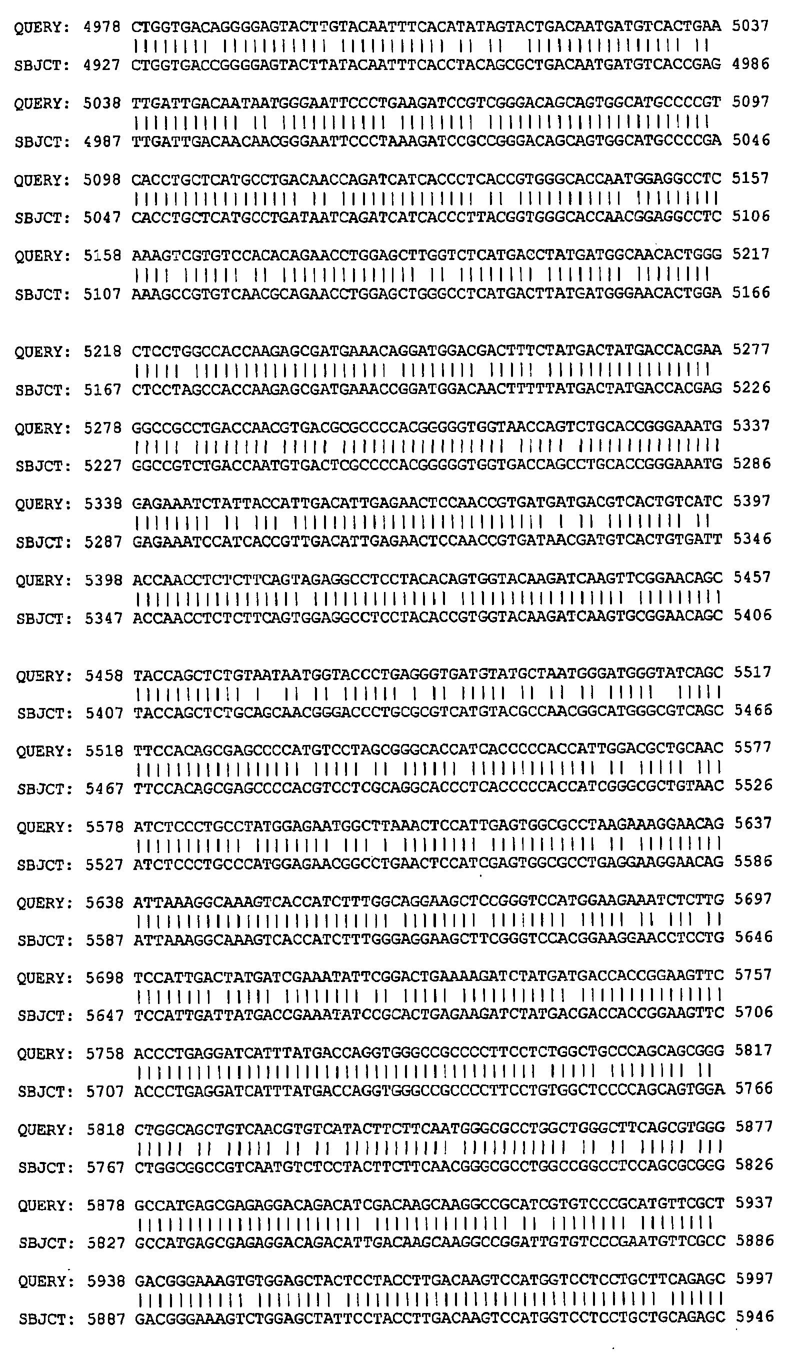

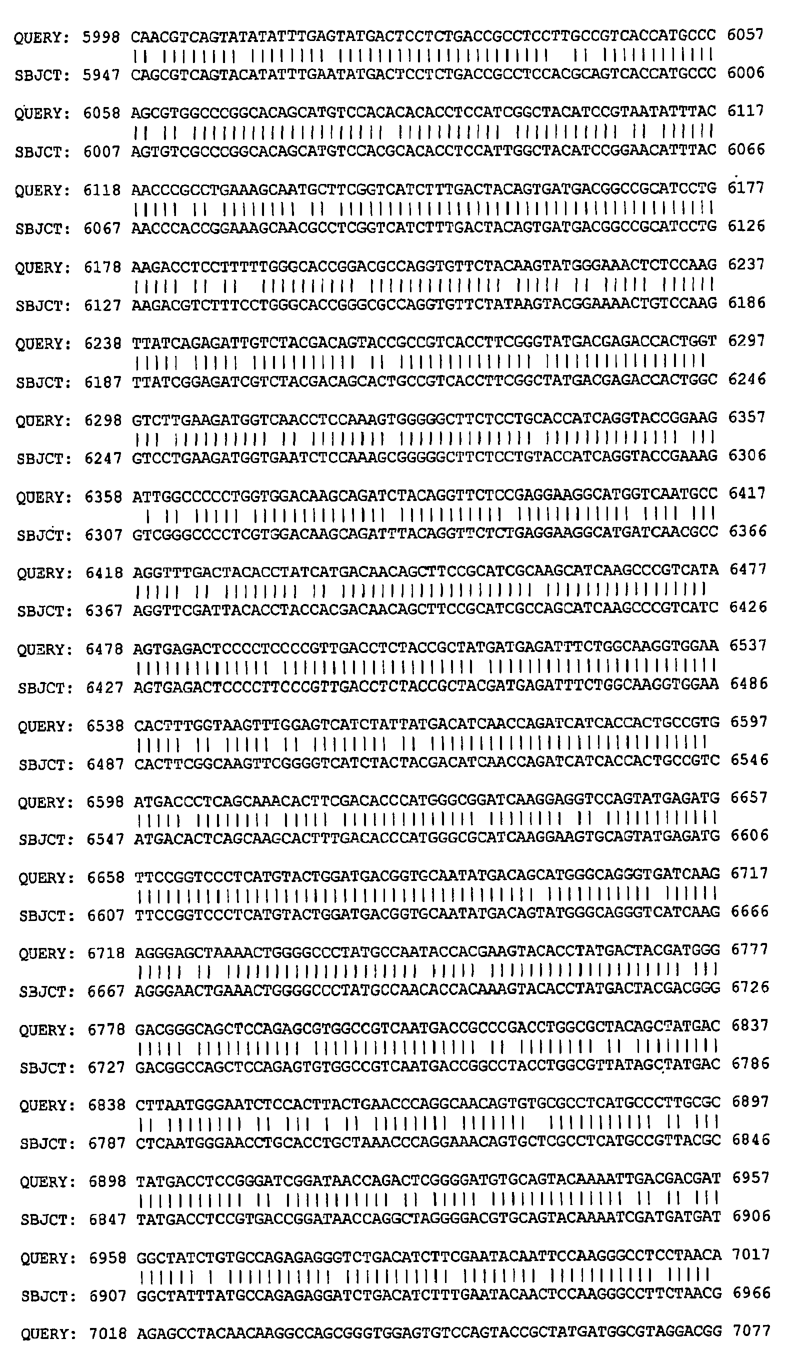

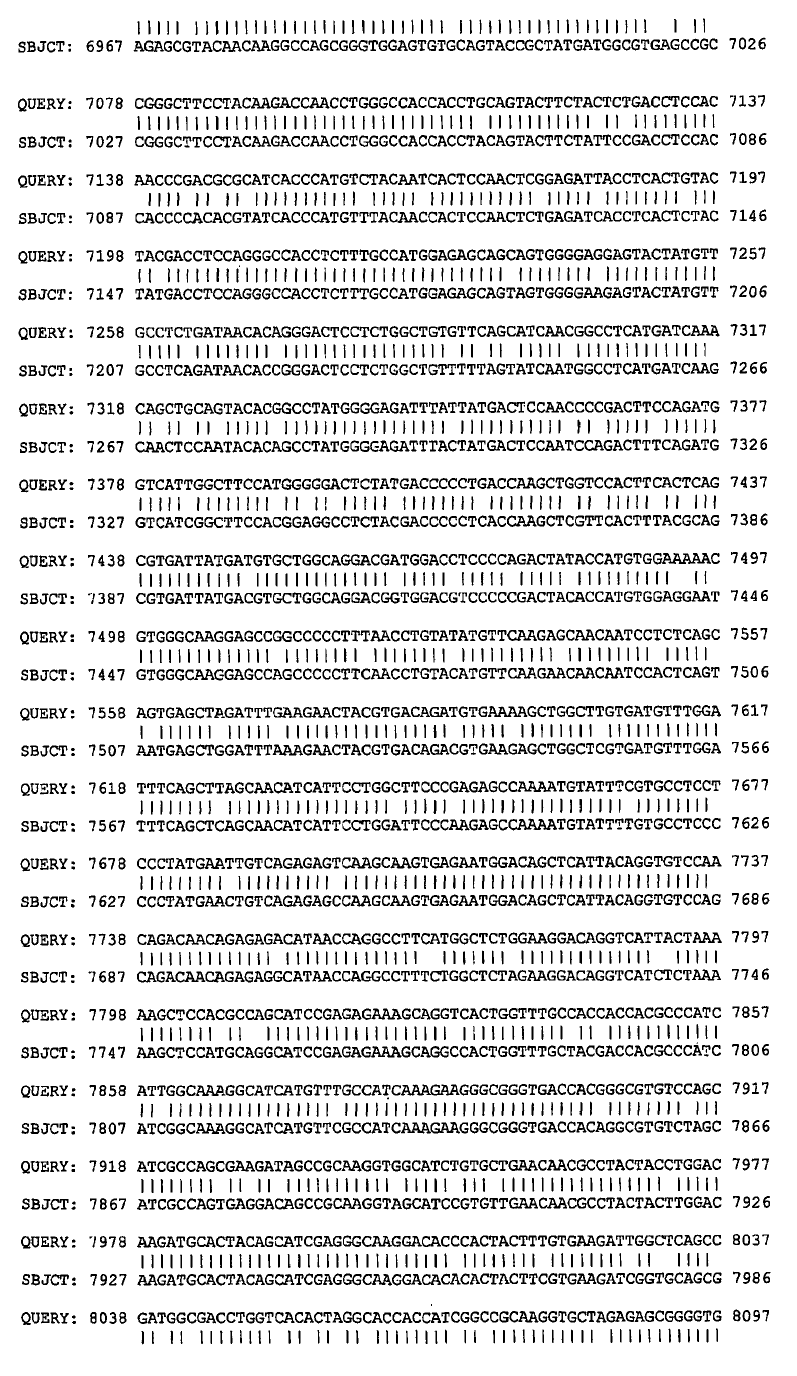

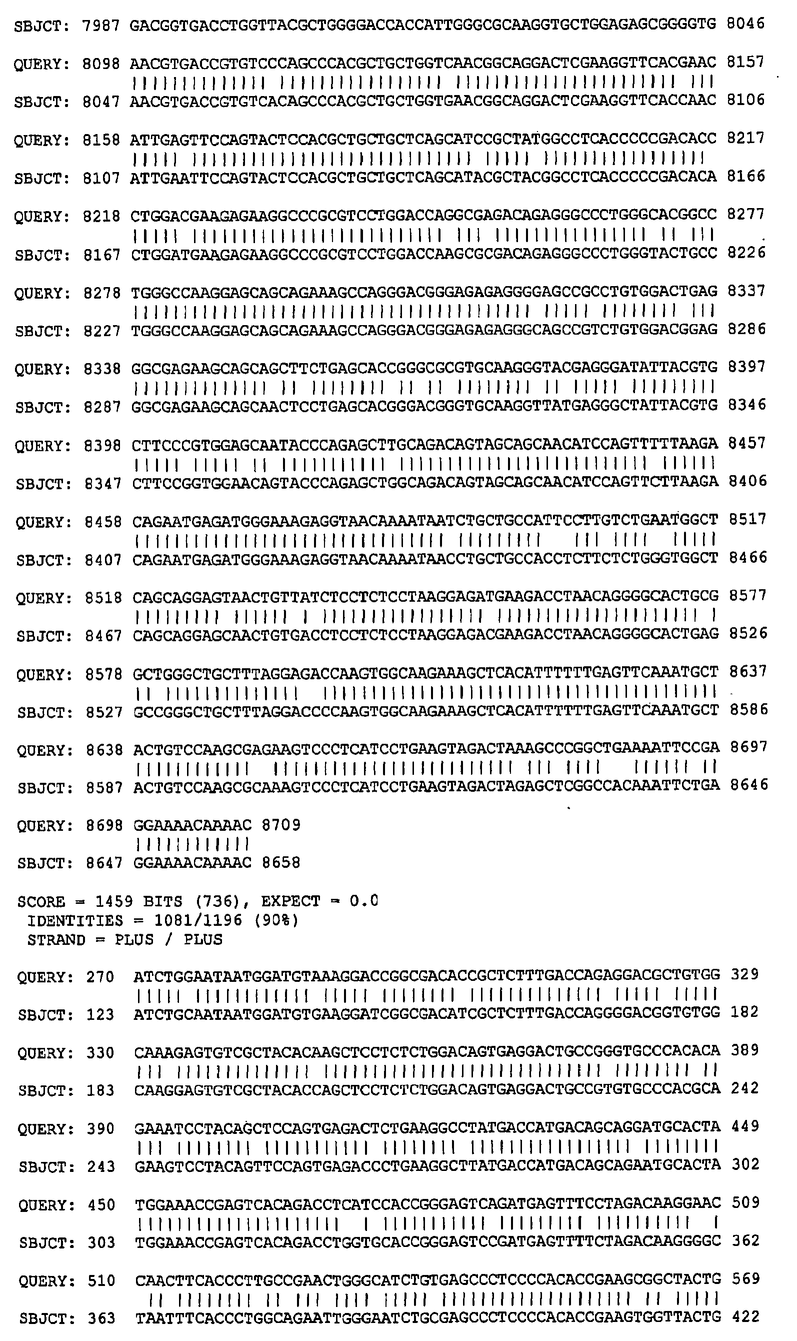

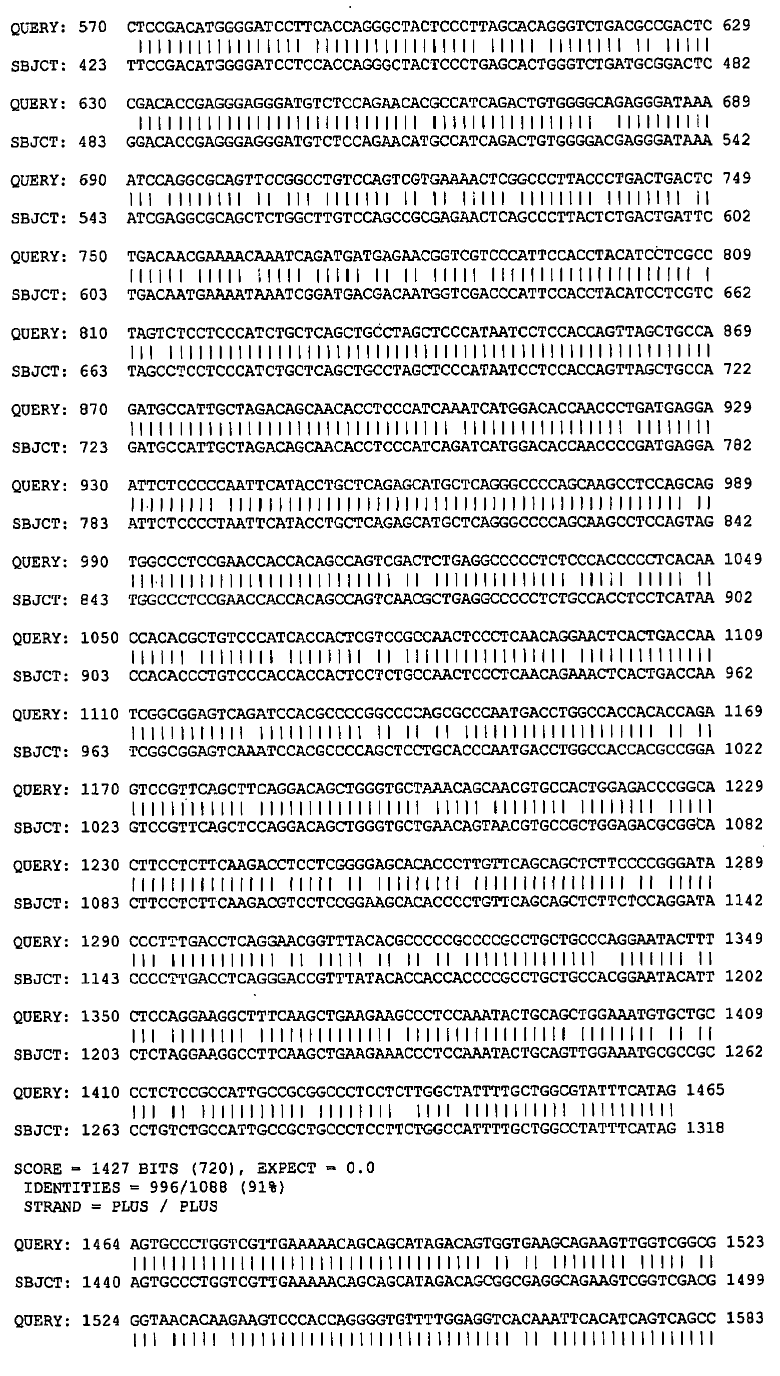

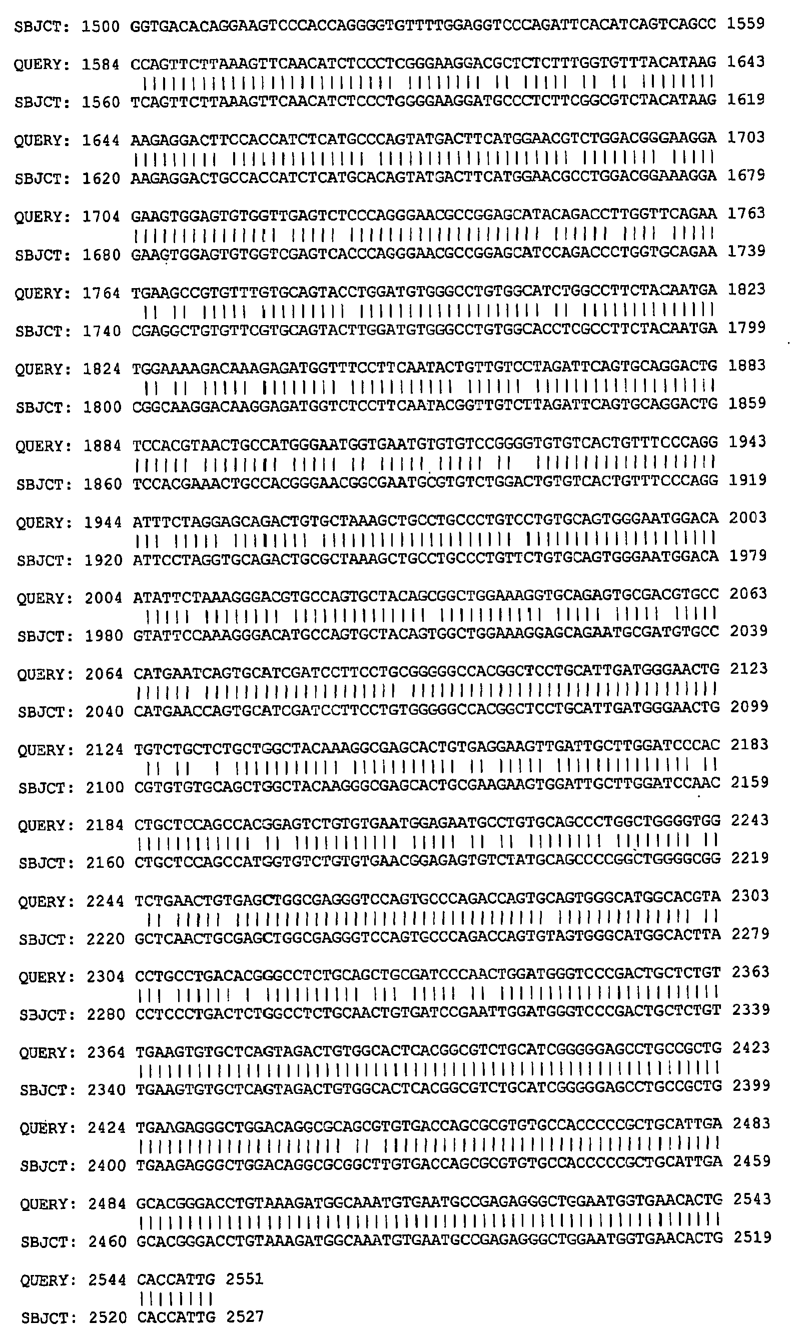

In this search it was also found that the FCTR3bcd and e nucleic acid had homology to six fragments of *Gallus gallus* partial mRNA for teneurin-2. It has 2780 of 3449 bases (80%) identical to bases 3386-6834, 1553 of 1862 bases (83%) identical to bases 1414-3275, 540 of 628 bases (85%) identical to bases 587-1214, 593 of 725 bases (81%) identical to bases 7084-7808, 429 of 515 bases (83%) identical to bases 7895-8409, and 397 of 475 bases (83%) identical to bases 20-494 of *Gallus gallus* partial mRNA for teneurin-2. (EMBL Acc: GGA278031) (Table 3O).

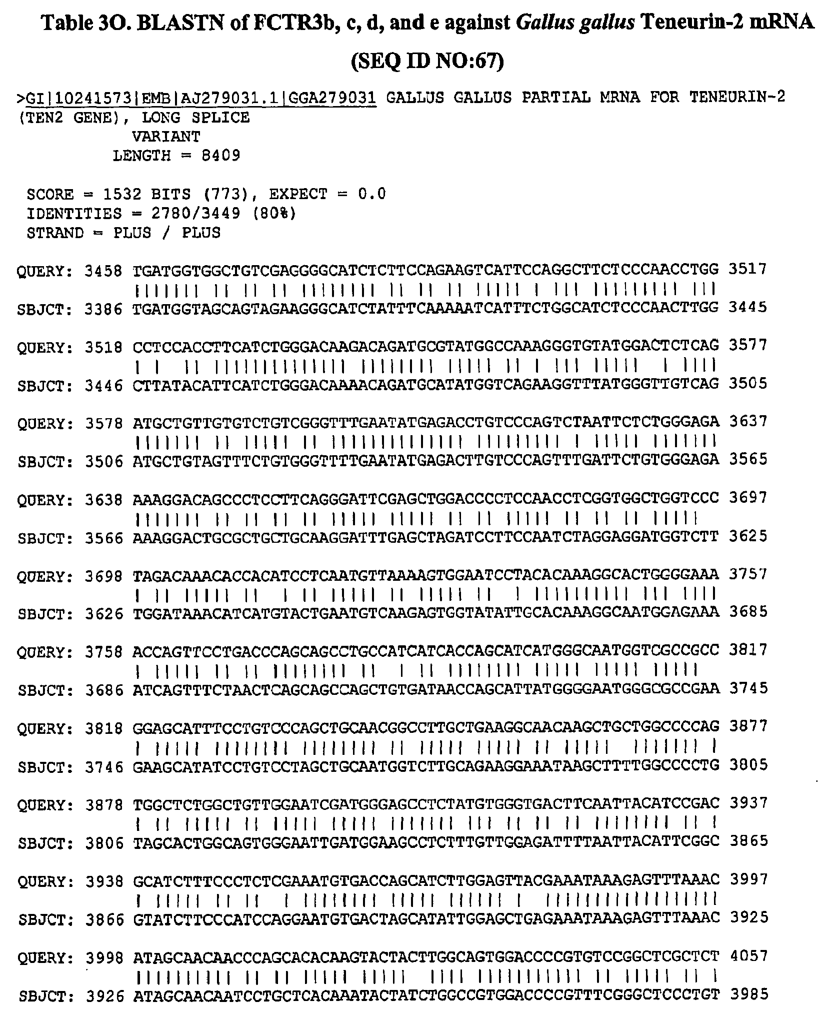

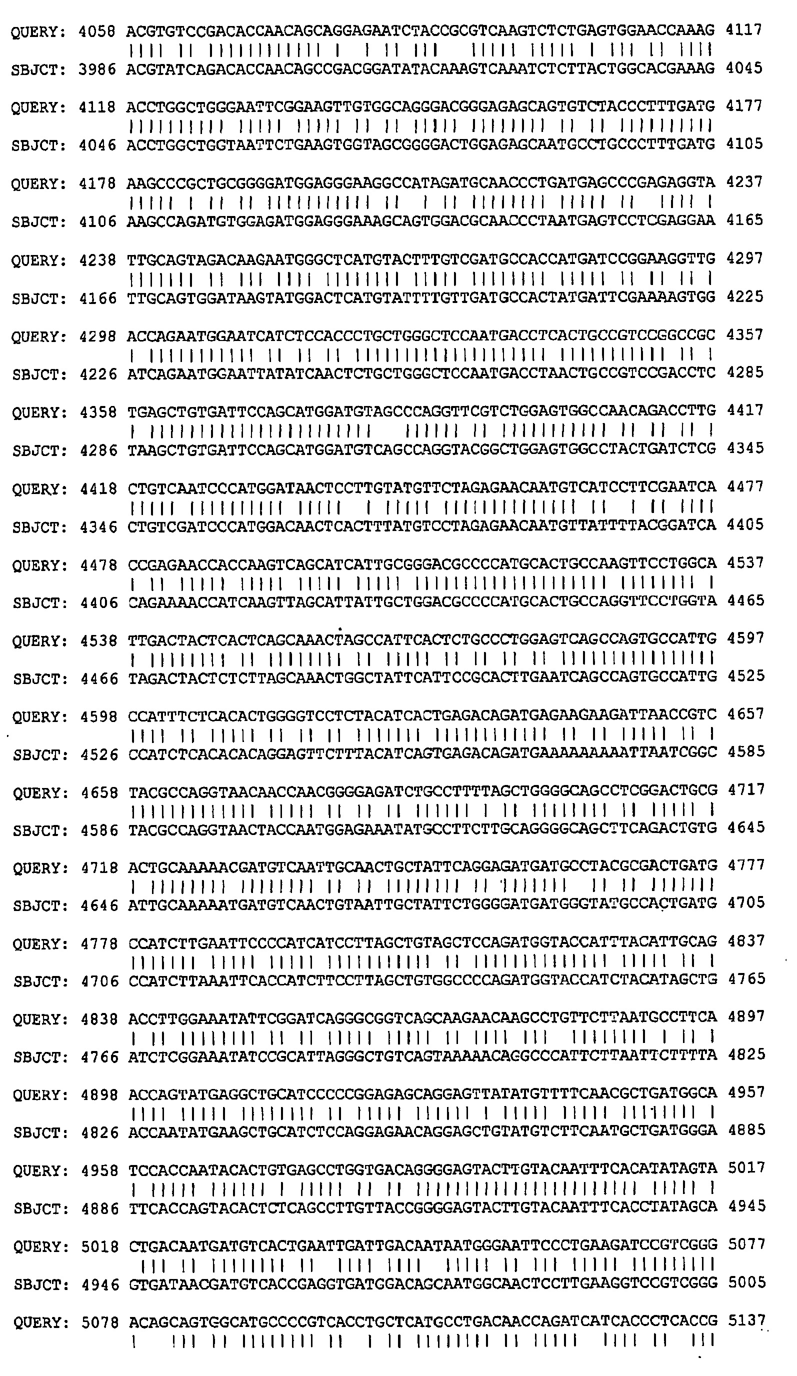

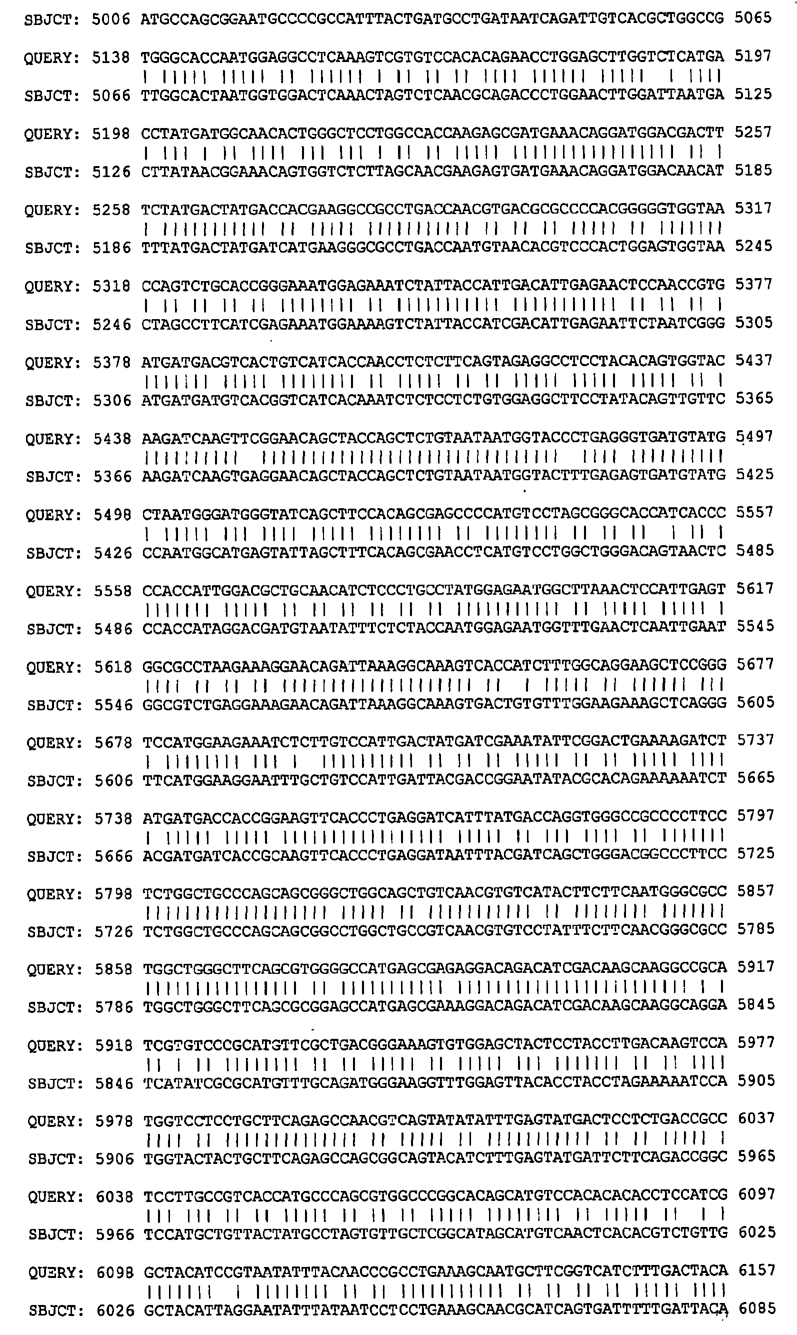

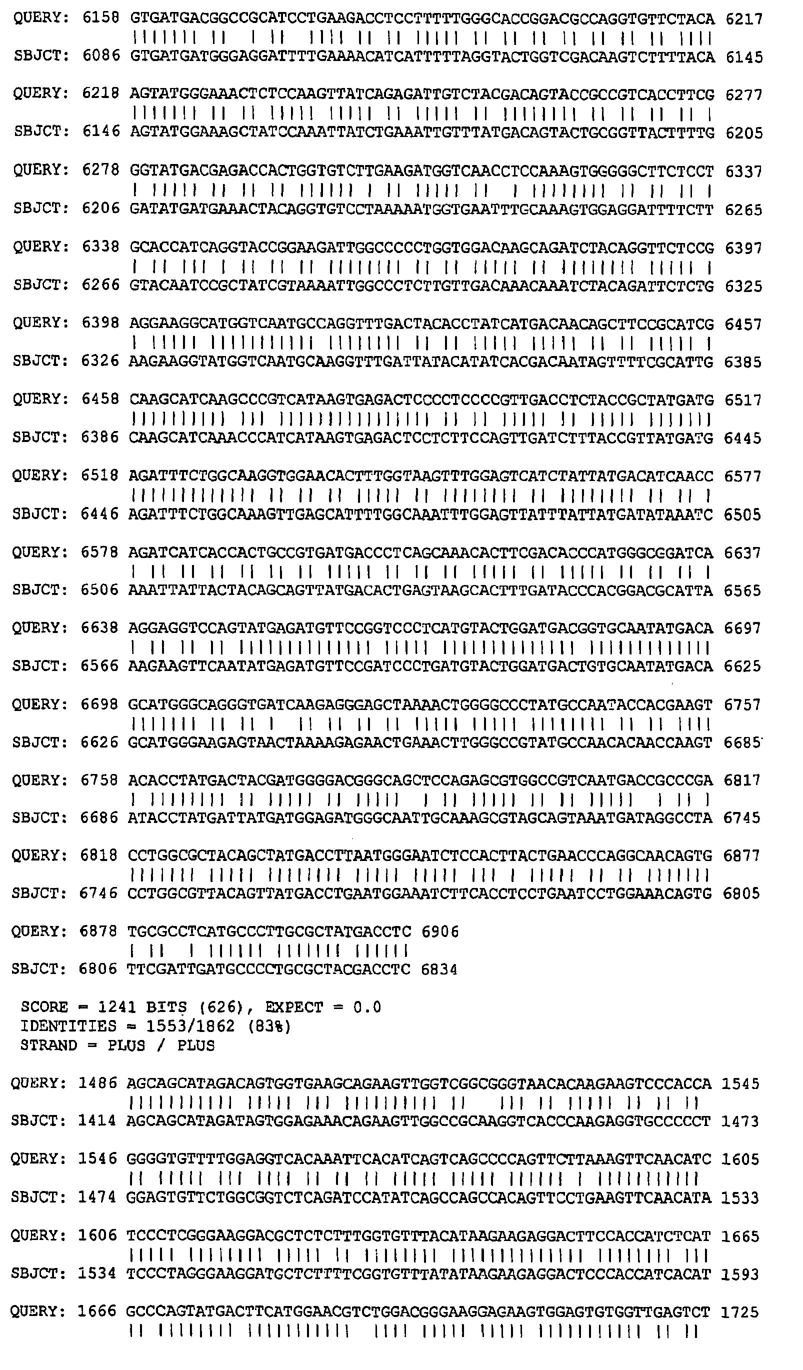

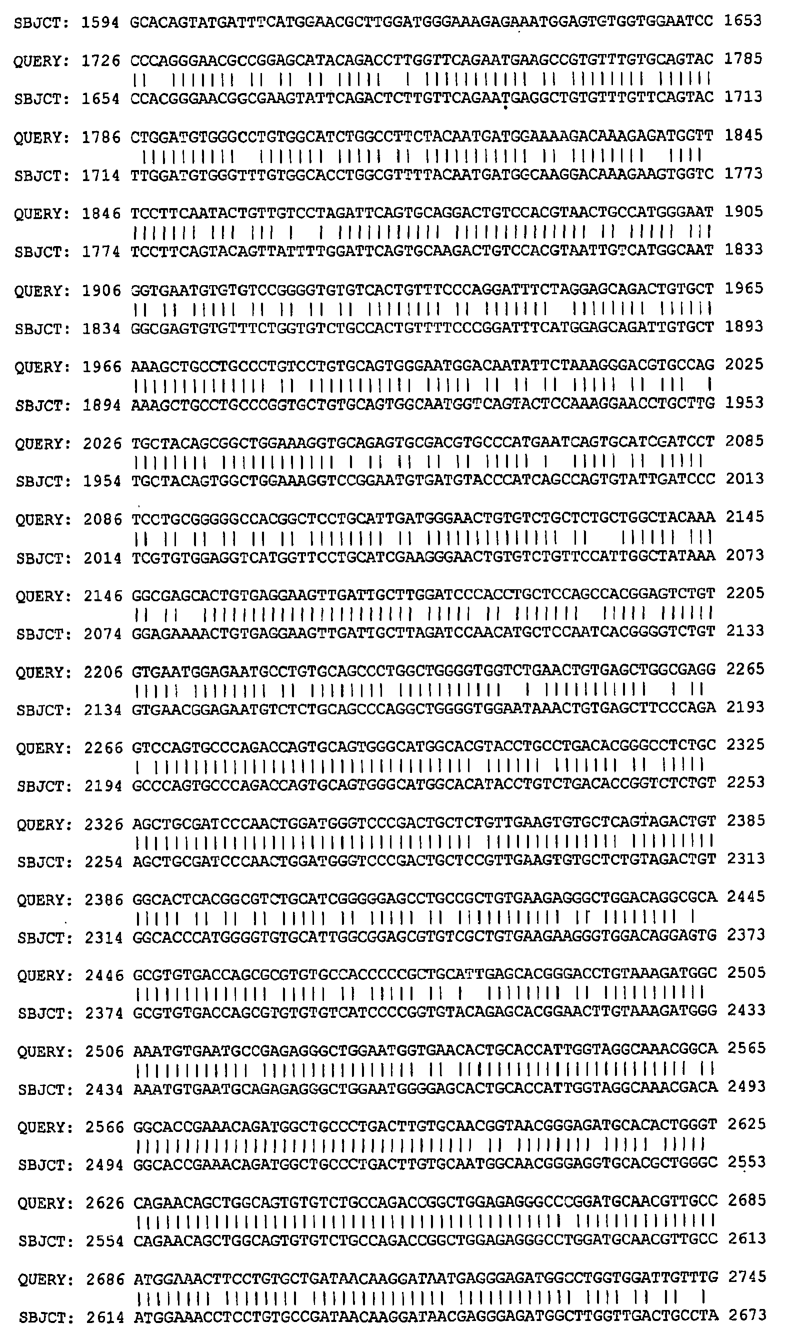

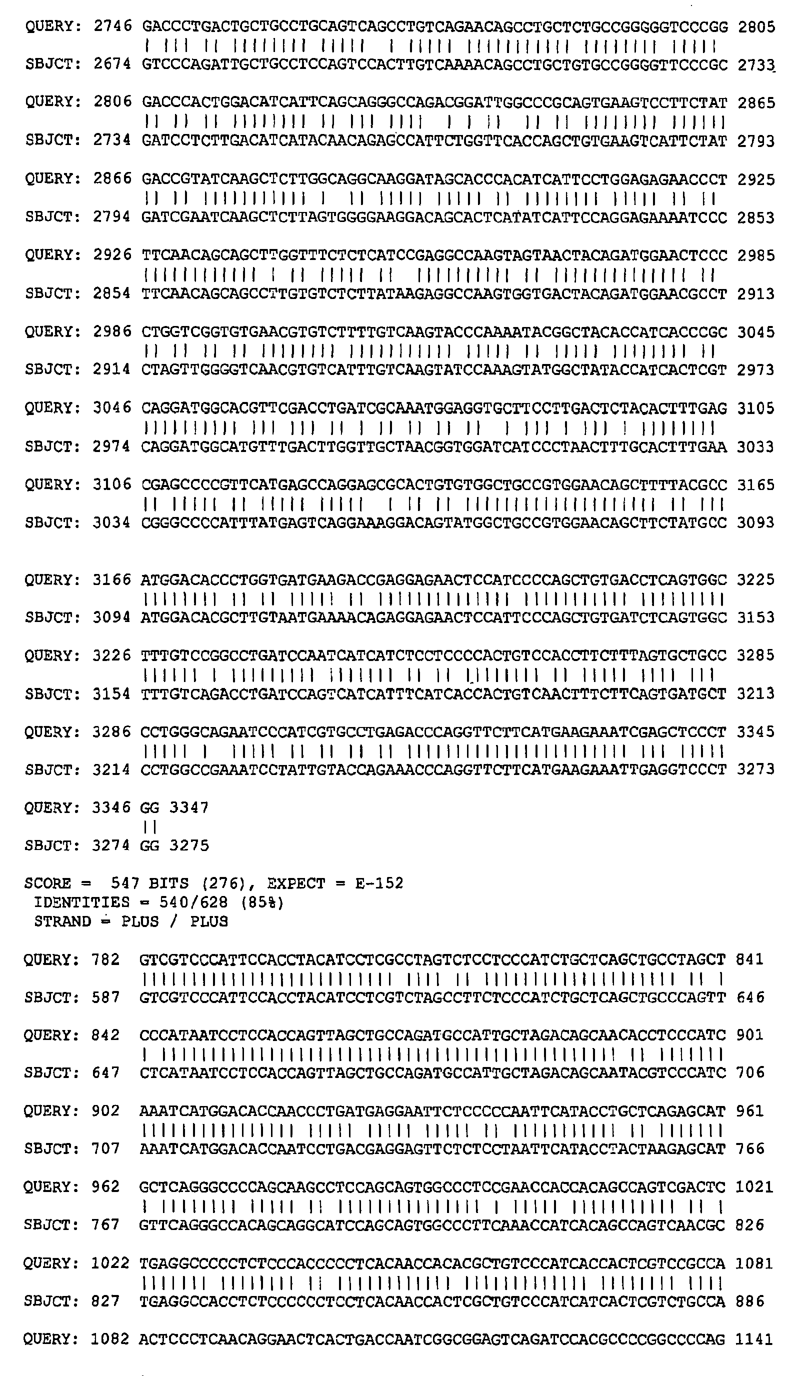

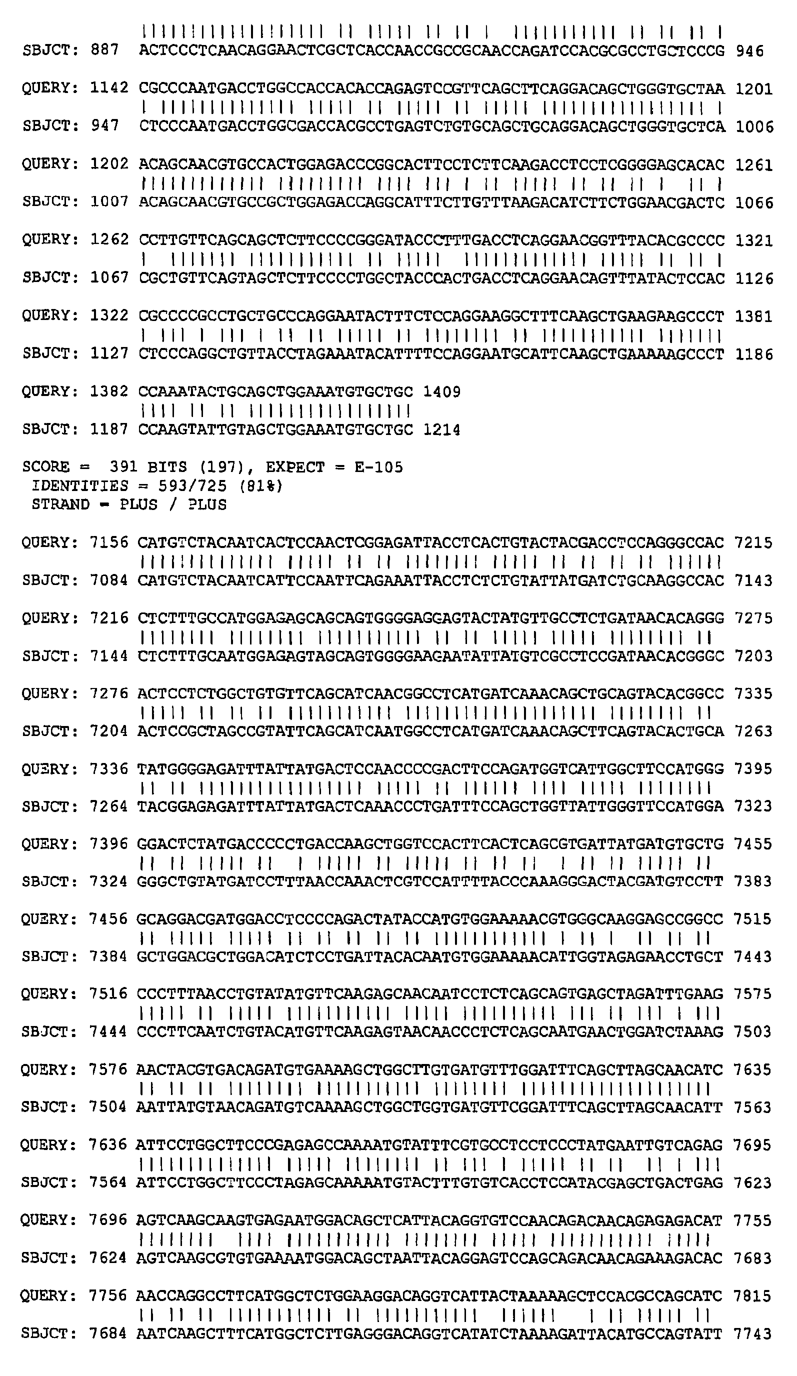

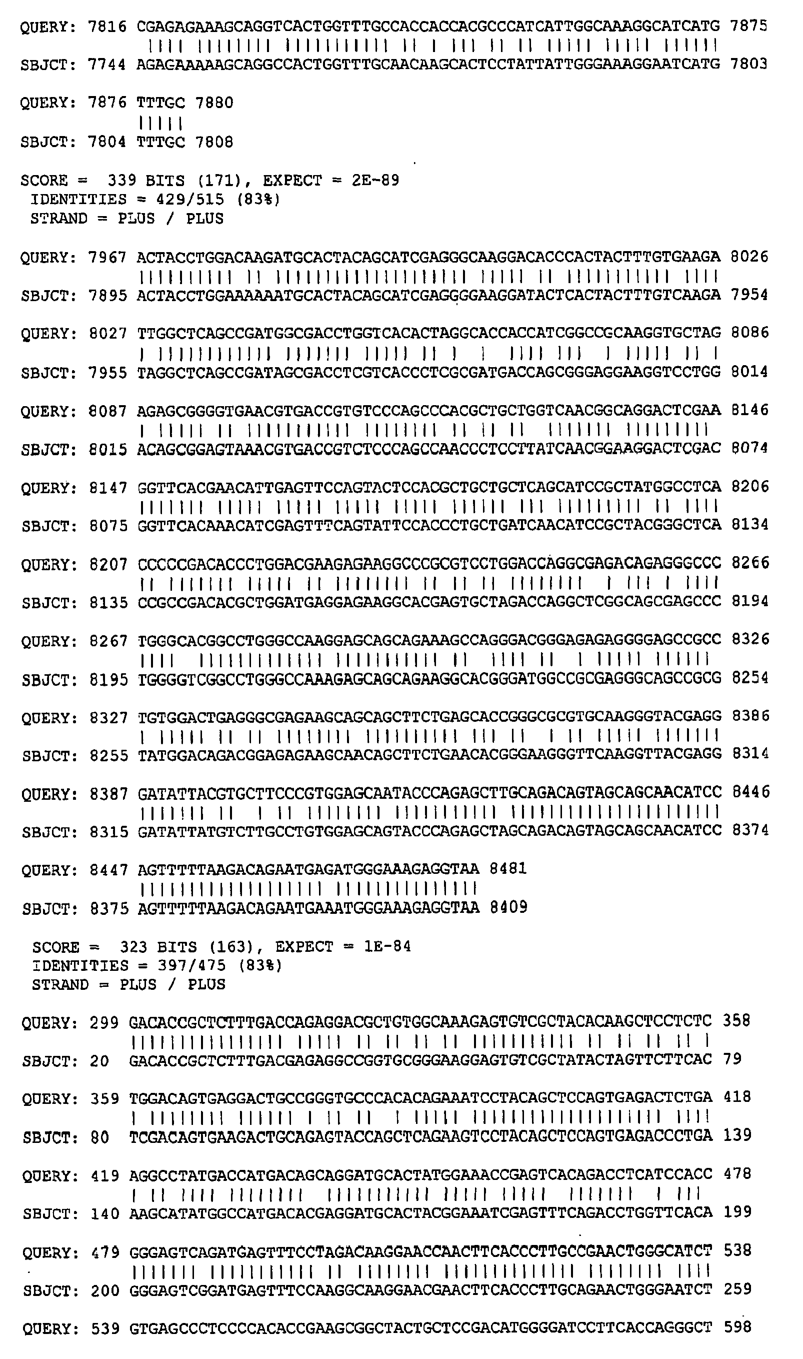

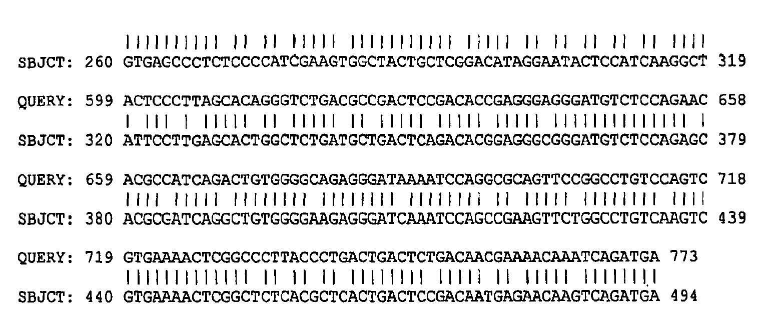

The full FCTR3a amino acid sequence also has 342 of 383 amino acid residues (89%) identical to, and 342 of 383 residues (89%) positive with, the 276 amino acid residue Odd Oz/ten-m homolog 2 (*Drosophila*) (GenBank Acc: NP_035986.2) (SEQ ID NO:68) (Table 3P).

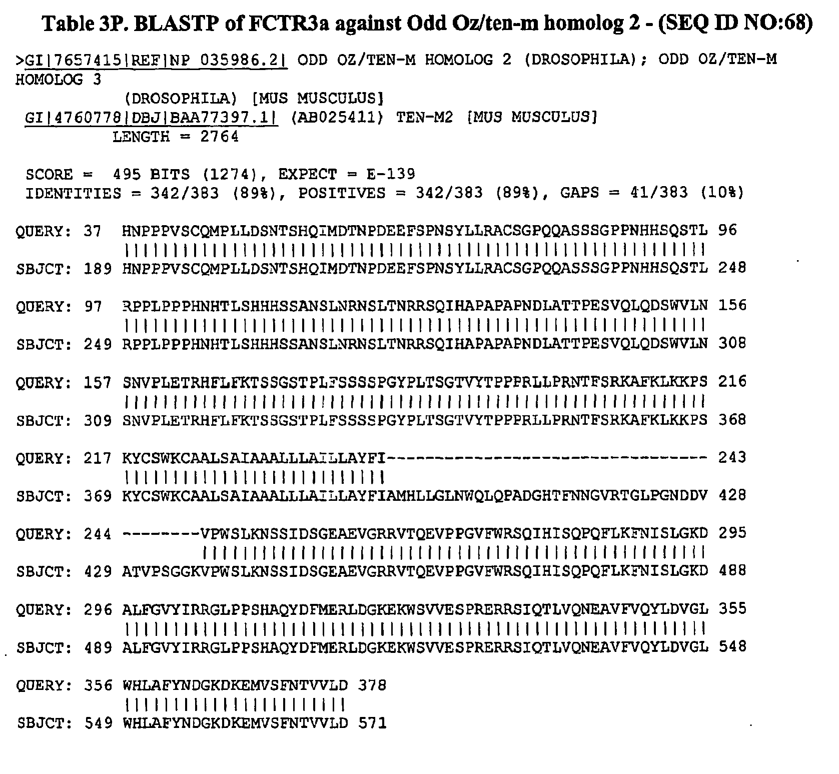

The full FCTR3b amino acid sequence has 2442 of 2802 amino acid residues (87%) identical to, and 2532 of 2802 residues (90%) positive with, the 2802 amino acid residue teneurin-2 [*Gallus gallus*] (GenBank Acc: AJ279031) (SEQ ID NO:69) (Table 3Q).

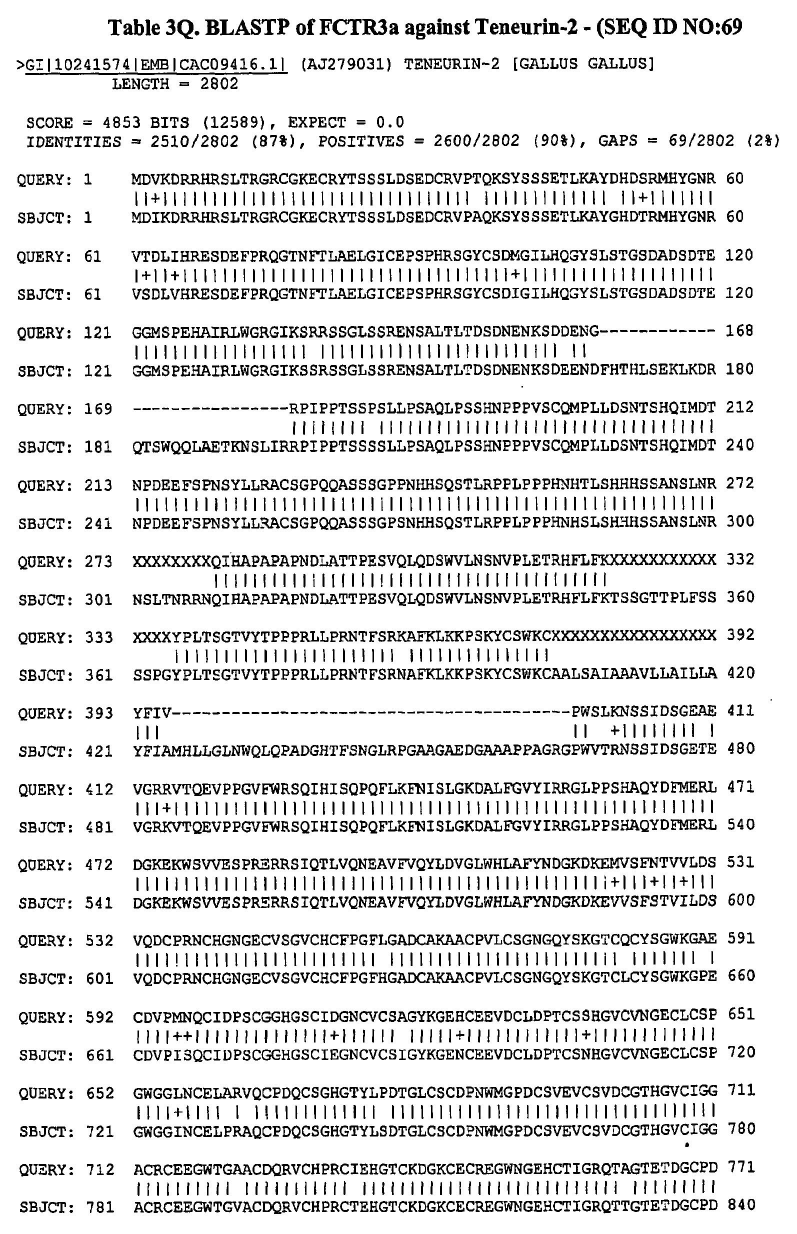

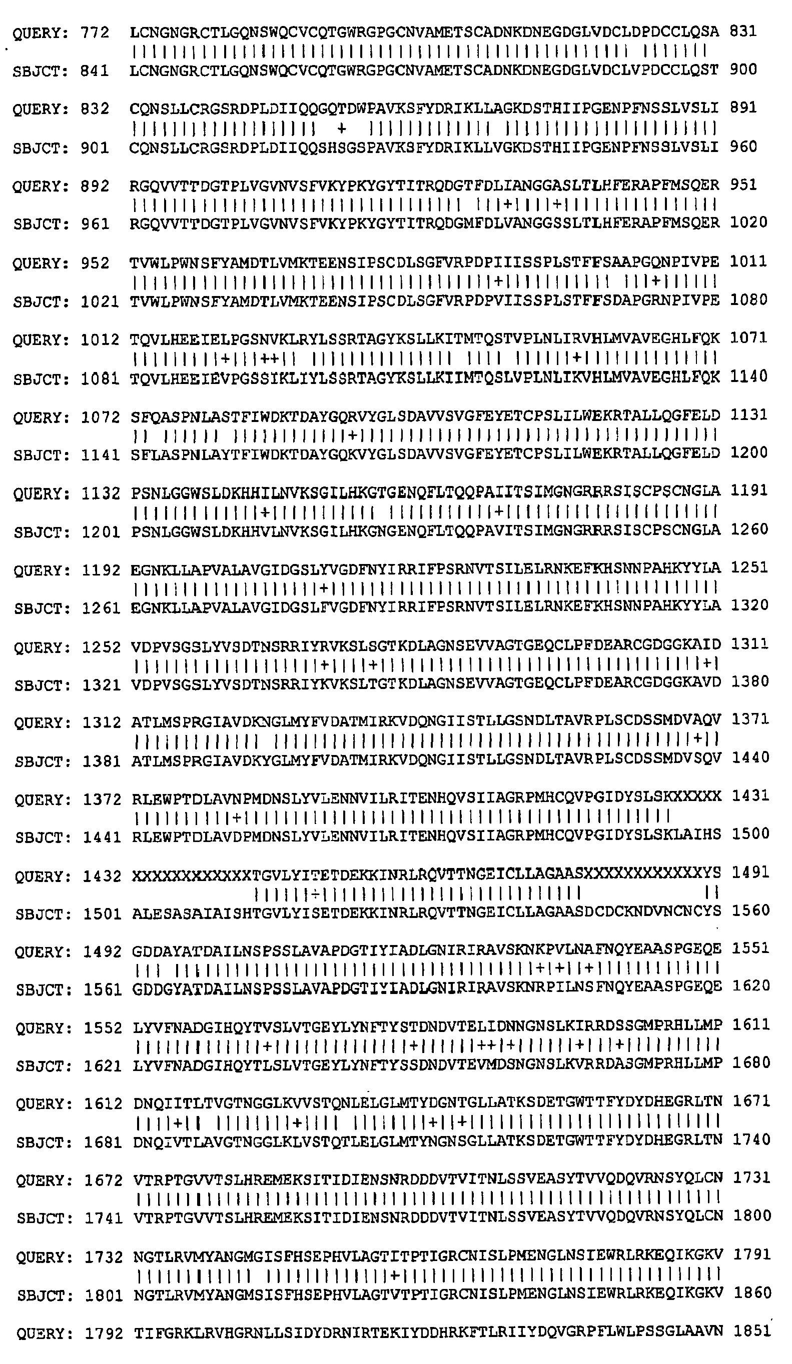

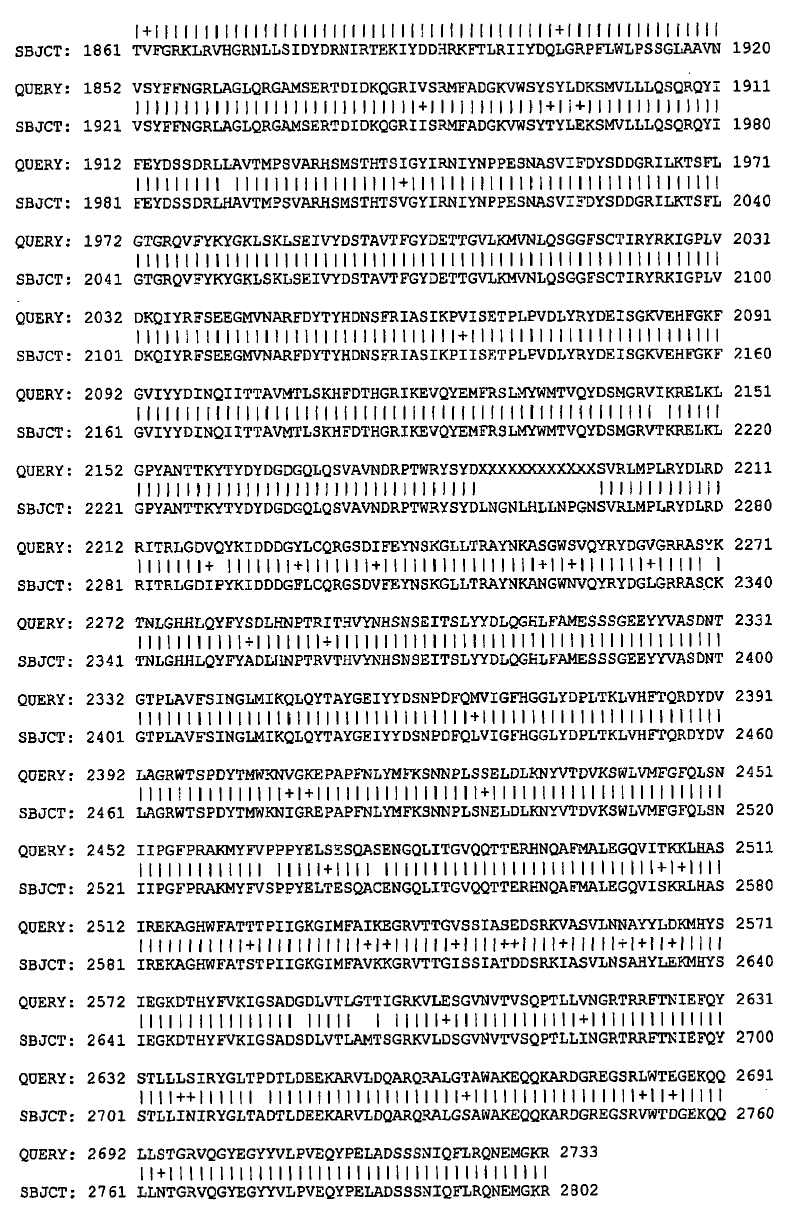

The FCTR3bcde and f amino acid sequences have 1524 of 2352 amino acid residues (64%) identical to, and 1881 of 2532 residues (79%) positive with, the amino acid residues 429-2771, 93 of 157 residues (59%) identical to and 118 of 157 residues (74%) positive with amino acid residues 1-155, and 59 of 152 residues (38%) identical to and 68 of 152 residues (43%) positive with amino acid residues 211-361 of Ten-m4 [*Mus musculus*] (ptnr: GenBank Acc: BAA77399.1) (SEQ ID NO:70) (Table 3R).

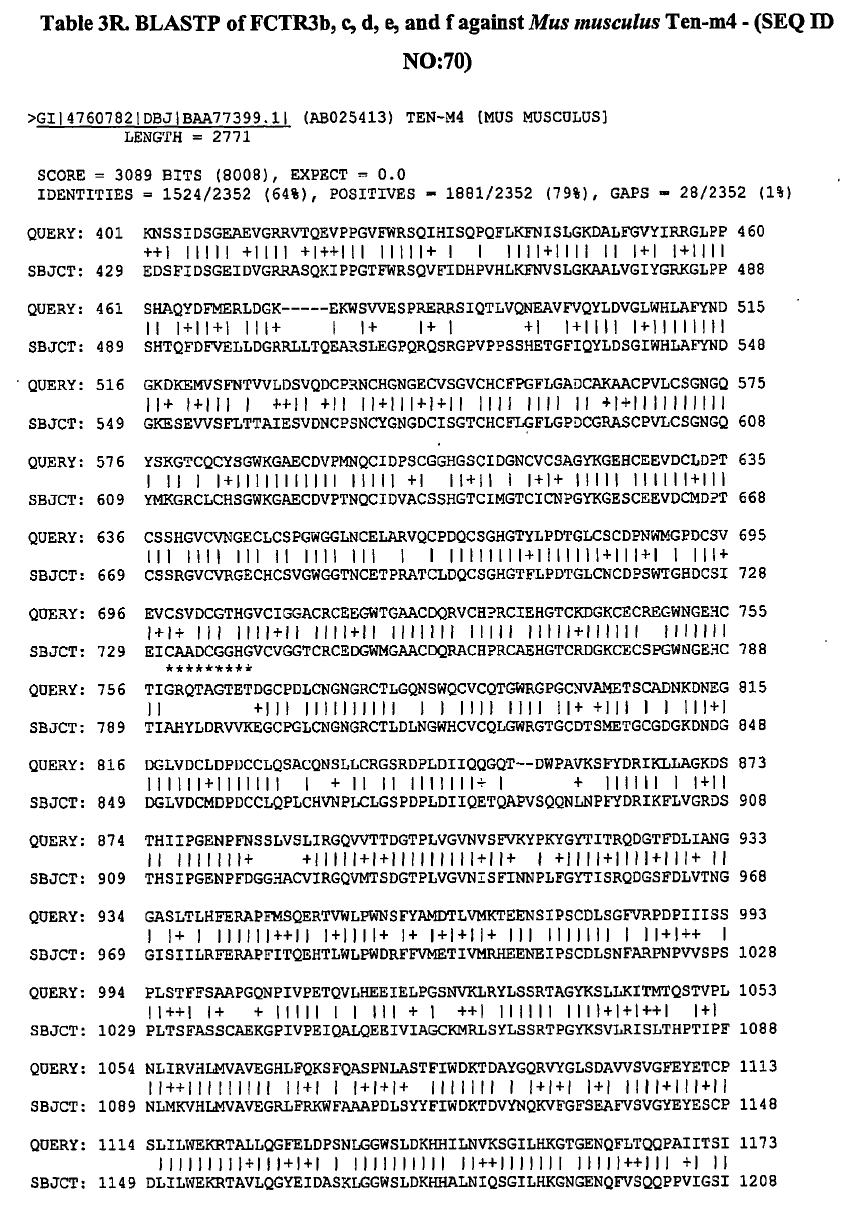

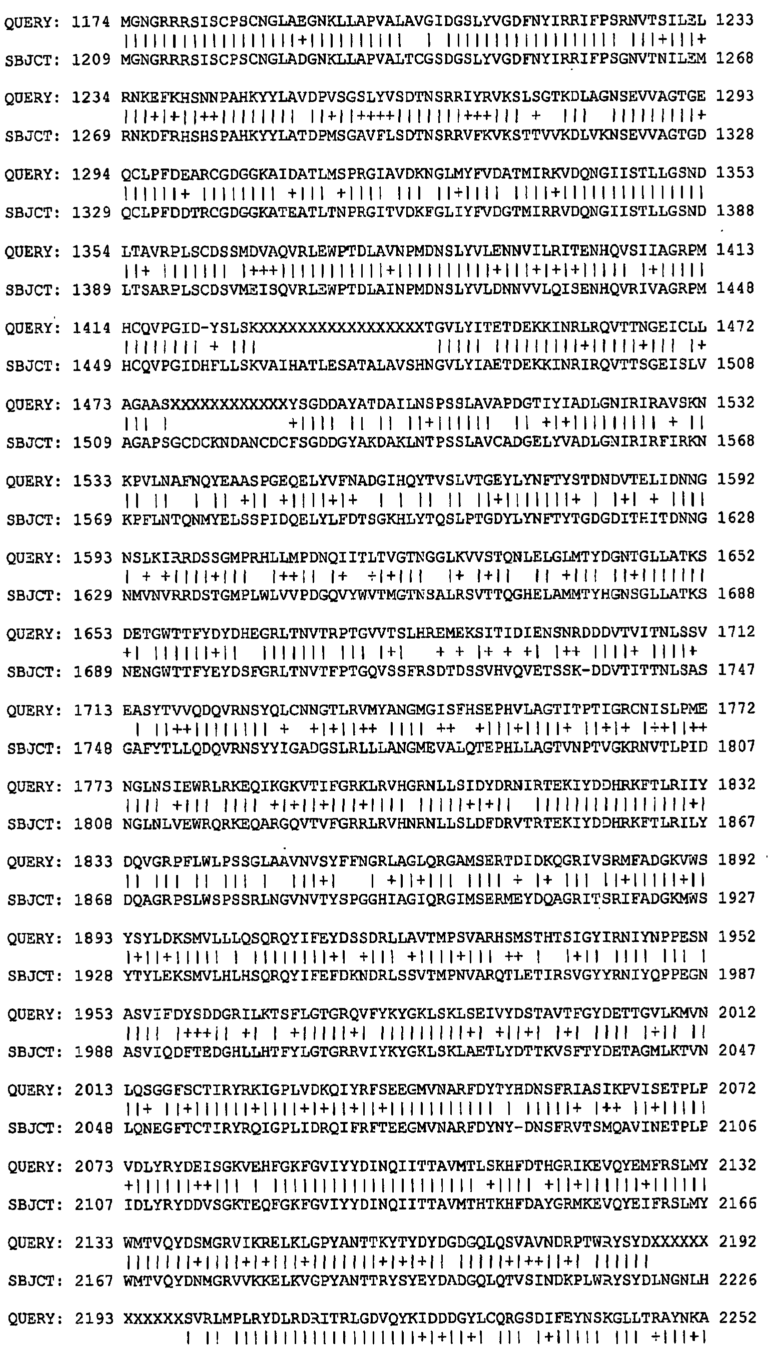

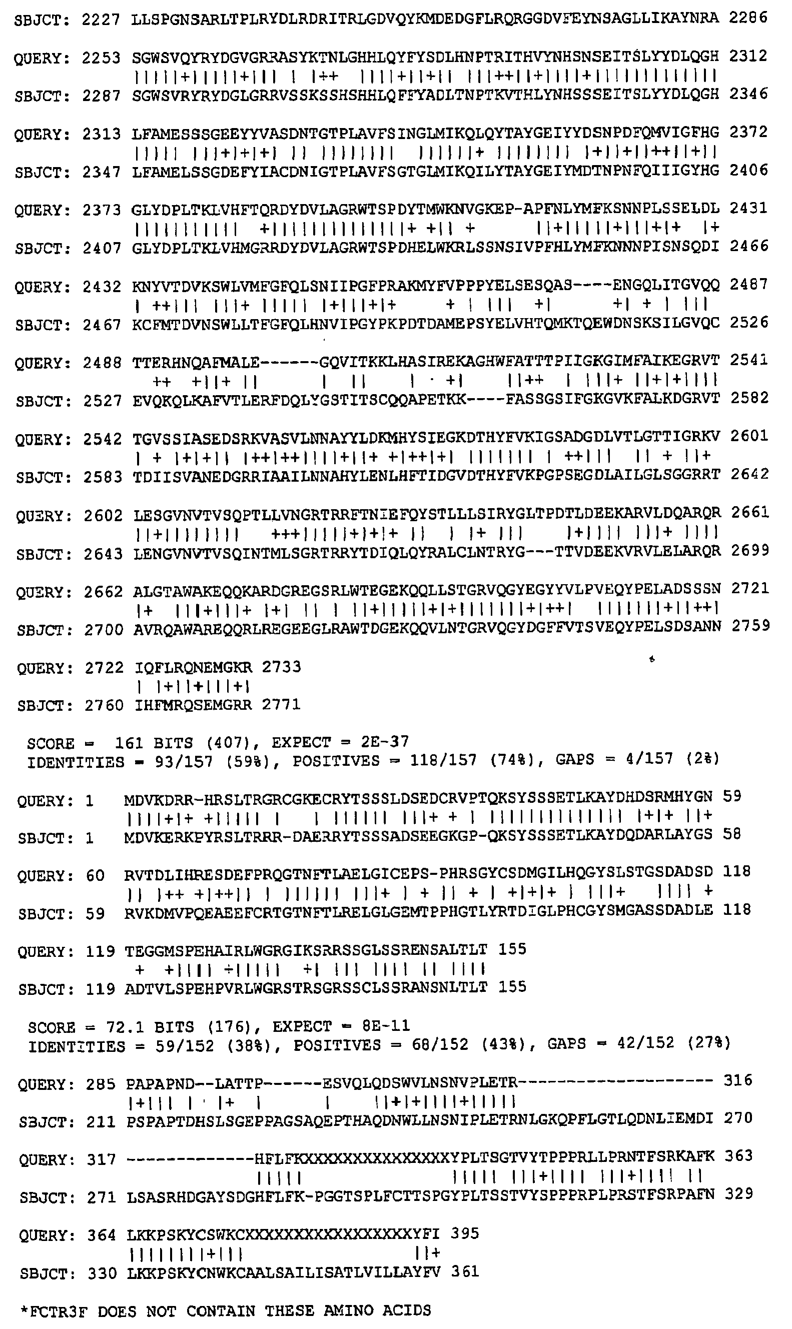

The 997-2733 amino acid fragment of the FCTR3bcde and f protein was also found to have 1695 of 1737 amino acid residues (97%) identical to, and 1695 of 1737 residues (97%) positive with the amino a 1737 amino acid residue protein KIAA1127 protein [*Homo sapiens*] (GenBank Acc:(AB032953) (SEQ ID NO:71), (Table 3S).

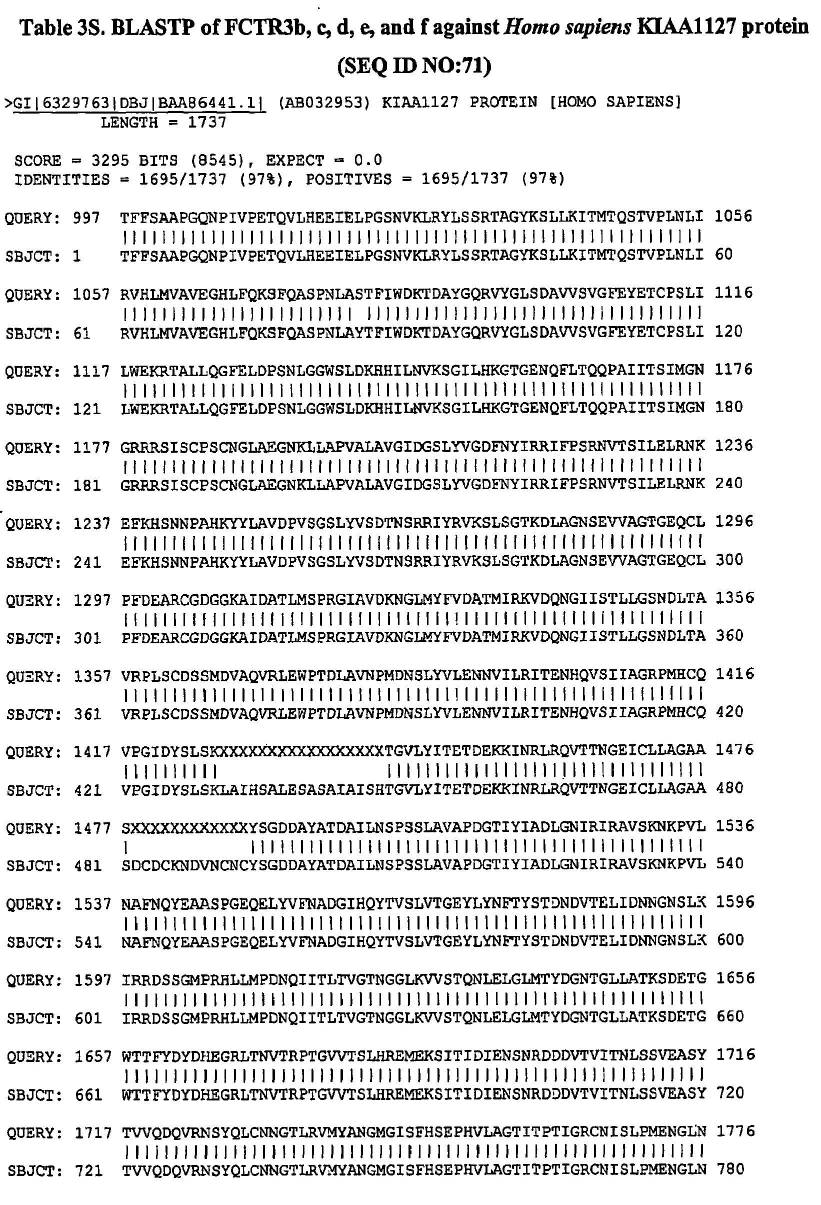

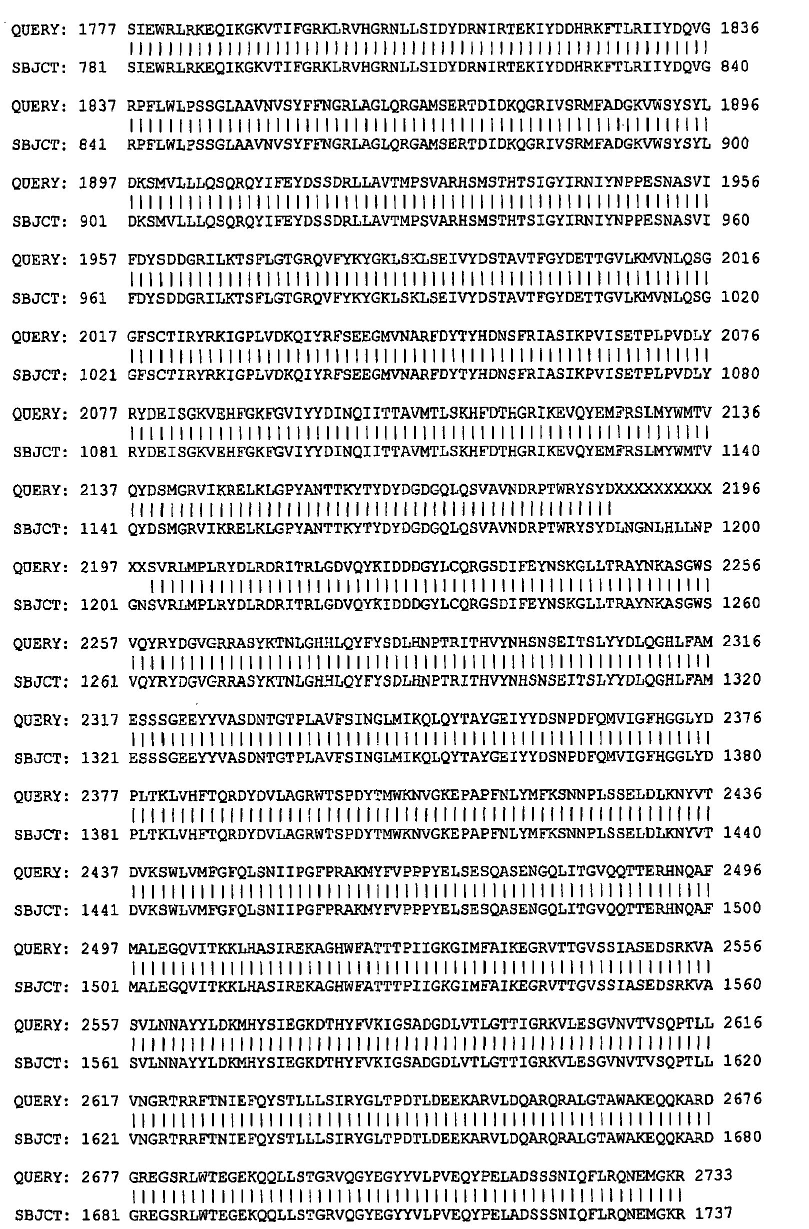

The amino acid sequences of the FCTR3bcde and f proteins were also found to have 2528 of 2774 amino acid residues (91%) identical to, and 2557 of 2774 residues (92%) positive with, the 2765 amino acid residue protein neurestin alpha [*Rattus norvegicus*] (GenBank Acc:AF086607) (SEQ ID NO:72), shown in Table 3T.

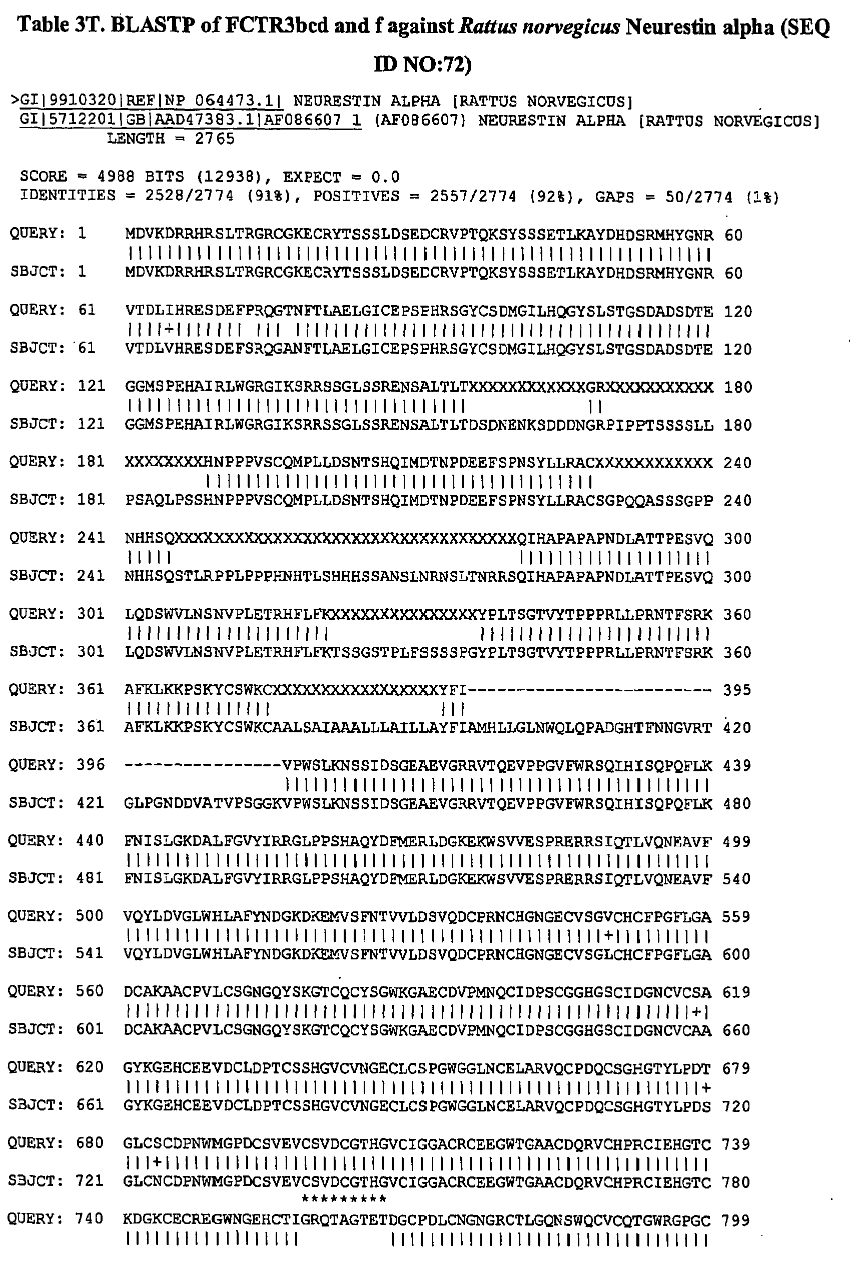

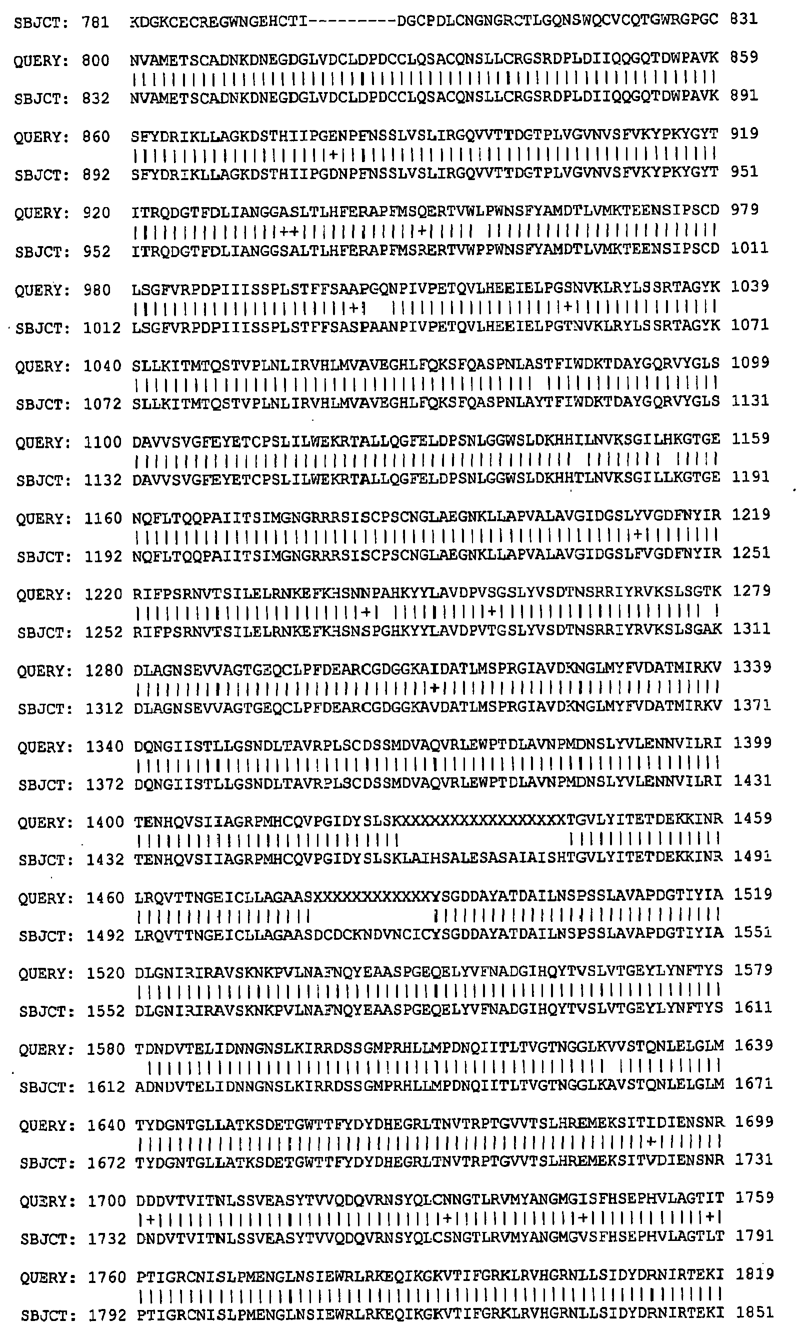

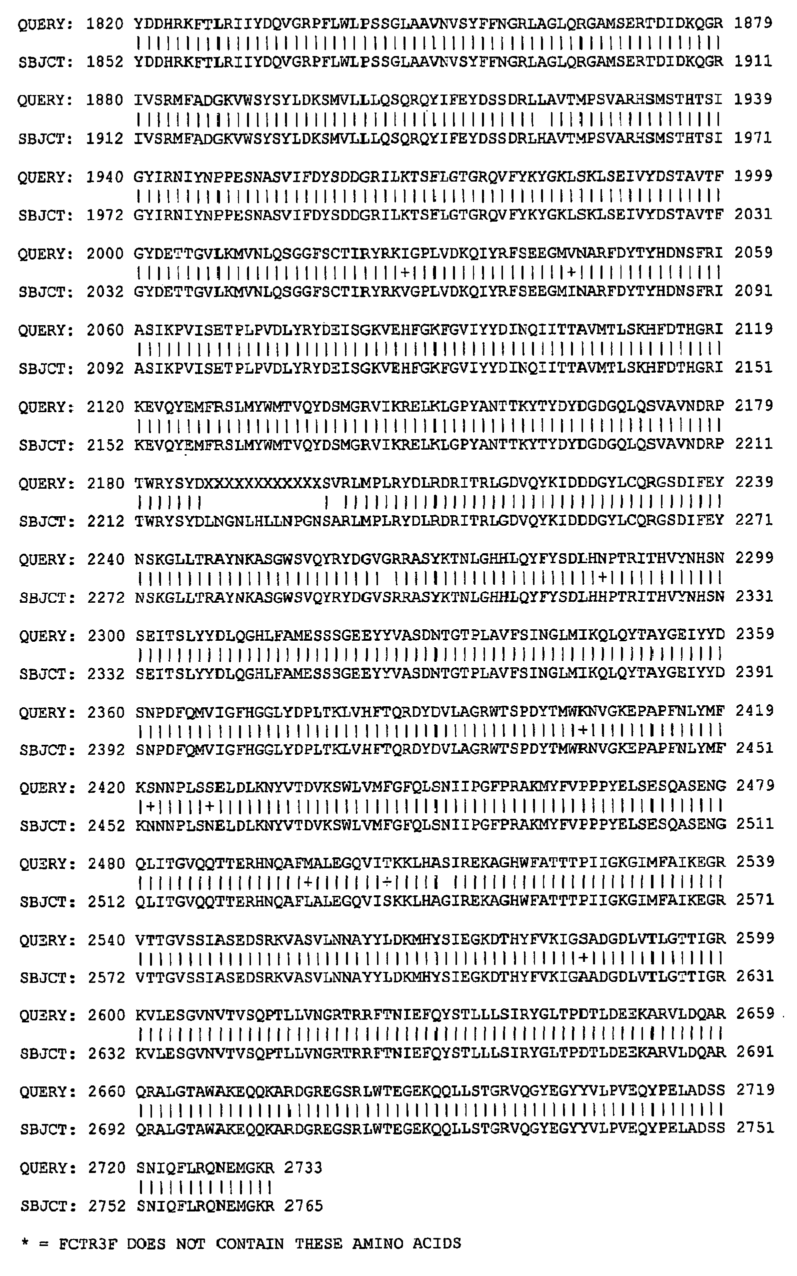

The amino acid sequences of the FCTR3bcde and f proteins were also found to have 2536 of 2774 amino acid residues (91%) identical to, and 2558 of 2774 residues (91%) positive with, the 2764 amino acid residue protein Odd Oz/ten-m homolog 2 (*Drosophila*) (GenBank Acc:NP_035986.2) (SEQ ID NO:65), shown in Table 3U.

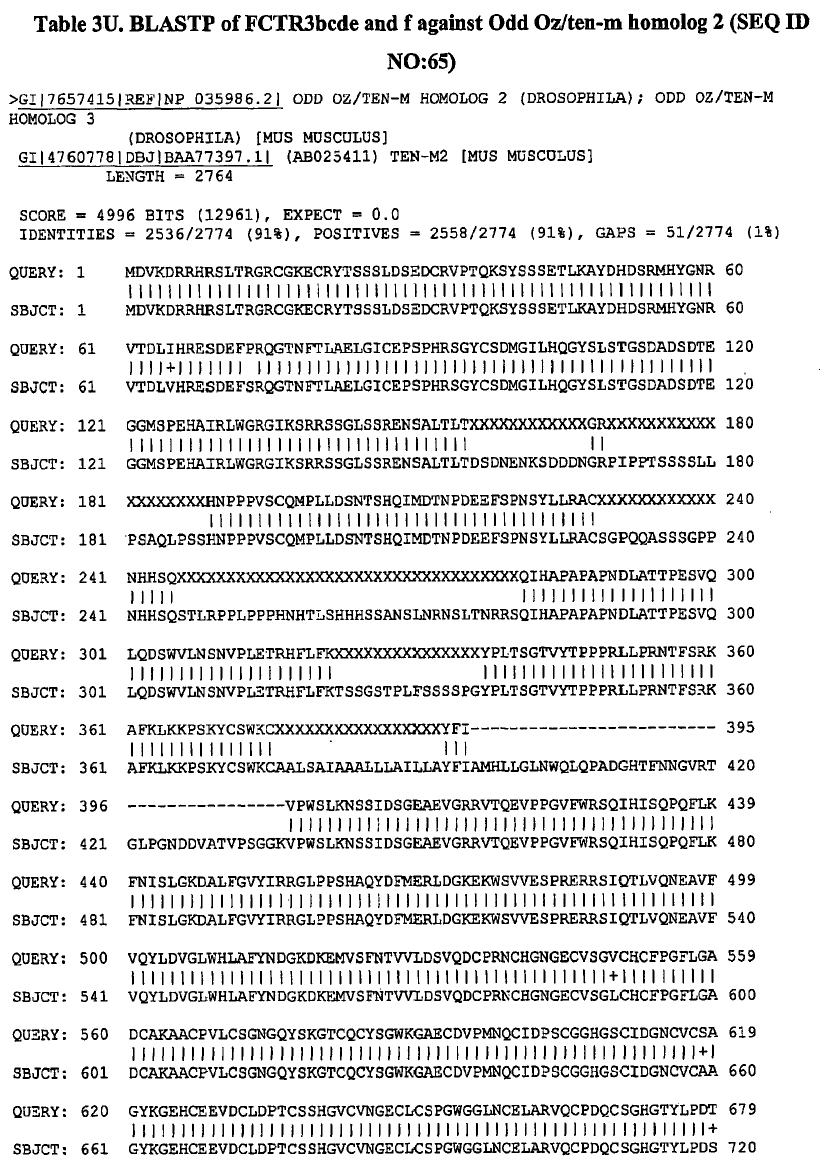

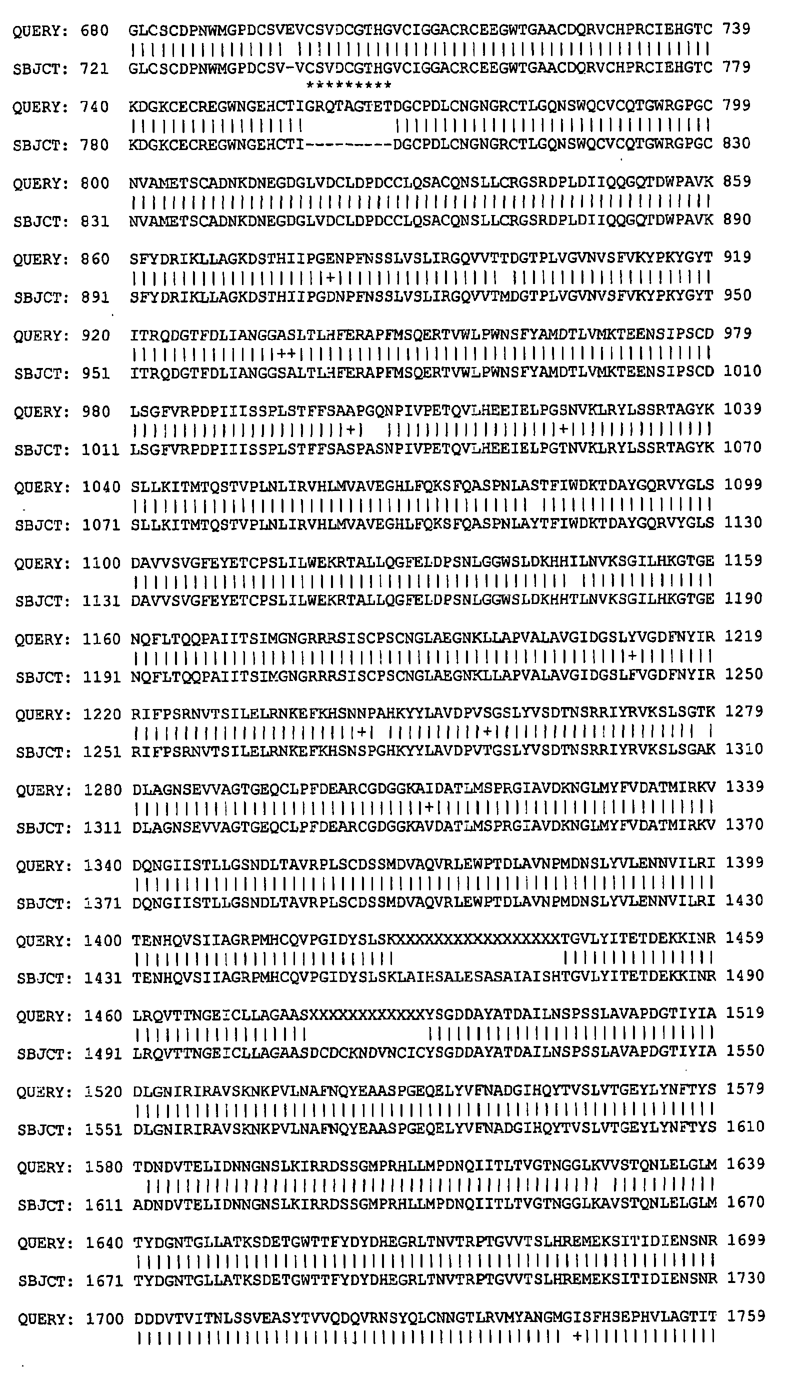

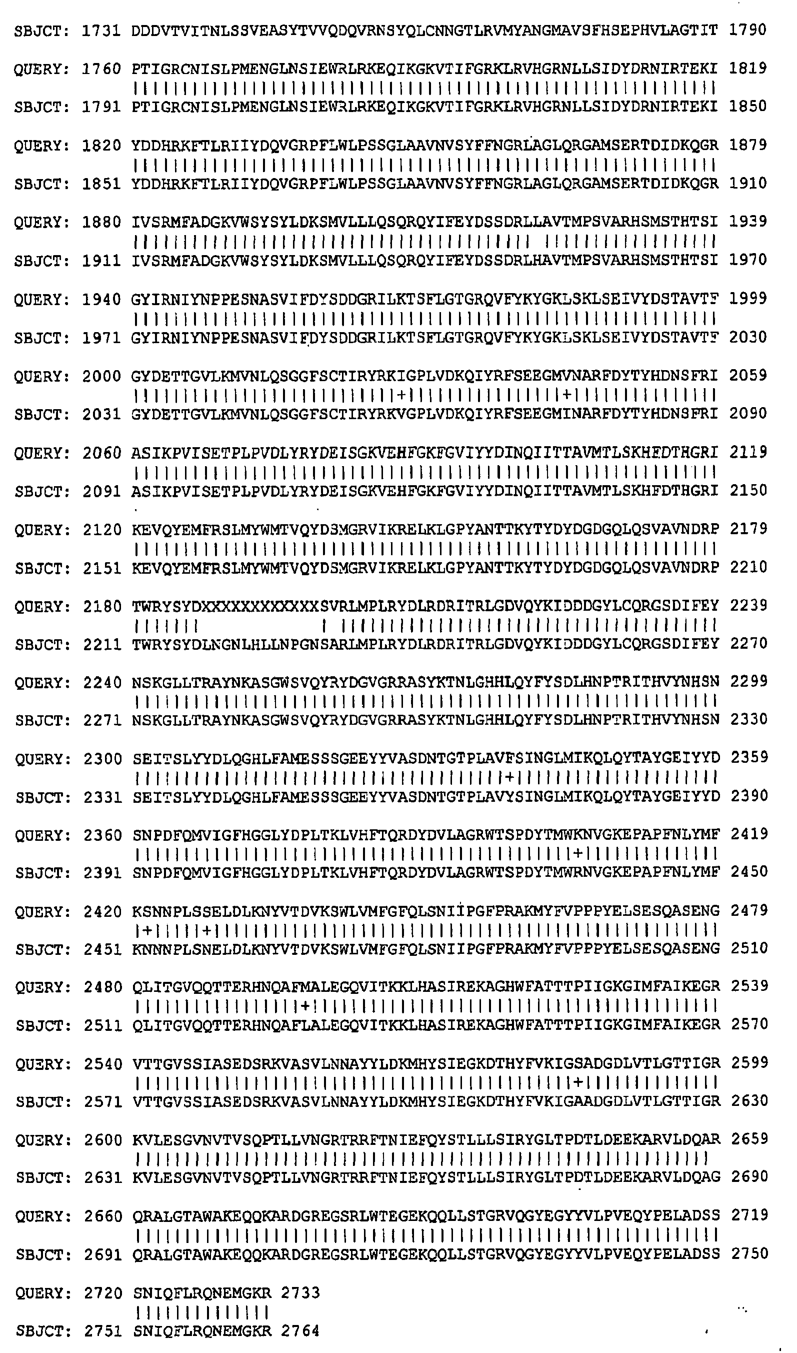

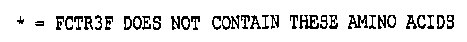

FCTR3 is related to rat neurestin, a gene implicated in neuronal development (Otaki JM, Firestein S Dev Biol 1999 Aug 1;212(1):165-81) Neurestin shows homology to human gamma-heregulin, a Drosophila receptor-type pair-rule gene product, Odd Oz (Odz) / Ten(m), and Ten(a). Neurestin has putative roles in synapse formation and brain morphogenesis. A mouse neurestin homolog, DOC4, has independently been isolated from the NIH-3T3 fibroblasts. DOC4 is also known as tenascin M (TNM), a *Drosophila* pair-rule gene homolog containing extracellular EGF-like repeats. The significant homology to these molecules and in particular, γ-heregulin, have important implications regarding the potential contribution of FCTR3 to disease progression. Heregulin is the ligand for HER-2/ErbB2/NEU, a proto-oncogene receptor tyrosine kinase implicated in breast and prostate cancer progression that was originally identified in rat neuro/glioblastoma cell lines. Extopic expression of HER-2/ErbB2/NEU in MDA-MB-435 breast adenocarcinoma cells confers chemoresistance to Taxol-induced apoptosis relative to vector transfected control cells (Yu et al. Overexpression of ErbB2 blocks Taxol-induced apoptosis by up-regulation of p21Cip1, which inhibits p34Cdc2 kinase. Molec. Cell 2: 581-591, 1998).

### FCTR3 related tenascins and cancer biology

As mentioned, FCTR3 also has significant homology to DOC4, (AKA tenascin M), a *Drosophila* pair-rule gene homolog containing extracellular EGF-like repeats. The tenascins are a growing family of extracellular matrix proteins that play prominent roles in tissue interactions critical to embryogenesis. Overexpression of tenascins has been described in multiple human solid malignancies.

The role of the tenascin family of related proteins is to regulate epithelial-stromal interactions, participate in fibronectin-dependent cell attachment and interaction. Indeed, tenascin-C (TN) is overexpressed in the stroma of malignant ovarian tumours particularly at the interface between epithelia and stroma leading to suggestions that it may be involved in the process of invasion (Wilson et al (1996) Br J Cancer 74: 999-1004). Tenascin-C is considered a therapeutic target for certain malignant brain tumors (Gladson CL : J Neuropathol Exp Neurol 1999 Oct;58(10):1029-40). Stromal or moderate to strong periductal Tenascin-C expression in DCIS (ductal carcinoma in situ) correlates with tumor cell invasion. (Jahkola et al. Eur J Cancer 1998 Oct;34(11):1687-92. Tenascin-C expression at the invasion border of early breast cancer is a useful predictor of local and distant recurrence. Jahkola T, et al. Br J Cancer. 1998 Dec;78(11):1507-13). Tenascin (TN) is an extracellular matrix protein found in areas of cell migration during development and expressed at high levels in migratory glioma cells. Treasurywala S, Berens ME Glia 1998 Oct;24(2):236-43 Migration arrest in glioma cells is dependent on the alphaV integrin subunit. Phillips GR, Krushel LA, Crossin KL J Cell Sci 1998 Apr;111 (Pt 8):1095-104 Domains of tenascin involved in glioma migration. Finally, tenascin expression in hormone-dependent tissues of breast and endometrium indicate that Tenascin expression reflects malignant progression and is down-regulated by antiprogestins during terminal differentiation of rat mammary tumors (Vollmer et al. Cancer Res 1992 Sep 1;52(17):4642-8)

### Potential role of FCTR3 in oncologic disease progression:

Based on the bioactivity described in the medical literature for related molecules, FCTR3 may play a role in one or more aspects of tumor cell biology that alter the interactions of tumor epithelial cells with stromal components. In consideration, FCTR3 may play a role in the following malignant properties:
Autocrine/paracrine stimulation of tumor cell proliferation
Autocrine/paracrine stimulation of tumor cell survival and tumor cell resistance to cytotoxic therapy
Local tissue remodeling, paranechmal and basement membrane invasion and motility of tumor cells thereby contributing to metastasis.
Tumor-mediated immunosuppression of T-cell mediated immune effector cells and pathways resulting in tumor escape from immune surveilance.

### Therapeutic intervention targeting FCTR3 in oncologic and central nervous system indications:

Predicted disease indications from expression profiling in 41 normal human tissues and 55 human cancer cell lines (see Example 2) include a subset of human gliomas, astrocytomas, mixed glioma/astrocytomas, renal cells carcinoma, breast adenocarcinoma, ovarian cancer, melanomas. Targeting of FCTR3 by human or humanized monoclonal antibodies designed to disrupt predicted interactions of FCTR3 with its cognate ligand may result in significant anti-tumor/anti-metastatic activity and the amelioration of associated symptomatology. Identification of small molecules that specifically/selectively interfere with downstream signaling components engaged by FCTR3/ligandinteractions would also be expected to result in significant anti-tumor/anti-metastatic activity and the amelioration of associated symptomatology. Likewise, modified antisense ribonucleotides or antisense gene expression constructs (plasmids, adenovirus, adeno-associated viruses, "naked" DNA approaches) designed to diminish the expression of FCTR3 transcripts/messenger RNA (mRNA) would be anticipated based on predicted properties of FCTR3 to have anti-tumor impact.

Based on the relatedness to neurestin and heregulins, as well as its high level expression in brain tissue, FCTR3 may also be used for remyelination in order to promote regeneration/repair/remyleination of injured central nervous system cells resulting from ischemia, brain trauma and various neurodegenerative diseases.. This postulate is based on reports indicating that neuregulin, glial growth factor 2, diminishes autoimmune demyelination and enhances remyelination in a chronic relapsing model for multiple sclerosis (Cannella et al.. Proc. Nat. Acad. Sci. 95: 10100-10105, 1998). The expression of the related molecule neurestin can be induced in external tufted cells during regeneration of olfactory sensory neurons.

### FCTR4

FCTR4 is a plasma membrane protein related to NF-Kappa-B P65delta3 protein. The clone is expressed in fetal liver tissues.

The novel FCTR4 nucleic acid of 609 nucleotides (also referred to as 29692275.0.1) is shown in Table 4A. An ORF begins with an ATG initiation codon at nucleotides 99-101 and ends with a TAA codon at nucleotides 522-524. A putative untranslated region upstream from the initiation codon and downstream from the termination codon is underlined in Table 4A, and the start and stop codons are in bold letters.

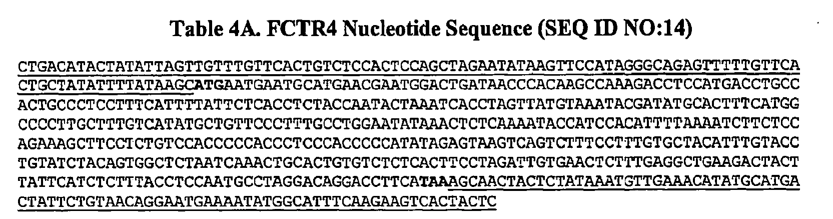

The FCTR4 protein encoded by SEQ ID NO:14 has 141 amino acid residues and is presented using the one-letter code in Table 4B. The Psort profile for FCTR4 predicts that this sequence has no N-terminal signal peptide and is likely to be localized at the plasma membrane with a certainty of 0.6000. The most likely cleavage site for a peptide is between amino acids 39 and 40, *i.e.,* at the dash in the amino acid sequence ACT-CCA, based on the SignalP result. The predicted molecular weight of this protein is 16051.5 Daltons.

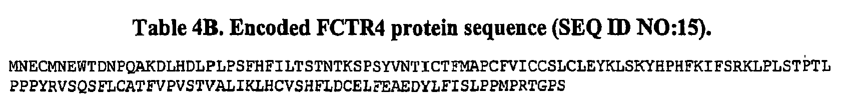

The predicted amino acid sequence was searched in the publicly available GenBank database FCTR4 protein showed 30 % identities (22 over 72 amino acids) and 43% homologies (31over 72 amino acids) with hypothetical 10 kD protein of *Trypanosoma cru*zi (86 aa; ACC:Q99233) shown in Table 4C. The best homologies with a human protein were 54 % identities (114 over 343 amino acids) with NF-Kappa-B P65delta3 protein (71 aa fragment; ACC:Q13313) (SEQ ID NO:77).

**Table 4C. BLASTP of FCTR4 against protein sequences**

| BLAST X search results are shown below: |
|---|
| ptnr:SPTREMBL-ACC:Q99233 HYPOTHETICAL 10 KD PROTEIN +3, 68, 0.60, 1, (SEQ ID NO:73) |
| ptnr:SPTREMBL-ACC:Q16896 GABA RECEPTOR SUBUNIT - AEDES +3, 66, 0.81, 4 (SEQ ID NO:74) |
| ptnr:SPTREMBL-ACC:076473 GABA RECEPTOR SUBUNIT - LEPTI... +3, 66, 0.99, 2 (SEQ ID NO:75) |
| ptnr:TREMBLNEW-ACC:AAD28317 F13J11.13 PROTEIN - Arabid... +3, 62, 0.99, 1 (SEQ ID NO:76) |

Based upon homology, FCTR4 proteins and each homologous protein or peptide may share at least some activity.

### FCTR5

FCTR5 is a protein bearing sequence homology to human complement C1R component precursor. The clone is expressed in breast, heart, lung, fetal lung, salivary gland, adrenal gland, spleen, kidney, and fetal kidney.

The novel FCTR5 nucleic acid of 1667 nucleotides (also referred to as 32125243.0.21) is shown in Table 5A. An ORF begins with an ATG initiation codon at nucleotides 34-36 and ends with a TGA codon at nucleotides 1495-1497. A putative untranslated region upstream from the initiation codon and downstream from the termination codon is underlined in Table 5A, and the start and stop codons are in bold letters.

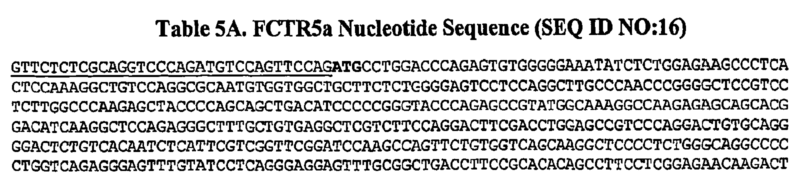

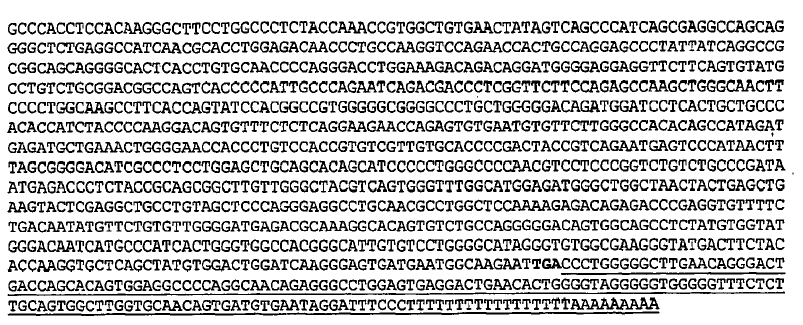

The FCTR5 protein encoded by SEQ ID NO:16 has 487 amino acid residues, and is presented using the one-letter code in Table 5B. FCTR5 was searched against other databases using SignalPep and PSort search protocols. The FCTR5 protein is most likely microbody (peroxisome) (Certainty=0.6406) and seems to have no N-terminal signal sequence. The predicted molecular weight of FCTR5 protein is 53511.9 daltons.

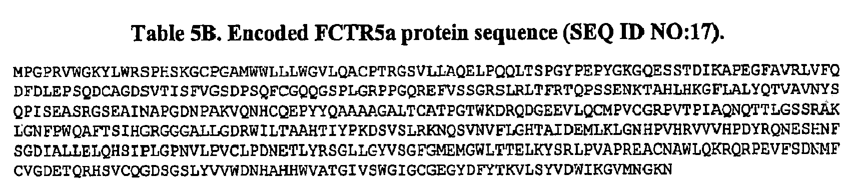

An alternative embodiment, FCTR5b, is a 1691 base sequence shown in Table 5C.

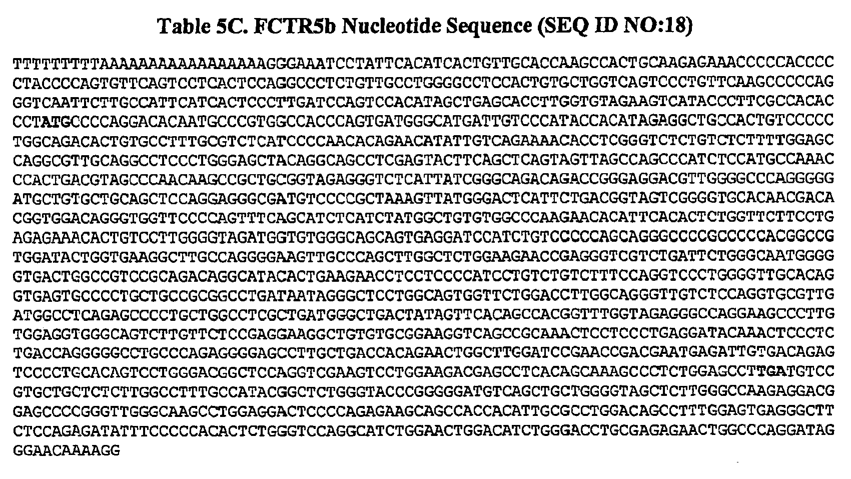

The FCTR5b protein encoded by SEQ ID NO:18 has 487 amino acid residues, and is presented using the one-letter code in Table 5D. FCTR5 was searched against other databases using SignalPep and PSort search protocols. The FCTR5b protein is most likely microbody (peroxisome) (Certainty=0.6406) and seems to have no N-terminal signal sequence. The predicted molecular weight of FCTR5 protein is 53511.9 daltons.

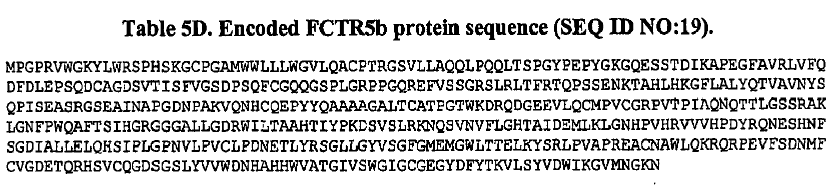

The predicted amino acid sequence was searched in the publicly available GenBank database FCTR5a protein showed 58 % identities (177 over 302 amino acids) and 74 % homologies (226 over 302 amino acids) with human complement C1R component precursor (EC 3.4.21.41) (705 aa.; ACC:P00736). Based upon homology, FCTR5 proteins and each homologous protein or peptide may share at least some activity.

In a search of sequence databases, it was found, for example, that the nucleic acid sequence the nucleotides 17-1594 of FCTR5a have 1575 of 1578 bases (99 %) identical to *Homo sapiens* complement C1r-like proteinase precursor (GENBANK-ID: XM_007061.1) (SEQ ID NO:78) (Table 5E).

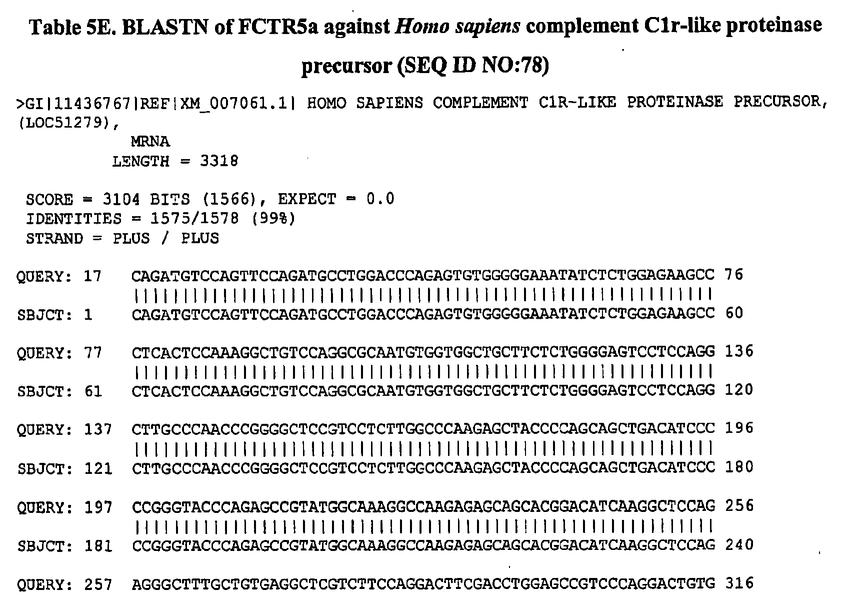

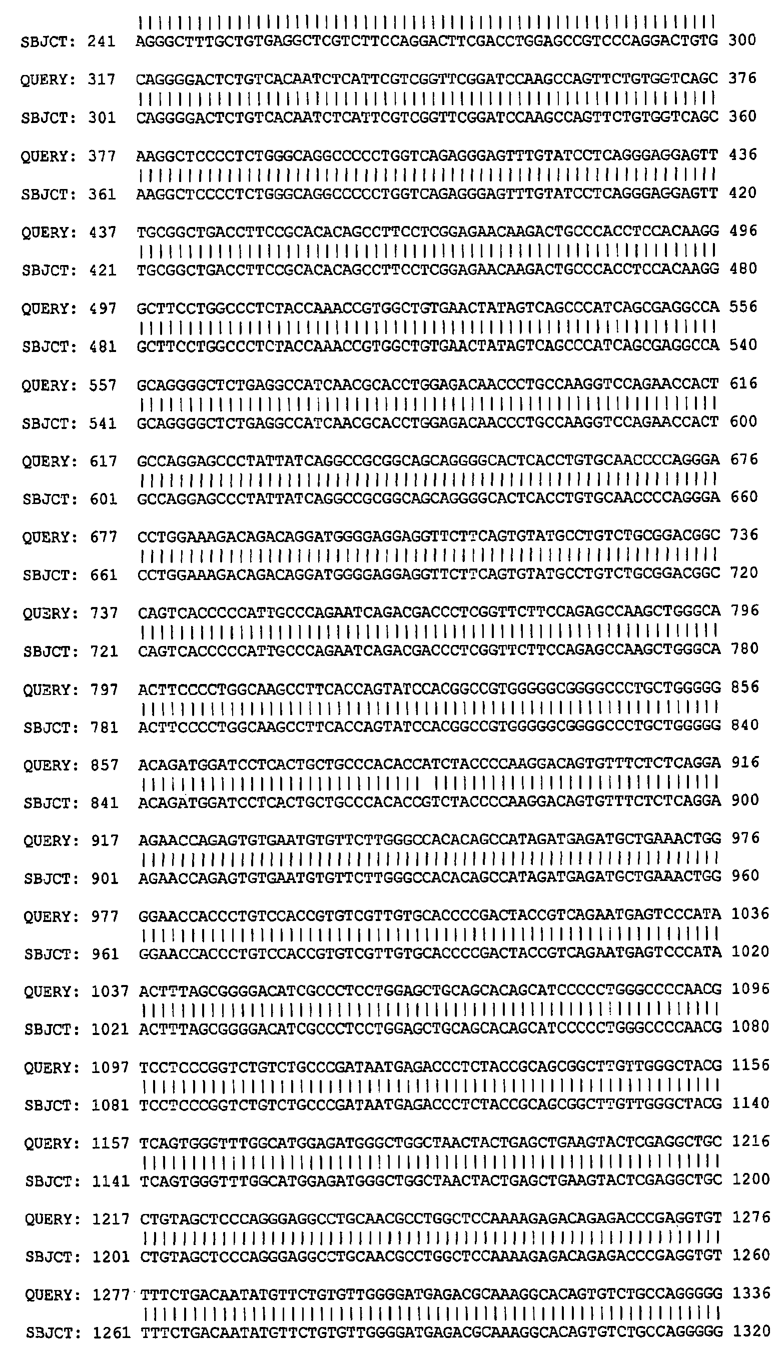

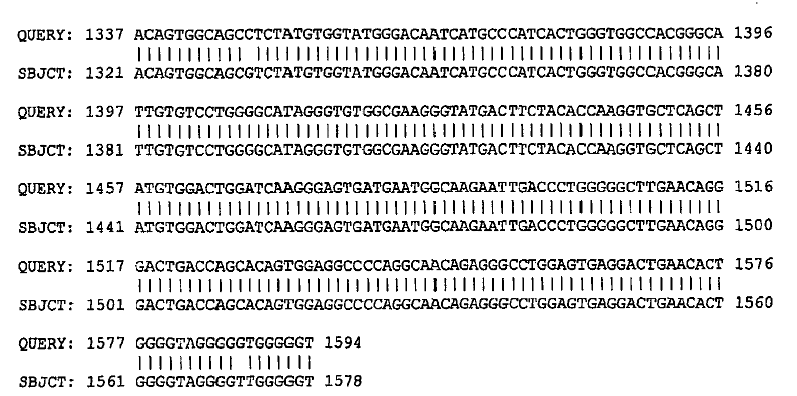

In this search it was also found that the FCTR5a nucleic acid had homology to three fragments of *Homo sapiens* complement component 1, r subcomponent. It has 102 of 117 bases (87%) identical to 1458-1574, 82 of 94 bases (87%) identical to 2052-2145, and 54 of 63 bases (85%) identical to 1678-1740 all fragments of *Homo sapiens* complement component 1, r subcomponent (GenBank Acc: NM_001733.1) (Table 5F).

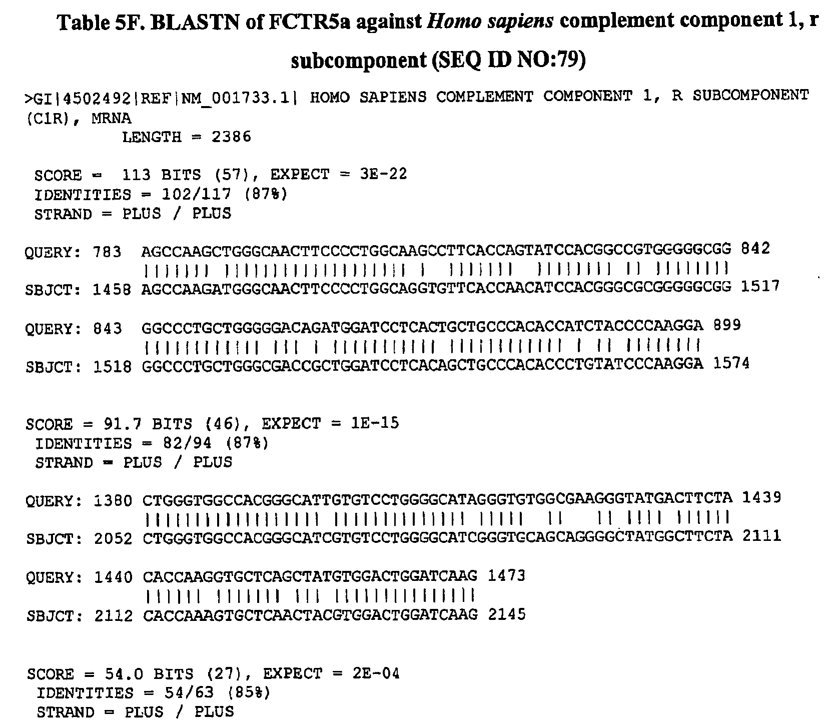

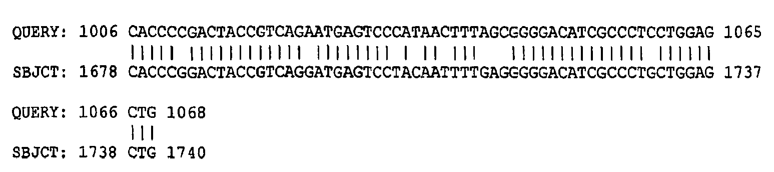

The amino acid sequence of the protein of FCTR5a 485 of 487 amino acid residues (99%) identical to, and 487 of 487 residues (100%) positive with, the 487 amino acid complement C1r-like proteinase precursor from *Homo sapiens* (GenBank-ACC: AAF44349.1) (SEQ ID NO:80) (Table 5G).

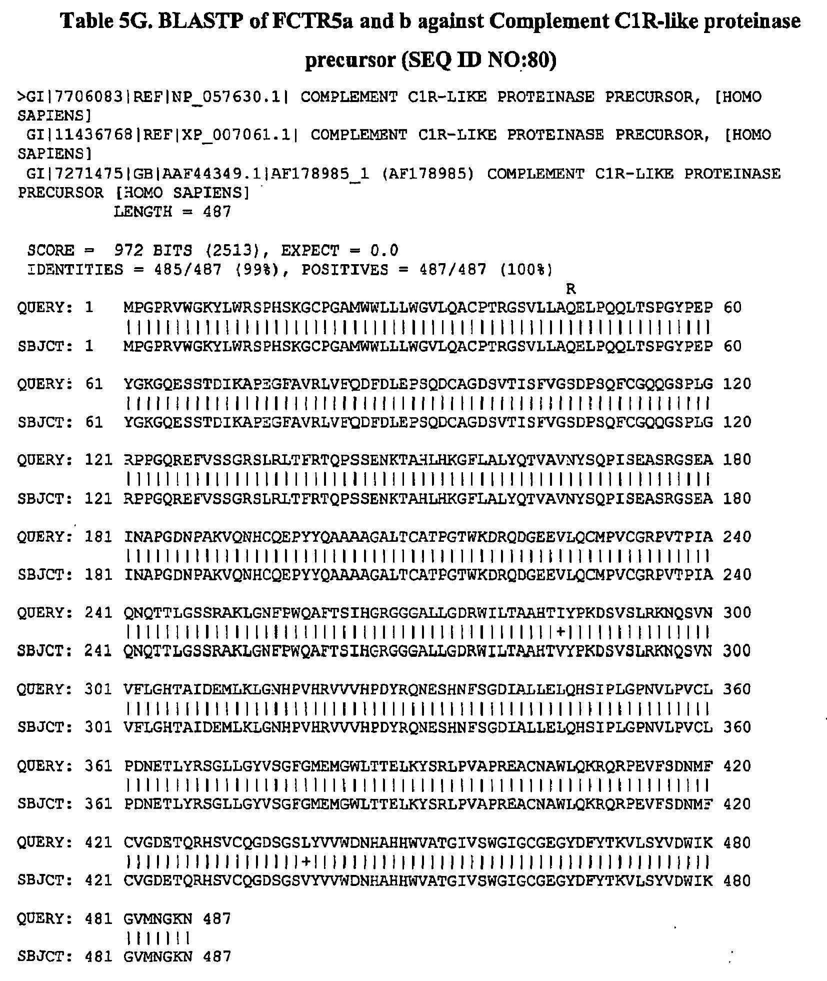

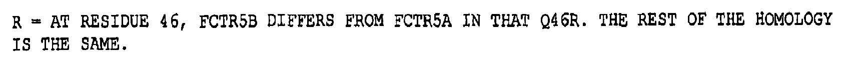

The full amino acid sequence of the protein of FCTR5 a has 175 of 303 amino acid residues (58%) identical to, and 226 of 303 residues (74%) positive with the 400-701 amino acid segment, 72 of 157 residues (45%) identical and 94 of 157 residues (59%) positive with amino acids 1-155, and 36 of 139 residues (25%) identical and 58 of 139 residues (40%) positive with amino acids 188-312 of the 705 amino acid Complement C1R Component Precursor from *Homo sapiens* (GenBank-ACC: AAA51851.1) (SEQ ID NO:43) (Table 5H).

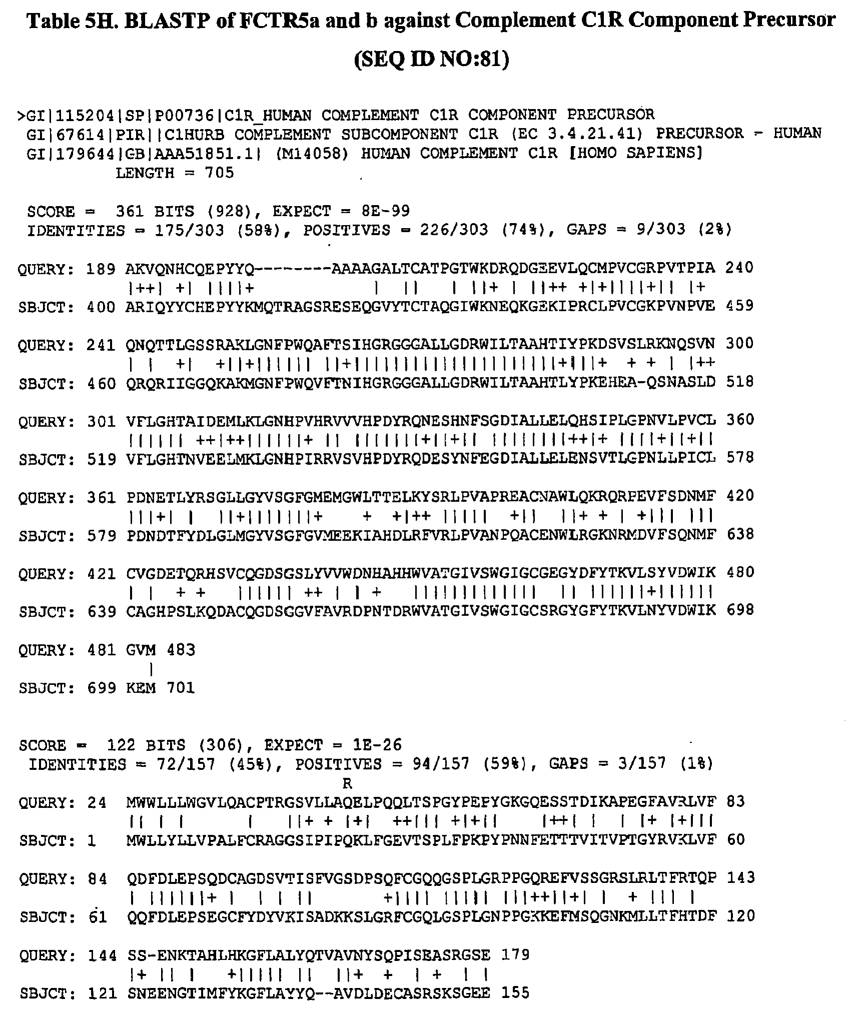

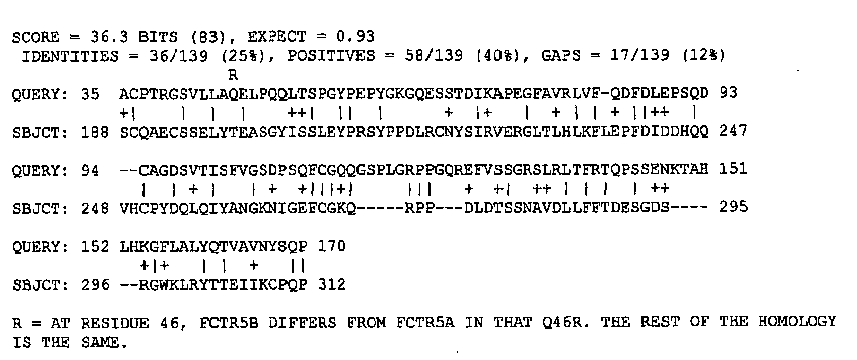

Based upon homology, FCTR5 proteins and each homologous protein or peptide may share at least some activity.

### FCTR6

The novel nucleic acid of 1078 nucleotides FCTR6a (also designated 27455183.0.19) encoding a novel human blood coagulation factor XI-like protein is shown in Table 6A. An ORF was identified beginning with an ATG initiation codon at nucleotides 243-245 and ending with a TAA codon at nucleotides 1044-1046. A putative untranslated region upstream from the initiation codon and downstream from the termination codon is underlined in Table 6A, and the start and stop codons are in bold letters.

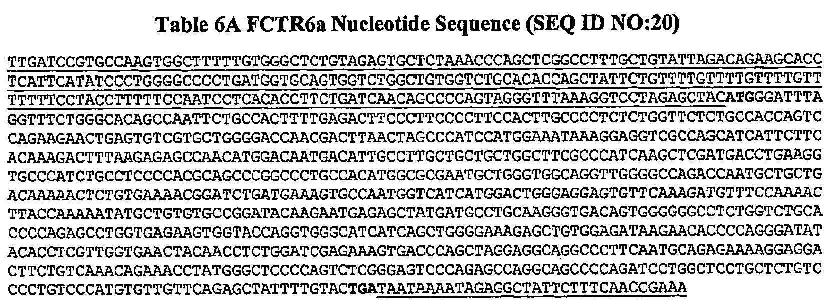

The FCTR6a protein encoded by SEQ ID NO:20 has 267 amino acid residues and is presented using the one-letter code in Table 6B. FCTR6a was searched against other databases using SignalPep and PSort search protocols. The FCTR6a protein is most likely mitochondrial matrix space (Certainty= 0.4372) and seems to have no N-terminal signal sequence. The predicted molecular weight of FCTR6a protein is 29412.8 daltons.

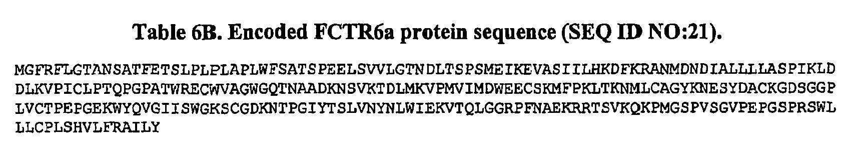

In an alternative embodiment, FCTR6b (alternatively referred to as 27455183.0.145) has the 1334 residue sequence shown in Table 6C. An ORF was identified beginning with an ATG initiation codon at nucleotides 499-501 and ending with a TAA codon at nucleotides 1300-1302. A putative untranslated region upstream from the initiation codon and downstream from the termination codon is underlined in Table 6C, and the start and stop codons are in bold letters.

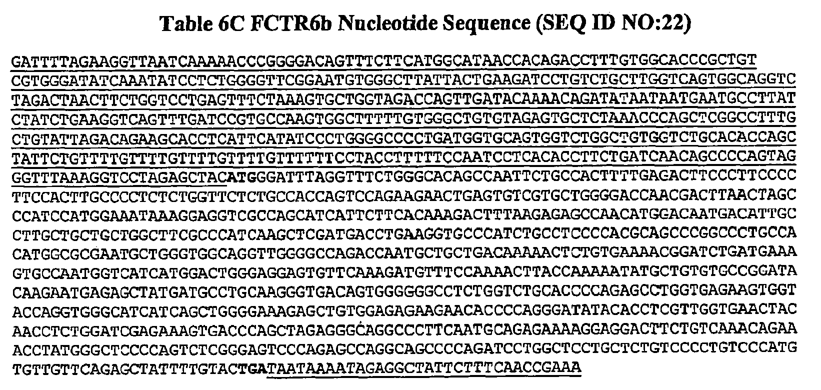

The FCTR6b protein encoded by SEQ ID NO:22 has 267 amino acid residues and is presented using the one-letter code in Table 6B. The Psort profile for FCTR4 predicts that this sequence has no N-terminal signal peptide and is likely to be localized at the mitochondrial matrix space (Certainty=0.4372). The predicted molecular weight of this protein is 29498.9 Daltons.

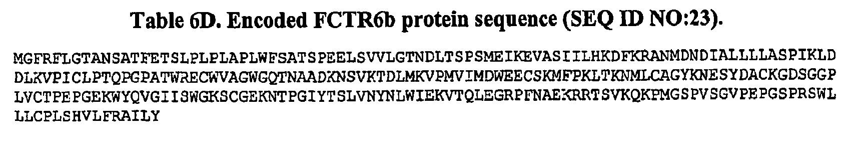

In a search of sequence databases, it was found, for example, that the FCTR6a nucleic acid sequence has 853 of 897 bases (95 %) identical to bases 551-1447, and 346 of 388 bases (89%) identical to bases 127-513 of *Macaca fascicularis* brain cDNA, clone QccE-17034 (GENBANK-ID: |AB046651) (Table 6E).

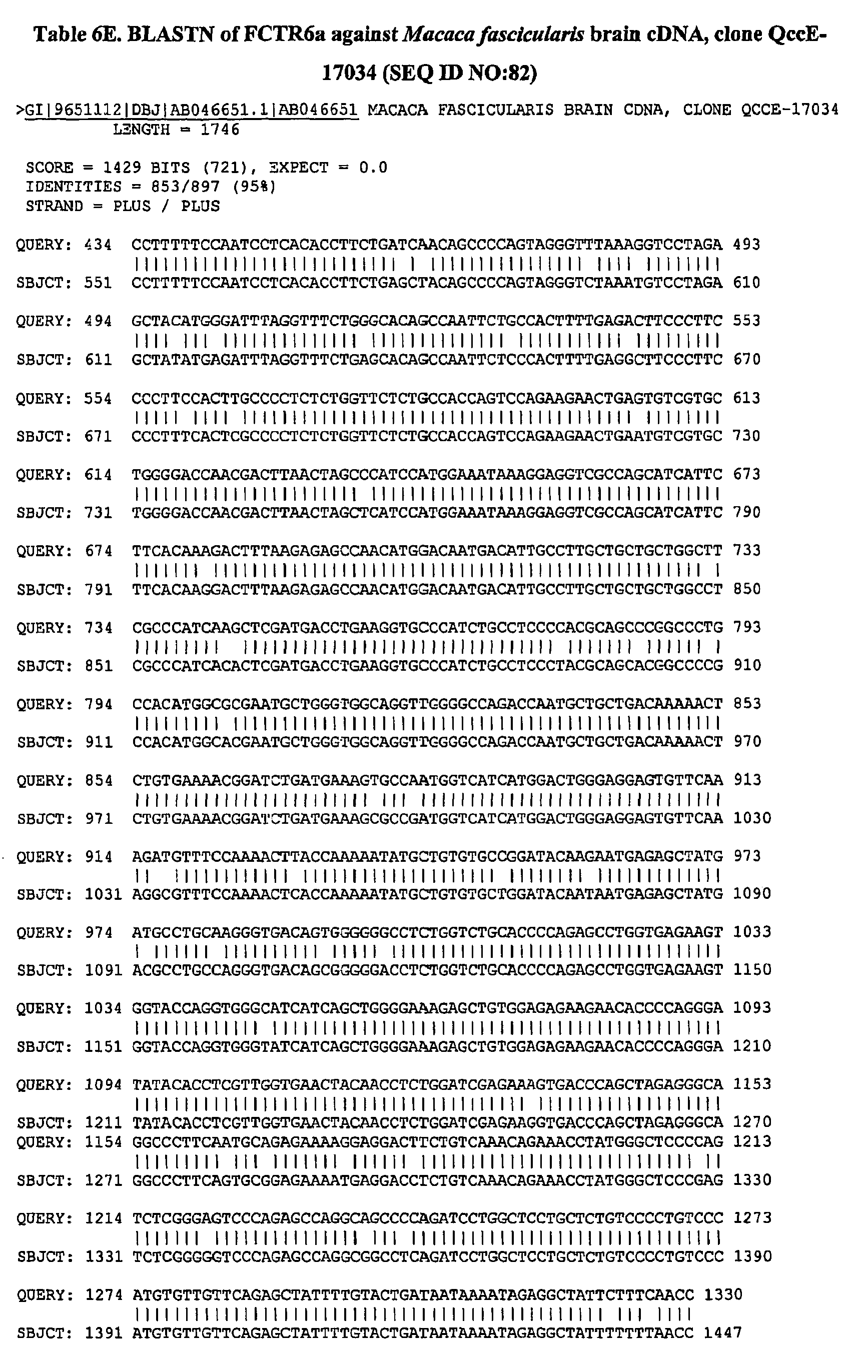

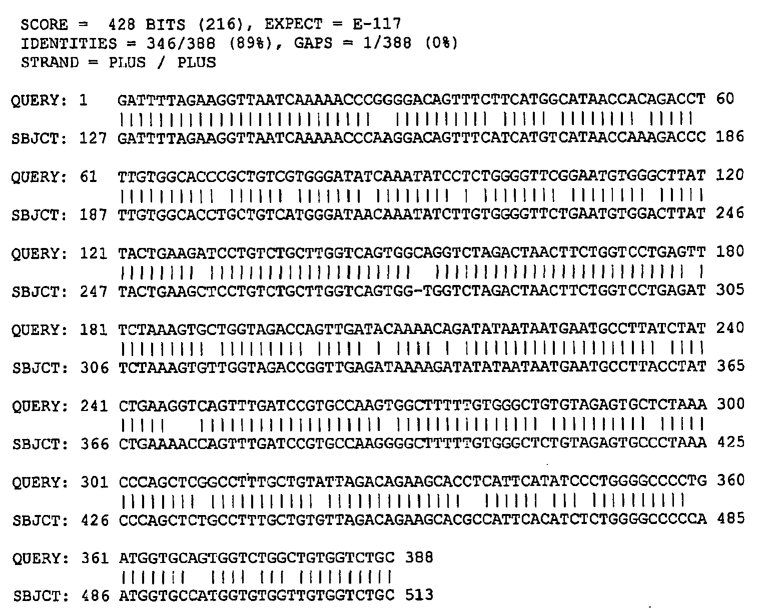

In a search of sequence databases, it was found, for example, that the FCTR6a nucleic acid sequence has 295 of 378 bases (78 %) identical to bases 410-779 of *Mus musculus* adult male testis cDNA, RIKEN full-length enriched (GENBANK-ID:AK09660) (Table 6F).

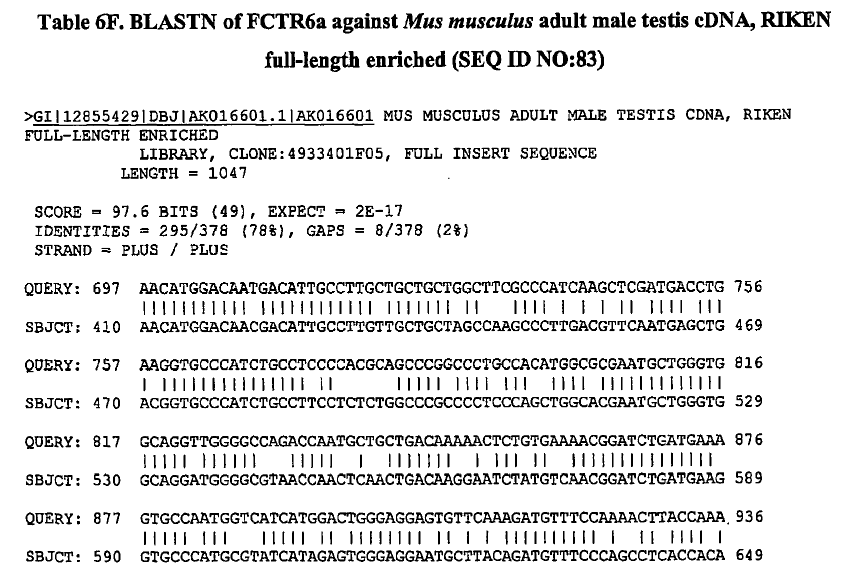

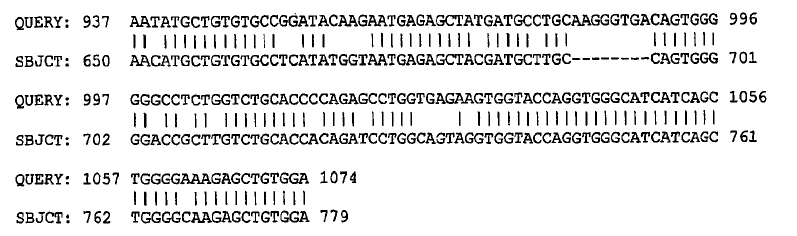

The FCTR6a amino acid has 247 of 267 amino acid residues (92%) identical to, and 251 of 307 residues (94%) positive with, the 267 amino acid hypothetical protein [*Macaca fascicularis*] (GenBank: AB046651) (SEQ ID NO:84) (Table 6G).

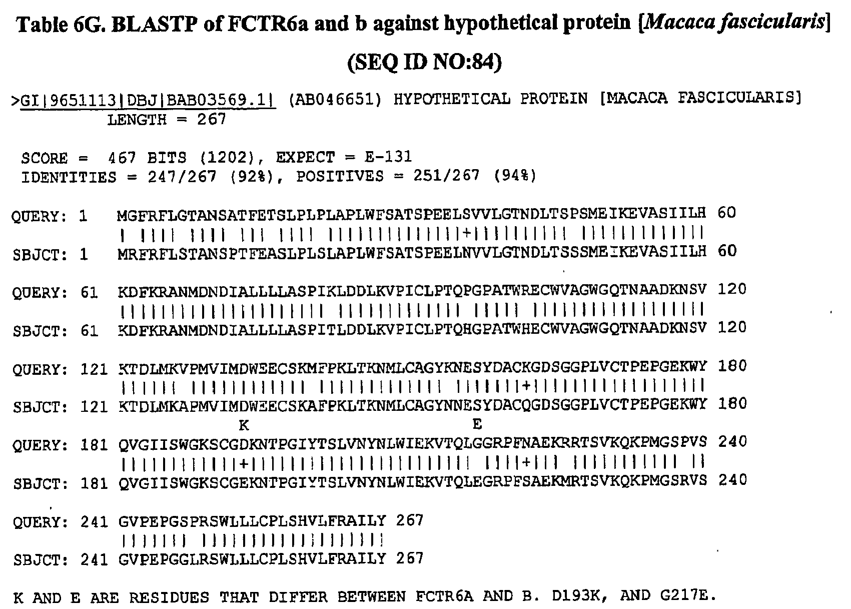

The FCTR6a amino acid has 80 of 201 amino acid residues (39%) identical to, and 119 of 201 residues (58%) positive with, the 638 amino acid plasma kallikrein B1 precursor (GENBANK-ID:NP_000883.1) (SEQ ID NO:85) (Table 6H).

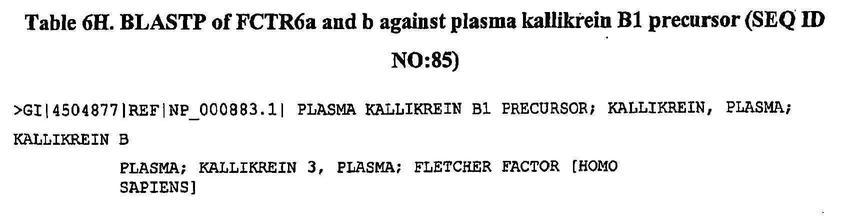

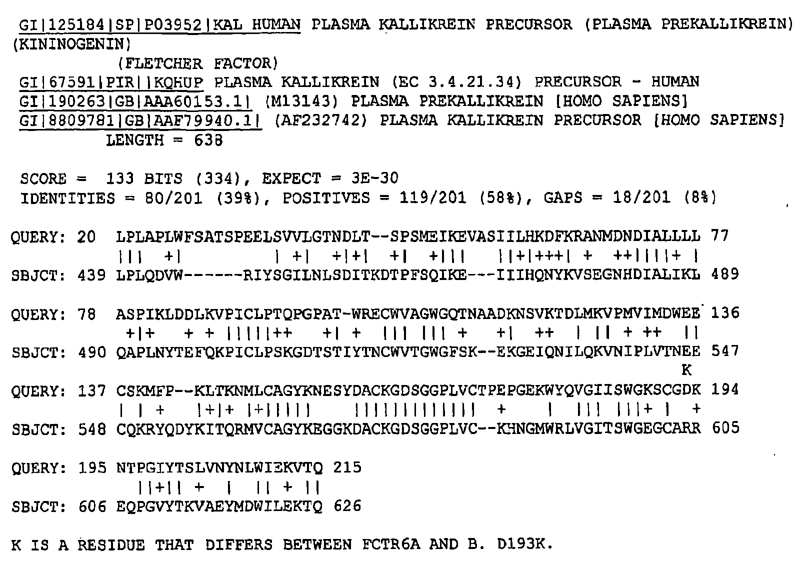

The FCTR6a amino acid has 73 of 183 amino acid residues (39%) identical to, and 110 of 183 residues (59%) positive with, the 643 amino acid kallikrein [*Sus scrofa*] (GENBANK-ID:BAA37147.1) (SEQ ID NO:86) (Table 6I).

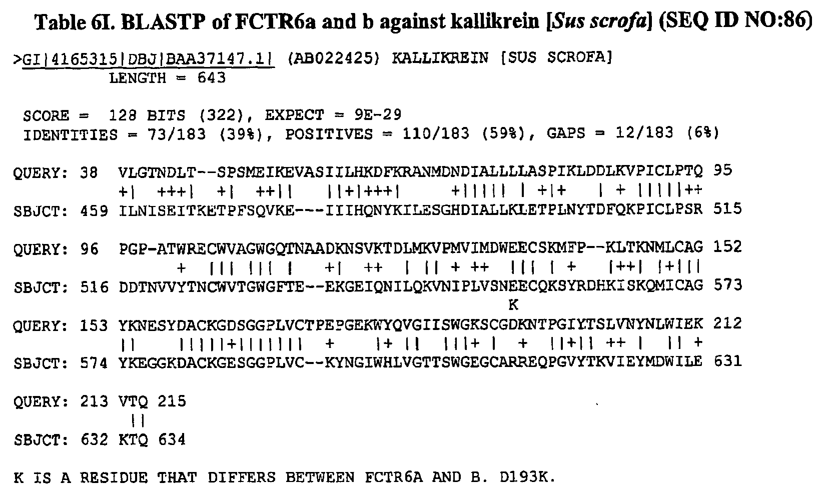

The FCTR6a amino acid has 81 of 205 amino acid residues (39%) identical to, and 112 of 205 residues (54%) positive with, the 625 amino acid Coagulation factor XI [*Homo sapiens*] (embCAA64368.1) (SEQ ID NO:87) (Table 6J).

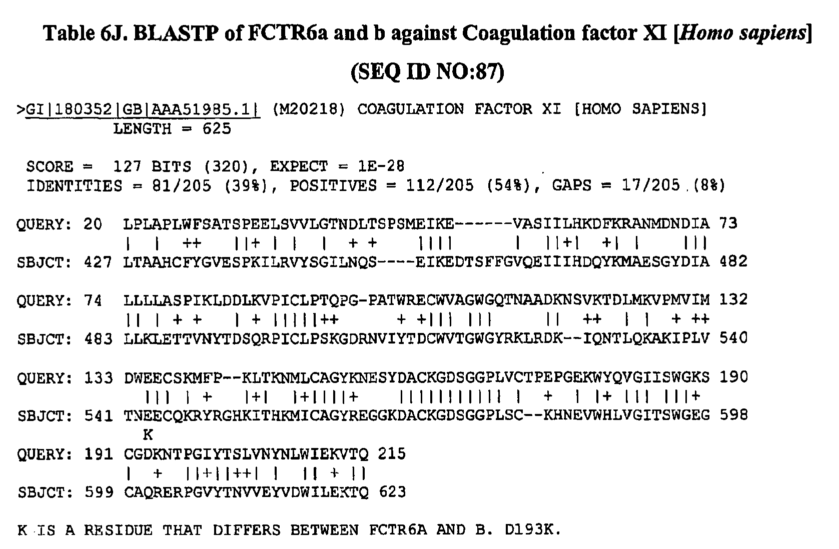

The number of new cases of renal cell carcinoma in the United States in 1996 was projected to be 30,600 with an estimated 12,000 deaths. Tumors with a proposed histogenesis from the proximal tubule (clear-cell and chromophilic tumors) amount to 85% of renal cancers, whereas tumors with a proposed histogenesis from the connecting tubule/collecting duct (chromophobic-, oncocytic-, and duct Bellini-type tumors) amount to only 11%.

Adenocarcinomas may be separated into clear cell and granular cell carcinomas, although the 2 cell types may occur together in some tumors. The distinction between well-differentiated renal carcinomas and renal adenomas can be difficult. The diagnosis is usually made arbitrarily on the basis of size of the mass, but size alone should not influence the treatment approach, since metastases can occur with lesions as small as 0.5 centimeters.

While radical nephrectomy with regional lymphadenectomy, is the accepted, often curative therapy for stage I (localized disease) renal cell cancer, very little therapy is available for advance disease that represent about 70% of the patients. Radiotherapy as a postoperative adjuvant has not been effective, and when used preoperatively, may decrease local recurrence but does not appear to improve 5-yr survival. A chemotherapeutic agent capable of significantly altering the course of metastastic renal cell carcinoma has not been identified. (Renal Cell Cancer (PDQ®) Treatment - Health Professionals, Cancernet, NCl)

There is therefore a need to identify genes that are differentially modulated in renal-cell carcinomas. In addition there is a need for methods to assay candidate therapeutic substances for modulating expression of these genes. These substances might be recombinant protein expressed by the identified genes or antibodies that bind to the identified proteins. There is yet additionally a need for an effective method of identifying target molecules or related components. These and related needs and defects are addressed in the present invention.

### Novel kallikrein-like/coagulation factor XI-like Proteins and Nucleic Acids Encoding Same

FCTR6 is surprisingly found to be differentially expressed in clear cell Renal cell carcinoma tissues vs the normal adjacent kidney tissues. The present invention discloses a novel protein encoded by a cDNA and/or by genomic DNA and proteins similar to it, namely, new proteins bearing sequence similarity to kallikrein-like, nucleic acids that encode these proteins or fragments thereof, and antibodies that bind immunospecifically to a protein of the invention. It may have use as a therapeutic agent in the treatmentof renal cancer and liver cirrhosis.

### The utility of kallikrein family members in protein therapy of Renal cancer

The treatment of renal cell carcinoma with recombinant kallikrein could improve disease outcome through several potential mechanisms. The literature suggests that members of this protein family are inhibitory to the process of angiogenesis, a process of vital importance to tumor progression. Renal cell carcinoma is known to be a highly angiogenic cancer. Thus, treatment of renal cell carcinoma with kallikrein may effectively shutdown the active recruitment of a blood supply to a tumor. Members of this protein family are known to play a role in vascular coagulation. Similar to anti-angiogenic therapy, a factor produced by cancer cells that is pro-coagulatory may also act to inhibit cancer growth by effectively "clogging" the tumor vascular supply. In addition, through its proteolytic activity, kallikrein may degrade ECM proteins or growth factors necessary for the progressive growth of cancer cells. Following is a relevant reference underlining the importance of Kallikrein in cancer therapy.

### The New Human Kallikrein Gene Family: Implications in Carcinogenesis.

Diamandis EP; Yousef GM; Luo I; Magklara I; Obiezu CV Department of Pathology and Laboratory Medicine, Mount Sinai Hospital, Toronto, Ontario, Canada.
Trends Endocrinol Metab 2000 Mar;11(2):54-60.

ABSTRACT: The traditional human kallikrein gene family consists of three genes, namely KLK1 [encoding human kallikrein 1 (hK1) or pancreatic/renal kallikrein], KLK2 (encoding hK2, previously known as human glandular kallikrein 1) and KLK3 [encoding hK3 or prostate-specific antigen (PSA)]. KLK2 and KLK3 have important applications in prostate cancer diagnostics and, more recently, in breast cancer diagnostics. During the past two to three years, new putative members of the human kallikrein gene family have been identified, including the PRSSL1 gene [encoding normal epithelial cell-specific 1 gene (NES1)], the gene encoding zyme/protease M/neurosin, the gene encoding prostase/KLK-L1, and the genes encoding neuropsin, stratum corneum chymotryptic enzyme and trypsin-like serine protease. Another five putative kallikrein genes, provisionally named KLK-L2, KLK-L3, KLK-L4, KLK-L5 and KLK-L6, have also been identified. Many of the newly identified kallikrein-like genes are regulated by steroid hormones, and a few kallikreins (NES1, protease M, PSA) are known to be downregulated in breast and possibly other cancers. NES1 appears to be a novel breast cancer tumor suppressor protein and PSA a potent inhibitor of angiogenesis. This brief review summarizes recent developments and possible applications of the newly defined and expanded human kallikrein gene locus.

### The utility of kallikrein-like/coagulation factor XI-like family members in protein therapy of liver cirrosis

Results related to inflammation shown below in Example A, Table CC3, panel 4, indicate over-expression of 27455183.0.19 in the liver cirrhosis sample, as compared to panel 1 data (Table CC1), where there is little or no expression in normal adult liver. Panel 4 was generated from various human cell lines that were untreated or resting as well as the same cells that were treated with a wide variety of immune modulatory molecules. There are several disease tissues represented as well as organ controls.

### Potential Role(s) of FCTR6 in Inflammation:

Liver cirrhosis occurs in patients with hepatitis C and also in alcoholics. This protein is 41% related to coagulation factor XI and its potential role in liver cirrhosis may be related to cleavage of kininogen. A reference for this follows:
Thromb Haemost 2000 May;83(5):709-14 High molecular weight kininogen is cleaved by FXIa at three sites: Arg409-Arg410, Lys502-Thr503 and Lys325-Lys326. Mauron T, Lammle B, Wuillemin WA Central Hematology Laboratory, University of Bern, Inselspital, Switzerland.

### Abstract:

We investigated the cleavage of high molecular weight kininogen (HK) by activated coagulation factor XI (FXIa) in vitro. Incubation of HK with FXIa resulted in the generation of cleavage products which were subjected to SDS-Page and analyzed by silverstaining, ligand-blotting and immunoblotting, respectively. Upon incubation with FXIa, bands were generated at 111, 100, 88 kDa on nonreduced and at 76, 62 and 51 kDa on reduced gels. Amino acid sequence analysis of the reaction mixtures revealed three cleavage sites at Arg409-Arg410, at Lys502-Thr503 and at Lys325-Lys326. Analysis of HK-samples incubated with FXIa for 3 min, 10 min and 120 min indicated HK to be cleaved first at Arg409-Arg410, followed by cleavage at Lys502-Thr503 and then at Lys325-Lys326. In conclusion, HK is cleaved by FXIa at three sites. Cleavage of HK by FXIa results in the loss of the surface binding site of HK, which may constitute a mechanism of inactivation of HK and of control of contact system activation.

### Impact of Therapeutic Targeting of FCTR6 in Inflammation:

Therapeutic targeting of FCTR6 with a monoclonal antibody is anticipated to limit or block the extent of breakdown of kininogen and thereby reduce the degradation of liver that occurs in liver cirrhosis. A pertinent reference is:
Thromb Haemost 1999 Nov;82(5):1428-32 Parallel reduction of plasma levels of high and low molecular weight kininogen in patients with cirrhosis.
Cugno M, Scott CF, Salerno F, Lorenzano E, Muller-Esterl W, Agostoni A, Colman RW Department of Internal Medicine, IRCCS Maggiore Hospital, University of Milan, Italy. massimo.cugno@unimi.it

### Abstract:

Little is known about the regulation of high-molecular-weight-kininogen (HK) and low-molecular-weight-kininogen (LK) or the relationship of each to the degree of liver function impairment in patients with cirrhosis. In this study, we evaluated HK and LK quantitatively by a recently described particle concentration fluorescence immunoassay (PCFIA) and qualitatively by SDS PAGE and immunoblotting analyses in plasma from 33 patients with cirrhosis presenting various degrees of impairment of liver function. Thirty-three healthy subjects served as normal controls. Patients with cirrhosis had significantly lower plasma levels ofHK (median 49 microg/ml [range 22-99 microg/ml]) and LK (58 microg/ml [15-100 microg/ml]) than normal subjects (HK 83 microg/ml [65-115 microg/ml]; LK 80 microg/ml [45-120 microg/ml]) (p<0.0001). The plasma concentrations of HK and LK were directly related to plasma levels of cholinesterase (P<0.0001) and albumin (P<0.0001 and P<0.001) and inversely to the Child-Pugh score (P<0.0001) and to prothrombin time ratio (P<0.0001) (reflecting the clinical and laboratory abnormalities in liver disease). Similar to normal individuals, in patients with cirrhosis, plasma HK and LK levels paralleled one another, suggesting that a coordinate regulation of those proteins persists in liver disease. SDS PAGE and immunoblotting analyses of kininogens in cirrhotic plasma showed a pattern similar to that observed in normal controls for LK (a single band at 66 kDa) with some lower molecular weight forms noted in cirrhotic plasma. A slight increase of cleavage of HK (a major band at 130 kDa and a faint but increased band at 107 kDa) was evident. The increased cleavage ofHK was confirmed by the lower cleaved kininogen index (CKI), as compared to normal controls. These data suggest a defect in hepatic synthesis as well as increased destructive cleavage of both kininogens in plasma from patients with cirrhosis. The decrease of important regulatory proteins like kininogens may contribute to the imbalance in coagulation and fibrinolytic systems, which frequently occurs in cirrhotic patients.

In summary, the differential expression of FCTR6 (Kallikrein family) in renal cell carcinoma is an important finding that could have immense potential in renal carcinogenesis. In additon, overexpression of the above gene in liver cirrhosis demonstrates its anticipated use as an immunotherapeutic target.

### FCTR7

The novel nucleic acid of 1498 nucleotides FCTR7 (also designated. 32592466.0.64) encoding a novel trypsin inhibitor-like protein is shown in Table 7A. An ORF begins with an ATG initiation codon at nucleotides 470-472 and ends with a TAA codon at nucleotides 1369-1371. Putative untranslated regions, if any, are found upstream from the initiation codon and downstream from the termination codon.

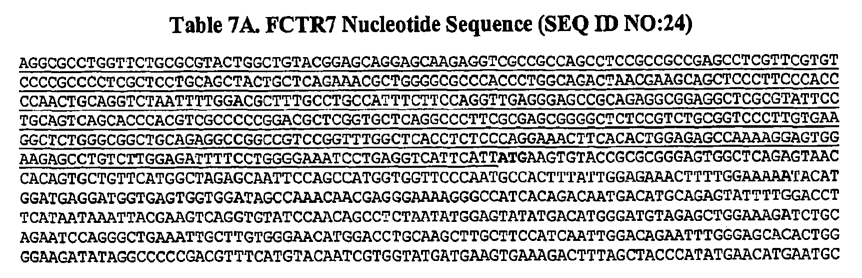

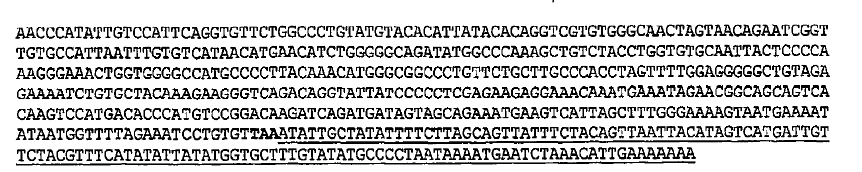

The FCTR7 protein encoded by SEQ ID NO:24 has 300 amino acid residues and is presented using the one-letter code in Table 7B. The FCTR7 gene was found to be expressed in: brain; germ cell tumors. FCTR7 gene maps to Unigene cluster Hs.182364 which is expressed in the following tissues: brain, breast, ear, germ cell, heart, liver, lung, whole embryo, ovary, pancreas, pooled, prostate, stomach, testis, uterus, vascular. Therefore the FCTR7 protein described in this invention is also expressed in the above tissues.

The SignalP, Psort and/or Hydropathy profile for FCTR7 predict that this sequence has a signal peptide and is likely to be localized outside of the cell with a certainty of 0.4228. The SignalP shows a cleavage site between amino acids 20 and 21, *i.e.,* at the dash in the sequence amino acid ARA-IP. The predicted molecular weight of FCTR7 is 34739.9 Daltons. Hydropathy profile shows an amino terminal hydrophobic region. This region could function as a signal peptide and target the invention to be secreted or plasma membrane localized.

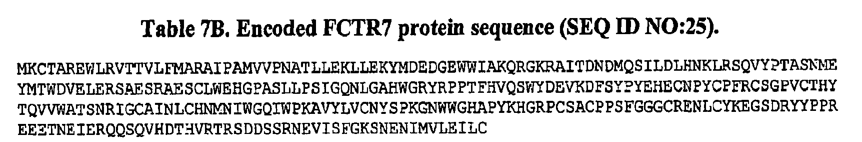

This gene maps to Unigene cluster Hs.182364 which has been assigned the following mapping information shown in table 7C. Therefore the chromosomal assignment for this gene is the same as that for Unigene cluster 182364.

**Table 7C. Mapping Information.**

| **Chromosome:** | **8** |
|---|---|
| **Gene Map 98:** | Marker SHGC-32056 , Interval D8S279-D8S526 |
| **Gene Map 98:** | Marker SGC32056, Interval D8S526-D8S275 |
| **Gene Map 98:** | Marker sts-G20223 , Interval D8S526-D8S275 |
| **Gene Map 98:** | Marker stSG30385 , Interval D8S526-D8S275 |
| **Whitehead map:** | EST67946, Chr.8 |
| **dbSTS entries:** | G25853, G29349, G20223 |

The predicted amino acid sequence was searched in the publicly available GenBank database
FCTR7 protein showed Score = 743 (261.5 bits), Expect =1.4e-73, P =1.4e-73, 54 % identities (129 over 237 amino acids) and 43% homologies (167 over 237 amino acids) with human 25 kD trypsin inhibitor protein (258 aa; ACC:O43692) (Table 7D).

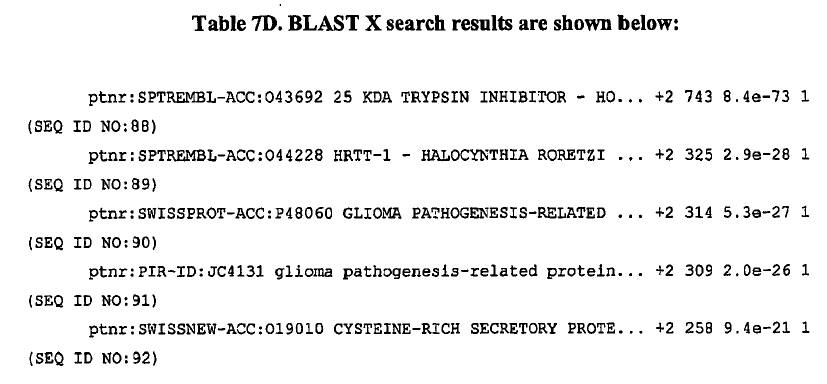

The nucleotide sequence of FCTR7 has 954 of 957 residues (99 %) identical to the 1-957 base segment, and 174 of 175 residues (99%) identical to bases 1317-1953 of the 2664 nucleotide *Homo sapiens* putative secretory protein precursor, mRNA (GenBank-ACC: AF142573) (SEQ ID NO:93) (Table 7E).

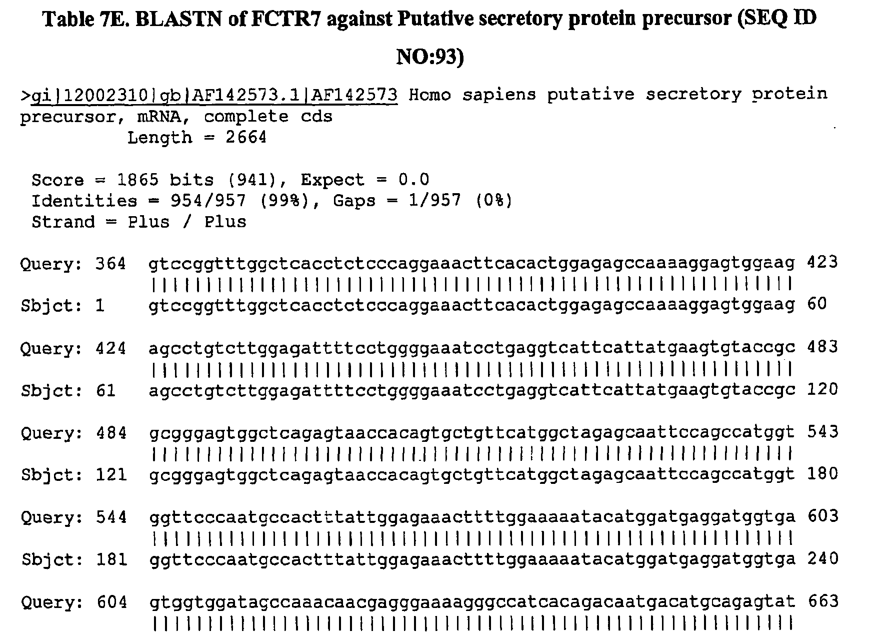

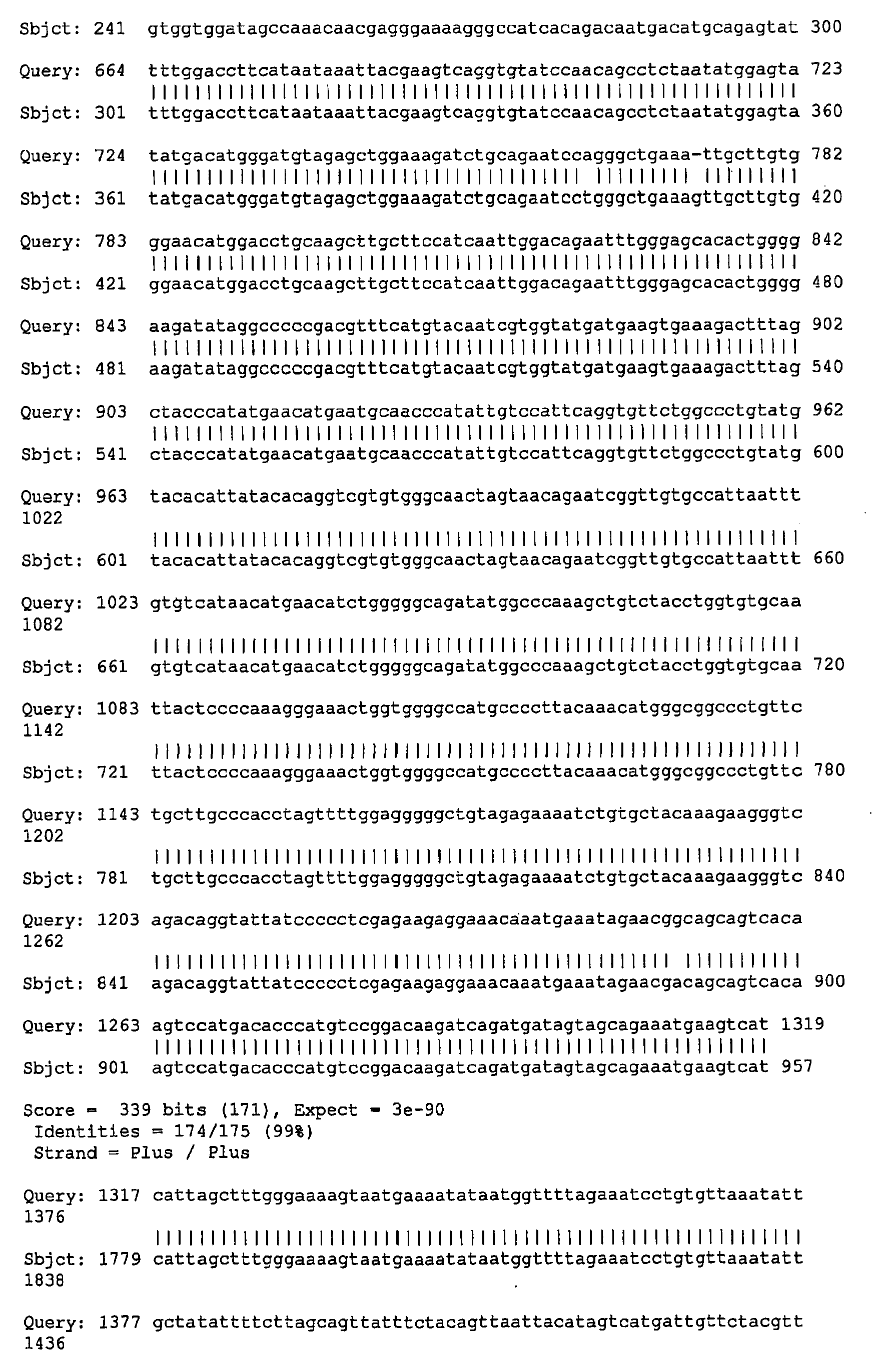

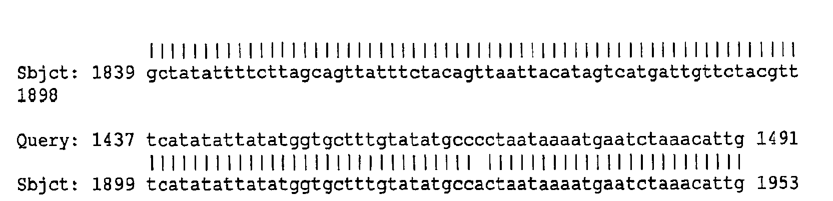

The FCTR7 amino acid has 284 of 285 amino acid residues (99%) identical to, and 284 of 285 ammo acid residues (99%) similar to, the 500 amino acid Putative secretory protein precursor [*Homo sapiens*] (GenBank-Acc No.: AF142573) (SEQ ID NO:94) (Table 7F).

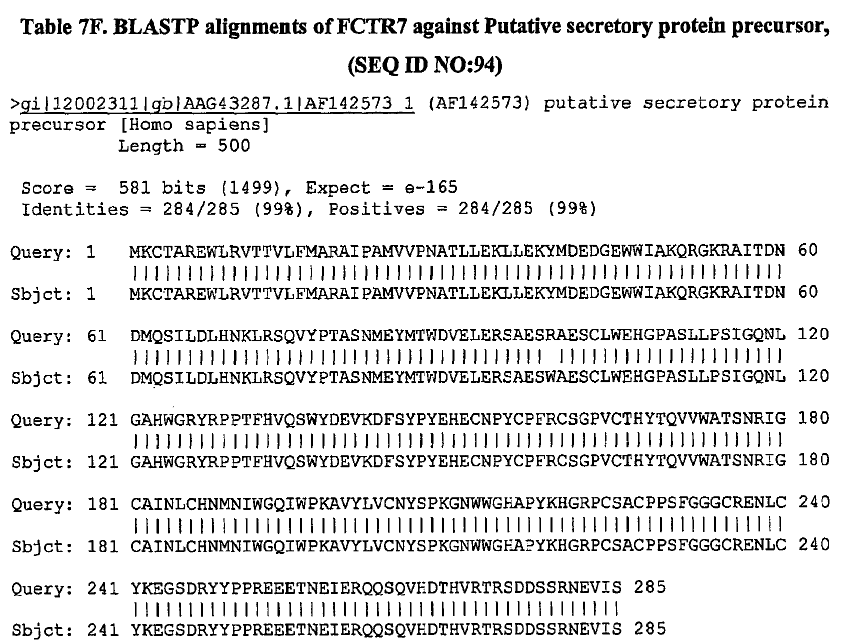

The FCTR7 amino acid has 137 of 176 amino acid residues (78%) identical to, and 151 of 176 amino acid residues (86%) similar to, the 188 amino acid Late gestation lung protein 1 [*Rattus norvegicus*] (GenBank-Acc No.: AF109674) (SEQ ID NO:95) (Table 7G).

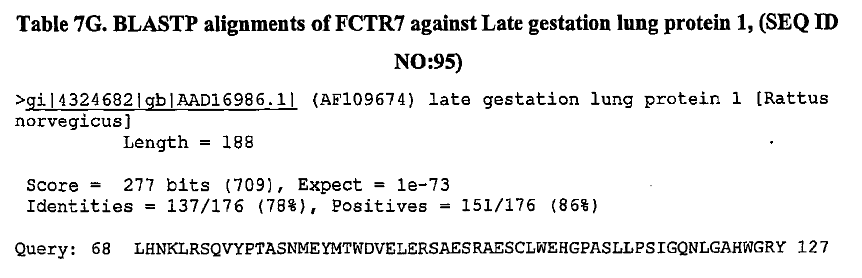

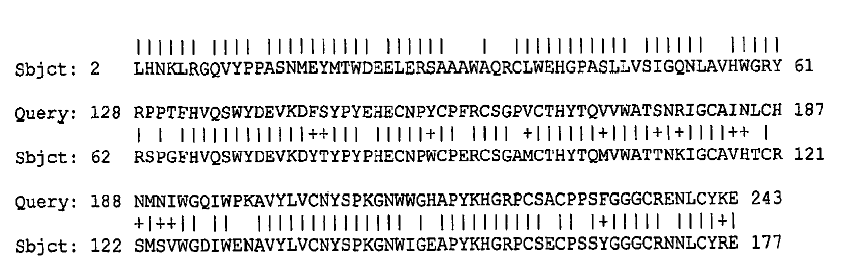

The FCTR7 amino acid has 130 of 237 amino acid residues (55%) identical to, and 165 of 237 amino acid residues (70%) similar to, the 258 amino acid R3H domain-containing preproprotein; 25 kDa trypsin inhibitor [*Homo sapiens*] (GenBank-Acc No.: D45027) (SEQ ID NO:96) (Table 7H).

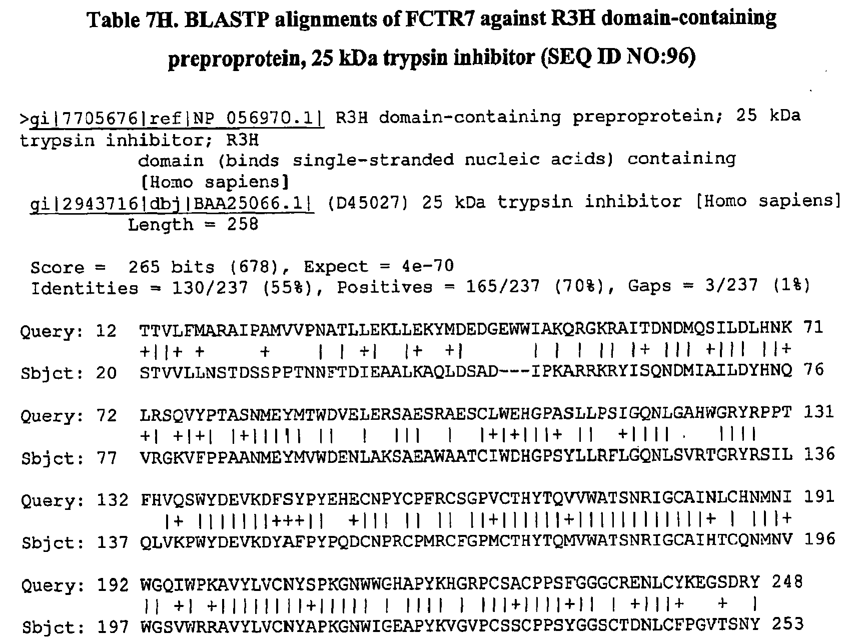

The FCTR7 amino acid has 109 of 233 amino acid residues (47%) identical to, and 146 of 233 amino acid residues (63%) similar to, the 253 amino acid Novel protein similar to a trypsin inhibitor [*Homo sapiens*] 25 kDa trypsin inhibitor (EMBLAcc No.: AL117382) (SEQ ID NO:97) (Table 7I).

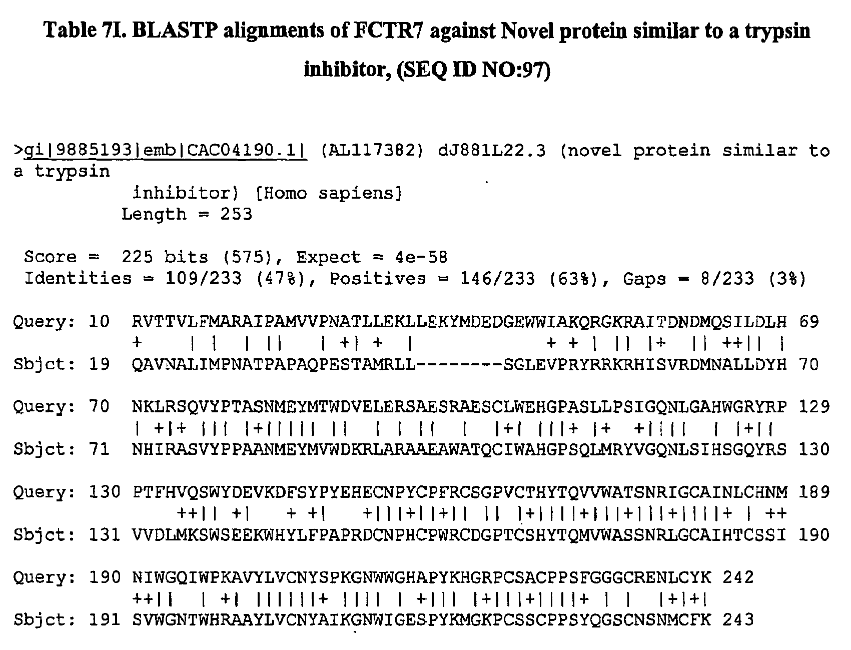

The FCTR7 amino acid has 129 of 237 amino acid residues (54%) identical to, and 167 of 237 amino acid residues (70%) similar to, the 258 amino acid 25 kDa Trypsin Inhibitor from *Homo sapiens* (EMBLAcc No.: 043692) (SEQ ID NO:88) (Table 7J).

**Table 7J. BLASTP alignments of FCTR7 against 25 kDa Trypsin Inhibitor, (SEQ ID NO:88)**

| |
|---|
| **ptnr:SPTREMBL-ACC:043692 25 KDA TRYPSIN INHIBITOR - Homo sapiens (Human), 258 aa.** |
| Score = 743 (261.5 bits), Expect = 1.6e-73, P = 1.6e-73 |
| Identities = 129/237 (54%), Positives = 167/237 (70%) |

The FCTR7 amino acid has 79 of 193 amino acid residues (40%) identical to, and 110 of 193 amino acid residues (56%) similar to, the 266 amino acid Glioma Pathogenesis-Related Protein (RTVP-1 Protein) - *Homo sapiens* (SWISSPROT Acc No.: P48060) (SEQ ID NO:90) (Table 7K).

**Table 7K. BLASTP alignments of FCTR7 against Glioma Pathogenesis-Related Protein, (SEQ ID NO:90)**

| |
|---|
| **ptnr:SWISSPROT-ACC:P48060 GLIOMA PATHOGENESIS-RELATED PROTEIN (RTVP-1 PROTEIN) - Homo sapiens (Human), 266 aa** |
| Score = 314 (110.5 bits), Expect = 4.7e-28, P = 4.7e-28 |
| Identities = 79/193 (40%), Positives = 110/193 (56%) |

The FCTR7 amino acid has 66 of 186 amino acid residues (35%) identical to, and 91 of 186 amino acid residues (48%) similar to, the 186 amino acid Neutrophil granules matrix glycoprotein SGP28 precursor from *Homo sapiens* (SWISSPROT Acc No.: S68691) (SEQ ID NO:98) (Table 7L).

**Table 7L. BLASTP alignments of FCTR7 against Neutrophil granules matrix glycoprotein, (SEQ ID NO:98)**

| |
|---|
| **ptnr:PIR-ID:S68691 neutrophil granules matrix glycoprotein SGP28 precursor - human** |
| Score = 254 (89.4 bits), Expect = 1.1e-21, P = 1.1e-21 |
| Identities = 66/186 (35%), Positives = 91/186 (48%) |

A novel developmentally regulated gene with homology to a tumor derived trypsin inhibitor is expressed in lung mesenchyme, as described in Am. J. Physiol. 0:0-0(1999). cDNA cloning of a novel trypsin inhibitor with similarity to pathogenesis-related proteins, and its frequent expression in human brain cancer cells is disclosed in Biochim. Biophys. Acta 1395:202-208(1998). RTVP-1, a novel human gene with sequence similarity to genes of diverse species, is expressed in tumor cell lines of glial but not neuronal origin, as published in Gene 180:125-130(1996). The human glioma pathogenesis-related protein is structurally related to plan pathogenesis-related proteins and its gene is expressed specifically in brain tumors (Gene 159:131-135(1995)). Structure comparison of human glioma pathogenesis-related protein GliPR and the plant pathogenesis-related protein P14a indicates a functional link between the human immune system and a plant defense system (Proc. Natl. Acad. Sci. U.S.A. 95:2262-2266(1998)). GliPR is highly expressed in the human brain tumor, glioblastoma multiform/astrocytome, but neither in normal fetal or adult brain tissue, nor in other nervous system tumors. GliPR belongs to a family that groups mammalian SCP/TPX1; insects AG3/AG5; FUNGI SC7/SC14 and plants PR-1. SGP28, a novel matrix glycoprotein in specific granules of human neutrophils with similarity to a human testis-specific gene product and to a rodent sperm-coating glycoprotein (FEBS Lett. 380, 246-250, 1996). The primary structure and properties of helothermine, a peptide toxin that blocks ryanodine receptors is described in Biophys. J. 68:2280-2288(1995). As GliPR, Helothermine belongs to a family that groups mammalian SCP/TPX1; insects AG3/AG5; FUNGI SC7/SC14 and plants PR-1.

Based upon homology, FCTR7 protein and each homologous protein or peptide may share at least some activity.

### Therapeutic uses:

FCTR7 protein has homology to trypsin inhibitors, Q91055 helothermine, tumor derived tyrpsin inhibitors, glioma pathogenesis-related protein, Q9Z0U6 LATE GESTATION LUNG PROTEIN 1, and to the Prosite family which groups mammalian SCP/TPX1;1NSECTS AG3/AG5; FUNGI SC7/SC14 AND PLANTS PR-1 proteins. Therefore the FCTR7 protein disclosed in this invention could function like the proteins which it has homology to. These functions include tissue development in *vitro* and *in vivo,* and cancer pathogenesis.

Based the tissue expression pattern, the gene is implicated in diseases of tissues in which it is expressed. These diseases include but are not limited to:
- Glioma,
- cancer,
- lung diseases,
- gestation,
- male and female reproductive diseases,
- deafness,
- neurological disorders,
- gastric disorders, and
- pancreatic diseases like diabetes.

These materials are further useful in the generation of antibodies that bind immunospecifically to the novel FCTR7 substances for use in therapeutic or diagnostic methods. These antibodies may be generated according to methods known in the art, using prediction from hydrophobicity charts, as described in the "Anti-FCTRX Antibodies" section below. In one embodiment, a contemplated FCTR7 epitope is from aa 40 to 120. In another embodiment, a FCTR7 epitope is from aa 130 to 170. In additional embodiments, FCTR7 epitopes are from aa 210 to 230, and from aa 240 to 280.

**TABLE 8A: Summary Of Nucleic Acids And Proteins Of The Invention**

| **Name** | **Tables** | **Clone; Description of Homolog** | **Nucleic Acid SEQ ID NO** | **Amino Acid SEQ ID NO** |
|---|---|---|---|---|
| FCTR1 | 1A, 1B, | 58092213.0.36 follistatin-like protein | 1 | 2 |
| FCTR2 | 2A, 2B | AC012614_1.0.123; KIAA1061-like protein | 3 | 4 |
| FCTR3 | 3A, 3B 3A, 3B | 10129612.0.118; neurestin-like protein | 5 | 6 |
| | 3C, 3D | 10129612.0.405; neurestin-like protein | 7 | 8 |
| | 3E | 10129612.0.154; neurestin-like protein | 9 | |
| | 3F | 10129612.0.67; neurestin-like protein | 10 | |
| | 3G | 10129612.0.258; neurestin-like protein | 11 | |
| | 3H,3I | 10129612.0.352; neurestin-like protein | 12 | 13 |
| FCTR4 | 4A, 4B | 29692275.0.1; NF-Kappa-B P65delta3-like protein | 14 | 15 |
| FCTR5 | 5A, 5B | 32125243.0.21; human complement C1R component precursor -like protein | 16 | 17 |
| | 5C, 5D | | 18 | 19 |
| FCTR6 | 6A, 6B | 27455183.0.19; novel human blood coagulation factor XI -like protein | 20 | 21 |
| | 6C, 6D | 27455183.0.145; novel human blood coagulation factor XI -like protein | 22 | 23 |
| FCTR7 7A, 7B | 7A, 7B | 32592466.0.64; trypsin inhibitor -like protein | 24 | 25 |
| FCTR1 | Example 2 | Ag809 Forward | 26 | |
| FCTR1 | Example 2 | Ag809 Probe | 27 | |
| FCTR1 | Example 2 | Ag809 Reverse | 28 | |
| FCTR4 | Example 2 | Ag2773 Forward | 29 | |
| FCTR4 | Example 2 | Ag2773 Probe | 30 | |
| FCTR4 | Example 2 | Ag2773 Reverse | 31 | |
| FCTR5 | Example 2 | Ag427 Forward | 32 | |
| FCTR5 | Example 2 | Ag427 Probe | 33 | |
| FCTR5 | Example 2 | Ag427 Reverse | 34 | |
| FCTR6 | Example 2 | Ag1541 Forward | 35 | |
| FCTR6 | Example 2 | Ag1541 Probe | 36 | |
| FCTR6 | Example 2 | Ag1541 Reverse | 37 | |

**TABLE 8B: Summary of Query Sequences Disclosed**

| **Table** | **Database** | **Acc. No.** | **Sequence Name** | **Species** | **SEQ ID NO.** |
|---|---|---|---|---|---|
| 1C, 1K | remtrEmbl | BAA21725 | IGFBP-like protein | mouse | 38 |
| 1D | sptrEmbl | Q61581 | Follistatin-like protein-2 | Mouse | 39 |
| 1E | SptrEmbl | Q07822 | Mac25 protein | Human | 40 |
| 1F, 1K | SptrEmbl | 088812 | Mac25 protein | Mouse | 41 |
| 1G, 1K | SptrEmbl | Q16270 | Prostacyclin-stimulating factor | Human | 42 |
| 1H, 1K | PIR | B40098 | Colorectal cancer suppressor | Rat | 43 |
| 1I | TrEmblne w | AAD9360 | PTP sigma (brain) precursor | Human | 44 |
| 1J | SptrEmbl | Q13332 | PTP sigma precursor | Human | 45 |
| 2C | GenBank | AB028984 | KIAA1061 cDNA | Human | 46 |
| 2D | TrEmblne w | BAA85677 | KIAA1263 | Human | 47 |
| 2E | TrEmblne w | BAA83013 | KIAA1061 protein fragment | Human | 48 |
| 2F | Embl | CAB70877.1 | Hypothetical protein DKFzp566D234.1 | Human | 49 |
| 2G | GenBank | Q62632 | Follistatin-related protein-1 precursor | Rat | 50 |
| 2H | GenBank | Q62536 | Follistatin-related protein-1 precursor | Mouse | 51 |
| 21 | GenBank | JG0187 | Follistatin related protein | African clawed frog | 52 |
| 2J | GenBank | Q12841 | Follistatin related protein-1 precursor | Human | 53 |
| 2K | Embl | CAB42968.1 | Flik protein | Chicken | 54 |
| 2L | GenBank | T13822 | Frazzled gene protein | Fruit fly | 55 |
| 2M | GenBank | AAC38849.1 | Roundabout 1 | Fruit fly | 56 |
| 2N | GenBank | 060469 | Down Syndrome Cell Adhesion Molecule Precursor | Human' | 57 |
| 20 | SwissProt | Q13449 | Limbic system-associated membrane protein precursor | Human | 58 |
| 2P | SptrEmbl | 070246 | Putative neuronal cell adhesion molecule, short form | Mouse | 59 |
| 2Q | SptrEmbl | 002869 | CHLAMP, G11-isoform precursor | Chicken | 60 |
| 2R | SwissProt | Q62813 | Limbic system-associated membrane protein precursor | Rat | 61 |
| 3J | GenBank | NM_011856.2 | Odd Oz/ten-m homology 2 | Fruit fly | 62 |
| 3K | Embl | AJ245711.1 | Teneurin-2 cDNA, short splice variant | Chicken | 63 |
| 3L | GenBank | AB032953 | KIAA 1127 cDNA | Human | 64 |
| 3M, 3U | GenBank | AB025411 | Ten-m2 cDNA | Mouse | 65 |
| 3N | GenBank | NM_020088.1 | Neurestin alpha cDNA | Rat | 66 |
| 30 | Embl | GGA278031 | Teneurin-2 | Chicken | 67 |
| 3P | GenBank | NP_035986.2 | Odd Oz/ten-m homology 2 | Fruit fly | 68 |
| 3Q | Embl | CAC09416.1 | Teneurin-2 | Chicken | 69 |
| 3R | GenBank | BAA77399.1 | Ten-m4 | Mouse | 70 |
| 3S | GenBank | AB032953 | KIAA1127 protein | Human | 71 |
| 3T | GenBank | AF086607 | Neurestin alpha | Rat | 72 |
| 4C | SptrEmbl | Q99233 | Hypothetical 10 kD protein | Trypanos ome | 73 |
| 4C | SptrEmbl | Q16896 | GABA receptor subunit | | 74 |
| 4C | SptrEmbl | 076473 | GABA receptor subunit | | 75 |
| 4C | TrEmblne w | AAD28317 | FI3J11.13 protein | | 76 |
| Text p. 90 | SptrEmb1 | Q13313 | NF-kappa B P65 delta 3 protein | Human | 77 |
| 5E | GenBank | XM_007061.1 | Complement C1R-like proteinase precursor | Human | 78 |
| 5F | GenBank | NM_001733.1 | Complement component 1, R subcomponent cDNA | Human | 79 |
| 5G | GenBank | AAF44349.1 | Complement C1R-like proteinase precursor | Human | 80 |
| 5H | GenBank | AAA5185.1 | Complement C1R component precursor | Human | 81 |
| 6E | GenBank | AB046651 | Brain cDNA clone Qcc-17034 | Macaque | 82 |
| 6F | GenBank | AK09660 | Adult testis cDNA, RIKEN full length enriched | Mouse | 83 |
| 6G | GenBank | AB046651 | Hypothetical protein | Macaque | 84 |
| 6H | GenBank | NP_000838.1 | Plasma kallikrein B1 precursor | Human | 85 |
| 6I | GenBank | BAA37147.1 | Kallikrein | Pig | 86 |
| 6J | Embl | CAA64368.1 | Coagulation factor XI | Human | 87 |
| 7D, 7J | SptrEmbl | 043692 | 25 kDa trypsin inhibitor | Human | 88 |
| 7D | SptrEmb1 | 044228 | HRTT-1 | | 89 |
| 7D, 7K | SptrEmbl | P418060 | Glioma pathogenesis-related protein | Human | 90 |
| 7D | PIR-ID | JC4131 | Glioma pathogenesis-related protein | Human | 91 |
| 7D | SwissProt | O19010 | Cysteine-rcih secretory protein | | 92 |
| 7E | GenBank | AF142573 | Putatitive secretory protein precursor cDNA | Human | 93 |
| 7F | GenBank | AF142573 | Putative secretory protein precursor | Human | 94 |
| 7G | GenBank | AF109674 | Late gestation lung protein 1 | Rat | 95 |
| 7H | GenBank | D45027 | R3H domain containing preprotein, 25 kDa trypsin inhibitor | Human | 96 |
| 7I | Embl | AL117382 | Novel protein similar to a trypsin inhibitor | Human | 97 |
| 7L | PIR-ID | S68691 | Neutrophil granules matrix glycoprotein SGP28 precursor | Human | 98 |

### FCTRX Nucleic Acids and Polypeptides

One aspect of the invention pertains to isolated nucleic acid molecules that encode FCTRX polypeptides or biologically-active portions thereof. Also included in the invention are nucleic acid fragments sufficient for use as hybridization probes to identify FCTRX-encoding nucleic acids (*e.g*., FCTRX mRNAs) and fragments for use as PCR primers for the amplification and/or mutation of FCTRX nucleic acid molecules. As used herein, the term "nucleic acid molecule" is intended to include DNA molecules (*e.g.*, cDNA or genomic DNA), RNA molecules (*e.g.,* mRNA), analogs of the DNA or RNA generated using nucleotide analogs, and derivatives, fragments and homologs thereof. The nucleic acid molecule may be single-stranded or double-stranded, but preferably is comprised double-stranded DNA.

An FCTRX nucleic acid can encode a mature FCTRX polypeptide. As used herein, a "mature" form of a polypeptide or protein disclosed in the present invention is the product of a naturally occurring polypeptide or precursor form or proprotein. The naturally occurring polypeptide, precursor or proprotein includes, by way of nonlimiting example, the full length gene product, encoded by the corresponding gene. Alternatively, it may be defined as the polypeptide, precursor or proprotein encoded by an ORF described herein. The product "mature" form arises, again by way of nonlimiting example, as a result of one or more naturally occurring processing steps as they may take place within the cell, or host cell, in which the gene product arises. Examples of such processing steps leading to a "mature" form of a polypeptide or protein include the cleavage of the N-terminal methionine residue encoded by the initiation codon of an ORF, or the proteolytic cleavage of a signal peptide or leader sequence. Thus a mature form arising from a precursor polypeptide or protein that has residues 1 to N, where residue 1 is the N-terminal methionine, would have residues 2 through N remaining after removal of the N-terminal methionine. Alternatively, a mature form arising from a precursor polypeptide or protein having residues 1 to N, in which an N-terminal signal sequence from residue 1 to residue M is cleaved, would have the residues from residue M+1 to residue N remaining. Further as used herein, a "mature" form of a polypeptide or protein may arise from a step of post-translational modification other than a proteolytic cleavage event. Such additional processes include, by way of non-limiting example, glycosylation, myristoylation or phosphorylation. In general, a mature polypeptide or protein may result from the operation of only one of these processes, or a combination of any of them.

The term "probes", as utilized herein, refers to nucleic acid sequences of variable length, preferably between at least about 10 nucleotides (nt), 100 nt, or as many as approximately, *e.g.,* 6,000 nt, depending upon the specific use. Probes are used in the detection of identical, similar, or complementary nucleic acid sequences. Longer length probes are generally obtained from a natural or recombinant source, are highly specific, and much slower to hybridize than shorter-length oligomer probes. Probes may be single- or double-stranded and designed to have specificity in PCR, membrane-based hybridization technologies, or ELISA-like technologies.

The term "isolated" nucleic acid molecule, as utilized herein, is one which is separated from other nucleic acid molecules which are present in the natural source of the nucleic acid. Preferably, an "isolated" nucleic acid is free of sequences which naturally flank the nucleic acid (*i.e*., sequences located at the 5'- and 3'-termini of the nucleic acid) in the genomic DNA of the organism from which the nucleic acid is derived. For example, in various embodiments, the isolated FCTRX nucleic acid molecules can contain less than about 5 kb, 4 kb, 3 kb, 2 kb, 1 kb, 0.5 kb or 0.1 kb of nucleotide sequences which naturally flank the nucleic acid molecule in genomic DNA of the cell/tissue from which the nucleic acid is derived (*e.g*., brain, heart, liver, spleen, etc.). Moreover, an "isolated" nucleic acid molecule, such as a cDNA molecule, can be substantially free of other cellular material or culture medium when produced by recombinant techniques, or of chemical precursors or other chemicals when chemically synthesized.

A nucleic acid molecule of the invention, *e.g*., a nucleic acid molecule having the nucleotide sequence of SEQ ID NOS:1, 3, 5, 7, 9, 10, 11, 12, 14, 16, 18, 20, 22, and 24, or a complement of this aforementioned nucleotide sequence, can be isolated using standard molecular biology techniques and the sequence information provided herein. Using all or a portion of the nucleic acid sequence of SEQ ID NOS:1, 3, 5, 7, 9, 10, 11, 12, 14, 16, 18, 20, 22, and 24 as a hybridization probe, FCTRX molecules can be isolated using standard hybridization and cloning techniques (*e.g*., as described in Sambrook, et al., (eds.), MOLECULAR CLONING: A LABORATORY MANUAL 2nd Ed., Cold Spring Harbor Laboratory Press, Cold Spring Harbor, NY, 1989; and Ausubel, et al., (eds.), CURRENT PROTOCOLS IN MOLECULAR BIOLOGY, John Wiley & Sons, New York, NY, 1993.)

A nucleic acid of the invention can be amplified using cDNA, mRNA or alternatively, genomic DNA, as a template and appropriate oligonucleotide primers according to standard PCR amplification techniques. The nucleic acid so amplified can be cloned into an appropriate vector and characterized by DNA sequence analysis. Furthermore, oligonucleotides corresponding to FCTRX nucleotide sequences can be prepared by standard synthetic techniques, *e.g*., using an automated DNA synthesizer.

As used herein, the term "oligonucleotide" refers to a series of linked nucleotide residues, which oligonucleotide has a sufficient number of nucleotide bases to be used in a PCR reaction. A short oligonucleotide sequence may be based on, or designed from, a genomic or cDNA sequence and is used to amplify, confirm, or reveal the presence of an identical, similar or complementary DNA or RNA in a particular cell or tissue. Oligonucleotides comprise portions of a nucleic acid sequence having about 10 nt, 50 nt, or 100 nt in length, preferably about 15 nt to 30 nt in length. In one embodiment of the invention, an oligonucleotide comprising a nucleic acid molecule less than 100 nt in length would further comprise at least 6 contiguous nucleotides of SEQ ID NOS:1, 3, 5, 7, 9,10,11, 12, 14, 16, 18, 20, 22, and 24, or a complement thereof. Oligonucleotides may be chemically synthesized and may also be used as probes.

In another embodiment, an isolated nucleic acid molecule of the invention comprises a nucleic acid molecule that is a complement of the nucleotide sequence shown in SEQ ID NOS:1, 3, 5, 7, 9, 10, 11, 12, 14, 16, 18, 20, 22, and 24, or a portion of this nucleotide sequence (*e.g*., a fragment that can be used as a probe or primer or a fragment encoding a biologically-active portion of an FCTRX polypeptide). A nucleic acid molecule that is complementary to the nucleotide sequence shown in SEQ ID NOS:1, 3, 5, 7, 9,10, 11, 12, 14, 16, 18, 20, 22, and 24, is one that is sufficiently complementary to the nucleotide sequence shown in SEQ ID NOS:1, 3, 5, 7, 9, 10, 11, 12, 14, 16, 18, 20, 22, and 24, that it can hydrogen bond with little or no mismatches to the nucleotide sequence shown in SEQ ID NOS:1, 3, 5, 7, 9, 10,11, 12,14, 16,18, 20, 22, and 24, thereby forming a stable duplex.

As used herein, the term "complementary" refers to Watson-Crick or Hoogsteen base pairing between nucleotides units of a nucleic acid molecule, and the term "binding" means the physical or chemical interaction between two polypeptides or compounds or associated polypeptides or compounds or combinations thereof. Binding includes ionic, non-ionic, van der Waals, hydrophobic interactions, and the like. A physical interaction can be either direct or indirect. Indirect interactions may be through or due to the effects of another polypeptide or compound. Direct binding refers to interactions that do not take place through, or due to, the effect of another polypeptide or compound, but instead are without other substantial chemical intermediates.

Fragments provided herein are defined as sequences of at least 6 (contiguous) nucleic acids or at least 4 (contiguous) amino acids, a length sufficient to allow for specific hybridization in the case of nucleic acids or for specific recognition of an epitope in the case of amino acids, respectively, and are at most some portion less than a full length sequence. Fragments may be derived from any contiguous portion of a nucleic acid or amino acid sequence of choice. Derivatives are nucleic acid sequences or amino acid sequences formed from the native compounds either directly or by modification or partial substitution. Analogs are nucleic acid sequences or amino acid sequences that have a structure similar to, but not identical to, the native compound but differs from it in respect to certain components or side chains. Analogs may be synthetic or from a different evolutionary origin and may have a similar or opposite metabolic activity compared to wild type. Homologs are nucleic acid sequences or amino acid sequences of a particular gene that are derived from different species.

Derivatives and analogs may be full length or other than full length, if the derivative or analog contains a modified nucleic acid or amino acid, as described below. Derivatives or analogs of the nucleic acids or proteins of the invention include, but are not limited to, molecules comprising regions that are substantially homologous to the nucleic acids or proteins of the invention, in various embodiments, by at least about 70%, 80%, or 95% identity (with a preferred identity of 80-95%) over a nucleic acid or amino acid sequence of identical size or when compared to an aligned sequence in which the alignment is done by a computer homology program known in the art, or whose encoding nucleic acid is capable of hybridizing to the complement of a sequence encoding the aforementioned proteins under stringent, moderately stringent, or low stringent conditions. See *e.g*. Ausubel, et al., CURRENT PROTOCOLS IN MOLECULAR BIOLOGY, John Wiley & Sons, New York, NY, 1993, and below.

A "homologous nucleic acid sequence" or "homologous amino acid sequence," or variations thereof, refer to sequences characterized by a homology at the nucleotide level or amino acid level as discussed above. Homologous nucleotide sequences encode those sequences coding for isoforms of FCTRX polypeptides. Isoforms can be expressed in different tissues of the same organism as a result of, for example, alternative splicing of RNA. Alternatively, isoforms can be encoded by different genes. In the invention, homologous nucleotide sequences include nucleotide sequences encoding for an FCTRX polypeptide of species other than humans, including, but not limited to: vertebrates, and thus can include, *e.g*., frog, mouse, rat, rabbit, dog, cat cow, horse, and other organisms. Homologous nucleotide sequences also include, but are not limited to, naturally occurring allelic variations and mutations of the nucleotide sequences set forth herein. A homologous nucleotide sequence does not, however, include the exact nucleotide sequence encoding human FCTRX protein. Homologous nucleic acid sequences include those nucleic acid sequences that encode conservative amino acid substitutions (see below) in SEQ ID NOS:2, 4, 6, 8, 13, 15, 17, 19, 21, 23, and 25, as well as a polypeptide possessing FCTRX biological activity. Various biological activities of the FCTRX proteins are described below.

An FCTRX polypeptide is encoded by the open reading frame ("ORF") of an FCTRX nucleic acid. An ORF corresponds to a nucleotide sequence that could potentially be translated into a polypeptide. A stretch of nucleic acids comprising an ORF is uninterrupted by a stop codon. An ORF that represents the coding sequence for a full protein begins with an ATG "start" codon and terminates with one of the three "stop" codons, namely, TAA, TAG, or TGA. For the purposes of this invention, an ORF may be any part of a coding sequence, with or without a start codon, a stop codon, or both. For an ORF to be considered as a good candidate for coding for a *bona fide* cellular protein, a minimum size requirement is often set, *e.g*., a stretch of DNA that would encode a protein of 50 amino acids or more.

The nucleotide sequences determined from the cloning of the human FCTRX genes allows for the generation of probes and primers designed for use in identifying and/or cloning FCTRX homologues in other cell types, *e.g.* from other tissues, as well as FCTRX homologues from other vertebrates. The probe/primer typically comprises substantially purified oligonucleotide. The oligonucleotide typically comprises a region of nucleotide sequence that hybridizes under stringent conditions to at least about 12, 25, 50, 100, 150, 200, 250, 300, 350 or 400 consecutive sense strand nucleotide sequence of SEQ ID NOS:1, 3, 5, 7, 9, 10, 11, 12, 14, 16, 18, 20, 22, and 24; or an anti-sense strand nucleotide sequence of SEQ ID NOS:1, 3, 5, 7, 9, 10, 11, 12, 14, 16, 18, 20, 22, and 24; or of a naturally occurring mutant of SEQ ID NOS:1, 3, 5, 7, 9, 10, 11, 12, 14, 16, 18, 20, 22, and 24.

Probes based on the human FCTRX nucleotide sequences can be used to detect transcripts or genomic sequences encoding the same or homologous proteins. In various embodiments, the probe further comprises a label group attached thereto, *e.g*. the label group can be a radioisotope, a fluorescent compound, an enzyme, or an enzyme co-factor. Such probes can be used as a part of a diagnostic test kit for identifying cells or tissues which mis-express an FCTRX protein, such as by measuring a level of an FCTRX-encoding nucleic acid in a sample of cells from a subject *e.g*., detecting FCTRX mRNA levels or determining whether a genomic FCTRX gene has been mutated or deleted.

"A polypeptide having a biologically-active portion of an FCTRX polypeptide" refers to polypeptides exhibiting activity similar, but not necessarily identical to, an activity of a polypeptide of the invention, including mature forms, as measured in a particular biological assay, with or without dose dependency. A nucleic acid fragment encoding a "biologically-active portion of FCTRX" can be prepared by isolating a portion of SEQ ID NOS:1, 3, 5, 7, 9, 10, 11, 12, 14, 16, 18, 20, 22, and 24, that encodes a polypeptide having an FCTRX biological activity (the biological activities of the FCTRX proteins are described below), expressing the encoded portion of FCTRX protein (*e.g*., by recombinant expression *in vitro*) and assessing the activity of the encoded portion of FCTRX.

### FCTRX Nucleic Acid and Polypeptide Variants

The invention further encompasses nucleic acid molecules that differ from the nucleotide sequences shown in SEQ ID NOS:1, 3, 5, 7, 9, 10, 11, 12, 14, 16,18, 20, 22, and 24, due to degeneracy of the genetic code and thus encode the same FCTRX proteins as that encoded by the nucleotide sequences shown in SEQ ID NO NOS:1, 3, 5,7,9,10,11,12,14, 16, 18, 20, 22, and 24. In another embodiment, an isolated nucleic acid molecule of the invention has a nucleotide sequence encoding a protein having an amino acid sequence shown in SEQ ID NOS:2, 4, 6, 8, 13, 15, 17, 19, 21, 23, and 25.

In addition to the human FCTRX nucleotide sequences shown in SEQ ID NOS:1, 3, 5, 7, 9, 10, 11, 12, 14, 16, 18, 20, 22, and 24, it will be appreciated by those skilled in the art that DNA sequence polymorphisms that lead to changes in the amino acid sequences of the FCTRX polypeptides may exist within a population (*e.g*., the human population). Such genetic polymorphism in the FCTRX genes may exist among individuals within a population due to natural allelic variation. As used herein, the terms "gene" and "recombinant gene" refer to nucleic acid molecules comprising an open reading frame (ORF) encoding an FCTRX protein, preferably a vertebrate FCTRX protein. Such natural allelic variations can typically result in 1-5% variance in the nucleotide sequence of the FCTRX genes. Any and all such nucleotide variations and resulting amino acid polymorphisms in the FCTRX polypeptides, which are the result of natural allelic variation and that do not alter the functional activity of the FCTRX polypeptides, are intended to be within the scope of the invention.

Moreover, nucleic acid molecules encoding FCTRX proteins from other species, and thus that have a nucleotide sequence that differs from the human sequence of SEQ ID NOS:1, 3, 5,7, 9, 10, 11, 12, 14, 16,18, 20, 22, and 24, are intended to be within the scope of the invention. Nucleic acid molecules corresponding to natural allelic variants and homologues of the FCTRX cDNAs of the invention can be isolated based on their homology to the human FCTRX nucleic acids disclosed herein using the human cDNAs, or a portion thereof, as a hybridization probe according to standard hybridization techniques under stringent hybridization conditions.

Accordingly, in another embodiment, an isolated nucleic acid molecule of the invention is at least 6 nucleotides in length and hybridizes under stringent conditions to the nucleic acid molecule comprising the nucleotide sequence of SEQ ID NOS:1, 3, 5, 7, 9,10, 11, 12, 14, 16, 18, 20, 22, and 24. In another embodiment, the nucleic acid is at least 10, 25, 50, 100, 250, 500, 750, 1000, 1500, or 2000 or more nucleotides in length. In yet another embodiment, an isolated nucleic acid molecule of the invention hybridizes to the coding region. As used herein, the term "hybridizes under stringent conditions" is intended to describe conditions for hybridization and washing under which nucleotide sequences at least 60% homologous to each other typically remain hybridized to each other.

Homologs *(i.e.,* nucleic acids encoding FCTRX proteins derived from species other than human) or other related sequences (*e.g.*, paralogs) can be obtained by low, moderate or high stringency hybridization with all or a portion of the particular human sequence as a probe using methods well known in the art for nucleic acid hybridization and cloning.

As used herein, the phrase "stringent hybridization conditions" refers to conditions under which a probe, primer or oligonucleotide will hybridize to its target sequence, but to no other sequences. Stringent conditions are sequence-dependent and will be different in different circumstances. Longer sequences hybridize specifically at higher temperatures than shorter sequences. Generally, stringent conditions are selected to be about 5°C lower than the thermal melting point (Tm) for the specific sequence at a defined ionic strength and pH. The Tm is the temperature (under defined ionic strength, pH and nucleic acid concentration) at which 50% of the probes complementary to the target sequence hybridize to the target sequence at equilibrium. Since the target sequences are generally present at excess, at Tm, 50% of the probes are occupied at equilibrium. Typically, stringent conditions will be those in which the salt concentration is less than about 1.0 M sodium ion, typically about 0.01 to 1.0 M sodium ion (or other salts) at pH 7.0 to 8.3 and the temperature is at least about 30°C for short probes, primers or oligonucleotides (*e.g*., 10 nt to 50 nt) and at least about 60°C for longer probes, primers and oligonucleotides. Stringent conditions may also be achieved with the addition of destabilizing agents, such as formamide.

Stringent conditions are known to those skilled in the art and can be found in Ausubel, et al., (eds.), CURRENT PROTOCOLS IN MOLECULAR BIOLOGY, John Wiley & Sons, N.Y. (1989), 6.3.1-6.3.6. Preferably, the conditions are such that sequences at least about 65%, 70%, 75%, 85%, 90%, 95%, 98%, or 99% homologous to each other typically remain hybridized to each other. A non-limiting example of stringent hybridization conditions are hybridization in a high salt buffer comprising 6X SSC, 50 mM Tris-HCl (pH 7.5), 1 mM EDTA, 0.02% PVP, 0.02% Ficoll, 0.02% BSA, and 500 mg/ml denatured salmon sperm DNA at 65°C, followed by one or more washes in 0.2X SSC, 0.01% BSA at 50°C. An isolated nucleic acid molecule of the invention that hybridizes under stringent conditions to the sequences of SEQ ID NOS:1, 3, 5, 7, 9, 10, 11, 12, 14, 16, 18, 20, 22, and 24, corresponds to a naturally-occurring nucleic acid molecule. As used herein, a "naturally-occurring" nucleic acid molecule refers to an RNA or DNA molecule having a nucleotide sequence that occurs in nature (*e.g*., encodes a natural protein).

In a second embodiment, a nucleic acid sequence that is hybridizable to the nucleic acid molecule comprising the nucleotide sequence of SEQ ID NOS:1, 3, 5, 7, 9, 10, 11, 12, 14, 16, 18, 20, 22, and 24, or fragments, analogs or derivatives thereof, under conditions of moderate stringency is provided. A non-limiting example of moderate stringency hybridization conditions are hybridization in 6X SSC, 5X Denhardt's solution, 0.5% SDS and 100 mg/ml denatured salmon sperm DNA at 55°C, followed by one or more washes in 1X SSC, 0.1% SDS at 37°C. Other conditions of moderate stringency that may be used are well-known within the art. See, *e.g.,* Ausubel, et al. (eds.), 1993, CURRENT PROTOCOLS INMOLECULAR BIOLOGY, John Wiley & Sons, NY, and Kriegler, 1990; GENE TRANSFER AND EXPRESSION, A LABORATORY MANUAL, Stockton Press, NY.

In a third embodiment, a nucleic acid that is hybridizable to the nucleic acid molecule comprising the nucleotide sequences of SEQ ID NOS: 1, 3, 5, 7, 9, 10, 11, 12, 14, 16, 18, 20, 22, and 24, or fragments, analogs or derivatives thereof, under conditions of low stringency, is provided. A non-limiting example of low stringency hybridization conditions are hybridization in 35% formamide, 5X SSC, 50 mM Tris-HCl (pH 7.5), 5 mM EDTA, 0.02% PVP, 0.02% Ficoll, 0.2% BSA, 100 mg/ml denatured salmon sperm DNA, 10% (wt/vol) dextran sulfate at 40°C, followed by one or more washes in 2X SSC, 25 mM Tris-HCl (pH 7.4), 5 mM EDTA, and 0.1% SDS at 50°C. Other conditions of low stringency that may be used are well known in the art (*e.g*., as employed for cross-species hybridizations). See, *e.g.,* Ausubel, et al. (eds.), 1993, CURRENT PROTOCOLS IN MOLECULAR BIOLOGY, John Wiley & Sons, NY, and Kriegler, 1990, GENE TRANSFER AND EXPRESSION, A LABORATORY MANUAL, Stockton Press, NY; Shilo and Weinberg, 1981. Proc Natl Acad Sci USA 78: 6789-6792.

### Conservative Mutations

In addition to naturally-occurring allelic variants of FCTRX sequences that may exist in the population, the skilled artisan will further appreciate that changes can be introduced by mutation into the nucleotide sequences of SEQ ID NO NOS:1, 3, 5, 7, 9, 10, 11, 12, 14, 16, 18, 20, 22, and 24, thereby leading to changes in the amino acid sequences of the encoded FCTRX proteins, without altering the functional ability of said FCTRX proteins. For example, nucleotide substitutions leading to amino acid substitutions at "non-essential" amino acid residues can be made in the sequence of SEQ ID NOS:2, 4, 6, 8, 13, 15, 17, 19, 21, 23, and 25. A "non-essential" amino acid residue is a residue that can be altered from the wild-type sequences of the FCTRX proteins without altering their biological activity, whereas an "essential" amino acid residue is required for such biological activity. For example, amino acid residues that are conserved among the FCTRX proteins of the invention are predicted to be particularly non-amenable to alteration. Amino acids for which conservative substitutions can be made are well-known within the art.

Another aspect of the invention pertains to nucleic acid molecules encoding FCTRX proteins that contain changes in amino acid residues that are not essential for activity. Such FCTRX proteins differ in amino acid sequence from SEQ ID NOS:2, 4, 6, 8, 13, 15, 17, 19, 21, 23, and 25, yet retain biological activity. In one embodiment, the isolated nucleic acid molecule comprises a nucleotide sequence encoding a protein, wherein the protein comprises an amino acid sequence at least about 45% homologous to the amino acid sequences of SEQ ID NOS:2, 4, 6, 8, 13, 15, 17, 19, 21, 23, and 25. Preferably, the protein encoded by the nucleic acid molecule is at least about 60% homologous to SEQ ID NOS:2, 4, 6, 8, 13, 15, 17, 19, 21, 23, and 25; more preferably at least about 70% homologous to SEQ ID NOS:2, 4, 6, 8, 13, 15, 17, 19, 21, 23, and 25; still more preferably at least about 80% homologous to SEQ ID NOS:2, 4, 6, 8, 13, 15, 17, 19, 21, 23, and 25; even more preferably at least about 90% homologous to SEQ ID NOS:2, 4, 6, 8, 13, 15, 17, 19, 21, 23, and 25; and most preferably at least about 95% homologous to SEQ ID NOS:2, 4, 6, 8,13,15, 17, 19, 21, 23, and 25.

An isolated nucleic acid molecule encoding an FCTRX protein homologous to the protein of SEQ ID NOS:2, 4, 6, 8,13, 15,17, 19, 21, 23, and 25, can be created by introducing one or more nucleotide substitutions, additions or deletions into the nucleotide sequence of SEQ ID NOS: 1, 3, 5, 7, 9, 10, 11, 12, 14, 16, 18, 20, 22, and 24, such that one or more amino acid substitutions, additions or deletions are introduced into the encoded protein.

Mutations can be introduced into SEQ ID NOS:2, 4, 6, 8,13,15,17,19, 21, 23, and 25, by standard techniques, such as site-directed mutagenesis and PCR-mediated mutagenesis. Preferably, conservative amino acid substitutions are made at one or more predicted, non-essential amino acid residues. A "conservative amino acid substitution" is one in which the amino acid residue is replaced with an amino acid residue having a similar side chain. Families of amino acid residues having similar side chains have been defined within the art. These families include amino acids with basic side chains (*e.g.,* lysine, arginine, histidine), acidic side chains (*e.g.,* aspartic acid, glutamic acid), uncharged polar side chains (*e.g.,* glycine, asparagine, glutamine, serine, threonine, tyrosine, cysteine), nonpolar side chains (*e.g*., alanine, valine, leucine, isoleucine, proline, phenylalanine, methionine, tryptophan), beta-branched side chains (*e.g.,* threonine, valine, isoleucine) and aromatic side chains (e.g., tyrosine, phenylalanine, tryptophan, histidine). Thus, a predicted non-essential amino acid residue in the FCTRX protein is replaced with another amino acid residue from the same side chain family. Alternatively, in another embodiment, mutations can be introduced randomly along all or part of an FCTRX coding sequence, such as by saturation mutagenesis, and the resultant mutants can be screened for FCTRX biological activity to identify mutants that retain activity. Following mutagenesis of SEQ ID NOS:2, 4, 6, 8,13,15,17,19,21,23, and 25, the encoded protein can be expressed by any recombinant technology known in the art and the activity of the protein can be determined.

The relatedness of amino acid families may also be determined based on side chain interactions. Substituted amino acids may be fully conserved "strong" residues or fully conserved "weak" residues. The "strong" group of conserved amino acid residues may be any one of the following groups: STA, NEQK, NHQK, NDEQ, QHRK, MILV, MILF, HY, FYW, wherein the single letter amino acid codes are grouped by those amino acids that may be substituted for each other. Likewise, the "weak" group of conserved residues may be any one of the following: CSA, ATV, SAG, STNK, STPA, SGND, SNDEQK, NDEQHK, NEQHRK, VLIM, HFY, wherein the letters within each group represent the single letter amino acid code.

In one embodiment, a mutant FCTRX protein can be assayed for (i) the ability to form protein:protein interactions with other FCTRX proteins, other cell-surface proteins, or biologically-active portions thereof, (*ii*) complex formation between a mutant FCTRX protein and an FCTRX ligand; or (*iii*) the ability of a mutant FCTRX protein to bind to an intracellular target protein or biologically-active portion thereof; (*e.g*. avidin proteins).

In yet another embodiment, a mutant FCTRX protein can be assayed for the ability to regulate a specific biological function (*e.g*., regulation of insulin release).

### Antisense Nucleic Acids

Another aspect of the invention pertains to isolated antisense nucleic acid molecules that are hybridizable to or complementary to the nucleic acid molecule comprising the nucleotide sequence of SEQ ID NOS:1, 3, 5, 7, 9, 10, 11, 12, 14, 16, 18, 20, 22, and 24, or fragments, analogs or derivatives thereof. An "antisense" nucleic acid comprises a nucleotide sequence that is complementary to a "sense" nucleic acid encoding a protein (*e.g.,* complementary to the coding strand of a double-stranded cDNA molecule or complementary to an mRNA sequence). In specific aspects, antisense nucleic acid molecules are provided that comprise a sequence complementary to at least about 10, 25, 50, 100, 250 or 500 nucleotides or an entire FCTRX coding strand, or to only a portion thereof. Nucleic acid molecules encoding fragments, homologs, derivatives and analogs of an FCTRX protein of SEQ ID NOS:2, 4, 6, 8, 13, 15, 17, 19, 21, 23, and 25; or antisense nucleic acids complementary to an FCTRX nucleic acid sequence of SEQ ID NOS:1, 3, 5, 7, 9, 10, 11, 12, 14, 16, 18, 20, 22, and 24, are additionally provided.

In one embodiment, an antisense nucleic acid molecule is antisense to a "coding region" of the coding strand of a nucleotide sequence encoding an FCTRX protein. The term "coding region" refers to the region of the nucleotide sequence comprising codons which are translated into amino acid residues. In another embodiment, the antisense nucleic acid molecule is antisense to a "noncoding region" of the coding strand of a nucleotide sequence encoding the FCTRX protein. The term "noncoding region" refers to 5' and 3' sequences which flank the coding region that are not translated into amino acids (*i.e.,* also referred to as 5' and 3' untranslated regions).

Given the coding strand sequences encoding the FCTRX protein disclosed herein, antisense nucleic acids of the invention can be designed according to the rules of Watson and Crick or Hoogsteen base pairing. The antisense nucleic acid molecule can be complementary to the entire coding region of FCTRX mRNA, but more preferably is an oligonucleotide that is antisense to only a portion of the coding or noncoding region of FCTRX mRNA. For example, the antisense oligonucleotide can be complementary to the region surrounding the translation start site of FCTRX mRNA. An antisense oligonucleotide can be, for example, about 5, 10, 15, 20, 25, 30, 35, 40, 45 or 50 nucleotides in length. An antisense nucleic acid of the invention can be constructed using chemical synthesis or enzymatic ligation reactions using procedures known in the art. For example, an antisense nucleic acid (*e.g*., an antisense oligonucleotide) can be chemically synthesized using naturally-occurring nucleotides or variously modified nucleotides designed to increase the biological stability of the molecules or to increase the physical stability of the duplex formed between the antisense and sense nucleic acids (*e.g*., phosphorothioate derivatives and acridine substituted nucleotides can be used).

Examples of modified nucleotides that can be used to generate the antisense nucleic acid include: 5-ffuorouracil, 5-bromouracil, 5-chlorouracil, 5-iodouracil, hypoxanthine, xanthine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine. Alternatively, the antisense nucleic acid can be produced biologically using an expression vector into which a nucleic acid has been subcloned in an antisense orientation (*i.e*., RNA transcribed from the inserted nucleic acid will be of an antisense orientation to a target nucleic acid of interest, described further in the following subsection).

The antisense nucleic acid molecules of the invention are typically administered to a subject or generated *in situ* such that they hybridize with or bind to cellular mRNA and/or genomic DNA encoding an FCTRX protein to thereby inhibit expression of the protein (*e.g.*, by inhibiting transcription and/or translation). The hybridization can be by conventional nucleotide complementarity to form a stable duplex, or, for example, in the case of an antisense nucleic acid molecule that binds to DNA duplexes, through specific interactions in the major groove of the double helix. An example of a route of administration of antisense nucleic acid molecules of the invention includes direct injection at a tissue site. Alternatively, antisense nucleic acid molecules can be modified to target selected cells and then administered systemically. For example, for systemic administration, antisense molecules can be modified such that they specifically bind to receptors or antigens expressed on a selected cell surface (*e.g.*, by linking the antisense nucleic acid molecules to peptides or antibodies that bind to cell surface receptors or antigens). The antisense nucleic acid molecules can also be delivered to cells using the vectors described herein. To achieve sufficient nucleic acid molecules, vector constructs in which the antisense nucleic acid molecule is placed under the control of a strong pol II or pol III promoter are preferred.

In yet another embodiment, the antisense nucleic acid molecule of the invention is an α-anomeric nucleic acid molecule. An α-anomeric nucleic acid molecule forms specific double-stranded hybrids with complementary RNA in which, contrary to the usual β-units, the strands run parallel to each other. See, *e.g*., Gaultier, et al., 1987. Nucl. Acids Res. 15: 6625-6641. The antisense nucleic acid molecule can also comprise a 2'-o-methylribonucleotide (see, *e.g.,* Inoue, et al. 1987. Nucl. Acids Res. 15: 6131-6148) or a chimeric RNA-DNA analogue (see, *e.g.,* Inoue, et al., 1987. FEBS Lett. 215: 327-330.

### Ribozymes and PNA Moieties

Nucleic acid modifications include, by way of non-limiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject.

In one embodiment, an antisense nucleic acid of the invention is a ribozyme. Ribozymes are catalytic RNA molecules with ribonuclease activity that are capable of cleaving a single-stranded nucleic acid, such as an mRNA, to which they have a complementary region. Thus, ribozymes (*e.g.,* hammerhead ribozymes as described in Haselhoff and Gerlach 1988. Nature 334: 585-591) can be used to catalytically cleave FCTRX mRNA transcripts to thereby inhibit translation of FCTRX mRNA. A ribozyme having specificity for an FCTRX-encoding nucleic acid can be designed based upon the nucleotide sequence of an FCTRX cDNA disclosed herein (*i.e*., SEQ ID NOS:1, 3, 5, 7, 9, 10, 11, 12, 14, 16, 18, 20, 22, and 24). For example, a derivative of a *Tetrahymena* L-19 IVS RNA can be constructed in which the nucleotide sequence of the active site is complementary to the nucleotide sequence to be cleaved in an FCTRX-encoding mRNA. See, *e.g*., U.S. Patent 4,987,071 to Cech, *et al.* and U.S. Patent 5,116,742 to Cech, *et al.* FCTRX mRNA can also be used to select a catalytic RNA having a specific ribonuclease activity from a pool of RNA molecules. See, *e.g*., Bartel et al., (1993) Science 261:1411-1418.

Alternatively, FCTRX gene expression can be inhibited by targeting nucleotide sequences complementary to the regulatory region of the FCTRX nucleic acid *(e.g.,* the FCTRX promoter and/or enhancers) to form triple helical structures that prevent transcription of the FCTRX gene in target cells. *See, e.g.,* Helene, 1991. Anticancer Drug Des. 6: 569-84; Helene, et al. 1992. Ann. N.Y. Acad. Sci. 660: 27-36; Maher, 1992. Bioassays 14: 807-15.

In various embodiments, the FCTRX nucleic acids can be modified at the base moiety, sugar moiety or phosphate backbone to improve, *e.g.*, the stability, hybridization, or solubility of the molecule. For example, the deoxyribose phosphate backbone of the nucleic acids can be modified to generate peptide nucleic acids. *See, e.g.,* Hyrup, et al., 1996. Bioorg Med Chem 4: 5-23. As used herein, the terms "peptide nucleic acids" or "PNAs" refer to nucleic acid mimics (*e.g.,* DNA mimics) in which the deoxyribose phosphate backbone is replaced by a pseudopeptide backbone and only the four natural nucleobases are retained. The neutral backbone of PNAs has been shown to allow for specific hybridization to DNA and RNA under conditions of low ionic strength. The synthesis of PNA oligomers can be performed using standard solid phase peptide synthesis protocols as described in Hyrup, *et al.,* 1996. *supra;* Perry-O'Keefe, et al., 1996. Proc. Natl. Acad. Sci. USA 93: 14670-14675.

PNAs of FCTRX can be used in therapeutic and diagnostic applications. For example, PNAs can be used as antisense or antigene agents for sequence-specific modulation of gene expression by, *e.g.,* inducing transcription or translation arrest or inhibiting replication. PNAs of FCTRX can also be used, for example, in the analysis of single base pair mutations in a gene (*e.g*., PNA directed PCR clamping; as artificial restriction enzymes when used in combination with other enzymes, *e.g.,* S₁ nucleases *(see,* Hyrup, *et al.,* 1996.*supra);* or as probes or primers for DNA sequence and hybridization *(see,* Hyrup, *et al.,* 1996, *supra*; Perry-O'Keefe, *et al.,* 1996. *supra*).

In another embodiment, PNAs of FCTRX can be modified, *e.g*., to enhance their stability or cellular uptake, by attaching lipophilic or other helper groups to PNA, by the formation of PNA-DNA chimeras, or by the use of liposomes or other techniques of drug delivery known in the art. For example, PNA-DNA chimeras of FCTRX can be generated that may combine the advantageous properties of PNA and DNA. Such chimeras allow DNA recognition enzymes (*e.g*., RNase H and DNA polymerases) to interact with the DNA portion while the PNA portion would provide high binding affinity and specificity. PNA-DNA chimeras can be linked using linkers of appropriate lengths selected in terms of base stacking, number of bonds between the nucleobases, and orientation (*see,* Hyrup, etal., 1996. *supra*). The synthesis of PNA-DNA chimeras can be performed as described in Hyrup*, et al.,* 1996. *supra* and Finn, et al., 1996. Nucl Acids Res 24: 3357-3363. For example, a DNA chain can be synthesized on a solid support using standard phosphoramidite coupling chemistry, and modified nucleoside analogs, *e.g*., 5'-(4-methoxytrityl)amino-5'-deoxy-thymidine phosphoramidite, can be used between the PNA and the 5' end of DNA. *See, e.g.,* Mag, et al., 1989. Nucl Acid Res 17: 5973-5988. PNA monomers are then coupled in a stepwise manner to produce a chimeric molecule with a 5' PNA segment and a 3' DNA segment. *See, e.g.,* Finn, *et al.,* 1996. *supra.* Alternatively, chimeric molecules can be synthesized with a 5'DNA segment and a 3' PNA segment. *See, e.g.,* Petersen, et al., 1975. Bioorg. Med. Chem. Lett. 5: 1119-11124.

In other embodiments, the oligonucleotide may include other appended groups such as peptides *(e.g.,* for targeting host cell receptors *in vivo*), or agents facilitating transport across the cell membrane *(see, e.g.,* Letsinger, et al., 1989. Proc. Natl. Acad. Sci. U.S.A. 86: 6553-6556; Lemaitre, et al., 1987. Proc. Natl. Acad. Sci. 84: 648-652; PCT Publication No. WO88/09810) or the blood-brain barrier *(see, e.g.,* PCT Publication No. WO 89/10134). In addition, oligonucleotides can be modified with hybridization triggered cleavage agents (*see, e.g.,* Krol, et al., 1988. BioTechniques 6:958-976) or intercalating agents *(see, e.g.,* Zon, 1988. Pharm. Res. 5: 539-549). To this end, the oligonucleotide may be conjugated to another molecule, *e.g*., a peptide, a hybridization triggered cross-linking agent, a transport agent, a hybridization-triggered cleavage agent, and the like.

### FCTRX Polypeptides

A polypeptide according to the invention includes a polypeptide including the amino acid sequence of FCTRX polypeptides whose sequences are provided in SEQ ID NOS:2, 4, 6, 8,13,15,17,19,21,23, and 25. The invention also includes a mutant or variant protein any of whose residues may be changed from the corresponding residues shown in SEQ ID NOS:2, 4, 6, 8, 13, 15, 17, 19, 21, 23, and 25, while still encoding a protein that maintains its FCTRX activities and physiological functions, or a functional fragment thereof.

In general, an FCTRX variant that preserves FCTRX-like function includes any variant in which residues at a particular position in the sequence have been substituted by other amino acids, and further include the possibility of inserting an additional residue or residues between two residues of the parent protein as well as the possibility of deleting one or more residues from the parent sequence. Any amino acid substitution, insertion, or deletion is encompassed by the invention. In favorable circumstances, the substitution is a conservative substitution as defined above.

One aspect of the invention pertains to isolated FCTRX proteins, and biologically-active portions thereof, or derivatives, fragments, analogs or homologs thereof. Also provided are polypeptide fragments suitable for use as immunogens to raise anti-FCTRX antibodies. In one embodiment, native FCTRX proteins can be isolated from cells or tissue sources by an appropriate purification scheme using standard protein purification techniques. In another embodiment, FCTRX proteins are produced by recombinant DNA techniques. Alternative to recombinant expression, an FCTRX protein or polypeptide can be synthesized chemically using standard peptide synthesis techniques.

An "isolated" or "purified" polypeptide or protein or biologically-active portion thereof is substantially free of cellular material or other contaminating proteins from the cell or tissue source from which the FCTRX protein is derived, or substantially free from chemical precursors or other chemicals when chemically synthesized. The language "substantially free of cellular material" includes preparations of FCTRX proteins in which the protein is separated from cellular components of the cells from which it is isolated or recombinantly-produced. In one embodiment, the language "substantially free of cellular material" includes preparations of FCTRX proteins having less than about 30% (by dry weight) of non-FCTRX proteins (also referred to herein as a "contaminating protein"), more preferably less than about 20% of non-FCTRX proteins, still more preferably less than about 10% of non-FCTRX proteins, and most preferably less than about 5% of non-FCTRX proteins. When the FCTRX protein or biologically-active portion thereof is recombinantly-produced, it is also preferably substantially free of culture medium, *i.e.,* culture medium represents less than about 20%, more preferably less than about 10%, and most preferably less than about 5% of the volume of the FCTRX protein preparation.

The language "substantially free of chemical precursors or other chemicals" includes preparations of FCTRX proteins in which the protein is separated from chemical precursors or other chemicals that are involved in the synthesis of the protein. In one embodiment, the language "substantially free of chemical precursors or other chemicals" includes preparations of FCTRX proteins having less than about 30% (by dry weight) of chemical precursors or non-FCTRX chemicals, more preferably less than about 20% chemical precursors or non-FCTRX chemicals, still more preferably less than about 10% chemical precursors or non-FCTRX chemicals, and most preferably less than about 5% chemical precursors or non-FCTRX chemicals.

Biologically-active portions of FCTRX proteins include peptides comprising amino acid sequences sufficiently homologous to or derived from the amino acid sequences of the FCTRX proteins (*e.g.,* the amino acid sequence shown in SEQ ID NOS:2, 4, 6, 8, 13, 15, 17, 19, 21, 23, and 25) that include fewer amino acids than the full-length FCTRX proteins, and exhibit at least one activity of an FCTRX protein. Typically, biologically-active portions comprise a domain or motif with at least one activity of the FCTRX protein. A biologically-active portion of an FCTRX protein can be a polypeptide which is, for example, 10, 25, 50, 100 or more amino acid residues in length.

Moreover, other biologically-active portions, in which other regions of the protein are deleted, can be prepared by recombinant techniques and evaluated for one or more of the functional activities of a native FCTRX protein.

In an embodiment, the FCTRX protein has an amino acid sequence shown in SEQ ID NOS:2, 4, 6, 8, 13, 15, 17, 19, 21, 23, and 25. In other embodiments, the FCTRX protein is substantially homologous to SEQ ID NOS:2, 4, 6, 8, 13, 15, 17, 19, 21, 23, and 25, and retains the functional activity of the protein of SEQ ID NOS:2, 4, 6, 8, 13, 15, 17, 19, 21, 23, and 25, yet differs in amino acid sequence due to natural allelic variation or mutagenesis, as described in detail, below. Accordingly, in another embodiment, the FCTRX protein is a protein that comprises an amino acid sequence at least about 45% homologous to the amino acid sequence of SEQ ID NOS:2, 4, 6, 8, 13,15, 17, 19, 21, 23, and 25, and retains the functional activity of the FCTRX proteins of SEQ ID NOS:2, 4, 6, 8, 13, 15, 17, 19, 21, 23, and 25.

### Determining Homology Between Two or More Sequences

To determine the percent homology of two amino acid sequences or of two nucleic acids, the sequences are aligned for optimal comparison purposes (*e.g.,* gaps can be introduced in the sequence of a first amino acid or nucleic acid sequence for optimal alignment with a second amino or nucleic acid sequence). The amino acid residues or nucleotides at corresponding amino acid positions or nucleotide positions are then compared. When a position in the first sequence is occupied by the same amino acid residue or nucleotide as the corresponding position in the second sequence, then the molecules are homologous at that position (*i.e.,* as used herein amino acid or nucleic acid "homology" is equivalent to amino acid or nucleic acid "identity").

The nucleic acid sequence homology may be determined as the degree of identity between two sequences. The homology may be determined using computer programs known in the art, such as GAP software provided in the GCG program package. *See,* Needleman and Wunsch, 1970. J Mol Biol 48: 443-453. Using GCG GAP software with the following settings for nucleic acid sequence comparison: GAP creation penalty of 5.0 and GAP extension penalty of 0.3, the coding region of the analogous nucleic acid sequences referred to above exhibits a degree of identity preferably of at least 70%, 75%, 80%, 85%, 90%, 95%, 98%, or 99%, with the CDS (encoding) part of the DNA sequence shown in SEQ ID NOS:1, 3, 5, 7, 9, 10, 11, 12, 14, 16, 18, 20, 22, and 24.

The term "sequence identity" refers to the degree to which two polynucleotide or polypeptide sequences are identical on a residue-by-residue basis over a particular region of comparison. The term "percentage of sequence identity" is calculated by comparing two optimally aligned sequences over that region of comparison, determining the number of positions at which the identical nucleic acid base (*e.g*., A, T, C, G, U, or I, in the case of nucleic acids) occurs in both sequences to yield the number of matched positions, dividing the number of matched positions by the total number of positions in the region of comparison (*i.e.,* the window size), and multiplying the result by 100 to yield the percentage of sequence identity. The term "substantial identity" as used herein denotes a characteristic of a polynucleotide sequence, wherein the polynucleotide comprises a sequence that has at least 80 percent sequence identity, preferably at least 85 percent identity and often 90 to 95 percent sequence identity, more usually at least 99 percent sequence identity as compared to a reference sequence over a comparison region.

### Chimeric and Fusion Proteins

The invention also provides FCTRX chimeric or fusion proteins. As used herein, an FCTRX "chimeric protein" or "fusion protein" comprises an FCTRX polypeptide operatively-linked to a non-FCTRX polypeptide. An "FCTRX polypeptide" refers to a polypeptide having an amino acid sequence corresponding to an FCTRX protein (SEQ ID NOS:2, 4, 6, 8, 13, 15, 17, 19, 21, 23, and 25), whereas a "non-FCTRX polypeptide" refers to a polypeptide having an amino acid sequence corresponding to a protein that is not substantially homologous to the FCTRX protein, *e.g.,* a protein that is different from the FCTRX protein and that is derived from the same or a different organism. Within an FCTRX fusion protein the FCTRX polypeptide can correspond to all or a portion of an FCTRX protein. In one embodiment, an FCTRX fusion protein comprises at least one biologically-active portion of an FCTRX protein. In another embodiment, an FCTRX fusion protein comprises at least two biologically-active portions of an FCTRX protein. In yet another embodiment, an FCTRX fusion protein comprises at least three biologically-active portions of an FCTRX protein. Within the fusion protein, the term "operatively-linked" is intended to indicate that the FCTRX polypeptide and the non-FCTRX polypeptide are fused in-frame with one another. The non-FCTRX polypeptide can be fused to the N-terminus or C-terminus of the FCTRX polypeptide.

In one embodiment, the fusion protein is a GST-FCTRX fusion protein in which the FCTRX sequences are fused to the C-terminus of the GST (glutathione S-transferase) sequences. Such fusion proteins can facilitate the purification of recombinant FCTRX polypeptides.

In another embodiment, the fusion protein is an FCTRX protein containing a heterologous signal sequence at its N-terminus. In certain host cells (*e.g.,* mammalian host cells), expression and/or secretion of FCTRX can be increased through use of a heterologous signal sequence.

In yet another embodiment, the fusion protein is an FCTRX-immunoglobulin fusion protein in which the FCTRX sequences are fused to sequences derived from a member of the immunoglobulin protein family. The FCTRX-immunoglobulin fusion proteins of the invention can be incorporated into pharmaceutical compositions and administered to a subject to inhibit an interaction between an FCTRX ligand and an FCTRX protein on the surface of a cell, to thereby suppress FCTRX-mediated signal transduction *in vivo.* The FCTRX-immunoglobulin fusion proteins can be used to affect the bioavailability of an FCTRX cognate ligand. Inhibition of the FCTRX ligand/FCTRX interaction may be useful therapeutically for both the treatment of proliferative and differentiative disorders, as well as modulating (*e.g.* promoting or inhibiting) cell survival. Moreover, the FCTRX-immunoglobulin fusion proteins of the invention can be used as immunogens to produce anti-FCTRX antibodies in a subject, to purify FCTRX ligands, and in screening assays to identify molecules that inhibit the interaction of FCTRX with an FCTRX ligand.

An FCTRX chimeric or fusion protein of the invention can be produced by standard recombinant DNA techniques. For example, DNA fragments coding for the different polypeptide sequences are ligated together in-frame in accordance with conventional techniques, *e.g.*, by employing blunt-ended or stagger-ended termini for ligation, restriction enzyme digestion to provide for appropriate termini, filling-in of cohesive ends as appropriate, alkaline phosphatase treatment to avoid undesirable joining, and enzymatic ligation. In another embodiment, the fusion gene can be synthesized by conventional techniques including automated DNA synthesizers. Alternatively, PCR amplification of gene fragments can be carried out using anchor primers that give rise to complementary overhangs between two consecutive gene fragments that can subsequently be annealed and reamplified to generate a chimeric gene sequence (*see, e.g.,* Ausubel, et al. (eds.) CURRENT PROTOCOLS IN MOLECULAR BIOLOGY, John Wiley & Sons, 1992). Moreover, many expression vectors are commercially available that already encode a fusion moiety (*e.g.,* a GST polypeptide). An FCTRX-encoding nucleic acid can be cloned into such an expression vector such that the fusion moiety is linked in-frame to the FCTRX protein.

### FCTRX Agonists and Antagonists

The invention also pertains to variants of the FCTRX proteins that function as either FCTRX agonists (*i.e.,* mimetics) or as FCTRX antagonists. Variants of the FCTRX protein can be generated by mutagenesis (*e.g.,* discrete point mutation or truncation of the FCTRX protein). An agonist of the FCTRX protein can retain substantially the same, or a subset of, the biological activities of the naturally occurring form of the FCTRX protein. An antagonist of the FCTRX protein can inhibit one or more of the activities of the naturally occurring form of the FCTRX protein by, for example, competitively binding to a downstream or upstream member of a cellular signaling cascade which includes the FCTRX protein. Thus, specific biological effects can be elicited by treatment with a variant of limited function. In one embodiment, treatment of a subject with a variant having a subset of the biological activities of the naturally occurring form of the protein has fewer side effects in a subject relative to treatment with the naturally occurring form of the FCTRX proteins.

Variants of the FCTRX proteins that function as either FCTRX agonists (*i.e*., mimetics) or as FCTRX antagonists can be identified by screening combinatorial libraries of mutants (*e.g*., truncation mutants) of the FCTRX proteins for FCTRX protein agonist or antagonist activity. In one embodiment, a variegated library of FCTRX variants is generated by combinatorial mutagenesis at the nucleic acid level and is encoded by a variegated gene library. A variegated library of FCTRX variants can be produced by, for example, enzymatically ligating a mixture of synthetic oligonucleotides into gene sequences such that a degenerate set of potential FCTRX sequences is expressible as individual polypeptides, or alternatively, as a set of larger fusion proteins (*e.g.,* for phage display) containing the set of FCTRX sequences therein. There are a variety of methods which can be used to produce libraries of potential FCTRX variants from a degenerate oligonucleotide sequence. Chemical synthesis of a degenerate gene sequence can be performed in an automatic DNA synthesizer, and the synthetic gene then ligated into an appropriate expression vector. Use of a degenerate set of genes allows for the provision, in one mixture, of all of the sequences encoding the desired set of potential FCTRX sequences. Methods for synthesizing degenerate oligonucleotides are well-known within the art. *See, e.g.,* Narang, 1983. Tetrahedron 39: 3; Itakura, et al., 1984. Annu. Rev. Biochem. 53: 323; Itakura, et al., 1984. Science 198: 1056; Ike, et al., 1983. Nucl. Acids Res. 11: 477.

### Polypeptide Libraries

In addition, libraries of fragments of the FCTRX protein coding sequences can be used to generate a variegated population of FCTRX fragments for screening and subsequent selection of variants of an FCTRX protein. In one embodiment, a library of coding sequence fragments can be generated by treating a double stranded PCR fragment of an FCTRX coding sequence with a nuclease under conditions wherein nicking occurs only about once per molecule, denaturing the double stranded DNA, renaturing the DNA to form double-stranded DNA that can include sense/antisense pairs from different nicked products, removing single stranded portions from reformed duplexes by treatment with S₁ nuclease, and ligating the resulting fragment library into an expression vector. By this method, expression libraries can be derived which encodes N-terminal and internal fragments of various sizes of the FCTRX proteins.

Various techniques are known in the art for screening gene products of combinatorial libraries made by point mutations or truncation, and for screening cDNA libraries for gene products having a selected property. Such techniques are adaptable for rapid screening of the gene libraries generated by the combinatorial mutagenesis of FCTRX proteins. The most widely used techniques, which are amenable to high throughput analysis, for screening large gene libraries typically include cloning the gene library into replicable expression vectors, transforming appropriate cells with the resulting library of vectors, and expressing the combinatorial genes under conditions in which detection of a desired activity facilitates isolation of the vector encoding the gene whose product was detected. Recursive ensemble mutagenesis (REM), a new technique that enhances the frequency of functional mutants in the libraries, can be used in combination with the screening assays to identify FCTRX variants. *See, e.g.,* Arkin and Yourvan, 1992. Proc. Natl. Acad. Sci. USA 89: 7811-7815; Delgrave, et al., 1993. Protein Engineering 6:327-331.

### Anti-FCTRX Antibodies

The invention encompasses antibodies and antibody fragments, such as F_{ab} or (F_{ab})₂. that bind immunospecifically to any of the FCTRX polypeptides of said invention.

An isolated FCTRX protein, or a portion or fragment thereof, can be used as an immunogen to generate antibodies that bind to FCTRX polypeptides using standard techniques for polyclonal and monoclonal antibody preparation. The full-length FCTRX proteins can be used or, alternatively, the invention provides antigenic peptide fragments of FCTRX proteins for use as immunogens. The antigenic FCTRX peptides comprises at least 4 amino acid residues of the amino acid sequence shown in SEQ ID NO NOS:2, 4, 6, 8, 13, 15, 17, 19, 21, 23, and 25, and encompasses an epitope of FCTRX such that an antibody raised against the peptide forms a specific immune complex with FCTRX. Preferably, the antigenic peptide comprises at least 6, 8, 10, 15, 20, or 30 amino acid residues. Longer antigenic peptides are sometimes preferable over shorter antigenic peptides, depending on use and according to methods well known to someone skilled in the art.

In certain embodiments of the invention, at least one epitope encompassed by the antigenic peptide is a region of FCTRX that is located on the surface of the protein (*e.g*., a hydrophilic region). As a means for targeting antibody production, hydropathy plots showing regions of hydrophilicity and hydrophobicity may be generated by any method well known in the art, including, for example, the Kyte Doolittle or the Hopp Woods methods, either with or without Fourier transformation (*see, e.g.,* Hopp and Woods, 1981. Proc. Nat. Acad. Sci. USA 78: 3824-3828; Kyte and Doolittle, 1982. J. Mol. Biol. 157: 105-142, each incorporated herein by reference in their entirety).

As disclosed herein, FCTRX protein sequences of SEQ ID NOS:2, 4, 6, 8, 13, 15, 17, 19, 21, 23, and 25, or derivatives, fragments, analogs or homologs thereof, may be utilized as immunogens in the generation of antibodies that immunospecifically-bind these protein components. The term "antibody" as used herein refers to immunoglobulin molecules and immunologically-active portions of immunoglobulin molecules, *i*.*e.*, molecules that contain an antigen binding site that specifically-binds (immunoreacts with) an antigen, such as FCTRX. Such antibodies include, but are not limited to, polyclonal, monoclonal, chimeric, single chain, F_{ab} and F_{(ab')2} fragments, and an F_{ab} expression library. In a specific embodiment, antibodies to human FCTRX proteins are disclosed. Various procedures known within the art may be used for the production of polyclonal or monoclonal antibodies to an FCTRX protein sequence of SEQ ID NOS:2, 4, 6, 8, 13,15,17, 19, 21, 23, and 25, or a derivative, fragment, analog or homolog thereof. Some of these proteins are discussed below.

For the production of polyclonal antibodies, various suitable host animals (*e.g.,* rabbit, goat, mouse or other mammal) may be immunized by injection with the native protein, or a synthetic variant thereof, or a derivative of the foregoing. An appropriate immunogenic preparation can contain, for example, recombinantly-expressed FCTRX protein or a chemically-synthesized FCTRX polypeptide. The preparation can further include an adjuvant. Various adjuvants used to increase the immunological response include, but are not limited to, Freund's (complete and incomplete), mineral gels (*e.g*., aluminum hydroxide), surface active substances (*e.g*., lysolecithin, pluronic polyols, polyanions, peptides, oil emulsions, dinitrophenol, etc.), human adjuvants such as *Bacille Calmette-Guerin* and *Corynebacterium parvum,* or similar immunostimulatory agents. If desired, the antibody molecules directed against FCTRX can be isolated from the mammal (*e.g.*, from the blood) and further purified by well known techniques, such as protein A chromatography to obtain the IgG fraction.

The term "monoclonal antibody" or "monoclonal antibody composition", as used herein, refers to a population of antibody molecules that contain only one species of an antigen binding site capable of immunoreacting with a particular epitope of FCTRX. A monoclonal antibody composition thus typically displays a single binding affinity for a particular FCTRX protein with which it immunoreacts. For preparation of monoclonal antibodies directed towards a particular FCTRX protein, or derivatives, fragments, analogs or homologs thereof, any technique that provides for the production of antibody molecules by continuous cell line culture may be utilized. Such techniques include, but are not limited to, the hybridoma technique *(see, e.g.,* Kohler & Milstein, 1975. Nature 256: 495-497); the trioma technique; the human B-cell hybridoma technique (*see, e.g.,* Kozbor, et al., 1983. Immunol. Today 4: 72) and the EBV hybridoma technique to produce human monoclonal antibodies (*see, e.g.,* Cole, et al., 1985. In: MONOCLONAL ANTIBODIES AND CANCER THERAPY, Alan R. Liss, Inc., pp. 77-96). Human monoclonal antibodies may be utilized in the practice of the invention and may be produced by using human hybridomas *(see, e.g.,* Cote, et al., 1983. Proc Natl Acad Sci USA 80: 2026-2030) or by transforming human B-cells with Epstein Barr Virus *in vitro* (*see, e.g.,* Cole, et al., 1985. In: MONOCLONAL ANTIBODIES AND CANCER THERAPY, Alan R. Liss, Inc., pp. 77-96). Each of the above citations is incorporated herein by reference in their entirety.

According to the invention, techniques can be adapted for the production of single-chain antibodies specific to an FCTRX protein (*see, e.g.,* U.S. Patent No. 4,946,778). In addition, methods can be adapted for the construction of F_{ab} expression libraries (*see, e.g.,* Huse, et al., 1989. Science 246: 1275-1281) to allow rapid and effective identification of monoclonal F_{ab} fragments with the desired specificity for an FCTRX protein or derivatives, fragments, analogs or homologs thereof. Non-human antibodies can be "humanized" by techniques well known in the art. *See, e.g.,* U.S. Patent No. 5,225,539. Antibody fragments that contain the idiotypes to an FCTRX protein may be produced by techniques known in the art including, but not limited to: (*i*) an F_{(ab')2} fragment produced by pepsin digestion of an antibody molecule; (*ii*) an F_{ab} fragment generated by reducing the disulfide bridges of an F_{(ab')2} fragment; (*iii*) an F_{ab} fragment generated by the treatment of the antibody molecule with papain and a reducing agent; and (*iv*) Fᵥ fragments.

Additionally, recombinant anti-FCTRX antibodies, such as chimeric and humanized monoclonal antibodies, comprising both human and non-human portions, which can be made using standard recombinant DNA techniques, are within the scope of the invention. Such chimeric and humanized monoclonal antibodies can be produced by recombinant DNA techniques known in the art, for example using methods described in International Application No. PCT/US86/02269; European Patent Application No. 184,187; European Patent Application No. 171,496; European Patent Application No. 173,494; PCT International Publication No. WO 86/01533; U.S. Patent No. 4,816,567; U.S. Pat. No. 5,225,539; European Patent Application No. 125,023; Better, et al., 1988. Science 240: 1041-1043; Liu, et al., 1987. Proc. Natl. Acad. Sci. USA 84: 3439-3443; Liu, et al., 1987. J. Immunol. 139: 3521-3526; Sun, et al., 1987. Proc. Natl. Acad. Sci. USA 84: 214-218; Nishimura, et al., 1987. Cancer Res. 47: 999-1005; Wood, et al., 1985. Nature 314 :446-449; Shaw, et al., 1988. J. Natl. Cancer Inst. 80: 1553-1559); Morrison(1985) Science 229:1202-1207; Oi, et al. (1986) BioTechniques 4:214; Jones, et al., 1986. Nature 321: 552-525; Verhoeyan, et al., 1988. Science 239: 1534; and Beidler, et al., 1988. J. Immunol. 141: 4053-4060. Each of the above citations are incorporated herein by reference in their entirety.

In one embodiment, methods for the screening of antibodies that possess the desired specificity include, but are not limited to, enzyme-linked immunosorbent assay (ELISA) and other immunologically-mediated techniques known within the art. In a specific embodiment, selection of antibodies that are specific to a particular domain of an FCTRX protein is facilitated by generation of hybridomas that bind to the fragment of an FCTRX protein possessing such a domain. Thus, antibodies that are specific for a desired domain within an FCTRX protein, or derivatives, fragments, analogs or homologs thereof, are also provided herein.

Anti-FCTRX antibodies may be used in methods known within the art relating to the localization and/or quantitation of an FCTRX protein (*e.g*., for use in measuring levels of the FCTRX protein within appropriate physiological samples, for use in diagnostic methods, for use in imaging the protein, and the like). In a given embodiment, antibodies for FCTRX proteins, or derivatives, fragments, analogs or homologs thereof, that contain the antibody derived binding domain, are utilized as pharmacologically-active compounds (hereinafter "Therapeutics").

An anti-FCTRX antibody (*e.g.,* monoclonal antibody) can be used to isolate an FCTRX polypeptide by standard techniques, such as affinity chromatography or immunoprecipitation. An anti-FCTRX antibody can facilitate the purification of natural FCTRX polypeptide from cells and of recombinantly-produced FCTRX polypeptide expressed in host cells. Moreover, an anti-FCTRX antibody can be used to detect FCTRX protein (*e.g.,* in a cellular lysate or cell supernatant) in order to evaluate the abundance and pattern of expression of the FCTRX protein. Anti-FCTRX antibodies can be used diagnostically to monitor protein levels in tissue as part of a clinical testing procedure, *e.g*., to, for example, determine the efficacy of a given treatment regimen. Detection can be facilitated by coupling (*i.e*., physically linking) the antibody to a detectable substance. Examples of detectable substances include various enzymes, prosthetic groups, fluorescent materials, luminescent materials, bioluminescent materials, and radioactive materials. Examples of suitable enzymes include horseradish peroxidase, alkaline phosphatase, β-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin, and examples of suitable radioactive material include ¹²⁵I, ¹³¹I, ³⁵S or ³H.

### FCTRX Recombinant Expression Vectors and Host Cells

Another aspect of the invention pertains to vectors, preferably expression vectors, containing a nucleic acid encoding an FCTRX protein, or derivatives, fragments, analogs or homologs thereof. As used herein, the term "vector" refers to a nucleic acid molecule capable of transporting another nucleic acid to which it has been linked. One type of vector is a "plasmid", which refers to a circular double stranded DNA loop into which additional DNA segments can be ligated. Another type of vector is a viral vector, wherein additional DNA segments can be ligated into the viral genome. Certain vectors are capable of autonomous replication in a host cell into which they are introduced (*e.g*., bacterial vectors having a bacterial origin of replication and episomal mammalian vectors). Other vectors (*e.g.,* non-episomal mammalian vectors) are integrated into the genome of a host cell upon introduction into the host cell, and thereby are replicated along with the host genome. Moreover, certain vectors are capable of directing the expression of genes to which they are operatively-linked. Such vectors are referred to herein as "expression vectors". In general, expression vectors of utility in recombinant DNA techniques are often in the form of plasmids. In the present specification, "plasmid" and "vector" can be used interchangeably as the plasmid is the most commonly used form of vector. However, the invention is intended to include such other forms of expression vectors, such as viral vectors (*e.g.,* replication defective retroviruses, adenoviruses and adeno-associated viruses), which serve equivalent functions.

The recombinant expression vectors of the invention comprise a nucleic acid of the invention in a form suitable for expression of the nucleic acid in a host cell, which means that the recombinant expression vectors include one or more regulatory sequences, selected on the basis of the host cells to be used for expression, that is operatively-linked to the nucleic acid sequence to be expressed. Within a recombinant expression vector, "operably-linked" is intended to mean that the nucleotide sequence of interest is linked to the regulatory sequence(s) in a manner that allows for expression of the nucleotide sequence (*e.g*., in an in *vitro* transcription/translation system or in a host cell when the vector is introduced into the host cell).

The term "regulatory sequence" is intended to includes promoters, enhancers and other expression control elements (*e.g.,* polyadenylation signals). Such regulatory sequences are described, for example, in Goeddel, GENE EXPRESSION TECHNOLOGY: METHODS IN ENZYMOLOGY 185, Academic Press, San Diego, Calif. (1990). Regulatory sequences include those that direct constitutive expression of a nucleotide sequence in many types of host cell and those that direct expression of the nucleotide sequence only in certain host cells (*e.g*., tissue-specific regulatory sequences). It will be appreciated by those skilled in the art that the design of the expression vector can depend on such factors as the choice of the host cell to be transformed, the level of expression of protein desired, etc. The expression vectors of the invention can be introduced into host cells to thereby produce proteins or peptides, including fusion proteins or peptides, encoded by nucleic acids as described herein (*e.g.,* FCTRX proteins, mutant forms of FCTRX proteins, fusion proteins, etc.).

The recombinant expression vectors of the invention can be designed for expression of FCTRX proteins in prokaryotic or eukaryotic cells. For example, FCTRX proteins can be expressed in bacterial cells such as *Escherichia coli,* insect cells (using baculovirus expression vectors) yeast cells or mammalian cells. Suitable host cells are discussed further in Goeddel, GENE EXPRESSION TECHNOLOGY: METHODS IN ENZYMOLOGY 185, Academic Press, San Diego, Calif. (1990). Alternatively, the recombinant expression vector can be transcribed and translated *in vitro*, for example using T7 promoter regulatory sequences and T7 polymerase.

Expression of proteins in prokaryotes is most often carried out in *Escherichia coli* with vectors containing constitutive or inducible promoters directing the expression of either fusion or non-fusion proteins. Fusion vectors add a number of amino acids to a protein encoded therein, usually to the amino terminus of the recombinant protein. Such fusion vectors typically serve three purposes: (*i*) to increase expression of recombinant protein; (*ii*) to increase the solubility of the recombinant protein; and (*iii*) to aid in the purification of the recombinant protein by acting as a ligand in affinity purification. Often, in fusion expression vectors, a proteolytic cleavage site is introduced at the junction of the fusion moiety and the recombinant protein to enable separation of the recombinant protein from the fusion moiety subsequent to purification of the fusion protein. Such enzymes, and their cognate recognition sequences, include Factor Xa, thrombin and enterokinase. Typical fusion expression vectors include pGEX (Pharmacia Biotech Inc; Smith and Johnson, 1988. Gene 67: 31-40), pMAL (New England Biolabs, Beverly, Mass.) and pRIT5 (Pharmacia, Piscataway, N.J.) that fuse glutathione S-transferase (GST), maltose E binding protein, or protein A, respectively, to the target recombinant protein.

Examples of suitable inducible non-fusion *E. coli* expression vectors include pTrc (Amrann et al., (1988) Gene 69:301-315) and pET 11d (Studier et al., GENE EXPRESSION TECHNOLOGY: METHODS IN ENZYMOLOGY 185, Academic Press, San Diego, Calif. (1990) 60-89).

One strategy to maximize recombinant protein expression in *E. coli* is to express the protein in a host bacteria with an impaired capacity to proteolytically cleave the recombinant protein. *See, e.g.,* Gottesman, GENE EXPRESSION TECHNOLOGY: METHODS IN ENZYMOLOGY 185, Academic Press, San Diego, Calif. (1990) 119-128. Another strategy is to alter the nucleic acid sequence of the nucleic acid to be inserted into an expression vector so that the individual codons for each amino acid are those preferentially utilized in *E. coli (see, e.g.,* Wada, et al., 1992. Nucl. Acids Res. 20: 2111-2118). Such alteration of nucleic acid sequences of the invention can be carried out by standard DNA synthesis techniques.

In another embodiment, the FCTRX expression vector is a yeast expression vector. Examples of vectors for expression in yeast *Saccharomyces cerivisae* include pYepSecl (Baldari, et al., 1987. EMBO J. 6: 229-234), pMFa (Kurjan and Herskowitz, 1982. Cell 30: 933-943) , pJRY88 (Schultz et al., 1987. Gene 54: 113-123), pYES2 (Invitrogen Corporation, San Diego, Calif.), and picZ (InVitrogen Corp, San Diego, Calif.).

Alternatively, FCTRX can be expressed in insect cells using baculovirus expression vectors. Baculovirus vectors available for expression of proteins in cultured insect cells (*e.g.,* SF9 cells) include the pAc series (Smith, et al., 1983. Mol. Cell. Biol. 3: 2156-2165) and the pVL series (Lucklow and Summers, 1989. Virology 170: 31-39).

In yet another embodiment, a nucleic acid of the invention is expressed in mammalian cells using a mammalian expression vector. Examples of mammalian expression vectors include pCDM8 (Seed, 1987. Nature 329: 840) and pMT2PC (Kaufman, et al., 1987. EMBO J. 6: 187-195). When used in mammalian cells, the expression vector's control functions are often provided by viral regulatory elements. For example, commonly used promoters are derived from polyoma, adenovirus 2, cytomegalovirus, and simian virus 40. For other suitable expression systems for both prokaryotic and eukaryotic cells see, *e.g*., Chapters 16 and 17 of Sambrook, et al., MOLECULAR CLONING: A LABORATORY MANUAL. 2nd ed., Cold Spring Harbor Laboratory, Cold Spring Harbor Laboratory Press, Cold Spring Harbor, N.Y., 1989.

In another embodiment, the recombinant mammalian expression vector is capable of directing expression of the nucleic acid preferentially in a particular cell type (*e.g*., tissue-specific regulatory elements are used to express the nucleic acid). Tissue-specific regulatory elements are known in the art. Non-limiting examples of suitable tissue-specific promoters include the albumin promoter (liver-specific; Pinkert, et al., 1987. Genes Dev. 1: 268-277), lymphoid-specific promoters (Calame and Eaton, 1988. Adv. Immunol. 43: 235-275), in particular promoters of T cell receptors (Winoto and Baltimore, 1989. EMBO J. 8: 729-733) and immunoglobulins (Banerji, et al., 1983. Cell 33: 729-740; Queen and Baltimore, 1983. Cell 33: 741-748), neuron-specific promoters (*e.g*., the neurofilament promoter; Byme and Ruddle, 1989. Proc. Natl. Acad. Sci. USA 86: 5473-5477), pancreas-specific promoters (Edlund, et al., 1985. Science 230: 912-916), and mammary gland-specific promoters (*e.g.,* milk whey promoter; U.S. Pat. No. 4,873,316 and European Application Publication No. 264,166). Developmentally-regulated promoters are also encompassed, *e.g.,* the murine hox promoters (Kessel and Gruss, 1990. Science 249: 374-379) and the α-fetoprotein promoter (Campes and Tilghman, 1989. Genes Dev. 3: 537-546).

The invention further provides a recombinant expression vector comprising a DNA molecule of the invention cloned into the expression vector in an antisense orientation. That is, the DNA molecule is operatively-linked to a regulatory sequence in a manner that allows for expression (by transcription of the DNA molecule) of an RNA molecule that is antisense to FCTRX mRNA. Regulatory sequences operatively linked to a nucleic acid cloned in the antisense orientation can be chosen that direct the continuous expression of the antisense RNA molecule in a variety of cell types, for instance viral promoters and/or enhancers, or regulatory sequences can be chosen that direct constitutive, tissue specific or cell type specific expression of antisense RNA. The antisense expression vector can be in the form of a recombinant plasmid, phagemid or attenuated virus in which antisense nucleic acids are produced under the control of a high efficiency regulatory region, the activity of which can be determined by the cell type into which the vector is introduced. For a discussion of the regulation of gene expression using antisense genes *see, e.g.,* Weintraub, et al., "Antisense RNA as a molecular tool for genetic analysis," Reviews-Trends in Genetics, Vol. 1(1) 1986.

Another aspect of the invention pertains to host cells into which a recombinant expression vector of the invention has been introduced. The terms "host cell" and "recombinant host cell" are used interchangeably herein. It is understood that such terms refer not only to the particular subject cell but also to the progeny or potential progeny of such a cell. Because certain modifications may occur in succeeding generations due to either mutation or environmental influences, such progeny may not, in fact, be identical to the parent cell, but are still included within the scope of the term as used herein.

A host cell can be any prokaryotic or eukaryotic cell. For example, FCTRX protein can be expressed in bacterial cells such as *E*. *coli,* insect cells, yeast or mammalian cells (such as Chinese hamster ovary cells (CHO) or COS cells). Other suitable host cells are known to those skilled in the art.

Vector DNA can be introduced into prokaryotic or eukaryotic cells via conventional transformation or transfection techniques. As used herein, the terms "transformation" and "transfection" are intended to refer to a variety of art-recognized techniques for introducing foreign nucleic acid (*e.g*., DNA) into a host cell, including calcium phosphate or calcium chloride co-precipitation, DEAE-dextran-mediated transfection, lipofection, or electroporation. Suitable methods for transforming or transfecting host cells can be found in Sambrook, et al. (MOLECULAR CLONING: A LABORATORY MANUAL. 2nd ed., Cold Spring Harbor Laboratory, Cold Spring Harbor Laboratory Press, Cold Spring Harbor, N.Y., 1989), and other laboratory manuals.

For stable transfection of mammalian cells, it is known that, depending upon the expression vector and transfection technique used, only a small fraction of cells may integrate the foreign DNA into their genome. In order to identify and select these integrants, a gene that encodes a selectable marker (*e.g*., resistance to antibiotics) is generally introduced into the host cells along with the gene of interest. Various selectable markers include those that confer resistance to drugs, such as G418, hygromycin and methotrexate. Nucleic acid encoding a selectable marker can be introduced into a host cell on the same vector as that encoding FCTRX or can be introduced on a separate vector. Cells stably transfected with the introduced nucleic acid can be identified by drug selection (*e.g.,* cells that have incorporated the selectable marker gene will survive, while the other cells die).

A host cell of the invention, such as a prokaryotic or eukaryotic host cell in culture, can be used to produce (*i.e.,* express) FCTRX protein. Accordingly, the invention further provides methods for producing FCTRX protein using the host cells of the invention. In one embodiment, the method comprises culturing the host cell of invention (into which a recombinant expression vector encoding FCTRX protein has been introduced) in a suitable medium such that FCTRX protein is produced. In another embodiment, the method further comprises isolating FCTRX protein from the medium or the host cell.

### Transgenic FCTRX Animals

The host cells of the invention can also be used to produce non-human transgenic animals. For example, in one embodiment, a host cell of the invention is a fertilized oocyte or an embryonic stem cell into which FCTRX protein-coding sequences have been introduced. Such host cells can then be used to create non-human transgenic animals in which exogenous FCTRX sequences have been introduced into their genome or homologous recombinant animals in which endogenous FCTRX sequences have been altered. Such animals are useful for studying the function and/or activity of FCTRX protein and for identifying and/or evaluating modulators of FCTRX protein activity. As used herein, a "transgenic animal" is a non-human animal, preferably a mammal, more preferably a rodent such as a rat or mouse, in which one or more of the cells of the animal includes a transgene. Other examples of transgenic animals include non-human primates, sheep, dogs, cows, goats, chickens, amphibians, etc. A transgene is exogenous DNA that is integrated into the genome of a cell from which a transgenic animal develops and that remains in the genome of the mature animal, thereby directing the expression of an encoded gene product in one or more cell types or tissues of the transgenic animal. As used herein, a "homologous recombinant animal" is a non-human animal, preferably a mammal, more preferably a mouse, in which an endogenous FCTRX gene has been altered by homologous recombination between the endogenous gene and an exogenous DNA molecule introduced into a cell of the animal, *e.g*., an embryonic cell of the animal, prior to development of the animal.

A transgenic animal of the invention can be created by introducing FCTRX-encoding nucleic acid into the male pronuclei of a fertilized oocyte (*e.g*., by microinjection, retroviral infection) and allowing the oocyte to develop in a pseudopregnant female foster animal. The human FCTRX cDNA sequences of SEQ ID NOS:1, 3, 5, 7, 9, 10,11, 12, 14, 16,18, 20, 22, and 24, can be introduced as a transgene into the genome of a non-human animal. Alternatively, a non-human homologue of the human FCTRX gene, such as a mouse FCTRX gene, can be isolated based on hybridization to the human FCTRX cDNA (described further *supra*) and used as a transgene. Intronic sequences and polyadenylation signals can also be included in the transgene to increase the efficiency of expression of the transgene. A tissue-specific regulatory sequence(s) can be operably-linked to the FCTRX transgene to direct expression of FCTRX protein to particular cells. Methods for generating transgenic animals via embryo manipulation and microinjection, particularly animals such as mice, have become conventional in the art and are described, for example, in U.S. Patent Nos. 4,736,866; 4,870,009; and 4,873,191; and Hogan, 1986. In: MANIPULATING THE MOUSE EMBRYO, Cold Spring Harbor Laboratory Press, Cold Spring Harbor, N.Y. Similar methods are used for production of other transgenic animals. A transgenic founder animal can be identified based upon the presence of the FCTRX transgene in its genome and/or expression of FCTRX mRNA in tissues or cells of the animals. A transgenic founder animal can then be used to breed additional animals carrying the transgene. Moreover, transgenic animals carrying a transgene-encoding FCTRX protein can further be bred to other transgenic animals carrying other transgenes.

To create a homologous recombinant animal, a vector is prepared which contains at least a portion of an FCTRX gene into which a deletion, addition or substitution has been introduced to thereby alter, *e.g*., functionally disrupt, the FCTRX gene. The FCTRX gene can be a human gene (*e.g.,* the cDNA of SEQ ID NOS:1, 3, 5, 7, 9, 10, 11, 12, 14, 16,18, 20, 22, and 24), but more preferably, is a non-human homologue of a human FCTRX gene. For example, a mouse homologue of human FCTRX gene of SEQ ID NOS:1, 3, 5, 7, 9, 10, 11, 12, 14, 16, 18, 20, 22, and 24, can be used to construct a homologous recombination vector suitable for altering an endogenous FCTRX gene in the mouse genome. In one embodiment, the vector is designed such that, upon homologous recombination, the endogenous FCTRX gene is functionally disrupted (*i.e.,* no longer encodes a functional protein; also referred to as a "knock out" vector).

Alternatively, the vector can be designed such that, upon homologous recombination, the endogenous FCTRX gene is mutated or otherwise altered but still encodes functional protein (*e.g.,* the upstream regulatory region can be altered to thereby alter the expression of the endogenous FCTRX protein). In the homologous recombination vector, the altered portion of the FCTRX gene is flanked at its 5'- and 3'-termini by additional nucleic acid of the FCTRX gene to allow for homologous recombination to occur between the exogenous FCTRX gene carried by the vector and an endogenous FCTRX gene in an embryonic stem cell. The additional flanking FCTRX nucleic acid is of sufficient length for successful homologous recombination with the endogenous gene. Typically, several kilobases of flanking DNA (both at the 5'- and 3'-termini) are included in the vector. *See, e.g.,* Thomas, et al., 1987. Cell 51: 503 for a description of homologous recombination vectors. The vector is ten introduced into an embryonic stem cell line (*e.g*., by electroporation) and cells in which the introduced FCTRX gene has homologously-recombined with the endogenous FCTRX gene are selected. *See, e.g.,* Li, et al., 1992. Cell 69: 915.

The selected cells are then injected into a blastocyst of an animal (*e.g.,* a mouse) to form aggregation chimeras. *See, e.g.,* Bradley, 1987. In: TERATOCARCINOMAS AND EMBRYONIC STEM CELLS: A PRACTICAL APPROACH, Robertson, ed. IRL, Oxford, pp. 113-152. A chimeric embryo can then be implanted into a suitable pseudopregnant female foster animal and the embryo brought to term. Progeny harboring the homologously-recombined DNA in their germ cells can be used to breed animals in which all cells of the animal contain the homologously-recombined DNA by germline transmission of the transgene. Methods for constructing homologous recombination vectors and homologous recombinant animals are described further in Bradley, 1991. Curr. Opin. Biotechnol. 2: 823-829; PCT International Publication Nos.: WO 90/11354; WO 91/01140; WO 92/0968; and WO 93/04169.

In another embodiment, transgenic non-humans animals can be produced that contain selected systems that allow for regulated expression of the transgene. One example of such a system is the cre/loxP recombinase system of bacteriophage P1. For a description of the cre/loxP recombinase system, *See, e.g.,* Lakso, et al., 1992. Proc. Natl. Acad. Sci. USA 89: 6232-6236. Another example of a recombinase system is the FLP recombinase system of *Saccharomyces cerevisiae. See,* O'Gorman, et al., 1991. Science 251:1351-1355. If a cre/loxP recombinase system is used to regulate expression of the transgene, animals containing transgenes encoding both the Cre recombinase and a selected protein are required. Such animals can be provided through the construction of "double" transgenic animals, *e.g*., by mating two transgenic animals, one containing a transgene encoding a selected protein and the other containing a transgene encoding a recombinase.

Clones of the non-human transgenic animals described herein can also be produced according to the methods described in Wilmut, et al., 1997. Nature 385: 810-813. In brief, a cell (*e.g.,* a somatic cell) from the transgenic animal can be isolated and induced to exit the growth cycle and enter G₀ phase. The quiescent cell can then be fused, *e.g.,* through the use of electrical pulses, to an enucleated oocyte from an animal of the same species from which the quiescent cell is isolated. The reconstructed oocyte is then cultured such that it develops to morula or blastocyte and then transferred to pseudopregnant female foster animal. The offspring borne of this female foster animal will be a clone of the animal from which the cell (*e.g*., the somatic cell) is isolated.

### Pharmaceutical Compositions

The FCTRX nucleic acid molecules, FCTRX proteins, and anti-FCTRX antibodies (also referred to herein as "active compounds") of the invention, and derivatives, fragments, analogs and homologs thereof, can be incorporated into pharmaceutical compositions suitable for administration. Such compositions typically comprise the nucleic acid molecule, protein, or antibody and a pharmaceutically acceptable carrier. As used herein, "pharmaceutically acceptable carrier" is intended to include any and all solvents, dispersion media, coatings, antibacterial and antifungal agents, isotonic and absorption delaying agents, and the like, compatible with pharmaceutical administration. Suitable carriers are described in the most recent edition of Remington's Pharmaceutical Sciences, a standard reference text in the field, which is incorporated herein by reference. Preferred examples of such carriers or diluents include, but are not limited to, water, saline, finger's solutions, dextrose solution, and 5% human serum albumin. Liposomes and non-aqueous vehicles such as fixed oils may also be used. The use of such media and agents for pharmaceutically active substances is well known in the art. Except insofar as any conventional media or agent is incompatible with the active compound, use thereof in the compositions is contemplated. Supplementary active compounds can also be incorporated into the compositions.

A pharmaceutical composition of the invention is formulated to be compatible with its intended route of administration. Examples of routes of administration include parenteral, *e.g*., intravenous, intradermal, subcutaneous, oral (*e.g*., inhalation), transdermal (*i.e.,* topical), transmucosal, and rectal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid (EDTA); buffers such as acetates, citrates or phosphates, and agents for the adjustment of tonicity such as sodium chloride or dextrose. The pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL^{™} (BASF, Parsippany, N.J.) or phosphate buffered saline (PBS). In all cases, the composition must be sterile and should be fluid to the extent that easy syringeability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as manitol, sorbitol, sodium chloride in the composition. Prolonged absorption of the injectable compositions can be brought about by including in the composition an agent which delays absorption, for example, aluminum monostearate and gelatin.

Sterile injectable solutions can be prepared by incorporating the active compound (*e.g.,* an FCTRX protein or anti-FCTRX antibody) in the required amount in an appropriate solvent with one or a combination of ingredients enumerated above, as required, followed by filtered sterilization. Generally, dispersions are prepared by incorporating the active compound into a sterile vehicle that contains a basic dispersion medium and the required other ingredients from those enumerated above. In the case of sterile powders for the preparation of sterile injectable solutions, methods of preparation are vacuum drying and freeze-drying that yields a powder of the active ingredient plus any additional desired ingredient from a previously sterile-filtered solution thereof.

Oral compositions generally include an inert diluent or an edible carrier. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

For administration by inhalation, the compounds are delivered in the form of an aerosol spray from pressured container or dispenser which contains a suitable propellant, *e.g*., a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

The compounds can also be prepared in the form of suppositories (*e.g.,* with conventional suppository bases such as cocoa butter and other glycerides) or retention enemas for rectal delivery.

In one embodiment, the active compounds are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art, for example, as described in U.S. Patent No. 4,522,811.

It is especially advantageous to formulate oral or parenteral compositions in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier. The specification for the dosage unit forms of the invention are dictated by and directly dependent on the unique characteristics of the active compound and the particular therapeutic effect to be achieved, and the limitations inherent in the art of compounding such an active compound for the treatment of individuals.

The nucleic acid molecules of the invention can be inserted into vectors and used as gene therapy vectors. Gene therapy vectors can be delivered to a subject by, for example, intravenous injection, local administration *(see, e.g.,* U.S. Patent No. 5,328,470) or by stereotactic injection *(see, e.g.,* Chen, et al., 1994. Proc. Natl. Acad. Sci. USA 91: 3054-3057). The pharmaceutical preparation of the gene therapy vector can include the gene therapy vector in an acceptable diluent, or can comprise a slow release matrix in which the gene delivery vehicle is imbedded. Alternatively, where the complete gene delivery vector can be produced intact from recombinant cells, *e.g*., retroviral vectors, the pharmaceutical preparation can include one or more cells that produce the gene delivery system.

The pharmaceutical compositions can be included in a container, pack, or dispenser together with instructions for administration.

### Screening and Detection Methods

The isolated nucleic acid molecules of the invention can be used to express FCTRX protein (*e.g*., via a recombinant expression vector in a host cell in gene therapy applications), to detect FCTRX mRNA (*e.g.,* in a biological sample) or a genetic lesion in an FCTRX gene, and to modulate FCTRX activity, as described further, below. In addition, the FCTRX proteins can be used to screen drugs or compounds that modulate the FCTRX protein activity or expression as well as to treat disorders characterized by insufficient or excessive production of FCTRX protein or production of FCTRX protein forms that have decreased or aberrant activity compared to FCTRX wild-type protein (*e.g*.; diabetes (regulates insulin release); obesity (binds and transport lipids); metabolic disturbances associated with obesity, the metabolic syndrome X as well as anorexia and wasting disorders associated with chronic diseases and various cancers, and infectious disease(possesses anti-microbial activity) and the various dyslipidemias. In addition, the anti-FCTRX antibodies of the invention can be used to detect and isolate FCTRX proteins and modulate FCTRX activity. In yet a further aspect, the invention can be used in methods to influence appetite, absorption of nutrients and the disposition of metabolic substrates in both a positive and negative fashion.

The invention further pertains to novel agents identified by the screening assays described herein and uses thereof for treatments as described, *supra.*

### Screening Assays

The invention provides a method (also referred to herein as a "screening assay") for identifying modulators, *i.e*., candidate or test compounds or agents (*e.g*., peptides, peptidomimetics, small molecules or other drugs) that bind to FCTRX proteins or have a stimulatory or inhibitory effect on, *e.g.,* FCTRX protein expression or FCTRX protein activity. The invention also includes compounds identified in the screening assays described herein.

In one embodiment, the invention provides assays for screening candidate or test compounds which bind to or modulate the activity of the membrane-bound form of an FCTRX protein or polypeptide or biologically-active portion thereof. The test compounds of the invention can be obtained using any of the numerous approaches in combinatorial library methods known in the art, including: biological libraries; spatially addressable parallel solid phase or solution phase libraries; synthetic library methods requiring deconvolution; the "one-bead one-compound" library method; and synthetic library methods using affinity chromatography selection. The biological library approach is limited to peptide libraries, while the other four approaches are applicable to peptide, non-peptide oligomer or small molecule libraries of compounds. *See, e.g.,* Lam, 1997. *Anticancer Drug Design* 12: 145.

A "small molecule" as used herein, is meant to refer to a composition that has a molecular weight of less than about 5 kD and most preferably less than about 4 kD. Small molecules can be, *e.g.,* nucleic acids, peptides, polypeptides, peptidomimetics, carbohydrates, lipids or other organic or inorganic molecules. Libraries of chemical and/or biological mixtures, such as fungal, bacterial, or algal extracts, are known in the art and can be screened with any of the assays of the invention.

Examples of methods for the synthesis of molecular libraries can be found in the art, for example in: DeWitt, et al., 1993. Proc. Natl. Acad. Sci. U.S.A. 90: 6909; Erb, et al., 1994. Proc. Natl. Acad. Sci. U.S.A. 91: 11422; Zuckermann, et al., 1994. J. Med. Chem. 37: 2678; Cho, et al., 1993. Science 261: 1303; Carrell, et al., 1994. Angew. Chem. Int. Ed. Engl. 33:2059; Carell, et al., 1994. Angew. Chem. Int. Ed. Engl. 33: 2061; and Gallop, et al., 1994. J. Med. Chem. 37: 1233.

Libraries of compounds may be presented in solution (*e.g*., Houghten, 1992. Biotechniques 13: 412-421), or on beads (Lam, 1991. Nature 354: 82-84), on chips (Fodor, 1993. Nature 364: 555-556), bacteria (Ladner, U.S. Patent No. 5,223,409), spores (Ladner, U.S. Patent 5,233,409), plasmids (Cull, et al., 1992. Proc. Natl. Acad. Sci. USA 89: 1865-1869) or on phage (Scott and Smith, 1990. Science 249: 386-390; Devlin, 1990. Science 249: 404-406; Cwirla, et al., 1990. Proc. Natl. Acad. Sci. U.S.A. 87: 6378-6382; Felici, 1991. J. Mol. Biol. 222: 301-310; Ladner, U.S. Patent No. 5,233,409.).

In one embodiment, an assay is a cell-based assay in which a cell which expresses a membrane-bound form of FCTRX protein, or a biologically-active portion thereof, on the cell surface is contacted with a test compound and the ability of the test compound to bind to an FCTRX protein determined. The cell, for example, can of mammalian origin or a yeast cell. Determining the ability of the test compound to bind to the FCTRX protein can be accomplished, for example, by coupling the test compound with a radioisotope or enzymatic label such that binding of the test compound to the FCTRX protein or biologically-active portion thereof can be determined by detecting the labeled compound in a complex. For example, test compounds can be labeled with ¹²⁵I, ³⁵S, ¹⁴C, or 3H, either directly or indirectly, and the radioisotope detected by direct counting of radio emission or by scintillation counting. Alternatively, test compounds can be enzymatically-labeled with, for example, horseradish peroxidase, alkaline phosphatase, or luciferase, and the enzymatic label detected by determination of conversion of an appropriate substrate to product. In one embodiment, the assay comprises contacting a cell which expresses a membrane-bound form of FCTRX protein, or a biologically-active portion thereof, on the cell surface with a known compound which binds FCTRX to form an assay mixture, contacting the assay mixture with a test compound, and determining the ability of the test compound to interact with an FCTRX protein, wherein determining the ability of the test compound to interact with an FCTRX protein comprises determining the ability of the test compound to preferentially bind to FCTRX protein or a biologically-active portion thereof as compared to the known compound.

In another embodiment, an assay is a cell-based assay comprising contacting a cell expressing a membrane-bound form of FCTRX protein, or a biologically-active portion thereof, on the cell surface with a test compound and determining the ability of the test compound to modulate (*e.g.,* stimulate or inhibit) the activity of the FCTRX protein or biologically-active portion thereof. Determining the ability of the test compound to modulate the activity of FCTRX or a biologically-active portion thereof can be accomplished, for example, by determining the ability of the FCTRX protein to bind to or interact with an FCTRX target molecule. As used herein, a "target molecule" is a molecule with which an FCTRX protein binds or interacts in nature, for example, a molecule on the surface of a cell which expresses an FCTRX interacting protein, a molecule on the surface of a second cell, a molecule in the extracellular milieu, a molecule associated with the internal surface of a cell membrane or a cytoplasmic molecule. An FCTRX target molecule can be a non-FCTRX molecule or an FCTRX protein or polypeptide of the invention. In one embodiment, an FCTRX target molecule is a component of a signal transduction pathway that facilitates transduction of an extracellular signal (*e.g.* a signal generated by binding of a compound to a membrane-bound FCTRX molecule) through the cell membrane and into the cell. The target, for example, can be a second intercellular protein that has catalytic activity or a protein that facilitates the association of downstream signaling molecules with FCTRX.

Determining the ability of the FCTRX protein to bind to or interact with an FCTRX target molecule can be accomplished by one of the methods described above for determining direct binding. In one embodiment, determining the ability of the FCTRX protein to bind to or interact with an FCTRX target molecule can be accomplished by determining the activity of the target molecule. For example, the activity of the target molecule can be determined by detecting induction of a cellular second messenger of the target (*i.e.* intracellular Ca²⁺, diacylglycerol, IP₃, etc.), detecting catalytic/enzymatic activity of the target an appropriate substrate, detecting the induction of a reporter gene (comprising an FCTRX-responsive regulatory element operatively linked to a nucleic acid encoding a detectable marker, *e.g*., luciferase), or detecting a cellular response, for example, cell survival, cellular differentiation, or cell proliferation.

In yet another embodiment, an assay of the invention is a cell-free assay comprising contacting an FCTRX protein or biologically-active portion thereof with a test compound and determining the ability of the test compound to bind to the FCTRX protein or biologically-active portion thereof. Binding of the test compound to the FCTRX protein can be determined either directly or indirectly as described above. In one such embodiment, the assay comprises contacting the FCTRX protein or biologically-active portion thereof with a known compound which binds FCTRX to form an assay mixture, contacting the assay mixture with a test compound, and determining the ability of the test compound to interact with an FCTRX protein, wherein determining the ability of the test compound to interact with an FCTRX protein comprises determining the ability of the test compound to preferentially bind to FCTRX or biologically-active portion thereof as compared to the known compound.

In still another embodiment, an assay is a cell-free assay comprising contacting FCTRX protein or biologically-active portion thereof with a test compound and determining the ability of the test compound to modulate (*e.g*. stimulate or inhibit) the activity of the FCTRX protein or biologically-active portion thereof. Determining the ability of the test compound to modulate the activity of FCTRX can be accomplished, for example, by determining the ability of the FCTRX protein to bind to an FCTRX target molecule by one of the methods described above for determining direct binding. In an alternative embodiment, determining the ability of the test compound to modulate the activity of FCTRX protein can be accomplished by determining the ability of the FCTRX protein further modulate an FCTRX target molecule. For example, the catalytic/enzymatic activity of the target molecule on an appropriate substrate can be determined as described, *supra.*

In yet another embodiment, the cell-free assay comprises contacting the FCTRX protein or biologically-active portion thereof with a known compound which binds FCTRX protein to form an assay mixture, contacting the assay mixture with a test compound, and determining the ability of the test compound to interact with an FCTRX protein, wherein determining the ability of the test compound to interact with an FCTRX protein comprises determining the ability of the FCTRX protein to preferentially bind to or modulate the activity of an FCTRX target molecule.

The cell-free assays of the invention are amenable to use of both the soluble form or the membrane-bound form of FCTRX protein. In the case of cell-free assays comprising the membrane-bound form of FCTRX protein, it may be desirable to utilize a solubilizing agent such that the membrane-bound form of FCTRX protein is maintained in solution. Examples of such solubilizing agents include non-ionic detergents such as n-octylglucoside, n-dodecylglucoside, n-dodecylmaltoside, octanoyl-N-methylglucamide, decanoyl-N-methylglucamide, Triton^{®} X-100, Triton^{®} X-114, Thesit^{®}, Isotridecypoly(ethylene glycol ether)ₙ, N-dodecyl--N,N-dimethyl-3-ammonio-1-propane sulfonate, 3-(3-cholamidopropyl) dimethylamminiol-1-propane sulfonate (CHAPS), or 3-(3-cholamidopropyl)dimethylamminiol-2-hydroxy-1-propane sulfonate (CHAPSO).

In more than one embodiment of the above assay methods of the invention, it may be desirable to immobilize either FCTRX protein or its target molecule to facilitate separation of complexed from uncomplexed forms of one or both of the proteins, as well as to accommodate automation of the assay. Binding of a test compound to FCTRX protein, or interaction of FCTRX protein with a target molecule in the presence and absence of a candidate compound, can be accomplished in any vessel suitable for containing the reactants. Examples of such vessels include microtiter plates, test tubes, and micro-centrifuge tubes. In one embodiment, a fusion protein can be provided that adds a domain that allows one or both of the proteins to be bound to a matrix. For example, GST-FCTRX fusion proteins or GST-target fusion proteins can be adsorbed onto glutathione sepharose beads (Sigma Chemical, St. Louis, MO) or glutathione derivatized microtiter plates, that are then combined with the test compound or the test compound and either the non-adsorbed target protein or FCTRX protein, and the mixture is incubated under conditions conducive to complex formation (*e.g*., at physiological conditions for salt and pH). Following incubation, the beads or microtiter plate wells are washed to remove any unbound components, the matrix immobilized in the case of beads, complex determined either directly or indirectly, for example, as described, *supra.* Alternatively, the complexes can be dissociated from the matrix, and the level of FCTRX protein binding or activity determined using standard techniques.

Other techniques for immobilizing proteins on matrices can also be used in the screening assays of the invention. For example, either the FCTRX protein or its target molecule can be immobilized utilizing conjugation of biotin and streptavidin. Biotinylated FCTRX protein or target molecules can be prepared from biotin-NHS (N-hydroxy-succinimide) using techniques well-known within the art (*e.g.,* biotinylation kit, Pierce Chemicals, Rockford, Ill.), and immobilized in the wells of streptavidin-coated 96 well plates (Pierce Chemical). Alternatively, antibodies reactive with FCTRX protein or target molecules, but which do not interfere with binding of the FCTRX protein to its target molecule, can be derivatized to the wells of the plate, and unbound target or FCTRX protein trapped in the wells by antibody conjugation. Methods for detecting such complexes, in addition to those described above for the GST-immobilized complexes, include immunodetection of complexes using antibodies reactive with the FCTRX protein or target molecule, as well as enzyme-linked assays that rely on detecting an enzymatic activity associated with the FCTRX protein or target molecule.

In another embodiment, modulators of FCTRX protein expression are identified in a method wherein a cell is contacted with a candidate compound and the expression of FCTRX mRNA or protein in the cell is determined. The level of expression of FCTRX mRNA or protein in the presence of the candidate compound is compared to the level of expression of FCTRX mRNA or protein in the absence of the candidate compound. The candidate compound can then be identified as a modulator of FCTRX mRNA or protein expression based upon this comparison. For example, when expression of FCTRX mRNA or protein is greater (*i.e*., statistically significantly greater) in the presence of the candidate compound than in its absence, the candidate compound is identified as a stimulator of FCTRX mRNA or protein expression. Alternatively, when expression of FCTRX mRNA or protein is less (statistically significantly less) in the presence of the candidate compound than in its absence, the candidate compound is identified as an inhibitor of FCTRX mRNA or protein expression. The level of FCTRX mRNA or protein expression in the cells can be determined by methods described herein for detecting FCTRX mRNA or protein.

In yet another aspect of the invention, the FCTRX proteins can be used as "bait proteins" in a two-hybrid assay or three hybrid assay *(see, e.g.,* U.S. Patent No. 5,283,317; Zervos, et al., 1993. Cell 72: 223-232; Madura, et al., 1993. J. Biol. Chem. 268: 12046-12054; Bartel, et al., 1993. Biotechniques 14: 920-924; Iwabuchi, et al., 1993. Oncogene 8: 1693-1696; and Brent WO 94/10300), to identify other proteins that bind to or interact with FCTRX ("FCTRX-binding proteins" or "FCTRX-bp") and modulate FCTRX activity. Such FCTRX-binding proteins are also likely to be involved in the propagation of signals by the FCTRX proteins as, for example, upstream or downstream elements of the FCTRX pathway.

The two-hybrid system is based on the modular nature of most transcription factors, which consist of separable DNA-binding and activation domains. Briefly, the assay utilizes two different DNA constructs. In one construct, the gene that codes for FCTRX is fused to a gene encoding the DNA binding domain of a known transcription factor (*e.g.*, GAL-4). In the other construct, a DNA sequence, from a library of DNA sequences, that encodes an unidentified protein ("prey" or "sample") is fused to a gene that codes for the activation domain of the known transcription factor. If the "bait" and the "prey" proteins are able to interact, *in vivo,* forming an FCTRX-dependent complex, the DNA-binding and activation domains of the transcription factor are brought into close proximity. This proximity allows transcription of a reporter gene (*e.g.,* LacZ) that is operably linked to a transcriptional regulatory site responsive to the transcription factor. Expression of the reporter gene can be detected and cell colonies containing the functional transcription factor can be isolated and used to obtain the cloned gene that encodes the protein which interacts with FCTRX.

The invention further pertains to novel agents identified by the aforementioned screening assays and uses thereof for treatments as described herein.

### Detection Assays

Portions or fragments of the cDNA sequences identified herein (and the corresponding complete gene sequences) can be used in numerous ways as polynucleotide reagents. By way of example, and not of limitation, these sequences can be used to: (*i*) map their respective genes on a chromosome; and, thus, locate gene regions associated with genetic disease; (*ii*) identify an individual from a minute biological sample (tissue typing); and (*iii*) aid in forensic identification of a biological sample. Some of these applications are described in the subsections, below.

### Chromosome Mapping

Once the sequence (or a portion of the sequence) of a gene has been isolated, this sequence can be used to map the location of the gene on a chromosome. This process is called chromosome mapping. Accordingly, portions or fragments of the FCTRX sequences, SEQ ID NOS:1, 3, 5, 7, 9, 10, 11, 12, 14, 16, 18, 20, 22, and 24, or fragments or derivatives thereof, can be used to map the location of the FCTRX genes, respectively, on a chromosome. The mapping of the FCTRX sequences to chromosomes is an important first step in correlating these sequences with genes associated with disease.

Briefly, FCTRX genes can be mapped to chromosomes by preparing PCR primers (preferably 15-25 bp in length) from the FCTRX sequences. Computer analysis of the FCTRX, sequences can be used to rapidly select primers that do not span more than one exon in the genomic DNA, thus complicating the amplification process. These primers can then be used for PCR screening of somatic cell hybrids containing individual human chromosomes. Only those hybrids containing the human gene corresponding to the FCTRX sequences will yield an amplified fragment.

Somatic cell hybrids are prepared by fusing somatic cells from different mammals (*e.g*., human and mouse cells). As hybrids of human and mouse cells grow and divide, they gradually lose human chromosomes in random order, but retain the mouse chromosomes. By using media in which mouse cells cannot grow, because they lack a particular enzyme, but in which human cells can, the one human chromosome that contains the gene encoding the needed enzyme will be retained. By using various media, panels of hybrid cell lines can be established. Each cell line in a panel contains either a single human chromosome or a small number of human chromosomes, and a full set of mouse chromosomes, allowing easy mapping of individual genes to specific human chromosomes. *See, e.g.,* D'Eustachio, et al., 1983. Science 220: 919-924. Somatic cell hybrids containing only fragments of human chromosomes can also be produced by using human chromosomes with translocations and deletions.

PCR mapping of somatic cell hybrids is a rapid procedure for assigning a particular sequence to a particular chromosome. Three or more sequences can be assigned per day using a single thermal cycler. Using the FCTRX sequences to design oligonucleotide primers, sub-localization can be achieved with panels of fragments from specific chromosomes.

Fluorescence *in situ* hybridization (FISH) of a DNA sequence to a metaphase chromosomal spread can further be used to provide a precise chromosomal location in one step. Chromosome spreads can be made using cells whose division has been blocked in metaphase by a chemical like colcemid that disrupts the mitotic spindle. The chromosomes can be treated briefly with trypsin, and then stained with Giemsa. A pattern of light and dark bands develops on each chromosome, so that the chromosomes can be identified individually. The FISH technique can be used with a DNA sequence as short as 500 or 600 bases. However, clones larger than 1,000 bases have a higher likelihood of binding to a unique chromosomal location with sufficient signal intensity for simple detection. Preferably 1,000 bases, and more preferably 2,000 bases, will suffice to get good results at a reasonable amount of time. For a review of this technique, *see,* Verma, et al., HUMAN CHROMOSOMES: A MANUAL OF BASIC TECHNIQUES (Pergamon Press, New York 1988).

Reagents for chromosome mapping can be used individually to mark a single chromosome or a single site on that chromosome, or panels of reagents can be used for marking multiple sites and/or multiple chromosomes. Reagents corresponding to noncoding regions of the genes actually are preferred for mapping purposes. Coding sequences are more likely to be conserved within gene families, thus increasing the chance of cross hybridizations during chromosomal mapping.

Once a sequence has been mapped to a precise chromosomal location, the physical position of the sequence on the chromosome can be correlated with genetic map data. Such data are found, *e.g*., in McKusick, MENDELIAN INHERITANCE IN MAN, available on-line through Johns Hopkins University Welch Medical Library). The relationship between genes and disease, mapped to the same chromosomal region, can then be identified through linkage analysis (co-inheritance of physically adjacent genes), described in, *e.g.,* Egeland, et al., 1987. Nature, 325: 783-787.

Moreover, differences in the DNA sequences between individuals affected and unaffected with a disease associated with the FCTRX gene, can be determined. If a mutation is observed in some or all of the affected individuals but not in any unaffected individuals, then the mutation is likely to be the causative agent of the particular disease. Comparison of affected and unaffected individuals generally involves first looking for structural alterations in the chromosomes, such as deletions or translocations that are visible from chromosome spreads or detectable using PCR based on that DNA sequence. Ultimately, complete sequencing of genes from several individuals can be performed to confirm the presence of a mutation and to distinguish mutations from polymorphisms.

### Tissue Typing

The FCTRX sequences of the invention can also be used to identify individuals from minute biological samples. In this technique, an individual's genomic DNA is digested with one or more restriction enzymes, and probed on a Southern blot to yield unique bands for identification. The sequences of the invention are useful as additional DNA markers for RFLP ("restriction fragment length polymorphisms," described in U.S. Patent No. 5,272,057).

Furthermore, the sequences of the invention can be used to provide an alternative technique that determines the actual base-by-base DNA sequence of selected portions of an individual's genome. Thus, the FCTRX sequences described herein can be used to prepare two PCR primers from the 5'- and 3'-termini of the sequences. These primers can then be used to amplify an individual's DNA and subsequently sequence it.

Panels of corresponding DNA sequences from individuals, prepared in this manner, can provide unique individual identifications, as each individual will have a unique set of such DNA sequences due to allelic differences. The sequences of the invention can be used to obtain such identification sequences from individuals and from tissue. The FCTRX sequences of the invention uniquely represent portions of the human genome. Allelic variation occurs to some degree in the coding regions of these sequences, and to a greater degree in the noncoding regions. It is estimated that allelic variation between individual humans occurs with a frequency of about once per each 500 bases. Much of the allelic variation is due to single nucleotide polymorphisms (SNPs), which include restriction fragment length polymorphisms (RFLPs).

Each of the sequences described herein can, to some degree, be used as a standard against which DNA from an individual can be compared for identification purposes. Because greater numbers of polymorphisms occur in the noncoding regions, fewer sequences are necessary to differentiate individuals. The noncoding sequences can comfortably provide positive individual identification with a panel of perhaps 10 to 1,000 primers that each yield a noncoding amplified sequence of 100 bases. If predicted coding sequences, such as those in SEQ ID NOS:1, 3, 5, 7, 9, 10, 11, 12, 14, 16, 18, 20, 22, and 24, are used, a more appropriate number of primers for positive individual identification would be 500-2,000.

### Predictive Medicine

The invention also pertains to the field of predictive medicine in which diagnostic assays, prognostic assays, pharmacogenomics, and monitoring clinical trials are used for prognostic (predictive) purposes to thereby treat an individual prophylactically. Accordingly, one aspect of the invention relates to diagnostic assays for determining FCTRX protein and/or nucleic acid expression as well as FCTRX activity, in the context of a biological sample (*e.g.,* blood, serum, cells, tissue) to thereby determine whether an individual is afflicted with a disease or disorder, or is at risk of developing a disorder, associated with aberrant FCTRX expression or activity. The disorders include Also within the scope of the invention is the use of a Therapeutic in the manufacture of a medicament for treating or preventing disorders or syndromes including, *e.g*., Colorectal cancer, adenomatous polyposis coli, myelogenous leukemia, congenital ceonatal alloimmune thrombocytopenia, multiple human solid malignancies, malignant ovarian tumours particularly at the interface between epithelia and stroma, malignant brain tumors, mammary tumors, human gliomas, astrocytomas, mixed glioma/astrocytomas, renal cells carcinoma, breast adenocarcinoma, ovarian cancer, melanomas, renal cell carcinoma, clear cell and granular cell carcinomas, autocrine/paracrine stimulation of tumor cell proliferation, autocrine/paracrine stimulation of tumor cell survival and tumor cell resistance to cytotoxic therapy, paranechmal and basement membrane invasion and motility of tumor cells thereby contributing to metastasis, tumor-mediated immunosuppression of T-cell mediated immune effector cells and pathways resulting in tumor escape from immune surveilance, neurological disorders, neurodegenerative disorders, nerve trauma, familial myelodysplastic syndrome, Charcot-Marie-Tooth neuropathy, demyelinating Gardner syndrome, familial myelodysplastic syndrome; mental health conditions, immunological disorders, allergy and infection, asthma, bronchial asthma, Avellino type eosinophilia, lung diseases, reproductive disorders, male infertility, female reproductive system disorders, male and female reproductive diseases, hemangioma, deafness, glycoprotein Ia deficiency, desmoid disease, turcot syndrome, liver cirrhosis, hepatitis C, gastric disorders, pancreatic diseases like diabetes, Schistosoma mansoni infection, Spinocerebellar ataxia, Plasmodium falciparum parasitemia, Corneal dystrophy -Groenouw type I, Corneal dystrophy - lattice type I, and Reis-Bucklers corneal dystrophy. The invention also provides for prognostic (or predictive) assays for determining whether an individual is at risk of developing a disorder associated with FCTRX protein, nucleic acid expression or activity. For example, mutations in an FCTRX gene can be assayed in a biological sample. Such assays can be used for prognostic or predictive purpose to thereby prophylactically treat an individual prior to the onset of a disorder characterized by or associated with FCTRX protein, nucleic acid expression, or biological activity.

Another aspect of the invention provides methods for determining FCTRX protein, nucleic acid expression or activity in an individual to thereby select appropriate therapeutic or prophylactic agents for that individual (referred to herein as "pharmacogenomics"). Pharmacogenomics allows for the selection of agents (*e.g*., drugs) for therapeutic or prophylactic treatment of an individual based on the genotype of the individual (*e.g.*, the genotype of the individual examined to determine the ability of the individual to respond to a particular agent.)

Yet another aspect of the invention pertains to monitoring the influence of agents (*e.g.,* drugs, compounds) on the expression or activity of FCTRX in clinical trials.

These and other agents are described in further detail in the following sections.

### Diagnostic Assays

An exemplary method for detecting the presence or absence of FCTRX in a biological sample involves obtaining a biological sample from a test subject and contacting the biological sample with a compound or an agent capable of detecting FCTRX protein or nucleic acid (*e.g.,* mRNA, genomic DNA) that encodes FCTRX protein such that the presence of FCTRX is detected in the biological sample. An agent for detecting FCTRX mRNA or genomic DNA is a labeled nucleic acid probe capable of hybridizing to FCTRX mRNA or genomic DNA. The nucleic acid probe can be, for example, a full-length FCTRX nucleic acid, such as the nucleic acid of SEQ ID NOS:1, 3, 5, 7, 9,10, 11, 12, 14, 16, 18, 20, 22, and 24, or a portion thereof, such as an oligonucleotide of at least 15, 30, 50,100, 250 or 500 nucleotides in length and sufficient to specifically hybridize under stringent conditions to FCTRX mRNA or genomic DNA. Other suitable probes for use in the diagnostic assays of the invention are described herein.

An agent for detecting FCTRX protein is an antibody capable of binding to FCTRX protein, preferably an antibody with a detectable label. Antibodies can be polyclonal, or more preferably, monoclonal. An intact antibody, or a fragment thereof (*e.g.,* Fab or F(ab')₂) can be used. The term "labeled", with regard to the probe or antibody, is intended to encompass direct labeling of the probe or antibody by coupling (*i.e*., physically linking) a detectable substance to the probe or antibody, as well as indirect labeling of the probe or antibody by reactivity with another reagent that is directly labeled. Examples of indirect labeling include detection of a primary antibody using a fluoreseently-labeled secondary antibody and end-labeling of a DNA probe with biotin such that it can be detected with fluorescently-labeled streptavidin. The term "biological sample" is intended to include tissues, cells and biological fluids isolated from a subject, as well as tissues, cells and fluids present within a subject. That is, the detection method of the invention can be used to detect FCTRX mRNA, protein, or genomic DNA in a biological sample *in vitro* as well as *in vivo.* For example, in *vitro* techniques for detection of FCTRX mRNA include Northern hybridizations and *in situ* hybridizations. *In vitro* techniques for detection of FCTRX protein include enzyme linked immunosorbent assays (ELISAs), Western blots, immunoprecipitations, and immunofluorescence. *In vitro* techniques for detection of FCTRX genomic DNA include Southern hybridizations. Furthermore, *in vivo* techniques for detection of FCTRX protein include introducing into a subject a labeled anti-FCTRX antibody. For example, the antibody can be labeled with a radioactive marker whose presence and location in a subject can be detected by standard imaging techniques.

In one embodiment, the biological sample contains protein molecules from the test subject. Alternatively, the biological sample can contain mRNA molecules from the test subject or genomic DNA molecules from the test subject. A preferred biological sample is a peripheral blood leukocyte sample isolated by conventional means from a subject.

In another embodiment, the methods further involve obtaining a control biological sample from a control subject, contacting the control sample with a compound or agent capable of detecting FCTRX protein, mRNA, or genomic DNA, such that the presence of FCTRX protein, mRNA or genomic DNA is detected in the biological sample, and comparing the presence of FCTRX protein, mRNA or genomic DNA in the control sample with the presence of FCTRX protein, mRNA or genomic DNA in the test sample.

The invention also encompasses kits for detecting the presence of FCTRX in a biological sample. For example, the kit can comprise: a labeled compound or agent capable of detecting FCTRX protein or mRNA in a biological sample; means for determining the amount of FCTRX in the sample; and means for comparing the amount of FCTRX in the sample with a standard. The compound or agent can be packaged in a suitable container. The kit can further comprise instructions for using the kit to detect FCTRX protein or nucleic acid.

### Prognostic Assays

The diagnostic methods described herein can furthermore be utilized to identify subjects having or at risk of developing a disease or disorder associated with aberrant FCTRX expression or activity. For example, the assays described herein, such as the preceding diagnostic assays or the following assays, can be utilized to identify a subject having or at risk of developing a disorder associated with FCTRX protein, nucleic acid expression or activity. Alternatively, the prognostic assays can be utilized to identify a subject having or at risk for developing a disease or disorder. Thus, the invention provides a method for identifying a disease or disorder associated with aberrant FCTRX expression or activity in which a test sample is obtained from a subject and FCTRX protein or nucleic acid (*e.g*., mRNA, genomic DNA) is detected, wherein the presence of FCTRX protein or nucleic acid is diagnostic for a subject having or at risk of developing a disease or disorder associated with aberrant FCTRX expression or activity. As used herein, a "test sample" refers to a biological sample obtained from a subject of interest. For example, a test sample can be a biological fluid (*e.g*., serum), cell sample, or tissue.

Furthermore, the prognostic assays described herein can be used to determine whether a subject can be administered an agent (*e.g*., an agonist, antagonist, peptidomimetic, protein, peptide, nucleic acid, small molecule, or other drug candidate) to treat a disease or disorder associated with aberrant FCTRX expression or activity. For example, such methods can be used to determine whether a subject can be effectively treated with an agent for a disorder. Thus, the invention provides methods for determining whether a subject can be effectively treated with an agent for a disorder associated with aberrant FCTRX expression or activity in which a test sample is obtained and FCTRX protein or nucleic acid is detected (*e.g.,* wherein the presence of FCTRX protein or nucleic acid is diagnostic for a subject that can be administered the agent to treat a disorder associated with aberrant FCTRX expression or activity).

The methods of the invention can also be used to detect genetic lesions in an FCTRX gene, thereby determining if a subject with the lesioned gene is at risk for a disorder characterized by aberrant cell proliferation and/or differentiation. In various embodiments, the methods include detecting, in a sample of cells from the subject, the presence or absence of a genetic lesion characterized by at least one of an alteration affecting the integrity of a gene encoding an FCTRX-protein, or the misexpression of the FCTRX gene. For example, such genetic lesions can be detected by ascertaining the existence of at least one of: (*i*) a deletion of one or more nucleotides from an FCTRX gene; (*ii*) an addition of one or more nucleotides to an FCTRX gene; (*iii*) a substitution of one or more nucleotides of an FCTRX gene, (*iv*) a chromosomal rearrangement of an FCTRX gene; (*v*) an alteration in the level of a messenger RNA transcript of an FCTRX gene, (*vi*) aberrant modification of an FCTRX gene, such as of the methylation pattern of the genomic DNA, (*vii*) the presence of a non-wild-type splicing pattern of a messenger RNA transcript of an FCTRX gene, (*viii*) a non-wild-type level of an FCTRX protein, (*ix*) allelic loss of an FCTRX gene, and (*x*) inappropriate post-translational modification of an FCTRX protein. As described herein, there are a large number of assay techniques known in the art which can be used for detecting lesions in an FCTRX gene. A preferred biological sample is a peripheral blood leukocyte sample isolated by conventional means from a subject. However, any biological sample containing nucleated cells may be used, including, for example, buccal mucosal cells.

In certain embodiments, detection of the lesion involves the use of a probe/primer in a polymerase chain reaction (PCR) *(see, e.g.,* U.S. Patent Nos. 4,683,195 and 4,683,202), such as anchor PCR or RACE PCR, or, alternatively, in a ligation chain reaction (LCR) (*see, e.g.,* Landegran, et al., 1988. Science 241: 1077-1080; and Nakazawa, et al., 1994. Proc. Natl. Acad. Sci. USA 91: 360-364), the latter of which can be particularly useful for detecting point mutations in the FCTRX-gene *(see,* Abravaya, et al., 1995. Nucl. Acids Res. 23: 675-682). This method can include the steps of collecting a sample of cells from a patient, isolating nucleic acid (*e.g*., genomic, mRNA or both) from the cells of the sample, contacting the nucleic acid sample with one or more primers that specifically hybridize to an FCTRX gene under conditions such that hybridization and amplification of the FCTRX gene (if present) occurs, and detecting the presence or absence of an amplification product, or detecting the size of the amplification product and comparing the length to a control sample. It is anticipated that PCR and/or LCR may be desirable to use as a preliminary amplification step in conjunction with any of the techniques used for detecting mutations described herein.

Alternative amplification methods include: self sustained sequence replication (*see*, Guatelli, et al., 1990. Proc. Natl. Acad. Sci. USA 87: 1874-1878), transcriptional amplification system (*see,* Kwoh, et al., 1989. Proc. Natl. Acad. Sci. USA 86: 1173-1177); Qβ Replicase (*see,* Lizardi, et al, 1988. BioTechnology 6: 1197), or any other nucleic acid amplification method, followed by the detection of the amplified molecules using techniques well known to those of skill in the art. These detection schemes are especially useful for the detection of nucleic acid molecules if such molecules are present in very low numbers.

In an alternative embodiment, mutations in an FCTRX gene from a sample cell can be identified by alterations in restriction enzyme cleavage patterns. For example, sample and control DNA is isolated, amplified (optionally), digested with one or more restriction endonucleases, and fragment length sizes are determined by gel electrophoresis and compared. Differences in fragment length sizes between sample and control DNA indicates mutations in the sample DNA. Moreover, the use of sequence specific ribozymes *(see, e.g.,* U.S. Patent No. 5,493,531) can be used to score for the presence of specific mutations by development or loss of a ribozyme cleavage site.

In other embodiments, genetic mutations in FCTRX can be identified by hybridizing a sample and control nucleic acids, *e.g*., DNA or RNA, to high-density arrays containing hundreds or thousands of oligonucleotides probes. *See, e.g.,* Cronin, et al., 1996. Human Mutation 7: 244-255; Kozal, et al., 1996. Nat. Med. 2: 753-759. For example, genetic mutations in FCTRX can be identified in two dimensional arrays containing light-generated DNA probes as described in Cronin, *et al., supra.* Briefly, a first hybridization array of probes can be used to scan through long stretches of DNA in a sample and control to identify base changes between the sequences by making linear arrays of sequential overlapping probes. This step allows the identification of point mutations. This is followed by a second hybridization array that allows the characterization of specific mutations by using smaller, specialized probe arrays complementary to all variants or mutations detected. Each mutation array is composed of parallel probe sets, one complementary to the wild-type gene and the other complementary to the mutant gene.

In yet another embodiment, any of a variety of sequencing reactions known in the art can be used to directly sequence the FCTRX gene and detect mutations by comparing the sequence of the sample FCTRX with the corresponding wild-type (control) sequence. Examples of sequencing reactions include those based on techniques developed by Maxim and Gilbert, 1977. Proc. Natl. Acad. Sci. USA 74: 560 or Sanger, 1977. Proc. Natl. Acad. Sci. USA 74: 5463. It is also contemplated that any of a variety of automated sequencing procedures can be utilized when performing the diagnostic assays (*see, e.g.,* Naeve, et al., 1995. Biotechniques 19: 448), including sequencing by mass spectrometry (see, *e.g*., PCT International Publication No. WO 94/16101; Cohen, et al., 1996. Adv. Chromatography 36: 127-162; and Griffin, et al., 1993. Appl. Biochem. Biotechnol. 38: 147-159).

Other methods for detecting mutations in the FCTRX gene include methods in which protection from cleavage agents is used to detect mismatched bases in RNA/RNA or RNA/DNA heteroduplexes. *See, e.g.,* Myers, et al., 1985. Science 230: 1242. In general, the art technique of "mismatch cleavage" starts by providing heteroduplexes of formed by hybridizing (labeled) RNA or DNA containing the wild-type FCTRX sequence with potentially mutant RNA or DNA obtained from a tissue sample. The double-stranded duplexes are treated with an agent that cleaves single-stranded regions of the duplex such as which will exist due to basepair mismatches between the control and sample strands. For instance, RNA/DNA duplexes can be treated with RNase and DNA/DNA hybrids treated with S₁ nuclease to enzymatically digesting the mismatched regions. In other embodiments, either DNA/DNA or RNA/DNA duplexes can be treated with hydroxylamine or osmium tetroxide and with piperidine in order to digest mismatched regions. After digestion of the mismatched regions, the resulting material is then separated by size on denaturing polyacrylamide gels to determine the site of mutation. *See, e.g.,* Cotton, et al., 1988. Proc. Natl. Acad. Sci. USA 85: 4397; Saleeba, et al., 1992. Methods Enzymol. 217: 286-295. In an embodiment, the control DNA or RNA can be labeled for detection.

In still another embodiment, the mismatch cleavage reaction employs one or more proteins that recognize mismatched base pairs in double-stranded DNA (so called "DNA mismatch repair" enzymes) in defined systems for detecting and mapping point mutations in FCTRX cDNAs obtained from samples of cells. For example, the mutY enzyme of *E. coli* cleaves A at G/A mismatches and the thymidine DNA glycosylase from HeLa cells cleaves T at G/T mismatches. *See, e.g.,* Hsu, et al., 1994. Carcinogenesis 15: 1657-1662. According to an exemplary embodiment, a probe based on an FCTRX sequence, *e.g*., a wild-type FCTRX sequence, is hybridized to a cDNA or other DNA product from a test cell(s). The duplex is treated with a DNA mismatch repair enzyme, and the cleavage products, if any, can be detected from electrophoresis protocols or the like. *See, e.g.,* U.S. Patent No. 5,459,039.

In other embodiments, alterations in electrophoretic mobility will be used to identify mutations in FCTRX genes. For example, single strand conformation polymorphism (SSCP) may be used to detect differences in electrophoretic mobility between mutant and wild type nucleic acids. *See, e.g.,* Orita, et al., 1989. Proc. Natl. Acad. Sci. USA: 86: 2766; Cotton, 1993. Mutat. Res. 285: 125-144; Hayashi, 1992. Genet. Anal. Tech. Appl. 9: 73-79. Single-stranded DNA fragments of sample and control FCTRX nucleic acids will be denatured and allowed to renature. The secondary structure of single-stranded nucleic acids varies according to sequence, the resulting alteration in electrophoretic mobility enables the detection of even a single base change. The DNA fragments may be labeled or detected with labeled probes. The sensitivity of the assay may be enhanced by using RNA (rather than DNA), in which the secondary structure is more sensitive to a change in sequence. In one embodiment, the subject method utilizes heteroduplex analysis to separate double stranded heteroduplex molecules on the basis of changes in electrophoretic mobility. *See, e.g.,* Keen, et al., 1991. Trends Genet. 7: 5.

In yet another embodiment, the movement of mutant or wild-type fragments in polyacrylamide gels containing a gradient of denaturant is assayed using denaturing gradient gel electrophoresis (DGGE). *See, e.g.,* Myers, et al., 1985. Nature 313: 495. When DGGE is used as the method of analysis, DNA will be modified to insure that it does not completely denature, for example by adding a GC clamp of approximately 40 bp of high-melting GC-rich DNA by PCR. In a further embodiment, a temperature gradient is used in place of a denaturing gradient to identify differences in the mobility of control and sample DNA. *See, e.g.,* Rosenbaum and Reissner, 1987. Biophys. Chem. 265: 12753.

Examples of other techniques for detecting point mutations include, but are not limited to, selective oligonucleotide hybridization, selective amplification, or selective primer extension. For example, oligonucleotide primers may be prepared in which the known mutation is placed centrally and then hybridized to target DNA under conditions that permit hybridization only if a perfect match is found. *See, e.g.,* Saiki, et al., 1986. Nature 324: 163; Saiki, et al., 1989. Proc. Natl. Acad. Sci. USA 86: 6230. Such allele specific oligonucleotides are hybridized to PCR amplified target DNA or a number of different mutations when the oligonucleotides are attached to the hybridizing membrane and hybridized with labeled target DNA.

Alternatively, allele specific amplification technology that depends on selective PCR amplification may be used in conjunction with the instant invention. Oligonucleotides used as primers for specific amplification may carry the mutation of interest in the center of the molecule (so that amplification depends on differential hybridization; *see, e.g.,* Gibbs, et al., 1989. Nucl. Acids Res. 17: 2437-2448) or at the extreme 3'-terminus of one primer where, under appropriate conditions, mismatch can prevent, or reduce polymerase extension (*see, e.g.,* Prossner, 1993. Tibtech. 11: 238). In addition it may be desirable to introduce a novel restriction site in the region of the mutation to create cleavage-based detection. *See, e.g.,* Gasparini, et al., 1992. Mol. Cell Probes 6: 1. It is anticipated that in certain embodiments amplification may also be performed using *Taq* ligase for amplification. *See, e.g.,* Barany, 1991. Proc. Natl. Acad. Sci. USA 88: 189. In such cases, ligation will occur only if there is a perfect match at the 3'-terminus of the 5' sequence, making it possible to detect the presence of a known mutation at a specific site by looking for the presence or absence of amplification.

The methods described herein may be performed, for example, by utilizing pre-packaged diagnostic kits comprising at least one probe nucleic acid or antibody reagent described herein, which may be conveniently used, *e.g*., in clinical settings to diagnose patients exhibiting symptoms or family history of a disease or illness involving an FCTRX gene.

Furthermore, any cell type or tissue, preferably peripheral blood leukocytes, in which FCTRX is expressed may be utilized in the prognostic assays described herein. However, any biological sample containing nucleated cells may be used, including, for example, buccal mucosal cells.

### Pharmacogenomics

Agents, or modulators that have a stimulatory or inhibitory effect on FCTRX activity (*e.g*., FCTRX gene expression), as identified by a screening assay described herein can be administered to individuals to treat (prophylactically or therapeutically) disorders (The disorders include metabolic disorders, Also within the scope of the invention is the use of a Therapeutic in the manufacture of a medicament for treating or preventing disorders or syndromes including, *e.g*., Colorectal cancer, adenomatous polyposis coli, myelogenous leukemia, congenital ceonatal alloimmune thrombocytopenia, multiple human solid malignancies, malignant ovarian tumours particularly at the interface between epithelia and stroma, malignant brain tumors, mammary tumors, human gliomas, astrocytomas, mixed glioma/astrocytomas, renal cells carcinoma, breast adenocarcinoma, ovarian cancer, melanomas, renal cell carcinoma, clear cell and granular cell carcinomas, autocrine/paracrine stimulation of tumor cell proliferation, autocrine/paracrine stimulation of tumor cell survival and tumor cell resistance to cytotoxic therapy, paranechmal and basement membrane invasion and motility of tumor cells thereby contributing to metastasis, tumor-mediated immunosuppression of T-cell mediated immune effector cells and pathways resulting in tumor escape from immune surveilance, neurological disorders, neurodegenerative disorders, nerve trauma, familial myelodysplastic syndrome, Charcot-Marie-Tooth neuropathy, demyelinating Gardner syndrome, familial myelodysplastic syndrome; mental health conditions, immunological disorders, allergy and infection, asthma, bronchial asthma, Avellino type eosinophilia, lung diseases, reproductive disorders, male infertility, female reproductive system disorders, male and female reproductive diseases, hemangioma, deafness, glycoprotein Ia deficiency, desmoid disease, turcot syndrome, liver cirrhosis, hepatitis C, gastric disorders, pancreatic diseases like diabetes, Schistosoma mansoni infection, Spinocerebellar ataxia, Plasmodium falciparum parasitemia, Corneal dystrophy -Groenouw type I, Corneal dystrophy - lattice type I, and Reis-Bucklers corneal dystrophy) In conjunction with such treatment, the pharmacogenomics (*i.e*., the study of the relationship between an individual's genotype and that individual's response to a foreign compound or drug) of the individual may be considered. Differences in metabolism of therapeutics can lead to severe toxicity or therapeutic failure by altering the relation between dose and blood concentration of the pharmacologically active drug. Thus, the pharmacogenomics of the individual permits the selection of effective agents (*e.g*., drugs) for prophylactic or therapeutic treatments based on a consideration of the individual's genotype. Such pharmacogenomics can further be used to determine appropriate dosages and therapeutic regimens. Accordingly, the activity of FCTRX protein, expression of FCTRX nucleic acid, or mutation content of FCTRX genes in an individual can be determined to thereby select appropriate agent(s) for therapeutic or prophylactic treatment of the individual.

Pharmacogenomics deals with clinically significant hereditary variations in the response to drugs due to altered drug disposition and abnormal action in affected persons. See *e.g*., Eichelbaum, 1996. Clin. Exp. Pharmacol. Physiol., 23: 983-985; Linder, 1997. Clin. Chem., 43: 254-266. In general, two types of pharmacogenetic conditions can be differentiated. Genetic conditions transmitted as a single factor altering the way drugs act on the body (altered drug action) or genetic conditions transmitted as single factors altering the way the body acts on drugs (altered drug metabolism). These pharmacogenetic conditions can occur either as rare defects or as polymorphisms. For example, glucose-6-phosphate dehydrogenase (G6PD) deficiency is a common inherited enzymopathy in which the main clinical complication is hemolysis after ingestion of oxidant drugs (anti-malarials, sulfonamides, analgesics, nitrofurans) and consumption of fava beans.

As an illustrative embodiment, the activity of drug metabolizing enzymes is a major determinant of both the intensity and duration of drug action. The discovery of genetic polymorphisms of drug metabolizing enzymes (*e.g*., N-acetyltransferase 2 (NAT 2) and cytochrome P450 enzymes CYP2D6 and CYP2C19) has provided an explanation as to why some patients do not obtain the expected drug effects or show exaggerated drug response and serious toxicity after taking the standard and safe dose of a drug. These polymorphisms are expressed in two phenotypes in the population, the extensive metabolizer (EM) and poor metabolizer (PM). The prevalence of PM is different among different populations. For example, the gene coding for CYP2D6 is highly polymorphic and several mutations have been identified in PM, which all lead to the absence of functional CYP2D6. Poor metabolizers of CYP2D6 and CYP2C19 quite frequently experience exaggerated drug response and side effects when they receive standard doses. If a metabolite is the active therapeutic moiety, PM show no therapeutic response, as demonstrated for the analgesic effect of codeine mediated by its CYP2D6-formed metabolite morphine. At the other extreme are the so called ultra-rapid metabolizers who do not respond to standard doses. Recently, the molecular basis of ultra-rapid metabolism has been identified to be due to CYP2D6 gene amplification.

Thus, the activity of FCTRX protein, expression of FCTRX nucleic acid, or mutation content of FCTRX genes in an individual can be determined to thereby select appropriate agent(s) for therapeutic or prophylactic treatment of the individual. In addition, pharmacogenetic studies can be used to apply genotyping of polymorphic alleles encoding drug-metabolizing enzymes to the identification of an individual's drug responsiveness phenotype. This knowledge, when applied to dosing or drug selection, can avoid adverse reactions or therapeutic failure and thus enhance therapeutic or prophylactic efficiency when treating a subject with an FCTRX modulator, such as a modulator identified by one of the exemplary screening assays described herein.

### Monitoring of Effects During Clinical Trials

Monitoring the influence of agents (*e.g.,* drugs, compounds) on the expression or activity of FCTRX (*e.g.,* the ability to modulate aberrant cell proliferation and/or differentiation) can be applied not only in basic drug screening, but also in clinical trials. For example, the effectiveness of an agent determined by a screening assay as described herein to increase FCTRX gene expression, protein levels, or upregulate FCTRX activity, can be monitored in clinical trails of subjects exhibiting decreased FCTRX gene expression, protein levels, or downregulated FCTRX activity. Alternatively, the effectiveness of an agent determined by a screening assay to decrease FCTRX gene expression, protein levels, or downregulate FCTRX activity, can be monitored in clinical trails of subjects exhibiting increased FCTRX gene expression, protein levels, or upregulated FCTRX activity. In such clinical trials, the expression or activity of FCTRX and, preferably, other genes that have been implicated in, for example, a cellular proliferation or immune disorder can be used as a "read out" or markers of the immune responsiveness of a particular cell.

By way of example, and not of limitation, genes, including FCTRX, that are modulated in cells by treatment with an agent (*e.g.,* compound, drug or small molecule) that modulates FCTRX activity (*e.g*., identified in a screening assay as described herein) can be identified. Thus, to study the effect of agents on cellular proliferation disorders, for example, in a clinical trial, cells can be isolated and RNA prepared and analyzed for the levels of expression of FCTRX and other genes implicated in the disorder. The levels of gene expression (*i.e.,* a gene expression pattern) can be quantified by Northern blot analysis or RT-PCR, as described herein, or alternatively by measuring the amount of protein produced, by one of the methods as described herein, or by measuring the levels of activity of FCTRX or other genes. In this manner, the gene expression pattern can serve as a marker, indicative of the physiological response of the cells to the agent. Accordingly, this response state may be determined before, and at various points during, treatment of the individual with the agent.

In one embodiment, the invention provides a method for monitoring the effectiveness of treatment of a subject with an agent (*e.g*., an agonist, antagonist, protein, peptide, peptidomimetic, nucleic acid, small molecule, or other drug candidate identified by the screening assays described herein) comprising the steps of (i) obtaining a pre-administration sample from a subject prior to administration of the agent; (*ii*) detecting the level of expression of an FCTRX protein, mRNA, or genomic DNA in the preadministration sample; (*iii*) obtaining one or more post-administration samples from the subject; (iv) detecting the level of expression or activity of the FCTRX protein, mRNA, or genomic DNA in the post-administration samples; (*v*) comparing the level of expression or activity of the FCTRX protein, mRNA, or genomic DNA in the pre-administration sample with the FCTRX protein, mRNA, or genomic DNA in the post administration sample or samples; and (vi) altering the administration of the agent to the subject accordingly. For example, increased administration of the agent may be desirable to increase the expression or activity of FCTRX to higher levels than detected, *i.e.,* to increase the effectiveness of the agent Alternatively, decreased administration of the agent may be desirable to decrease expression or activity of FCTRX to lower levels than detected, *i.e*., to decrease the effectiveness of the agent.

### Methods of Treatment

The invention provides for both prophylactic and therapeutic methods of treating a subject at risk of (or susceptible to) a disorder or having a disorder associated with aberrant FCTRX expression or activity. The disorders include cardiomyopathy, atherosclerosis, hypertension, congenital heart defects, aortic stenosis, atrial septal defect (ASD), atrioventricular (A-V) canal defect, ductus arteriosus, pulmonary stenosis, subaortic stenosis, ventricular septal defect (VSD), valve diseases, tuberous sclerosis, scleroderma, obesity, transplantation, adrenoleukodystrophy, congenital adrenal hyperplasia, prostate cancer, neoplasm; adenocarcinoma, lymphoma, uterus cancer, fertility, hemophilia, hypercoagulation, idiopathic thrombocytopenic purpura, immunodeficiencies, graft versus host disease, AIDS, bronchial asthma, Crohn's disease; multiple sclerosis, treatment of Albright Hereditary Ostoeodystrophy, and other diseases, disorders and conditions of the like.

These methods of treatment will be discussed more fully, below.

### Disease and Disorders

Diseases and disorders that are characterized by increased (relative to a subject not suffering from the disease or disorder) levels or biological activity may be treated with Therapeutics that antagonize (*i.e*., reduce or inhibit) activity. Therapeutics that antagonize activity may be administered in a therapeutic or prophylactic manner. Therapeutics that may be utilized include, but are not limited to: (*i*) an aforementioned peptide, or analogs, derivatives, fragments or homologs thereof; (*ii*) antibodies to an aforementioned peptide; (*iii*) nucleic acids encoding an aforementioned peptide; (*iv*) administration of antisense nucleic acid and nucleic acids that are "dysfunctional" (*i.e.,* due to a heterologous insertion within the coding sequences of coding sequences to an aforementioned peptide) that are utilized to "knockout" endoggenous function of an aforementioned peptide by homologous recombination (*see, e.g.,* Capecchi, 1989. Science 244: 1288-1292); or (v) modulators (*i.e.,* inhibitors, agonists and antagonists, including additional peptide mimetic of the invention or antibodies specific to a peptide of the invention) that alter the interaction between an aforementioned peptide and its binding partner.

Diseases and disorders that are characterized by decreased (relative to a subject not suffering from the disease or disorder) levels or biological activity may be treated with Therapeutics that increase (*i.e.,* are agonists to) activity. Therapeutics that upregulate activity may be administered in a therapeutic or prophylactic manner. Therapeutics that may be utilized include, but are not limited to, an aforementioned peptide, or analogs, derivatives, fragments or homologs thereof; or an agonist that increases bioavailability.

Increased or decreased levels can be readily detected by quantifying peptide and/or RNA, by obtaining a patient tissue sample (*e.g*., from biopsy tissue) and assaying it *in vitro* for RNA or peptide levels, structure and/or activity of the expressed peptides (or mRNAs of an aforementioned peptide). Methods that are well-known within the art include, but are not limited to, immunoassays (*e.g.,* by Western blot analysis, immunoprecipitation followed by sodium dodecyl sulfate (SDS) polyacrylamide gel electrophoresis, immunocytochemistry, etc.) and/or hybridization assays to detect expression of mRNAs (*e.g*., Northern assays, dot blots, *in situ* hybridization, and the like).

### Prophylactic Methods

In one aspect, the invention provides a method for preventing, in a subject, a disease or condition associated with an aberrant FCTRX expression or activity, by administering to the subject an agent that modulates FCTRX expression or at least one FCTRX activity. Subjects at risk for a disease that is caused or contributed to by aberrant FCTRX expression or activity can be identified by, for example, any or a combination of diagnostic or prognostic assays as described herein. Administration of a prophylactic agent can occur prior to the manifestation of symptoms characteristic of the FCTRX aberrancy, such that a disease or disorder is prevented or, alternatively, delayed in its progression. Depending upon the type of FCTRX aberrancy, for example, an FCTRX agonist or FCTRX antagonist agent can be used for treating the subject. The appropriate agent can be determined based on screening assays described herein. The prophylactic methods of the invention are further discussed in the following subsections.

### Therapeutic Methods

Another aspect of the invention pertains to methods of modulating FCTRX expression or activity for therapeutic purposes. The modulatory method of the invention involves contacting a cell with an agent that modulates one or more of the activities of FCTRX protein activity associated with the cell. An agent that modulates FCTRX protein activity can be an agent as described herein, such as a nucleic acid or a protein, a naturally-occurring cognate ligand of an FCTRX protein, a peptide, an FCTRX peptidomimetic, or other small molecule. In one embodiment, the agent stimulates one or more FCTRX protein activity. Examples of such stimulatory agents include active FCTRX protein and a nucleic acid molecule encoding FCTRX that has been introduced into the cell. In another embodiment, the agent inhibits one or more FCTRX protein activity. Examples of such inhibitory agents include antisense FCTRX nucleic acid molecules and anti-FCTRX antibodies. These modulatory methods can be performed *in vitro* (*e.g.,* by culturing the cell with the agent) or, alternatively, *in vivo* (*e.g.,* by administering the agent to a subject). As such, the invention provides methods of treating an individual afflicted with a disease or disorder characterized by aberrant expression or activity of an FCTRX protein or nucleic acid molecule. In one embodiment, the method involves administering an agent (*e.g.*, an agent identified by a screening assay described herein), or combination of agents that modulates (*e.g*., up-regulates or down-regulates) FCTRX expression or activity. In another embodiment, the method involves administering an FCTRX protein or nucleic acid molecule as therapy to compensate for reduced or aberrant FCTRX expression or activity.

Stimulation of FCTRX activity is desirable in situations in which FCTRX is abnormally downregulated and/or in which increased FCTRX activity is likely to have a beneficial effect. One example of such a situation is where a subject has a disorder characterized by aberrant cell proliferation and/or differentiation (*e.g.,* cancer or immune associated disorders). Another example of such a situation is where the subject has a gestational disease (*e.g*., preclampsia).

### Determination of the Biological Effect of the Therapeutic

In various embodiments of the invention, suitable *in vitro* or *in vivo* assays are performed to determine the effect of a specific Therapeutic and whether its administration is indicated for treatment of the affected tissue.

In various specific embodiments, *in vitro* assays may be performed with representative cells of the type(s) involved in the patient's disorder, to determine if a given Therapeutic exerts the desired effect upon the cell type(s). Compounds for use in therapy may be tested in suitable animal model systems including, but not limited to rats, mice, chicken, cows, monkeys, rabbits, and the like, prior to testing in human subjects. Similarly, for *in vivo* testing, any of the animal model system known in the art may be used prior to administration to human subjects.

### Prophylactic and Therapeutic Uses of the Compositions of the Invention

The FCTRX nucleic acids and proteins of the invention are useful in potential prophylactic and therapeutic applications implicated in a variety of disorders including, but not limited to: Also within the scope of the invention is the use of a Therapeutic in the manufacture of a medicament for treating or preventing disorders or syndromes including, *e.g*., Colorectal cancer, adenomatous polyposis coli, myelogenous leukemia, congenital ceonatal alloimmune thrombocytopenia, multiple human solid malignancies, malignant ovarian tumours particularly at the interface between epithelia and stroma, malignant brain tumors, mammary tumors, human gliomas, astrocytomas, mixed glioma/astrocytomas, renal cells carcinoma, breast adenocarcinoma, ovarian cancer, melanomas, renal cell carcinoma, clear cell and granular cell carcinomas, autocrine/paracrine stimulation of tumor cell proliferation, autocrine/paracrine stimulation of tumor cell survival and tumor cell resistance to cytotoxic therapy, paranechmal and basement membrane invasion and motility of tumor cells thereby contributing to metastasis, tumor-mediated immunosuppression of T-cell mediated immune effector cells and pathways resulting in tumor escape from immune surveilance, neurological disorders, neurodegenerative disorders, nerve trauma, familial myelodysplastic syndrome, Charcot-Marie-Tooth neuropathy, demyelinating Gardner syndrome, familial myelodysplastic syndrome; mental health conditions, immunological disorders, allergy and infection, asthma, bronchial asthma, Avellino type eosinophilia, lung diseases, reproductive disorders, male infertility, female reproductive system disorders, male and female reproductive diseases, hemangioma, deafness, glycoprotein Ia deficiency, desmoid disease, turcot syndrome, liver cirrhosis, hepatitis C, gastric disorders, pancreatic diseases like diabetes, Schistosoma mansoni infection, Spinocerebellar ataxia, Plasmodium falciparum parasitemia, Corneal dystrophy -Groenouw type I, Corneal dystrophy - lattice type I, and Reis-Bucklers corneal dystrophy.

As an example, a cDNA encoding the FCTRX protein of the invention may be useful in gene therapy, and the protein may be useful when administered to a subject in need thereof. By way of non-limiting example, the compositions of the invention will have efficacy for treatment of patients suffering from: Also within the scope of the invention is the use of a Therapeutic in the manufacture of a medicament for treating or preventing disorders or syndromes including, *e.g.,* Colorectal cancer, adenomatous polyposis coli, myelogenous leukemia, congenital ceonatal alloimmune thrombocytopenia, multiple human solid malignancies, malignant ovarian tumours particularly at the interface between epithelia and stroma, malignant brain tumors, mammary tumors, human gliomas, astrocytomas, mixed glibma/astrocytomas, renal cells carcinoma, breast adenocarcinoma, ovarian cancer, melanomas, renal cell carcinoma, clear cell and granular cell carcinomas, autocrine/paracrine stimulation of tumor cell proliferation, autocrine/paracrine stimulation of tumor cell survival and tumor cell resistance to cytotoxic therapy, paranechmal and basement membrane invasion and motility of tumor cells thereby contributing to metastasis, tumor-mediated immunosuppression of T-cell mediated immune effector cells and pathways resulting in tumor escape from immune surveilance, neurological disorders, neurodegenerative disorders, nerve trauma, familial myelodysplastic syndrome, Charcot-Marie-Tooth neuropathy, demyelinating Gardner syndrome, familial myelodysplastic syndrome; mental health conditions, immunological disorders, allergy and infection, asthma, bronchial asthma, Avellino type eosinophilia, lung diseases, reproductive disorders, male infertility, female reproductive system disorders, male and female reproductive diseases, hemangioma, deafness, glycoprotein Ia deficiency, desmoid disease, turcot syndrome, liver cirrhosis, hepatitis C, gastric disorders, pancreatic diseases like diabetes, Schistosoma mansoni infection, Spinocerebellar ataxia, Plasmodium falciparum parasitemia, Corneal dystrophy -Groenouw type I, Corneal dystrophy - lattice type I, and Reis-Bucklers corneal dystrophy.

Both the novel nucleic acid encoding the FCTRX protein, and the FCTRX protein of the invention, or fragments thereof, may also be useful in diagnostic applications, wherein the presence or amount of the nucleic acid or the protein are to be assessed. A further use could be as an anti-bacterial molecule (*i.e*., some peptides have been found to possess anti-bacterial properties). These materials are further useful in the generation of antibodies which immunospecifically-bind to the novel substances of the invention for use in therapeutic or diagnostic methods.

### EXAMPLES

The following examples illustrate by way of non-limiting example various aspects of the invention.

The following examples illustrate by way of non-limiting example various aspects of the invention.

### Example 1: Method of Identifying the Nucleic Acids

The novel nucleic acids of the invention were identified by TblastN using a proprietary sequence file, run against the Genomic Daily Files made available by GenBank. The nucleic acids were further predicted by the proprietary software program GenScan^{™}, including selection of exons. These were further modified by means of similarities using BLAST searches. The sequences were then manually corrected for apparent inconsistencies, thereby obtaining the sequences encoding the full-length proteins.

### Example 2. Quantitative expression analysis of FCTR2 in various cells and tissues

The quantitative expression of various clones was assessed using microtiter plates containing RNA samples from a variety of normal and pathology-derived cells, cell lines and tissues using real time quantitative PCR (RTQ PCR; TAQMAN^{®}). RTQ PCR was performed on a Perkin-Elmer Biosystems ABI PRISM® 7700 Sequence Detection System. Various collections of samples are assembled on the plates, and referred to as Panel 1 (containing cells and cell lines from normal and cancer sources), Panel 2 (containing samples derived from tissues, in particular from surgical samples, from normal and cancer sources), Panel 3 (containing samples derived from a wide variety of cancer sources) and Panel 4 (containing cells and cell lines from normal cells and cells related to inflammatory conditions).

First, the RNA samples were normalized to constitutively expressed genes such as β-actin and GAPDH. RNA (~50 ng total or ~1 ng polyA+) was converted to cDNA using the TAQMAN® Reverse Transcription Reagents Kit (PE Biosystems, Foster City, CA; Catalog No. N808-0234) and random hexamers according to the manufacturer's protocol. Reactions were performed in 20 ul and incubated for 30 min. at 48°C. cDNA (5 ul) was then transferred to a separate plate for the TAQMAN® reaction using β-actin and GAPDH TAQMAN® Assay Reagents (PE Biosystems; Catalog Nos. 4310881E and 4310884E, respectively) and TAQMAN® universal PCR Master Mix (PE Biosystems; Catalog No. 4304447) according to the manufacturer's protocol. Reactions were performed in 25 ul using the following parameters: 2 min. at 50°C; 10 min. at 95°C; 15 sec. at 95°C/1 min. at 60°C (40 cycles). Results were recorded as CT values (cycle at which a given sample crosses a threshold level of fluorescence) using a log scale, with the difference in RNA concentration between a given sample and the sample with the lowest CT value being represented as 2 to the power of delta CT. The percent relative expression is then obtained by taking the reciprocal of this RNA difference and multiplying by 100. The average CT values obtained for β-actin and GAPDH were used to normalize RNA samples. The RNA sample generating the highest CT value required no further diluting, while all other samples were diluted relative to this sample according to their β-actin /GAPDH average CT values.

Normalized RNA (5 ul) was converted to cDNA and analyzed via TAQMAN® using One Step RT-PCR Master Mix Reagents (PE Biosystems; Catalog No. 4309169) and gene-specific primers according to the manufacturer's instructions. Probes and primers were designed for each assay according to Perkin Elmer Biosystem's *Primer Express* Software package (version I for Apple Computer's Macintosh Power PC) or a similar algorithm using the target sequence as input. Default settings were used for reaction conditions and the following parameters were set before selecting primers: primer concentration = 250 nM, primer melting temperature (Tₘ) range = 58°-60° C, primer optimal Tm = 59° C, maximum primer difference = 2° C, probe does not have 5' G, probe Tₘ must be 10° C greater than primer Tₘ, amplicon size 75 bp to 100 bp. The probes and primers selected (see below) were synthesized by Synthegen (Houston, TX, USA). Probes were double purified by HPLC to remove uncoupled dye and evaluated by mass spectroscopy to verify coupling of reporter and quencher dyes to the 5' and 3' ends of the probe, respectively. Their final concentrations were: forward and reverse primers, 900 nM each, and probe, 200nM.

PCR conditions: Normalized RNA from each tissue and each cell line was spotted in each well of a 96 well PCR plate (Perkin Elmer Biosystems). PCR cocktails including two probes (a probe specific for the target clone and another gene-specific probe multiplexed with the target probe) were set up using 1X TaqMan^{™} PCR Master Mix for the PE Biosystems 7700, with 5 mM MgC12, dNTPs (dA, G, C, U at 1:1:1:2 ratios), 0.25 U/ml AmpliTaq Gold^{™} (PE Biosystems), and 0.4 U/µl RNase inhibitor, and 0.25 U/µl reverse transcriptase. Reverse transcription was performed at 48° C for 30 minutes followed by amplification/PCR cycles as follows: 95° C 10 min, then 40 cycles of 95° C for 15 seconds, 60° C for 1 minute.

In the results for Panel 1, the following abbreviations are used:
ca. = carcinoma,
* = established from metastasis,
met = metastasis,
s cell var= small cell variant,
non-s = non-sm =non-small,
squam = squamous,
pl. eff = pl effusion = pleural effusion,
glio = glioma,
astro = astrocytoma, and
neuro = neuroblastoma.

### Panel 2

The plates for Panel 2 generally include 2 control wells and 94 test samples composed ofRNA or cDNA isolated from human tissue procured by surgeons working in close cooperation with the National Cancer Institute's Cooperative Human Tissue Network (CHTN) or the National Disease Research Initiative (NDRI). The tissues are derived from human malignancies and in cases where indicated many malignant tissues have "matched margins" obtained from noncancerous tissue just adjacent to the tumor. These are termed normal adjacent tissues and are denoted "NAT" in the results below. The tumor tissue and the "matched margins" are evaluated by two independent pathologists (the surgical pathologists and again by a pathologists at NDRI or CHTN). This analysis provides a gross histopathological assessment of tumor differentiation grade. Moreover, most samples include the original surgical pathology report that provides information regarding the clinical stage of the patient. These matched margins are taken from the tissue surrounding (i.e. immediately proximal) to the zone of surgery (designated "NAT", for normal adjacent tissue, in Table RR). In addition, RNA and cDNA samples were obtained from various human tissues derived from autopsies performed on elderly people or sudden death victims (accidents, etc.). These tissue were ascertained to be free of disease and were purchased from various commercial sources such as Clontech (Palo Alto, CA), Research Genetics, and Invitrogen.

RNA integrity from all samples is controlled for quality by visual assessment of agarose gel electropherograms using 28S and 18S ribosomal RNA staining intensity ratio as a guide (2:1 to 2.5:1 28s:18s) and the absence of low molecular weight RNAs that would be indicative of degradation products. Samples are controlled against genomic DNA contamination by RTQ PCR reactions run in the absence of reverse transcriptase using probe and primer sets designed to amplify across the span of a single exon.

### Panel 4

Panel 4 includes samples on a 96 well plate (2 control wells, 94 test samples) composed of RNA (Panel 4r) or cDNA (Panel 4d) isolated from various human cell lines or tissues related to inflammatory conditions. Total RNA from control normal tissues such as colon and lung (Stratagene ,La Jolla, CA) and thymus and kidney (Clontech) were employed. Total RNA from liver tissue from cirrhosis patients and kidney from lupus patients was obtained from BioChain (Biochain Institute, Inc., Hayward, CA). Intestinal tissue for RNA preparation from patients diagnosed as having Crohn's disease and ulcerative colitis was obtained from the National Disease Research Interchange (NDRI) (Philadelphia, PA).

Astrocytes, lung fibroblasts, dermal fibroblasts, coronary artery smooth muscle cells, small airway epithelium, bronchial epithelium, microvascular dermal endothelial cells, microvascular lung endothelial cells, human pulmonary aortic endothelial cells, human umbilical vein endothelial cells were all purchased from Clonetics (Walkersville, MD) and grown in the media supplied for these cell types by Clonetics. These primary cell types were activated with various cytokines or combinations of cytokines for 6 and/or 12-14 hours, as indicated. The following cytokines were used; IL-1 beta at approximately 1-5 ng/ml, TNF alpha at approximately 5-10 ng/ml, IFN gamma at approximately 20-50 ng/ml, IL-4 at approximately 5-10 ng/ml, IL-9 at approximately 5-10 ng/ml, IL-13 at approximately 5-10 ng/ml. Endothelial cells were sometimes starved for various times by culture in the basal media from Clonetics with 0.1% serum.

Mononuclear cells were prepared from blood of employees at CuraGen Corporation, using Ficoll. LAK cells were prepared from these cells by culture in DMEM 5% FCS (Hyclone), 100 µM non essential amino acids (Gibco/Life Technologies, Rockville, MD), 1 mM sodium pyruvate (Gibco), mercaptoethanol 5.5 x 10⁻⁵ M (Gibco), and 10 mM Hepes (Gibco) and Interleukin 2 for 4-6 days. Cells were then either activated with 10-20 ng/ml PMA and 1-2 µg/ml ionomycin, IL-12 at 5-10 ng/ml, IFN gamma at 20-50 ng/ml and IL-18 at 5-10 ng/ml for 6 hours. In some cases, mononuclear cells were cultured for 4-5 days in DMEM 5% FCS (Hyclone), 100 µM non essential amino acids (Gibco), 1 mM sodium pyruvate (Gibco), mercaptoethanol 5.5 x 10⁻⁵ M (Gibco), and 10 mM Hepes (Gibco) with PHA (phytohemagglutinin) or PWM (pokeweed mitogen) at approximately 5 µg/ml. Samples were taken at 24, 48 and 72 hours for RNA preparation. MLR (mixed lymphocyte reaction) samples were obtained by taking blood from two donors, isolating the mononuclear cells using Ficoll and mixing the isolated mononuclear cells 1:1 at a final concentration of approximately 2x10⁶ cells/ml in DMEM 5% FCS (Hyclone), 100 µM non essential amino acids (Gibco), 1 mM sodium pyruvate (Gibco), mercaptoethanol (5.5 x 10⁻⁵ M) (Gibco), and 10 mM Hepes (Gibco). The MLR was cultured and samples taken at various time points ranging from 1- 7 days for RNA preparation.

Monocytes were isolated from mononuclear cells using CD14 Miltenyi Beads, +ve VS selection columns and a Vario Magnet according to the manufacturer's instructions. Monocytes were differentiated into dendritic cells by culture in DMEM 5% fetal calf serum (FCS) (Hyclone, Logan, UT), 100 µM non essential amino acids (Gibco), 1 mM sodium pyruvate (Gibco), mercaptoethanol 5.5 x 10⁻⁵ M (Gibco), and 10 mM Hepes (Gibco), 50 ng/ml GMCSF and 5 ng/ml IL-4 for 5-7 days. Macrophages were prepared by culture of monocytes for 5-7 days in DMEM 5% FCS (Hyclone),100 µM non essential amino acids (Gibco), 1 mM sodium pyruvate (Gibco), mercaptoethanol 5.5 x 10⁻⁵ M (Gibco), 10 mM Hepes (Gibco) and 10% AB Human Serum or MCSF at approximately 50 ng/ml. Monocytes, macrophages and dendritic cells were stimulated for 6 and 12-14 hours with lipopolysaccharide (LPS) at 100 ng/ml. Dendritic cells were also stimulated with anti-CD40 monoclonal antibody (Pharmingen) at 10 µg/ml for 6 and 12-14 hours.

CD4 lymphocytes, CD8 lymphocytes and NK cells were also isolated from mononuclear cells using CD4, CD8 and CD56 Miltenyi beads, positive VS selection columns and a Vario Magnet according to the manufacturer's instructions. CD45RA and CD45RO CD4 lymphocytes were isolated by depleting mononuclear cells of CD8, CD56, CD14 and CD19 cells using CD8, CD56, CD14 and CD19 Miltenyi beads and+ve selection. Then CD45RO beads were used to isolate the CD45RO CD4 lymphocytes with the remaining cells being CD45RA CD4 lymphocytes. CD45RA CD4, CD45RO CD4 and CD8 lymphocytes were placed in DMEM 5% FCS (Hyclone), 100 µM non essential amino acids (Gibco), 1 mM sodium pyruvate (Gibco), mercaptoethanol 5.5 x 10⁻⁵ M (Gibco), and 10 mM Hepes (Gibco) and plated at 10⁶ cells/ml onto Falcon 6 well tissue culture plates that had been coated overnight with 0.5 µg/ml anti-CD28 (Pharmingen) and 3 ug/ml anti-CD3 (OKT3, ATCC) in PBS. After 6 and 24 hours, the cells were harvested for RNA preparation. To prepare chronically activated CD8 lymphocytes, we activated the isolated CD8 lymphocytes for 4 days on anti-CD28 and anti-CD3 coated plates and then harvested the cells and expanded them in DMEM 5% FCS (Hyclone), 100 µM non essential amino acids (Gibco), 1 mM sodium pyruvate (Gibco), mercaptoethanol 5.5 x 10⁻⁵ M (Gibco), and 10 mM Hepes (Gibco) and IL-2. The expanded CD8 cells were then activated again with plate bound anti-CD3 and anti-CD28 for 4 days and expanded as before. RNA was isolated 6 and 24 hours after the second activation and after 4 days of the second expansion culture. The isolated NK cells were cultured in DMEM 5% FCS (Hyclone), 100 µM non essential amino acids (Gibco), 1 mM sodium pyruvate (Gibco), mercaptoethanol 5.5 x 10⁻⁵ M (Gibco), and 10 mM Hepes (Gibco) and IL-2 for 4-6 days before RNA was prepared.

To obtain B cells, tonsils were procured from NDRI. The tonsil was cut up with sterile dissecting scissors and then passed through a sieve. Tonsil cells were then spun down and resupended at 10⁶ cells/ml in DMEM 5% FCS (Hyclone), 100 µM non essential amino acids (Gibco), 1 mM sodium pyruvate (Gibco), mercaptoethanol 5.5 x 10⁻⁵ M (Gibco), and 10 mM Hepes (Gibco). To activate the cells, we used PWM at 5 µg/ml or anti-CD40 (Pharmingen) at approximately 10 µg/ml and IL-4 at 5-10 ng/ml. Cells were harvested for RNA preparation at 24,48 and 72 hours.

To prepare the primary and secondary Th1/Th2 and Tr1 cells, six-well Falcon plates were coated overnight with 10 µg/ml anti-CD28 (Pharmingen) and 2 µg/ml OKT3 (ATCC), and then washed twice with PBS. Umbilical cord blood CD4 lymphocytes (Poietic Systems, German Town, MD) were cultured at 10⁵ -10⁶ cells/ml in DMEM 5% FCS (Hyclone), 100 µM non essential amino acids (Gibco), 1 mM sodium pyruvate (Gibco), mercaptoethanol 5.5 x 10⁻⁵ M (Gibco), 10 mM Hepes (Gibco) and IL-2 (4 ng/ml). IL-12 (5 ng/ml) and anti-IL4 (1 µg/ml) were used to direct to Th1, while IL-4 (5 ng/ml) and anti-IFN gamma (1 µg/ml) were used to direct to Th2 and IL-10 at 5 ng/ml was used to direct to Tr1. After 4-5 days, the activated Th1, Th2 and Tr1 lymphocytes were washed once in DMEM and expanded for 4-7 days in DMEM 5% FCS (Hyclone), 100 µM non essential amino acids (Gibco), 1 mM sodium pyruvate (Gibco), mercaptoethanol 5.5 x 10⁻⁵ M (Gibco),10 mM Hepes (Gibco) and IL-2 (1 ng/ml). Following this, the activated Th1, Th2 and Tr1 lymphocytes were re-stimulated for 5 days with anti-CD28/OKT3 and cytokincs as described above, but with the addition of anti-CD95L (1 µg/ml) to prevent apoptosis. After 4-5 days, the Th1, Th2 and Tr1 lymphocytes were washed and then expanded again with IL-2 for 4-7 days. Activated Th1 and Th2 lymphocytes were maintained in this way for a maximum of three cycles. RNA was prepared from primary and secondary Th1, Th2 and Tr1 after 6 and 24 hours following the second and third activations with plate bound anti-CD3 and anti-CD28 mAbs and 4 days into the second and third expansion cultures in Interleukin 2.

The following leukocyte cells lines were obtained from the ATCC: Ramos, EOL-1, KU-812. EOL cells were further differentiated by culture in 0.1 mM dbcAMP at 5 x10⁵ cells/ml for 8 days, changing the media every 3 days and adjusting the cell concentration to 5 x10⁵ cells/ml. For the culture of these cells, we used DMEM or RPMI (as recommended by the ATCC), with the addition of 5% FCS (Hyclone), 100 µM non essential amino acids (Gibco), 1 mM sodium pyruvate (Gibco), mercaptoethanol 5.5 x 10⁻⁵ M (Gibco), 10 mM Hepes (Gibco). RNA was either prepared from resting cells or cells activated with PMA at 10 ng/ml and ionomycin at 1 µg/ml for 6 and 14 hours. Keratinocyte line CCD106 and an airway epithelial tumor line NCI-H292 were also obtained from the ATCC. Both were cultured in DMEM 5% FCS (Hyclone), 100 µM non essential amino acids (Gibco), 1 mM sodium pyruvate (Gibco), mercaptoethanol 5.5 x 10⁻⁵ M (Gibco), and 10 mM Hepes (Gibco). CCD1106 cells were activated for 6 and 14 hours with approximately 5 ng/ml TNF alpha and 1 ng/ml IL-1 beta, while NCI-H292 cells were activated for 6 and 14 hours with the following cytokines: 5 ng/ml IL-4, 5 ng/ml IL-9, 5 ng/ml IL-13 and 25 ng/ml IFN gamma.

For these cell lines and blood cells, RNA was prepared by lysing approximately 10⁷ cells/ml using Trizol (Gibco BRL). Briefly, 1/10 volume ofbromochloropropane (Molecular Research Corporation) was added to the RNA sample, vortexed and after 10 minutes at room temperature, the tubes were spun at 14,000 rpm in a Sorvall SS34 rotor. The aqueous phase was removed and placed in a 15 ml Falcon Tube. An equal volume of isopropanol was added and left at -20 degrees C overnight. The precipitated RNA was spun down at 9,000 rpm for 15 min in a Sorvall SS34 rotor and washed in 70% ethanol. The pellet was redissolved in 300 µl of RNAse-free water and 35 µl buffer (Promega) 5 µl DTT, 7 µl RNAsin and 8 µl DNAse were added. The tube was incubated at 37 degrees C for 30 minutes to remove contaminating genomic DNA, extracted once with phenol chloroform and reprecipitated with 1/10 volume of 3 M sodium acetate and 2 volumes of 100% ethanol. The RNA was spun down and placed in RNAse free water. RNA was stored at -80 degrees C.

The above detailed procedures were carried out to obtain the taqman profiles of the clones in question.

Given below are the Primers and the Taqman results for the following clones:
58092213.0.36 - Probe Name: Ag809 (Table 9 and Table 10)
29692275.0.1 - Probe Name: Ag2773 (Table 11 and Table 12)
32125243.0.21 - Probe Name: Ag427 (Table 13 and Table 14)
27455183.0.19 - Probe Name: Ag1541 (Table 15 and Table 16, 17, 18)

**Table 8: Primer Design for Probe Ag809 (FCTR1)**

| **Primer** | **Sequences** | **TM** | **Length** | **Start Pos** | **SEQID NO** |
|---|---|---|---|---|---|
| Forward | 5'-LATGTGATCTTTGGCTGTGAAGT-3' | 58.7 | 22 | 337 | 24 |
| Probe | FAM-5'-CTACCCCATGGCCTCCATCGAGT-3'-TAMRA | 69.4 | 23 | 365 | 25 |
| Reverse | 5'-GGATGTCCAAGCCATCCTT-3' | 59.9 | 19 | 393 | 26 |

**TABLE 9: TAQMAN RESULTS FOR FCTR1**

| **Tissue_Name** | **Panel** | **1 Tissue_Name** | **Panel 2D** | **Tissue_Name** | **Panel 4D** |
|---|---|---|---|---|---|
| Liver adenocarcinoma | 79.6 | Normal Colon GENPAK 061003 | 6.8 | 93768_Secondary Th1_anti-CD28/anti-CD3 | 2.0 |
| Heart (fetal) | 43.8 | 83219 CC Well to Mod Diff (OD03866) | 6.1 | 93769_Secondary Th2_anti-CD28/anti-CD3 | 1.5 |
| Pancreas | 2.1 | 83220 CC NAT (OD03866) | 2.5 | 93770_Secondary Tr1_anti-CD28/anti-CD3 | 2.5 |
| Pancreatic ca. CAPAN 2 | 4.7 | 83221 CC Gr.2 rectosigmoid (OD03868) | 0.9 | 93573_Secondary Th1_resting day 4-6 in IL-2 | 1.0 |
| Adrenal gland | 2.3 | 83222 CC NAT (OD03868) | 1.2 | 93572_Secondary Th2_resting day 4-6 in IL-2 | 3.0 |
| Thyroid | 6.5 | 83235 CC Mod Diff (ODO3920) | 3.8 | 93571_Secondary Tr1_resting day 4-6 in IL-2 | 1.7 |
| Salivary gland | 12.3 | 83236 CC NAT (ODO3920) | 1.3 | 93568_primary Th1_anti-CD28/anti-CD3 | 0.4 |
| Pituitary gland | 8.7 | 83237 CC Gr.2 ascend colon (ODO3921) | 6.9 | 93569_primary Th2_anti-CD28/anti-CD3 | 1.5 |
| Brain (fetal) | 0.0 | 83238 CC NAT (ODO3921) | 4.0 | 93570_primary Tr1_anti-CD28/anti-CD3 | 2.0 |
| Brain (whole) | 3.0 | 83241 CC from Partial Hepatectomy (OD04309) | 1.2 | 93565_primary Th1_resting dy 4-6 in IL-2 | 5.4 |
| Brain (amygdala) | 2.4 | 83242 Liver NAT (ODO4309) | 0.6 | 93566_primary Th2_resting dy 4-6 In IL-2 | 3.1 |
| Brain (cerebellum) | 0.0 | 87472 Colon mets to lung (OD04451- 01) | 4.4 | 93567_prlmary Tr1_resting dy 4-6 in IL-2 | 0.0 |
| Brain (hippocampus) | 13.0 | 87473 Lung NAT (OD04451-02) | 1.2 | 93351_CD45RA CD4 lymphocyte anti-CD28/anti-CD3 | 11.2 |
| Brain (thalamus) | 3.0 | Normal Prostate Clontech A+ 6546-1 | 10.2 | 93352_CD45R0 CD4 lymphocyte_anti-CD28/anti-CD3 | 1.2 |
| Cerebral Cortex | 2.3 | 84140 Prostate Cancer (OD04410) | 41.8 | 93251_CD8 Lymphocytes_anti-CD28/anti-CD3 | 0.9 |
| Spinal cord | 2.6 | 84141 Prostate NAT (OD04410) | 25.7 | 93353_chronic CD8 Lymphocytes 2ry_resting dy 4-6 In IL-2 | 0.0 |
| CNS ca. (glio/astro) U87- MG | 12.1 | 87073 Prostate Cancer (OD04720-01) | 11.0 | 93574_chronic CD8 Lymphocytes 2ry_activated CD3/CD28 | 0.6 |
| CNS ca. (glio/astro) U-118-MG | 100.0 | 87074 Prostate NAT (ODO4720- 02) | 10.0 | 93354_CD4_none | 1.1 |
| CNS ca. (astro) SW1783 | 6.5 | Normal Lung GENPAK 061010 | 7.9 | 93252_Secondary Th1/Th2/Tr1_anti-CD95 CH11 | 0.0 |
| CNS ca.* (neuro; met) SK-N-AS | 52.1 | 83239 Lung Met to Muscle (ODO4286) | 6.5 | 93103_LAK cells_resting | 0.5 |
| CNS ca. (astro) SF-539 | 12.6 | 83240 Muscle NAT (OD04286) | 2.6 | 93788_LAK cells_IL-2 | 0.0 |
| CNS ca. (astro) SNB-75 | 11.9 | 84136 Lung Malignant Cancer (OD03126) | 14.8 | 93787_LAK cells_IL-2+IL-12 | 0.7 |
| CNS ca. (glio)SNB-19 | 0.0 | 84137 Lung NAT (OD03126) | 3.2 | 93789_LAK cells IL-2+IFN gamma | 1.1 |
| CNS ca. (glio)U251 | 0.9 | 84871 Lung Cancer (OD04404) | 2.1 | 93790_LAK cells-IL-2+ IL-18 | 0.3 |
| CNS ca. (glio) SF-295 | 12.6 | 84872 Lung NAT (OD04404) | 1.9 | 93104_LAK cells_PMA/ionomycin and IL-18 | 0.0 |
| Heart | 13.9 | 84875 Lung Cancer (OD04565) | 0.3 | 93578_NK Cells IL-2_resting | 1.3 |
| Skeletal muscle | 3.2 | 85950 Lung Cancer (OD04237-01) | 1.3 | 93109_Mixed Lymphocyte Reaction_Two Way MLR | 0.5 |
| Bone marrow | 3.6 | 85970 Lung NAT (OD04237-02) | 2.6 | 93110_Mixed Lymphocyte Reaction_Two Way MLR | 0.5 |
| Thymus | 4.2 | 83255 Ocular Mel Met to Liver (OD04310) | 0.1 | 93111_Mixed Lymphocyte Reaction_Two Way MLR | 2.7 |
| Spleen | 61.6 | 83256 Liver NAT (OD04310) | 0.6 | 93112_Mononuclear Cells (PBMCs)_resting | 0.0 |
| Lymph node | 3.3 | 84139 Melanoma Mets to Lung (OD04321) | 2.5 | 93113_Mononuclear Cells (PBMCs)_PWM | 1.3 |
| Colorectal | 11.9 | 84138 Lung NAT (OD04321) | 2.6 | 93114_Mononuclear Cells (PBMCs)_PHA-L | 1.0 |
| Stomach | 28.3 | Normal Kidney GENPAK 061008 | 5.6 | 93249_Ramos (B cell)_none | 1.2 |
| Small intestine | 4.5 | 83786 Kidney Ca, Nuclear grade 2 (OD04338) | 0.6 | 93250_Ramos (B cell)_ionomycin | 2.3 |
| Colon ca. SW480 | 46.7 | 83787 Kidney NAT (OD04338) | 3.7 | 93349_B iymphocytes_PWM | 4.3 |
| Colon ca.* (SW480 met)SW620 | 19.0 | 83788 Kidney Ca Nuclear grade 1/2 (OD04339) | 0.8 | 93350_B lymphoytes_CD40L and IL-4 | 1.4 |
| Colon ca. HT29 | 5.3 | 83789 Kidney NAT (OD04339) | 3.1 | 92665_EOL-1 (Eosinophil)_dbcAMP differentiated | 7.2 |
| Colon ca. HCT- 116 | 5.0 | 83790 Kidney Ca, Clear cell type (OD04340) | 1.5 | 93248_EOL-1 (Eosinophil)_dbcAMP/PMAionomyc in | 3.0 |
| Colon ca. CaCo- 2 | 49.3 | 83791 Kidney NAT (OD04340) | 5.1 | 93356_Dendritic Cells_none | 1.5 |
| 83219 CC Well to Mod Diff (ODO3866) | 3.0 | 83792 Kidney Ca, Nuclear grade 3 (OD04348) | 14.5 | 93355_Dendritic Cells_LPS 100 ng/ml | 0.7 |
| Colon ca. HCC- 2998 | 27.7 | 83793 Kidney NAT (ODO4348) | 2.5 | 93775_Dendritic Cells_antl-C040 | 0.5 |
| Gastric ca.* (liver met) NCl- N87 | 10.5 | 87474 Kidney Cancer (OD04622-01) | 1.7 | 93774_Monocytes_resting | 0.5 |
| Bladder | 3.7 | 87475 Kidney NAT (OD04622-03) | 2.0 | 93776_Monocytes_LPS 50 ng/ml | 0.0 |
| Trachea | 23.5 | 85973 Kidney Cancer (OD04450-01) | 0.3 | 93581_Macrophages_resting | 1.3 |
| Kidney | 1.8 | 85974 Kidney NAT (OD04450-03) | 2.0 | 93582_Macrophages_LPS 100 ng/ml | 1.8 |
| Kidney (fetal) | 1.9 | Kidney Cancer Clontech 8120607 | 7.0 | 93098_HUVEC (Endothelial)_none | 2.3 |
| Renal ca. 786-0 | 7.0 | Kidney NAT Clontech 8120608 | 1.5 | 93099_HUVEC (Endothelial)_starved | 9.0 |
| Renal ca. A498 | 6.8 | Kidney Cancer Clontech 8120613 | 2.0 | 93100_HUVEC (Endothelial)_IL-1B | 1.2 |
| Renal ca.RXF 393 | 4.7 | Kidney NAT Clontech 8120614 | 4.1 | 93779_HUVEC (Endothelial)_IFN gamma | 1.4 |
| Renal ca.ACHN | 9.8 | Kidney Cancer Clontech 9010320 | 2.2 | 93102_HUVEC (Endothelial)_TNF alpha + IFN gamma | 0.8 |
| Renal ca.UO-31 | 1.3 | Kidney NAT Clontech 9010321 | 3.5 | 93101_HUVEC (Endothelial)_TNF alpha + IL4 | 1.1 |
| Renal ca.TK-10 | 0.6 | Normal Uterus GENPAK 061018 | 3.1 | 93781_HUVEC (Endothelial)_IL-11 | 3.0 |
| Liver | 0.8 | Uterus Cancer GENPAK 064011 | 17.6 | 93583_Lung Microvascular Endothelial Cells_none | 0.8 |
| Liver (fetal) | 1.1 | Normal Thyroid Clontech A+ 6570-1 | 3.7 | 93584_Lung Microvascular Endothelial Cells_TNFa (4 ng/ml) and IL1b (1 ng/ml) | 0.5 |
| Liver ca. (hepatoblast) HepG2 | 54.0 | Thyroid Cancer GENPAK 064010 | 1.2 | 92662_Microvascular Dermal endothelium_none | 1.1 |
| Lung | 3.9 | Thyroid Cancer INVITROGEN A302152 | 0.6 | 92663_Microsvasular Dermal endothelium_TNFa (4 ng/ml) and IL1b (1 ng/ml) | 1.0 |
| Lung (fetal) | 9.0 | Thyroid NAT INVITROGEN A302153 | 2.6 | 93773_Bronchial epithelium_TNFa (4 ng/ml) and IL1b (1 ng/ml) ** | 0.0 |
| Lung ca. (small cell) LX-1 | 34.4 | Normal Breast GENPAK 061019 | 3.4 | 93347_Small Airway Epithelium_none | 0.4 |
| Lung ca. (small cell) NCl-H69 | 3.0 | 84877 Breast Cancer (OD04566) | 0.9 | 93348_Small Airway Epithelium_TNFa (4 ng/ml) and IL1 b (1 nglml) | 0.5 |
| Lung ca. (s.cell var.) SHP-77 | 13.0 | 85975 Breast Cancer (OD04590-01) | 67.8 | 92668_Coronery Artery SMC_resting | 5.8 |
| Lung ca. (large cell)NCl-H460 | 6.8 | 85976 Breast Cancer Mets (OD04590-03) | 51.1 | 92669_Coronery Artery SMC_TNFa (4 ng/ml) and IL 1b (1 ng/ml) | 2.3 |
| Lung ca. (non-sm. cell) A549 | 3.4 | 87070 Breast Cancer Metastasis (OD04655-05) | 12.7 | 93107_astrocytes_resting | 2.7 |
| Lung ca. (non-s.cell) NCl-H23 | 34.4 | GENPAK Breast Cancer 064006 | 8.9 | 93108_astrocytes_TNFa (4 ng/ml) and IL1b (1 ng/ml) | 0.0 |
| Lung ca (non-s.cell) HOP-62 | 10.5 | Breast Cancer Clontech 9100266 | 6.2 | 92666_KU-812 (Basophil)_resting | 6.8 |
| Lung ca. (non-s.cl) NCl-H522 | 47.6 | Breast NAT Clontech 9100265 | 3.3 | 92667_KU-812 (Basophil)_PMA/ionoycin | 8.4 |
| Lung ca. (squam.) SW 900 | 4.7 | Breast Cancer INVITROGEN A209073 | 3.4 | 93579_CCD1106 (Keratinocytes)_none | 1.6 |
| Lung ca. (squam.) NCI-H596 | 0.7 | Breast NAT INVITROGEN A2090734 | 8.7 | 93580_CCD1106 (Keratinocytes)_TNFa and IFNg | ** 1.4 |
| Mammary gland | 9.9 | Normal Liver GENPAK 061009 | 1.1 | 93791_Liver Cirrhosis | 4.2 |
| Breast ca.* (pl. effusion) MCF-7 | 5.6 | Liver Cancer GENPAK 064003 | 0.6 | 93792_Lupus Kidney | 1.9 |
| Breast ca.* (pl.ef) MDA-MB-231 | 21.3 | Liver Cancer Research Genetics RNA 1025 | 0.6 | 93577_NCl-H292 | 39.5 |
| Breast ca.* (pl. effusion) T47D | 66.0 | Liver Cancer Research Genetics RNA 1026 | 1.4 | 93358_NCI-H292_IL-4 | 39.0 |
| Breast ca. BT-549 | 7.6 | Paired Liver Cancer Tissue Research Genetics RNA 6004-T | 1.3 | 93360_NCl-H292_IL-9 | 65.5 |
| Breast ca.MDA-N | 18.7 | Paired Liver Tissue Research Genetics RNA 6004-N | 1.3 | 93359_NCl-H292_IL-13 | 37.1 |
| Ovary | 12.1 | Paired Liver Cancer Tissue Research Genetics RNA 6005-T | 1.1 | 93357_NCI-H292_IFN gamma | 31.9 |
| Ovarian ca.OVCAR-3 | 3.5 | Paired Liver Tissue Research Genetics RNA 6005-N | 0.3 | 93777_HPAEC_- | 0.5 |
| Ovarian ca.OVCAR-4 | 4.0 | Normal Bladder GENPAK061001 | 5.9 | 93778_HPAEC_IL-1 beta/TNA alpha | 1.2 |
| Ovarian ca. OVCAR-5 | 9.1 | Bladder Cancer Research | 1.7 | 93254_Normal Human Lung Fibroblast_none | 42.3 |
| | | Genetics RNA 1023 | | | |
| Ovarian ca. OVCAR-8 | 12.7 | Bladder Cancer INVITROGEN A302173 | 1.9 | 93253 Normal Human Lung Fibroblast_TNFa (4 ng/ml) and IL-1b (1 ng/ml) | 17.8 |
| Ovarian ca.IGROV-1 | 9.8 | 87071 Bladder Cancer (OD04718-01) | 2.0 | 93257 Normal Human Lung Fibroblast_IL-4 | 100.0 |
| Ovarian ca.* (ascites) SK-OV-3 | 0.4 | 87072 Bladder Normal Adjacent (OD04718-03) | 3.3 | 93256_Normal Human Lung Fibroblast_IL-9 | 72.7 |
| Uterus | 6.9 | Normal Ovary Res. Gen. | 2.2 | 93255_Normal Human Lung Fibroblast_IL-13 | 60.7 |
| Plancenta | 4.6 | Ovarian Cancer GENPAK 064008 | 29.1 | 93258_Normal Human Lung Fibroblast_IFN gamma | 81.8 |
| Prostate | 15.7 | 87492 Ovary Cancer (OD04768-07) | 100.0 | 93106_Dermal Fibroblasts CCD1070_resting | 76.8 |
| Prostate ca.* (bone met)PC-3 | 35.9 | 87493 Ovary NAT (OD04768-08) | 2.2 | 93361_Dermal Fibroblasts CCD1070_TNF alpha 4 ng/ml | 30.2 |
| Testis | 14.6 | Normal Stomach GENPAK 061017 | 13.1 | 93105_Dermal Fibroblasts CCD1070_IL-1 beta 1 ng/ml | 38.2 |
| Melanoma Hs688(A).T | 13.5 | NAT Stomach Clontech 9060359 | 8.8 | 93772_dermal fibroblast_IFN gamma _ | 34.2 |
| Melanoma* (met) Hs688(B).T | 71.2 | Gastric Cancer Clontech 9060395 | 2.5 | 93771_dennal fibroblast_IL-4 | 80.7 |
| Melanoma UACC-62 | 1.7 | NAT Stomach Clontech 9060394 | 9.7 | 93259_IBD Colitis 1** | 0.0 |
| Melanoma M14 | 9.5 | Gastric Cancer Clontech 9060397 | 15.9 | 93260 IBD Colitis 2 | 0.3 |
| Melanoma LOX IMVI | 2.4 | NAT Stomach Clontech 9060396 | 12.9 | 93261 IBD Crohns | 1.4 |
| Melanoma* (met)SK-MEL-5 | 3.4 | Gastric Cancer GENPAK 064005 | 12.1 | 735010_Colon_normal | 35.6 |
| Adipose | 5.9 | | | 735019_Lung_none | 11.0 |
| | | | | 64028-1_Thymus_none | 5.8 |
| | | | | 64030-1_Kidney_none | 9.7 |

Taqman results shown in Table 9 demonstrates that cFCTR1 is highly expressed by tumor cell lines and also overexpressed in tumor tissues, specifically breast and ovarian tumor compared to Normal Adjacent Tissues (NAT). There are reports that follistatin can act as a modulator of tumor growth and its expression also correlate with polycystic ovary syndrome, a benign form of ovarian tumor.

**Table 10: Primer Design for Probe Ag2773 (FCTR4)**

| **Primer** | **Sequences** | **TM** | **Length** | **Start Pos** | **SEQ ID NO** |
|---|---|---|---|---|---|
| Forward | 5'-CCTTGCTTTGTCATATGCTGTT-3' | 59.3 | 22 | 243 | 29 |
| Probe | FAM-5'-CCCITTGCCTGGAATATAAACTCTCA-3'-TAMRA | 64.6 | 26 | 265 | 30 |
| Reverse | 5'-AGAGGAAGCTTICTGGAGAAGA-3' | 58.9 | 22 | 313 | 31 |

**TABLE 11: TAQMAN RESULTS FOR CLONE FCTR4**

| **Tissue_Name** | **Panel 1D** | **Tissue_Name** | **Panel 2D** | **Tissue_Name** | **Panel 4D** |
|---|---|---|---|---|---|
| Liver adenocarcinoma | 18.3 | Normal Colon GENPAK 061003 | 41.2 | 93768_Secondary Th1_anti-CD28/anti-CD3 | 12.7 |
| Heart (fetal) | 4.3 | 83219 CC Well to Mod Diff (OD03866) | 5.2 | 93769_Secondary Th2_antf-CD28/anti-CD3 | 14.2 |
| Pancreas | 3.1 | 83220 CC NAT (OD03866) | 2.5 | 93770_Secondary Tr1_anti-CD28/anti-CD3 | 14.7 |
| Pancreatic ca.CAPAN 2 | 20.0 | 83221 CC Gr.2 rectosigmoid (ODO3868) | 0.7 | 93573_Secondary Th1_resting day 4-6 in IL-2 | 4.7 |
| Adrenal gland | 7.4 | 83222 CC NAT (OD03868) | 1.4 | 93572_Secondary Th2_resting day 4-6 in IL-2 | 3.5 |
| Thyroid | 6.8 | 83235 CC Mod Diff (ODO3920) | 14.0 | 93571_Secondary Tr1_resting day 4-6 in IL-2 | 7.0 |
| Salivary gland | 2.5 | 83236 CC NAT (ODO3920) | 13.9 | 93568_primary Th1_anti-CD28/anti-CD3 | 22.4 |
| Pituitary gland | 5.7 | 83237 CC Gr.2 ascend colon (ODO3921) | 16.2 | 93569_primary Th2_anti-CD28/anti-CD3 | 16.3 |
| Brain (fetal) | 14.4 | 83238 CC NAT (ODO3921) | 5.2 | 93570_primary Tr1_anti-CD28/anti-CD3 | 21.8 |
| Brain (whole) | 19.6 | 83241 CC from Partial Hepatectomy (ODO4309) | 13.9 | 93565_primary Th1_resting dy 4-6 in IL-2 | 30.2 |
| Brain (amygdala) | 3.7 | 83242 Liver NAT (ODO4309) | 12.7 | 93566_primary Th2_resting dy 4-6 in IL-2 | 14.4 |
| Brain (cerebellum) | 2.1 | 87472 Colon mets to lung (OD04451-01) | 3.4 | 93567_primary Tr1_resting dy 4-6 in IL-2 | 7.4 |
| Brain (hippocampus) | 22.7 | 87473 Lung NAT (OD04451-02) | 1.5 | 93351_CD45RA CD4 lymphocyte_anti-CD28/anti-CD3 | 7.6 |
| Brain (thalamus) | 7.4 | Normal Prostate Clontech A+ 6546-1 | 1.0 | 93352_CD45RO CD4 lymphocyte_anti-CD28/anti-CD3 | 11.1 |
| Cerebral Cortex | 47.3 | 84140 Prostate Cancer (OD04410) | 3.1 | 93251_CD8 Lymphocytes_anti-CD28/anti-CD3 | 9.6 |
| Spinal cord | 8.3 | 84141 Prostate NAT (OD04410) | 10.6 | 93353_chronic CD8 Lymphocytes 2ry_resting dy 4-6 in IL-2 | 9.7 |
| CNS ca. (glio/astro)U87-MG | 19.9 | 87073 Prostate Cancer (OD04720-01) | 9.7 | 93574_chronic CD8 Lymphocytes 2ry_activated CD3/CD28 | 6.2 |
| CNS ca. (glio/astro) U-118-MG | 57.0 | 87074 Prostate NAT (OD04720-02) | 8.3 | 93354_CD4_none | 6.4 |
| CNS ca. (astro) SW1783 | 10.0 | Normal Lung GENPAK 061010 | 36.6 | 93252_Secondary Th1/Th2/Tr1_anti-CD95 CH11 | 9.3 |
| CNS ca.* (neuro; met)SK-N-AS | 44.8 | 83239 Lung Met to Muscle (ODO4286) | 11.7 | 93103_LAK cells_resting | 11.0 |
| CNS ca. (astro) SF-539 | 37.4 | 83240 Musde NAT (OD04286) | 3.4 | 93788_LAK cells_IL-2 | 10.4 |
| CNS ca. (astro) SNB-75 | 62.0 | 84136 Lung Malignant Cancer (ODO3126) | 15.1 | 93787_LAK cells IL-2+IL-12 | 7.4 |
| CNS ca. (glio) SNB-19 | 24.8 | 84137 Lung NAT (OD03126) | 17.4 | 93789_LAK cells_IL-2+IFN gamma | 11.6 |
| CNS ca. (glio) U251 | 40.3 | 84871 Lung Cancer (OD04404) | 5.0 | 93790_LAK cells_IL-2+ IL-18 | 13.3 |
| CNS ca. (glio) SF-295 | 100.0 | 84872 Lung NAT (OD04404) | 6.3 | 93104_LAK cells_PMA/ionomycin and IL-18 | 4.8 |
| Heart | 0.0 | 84875 Lung Cancer (OD04565) | 3.2 | 93578_NK Cells IL-2 resting | 6.2 |
| Skeletal muscle | 0.0 | 85950 Lung Cancer (OD04237-01) | 15.8 | 93109_Mixed Lymphocyte Reaction_Two Way MLR | 12.3 |
| Bone marrow | 33.7 | 85970 Lung NAT (OD04237-02) | 10.5 | 93110 Mixed Lymphocyte Reaction_Two Way MLR | 8.7 |
| Thymus | 12.4 | 83255 Ocular Mel Met to Liver (OD04310) | 5.9 | 93111_Mixed Lymphocyte Reaction_Two Way MLR | 3.5 |
| Spleen | 21.3 | 83256 Liver NAT (OD04310) | 3.6 | 93112_Mononuclear Cells (PBMCs)_resting | 4.5 |
| Lymph node | 13.4 | 84139 Melanoma Mets to Lung (OD04321) | 10.6 | 93113_Mononuclear Cells (PBMCs)_PWM | 21.2 |
| Colorectal | 38.2 | 84138 Lung NAT (OD04321) | 10.6 | 93114_Mononuclear Cells (PBMCs) PHA-L | 8.9 |
| Stomach | 9.9 | Normal Kidney GENPAK 061008 | 26.2 | 93249 Ramos (B cell)_none | 100.0 |
| Small intestine | 17.9 | 83786 Kidney Ca, Nuclear grade 2 (OD04338) | 22.2 | 93250_Ramos (B cell)_ionomycin | 28.7 |
| Colon ca.SW480 | 27.7 | 83787 Kidney NAT (OD04338) | 11.7 | 93349_Blymphocytes_PWM | 20.0 |
| Colon ca.* (SW480 met)SW620 | 30.8 | 83788 Kidney Ca Nuclear grade 1/2 (OD04339) | 45.1 | 93350_B lymphoytes_CD40L and IL-4 | 7.8 |
| Colon ca.HT29 | 8.1 | 83789 Kidney NAT (OD04339) | 14.8 | 92665_EOL-1 (Eosinophil)_dbcAMP differentiated | 8.0 |
| Colon ca.HCT-116 | 35.4 | 83790 Kidney Ca, Clear cell type (OD04340) | 26.6 | 93248_EOL-1 (Eosinophil) dbcAMP/PMAionomycin | 3.8 |
| Colon ca. CaCo-2 | 37.6 | 83791 Kidney NAT (OD04340) | 10.4 | 93356 Dendritic Cells_none | 6.8 |
| 83219 CC Well to Mod Diff (ODO3866) | 17.8 | 83792 Kidney Ca, Nuclear grade 3 (OD04348) | 2.4 | 93355_Dendritic Cells_LPS 100 ng/ml | 3.3 |
| Colon ca.HCC-2998 | 19.9 | 83793 Kidney NAT (OD04348) | 18.8 | 93775_Dendritic Cells_anti-CD40 | 6.3 |
| Gastric ca.* (liver met) NCl-N87 | 73.2 | 87474 Kidney Cancer (OD04622-01) | 5.6 | 93774_Monocytes_resting | 10.6 |
| Bladder | 43.2 | 87475 Kidney NAT (OD04622-03) | 0.5 | 93776_Monocytes_LPS 50 ng/ml | 3.5 |
| Trachea | 10.3 | 85973 Kidney Cancer (OD04450-01) | 21.2 | 93581_Macrophages_resting | 7.6 |
| Kidney | 9.2 | 85974 Kidney NAT (OD04450-03) | 9.3 | 93582_Macrophages_LPS 100 ng/ml | 3.9 |
| Kidney (fetal) | 0.0 | Kidney Cancer Clontech 8120607 | 0.0 | 93098 HUVEC (Endothellal)_none | 8.5 |
| Renal ca.786-0 | 53.6 | Kidney NAT Clontech 8120608 | 0.9 | 93099_HUVEC (Endothellal)_starved | 17.9 |
| Renal ca. A498 | 36.1 | Kidney Cancer Clontech 8120613 | 0.0 | 93100_HUVEC (Endothelial)_IL-1 b | 6.0 |
| Renal ca.RXF 393 | 31.6 | Kidney NAT Clontech 8120614 | 0.9 | 93779_HWEC (Endothelial)_IFN gamma | 7.8 |
| Renal ca.ACHN | 21.6 | Kidney Cancer Clontech 9010320 | 2.7 | 93102_HUVEC (Endothelial)_TNF alpha + IFN gamma | 5.7 |
| Renal ca.UO-31 | 28.7 | Kidney NAT Clontech 9010321 | 5.0 | 93101_HWEC (Endothelial)_TNF alpha + IL4 | 5.6 |
| Renal ca.TK-10 | 7.0 | Normal Uterus GENPAK 061018 | 5.3 | 93781_HUVEC (Endothelial)_IL-1 | 4.9 |
| Liver | 14.2 | Uterus Cancer GENPAK 064011 | 9.0 | 93583_Lung Microvascular Endothelial Cells_none | 4.9 |
| Liver (fetal) | 14.5 | Normal Thyroid Clontech A+ 6570-1 | 3.4 | 93584_Lung Microvascular Endothelial Cells_TNFa (4 ng/ml) and IL1b (1 ng/ml) | 4.9 |
| Liver ca. (hepatoblast) HepG2 | 59.9 | Thyrold Cancer GENPAK 064010 | 1.8 | 92662_Microvascular Dermal endothelium_none | 8.6 |
| Lung | 17.8 | Thyroid Cancer INVITROGEN A302152 | 3.6 | 92663_Microsvasular Dermal endothelium_TNFa (4 ng/ml) and IL 1b (1 ng/ml) | 6.0 |
| Lung (fetal) | 9.6 | Thyroid NAT INVITROGEN A302153 | 4.9 | 93773_Bronchial epithelium_TNFa (4 ng/ml) and IL 1b (1 ng/ml)** | 0.9 |
| Lung ca. (small cell) LX-1 | 70.2 | Normal Breast GENPAK 061019 | 8.5 | 93347_Small Airway Epithelium_none | 1.3 |
| Lung ca. (small cell) NCI-H69 | 29.9 | 84877 Breast Cancer (OD04566) | 1.5 | 93348_Small Airway Epithelium_TNFa (4 ng/ml) and IL1b (1 ng/ml) | 13.2 |
| Lung ca. (s.cell var.) SHP-77 | 3.9 | 85975 Breast Cancer (OD04590-01) | 23.8 | 92668_Coronery Artery SMC_resting | 3.4 |
| Lung ca. (large cell)NCl-H460 | 2.0 | 85976 Breast Cancer Mets (OD04590-03) | 24.5 | 92669_Coronery Artery SMC_TNFa (4 ng/ml) and IL 1b (1 ng/ml) | 2.0 |
| Lung ca. (non-sm. cell) A549 | 28.5 | 87070 Breast Cancer Metastasis (OD04655-05) | 12.9 | 93107_astrocytes_resting | 4.7 |
| Lung ca. (non-s.cell) NCI-H23 | 36.1 | GENPAK Breast Cancer 064006 | 11.8 | 93108_astrocytes_TNFa (4 ng/ml) and IL1b (1 ng/ml) | 1.9 |
| Lung ca (non-s.cell) HOP-62 | 29.9 | Breast Cancer Clontech 9100266 | 3.2 | 92666_KU-812 (Basophil)_resting | 5.8 |
| Lung ca. (non-s.cl) NCI-H522 | 17.2 | Breast NAT Clontech 9100265 | 1.8 | 92667_KU-812 (Basophil)_PMA/ionoycin | 12.0 |
| Lung ca. (squam.) SW 900 | 63.7 | Breast Cancer INVITROGEN A209073 | 11.0 | 93579_CCD1106 (Keratinocytes)_none | 4.9 |
| Lung ca. (squam.) NCl-H596 | 10.0 | Breast NAT INVITROGEN A2090734 | 7.1 | 93580_CCD1106 (Keratinocytes) TNFa and IFNg** | 0.3 |
| Mammary gland | 4.6 | Normal Liver GENPAK 061009 | 8.8 | 93791_Liver Cirrhosis | 1.8 |
| Breast ca.* (pl. effusion) MCF-7 | 0.0 | Liver Cancer GENPAK 064003 | 4.9 | 93792_Lupus Kidney | 1.6 |
| Breast ca.* (pl.ef) MDA-MB-231 | 38.7 | Liver Cancer Research Genetics RNA 1025 | 1.0 | 93577_NCl-H292 | 11.1 |
| Breast ca.* (pl. effusion) T47D | 0.0 | Liver Cancer Research Genetics RNA 1026 | 0.8 | 93358_NCl-H292_IL-4 | 12.2 |
| Breast ca.BT-549 | 4.6 | Paired Liver Cancer Tissue Research Genetics RNA 6004-T | 3.0 | 93360_NCI-H292_IL-9 | 7.6 |
| Breast ca.MDA-N | 19.0 | Paired Liver Tissue Research Genetics RNA 6004-N | 7.3 | 93359_NCI-H292_IL-13 | 6.1 |
| Ovary | 1.7 | Paired Liver Cancer Tissue Research Genetics RNA 6005-T | 0.2 | 93357_NCl-H292_IFN gamma | 5.8 |
| Ovarian ca.OVCAR-3 | 4.8 | Paired Liver Tissue Research Genetics RNA 6005-N | 0.0 | 93777_HPAEC_- | 6.8 |
| Ovarian ca.OVCAR-4 | 0.0 | Normal Bladder GENPAK 061001 | 19.8 | 93778_HPAEC_IL-1 beta/TNA alpha | 5.4 |
| Ovarian ca.OVCAR-5 | 39.0 | Bladder Cancer Research Genetics RNA 1023 | 3.1 | 93254_Normal Human Lung Fibroblast none | 2.1 |
| Ovarian ca.OVCAR-8 | 36.6 | Bladder Cancer INVITROGEN A302173 | 9.9 | 93253_Normal Human Lung Fibroblast_TNFa (4 ng/ml) and IL-1b (1 ng/ml) | 1.9 |
| Ovarian ca.IGROV-1 | 0.0 | 87071 Bladder Cancer (OD04718-01) | 6.6 | 93257_Normal Human Lung Fibroblast_IL-4 | 3.6 |
| Ovarian ca.* (ascites) SK-OV-3 | 65.5 | 87072 Bladder Normal Adjacent (OD04718-03) | 4.0 | 93256_Normal Human Lung Fibroblast_IL-9 | 3.3 |
| Uterus | 1.6 | Normal Ovary Res. Gen. | 0.3 | 93255_Normal Human Lung Fibroblast_IL-13 | 2.3 |
| Plancenta | 8.9 | Ovarian Cancer GENPAK 064008 | 6.8 | 93258_Normal Human Lung Fibroblast IFN gamma | 2.9 |
| Prostate | 0.0 | 87492 Ovary Cancer (OD04768-07) | 100.0 | 93106_Dermal Fibroblasts CCD1070_resting | 5.6 |
| Prostate ca.* (bone met)PC-3 | 9.2 | 87493 Ovary NAT (OD04768-08) | 3.6 | 93361_Dermal Fibroblasts CCD1070_TNF alpha 4 ng/ml | 17.4 |
| Testis | 29.5 | Normal Stomach GENPAK 061017 | 8.6 | 93105_Dermal Fibroblasts CCD1070_IL-1 beta 1 ng/ml | 3.8 |
| Melanoma Hs688(A).T | 14.3 | NAT Stomach Clontech 9060359 | 0.7 | 93772_dermal fibroblast_IFN gamma | 2.6 |
| Melanoma* (met) Hs688(B).T | 22.9 | Gastric Cancer Clontech 9060395 | 3.9 | 93771_dermal fibroblast_IL-4 | 3.4 |
| Melanoma UACC-62 | 9.7 | NAT Stomach Clontech 9060394 | 5.3 | 93259_IBD Colitis 1** | 0.2 |
| Melanoma M14 | 12.7 | Gastric Cancer Clontech 9060397 | 13.2 | 93260_IBD Colitis 2 | 0.4 |
| Melanoma LOX IMVI | 4.5 | NAT Stomach Clontech 9060396 | 1.1 | 93261_IBD Crohns | 0.3 |
| Melanoma* | 21.8 | Gastric Cancer | 23.0 | 735010_Colon_normal | 3.3 |
| (met) SK-MEL-5 | | GENPAK 064005 | | | |
| Adipose | 6.7 | | | 735019_Lung_none | 3.9 |
| | | | | 64028-1_Thymus_none | 7.7 |
| | | | | 64030-1_Kidney_none | 21.8 |

Table 12 shows the taqman results of clone FCTR4 indicating overexpression in ovarian cancer as compared to Normal Adjacent Tissue (NAT). In addition, increased expression is demonstrated by ovarian tumor cell line suggesting that antibodies could be used to treat ovarian tumors.

**Table 13: Primer Design for Probe Ag427 (FCTR5)**

| **Primer** | **Sequences** | **Length** | **Start Po** | **SEQ ID NO** |
|---|---|---|---|---|
| Forward | 5'-GAGCTACAGGCAGCCTCGAGT-3' | 21 | 443 | 32 |
| Probe | TET-5'-TGGCCCAGCTGACCCTGCTCA-3'-TAMRA | 21 | | 33 |
| Reverse | 5'-GGCTACGTCAGTGGGTTTGG-3' | 20 | 449 | 34 |

**Table 14: Taqman results for FCTR5**

| **Tissue_Name** | **Panel 1** | **Tissue_Name** | **Panel 4D** |
|---|---|---|---|
| Endothelial cells | 10.7 | 93768_Secondary Th1_anti-CD28/anti-CD3 | 15.9 |
| Endothelial cells (treated) | 15.2 | 93769_Secondary Th2_anti-CD28/anti-CD3 | 14.7 |
| Pancreas | 16.2 | 93770_Secondary Tr1_anti-CD28/anti-CD3 | 21.9 |
| Pancreatic ca.CAPAN 2 | 10.5 | 93573_Secondary Th1_resting day 4-6 in IL-2 | 12.3 |
| Adipose | 45.1 | 93572_Secondary Th2_resting day 4-6 in IL-2 | 16.2 |
| Adrenal gland | 61.6 | 93571_Secondary Tr1_resting day 4-6 in IL-2 | 16.2 |
| Thyroid | 13.1 | 93568_primary Th1_anti-CD28/anti-CD3 | 13.9 |
| Salavary gland | 33.7 | 93569_primary Th2_anti-CD28/anti-CD3 | 14.6 |
| Pituitary gland | 15.8 | 93570_primary Tr1_anti-CD28/anti-CD3 | 26.2 |
| Brain (fetal) | 7.2 | 93565_primary Th1_resting dy 4-6 in IL-2 | 56.3 |
| Brain (whole) | 6.3 | 93566_primary Th2_resting dy 4-6 in IL-2 | 27.7 |
| Brain (amygdala) | 8.4 | 93567_primary Tr1_resting dy 4-6 in IL-2 | 31.6 |
| Brain (cerebellum) | 6.8 | 93351_CD45RA CD4 lymphocyte_anti-CD28/anti-CD3 | 12.1 |
| Brain (hippocampus) | 7.9 | 93352_CD45RO CD4 lymphocyte_anti-CD28/anti-CD3 | 17.1 |
| Brain (substantia nigra) | 9.5 | 93251_CD8 Lymphocytes_anti-CD28/anti-CD3 | 9.1 |
| Brain (thalamus) | 7.9 | 93353_chronic CD8 Lymphocytes 2ry_resting dy 4-6 in IL-2 | 13.4 |
| Brain (hypothalamus) | 23.0 | 93574_chronic CD8 Lymphocytes 2ry_activated CD3/CD28 | 9.2 |
| Spinal cord | 9.5 | 93354_CD4_none | 7.6 |
| CNS ca. (glio/astro)U87-MG | 12.6 | 93252_Secondary Th1/Th2/Tr1_anti-CD95 CH11 | 20.2 |
| CNS ca. (glio/astro)U-118-MG | 11.6 | 93103_LAK cells_resting | 57.0 |
| CNS ca. (astro)SW1783 | 4.3 | 93788_LAK cells_IL-2 | 18.8 |
| CNS ca.* (neuro; met)SK-N-AS | 10.4 | 93787_LAK cells_IL-2+IL-12 | 14.2 |
| CNS ca. (astro) SF-539 | 11.6 | 93789_LAK cells_IL-2+IFN gamma | 20.9 |
| CNS ca. (astro) SNB-75 | 4.4 | 93790_LAK cells_IL-2+ IL-18 | 14.8 |
| CNS ca. (glio)SNB-19 | 31.6 | 93104_LAK cells_PMA/ionomycin and IL-18 | 12.9 |
| CNS ca. (gllo)U251 | 17.3 | 93578_NK Cells IL-2_resting | 17.4 |
| CNS ca. (glio)SF-295 | 20.9 | 93109_Mixed Lymphocyte Reaction_Two Way MLR | 43.5 |
| Heart | 14.3 | 93110_Mixed Lymphocyte Reaction_Two Way MLR | 19.3 |
| Skeletal muscle | 11.7 | 93111_Mixed Lymphocyte Reaction_Two Way MLR | 12.6 |
| Bone marrow | 21.9 | 93112_Mononuclear Cells (PBMCs)_resting | 8.7 |
| Thymus | 20.9 | 93113_Mononuclear Cells (PBMCs)_PWM | 28.5 |
| Spleen | 23.8 | 93114_Mononuclear Cells (PBMCs)_PHA-L | 26.2 |
| Lymph node | 24.2 | 93249_Ramos (B cell)_pone | 0.3 |
| Colon (ascending) | 17.2 | 93250_Ramos (B cell)_ionomycin | 1.2 |
| Stomach | 11.1 | 93349_B lymphocytes_PWM | 25.7 |
| Small intestine | 21.5 | 93350_B lymphoytes_CD40L and IL-4 | 13.0 |
| Colon ca.SW480 | 12.2 | 92665_EOL-1 (Eosinophil)_dbcAMP differentiated | 26.4 |
| Colon ca.* (SW480 met)SW620 | 8.6 | 93248_EOL-1 CEosinophil)_dbcAMP/PMAjonomycin | 11.4 |
| Colon ca.HT29 | 16.2 | 93356_Dendritic Cells_none | 40.3 |
| Colon ca.HCT-116 | 8.1 | 93355_Dendritic Cells_LPS 100 ng/ml | 33.0 |
| Colon ca.CaCo-2 | 22.1 | 93775_Dendrltic Cells_anti-CD40 | 20.5 |
| Colon ca.HCT-15 | 18.6 | 93774_Monocytes_resting | 23.3 |
| Colon ca.HCC-2998 | 21.9 | 93776_Monocytes_LPS 50 ng/ml | 6.9 |
| Gastric ca.* (liver met) NCI-N87 | 42.9 | 93581_Macrophages_resting | 14.7 |
| Bladder | 95.3 | 93582_Macrophages_LPS 100 ng/ml | 64.6 |
| Trachea | 18.3 | 93098_HUVEC (Endothelial)_none | 6.8 |
| Kidney | 25.7 | 93099_HUVEC (Endothelial)_starved | 13.9 |
| Kidney (fetal) | 15.8 | 93100_HUVEC (Endothelial)_IL-1b | 7.5 |
| Renal ca.786-0 | 16.5 | 93779_HUVEC (Endothelial)_IFN gamma | 27.7 |
| Renal ca.A498 | 16.5 | 93102_HUVEC (Endothelial)_TNF alpha + IFN gamma | 11.8 |
| Renal ca.RXF 393 | 7.4 | 93101_HUVEC (Endothelial)_TNF alpha + IL4 | 6.7 |
| Renal ca.ACHN | 11.9 | 93781_HUVEC (Endothelial)_IL-11 | 10.4 |
| Renal ca.UO-31 | 15.8 | 93583_Lung Microvascular Endothelial Cells_none | 8.8 |
| Renal ca.TK-10 | 28.7 | 93584_Lung Microvascular Endothelial Cells_TNFa (4 ng/ml) and IL1 b (1 ng/ml) | 8.6 |
| Liver | 100.0 | 92662_Microvascular Dermal endothelium_none | 22.1 |
| Liver (fetal) | 81.8 | 92663_Microsvasular Dermal endothelium_TNFa (4 ng/ml) and IL1b (1 ng/ml) | 18.7 |
| Liver ca. (hepatoblast) HepG2 | 28.3 | 93773_Bronchial epithelium_TNFa (4 ng/ml) and IL 1b (1 ng/ml)** | 35.4 |
| Lung | 10.7 | 93347_Small Airway Epithelium_none | 10.9 |
| Lung (fetal) | 10.9 | 93348_Small Airway Epithelium_TNFa (4 ng/ml) and IL 1b (1 ng/ml) | 50.0 |
| Lung ca. (small cell) LX-1 | 24.3 | 92668 Coronery Artery SMC_resting | 27.9 |
| Lung ca. (small cell) NCI-H69 | 41.5 | 92669_Coronery Artery SMC_TNFa (4 ng/ml) and IL1b (1 ng/ml). | 25.4 |
| Lung ca. (s.cell var.) SHP-77 | 4.6 | 93107_astrocytes_resting | 7.4 |
| Lung ca. (large cell)NCI-H460 | 46.3 | 93108_astrocytes_TNFa (4 ng/ml) and IL 1b (1 ng/ml) | 10.7 |
| Lung ca. (non-sm. cell) A549 | 45.4 | 92666_KU-812 (Basophll)_resting | 3.2 |
| Lung ca. (non-s.cell) NCI-H23 | 54.3 | 92667 _KU-812 (Basophil)_PMAlionoycin | 6.7 |
| Lung ca (non-s.cell) HOP-62 | 50.7 | 93579_CCD1106 (Keratinocytes)_none | 12.2 |
| Lung ca. (non-s.cl) NCI-H522 | 38.4 | 93580_CCD1106 (Keratinocytes)_TNFa and IFNg** | 100.0 |
| Lung ca. (squam.) SW 900 | 30.8 | 93791_Liver Cirrhosis | 27.6 |
| Lung ca. (squam.) NCI-H596 | 15.5 | 93792_Lupus Kidney | 32.3 |
| Mammary gland | 65.5 | 93577_NCl-H292 | 77.4 |
| Breast ca.* (pl. effusion) MCF-7 | 4.4 | 93358 NCI-H292_IL-4 | 70.2 |
| Breast ca.* (pl.ef) MDA-MB-231 | 3.5 | 93360_NCl-H292 IL-9 | 54.3 |
| Breast ca.* (pl. effusion)T47D | 8.7 | 93359_NCI-H292 IL-13 | 47.0 |
| Breast ca. BT-549 | 5.7 | 93357_NCl-H292_IFN gamma | 52.9 |
| Breast ca.MDA-N | 16.6 | 93777 HPAEC - | 23.8 |
| Ovary | 20.5 | 93778_HPAEC_IL-1 beta/TNA alpha | 21.5 |
| Ovarian ca. OVCAR-3 | 21.6 | 93254_Normal Human Lung Fibroblast none | 49.3 |
| Ovarian ca.OVCAR-4 | 8.3 | 93253_Normal Human Lung Fibroblast_TNFa (4 ng/ml) and IL-1b (1 ng/ml) | 40.3 |
| Ovarian ca.OVCAR-5 | 26.1 | 93257_Normal Human Lung Fibroblast_IL-4 | 48.3 |
| Ovarian ca.OVCAR-8 | 48.0 | 93256_Normal Human Lung Fibroblast_IL-9 | 29.3 |
| Ovarian ca.IGROV-1 | 9.3 | 93255_Normal Human Lung Fibroblast_IL-13 | 73.7 |
| Ovarian ca.* (ascites)SK-OV-3 | 8.8 | 93258_Normal Human Lung Fibroblast_IFN gamma | 66.9 |
| Uterus | 13.4 | 93106_Dermal Fibroblasts CCD1070_resting | 20.2 |
| Plancenta | 9.4 | 93361_Dermal Fibroblasts CCD1070_TNF alpha 4 ng/ml | 35.1 |
| Prostate | 21.3 | 93105_Dermal Fibroblasts CCD1070_IL-1 beta 1 ng/ml | 15.0 |
| Prostate ca.* (bone met)PC-3 | 17.7 | 93772_dermal fibroblast_IFN gamma | 21.8 |
| Testis | 11.7 | 93771_dermal fibroblast_IL-4 | 21.2 |
| Melanoma Hs688(A).T | 9.0 | 93259_IBD Colitis 1** | 8.8 |
| Melanoma* (met) Hs688(B).T | 12.9 | 93260_IBD Colitis 2 | 3.5 |
| Melanoma UACC-62 | 12.4 | 93261_IBD Crohns | 1.3 |
| Melanoma M14 | 9.5 | 735010_Colon_normal | 20.3 |
| Melanoma LOX IMVI | 8.1 | 735019_Lung_none | 40.3 |
| Melanoma* (met) SK-MEL-5 | 8.8 | 64028-1_Thymus_none | 33.5 |
| Melanoma SK-MEL-28 | 8.0 | 64030-1_Kidney_none | 21.0 |

Taqman results in Table 14 show high expression of clone FCTR5 in bladder, liver and adrenal gland suggesting a possible role in the treatment of diseases involving these tissues.

**Table 15: Primer Design for Probe Ag1541 (FCTR6)**

| **Primer** | **Sequences** | **TM** | **Lengt** | **Start Pos.** | **EQ ID NO.** |
|---|---|---|---|---|---|
| Forward | 5'-AGAAGAACACCCCAGGGATATA-3' | 58.8 | 22 | 1076 | 35 |
| Probe | FAM-5'-CCTCGTTGGTGAACTACAACCTCTGG-3'-TAMRA | 67.9 | 26 | 1100 | 36 |
| Reverse | 5'-CCTCTAGCTGGGTCACTTTCTC-3' | 59.5 | 22 | 1129 | 37 |

**TABLE 16: TAQMAN RESULTS FOR FCTR6 (PANEL 1D)**

| **Tissue Name** | **Panel 1D** | |
|---|---|---|
| | **Run 1** | **Run 2** |
| Liver adenocarcinoma | 0.0 | 0.0 |
| Heart (fetal) | 0.0 | 0.0 |
| Pancreas | 0.0 | 0.0 |
| Pancreatic ca.CAPAN 2 | 0.0 | 0.0 |
| Adrenal gland | 0.0 | 0.0 |
| Thyroid | 0.0 | 0.0 |
| Salivary gland | 0.0 | 0.0 |
| Pituitary gland | 0.0 | 0.0 |
| Brain (fetal) | 0.5 | 0.4 |
| Brain (whole) | 1.1 | 1.7 |
| Brain (amygdala) | 0.0 | 1.8 |
| Brain (cerebellum) | 0.6 | 1.9 |
| Brain (hippocampus) | 3.3 | 3.4 |
| Brain (thalamus) | 1.0 | 1.2 |
| Cerebral Cortex | 1.6 | 2.6 |
| Spinal cord | 2.5 | 0.4 |
| CNS ca. (glio/astro)U87-MG | 0.0 | 0.0 |
| CNS ca. (glio/astro)U-118-MG | 0.0 | 0.0 |
| CNS ca. (astro)SW1783 | 0.0 | 0.0 |
| CNS ca.* (neuro; met)SK-N-AS | 0.0 | 0.0 |
| CNS ca. (astro)SF-539 | 0.0 | 0.0 |
| CNS ca. (astro) SNB-75 | 0.7 | 0.0 |
| CNS ca. (gllo)SNB-19 | 0.0 | 0.0 |
| CNS ca. (glio)U251 | 0.0 | 0.0 |
| CNS ca. (glio)SF-295 | 0.0 | 0.8 |
| Heart | 0.0 | 0.0 |
| Skeletal muscle | 0.0 | 0.0 |
| Bone marrow | 0.0 | 0.0 |
| Thymus | 0.0 | 0.0 |
| Spleen | 0.0 | 0.0 |
| Lymph node | 0.0 | 0.0 |
| Colorectal | 0.0 | 0.6 |
| Stomach | 1.9 | 0.0 |
| Small intestine | 0.0 | 1.0 |
| Colon ca. SW480 | 0.0 | 0.0 |
| Colon ca.* (SW480 met)SW620 | 0.0 | 0.0 |
| Colon ca. HT29 | 0.0 | 0.0 |
| Colon ca. HCT-116 | 0.6 | 0.4 |
| Colon ca.CaCo-2 | 1.5 | 0.0 |
| 83219 CC Well to Mod Diff (ODO3866) | 0.0 | 0.0 |
| Colon ca.HCC-2998 | 0.0 | 0.0 |
| Gastric ca.* (liver met) NCI-N87 | 1.2 | 0.0 |
| Bladder | 0.0 | 0.0 |
| Trachea | 0.0 | 0.4 |
| Kidney | 0.8 | 1.2 |
| Kidney (fetal) | 0.5 | 0.7 |
| Renal ca.786-0 | 0.0 | 0.0 |
| Renal ca.A498 | 0.0 | 0.0 |
| Renal ca.RXF 393 | 0.0 | 0.0 |
| Renal ca.ACHN | 0.0 | 0.0 |
| Renal ca. UO-31 | 0.0 | 0.0 |
| Renal ca.TK-10 | 0.0 | 0.0 |
| Liver | 0.0 | 0.0 |
| Liver (fetal) | 0.2 | 0.0 |
| Liver ca. (hepatoblast) HepG2 | 0.0 | 0.0 |
| Lung | 0.0 | 0.0 |
| Lung (fetal) | 0.0 | 0.0 |
| Lung ca. (small cell) LX-1 | 1.7 | 2.3 |
| Lung ca. (small cell)NCI-H69 | 0.0 | 0.0 |
| Lung ca. (s.cell var.) SHP-77 | 1.3 | 2.5 |
| Lung ca. (large cell)NCI-H460 | 0.0 | 0.0 |
| Lung ca. (non-sm. cell) A549 | 0.0 | 0.0 |
| Lung ca. (non-s.cell) NCI-H23 | 1.2 | 0.4 |
| Lung ca (non-s.cell) HOP-62 | 0.0 | 0.0 |
| Lung ca. (non-s.cl) NCl-H522 | 0.0 | 0.0 |
| Lung ca. (squam.) SW 900 | 0.0 | 0.7 |
| Lung ca. (squam.) NCI-H596 | 0.0 | 1.3 |
| Mammary gland | 0.0 | 1.5 |
| Breast ca.* (pl. effusion) MCF-7 | 0.0 | 0.0 |
| Breast ca.* (pl.ef) MDA-MB-231 | 5.8 | 0.5 |
| Breast ca.* (pl. effusion) T47D | 1.2 | 0.3 |
| Breast ca. BT-549 | 0.5 | 0.0 |
| Breast ca. MDA-N | 0.0 | 0.0 |
| Ovary | 0.0 | 0.0 |
| Ovarian ca. OVCAR-3 | 0.0 | 0.0 |
| Ovarian ca.OVCAR-4 | 0.0 | 0.0 |
| Ovarian ca.OVCAR-5 | 3.6 | 0.7 |
| Ovarian ca.OVCAR-8 | 0.0 | 0.0 |
| Ovarian ca.IGROV-1 | 0.0 | 0.0 |
| Ovarian ca.* (ascites) SK-OV-3 | 0.0 | 0.0 |
| Uterus | 0.0 | 0.0 |
| Plancenta | 0.0 | 0.0 |
| Prostate | 0.0 | 0.7 |
| Prostate ca.* (bone met)PC-3 | 0.0 | 0.0 |
| Testis | 100.0 | 100.0 |
| Melanoma Hs688(A).T | 0.0 | 0.0 |
| Melanoma* (met) Hs688(B).T | 0.0 | 0.0 |
| Melanoma UACC-62 | 0.0 | 0.0 |
| Melanoma M14 | 0.0 | 0.0 |
| Melanoma LOX IMVI | 0.0 | 0.0 |
| Melanoma* (met)SK-MEL-5 | 0.0 | 0.0 |
| Adipose | 0.5 | 0.0 |

**Table 17: Taqman Results for FCTR6 (Panel 2D)**

| **Tissue_Name** | **Panel 2D** | |
|---|---|---|
| | **Run 1** | **Run 2** |
| Normal Colon GENPAK 061003 | 5.4 | 2.4 |
| 83219 CC Well to Mod Diff (ODO3866) | 7.3 | 0.0 |
| 83220 CC NAT (ODO3866) | 5.8 | 1.5 |
| 83221 CC Gr.2 rectosigmoid (ODO3868) | 3.4 | 0.0 |
| 83222 CC NAT (ODO3868) | 0.0 | 0.0 |
| 83235 CC Mod Diff (ODO3920) | 11.0 | 1.4 |
| 83236 CC NAT (ODO3920) | 0.0 | 0.0 |
| 83237 CC Gr.2 ascend colon (ODO3921) | 6.2 | 2.5 |
| 83238 CC NAT (ODO3921) | 10.2 | 0.0 |
| 83241 CC from Partial Hepatectomy (ODO4309) | 3.6 | 0.0 |
| 83242 Liver NAT (ODO4309) | 0.0 | 2.4 |
| 87472 Colon mets to lung (OD04451-01) | 7.2 | 4.4 |
| 87473 Lung NAT (OD04451-02) | 0.0 | 0.0 |
| Normal Prostate Clontech A+ 6546-1 | 4.8 | 2.9 |
| 84140 Prostate Cancer (OD04410) | 3.5 | 0.0 |
| 84141 Prostate NAT (ODO4410) | 3.4 | 0.0 |
| 87073 Prostate Cancer (OD04720-01) | 9.0 | 8.5 |
| 87074 Prostate NAT (OD04720-02) | 0.0 | 0.0 |
| Normal Lung GENPAK 061010 | 17.7 | 6.5 |
| 83239 Lung Met to Musde (ODO4286) | 0.0 | 2.3 |
| 83240 Muscle NAT (ODO4286) | 0.0 | 0.0 |
| 84136 Lung Malignant Cancer (OD03126) | 6.5 | 5.7 |
| 84137 Lung NAT (OD03126) | 0.0 | 0.0 |
| 84871 Lung Cancer (OD04404) | 0.0 | 0.0 |
| 84872 Lung NAT (OD04404) | 0.0 | 0.0 |
| 84875 Lung Cancer (OD04565) | 0.0 | 0.0 |
| 85950 Lung Cancer (OD04237-01) | 0.0 | 0.0 |
| 85970 Lung NAT (OD04237-02) | 0.0 | 0.0 |
| 83255 Ocular Mel Met to Liver (ODO4310) | 4.3 | 0.0 |
| 83256 Liver NAT (ODO4310) | 0.0 | 0.0 |
| 84139 Melanoma Mets to Lung (OD04321) | 0.0 | 0.0 |
| 84138 Lung NAT (OD04321) | 0.0 | 0.0 |
| Normal Kidney GENPAK 061008 | 28.1 | 39.2 |
| 83786 Kidney Ca, Nuclear grade 2 (OD04338) | 0.0 | 3.0 |
| 83787 Kidney NAT (OD04338) | 22.7 | 31.6 |
| 83788 Kidney Ca Nuclear grade 1/2 (OD04339) | 0.0 | 3.1 |
| 83789 Kidney NAT (OD04339) | 97.3 | 100.0 |
| 83790 Kidney Ca, Clear cell type (OD04340) | 0.0 | 0.0 |
| 83791 Kidney NAT (OD04340) | 100.0 | 34.4 |
| 83792 Kidney Ca, Nuclear grade 3 (OD04348) | 2.0 | 4.9 |
| 83793 Kidney NAT (OD04348) | 30.2 | 19.9 |
| 87474 Kidney Cancer (OD04622-01) | 0.0 | 2.4 |
| 87475 Kidney NAT (OD04622-03) | 8.4 | 7.2 |
| 85973 Kidney Cancer (OD04450-01) | 0.0 | 0.0 |
| 85974 Kidney NAT (OD04450-03) | 47.3 | 12.9 |
| Kidney Cancer Clontech 8120607 | 0.0 | 0.0 |
| Kidney NAT Clontech 8120608 | 0.0 | 0.0 |
| Kidney Cancer Clontech 8120613 | 0.0 | 0.0 |
| Kidney NAT Clontech 8120614 | 20.6 | 22.9 |
| Kidney Cancer Clontech 9010320 | 0.0 | 0.0 |
| Kidney NAT Clontech 9010321 | 3.4 | 26.4 |
| Normal Uterus GENPAK 061018 | 0.0 | 0.0 |
| Uterus Cancer GENPAK 064011 | 14.9 | 0.0 |
| Normal Thyroid Clontech A+ 6570-1 | 0.0 | 0.0 |
| Thyroid Cancer GENPAK 064010 | 0.0 | 0.0 |
| Thyroid Cancer INVITROGEN A302152 | 0.0 | 0.0 |
| Thyroid NAT INVITROGEN A302153 | 0.0 | 0.0 |
| Normal Breast GENPAK 061019 | 5.2 | 3.5 |
| 84877 Breast Cancer (OD04566) | 0.0 | 0.0 |
| 85975 Breast Cancer (OD04590-01) | 0.0 | 0.0 |
| 85976 Breast Cancer Mets (OD04590-03) | 0.0 | 0.0 |
| 87070 Breast Cancer Metastasis (OD04655-05) | 0.0 | 0.0 |
| GENPAK Breast Cancer 064006 | 0.0 | 2.5 |
| Breast Cancer Clontech 9100266 | 6.2 | 0.0 |
| Breast NAT Clontech 9100265 | 0.0 | 0.0 |
| Breast Cancer INVITROGEN A209073 | 1.5 | 2.5 |
| Breast NAT INVITROGEN A2090734 | 24.3 | 26.2 |
| Normal Liver GENPAK 061009 | 10.5 | 2.7 |
| Liver Cancer GENPAK 064003 | 5.9 | 1.7 |
| Liver Cancer Research Genetics RNA 1025 | 21.6 | 11.0 |
| Liver Cancer Research Genetics RNA 1026 | 0.0 | 0.0 |
| Paired Liver Cancer Tissue Research Genetics RNA 6004-T | 3.3 | 13.5 |
| Paired Liver Tissue Research Genetics RNA 6004-N | 3.2 | 1.4 |
| Paired Liver Cancer Tissue Research Genetics RNA 6005-T | 0.0 | 0.0 |
| Paired Liver Tissue Research Genetics RNA 6005-N | 0.0 | 0.0 |
| Normal Bladder GENPAK 061001 | 0.0 | 0.0 |
| Bladder Cancer Research Genetics RNA 1023 | 0.0 | 0.0 |
| Bladder Cancer INVITROGEN A302173 | 4.6 | 2.3 |
| 87071 Bladder Cancer (OD04718-01) | 17.9 | 11.4 |
| 87072 Bladder Normal Adjacent (OD04718-03) | 0.0 | 0.0 |
| Normal Ovary Res. Gen. | 0.0 | 0.0 |
| Ovarian Cancer GENPAK 064008 | 1.7 | 4.8 |
| 87492 Ovary Cancer (OD04768-07) | 0.0 | 2.1 |
| 87493 Ovary NAT (OD04768-08) | 0.0 | 0.0 |
| Normal Stomach GENPAK 061017 | 3.3 | 2.9 |
| NAT Stomach Clontech 9060359 | 0.0 | 0.0 |
| Gastric Cancer Clontech 9060395 | 0.0 | 0.0 |
| NAT Stomach Clontech 9060394 | 0.0 | 0.0 |
| Gastric Cancer Clontech 9060397 | 0.0 | 0.0 |
| NAT Stomach Clontech 9060396 | 0.0 | 0.0 |
| Gastric Cancer GENPAK 064005 | 6.3 | 3.8 |

**Table 18: Taqman Results for clone 27455183.0.19 (Panel 4D)**

| **Tissue_Name** | **Panel 4D** | |
|---|---|---|
| | **Run 1** | **Run 2** |
| 93768_Secondary Th1_anti-CD28/anti-CD3 | 0.0 | 0.0 |
| 93769_Secondary Th2_anti-CD28/anti-CD3 | 0.0 | 0.0 |
| 93770_Secondary Tr1_anti-CD28/anti-CD3 | 13.5 | 17.1 |
| 93573_Secondary Th1_resting day 4-6 in IL-2 | 0.0 | 0.0 |
| 93572_Secondary Th2_resting day 4-6 in IL-2 | 0.0 | 0.0 |
| 93571_Secondary Tr1_resting day 4-6 in IL-2 | 0.0 | 0.0 |
| 93568_primary Th1_anti-CD28/anti-CD3 | 0.0 | 0.0 |
| 93569_primary Th2_anti-CD28/anti-CD3 | 0.0 | 0.0 |
| 93570_primary Tr1_anti-CD28/anti-GD3 | 0.0 | 0.0 |
| 93565_primary Th1_resting dy 4-6 in IL-2 | 0.0 | 0.0 |
| 93566_primary Th2 resting dy 4-6 in IL-2 | 0.0 | 0.0 |
| 93567_primary Tr1_resting dy 4-6 in IL-2 | 0.0 | 0.0 |
| 93351_CD45RA CD4 lymphocyte_anti-CD28/anti-CD3 | 0.0 | 0.0 |
| 93352_CD45RO CD4 lymphocyte_anti-CD28/anti-CD3 | 0.0 | 0.0 |
| 93251_CD8 Lymphocytes_anti-CD28/anti-CD3 | 0.0 | 0.0 |
| 93353_chronic CD8 Lymphocytes 2ry_resting dy 4-6 in IL-2 | 0.0 | 0.0 |
| 93574_chronic CD8 Lymphocytes 2ry_activated CD3/CD28 | 0.0 | 0.0 |
| 93354 CD4_none | 5.8 | 0.0 |
| 93252_Secondary Th1/Th2/Tr1_anti-CD95 CH11 | 0.0 | 0.0 |
| 93103_LAK cells_resting | 0.0 | 0.0 |
| 93788_LAK cells_IL-2 | 0.0 | 0.0 |
| 9378T_LAK celis_IL-2+IL-12 | 0.0 | 0.0 |
| 93789_LAK cells_IL-2+IFN gamma | 0.0 | 0.0 |
| 93790_LAK cells_IL-2+ IL-18 | 0.0 | 0.0 |
| 93104_LAK cells_PMA/lonomycin and IL-18 | 0.0 | 0.0 |
| 93578_NK Cells IL-2_resting | 0.0 | 0.0 |
| 93109_Mixed Lymphocyte Reaction_Two Way MLR | 0.0 | 0.0 |
| 93110_Mixed Lymphocyte Reaction_Two Way MLR | 0.0 | 0.0 |
| 93111_Mixed Lymphocyte Reaction_Two Way MLR | 0.0 | 0.0 |
| 93112_Mononuclear Cells (PBMCs)_resting | 0.0 | 0.0 |
| 93113_Mononuclear Cells (PBMCs)_PWM | 0.0 | 0.0 |
| 93114_Mononuclear Cells (PBMCs)_PHA-L | 0.0 | 0.0 |
| 93249_Ramos (B cell) none | 0.0 | 38.2 |
| 93250_Ramos (B cell)_ionomycin | 0.0 | 0.0 |
| 93349_B lymphocytes_PWM | 0.0 | 68.8 |
| 93350_B lymphoytes_CD40L and IL-4 | 31.0 | 0.0 |
| 92665_EOL-1 (Eosinophil)_dbcAMP differentiated | 0.0 | 0.0 |
| 93248_EOL-1 (Eosinophil)_dbcAMP/PMAionomycin | 0.0 | 0.0 |
| 93356_Dendritic Cells_none | 0.0 | 0.0 |
| 93355_Dendritlc Cells_LPS 100 ng/ml | 0.0 | 0.0 |
| 93775_Dendritic Cells_anti-CD40 | 32.5 | 0.0 |
| 93774_Monocytes_resting | 0.0 | 0.0 |
| 93776_Monocytes_LPS 50 ng/ml | 0.0 | 0.0 |
| 93581_Macrophages_resting | 0.0 | 0.0 |
| 93582_Macrophages_LPS 100 ng/ml | 0.0 | 0.0 |
| 93098_HUVEC (Endothelial)_none | 0.0 | 0.0 |
| 93099_HUVEC (Endothelial)_starved | 11.3 | 0.0 |
| 93100 HUVEC (Endothelial)_IL-1b | 0.0 | 14.6 |
| 93779_HUVEC (Endothelial)_IFN gamma | 0.0 | 0.0 |
| 93102_HUVEC (Endothelial)_TNF alpha + IFN gamma | 0.0 | 0.0 |
| 93101_HUVEC (Endothelial)_TNF alpha + IL4 | 0.0 | 0.0 |
| 93781_HUVEC (Endothelial)_IL-11 | 0.0 | 0.0 |
| 93583_Lung Microvascular Endothelial Cells_none | 0.0 | 0.0 |
| 93584_Lung Microvascular Endothelial Cells_TNFa (4 ng/ml) and IL1b (1 ng/ml) | 0.0 | 0.0 |
| 92662_Microvascular Dermal endothelium none | 0.0 | 0.0 |
| 92663_Microsvasular Dermal endothelium_TNFa (4 ng/ml) and IL1b (1 ng/ml) | 0.0 | 0.0 |
| 93773_Bronchial epithelium_TNFa (4 ng/ml) and IL 1b (1 ng/m)) ** | 0.0 | 0.0 |
| 93347 Small Airway Epithelium none | 0.0 | 0.0 |
| 93348_Small Airway Epithelium_TNFa (4 ng/ml) and IL1 b (1 ng/ml) | 0.0 | 0.0 |
| 92668_Coronery Artery SMC_resting | 0.0 | 0.0 |
| 92669_Coronery Artery SMC_TNFa (4 ng/ml) and IL1 b (1 ng/ml) | 0.0 | 0.0 |
| 93107_astrocytes_resting | 0.0 | 0.0 |
| 93108_astrocytes_TNFa (4 ng/ml) and lL1b (1 ng/ml) | 0.0 | 0.0 |
| 92666_KU-812 (Basophil)_resting | 0.0 | 40.3 |
| 92667_KU-812 (Basophil)_PMA/ionoycin | 0.0 | 0.0 |
| 93579_CCD1106 (Keratinocytes)_none | 0.0 | 0.0 |
| 93580_CCD1106 (Keratlnocytes)_TNFa and IFNg ** | 0.0 | 0.0 |
| 93791_Liver Cirrhosis | 100.0 | 99.3 |
| 93792_Lupus Kidney | 0.0 | 0.0 |
| 93577_NCI-H292 | 0.0 | 0.0 |
| 93358_NCI-H292_IL-4 | 0.0 | 0.0 |
| 93360_NCI-H292_IL-9 | 10.6 | 0.0 |
| 93359_NCI-H292_IL-13 | 0.0 | 65.5 |
| 93357_NCl-H292_IFN gamma | 0.0 | 24.8 |
| 93777_HPAEC_ - | 0.0 | 0.0 |
| 93778_HPAEC_IL-1 beta/TNA alpha | 0.0 | 0.0 |
| 93254 Normal Human Lung Fibroblast_none | 0.0 | 0.0 |
| 93253_Normal Human Lung Fibroblast_TNFa (4 ng/ml) and IL-1b (1 ng/ml) | 0.0 | 0.0 |
| 93257_Normal Human Lung Fibroblast_IL-4 | 0.0 | 0.0 |
| 93256_Normal Human Lung Fibroblast_IL-9 | 0.0 | 0.0 |
| 93255_Normal Human Lung Fibroblast_IL-13 | 0.0 | 0.0 |
| 93258_Normal Human Lung Fibroblast_IFN gamma | 0.0 | 0.0 |
| 93106_Dermal Fibroblasts CCD1070_resting | 0.0 | 0.0 |
| 93361_Dermal Fibroblasts CCD1070_TNF alpha 4 ng/ml | 0.0 | 43.8 |
| 93105_Dermal Fibroblasts CCD1070_IL-1 beta 1 ng/ml | 0.0 | 0.0 |
| 93772_dermal fibroblast_IFN gamma | 42.0 | 27.7 |
| 93771_dermal flbroblast_IL-4 | 10.7 | 90.1 |
| 93259_IBD Colitis 1** | 0.0 | 0.0 |
| 93260_IBD Colitis 2 | 13.8 | 0.0 |
| 93261_IBD Crohns | 0.0 | 46.7 |
| 735010_Colon_normal | 15.6 | 0.0 |
| 735019_Lung_none | 12.9 | 16.8 |
| 64028-1_Thymus none | 69.3 | 100.0 |
| 64030-1_Kidney_none | 0.0 | 0.0 |

Taqman results in Table 18 demonstrate that clone FCTR6 is differentially expressed in clear cell Renal cell carcinoma tissues versus the normal adjacent kidney tissues and thus could have a potential role in the treatment of renal cell carcinoma.

### EQUIVALENTS

Although particular embodiments have been disclosed herein in detail, this has been done by way of example for purposes of illustration only, and is not intended to be limiting with respect to the scope of the appended claims which follow. In particular, it is contemplated by the inventors that various substitutions, alterations, and modifications may be made to the invention without departing from the spirit and scope of the invention as defined by the claims. The choice of nucleic acid starting material, clone of interest, or library type is believed to be a matter of routine for a person of ordinary skill in the art with knowledge of the embodiments described herein. Other aspects, advantages, and modifications considered to be within the scope of the following claims.

### Annex to the application documents - subsequently filed sequences listing

SEQUENCE LISTING

<110> Curagen Corporation
      Vernet, Corine AM
      Fernandes, Elma
      Shimkets, Richard A
      Herrmann, John L
      Majumder, Kumud
      Mishra, Vishna
      Mezes, Peter S
      Rastelli, Luca

<120> NOVEL PROTEINS AND NUCLEIC ACIDS ENCODING SAME

<130> 15966-697-061

<140> PCT/US01/07160
<141> 2001-03-05

<150> 60/186,592
<151> 2000-03-03

<150> 60/187,293
<151> 2000-03-06

<150> 60/186,718
<151> 2000-03-03

<160> 98

<170> PatentIn Ver. 2.1

<210> 1
<211> 771
<212> DNA
<213> Homo sapiens

<220>
<221> CDS
<222> (438)..(752)

<400> 1
ggtcctcacc cccttcctct ctcccagcct cggtgtctgg ttacggctcc tctgctcgca 60

ttgtgacttt gggccaggct gggggaaatg acccgggagg gtcccatgcg gctacataaa 120

attggcagcc ttagaactag tgggaaggcg ggtgcgcgaa gtcgaggggc ggagagaggg 180

ggccggagga gctgctttct gaatccaagt tcgtgggctc tctcagaagt cctcaggacg 240

gagcagaggt ggccggcggg cccggctgac tgcgcctctg ctttctttcc ataacctttt 300

ctttcggact cgaatcacgg ctgctgcgaa gggtctagtt ccggacacta gggccccaga 360

tcgtgtcaca tccatatgac acttggaatg tgacagggca ggatgtgatc tttggctgtg 420

aagtgtttgc ctacccc atg gcc tcc atc gag tgg agg aag gat ggc ttg     470
                   Met Ala Ser Ile Glu Trp Arg Lys Asp Gly Leu
                     1               5                  10

gac atc cag ctg cca ggg gat gac ccc cac atc tct gtg cag ttt agg    518

Asp Ile Gln Leu Pro Gly Asp Asp Pro His Ile Ser Val Gln Phe Arg
            15                  20                  25

ggt gga ccc cag agg ttt gag gtg act ggc tgg ctg cag atc cag gct      566
Gly Gly Pro Gln Arg Phe Glu Val Thr Gly Trp Leu Gln Ile Gln Ala
        30                  35                  40

gtg cgt ccc agt gat gag ggc act tac cgc tgc ctt gcc cgc aat gcc      614
Val Arg Pro Ser Asp Glu Gly Thr Tyr Arg Cys Leu Ala Arg Asn Ala
    45                  50                  55

ctg ggt caa gtg gag gcc cct gct agc ttg aca gtg ctc aca cct gac      662
Leu Gly Gln Val Glu Ala Pro Ala Ser Leu Thr Val Leu Thr Pro Asp
60                  65                  70                  75

cag ctg aac tct aca ggc atc ccc cag ctg cga tca cta aac ctg gtt      710
Gln Leu Asn Ser Thr Gly Ile Pro Gln Leu Arg Ser Leu Asn Leu Val
                80                  85                  90

cct gag gag gag gct gag agt gaa gag aat gac gat tac tac              752
Pro Glu Glu Glu Ala Glu Ser Glu Glu Asn Asp Asp Tyr Tyr
            95                  100                 105

taggtccaga gctctggcc                                                 771


<210> 2
<211> 105
<212> PRT
<213> Homo sapiens

<400> 2
Met Ala Ser Ile Glu Trp Arg Lys Asp Gly Leu Asp Ile Gln Leu Pro
1               5                   10                  15

Gly Asp Asp Pro His Ile Ser Val Gln Phe Arg Gly Gly Pro Gln Arg
            20                  25                  30

Phe Glu Val Thr Gly Trp Leu Gln Ile Gln Ala Val Arg Pro Ser Asp
        35                  40                  45

Glu Gly Thr Tyr Arg Cys Leu Ala Arg Asn Ala Leu Gly Gln Val Glu
    50                  55                  60

Ala Pro Ala Ser Leu Thr Val Leu Thr Pro Asp Gln Leu Asn Ser Thr
65                  70                  75                  80

Gly Ile Pro Gln Leu Arg Ser Leu Asn Leu Val Pro Glu Glu Glu Ala
                85                  90                  95

Glu Ser Glu Glu Asn Asp Asp Tyr Tyr
            100                 105


<210> 3
<211> 5502
<212> DNA
<213> Homo sapiens

<220>

<221> CDS
<222> (420)..(2864)

<400> 3
caatttcaca caggaaacag ctatgccatg attacgcaag ttggtaccga gctcggatcc 60

actagtaacg gccgccagtg tgctggaatt cggcttactc actatagggc tcgagcggct 120

gcccgggcag gtcattaatt ccatttcttt ttagagtatc acagctttct ccttcactga 180

ccaccctttg cttcctgtca gaaagccctg gacagaactc tctgtgggat tctgcccatg 240

tttctgagat atcgcctcaa ttgtcctggc tgggctgtcg ggtctgcccg ttttacagat 300

gggcaaactg gagtgggaag tatccgggtg gcttcctcag gcctgcagct ggtggagcag 360

ctactgaaac aatcaggagc ccagaagctt tgaagtcaca agaagagaag actcccaga 419

atg cag tgt gat gtt ggt gat gga cgc ctg ttt cgc ctt tca ctt aaa 467
Met Gln Cys Asp Val Gly Asp Gly Arg Leu Phe Arg Leu Ser Leu Lys
1               5                   10                  15

cgt gcc ctt tcc agc tgc cct gac ctc ttt ggg ctt tcc agc cgc aac 515
Arg Ala Leu Ser Ser Cys Pro Asp Leu Phe Gly Leu Ser Ser Arg Asn
            20                  25                  30

gag ctg ctg gcc tcc tgc ggg aag aag ttc tgc agc cga ggg agc cgg 563
Glu Leu Leu Ala Ser Cys Gly Lys Lys Phe Cys Ser Arg Gly Ser Arg
        35                  40                  45

tgc gtg ctc agc agg aag aca ggg gag ccc gaa tgc cag tgc ctg gag 611
Cys Val Leu Ser Arg Lys Thr Gly Glu Pro Glu Cys Gln Cys Leu Glu
    50                  55                  60

gca tgc agg ccc agc tac gtg cct gtg tgc ggc tct gat ggg agg ttt 659
Ala Cys Arg Pro Ser Tyr Val Pro Val Cys Gly Ser Asp Gly Arg Phe
65                  70                  75                  80

tat gaa aac cac tgt aag ctc cac cgt gct gct tgc ctc ctg gga aag 707
Tyr Glu Asn His Cys Lys Leu His Arg Ala Ala Cys Leu Leu Gly Lys
                85                  90                  95

agg atc acc gtc atc cac agc aag gac tgt ttc ctc aaa ggt gac acg 755
Arg Ile Thr Val Ile His Ser Lys Asp Cys Phe Leu Lys Gly Asp Thr
            100                 105                 110

tgc acc atg gcc ggc tac gcc cgc ttg aag aat gtc ctt ctg gca ctc 803
Cys Thr Met Ala Gly Tyr Ala Arg Leu Lys Asn Val Leu Leu Ala Leu
        115                 120                 125

cag acc cgt ctg cag cca ctc caa gaa gga gac agc aga caa gac cct 851
Gln Thr Arg Leu Gln Pro Leu Gln Glu Gly Asp Ser Arg Gln Asp Pro
    130                 135                 140

gcc tcc cag aag cgc ctc ctg gtg gaa tct ctg ttc agg gac tta gat 899
Ala Ser Gln Lys Arg Leu Leu Val Glu Ser Leu Phe Arg Asp Leu Asp
145                 150                 155                 160

gca gat ggc aat ggc cac ctc agc agc tcc gaa ctg gct cag cat gtg 947
Ala Asp Gly Asn Gly His Leu Ser Ser Ser Glu Leu Ala Gln His Val
                165                 170                 175

ctg aag aag cag gac ctg gat gaa gac tta ctt ggt tgc tca cca ggt 995
Leu Lys Lys Gln Asp Leu Asp Glu Asp Leu Leu Gly Cys Ser Pro Gly
            180                 185                 190

gac ctc ctc cga ttt gac gat tac aac agt gac agc tcc ctg acc ctc 1043
Asp Leu Leu Arg Phe Asp Asp Tyr Asn Ser Asp Ser Ser Leu Thr Leu
        195                 200                 205

cgc gag ttc tac atg gcc ttc caa gtg gtt cag ctc agc ctc gcc ccc 1091
Arg Glu Phe Tyr Met Ala Phe Gln Val Val Gln Leu Ser Leu Ala Pro
    210                 215                 220

gag gac agg gtc agt gtg acc aca gtg acc gtg ggg ctg agc aca gtg 1139
Glu Asp Arg Val Ser Val Thr Thr Val Thr Val Gly Leu Ser Thr Val
225                 230                 235                 240

ctg acc tgc gcc gtc cat gga gac ctg agg cca cca atc atc tgg aag 1187
Leu Thr Cys Ala Val His Gly Asp Leu Arg Pro Pro Ile Ile Trp Lys
                245                 250                 255

cgc aac ggg ctc acc ctg aac ttc ctg gac ttg gaa gac atc aat gac 1235
Arg Asn Gly Leu Thr Leu Asn Phe Leu Asp Leu Glu Asp Ile Asn Asp
            260                 265                 270

ttt gga gag gat gat tcc ctg tac atc acc aag gtg acc acc atc cac 1283
Phe Gly Glu Asp Asp Ser Leu Tyr Ile Thr Lys Val Thr Thr Ile His
        275                 280                 285

atg ggc aat tac acc tgc cat gct tcc ggc cac gag cag ctg ttc cag 1331
Met Gly Asn Tyr Thr Cys His Ala Ser Gly His Glu Gln Leu Phe Gln
    290                 295                 300

acc cac gtc ctg cag gtg aat gtg ccg cca gtc atc cgt gtc tat cca 1379
Thr His Val Leu Gln Val Asn Val Pro Pro Val Ile Arg Val Tyr Pro
305                 310                 315                 320

gag agc cag gca cag gag cct gga gtg gca gcc agc cta aga tgc cat 1427
Glu Ser Gln Ala Gln Glu Pro Gly Val Ala Ala Ser Leu Arg Cys His
                325                 330                 335

gct gag ggc att ccc atg ccc aga atc act tgg ctg aaa aac ggc gtg 1475
Ala Glu Gly Ile Pro Met Pro Arg Ile Thr Trp Leu Lys Asn Gly Val
            340                 345                 350

gat gtc tca act cag atg tcc aaa cag ctc tcc ctt tta gcc aat ggg 1523
Asp Val Ser Thr Gln Met Ser Lys Gln Leu Ser Leu Leu Ala Asn Gly
        355                 360                 365

agc gaa ctc cac atc agc agt gtt cgg tat gaa gac aca ggg gca tac 1571
Ser Glu Leu His Ile Ser Ser Val Arg Tyr Glu Asp Thr Gly Ala Tyr
    370                 375                 380

acc tgc att gcc aaa aat gaa gtg ggt gtg gat gaa gat atc tcc tcg 1619
Thr Cys Ile Ala Lys Asn Glu Val Gly Val Asp Glu Asp Ile Ser Ser
385                 390                 395                 400

ctc ttc att gaa gac tca gct aga aag acc ctt gca aac atc ctg tgg 1667
Leu Phe Ile Glu Asp Ser Ala Arg Lys Thr Leu Ala Asn Ile Leu Trp
                405                 410                 415

cga gag gaa ggc ctc agc gtg gga aac atg ttc tat gtc ttc tcc gac 1715
Arg Glu Glu Gly Leu Ser Val Gly Asn Met Phe Tyr Val Phe Ser Asp
            420                 425                 430

gac ggt atc atc gtc atc cat cct gtg gac tgt gag atc cag agg cac 1763
Asp Gly Ile Ile Val Ile His Pro Val Asp Cys Glu Ile Gln Arg His
        435                 440                 445

ctc aaa ccc acg gaa aag att ttc atg agc tat gaa gaa atc tgt cct 1811
Leu Lys Pro Thr Glu Lys Ile Phe Met Ser Tyr Glu Glu Ile Cys Pro
    450                 455                 460

caa aga gaa aaa aat gca acc cag ccc tgc cag tgg gta tct gca gtc 1859
Gln Arg Glu Lys Asn Ala Thr Gln Pro Cys Gln Trp Val Ser Ala Val
465                 470                 475                 480

aat gtc cgg aac cgg tac atc tat gtg gcc cag cca gca ctg agc aga 1907
Asn Val Arg Asn Arg Tyr Ile Tyr Val Ala Gln Pro Ala Leu Ser Arg
                485                 490                 495

gtc ctt gtg gtc gac atc caa gcc cag aaa gtc cta cag tcc ata ggt 1955
Val Leu Val Val Asp Ile Gln Ala Gln Lys Val Leu Gln Ser Ile Gly
            500                 505                 510

gtg gac cct ctg ccg gct aag ctg tcc tat gac aag tca cat gac caa 2003
Val Asp Pro Leu Pro Ala Lys Leu Ser Tyr Asp Lys Ser His Asp Gln
        515                 520                 525

gtg tgg gtc ctg agc tgg ggg gac gtg cac aag tcc cga cca agt ctc 2051
Val Trp Val Leu Ser Trp Gly Asp Val His Lys Ser Arg Pro Ser Leu
    530                 535                 540

cag gtg atc aca gaa gcc agc acc ggc cag agc cag cac ctc atc cgc 2099
Gln Val Ile Thr Glu Ala Ser Thr Gly Gln Ser Gln His Leu Ile Arg
545                 550                 555                 560

aca ccc ttt gca gga gtg gat gat ttc ttc att ccc cca aca aac ctc 2147
Thr Pro Phe Ala Gly Val Asp Asp Phe Phe Ile Pro Pro Thr Asn Leu
                565                 570                 575

atc atc aac cac atc agg ttt ggc ttc atc ttc aac aag tct gat cct 2195
Ile Ile Asn His Ile Arg Phe Gly Phe Ile Phe Asn Lys Ser Asp Pro
            580                 585                 590

gca gtc cac aag gtg gac ctg gaa aca atg atg ccc ctc aag acc atc 2243
Ala Val His Lys Val Asp Leu Glu Thr Met Met Pro Leu Lys Thr Ile
        595                 600                 605

ggc ctg cac cac cat ggc tgc gtg ccc cag gcc atg gca cac acc cac 2291
Gly Leu His His His Gly Cys Val Pro Gln Ala Met Ala His Thr His
    610                 615                 620

ctg ggc ggc tac ttc ttc atc cag tgc cga cag gac agc ccc gcc tct 2339
Leu Gly Gly Tyr Phe Phe Ile Gln Cys Arg Gln Asp Ser Pro Ala Ser
625                 630                 635                 640

gct gcc cga cag ctg ctc gtt gac agt gtc aca gac tct gtg ctt ggc 2387
Ala Ala Arg Gln Leu Leu Val Asp Ser Val Thr Asp Ser Val Leu Gly
                645                 650                 655

ccc aat ggt gat gta aca ggc acc cca cac aca tcc ccc gac ggg cgc 2435

Pro Asn Gly Asp Val Thr Gly Thr Pro His Thr Ser Pro Asp Gly Arg
            660                 665                 670

ttc ata gtc agt gct gca gct gac agc ccc tgg ctg cac gtg cag gag 2483
Phe Ile Val Ser Ala Ala Ala Asp Ser Pro Trp Leu His Val Gln Glu
        675                 680                 685

atc aca gtg cgg ggc gag atc cag acc ctg tat gac ctg caa ata aac 2531
Ile Thr Val Arg Gly Glu Ile Gln Thr Leu Tyr Asp Leu Gln Ile Asn
    690                 695                 700

tcg ggc atc tca gac ttg gcc ttc cag cgc tcc ttc act gaa agc aat 2579
Ser Gly Ile Ser Asp Leu Ala Phe Gln Arg Ser Phe Thr Glu Ser Asn
705                 710                 715                 720

caa tac aac atc tac gcg gct ctg cac acg gag ccg gac ctg ctg ttc 2627
Gln Tyr Asn Ile Tyr Ala Ala Leu His Thr Glu Pro Asp Leu Leu Phe
                725                 730                 735

ctg gag ctg tcc acg ggg aag gtg ggc atg ctg aag aac tta aag gag 2675
Leu Glu Leu Ser Thr Gly Lys Val Gly Met Leu Lys Asn Leu Lys Glu
            740                 745                 750

cca ccc gca ggg cca gct cag ccc tgg ggg ggt acc cac aga atc atg 2723
Pro Pro Ala Gly Pro Ala Gln Pro Trp Gly Gly Thr His Arg Ile Met
        755                 760                 765

agg gac agt ggg ctg ttt gga cag tac ctc ctc aca cca gcc cga gag 2771
Arg Asp Ser Gly Leu Phe Gly Gln Tyr Leu Leu Thr Pro Ala Arg Glu
    770                 775                 780

tca ctg ttc ctc atc aat ggg aga caa aac acg ctg cgg tgt gag gtg 2819
Ser Leu Phe Leu Ile Asn Gly Arg Gln Asn Thr Leu Arg Cys Glu Val
785                 790                 795                 800

tca ggt ata aag ggg ggg acc aca gtg gtg tgg gtg ggt gag gta 2864
Ser Gly Ile Lys Gly Gly Thr Thr Val Val Trp Val Gly Glu Val
                805                 810                 815

tgaagggccc agagcagagc cctgggccaa ggaacacccc ctagtcctga cactgcagcc 2924

tcaagcaggt acgctgtaca tttttacaga caaaagcaaa aacctgtact cgctttgtgg 2984

ttcaacactg gtctccttgc aagtttccta gtataaggta tgcgctgcta ccaagattgg 3044

ggtttttttcg ttaggaagta tgatttatgc cttgagctac gatgagaaca tatgctgctg 3104

tgtaaaggga tcatttctgt gccaagctgc acaccgagtg acctggggac atcatggaac 3164

caagggatcc tgctctccaa gcagacacct ctgtcagttg ccttcacata gtcattgtcc 3224

cttactgcca gacccagcca gactttgccc tgacggagtg gcccggaagc agaggccgac 3284

caggagcagg ggcctccctc ccgaactgaa agcccatccg tcctcgcgtg ggaccgcatc 3344

ttctccctcg cagctgcttc ttgcttttct ttccatttga cttgctgtaa gcctgaggga 3404

gagccaacaa gacttactgc atcttggggg atggggaaat cactcacttt attttggaaa 3464

tttttgatta aaaaaaaatt ttataatctc aaatgctagt aagcagaaag atgctctccg 3524

aggtccaact atatccttcc ctgccttagg ccgagtctcg ggggtggtca caaccccaca 3584

tcccacagcc agaaagaaca atggtcatct gagaatactg gccctgtcga ctattgccac 3644

cctgcttctc caagagcaga ccaggccacc tcatccgtaa ggactcggtt ctgtgttggg 3704

accccaaaaa accagaacaa gttctgtgtg cctcctttca gcacagaagg gagacatctc 3764

attagtcagg tctggtaccc cagattcagg gcagactggg cttgcctggc aaggtatggg 3824

tggcctccag gctcaatgca gaaaccccaa ggacacgagt ggggccaggt gagttcctga 3884

agctatacct tttcaaaaca gattttgttt tcctacctgt ggcccatcca ctcctctctg 3944

gtaccccatc cccgcatcag cactgcagag agaacacatt tcggcgaggg ttttcttacc 4004

cacattcccc aatcaataca cacacactgc agaacccaga acagaaggcc acaggctggc 4064

actactgcat tctccttatg tgtctcaggc tgtggtgact ctcacatggg catcgaagaa 4124

gtacaaccca catagccctc tggagaccgc ctagatcaga gactcagcaa aaacaggctc 4184

gccttccctc tcccacatat gagtggaact tacatgtgtc ctggtttgaa tgatcatttt 4244

gcaagccaca cgggttggga gaggtggtct caccacagac gtctttgcta atttggccac 4304

cttcacctac tgacatgacc aggattttcc tttgccatta aggaatgaac tctttcaagg 4364

agaggaaacc ctagactctg tgtcactctc aacacacaca gctcctttca ctcctgcctg 4424

actgccaagc cacctgcatc ccccgcccca gatctcatga gatcaatcac ttgtatgtct 4484

cacgcaactt ggtccaccaa acgcctgtcc cctgtaactc ctaggggtgc gcctagacag 4544

gtacgtctgt tttttatttt aaaagatatg ctatgtagat ataagttgag gaagctcacc 4604

tcaaaagcct agaatgcagt ttcacagtag ctgggatgca tggatgaccc atctcacccc 4664

tttttttttc ctgcctcaat atcttgatat gttatgttta ctcccaatct cccattttta 4724

ccactaaaat tctccaactt tcataaactt ttttttggaa aaatttccat tgtatcagcc 4784

cctgacagaa aaaggatctc tgagcctaaa ggaggaaaag tcccaccaac taccagacca 4844

gaacacgagc ccctctgggc agcaggattc ctaagtcaaa gaccagtttg acccaaactg 4904

gccttttaaa ataatcagga gtgacagagt caacttctgc agcacctgct tctcccccac 4964

tgtcccttcc atcttggaat gtgtctaaaa aagcatagct gccctttgct gtcctcagag 5024

tgcatttcct ggagacggca ggcttaggtc tcactgacag catgccagac acaactgaat 5084

cgaagcaggc ctgaagccta ggtcagggtt tcaggagtcc agccccagga ggcaaagtca 5144

ccaatgcagg gaggtaaatg ccttttggca ggaaaaccaa tagagttggt tgggtgggga 5204

gtcaggggtg ggaggagaag gaggaagagg aggaaggcca gactggcctg ccctttctcc 5264

catacttcac cccagcagag gttcatggga cacagttgga aagccactgg gaggaaatgc 5324

ctcactacag gggggcctcc tgtagcaagc ccagccggta atcctcctaa tgaacccaca 5384

aggtcaattc acaactgata tcttagctat taaagaagta ctgactttac caaaagaatc 5444

atcaagaaag ctatttatat aaaccccctc agtcattttg aaataaaatt aattttac 5502

<210> 4
<211> 815
<212> PRT
<213> Homo sapiens

<400> 4
Met Gln Cys Asp Val Gly Asp Gly Arg Leu Phe Arg Leu Ser Leu Lys
1               5                   10                  15

Arg Ala Leu Ser Ser Cys Pro Asp Leu Phe Gly Leu Ser Ser Arg Asn
            20                  25                  30

Glu Leu Leu Ala Ser Cys Gly Lys Lys Phe Cys Ser Arg Gly Ser Arg
        35                  40                  45

Cys Val Leu Ser Arg Lys Thr Gly Glu Pro Glu Cys Gln Cys Leu Glu
    50                  55                  60

Ala Cys Arg Pro Ser Tyr Val Pro Val Cys Gly Ser Asp Gly Arg Phe
65                  70                  75                  80

Tyr Glu Asn His Cys Lys Leu His Arg Ala Ala Cys Leu Leu Gly Lys
                85                  90                  95

Arg Ile Thr Val Ile His Ser Lys Asp Cys Phe Leu Lys Gly Asp Thr
            100                 105                 110

Cys Thr Met Ala Gly Tyr Ala Arg Leu Lys Asn Val Leu Leu Ala Leu
        115                 120                 125

Gln Thr Arg Leu Gln Pro Leu Gln Glu Gly Asp Ser Arg Gln Asp Pro
    130                 135                 140

Ala Ser Gln Lys Arg Leu Leu Val Glu Ser Leu Phe Arg Asp Leu Asp
145                 150                 155                 160

Ala Asp Gly Asn Gly His Leu Ser Ser Ser Glu Leu Ala Gln His Val
                165                 170                 175

Leu Lys Lys Gln Asp Leu Asp Glu Asp Leu Leu Gly Cys Ser Pro Gly
            180                 185                 190

Asp Leu Leu Arg Phe Asp Asp Tyr Asn Ser Asp Ser Ser Leu Thr Leu
        195                 200                 205

Arg Glu Phe Tyr Met Ala Phe Gln Val Val Gln Leu Ser Leu Ala Pro
    210                 215                 220

Glu Asp Arg Val Ser Val Thr Thr Val Thr Val Gly Leu Ser Thr Val
225                 230                 235                 240

Leu Thr Cys Ala Val His Gly Asp Leu Arg Pro Pro Ile Ile Trp Lys
                245                 250                 255

Arg Asn Gly Leu Thr Leu Asn Phe Leu Asp Leu Glu Asp Ile Asn Asp

            260                 265                 270

Phe Gly Glu Asp Asp Ser Leu Tyr Ile Thr Lys Val Thr Thr Ile His
        275                 280                 285

Met Gly Asn Tyr Thr Cys His Ala Ser Gly His Glu Gln Leu Phe Gln
    290                 295                 300

Thr His Val Leu Gln Val Asn Val Pro Pro Val Ile Arg Val Tyr Pro
305                 310                 315                 320

Glu Ser Gln Ala Gln Glu Pro Gly Val Ala Ala Ser Leu Arg Cys His
                325                 330                 335

Ala Glu Gly Ile Pro Met Pro Arg Ile Thr Trp Leu Lys Asn Gly Val
            340                 345                 350

Asp Val Ser Thr Gln Met Ser Lys Gln Leu Ser Leu Leu Ala Asn Gly
        355                 360                 365

Ser Glu Leu His Ile Ser Ser Val Arg Tyr Glu Asp Thr Gly Ala Tyr
    370                 375                 380

Thr Cys Ile Ala Lys Asn Glu Val Gly Val Asp Glu Asp Ile Ser Ser
385                 390                 395                 400

Leu Phe Ile Glu Asp Ser Ala Arg Lys Thr Leu Ala Asn Ile Leu Trp
                405                 410                 415

Arg Glu Glu Gly Leu Ser Val Gly Asn Met Phe Tyr Val Phe Ser Asp
            420                 425                 430

Asp Gly Ile Ile Val Ile His Pro Val Asp Cys Glu Ile Gln Arg His
        435                 440                 445

Leu Lys Pro Thr Glu Lys Ile Phe Met Ser Tyr Glu Glu Ile Cys Pro
    450                 455                 460

Gln Arg Glu Lys Asn Ala Thr Gln Pro Cys Gln Trp Val Ser Ala Val
465                 470                 475                 480

Asn Val Arg Asn Arg Tyr Ile Tyr Val Ala Gln Pro Ala Leu Ser Arg
                485                 490                 495

Val Leu Val Val Asp Ile Gln Ala Gln Lys Val Leu Gln Ser Ile Gly
            500                 505                 510

Val Asp Pro Leu Pro Ala Lys Leu Ser Tyr Asp Lys Ser His Asp Gln
        515                 520                 525

Val Trp Val Leu Ser Trp Gly Asp Val His Lys Ser Arg Pro Ser Leu
    530                 535                 540

Gln Val Ile Thr Glu Ala Ser Thr Gly Gln Ser Gln His Leu Ile Arg
545                 550                 555                 560

Thr Pro Phe Ala Gly Val Asp Asp Phe Phe Ile Pro Pro Thr Asn Leu
                565                 570                 575

Ile Ile Asn His Ile Arg Phe Gly Phe Ile Phe Asn Lys Ser Asp Pro
            580                 585                 590

Ala Val His Lys Val Asp Leu Glu Thr Met Met Pro Leu Lys Thr Ile
        595                 600                 605

Gly Leu His His His Gly Cys Val Pro Gln Ala Met Ala His Thr His
    610                 615                 620

Leu Gly Gly Tyr Phe Phe Ile Gln Cys Arg Gln Asp Ser Pro Ala Ser
625                 630                 635                 640

Ala Ala Arg Gln Leu Leu Val Asp Ser Val Thr Asp Ser Val Leu Gly
                645                 650                 655

Pro Asn Gly Asp Val Thr Gly Thr Pro His Thr Ser Pro Asp Gly Arg
            660                 665                 670

Phe Ile Val Ser Ala Ala Ala Asp Ser Pro Trp Leu His Val Gln Glu
        675                 680                 685

Ile Thr Val Arg Gly Glu Ile Gln Thr Leu Tyr Asp Leu Gln Ile Asn
    690                 695                 700

Ser Gly Ile Ser Asp Leu Ala Phe Gln Arg Ser Phe Thr Glu Ser Asn
705                 710                 715                 720

Gln Tyr Asn Ile Tyr Ala Ala Leu His Thr Glu Pro Asp Leu Leu Phe
                725                 730                 735

Leu Glu Leu Ser Thr Gly Lys Val Gly Met Leu Lys Asn Leu Lys Glu
            740                 745                 750

Pro Pro Ala Gly Pro Ala Gln Pro Trp Gly Gly Thr His Arg Ile Met
        755                 760                 765

Arg Asp Ser Gly Leu Phe Gly Gln Tyr Leu Leu Thr Pro Ala Arg Glu
    770                 775                 780

Ser Leu Phe Leu Ile Asn Gly Arg Gln Asn Thr Leu Arg Cys Glu Val
785                 790                 795                 800

Ser Gly Ile Lys Gly Gly Thr Thr Val Val Trp Val Gly Glu Val
                805                 810                 815

<210> 5
<211> 1430
<212> DNA
<213> Homo sapiens

<220>
<221> CDS
<222> (69)..(1211)

<400> 5
aaaaaaggcg gggggtggac ttagcagtgt aatttgagac cggtggtaag gattggagcg 60

agctagag atg ctg cac gct gct aac aag gga agg aag cct tca gct gag 110
Met Leu His Ala Ala Asn Lys Gly Arg Lys Pro Ser Ala Glu
1               5                   10

gca ggt cgt ccc att cca cct aca tcc tcg cct agt ctc ctc cca tct 158
Ala Gly Arg Pro Ile Pro Pro Thr Ser Ser Pro Ser Leu Leu Pro Ser
15                  20                  25                  30

gct cag ctg cct agc tcc cat aat cct cca cca gtt agc tgc cag atg 206
Ala Gln Leu Pro Ser Ser His Asn Pro Pro Pro Val Ser Cys Gln Met
                35                  40                  45

cca ttg cta gac agc aac acc tcc cat caa atc atg gac acc aac cct 254
Pro Leu Leu Asp Ser Asn Thr Ser His Gln Ile Met Asp Thr Asn Pro
            50                  55                  60

gat gag gaa ttc tcc ccc aat tca tac ctg ctc aga gca tgc tca ggg 302
Asp Glu Glu Phe Ser Pro Asn Ser Tyr Leu Leu Arg Ala Cys Ser Gly
        65                  70                  75

ccc cag caa gcc tcc agc agt ggc cct ccg aac cac cac agc cag tcg 350
Pro Gln Gln Ala Ser Ser Ser Gly Pro Pro Asn His His Ser Gln Ser
    80                  85                  90

act ctg agg ccc cct ctc cca ccc cct cac aac cac acg ctg tcc cat 398
Thr Leu Arg Pro Pro Leu Pro Pro Pro His Asn His Thr Leu Ser His
95                  100                 105                 110

cac cac tcg tcc gcc aac tcc ctc aac agg aac tca ctg acc aat cgg 446
His His Ser Ser Ala Asn Ser Leu Asn Arg Asn Ser Leu Thr Asn Arg
                115                 120                 125

cgg agt cag atc cac gcc ccg gcc cca gcg ccc aat gac ctg gcc acc 494
Arg Ser Gln Ile His Ala Pro Ala Pro Ala Pro Asn Asp Leu Ala Thr
            130                 135                 140

aca cca gag tcc gtt cag ctt cag gac agc tgg gtg cta aac agc aac 542
Thr Pro Glu Ser Val Gln Leu Gln Asp Ser Trp Val Leu Asn Ser Asn
        145                 150                 155

gtg cca ctg gag acc cgg cac ttc ctc ttc aag acc tcc tcg ggg agc 590
Val Pro Leu Glu Thr Arg His Phe Leu Phe Lys Thr Ser Ser Gly Ser
    160                 165                 170

aca ccc ttg ttc agc agc tct tcc ccg gga tac cct ttg acc tca gga 638
Thr Pro Leu Phe Ser Ser Ser Ser Pro Gly Tyr Pro Leu Thr Ser Gly
175                 180                 185                 190

acg gtt tac acg ccc ccg ccc cgc ctg ctg ccc agg aat act ttc tcc 686
Thr Val Tyr Thr Pro Pro Pro Arg Leu Leu Pro Arg Asn Thr Phe Ser
                195                 200                 205

agg aag gct ttc aag ctg aag aag ccc tcc aaa tac tgc agc tgg aaa 734
Arg Lys Ala Phe Lys Leu Lys Lys Pro Ser Lys Tyr Cys Ser Trp Lys
            210                 215                 220

tgt gct gcc ctc tcc gcc att gcc gcg gcc ctc ctc ttg gct att ttg 782
Cys Ala Ala Leu Ser Ala Ile Ala Ala Ala Leu Leu Leu Ala Ile Leu
        225                 230                 235

ctg gcg tat ttc ata gtg ccc tgg tcg ttg aaa aac agc agc ata gac 830
Leu Ala Tyr Phe Ile Val Pro Trp Ser Leu Lys Asn Ser Ser Ile Asp
    240                 245                 250

agt ggt gaa gca gaa gtt ggt cgg cgg gta aca caa gaa gtc cca cca 878

Ser Gly Glu Ala Glu Val Gly Arg Arg Val Thr Gln Glu Val Pro Pro
255                 260                 265                 270

ggg gtg ttt tgg agg tca caa att cac atc agt cag ccc cag ttc tta     926
Gly Val Phe Trp Arg Ser Gln Ile His Ile Ser Gln Pro Gln Phe Leu
                275                 280                 285

aag ttc aac atc tcc ctc ggg aag gac gct ctc ttt ggt gtt tac ata     974
Lys Phe Asn Ile Ser Leu Gly Lys Asp Ala Leu Phe Gly Val Tyr Ile
            290                 295                 300

aga aga gga ctt cca cca tct cat gcc cag tat gac ttc atg gaa cgt     1022
Arg Arg Gly Leu Pro Pro Ser His Ala Gln Tyr Asp Phe Met Glu Arg
        305                 310                 315

ctg gac ggg aag gag aag tgg agt gtg gtt gag tct ccc agg gaa cgc     1070
Leu Asp Gly Lys Glu Lys Trp Ser Val Val Glu Ser Pro Arg Glu Arg
    320                 325                 330

cgg agc ata cag acc ttg gtt cag aat gaa gcc gtg ttt gtg cag tac     1118
Arg Ser Ile Gln Thr Leu Val Gln Asn Glu Ala Val Phe Val Gln Tyr
335                 340                 345                 350

ctg gat gtg ggc ctg tgg cat ctg gcc ttc tac aat gat gga aaa gac     1166
Leu Asp Val Gly Leu Trp His Leu Ala Phe Tyr Asn Asp Gly Lys Asp
                355                 360                 365

aaa gag atg gtt tcc ttc aat act gtt gtc cta gat ggg acc atc         1211
Lys Glu Met Val Ser Phe Asn Thr Val Val Leu Asp Gly Thr Ile
            370                 375                 380

tagttgcaga aaaacaagct cagggcgccc actgatttga cattatgatt cagtgcagga 1271

ctgtccacgt aactgccatg ggaatggtga atgtgtgtcc ggggtgtgtc actgtttccc 1331

aggatttcta ggagcagact gtgctaaaga ccttcctgcc ttgactttct gcaagacaat 1391

cattaataaa gctgctctgt aaatactaaa aaaaaaaca                        1430


<210> 6
<211> 381
<212> PRT
<213> Homo sapiens

<400> 6
Met Leu His Ala Ala Asn Lys Gly Arg Lys Pro Ser Ala Glu Ala Gly
1               5                   10                  15

Arg Pro Ile Pro Pro Thr Ser Ser Pro Ser Leu Leu Pro Ser Ala Gln
            20                  25                  30

Leu Pro Ser Ser His Asn Pro Pro Pro Val Ser Cys Gln Met Pro Leu
        35                  40                  45

Leu Asp Ser Asn Thr Ser His Gln Ile Met Asp Thr Asn Pro Asp Glu
    50                  55                  60

Glu Phe Ser Pro Asn Ser Tyr Leu Leu Arg Ala Cys Ser Gly Pro Gln
65                  70                  75                  80

Gln Ala Ser Ser Ser Gly Pro Pro Asn His His Ser Gln Ser Thr Leu
                85                  90                  95

Arg Pro Pro Leu Pro Pro Pro His Asn His Thr Leu Ser His His His
            100                 105                 110

Ser Ser Ala Asn Ser Leu Asn Arg Asn Ser Leu Thr Asn Arg Arg Ser
        115                 120                 125

Gln Ile His Ala Pro Ala Pro Ala Pro Asn Asp Leu Ala Thr Thr Pro
    130                 135                 140

Glu Ser Val Gln Leu Gln Asp Ser Trp Val Leu Asn Ser Asn Val Pro
145                 150                 155                 160

Leu Glu Thr Arg His Phe Leu Phe Lys Thr Ser Ser Gly Ser Thr Pro
                165                 170                 175

Leu Phe Ser Ser Ser Ser Pro Gly Tyr Pro Leu Thr Ser Gly Thr Val
            180                 185                 190

Tyr Thr Pro Pro Pro Arg Leu Leu Pro Arg Asn Thr Phe Ser Arg Lys
        195                 200                 205

Ala Phe Lys Leu Lys Lys Pro Ser Lys Tyr Cys Ser Trp Lys Cys Ala
    210                 215                 220

Ala Leu Ser Ala Ile Ala Ala Ala Leu Leu Leu Ala Ile Leu Leu Ala
225                 230                 235                 240

Tyr Phe Ile Val Pro Trp Ser Leu Lys Asn Ser Ser Ile Asp Ser Gly
                245                 250                 255

Glu Ala Glu Val Gly Arg Arg Val Thr Gln Glu Val Pro Pro Gly Val
            260                 265                 270

Phe Trp Arg Ser Gln Ile His Ile Ser Gln Pro Gln Phe Leu Lys Phe
        275                 280                 285

Asn Ile Ser Leu Gly Lys Asp Ala Leu Phe Gly Val Tyr Ile Arg Arg
    290                 295                 300

Gly Leu Pro Pro Ser His Ala Gln Tyr Asp Phe Met Glu Arg Leu Asp
305                 310                 315                 320

Gly Lys Glu Lys Trp Ser Val Val Glu Ser Pro Arg Glu Arg Arg Ser
                325                 330                 335

Ile Gln Thr Leu Val Gln Asn Glu Ala Val Phe Val Gln Tyr Leu Asp
            340                 345                 350

Val Gly Leu Trp His Leu Ala Phe Tyr Asn Asp Gly Lys Asp Lys Glu
        355                 360                 365

Met Val Ser Phe Asn Thr Val Val Leu Asp Gly Thr Ile
    370                 375                 380


<210> 7
<211> 9826

<212> DNA
<213> Homo sapiens

<220>
<221> CDS
<222> (280)..(8478)

<400> 7
tttaaatcct cataccttaa aggagatgtg tatataaggg agttggaacc agcattagat 60

gagttgacaa aaatgcagtt tcagttctag aggtctggga agtccaagaa caaggtgctg 120

gcagattgga ttccccgtga gggctttctt cctggcttga agttggctgc tttcctgctg 180

agacttctca tggcagagac tgagggtggc aaagtgacaa gtgccaaaac tcaggcctga 240

cttttctgaa aacatcagca ttctgccata tctggaata atg gat gta aag gac 294
Met Asp Val Lys Asp
1               5

cgg cga cac cgc tct ttg acc aga gga cgc tgt ggc aaa gag tgt cgc 342
Arg Arg His Arg Ser Leu Thr Arg Gly Arg Cys Gly Lys Glu Cys Arg
                10                  15                  20

tac aca agc tcc tct ctg gac agt gag gac tgc cgg gtg ccc aca cag 390
Tyr Thr Ser Ser Ser Leu Asp Ser Glu Asp Cys Arg Val Pro Thr Gln
            25                  30                  35

aaa tcc tac agc tcc agt gag act ctg aag gcc tat gac cat gac agc 438
Lys Ser Tyr Ser Ser Ser Glu Thr Leu Lys Ala Tyr Asp His Asp Ser
        40                  45                  50

agg atg cac tat gga aac cga gtc aca gac ctc atc cac cgg gag tca 486
Arg Met His Tyr Gly Asn Arg Val Thr Asp Leu Ile His Arg Glu Ser
    55                  60                  65

gat gag ttt cct aga caa gga acc aac ttc acc ctt gcc gaa ctg ggc 534
Asp Glu Phe Pro Arg Gln Gly Thr Asn Phe Thr Leu Ala Glu Leu Gly
70                  75                  80                  85

atc tgt gag ccc tcc cca cac cga agc ggc tac tgc tcc gac atg ggg 582
Ile Cys Glu Pro Ser Pro His Arg Ser Gly Tyr Cys Ser Asp Met Gly
                90                  95                  100

atc ctt cac cag ggc tac tcc ctt agc aca ggg tct gac gcc gac tcc 630
Ile Leu His Gln Gly Tyr Ser Leu Ser Thr Gly Ser Asp Ala Asp Ser
            105                 110                 115

gac acc gag gga ggg atg tct cca gaa cac gcc atc aga ctg tgg ggc 678
Asp Thr Glu Gly Gly Met Ser Pro Glu His Ala Ile Arg Leu Trp Gly
        120                 125                 130

aga ggg ata aaa tcc agg cgc agt tcc ggc ctg tcc agt cgt gaa aac 726
Arg Gly Ile Lys Ser Arg Arg Ser Ser Gly Leu Ser Ser Arg Glu Asn
    135                 140                 145

tcg gcc ctt acc ctg act gac tct gac aac gaa aac aaa tca gat gat 774
Ser Ala Leu Thr Leu Thr Asp Ser Asp Asn Glu Asn Lys Ser Asp Asp
150                 155                 160                 165

gag aac ggt cgt ccc att cca cct aca tcc tcg cct agt ctc ctc cca 822

Glu Asn Gly Arg Pro Ile Pro Pro Thr Ser Ser Pro Ser Leu Leu Pro
                170                 175                 180

tct gct cag ctg cct agc tcc cat aat cct cca cca gtt agc tgc cag 870
Ser Ala Gln Leu Pro Ser Ser His Asn Pro Pro Pro Val Ser Cys Gln
            185                 190                 195

atg cca ttg cta gac agc aac acc tcc cat caa atc atg gac acc aac 918
Met Pro Leu Leu Asp Ser Asn Thr Ser His Gln Ile Met Asp Thr Asn
        200                 205                 210

cct gat gag gaa ttc tcc ccc aat tca tac ctg ctc aga gca tgc tca 966
Pro Asp Glu Glu Phe Ser Pro Asn Ser Tyr Leu Leu Arg Ala Cys Ser
    215                 220                 225

ggg ccc cag caa gcc tcc agc agt ggc cct ccg aac cac cac agc cag 1014
Gly Pro Gln Gln Ala Ser Ser Ser Gly Pro Pro Asn His His Ser Gln
230                 235                 240                 245

tcg act ctg agg ccc cct ctc cca ccc cct cac aac cac acg ctg tcc 1062
Ser Thr Leu Arg Pro Pro Leu Pro Pro Pro His Asn His Thr Leu Ser
                250                 255                 260

cat cac cac tcg tcc gcc aac tcc ctc aac agg aac tca ctg acc aat 1110
His His His Ser Ser Ala Asn Ser Leu Asn Arg Asn Ser Leu Thr Asn
            265                 270                 275

cgg cgg agt cag atc cac gcc ccg gcc cca gcg ccc aat gac ctg gcc 1158
Arg Arg Ser Gln Ile His Ala Pro Ala Pro Ala Pro Asn Asp Leu Ala
        280                 285                 290

acc aca cca gag tcc gtt cag ctt cag gac agc tgg gtg cta aac agc 1206
Thr Thr Pro Glu Ser Val Gln Leu Gln Asp Ser Trp Val Leu Asn Ser
    295                 300                 305

aac gtg cca ctg gag acc cgg cac ttc ctc ttc aag acc tcc tcg ggg 1254
Asn Val Pro Leu Glu Thr Arg His Phe Leu Phe Lys Thr Ser Ser Gly
310                 315                 320                 325

agc aca ccc ttg ttc agc agc tct tcc ccg gga tac cct ttg acc tca 1302
Ser Thr Pro Leu Phe Ser Ser Ser Ser Pro Gly Tyr Pro Leu Thr Ser
                330                 335                 340

gga acg gtt tac acg ccc ccg ccc cgc ctg ctg ccc agg aat act ttc 1350
Gly Thr Val Tyr Thr Pro Pro Pro Arg Leu Leu Pro Arg Asn Thr Phe
            345                 350                 355

tcc agg aag gct ttc aag ctg aag aag ccc tcc aaa tac tgc agc tgg 1398
Ser Arg Lys Ala Phe Lys Leu Lys Lys Pro Ser Lys Tyr Cys Ser Trp
        360                 365                 370

aaa tgt gct gcc ctc tcc gcc att gcc gcg gcc ctc ctc ttg gct att 1446
Lys Cys Ala Ala Leu Ser Ala Ile Ala Ala Ala Leu Leu Leu Ala Ile
    375                 380                 385

ttg ctg gcg tat ttc ata gtg ccc tgg tcg ttg aaa aac agc agc ata 1494
Leu Leu Ala Tyr Phe Ile Val Pro Trp Ser Leu Lys Asn Ser Ser Ile
390                 395                 400                 405

gac agt ggt gaa gca gaa gtt ggt cgg cgg gta aca caa gaa gtc cca 1542
Asp Ser Gly Glu Ala Glu Val Gly Arg Arg Val Thr Gln Glu Val Pro

410                 415                 420

cca ggg gtg ttt tgg agg tca caa att cac atc agt cag ccc cag ttc     1590
Pro Gly Val Phe Trp Arg Ser Gln Ile His Ile Ser Gln Pro Gln Phe
            425                 430                 435

tta aag ttc aac atc tcc ctc ggg aag gac gct ctc ttt ggt gtt tac     1638
Leu Lys Phe Asn Ile Ser Leu Gly Lys Asp Ala Leu Phe Gly Val Tyr
        440                 445                 450

ata aga aga gga ctt cca cca tct cat gcc cag tat gac ttc atg gaa     1686
Ile Arg Arg Gly Leu Pro Pro Ser His Ala Gln Tyr Asp Phe Met Glu
    455                 460                 465

cgt ctg gac ggg aag gag aag tgg agt gtg gtt gag tct ccc agg gaa     1734
Arg Leu Asp Gly Lys Glu Lys Trp Ser Val Val Glu Ser Pro Arg Glu
470                 475                 480                 485

cgc cgg agc ata cag acc ttg gtt cag aat gaa gcc gtg ttt gtg cag     1782
Arg Arg Ser Ile Gln Thr Leu Val Gln Asn Glu Ala Val Phe Val Gln
                490                 495                 500

tac ctg gat gtg ggc ctg tgg cat ctg gcc ttc tac aat gat gga aaa     1830
Tyr Leu Asp Val Gly Leu Trp His Leu Ala Phe Tyr Asn Asp Gly Lys
            505                 510                 515

gac aaa gag atg gtt tcc ttc aat act gtt gtc cta gat tca gtg cag     1878
Asp Lys Glu Met Val Ser Phe Asn Thr Val Val Leu Asp Ser Val Gln
        520                 525                 530

gac tgt cca cgt aac tgc cat ggg aat ggt gaa tgt gtg tcc ggg gtg     1926
Asp Cys Pro Arg Asn Cys His Gly Asn Gly Glu Cys Val Ser Gly Val
    535                 540                 545

tgt cac tgt ttc cca gga ttt cta gga gca gac tgt gct aaa gct gcc     1974
Cys His Cys Phe Pro Gly Phe Leu Gly Ala Asp Cys Ala Lys Ala Ala
550                 555                 560                 565

tgc cct gtc ctg tgc agt ggg aat gga caa tat tct aaa ggg acg tgc     2022
Cys Pro Val Leu Cys Ser Gly Asn Gly Gln Tyr Ser Lys Gly Thr Cys
                570                 575                 580

cag tgc tac agc ggc tgg aaa ggt gca gag tgc gac gtg ccc atg aat     2070
Gln Cys Tyr Ser Gly Trp Lys Gly Ala Glu Cys Asp Val Pro Met Asn
            585                 590                 595

cag tgc atc gat cct tcc tgc ggg ggc cac ggc tcc tgc att gat ggg     2118
Gln Cys Ile Asp Pro Ser Cys Gly Gly His Gly Ser Cys Ile Asp Gly
        600                 605                 610

aac tgt gtc tgc tct gct ggc tac aaa ggc gag cac tgt gag gaa gtt     2166
Asn Cys Val Cys Ser Ala Gly Tyr Lys Gly Glu His Cys Glu Glu Val
    615                 620                 625

gat tgc ttg gat ccc acc tgc tcc agc cac gga gtc tgt gtg aat gga     2214
Asp Cys Leu Asp Pro Thr Cys Ser Ser His Gly Val Cys Val Asn Gly
630                 635                 640                 645

gaa tgc ctg tgc agc cct ggc tgg ggt ggt ctg aac tgt gag ctg gcg     2262
Glu Cys Leu Cys Ser Pro Gly Trp Gly Gly Leu Asn Cys Glu Leu Ala
                650                 655                 660

agg gtc cag tgc cca gac cag tgc agt ggg cat ggc acg tac ctg cct 2310
Arg Val Gln Cys Pro Asp Gln Cys Ser Gly His Gly Thr Tyr Leu Pro
            665                 670                 675

gac acg ggc ctc tgc agc tgc gat ccc aac tgg atg ggt ccc gac tgc 2358
Asp Thr Gly Leu Cys Ser Cys Asp Pro Asn Trp Met Gly Pro Asp Cys
        680                 685                 690

tct gtt gaa gtg tgc tca gta gac tgt ggc act cac ggc gtc tgc atc 2406
Ser Val Glu Val Cys Ser Val Asp Cys Gly Thr His Gly Val Cys Ile
    695                 700                 705

ggg gga gcc tgc cgc tgt gaa gag ggc tgg aca ggc gca gcg tgt gac 2454
Gly Gly Ala Cys Arg Cys Glu Glu Gly Trp Thr Gly Ala Ala Cys Asp
710                 715                 720                 725

cag cgc gtg tgc cac ccc cgc tgc att gag cac ggg acc tgt aaa gat 2502
Gln Arg Val Cys His Pro Arg Cys Ile Glu His Gly Thr Cys Lys Asp
                730                 735                 740

ggc aaa tgt gaa tgc cga gag ggc tgg aat ggt gaa cac tgc acc att 2550
Gly Lys Cys Glu Cys Arg Glu Gly Trp Asn Gly Glu His Cys Thr Ile
            745                 750                 755

ggt agg caa acg gca ggc acc gaa aca gat ggc tgc cct gac ttg tgc 2598
Gly Arg Gln Thr Ala Gly Thr Glu Thr Asp Gly Cys Pro Asp Leu Cys
        760                 765                 770

aac ggt aac ggg aga tgc aca ctg ggt cag aac agc tgg cag tgt gtc 2646
Asn Gly Asn Gly Arg Cys Thr Leu Gly Gln Asn Ser Trp Gln Cys Val
    775                 780                 785

tgc cag acc ggc tgg aga ggg ccc gga tgc aac gtt gcc atg gaa act 2694
Cys Gln Thr Gly Trp Arg Gly Pro Gly Cys Asn Val Ala Met Glu Thr
790                 795                 800                 805

tcc tgt gct gat aac aag gat aat gag gga gat ggc ctg gtg gat tgt 2742
Ser Cys Ala Asp Asn Lys Asp Asn Glu Gly Asp Gly Leu Val Asp Cys
                810                 815                 820

ttg gac cct gac tgc tgc ctg cag tca gcc tgt cag aac agc ctg ctc 2790
Leu Asp Pro Asp Cys Cys Leu Gln Ser Ala Cys Gln Asn Ser Leu Leu
            825                 830                 835

tgc cgg ggg tcc cgg gac cca ctg gac atc att cag cag ggc cag acg 2838
Cys Arg Gly Ser Arg Asp Pro Leu Asp Ile Ile Gln Gln Gly Gln Thr
        840                 845                 850

gat tgg ccc gca gtg aag tcc ttc tat gac cgt atc aag ctc ttg gca 2886
Asp Trp Pro Ala Val Lys Ser Phe Tyr Asp Arg Ile Lys Leu Leu Ala
    855                 860                 865

ggc aag gat agc acc cac atc att cct gga gag aac cct ttc aac agc 2934
Gly Lys Asp Ser Thr His Ile Ile Pro Gly Glu Asn Pro Phe Asn Ser
870                 875                 880                 885

agc ttg gtt tct ctc atc cga ggc caa gta gta act aca gat gga act 2982
Ser Leu Val Ser Leu Ile Arg Gly Gln Val Val Thr Thr Asp Gly Thr
                890                 895                 900

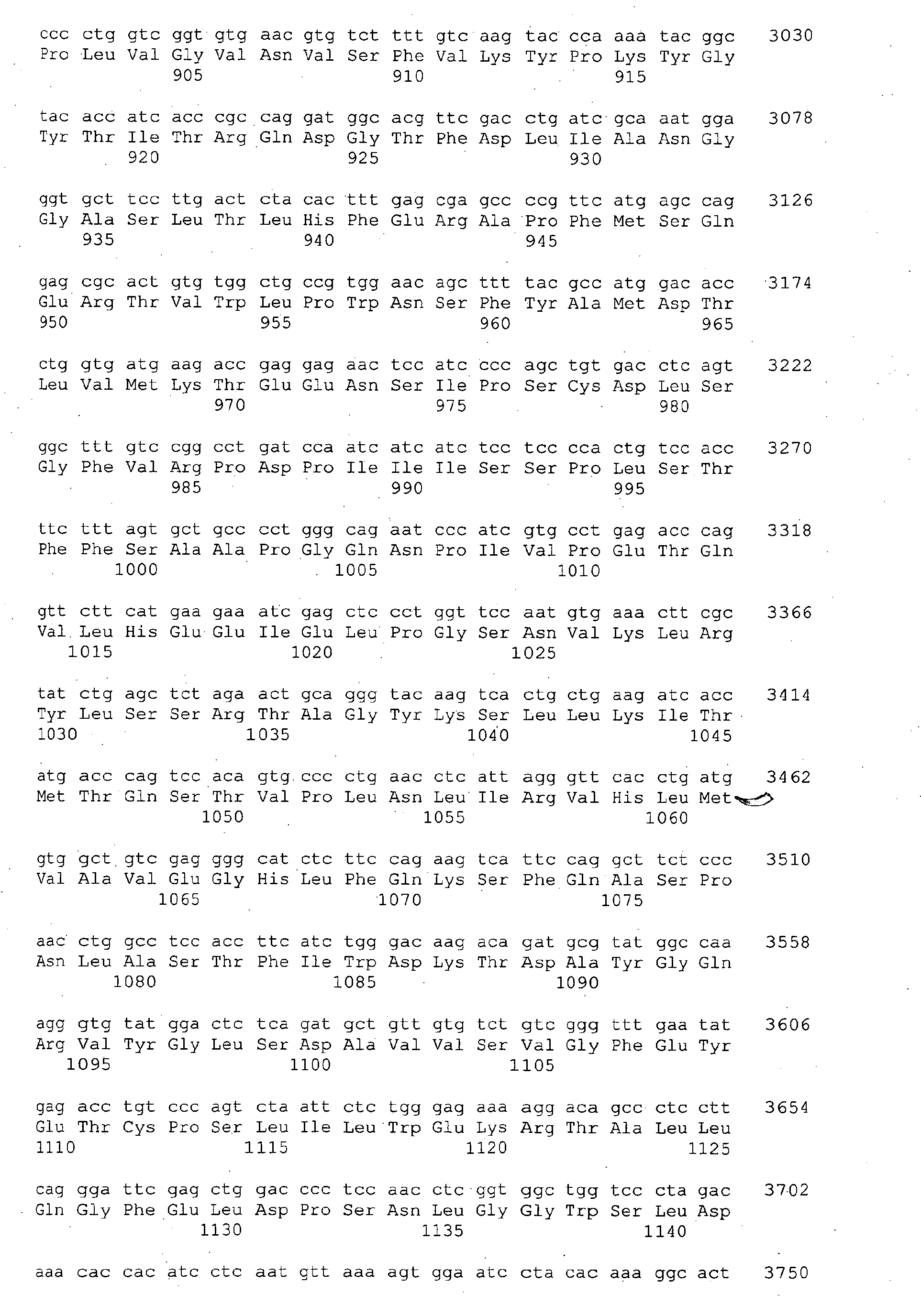
ccc ctg gtc ggt gtg aac gtg tct ttt gtc aag tac cca aaa tac ggc 3030
Pro Leu Val Gly Val Asn Val Ser Phe Val Lys Tyr Pro Lys Tyr Gly
905 910 915

tac acc atc acc cgc cag gat ggc acg ttc gac ctg atc gca aat gga 3078
Tyr Thr Ile Thr Arg Gln Asp Gly Thr Phe Asp Leu Ile Ala Asn Gly
920 925 930

ggt gct tcc ttg act cta cac ttt gag cga gcc ccg ttc atg agc cag 3126
Gly Ala Ser Leu Thr Leu His Phe Glu Arg Ala Pro Phe Met Ser Gln
935 940 945

gag cgc act gtg tgg ctg ccg tgg aac agc ttt tac gcc atg gac acc 3174
Glu Arg Thr Val Trp Leu Pro Trp Asn Ser Phe Tyr Ala Met Asp Thr
950 955 960 965

ctg gtg atg aag acc gag gag aac tcc atc ccc agc tgt gac ctc agt 3222
Leu Val Met Lys Thr Glu Glu Asn Ser Ile Pro Ser Cys Asp Leu Ser
970 975 980

ggc ttt gtc cgg cct gat cca atc atc atc tcc tcc cca ctg tcc acc 3270
Gly Phe Val Arg Pro Asp Pro Ile Ile Ile Ser Ser Pro Leu Ser Thr
985 990 995

ttc ttt agt gct gcc cct ggg cag aat ccc atc gtg cct gag acc cag 3318
Phe Phe Ser Ala Ala Pro Gly Gln Asn Pro Ile Val Pro Glu Thr Gln
1000 1005 1010

gtt ctt cat gaa gaa atc gag ctc cct ggt tcc aat gtg aaa ctt cgc 3366
Val Leu His Glu Glu Ile Glu Leu Pro Gly Ser Asn Val Lys Leu Arg
1015 1020 1025

tat ctg agc tct aga act gca ggg tac aag tca ctg ctg aag atc acc 3414
Tyr Leu Ser Ser Arg Thr Ala Gly Tyr Lys Ser Leu Leu Lys Ile Thr
1030 1035 1040 1045

atg acc cag tcc aca gtg ccc ctg aac ctc att agg gtt cac ctg atg 3462
Met Thr Gln Ser Thr Val Pro Leu Asn Leu Ile Arg Val His Leu Met
1050 1055 1060

gtg gct gtc gag ggg cat ctc ttc cag aag tca ttc cag gct tct ccc 3510
Val Ala Val Glu Gly His Leu Phe Gln Lys Ser Phe Gln Ala Ser Pro
1065 1070 1075

aac ctg gcc tcc acc ttc atc tgg gac aag aca gat gcg tat ggc caa 3558
Asn Leu Ala Ser Thr Phe Ile Trp Asp Lys Thr Asp Ala Tyr Gly Gln
1080 1085 1090

agg gtg tat gga ctc tca gat gct gtt gtg tct gtc ggg ttt gaa tat 3606
Arg Val Tyr Gly Leu Ser Asp Ala Val Val Ser Val Gly Phe Glu Tyr
1095 1100 1105

gag acc tgt ccc agt cta att ctc tgg gag aaa agg aca gcc ctc ctt 3654
Glu Thr Cys Pro Ser Leu Ile Leu Trp Glu Lys Arg Thr Ala Leu Leu
1110 1115 1120 1125

cag gga ttc gag ctg gac ccc tcc aac ctc ggt ggc tgg tcc cta gac 3702
Gln Gly Phe Glu Leu Asp Pro Ser Asn Leu Gly Gly Trp Ser Leu Asp
1130 1135 1140

aaa cac cac atc ctc aat gtt aaa agt gga atc cta cac aaa ggc act 3750

Lys His His Ile Leu Asn Val Lys Ser Gly Ile Leu His Lys Gly Thr
1145 1150 1155

ggg gaa aac cag ttc ctg acc cag cag cct gcc atc atc acc agc atc 3798
Gly Glu Asn Gln Phe Leu Thr Gln Gln Pro Ala Ile Ile Thr Ser Ile
1160 1165 1170

atg ggc aat ggt cgc cgc cgg agc att tcc tgt ccc agc tgc aac ggc 3846
Met Gly Asn Gly Arg Arg Arg Ser Ile Ser Cys Pro Ser Cys Asn Gly
1175 1180 1185

ctt gct gaa ggc aac aag ctg ctg gcc cca gtg gct ctg gct gtt gga 3894
Leu Ala Glu Gly Asn Lys Leu Leu Ala Pro Val Ala Leu Ala Val Gly
1190 1195 1200 1205

atc gat ggg agc ctc tat gtg ggt gac ttc aat tac atc cga cgc atc 3942
Ile Asp Gly Ser Leu Tyr Val Gly Asp Phe Asn Tyr Ile Arg Arg Ile
1210 1215 1220

ttt ccc tct cga aat gtg acc agc atc ttg gag tta cga aat aaa gag 3990
Phe Pro Ser Arg Asn Val Thr Ser Ile Leu Glu Leu Arg Asn Lys Glu
1225 1230 1235

ttt aaa cat agc aac aac cca gca cac aag tac tac ttg gca gtg gac 4038
Phe Lys His Ser Asn Asn Pro Ala His Lys Tyr Tyr Leu Ala Val Asp
1240 1245 1250

ccc gtg tcc ggc tcg ctc tac gtg tcc gac acc aac agc agg aga atc 4086
Pro Val Ser Gly Ser Leu Tyr Val Ser Asp Thr Asn Ser Arg Arg Ile
1255 1260 1265

tac cgc gtc aag tct ctg agt gga acc aaa gac ctg gct ggg aat tcg 4134
Tyr Arg Val Lys Ser Leu Ser Gly Thr Lys Asp Leu Ala Gly Asn Ser
1270 1275 1280 1285

gaa gtt gtg gca ggg acg gga gag cag tgt cta ccc ttt gat gaa gcc 4182
Glu Val Val Ala Gly Thr Gly Glu Gln Cys Leu Pro Phe Asp Glu Ala
1290 1295 1300

cgc tgc ggg gat gga ggg aag gcc ata gat gca acc ctg atg agc ccg 4230
Arg Cys Gly Asp Gly Gly Lys Ala Ile Asp Ala Thr Leu Met Ser Pro
1305 1310 1315

aga ggt att gca gta gac aag aat ggg ctc atg tac ttt gtc gat gcc 4278
Arg Gly Ile Ala Val Asp Lys Asn Gly Leu Met Tyr Phe Val Asp Ala
1320 1325 1330

acc atg atc cgg aag gtt gac cag aat gga atc atc tcc acc ctg ctg 4326
Thr Met Ile Arg Lys Val Asp Gln Asn Gly Ile Ile Ser Thr Leu Leu
1335 1340 1345

ggc tcc aat gac ctc act gcc gtc cgg ccg ctg agc tgt gat tcc agc 4374
Gly Ser Asn Asp Leu Thr Ala Val Arg Pro Leu Ser Cys Asp Ser Ser
1350 1355 1360 1365

atg gat gta gcc cag gtt cgt ctg gag tgg cca aca gac ctt gct gtc 4422
Met Asp Val Ala Gln Val Arg Leu Glu Trp Pro Thr Asp Leu Ala Val
1370 1375 1380

aat ccc atg gat aac tcc ttg tat gtt cta gag aac aat gtc atc ctt 4470
Asn Pro Met Asp Asn Ser Leu Tyr Val Leu Glu Asn Asn Val Ile Leu

1385                1390                1395

cga atc acc gag aac cac caa gtc agc atc att gcg gga cgc ccc atg    4518
Arg Ile Thr Glu Asn His Gln Val Ser Ile Ile Ala Gly Arg Pro Met
        1400                1405                1410

cac tgc caa gtt cct ggc att gac tac tca ctc agc aaa cta gcc att    4566
His Cys Gln Val Pro Gly Ile Asp Tyr Ser Leu Ser Lys Leu Ala Ile
    1415                1420                1425

cac tct gcc ctg gag tca gcc agt gcc att gcc att tct cac act ggg    4614
His Ser Ala Leu Glu Ser Ala Ser Ala Ile Ala Ile Ser His Thr Gly
1430                1435                1440                1445

gtc ctc tac atc act gag aca gat gag aag aag att aac cgt cta cgc    4662
Val Leu Tyr Ile Thr Glu Thr Asp Glu Lys Lys Ile Asn Arg Leu Arg
                1450                1455                1460

cag gta aca acc aac ggg gag atc tgc ctt tta gct ggg gca gcc tcg    4710
Gln Val Thr Thr Asn Gly Glu Ile Cys Leu Leu Ala Gly Ala Ala Ser
            1465                1470                1475

gac tgc gac tgc aaa aac gat gtc aat tgc aac tgc tat tca gga gat    4758
Asp Cys Asp Cys Lys Asn Asp Val Asn Cys Asn Cys Tyr Ser Gly Asp
        1480                1485                1490

gat gcc tac gcg act gat gcc atc ttg aat tcc cca tca tcc tta gct    4806
Asp Ala Tyr Ala Thr Asp Ala Ile Leu Asn Ser Pro Ser Ser Leu Ala
    1495                1500                1505

gta gct cca gat ggt acc att tac att gca gac ctt gga aat att cgg    4854
Val Ala Pro Asp Gly Thr Ile Tyr Ile Ala Asp Leu Gly Asn Ile Arg
1510                1515                1520                1525

atc agg gcg gtc agc aag aac aag cct gtt ctt aat gcc ttc aac cag    4902
Ile Arg Ala Val Ser Lys Asn Lys Pro Val Leu Asn Ala Phe Asn Gln
                1530                1535                1540

tat gag gct gca tcc ccc gga gag cag gag tta tat gtt ttc aac gct    4950
Tyr Glu Ala Ala Ser Pro Gly Glu Gln Glu Leu Tyr Val Phe Asn Ala
            1545                1550                1555

gat ggc atc cac caa tac act gtg agc ctg gtg aca ggg gag tac ttg    4998
Asp Gly Ile His Gln Tyr Thr Val Ser Leu Val Thr Gly Glu Tyr Leu
        1560                1565                1570

tac aat ttc aca tat agt act gac aat gat gtc act gaa ttg att gac    5046
Tyr Asn Phe Thr Tyr Ser Thr Asp Asn Asp Val Thr Glu Leu Ile Asp
    1575                1580                1585

aat aat ggg aat tcc ctg aag atc cgt cgg gac agc agt ggc atg ccc    5094
Asn Asn Gly Asn Ser Leu Lys Ile Arg Arg Asp Ser Ser Gly Met Pro
1590                1595                1600                1605

cgt cac ctg ctc atg cct gac aac cag atc atc acc ctc acc gtg ggc    5142
Arg His Leu Leu Met Pro Asp Asn Gln Ile Ile Thr Leu Thr Val Gly
                1610                1615                1620

acc aat gga ggc ctc aaa gtc gtg tcc aca cag aac ctg gag ctt ggt    5190
Thr Asn Gly Gly Leu Lys Val Val Ser Thr Gln Asn Leu Glu Leu Gly
            1625                1630                1635

ctc atg acc tat gat ggc aac act ggg ctc ctg gcc acc aag agc gat 5238
Leu Met Thr Tyr Asp Gly Asn Thr Gly Leu Leu Ala Thr Lys Ser Asp
1640 1645 1650

gaa aca gga tgg acg act ttc tat gac tat gac cac gaa ggc cgc ctg 5286
Glu Thr Gly Trp Thr Thr Phe Tyr Asp Tyr Asp His Glu Gly Arg Leu
1655 1660 1665

acc aac gtg acg cgc ccc acg ggg gtg gta acc agt ctg cac cgg gaa 5334
Thr Asn Val Thr Arg Pro Thr Gly Val Val Thr Ser Leu His Arg Glu
1670 1675 1680 1685

atg gag aaa tct att acc att gac att gag aac tcc aac cgt gat gat 5382
Met Glu Lys Ser Ile Thr Ile Asp Ile Glu Asn Ser Asn Arg Asp Asp
1690 1695 1700

gac gtc act gtc atc acc aac ctc tct tca gta gag gcc tcc tac aca 5430
Asp Val Thr Val Ile Thr Asn Leu Ser Ser Val Glu Ala Ser Tyr Thr
1705 1710 1715

gtg gta caa gat caa gtt cgg aac agc tac cag ctc tgt aat aat ggt 5478
Val Val Gln Asp Gln Val Arg Asn Ser Tyr Gln Leu Cys Asn Asn Gly
1720 1725 1730

acc ctg agg gtg atg tat gct aat ggg atg ggt atc agc ttc cac agc 5526
Thr Leu Arg Val Met Tyr Ala Asn Gly Met Gly Ile Ser Phe His Ser
1735 1740 1745

gag ccc cat gtc cta gcg ggc acc atc acc ccc acc att gga cgc tgc 5574
Glu Pro His Val Leu Ala Gly Thr Ile Thr Pro Thr Ile Gly Arg Cys
1750 1755 1760 1765

aac atc tcc ctg cct atg gag aat ggc tta aac tcc att gag tgg cgc 5622
Asn Ile Ser Leu Pro Met Glu Asn Gly Leu Asn Ser Ile Glu Trp Arg
1770 1775 1780

cta aga aag gaa cag att aaa ggc aaa gtc acc atc ttt ggc agg aag 5670
Leu Arg Lys Glu Gln Ile Lys Gly Lys Val Thr Ile Phe Gly Arg Lys
1785 1790 1795

ctc cgg gtc cat gga aga aat ctc ttg tcc att gac tat gat cga aat 5718
Leu Arg Val His Gly Arg Asn Leu Leu Ser Ile Asp Tyr Asp Arg Asn
1800 1805 1810

att cgg act gaa aag atc tat gat gac cac cgg aag ttc acc ctg agg 5766
Ile Arg Thr Glu Lys Ile Tyr Asp Asp His Arg Lys Phe Thr Leu Arg
1815 1820 1825

atc att tat gac cag gtg ggc cgc ccc ttc ctc tgg ctg ccc agc agc 5814
Ile Ile Tyr Asp Gln Val Gly Arg Pro Phe Leu Trp Leu Pro Ser Ser
1830 1835 1840 1845

ggg ctg gca gct gtc aac gtg tca tac ttc ttc aat ggg cgc ctg gct 5862
Gly Leu Ala Ala Val Asn Val Ser Tyr Phe Phe Asn Gly Arg Leu Ala
1850 1855 1860

ggg ctt cag cgt ggg gcc atg agc gag agg aca gac atc gac aag caa 5910
Gly Leu Gln Arg Gly Ala Met Ser Glu Arg Thr Asp Ile Asp Lys Gln
1865 1870 1875

ggc cgc atc gtg tcc cgc atg ttc gct gac ggg aaa gtg tgg agc tac 5958
Gly Arg Ile Val Ser Arg Met Phe Ala Asp Gly Lys Val Trp Ser Tyr
1880 1885 1890

tcc tac ctt gac aag tcc atg gtc ctc ctg ctt cag agc caa cgt cag 6006
Ser Tyr Leu Asp Lys Ser Met Val Leu Leu Leu Gln Ser Gln Arg Gln
1895 1900 1905

tat ata ttt gag tat gac tcc tct gac cgc ctc ctt gcc gtc acc atg 6054
Tyr Ile Phe Glu Tyr Asp Ser Ser Asp Arg Leu Leu Ala Val Thr Met
1910 1915 1920 1925

ccc agc gtg gcc cgg cac agc atg tcc aca cac acc tcc atc ggc tac 6102
Pro Ser Val Ala Arg His Ser Met Ser Thr His Thr Ser Ile Gly Tyr
1930 1935 1940

atc cgt aat att tac aac ccg cct gaa agc aat gct tcg gtc atc ttt 6150
Ile Arg Asn Ile Tyr Asn Pro Pro Glu Ser Asn Ala Ser Val Ile Phe
1945 1950 1955

gac tac agt gat gac ggc cgc atc ctg aag acc tcc ttt ttg ggc acc 6198
Asp Tyr Ser Asp Asp Gly Arg Ile Leu Lys Thr Ser Phe Leu Gly Thr
1960 1965 1970

gga cgc cag gtg ttc tac aag tat ggg aaa ctc tcc aag tta tca gag 6246
Gly Arg Gln Val Phe Tyr Lys Tyr Gly Lys Leu Ser Lys Leu Ser Glu
1975 1980 1985

att gtc tac gac agt acc gcc gtc acc ttc ggg tat gac gag acc act 6294
Ile Val Tyr Asp Ser Thr Ala Val Thr Phe Gly Tyr Asp Glu Thr Thr
1990 1995 2000 2005

ggt gtc ttg aag atg gtc aac ctc caa agt ggg ggc ttc tcc tgc acc 6342
Gly Val Leu Lys Met Val Asn Leu Gln Ser Gly Gly Phe Ser Cys Thr
2010 2015 2020

atc agg tac cgg aag att ggc ccc ctg gtg gac aag cag atc tac agg 6390
Ile Arg Tyr Arg Lys Ile Gly Pro Leu Val Asp Lys Gln Ile Tyr Arg
2025 2030 2035

ttc tcc gag gaa ggc atg gtc aat gcc agg ttt gac tac acc tat cat 6438
Phe Ser Glu Glu Gly Met Val Asn Ala Arg Phe Asp Tyr Thr Tyr His
2040 2045 2050

gac aac agc ttc cgc atc gca agc atc aag ccc gtc ata agt gag act 6486
Asp Asn Ser Phe Arg Ile Ala Ser Ile Lys Pro Val Ile Ser Glu Thr
2055 2060 2065

ccc ctc ccc gtt gac ctc tac cgc tat gat gag att tct ggc aag gtg 6534
Pro Leu Pro Val Asp Leu Tyr Arg Tyr Asp Glu Ile Ser Gly Lys Val
2070 2075 2080 2085

gaa cac ttt ggt aag ttt gga gtc atc tat tat gac atc aac cag atc 6582
Glu His Phe Gly Lys Phe Gly Val Ile Tyr Tyr Asp Ile Asn Gln Ile
2090 2095 2100

atc acc act gcc gtg atg acc ctc agc aaa cac ttc gac acc cat ggg 6630
Ile Thr Thr Ala Val Met Thr Leu Ser Lys His Phe Asp Thr His Gly
2105 2110 2115

cgg atc aag gag gtc cag tat gag atg ttc cgg tcc ctc atg tac tgg 6678

Arg Ile Lys Glu Val Gln Tyr Glu Met Phe Arg Ser Leu Met Tyr Trp
2120 2125 2130

atg acg gtg caa tat gac agc atg ggc agg gtg atc aag agg gag cta 6726
Met Thr Val Gln Tyr Asp Ser Met Gly Arg Val Ile Lys Arg Glu Leu
2135 2140 2145

aaa ctg ggg ccc tat gcc aat acc acg aag tac acc tat gac tac gat 6774
Lys Leu Gly Pro Tyr Ala Asn Thr Thr Lys Tyr Thr Tyr Asp Tyr Asp
2150 2155 2160 2165

ggg gac ggg cag ctc cag agc gtg gcc gtc aat gac cgc ccg acc tgg 6822
Gly Asp Gly Gln Leu Gln Ser Val Ala Val Asn Asp Arg Pro Thr Trp
2170 2175 2180

cgc tac agc tat gac ctt aat ggg aat ctc cac tta ctg aac cca ggc 6870
Arg Tyr Ser Tyr Asp Leu Asn Gly Asn Leu His Leu Leu Asn Pro Gly
2185 2190 2195

aac agt gtg cgc ctc atg ccc ttg cgc tat gac ctc cgg gat cgg ata 6918
Asn Ser Val Arg Leu Met Pro Leu Arg Tyr Asp Leu Arg Asp Arg Ile
2200 2205 2210

acc aga ctc ggg gat gtg cag tac aaa att gac gac gat ggc tat ctg 6966
Thr Arg Leu Gly Asp Val Gln Tyr Lys Ile Asp Asp Asp Gly Tyr Leu
2215 2220 2225

tgc cag aga ggg tct gac atc ttc gaa tac aat tcc aag ggc ctc cta 7014
Cys Gln Arg Gly Ser Asp Ile Phe Glu Tyr Asn Ser Lys Gly Leu Leu
2230 2235 2240 2245

aca aga gcc tac aac aag gcc agc ggg tgg agt gtc cag tac cgc tat 7062
Thr Arg Ala Tyr Asn Lys Ala Ser Gly Trp Ser Val Gln Tyr Arg Tyr
2250 2255 2260

gat ggc gta gga cgg cgg gct tcc tac aag acc aac ctg ggc cac cac 7110
Asp Gly Val Gly Arg Arg Ala Ser Tyr Lys Thr Asn Leu Gly His His
2265 2270 2275

ctg cag tac ttc tac tct gac ctc cac aac ccg acg cgc atc acc cat 7158
Leu Gln Tyr Phe Tyr Ser Asp Leu His Asn Pro Thr Arg Ile Thr His
2280 2285 2290

gtc tac aat cac tcc aac tcg gag att acc tca ctg tac tac gac ctc 7206
Val Tyr Asn His Ser Asn Ser Glu Ile Thr Ser Leu Tyr Tyr Asp Leu
2295 2300 2305

cag ggc cac ctc ttt gcc atg gag agc agc agt ggg gag gag tac tat 7254
Gln Gly His Leu Phe Ala Met Glu Ser Ser Ser Gly Glu Glu Tyr Tyr
2310 2315 2320 2325

gtt gcc tct gat aac aca ggg act cct ctg gct gtg ttc agc atc aac 7302
Val Ala Ser Asp Asn Thr Gly Thr Pro Leu Ala Val Phe Ser Ile Asn
2330 2335 2340

ggc ctc atg atc aaa cag ctg cag tac acg gcc tat ggg gag att tat 7350
Gly Leu Met Ile Lys Gln Leu Gln Tyr Thr Ala Tyr Gly Glu Ile Tyr
2345 2350 2355

tat gac tcc aac ccc gac ttc cag atg gtc att ggc ttc cat ggg gga 7398
Tyr Asp Ser Asn Pro Asp Phe Gln Met Val Ile Gly Phe His Gly Gly

2360                2365                2370

ctc tat gac ccc ctg acc aag ctg gtc cac ttc act cag cgt gat tat     7446
Leu Tyr Asp Pro Leu Thr Lys Leu Val His Phe Thr Gln Arg Asp Tyr
    2375                2380                2385

gat gtg ctg gca gga cga tgg acc tcc cca gac tat acc atg tgg aaa     7494
Asp Val Leu Ala Gly Arg Trp Thr Ser Pro Asp Tyr Thr Met Trp Lys
2390                2395                2400                2405

aac gtg ggc aag gag ccg gcc ccc ttt aac ctg tat atg ttc aag agc     7542
Asn Val Gly Lys Glu Pro Ala Pro Phe Asn Leu Tyr Met Phe Lys Ser
                2410                2415                2420

aac aat cct ctc agc agt gag cta gat ttg aag aac tac gtg aca gat     7590
Asn Asn Pro Leu Ser Ser Glu Leu Asp Leu Lys Asn Tyr Val Thr Asp
            2425                2430                2435

gtg aaa agc tgg ctt gtg atg ttt gga ttt cag ctt agc aac atc att     7638
Val Lys Ser Trp Leu Val Met Phe Gly Phe Gln Leu Ser Asn Ile Ile
        2440                2445                2450

cct ggc ttc ccg aga gcc aaa atg tat ttc gtg cct cct ccc tat gaa     7686
Pro Gly Phe Pro Arg Ala Lys Met Tyr Phe Val Pro Pro Pro Tyr Glu
    2455                2460                2465

ttg tca gag agt caa gca agt gag aat gga cag ctc att aca ggt gtc     7734
Leu Ser Glu Ser Gln Ala Ser Glu Asn Gly Gln Leu Ile Thr Gly Val
2470                2475                2480                2485

caa cag aca aca gag aga cat aac cag gcc ttc atg gct ctg gaa gga     7782
Gln Gln Thr Thr Glu Arg His Asn Gln Ala Phe Met Ala Leu Glu Gly
                2490                2495                2500

cag gtc att act aaa aag ctc cac gcc agc atc cga gag aaa gca ggt     7830
Gln Val Ile Thr Lys Lys Leu His Ala Ser Ile Arg Glu Lys Ala Gly
            2505                2510                2515

cac tgg ttt gcc acc acc acg ccc atc att ggc aaa ggc atc atg ttt     7878
His Trp Phe Ala Thr Thr Thr Pro Ile Ile Gly Lys Gly Ile Met Phe
        2520                2525                2530

gcc atc aaa gaa ggg cgg gtg acc acg ggc gtg tcc agc atc gcc agc     7926
Ala Ile Lys Glu Gly Arg Val Thr Thr Gly Val Ser Ser Ile Ala Ser
    2535                2540                2545

gaa gat agc cgc aag gtg gca tct gtg ctg aac aac gcc tac tac ctg     7974
Glu Asp Ser Arg Lys Val Ala Ser Val Leu Asn Asn Ala Tyr Tyr Leu
2550                2555                2560                2565

gac aag atg cac tac agc atc gag ggc aag gac acc cac tac ttt gtg     8022
Asp Lys Met His Tyr Ser Ile Glu Gly Lys Asp Thr His Tyr Phe Val
                2570                2575                2580

aag att ggc tca gcc gat ggc gac ctg gtc aca cta ggc acc acc atc     8070
Lys Ile Gly Ser Ala Asp Gly Asp Leu Val Thr Leu Gly Thr Thr Ile
            2585                2590                2595

ggc cgc aag gtg cta gag agc ggg gtg aac gtg acc gtg tcc cag ccc     8118
Gly Arg Lys Val Leu Glu Ser Gly Val Asn Val Thr Val Ser Gln Pro
        2600                2605                2610

acg ctg ctg gtc aac ggc agg act cga agg ttc acg aac att gag ttc 8166
Thr Leu Leu Val Asn Gly Arg Thr Arg Arg Phe Thr Asn Ile Glu Phe
2615 2620 2625

cag tac tcc acg ctg ctg ctc agc atc cgc tat ggc ctc acc ccc gac 8214
Gln Tyr Ser Thr Leu Leu Leu Ser Ile Arg Tyr Gly Leu Thr Pro Asp
2630 2635 2640 2645

acc ctg gac gaa gag aag gcc cgc gtc ctg gac cag gcg aga cag agg 8262
Thr Leu Asp Glu Glu Lys Ala Arg Val Leu Asp Gln Ala Arg Gln Arg
2650 2655 2660

gcc ctg ggc acg gcc tgg gcc aag gag cag cag aaa gcc agg gac ggg 8310
Ala Leu Gly Thr Ala Trp Ala Lys Glu Gln Gln Lys Ala Arg Asp Gly
2665 2670 2675

aga gag ggg agc cgc ctg tgg act gag ggc gag aag cag cag ctt ctg 8358
Arg Glu Gly Ser Arg Leu Trp Thr Glu Gly Glu Lys Gln Gln Leu Leu
2680 2685 2690

agc acc ggg cgc gtg caa ggg tac gag gga tat tac gtg ctt ccc gtg 8406
Ser Thr Gly Arg Val Gln Gly Tyr Glu Gly Tyr Tyr Val Leu Pro Val
2695 2700 2705

gag caa tac cca gag ctt gca gac agt agc agc aac atc cag ttt tta 8454
Glu Gln Tyr Pro Glu Leu Ala Asp Ser Ser Ser Asn Ile Gln Phe Leu
2710 2715 2720 2725

aga cag aat gag atg gga aag agg taacaaaata atctgctgcc attccttgtc 8508
Arg Gln Asn Glu Met Gly Lys Arg
2730

tgaatggctc agcaggagta actgttatct cctctcctaa ggagatgaag acctaacagg 8568

ggcactgcgg ctgggctgct ttaggagacc aagtggcaag aaagctcaca tttttgagt 8628

tcaaatgcta ctgtccaagc gagaagtccc tcatcctgaa gtagactaaa gcccggctga 8688

aaattccgag gaaaacaaaa caaacgaatg aatgaacaga cacacacaat gttccaagtt 8748

cccctaaaat atgacccact tgttctgggt ctacgcagaa aagagacgca aagtgtccaa 8808

aaggaacaaa agaacaaaaa cgaataagca aagaagaaaa caaacaaaaa caaaacaaaa 8868

caaacacacg gaccgataaa caaagaagcg aagataagaa agaaggcctc atatccaatt 8928

acctcactca ttcacatgtg agcgacacgc agacatccgc gagggccagc gtcaccagac 8988

cagctgcggg acaaaccact cagactgctt gtaggacaaa tacttctgac attttcgttt 9048

aagcaaatac aggtgcattt aaaacacgac tttgggggtg atttgtgtgt agcgcctggg 9108

gaggggggat aaaagaggag gagtgagcac tggaaatact ttttaaagaa aaaaaaacat 9168

gagggaataa aagaaattcc tatcaaaaat caaagtgaaa taataccatc cagcacttaa 9228

ctctcaggtc ccaactaagt ctggcctgag ctaatttatt tgagcgcaga gtgtaaaatt 9288

taattcaaaa tggtggctat aatcactaca gataaatttc atactctttt gtctttggag 9348

attccattgt ggacagtaat acgcagttac agggtgtagt ctgtttagat tccgtagttc 9408

gtgggtatca gtttcggtag aggtgcagca tcgtgacact tttgctaaca ggtaccactt 9468

ctgatcaccc tgtacataca tgagccgaaa ggcacaatca ctgtttcaga tttaaaatta 9528

ttagtgtgtt tgtttggtcc agaaactgag acaatcacat gacagtcacc acgaggagag 9588

aaaatttaaa aaataaaaat aaaaacaaaa aaaattttaa aaattaaaaa aacaaaaata 9648

aagtctaata agaactttgg tacaggaact tttttgtaat atacatgtat gaattgttca 9708

tcgagttttt atattaattt taatttgctg ctaagcaaag actagggaca ggcaaagata 9768

atttatggca aagtgtttaa attgtttata cataaataaa gtctctaaaa ctcctgtg 9826

<210> 8
<211> 2733
<212> PRT
<213> Homo sapiens

<400> 8
Met Asp Val Lys Asp Arg Arg His Arg Ser Leu Thr Arg Gly Arg Cys
1               5                   10                  15

Gly Lys Glu Cys Arg Tyr Thr Ser Ser Ser Leu Asp Ser Glu Asp Cys
            20                  25                  30

Arg Val Pro Thr Gln Lys Ser Tyr Ser Ser Ser Glu Thr Leu Lys Ala
        35                  40                  45

Tyr Asp His Asp Ser Arg Met His Tyr Gly Asn Arg Val Thr Asp Leu
    50                  55                  60

Ile His Arg Glu Ser Asp Glu Phe Pro Arg Gln Gly Thr Asn Phe Thr
65                  70                  75                  80

Leu Ala Glu Leu Gly Ile Cys Glu Pro Ser Pro His Arg Ser Gly Tyr
                85                  90                  95

Cys Ser Asp Met Gly Ile Leu His Gln Gly Tyr Ser Leu Ser Thr Gly
            100                 105                 110

Ser Asp Ala Asp Ser Asp Thr Glu Gly Gly Met Ser Pro Glu His Ala
        115                 120                 125

Ile Arg Leu Trp Gly Arg Gly Ile Lys Ser Arg Arg Ser Ser Gly Leu
    130                 135                 140

Ser Ser Arg Glu Asn Ser Ala Leu Thr Leu Thr Asp Ser Asp Asn Glu
145                 150                 155                 160

Asn Lys Ser Asp Asp Glu Asn Gly Arg Pro Ile Pro Pro Thr Ser Ser
                165                 170                 175

Pro Ser Leu Leu Pro Ser Ala Gln Leu Pro Ser Ser His Asn Pro Pro
            180                 185                 190

Pro Val Ser Cys Gln Met Pro Leu Leu Asp Ser Asn Thr Ser His Gln
        195                 200                 205

Ile Met Asp Thr Asn Pro Asp Glu Glu Phe Ser Pro Asn Ser Tyr Leu
    210                 215                 220

Leu Arg Ala Cys Ser Gly Pro Gln Gln Ala Ser Ser Ser Gly Pro Pro
225                 230                 235                 240

Asn His His Ser Gln Ser Thr Leu Arg Pro Pro Leu Pro Pro Pro His
                245                 250                 255

Asn His Thr Leu Ser His His His Ser Ser Ala Asn Ser Leu Asn Arg
            260                 265                 270

Asn Ser Leu Thr Asn Arg Arg Ser Gln Ile His Ala Pro Ala Pro Ala
        275                 280                 285

Pro Asn Asp Leu Ala Thr Thr Pro Glu Ser Val Gln Leu Gln Asp Ser
    290                 295                 300

Trp Val Leu Asn Ser Asn Val Pro Leu Glu Thr Arg His Phe Leu Phe
305                 310                 315                 320

Lys Thr Ser Ser Gly Ser Thr Pro Leu Phe Ser Ser Ser Ser Pro Gly
                325                 330                 335

Tyr Pro Leu Thr Ser Gly Thr Val Tyr Thr Pro Pro Pro Arg Leu Leu
            340                 345                 350

Pro Arg Asn Thr Phe Ser Arg Lys Ala Phe Lys Leu Lys Lys Pro Ser
        355                 360                 365

Lys Tyr Cys Ser Trp Lys Cys Ala Ala Leu Ser Ala Ile Ala Ala Ala
    370                 375                 380

Leu Leu Leu Ala Ile Leu Leu Ala Tyr Phe Ile Val Pro Trp Ser Leu
385                 390                 395                 400

Lys Asn Ser Ser Ile Asp Ser Gly Glu Ala Glu Val Gly Arg Arg Val
                405                 410                 415

Thr Gln Glu Val Pro Pro Gly Val Phe Trp Arg Ser Gln Ile His Ile
            420                 425                 430

Ser Gln Pro Gln Phe Leu Lys Phe Asn Ile Ser Leu Gly Lys Asp Ala
        435                 440                 445

Leu Phe Gly Val Tyr Ile Arg Arg Gly Leu Pro Pro Ser His Ala Gln
    450                 455                 460

Tyr Asp Phe Met Glu Arg Leu Asp Gly Lys Glu Lys Trp Ser Val Val
465                 470                 475                 480

Glu Ser Pro Arg Glu Arg Arg Ser Ile Gln Thr Leu Val Gln Asn Glu
                485                 490                 495

Ala Val Phe Val Gln Tyr Leu Asp Val Gly Leu Trp His Leu Ala Phe
            500                 505                 510

Tyr Asn Asp Gly Lys Asp Lys Glu Met Val Ser Phe Asn Thr Val Val
        515                 520                 525

Leu Asp Ser Val Gln Asp Cys Pro Arg Asn Cys His Gly Asn Gly Glu
    530                 535                 540

Cys Val Ser Gly Val Cys His Cys Phe Pro Gly Phe Leu Gly Ala Asp
545                 550                 555                 560

Cys Ala Lys Ala Ala Cys Pro Val Leu Cys Ser Gly Asn Gly Gln Tyr
                565                 570                 575

Ser Lys Gly Thr Cys Gln Cys Tyr Ser Gly Trp Lys Gly Ala Glu Cys
            580                 585                 590

Asp Val Pro Met Asn Gln Cys Ile Asp Pro Ser Cys Gly Gly His Gly
        595                 600                 605

Ser Cys Ile Asp Gly Asn Cys Val Cys Ser Ala Gly Tyr Lys Gly Glu
    610                 615                 620

His Cys Glu Glu Val Asp Cys Leu Asp Pro Thr Cys Ser Ser His Gly
625                 630                 635                 640

Val Cys Val Asn Gly Glu Cys Leu Cys Ser Pro Gly Trp Gly Gly Leu
                645                 650                 655

Asn Cys Glu Leu Ala Arg Val Gln Cys Pro Asp Gln Cys Ser Gly His
            660                 665                 670

Gly Thr Tyr Leu Pro Asp Thr Gly Leu Cys Ser Cys Asp Pro Asn Trp
        675                 680                 685

Met Gly Pro Asp Cys Ser Val Glu Val Cys Ser Val Asp Cys Gly Thr
    690                 695                 700

His Gly Val Cys Ile Gly Gly Ala Cys Arg Cys Glu Glu Gly Trp Thr
705                 710                 715                 720

Gly Ala Ala Cys Asp Gln Arg Val Cys His Pro Arg Cys Ile Glu His
                725                 730                 735

Gly Thr Cys Lys Asp Gly Lys Cys Glu Cys Arg Glu Gly Trp Asn Gly
            740                 745                 750

Glu His Cys Thr Ile Gly Arg Gln Thr Ala Gly Thr Glu Thr Asp Gly
        755                 760                 765

Cys Pro Asp Leu Cys Asn Gly Asn Gly Arg Cys Thr Leu Gly Gln Asn
    770                 775                 780

Ser Trp Gln Cys Val Cys Gln Thr Gly Trp Arg Gly Pro Gly Cys Asn
785                 790                 795                 800

Val Ala Met Glu Thr Ser Cys Ala Asp Asn Lys Asp Asn Glu Gly Asp
                805                 810                 815

Gly Leu Val Asp Cys Leu Asp Pro Asp Cys Cys Leu Gln Ser Ala Cys
            820                 825                 830

Gln Asn Ser Leu Leu Cys Arg Gly Ser Arg Asp Pro Leu Asp Ile Ile
        835                 840                 845

Gln Gln Gly Gln Thr Asp Trp Pro Ala Val Lys Ser Phe Tyr Asp Arg

850                 855                 860

Ile Lys Leu Leu Ala Gly Lys Asp Ser Thr His Ile Ile Pro Gly Glu
865                 870                 875                 880

Asn Pro Phe Asn Ser Ser Leu Val Ser Leu Ile Arg Gly Gln Val Val
                885                 890                 895

Thr Thr Asp Gly Thr Pro Leu Val Gly Val Asn Val Ser Phe Val Lys
            900                 905                 910

Tyr Pro Lys Tyr Gly Tyr Thr Ile Thr Arg Gln Asp Gly Thr Phe Asp
        915                 920                 925

Leu Ile Ala Asn Gly Gly Ala Ser Leu Thr Leu His Phe Glu Arg Ala
    930                 935                 940

Pro Phe Met Ser Gln Glu Arg Thr Val Trp Leu Pro Trp Asn Ser Phe
945                 950                 955                 960

Tyr Ala Met Asp Thr Leu Val Met Lys Thr Glu Glu Asn Ser Ile Pro
                965                 970                 975

Ser Cys Asp Leu Ser Gly Phe Val Arg Pro Asp Pro Ile Ile Ile Ser
            980                 985                 990

Ser Pro Leu Ser Thr Phe Phe Ser Ala Ala Pro Gly Gln Asn Pro Ile
        995                 1000                1005

Val Pro Glu Thr Gln Val Leu His Glu Glu Ile Glu Leu Pro Gly Ser
    1010                1015                1020

Asn Val Lys Leu Arg Tyr Leu Ser Ser Arg Thr Ala Gly Tyr Lys Ser
1025                1030                1035                1040

Leu Leu Lys Ile Thr Met Thr Gln Ser Thr Val Pro Leu Asn Leu Ile
                1045                1050                1055

Arg Val His Leu Met Val Ala Val Glu Gly His Leu Phe Gln Lys Ser
            1060                1065                1070

Phe Gln Ala Ser Pro Asn Leu Ala Ser Thr Phe Ile Trp Asp Lys Thr
        1075                1080                1085

Asp Ala Tyr Gly Gln Arg Val Tyr Gly Leu Ser Asp Ala Val Val Ser
    1090                1095                1100

Val Gly Phe Glu Tyr Glu Thr Cys Pro Ser Leu Ile Leu Trp Glu Lys
1105                1110                1115                1120

Arg Thr Ala Leu Leu Gln Gly Phe Glu Leu Asp Pro Ser Asn Leu Gly
                1125                1130                1135

Gly Trp Ser Leu Asp Lys His His Ile Leu Asn Val Lys Ser Gly Ile
            1140                1145                1150

Leu His Lys Gly Thr Gly Glu Asn Gln Phe Leu Thr Gln Gln Pro Ala
        1155                1160                1165

Ile Ile Thr Ser Ile Met Gly Asn Gly Arg Arg Arg Ser Ile Ser Cys
    1170                1175                1180

Pro Ser Cys Asn Gly Leu Ala Glu Gly Asn Lys Leu Leu Ala Pro Val
1185                1190                1195                1200

Ala Leu Ala Val Gly Ile Asp Gly Ser Leu Tyr Val Gly Asp Phe Asn
                1205                1210                1215

Tyr Ile Arg Arg Ile Phe Pro Ser Arg Asn Val Thr Ser Ile Leu Glu
            1220                1225                1230

Leu Arg Asn Lys Glu Phe Lys His Ser Asn Asn Pro Ala His Lys Tyr
        1235                1240                1245

Tyr Leu Ala Val Asp Pro Val Ser Gly Ser Leu Tyr Val Ser Asp Thr
    1250                1255                1260

Asn Ser Arg Arg Ile Tyr Arg Val Lys Ser Leu Ser Gly Thr Lys Asp
1265                1270                1275                1280

Leu Ala Gly Asn Ser Glu Val Val Ala Gly Thr Gly Glu Gln Cys Leu
                1285                1290                1295

Pro Phe Asp Glu Ala Arg Cys Gly Asp Gly Gly Lys Ala Ile Asp Ala
            1300                1305                1310

Thr Leu Met Ser Pro Arg Gly Ile Ala Val Asp Lys Asn Gly Leu Met
        1315                1320                1325

Tyr Phe Val Asp Ala Thr Met Ile Arg Lys Val Asp Gln Asn Gly Ile
    1330                1335                1340

Ile Ser Thr Leu Leu Gly Ser Asn Asp Leu Thr Ala Val Arg Pro Leu
1345                1350                1355                1360

Ser Cys Asp Ser Ser Met Asp Val Ala Gln Val Arg Leu Glu Trp Pro
                1365                1370                1375

Thr Asp Leu Ala Val Asn Pro Met Asp Asn Ser Leu Tyr Val Leu Glu
            1380                1385                1390

Asn Asn Val Ile Leu Arg Ile Thr Glu Asn His Gln Val Ser Ile Ile
        1395                1400                1405

Ala Gly Arg Pro Met His Cys Gln Val Pro Gly Ile Asp Tyr Ser Leu
    1410                1415                1420

Ser Lys Leu Ala Ile His Ser Ala Leu Glu Ser Ala Ser Ala Ile Ala
1425                1430                1435                1440

Ile Ser His Thr Gly Val Leu Tyr Ile Thr Glu Thr Asp Glu Lys Lys
                1445                1450                1455

Ile Asn Arg Leu Arg Gln Val Thr Thr Asn Gly Glu Ile Cys Leu Leu
            1460                1465                1470

Ala Gly Ala Ala Ser Asp Cys Asp Cys Lys Asn Asp Val Asn Cys Asn
        1475                1480                1485

Cys Tyr Ser Gly Asp Asp Ala Tyr Ala Thr Asp Ala Ile Leu Asn Ser
    1490                1495                1500

Pro Ser Ser Leu Ala Val Ala Pro Asp Gly Thr Ile Tyr Ile Ala Asp
1505                1510                1515                1520

Leu Gly Asn Ile Arg Ile Arg Ala Val Ser Lys Asn Lys Pro Val Leu
                1525                1530                1535

Asn Ala Phe Asn Gln Tyr Glu Ala Ala Ser Pro Gly Glu Gln Glu Leu
            1540                1545                1550

Tyr Val Phe Asn Ala Asp Gly Ile His Gln Tyr Thr Val Ser Leu Val
        1555                1560                1565

Thr Gly Glu Tyr Leu Tyr Asn Phe Thr Tyr Ser Thr Asp Asn Asp Val
    1570                1575                1580

Thr Glu Leu Ile Asp Asn Asn Gly Asn Ser Leu Lys Ile Arg Arg Asp
1585                1590                1595                1600

Ser Ser Gly Met Pro Arg His Leu Leu Met Pro Asp Asn Gln Ile Ile
                1605                1610                1615

Thr Leu Thr Val Gly Thr Asn Gly Gly Leu Lys Val Val Ser Thr Gln
            1620                1625                1630

Asn Leu Glu Leu Gly Leu Met Thr Tyr Asp Gly Asn Thr Gly Leu Leu
        1635                1640                1645

Ala Thr Lys Ser Asp Glu Thr Gly Trp Thr Thr Phe Tyr Asp Tyr Asp
    1650                1655                1660

His Glu Gly Arg Leu Thr Asn Val Thr Arg Pro Thr Gly Val Val Thr
1665                1670                1675                1680

Ser Leu His Arg Glu Met Glu Lys Ser Ile Thr Ile Asp Ile Glu Asn
                1685                1690                1695

Ser Asn Arg Asp Asp Asp Val Thr Val Ile Thr Asn Leu Ser Ser Val
            1700                1705                1710

Glu Ala Ser Tyr Thr Val Val Gln Asp Gln Val Arg Asn Ser Tyr Gln
        1715                1720                1725

Leu Cys Asn Asn Gly Thr Leu Arg Val Met Tyr Ala Asn Gly Met Gly
    1730                1735                1740

Ile Ser Phe His Ser Glu Pro His Val Leu Ala Gly Thr Ile Thr Pro
1745                1750                1755                1760

Thr Ile Gly Arg Cys Asn Ile Ser Leu Pro Met Glu Asn Gly Leu Asn
                1765                1770                1775

Ser Ile Glu Trp Arg Leu Arg Lys Glu Gln Ile Lys Gly Lys Val Thr
            1780                1785                1790

Ile Phe Gly Arg Lys Leu Arg Val His Gly Arg Asn Leu Leu Ser Ile
        1795                1800                1805

Asp Tyr Asp Arg Asn Ile Arg Thr Glu Lys Ile Tyr Asp Asp His Arg
    1810                1815                1820

Lys Phe Thr Leu Arg Ile Ile Tyr Asp Gln Val Gly Arg Pro Phe Leu

1825                1830                1835                1840

Trp Leu Pro Ser Ser Gly Leu Ala Ala Val Asn Val Ser Tyr Phe Phe
                1845                1850                1855

Asn Gly Arg Leu Ala Gly Leu Gln Arg Gly Ala Met Ser Glu Arg Thr
            1860                1865                1870

Asp Ile Asp Lys Gln Gly Arg Ile Val Ser Arg Met Phe Ala Asp Gly
        1875                1880                1885

Lys Val Trp Ser Tyr Ser Tyr Leu Asp Lys Ser Met Val Leu Leu Leu
    1890                1895                1900

Gln Ser Gln Arg Gln Tyr Ile Phe Glu Tyr Asp Ser Ser Asp Arg Leu
1905                1910                1915                1920

Leu Ala Val Thr Met Pro Ser Val Ala Arg His Ser Met Ser Thr His
                1925                1930                1935

Thr Ser Ile Gly Tyr Ile Arg Asn Ile Tyr Asn Pro Pro Glu Ser Asn
            1940                1945                1950

Ala Ser Val Ile Phe Asp Tyr Ser Asp Asp Gly Arg Ile Leu Lys Thr
        1955                1960                1965

Ser Phe Leu Gly Thr Gly Arg Gln Val Phe Tyr Lys Tyr Gly Lys Leu
    1970                1975                1980

Ser Lys Leu Ser Glu Ile Val Tyr Asp Ser Thr Ala Val Thr Phe Gly
1985                1990                1995                2000

Tyr Asp Glu Thr Thr Gly Val Leu Lys Met Val Asn Leu Gln Ser Gly
                2005                2010                2015

Gly Phe Ser Cys Thr Ile Arg Tyr Arg Lys Ile Gly Pro Leu Val Asp
            2020                2025                2030

Lys Gln Ile Tyr Arg Phe Ser Glu Glu Gly Met Val Asn Ala Arg Phe
        2035                2040                2045

Asp Tyr Thr Tyr His Asp Asn Ser Phe Arg Ile Ala Ser Ile Lys Pro
    2050                2055                2060

Val Ile Ser Glu Thr Pro Leu Pro Val Asp Leu Tyr Arg Tyr Asp Glu
2065                2070                2075                2080

Ile Ser Gly Lys Val Glu His Phe Gly Lys Phe Gly Val Ile Tyr Tyr
                2085                2090                2095

Asp Ile Asn Gln Ile Ile Thr Thr Ala Val Met Thr Leu Ser Lys His
            2100                2105                2110

Phe Asp Thr His Gly Arg Ile Lys Glu Val Gln Tyr Glu Met Phe Arg
        2115                2120                2125

Ser Leu Met Tyr Trp Met Thr Val Gln Tyr Asp Ser Met Gly Arg Val
    2130                2135                2140

Ile Lys Arg Glu Leu Lys Leu Gly Pro Tyr Ala Asn Thr Thr Lys Tyr
2145                2150                2155                2160

Thr Tyr Asp Tyr Asp Gly Asp Gly Gln Leu Gln Ser Val Ala Val Asn
                2165                2170                2175

Asp Arg Pro Thr Trp Arg Tyr Ser Tyr Asp Leu Asn Gly Asn Leu His
            2180                2185                2190

Leu Leu Asn Pro Gly Asn Ser Val Arg Leu Met Pro Leu Arg Tyr Asp
        2195                2200                2205

Leu Arg Asp Arg Ile Thr Arg Leu Gly Asp Val Gln Tyr Lys Ile Asp
    2210                2215                2220

Asp Asp Gly Tyr Leu Cys Gln Arg Gly Ser Asp Ile Phe Glu Tyr Asn
2225                2230                2235                2240

Ser Lys Gly Leu Leu Thr Arg Ala Tyr Asn Lys Ala Ser Gly Trp Ser
                2245                2250                2255

Val Gln Tyr Arg Tyr Asp Gly Val Gly Arg Arg Ala Ser Tyr Lys Thr
            2260                2265                2270

Asn Leu Gly His His Leu Gln Tyr Phe Tyr Ser Asp Leu His Asn Pro
        2275                2280                2285

Thr Arg Ile Thr His Val Tyr Asn His Ser Asn Ser Glu Ile Thr Ser
    2290                2295                2300

Leu Tyr Tyr Asp Leu Gln Gly His Leu Phe Ala Met Glu Ser Ser Ser
2305                2310                2315                2320

Gly Glu Glu Tyr Tyr Val Ala Ser Asp Asn Thr Gly Thr Pro Leu Ala
                2325                2330                2335

Val Phe Ser Ile Asn Gly Leu Met Ile Lys Gln Leu Gln Tyr Thr Ala
            2340                2345                2350

Tyr Gly Glu Ile Tyr Tyr Asp Ser Asn Pro Asp Phe Gln Met Val Ile
        2355                2360                2365

Gly Phe His Gly Gly Leu Tyr Asp Pro Leu Thr Lys Leu Val His Phe
    2370                2375                2380

Thr Gln Arg Asp Tyr Asp Val Leu Ala Gly Arg Trp Thr Ser Pro Asp
2385                2390                2395                2400

Tyr Thr Met Trp Lys Asn Val Gly Lys Glu Pro Ala Pro Phe Asn Leu
                2405                2410                2415

Tyr Met Phe Lys Ser Asn Asn Pro Leu Ser Ser Glu Leu Asp Leu Lys
            2420                2425                2430

Asn Tyr Val Thr Asp Val Lys Ser Trp Leu Val Met Phe Gly Phe Gln
        2435                2440                2445

Leu Ser Asn Ile Ile Pro Gly Phe Pro Arg Ala Lys Met Tyr Phe Val
    2450                2455                2460

Pro Pro Pro Tyr Glu Leu Ser Glu Ser Gln Ala Ser Glu Asn Gly Gln
2465                2470                2475                2480

Leu Ile Thr Gly Val Gln Gln Thr Thr Glu Arg His Asn Gln Ala Phe
                2485                2490                2495

Met Ala Leu Glu Gly Gln Val Ile Thr Lys Lys Leu His Ala Ser Ile
            2500                2505                2510

Arg Glu Lys Ala Gly His Trp Phe Ala Thr Thr Thr Pro Ile Ile Gly
        2515                2520                2525

Lys Gly Ile Met Phe Ala Ile Lys Glu Gly Arg Val Thr Thr Gly Val
    2530                2535                2540

Ser Ser Ile Ala Ser Glu Asp Ser Arg Lys Val Ala Ser Val Leu Asn
2545                2550                2555                2560

Asn Ala Tyr Tyr Leu Asp Lys Met His Tyr Ser Ile Glu Gly Lys Asp
                2565                2570                2575

Thr His Tyr Phe Val Lys Ile Gly Ser Ala Asp Gly Asp Leu Val Thr
            2580                2585                2590

Leu Gly Thr Thr Ile Gly Arg Lys Val Leu Glu Ser Gly Val Asn Val
        2595                2600                2605

Thr Val Ser Gln Pro Thr Leu Leu Val Asn Gly Arg Thr Arg Arg Phe
    2610                2615                2620

Thr Asn Ile Glu Phe Gln Tyr Ser Thr Leu Leu Leu Ser Ile Arg Tyr
2625                2630                2635                2640

Gly Leu Thr Pro Asp Thr Leu Asp Glu Glu Lys Ala Arg Val Leu Asp
                2645                2650                2655

Gln Ala Arg Gln Arg Ala Leu Gly Thr Ala Trp Ala Lys Glu Gln Gln
            2660                2665                2670

Lys Ala Arg Asp Gly Arg Glu Gly Ser Arg Leu Trp Thr Glu Gly Glu
        2675                2680                2685

Lys Gln Gln Leu Leu Ser Thr Gly Arg Val Gln Gly Tyr Glu Gly Tyr
    2690                2695                2700

Tyr Val Leu Pro Val Glu Gln Tyr Pro Glu Leu Ala Asp Ser Ser Ser
2705                2710                2715                2720

Asn Ile Gln Phe Leu Arg Gln Asn Glu Met Gly Lys Arg
                2725                2730

<210> 9
<211> 201
<212> DNA
<213> Homo sapiens

<400> 9
gctccaaagc gagctgggac cgaagactct aggctaagtt atctatgtag atggtgtcag 60
ggagcgaagc tactgaccga gctgctgtta catccagctt tttaattgcc taagcggtct 120
ggggcttgct tcgtcatttg gctttgctgt ggagcactcc tgtaaagcca gctgaattgt 180
acatcgaaga tccacccttt t                                           201

<210> 10
<211> 201
<212> DNA
<213> Homo sapiens

<400> 10
gctccaaagc gagctgggac cgaagactct aggctaagtt atctatgtag atggtgtcag 60
ggagcgaagc tactgaccga gctgctgtta catccagctt tttaattgcc taagcggtct 120
ggggcttgct tcgtcatttg gctttgctgt ggagcactcc tgtaaagcca gctgaattgt 180
acatcgaaga tccacccttt t 201

<210> 11
<211> 134
<212> DNA
<213> Homo sapiens

<400> 11
ccagcattag atgagttgac aaaaatgcag tttcagctct gaaggtctga aagattctgc 60
tgcaactaaa gctctgaaga ttctgctaca actatgacat ccattttctc ccacttcaga 120
caggatgaat acaa 134

<210> 12
<211> 9729
<212> DNA
<213> Homo sapiens

<220>
<221> CDS
<222> (210)..(8381)

<400> 12
   ccagcattag atgagttgac aaaaatgcag tttcagctct gaaggtctga aagattctgc 60
   tgcaactaaa gctctgaaga ttctgctaca actatgacat ccattttctc ccacttcaga 120
   caggatgaat acaaggtggc aaagtgacaa gtgccaaaac tcaggcctga ctttcctgaa 180
   aacatcagca ttctgccata tctggaata atg gat gta aag gac cgg cga cac 233
   Met Asp Val Lys Asp Arg Arg His
   1 5
   cgc tct ttg acc aga gga cgc tgt ggc aaa gag tgt cgc tac aca agc 281
   Arg Ser Leu Thr Arg Gly Arg Cys Gly Lys Glu Cys Arg Tyr Thr Ser
   10 15 20
   tcc tct ctg gac agt gag gac tgc cgg gtg ccc aca cag aaa tcc tac 329
   Ser Ser Leu Asp Ser Glu Asp Cys Arg Val Pro Thr Gln Lys Ser Tyr
   25 30 35 40
   agc tcc agt gag act ctg aag gcc tat gac cat gac agc agg atg cac 377
   Ser Ser Ser Glu Thr Leu Lys Ala Tyr Asp His Asp Ser Arg Met His
   45 50 55
   tat gga aac cga gtc aca gac ctc atc cac cgg gag tca gat gag ttt 425
   Tyr Gly Asn Arg Val Thr Asp Leu Ile His Arg Glu Ser Asp Glu Phe
   60 65 70
   cct aga caa gga acc aac ttc acc ctt gcc gaa ctg ggc atc tgt gag 473
   Pro Arg Gln Gly Thr Asn Phe Thr Leu Ala Glu Leu Gly Ile Cys Glu
   75 80 85
   ccc tcc cca cac cga agc ggc tac tgc tcc gac atg ggg atc ctt cac 521
   Pro Ser Pro His Arg Ser Gly Tyr Cys Ser Asp Met Gly Ile Leu His
   90 95 100
   cag ggc tac tcc ctt agc aca ggg tct gac gcc gac tcc gac acc gag 569
   Gln Gly Tyr Ser Leu Ser Thr Gly Ser Asp Ala Asp Ser Asp Thr Glu
   105 110 115 120
   gga ggg atg tct cca gaa cac gcc atc aga ctg tgg ggc aga ggg ata 617
   Gly Gly Met Ser Pro Glu His Ala Ile Arg Leu Trp Gly Arg Gly Ile
   125 130 135
   aaa tcc agg cgc agt tcc ggc ctg tcc agt cgt gaa aac tcg gcc ctt 665
   Lys Ser Arg Arg Ser Ser Gly Leu Ser Ser Arg Glu Asn Ser Ala Leu
   140 145 150
   acc ctg act gac tct gac aac gaa aac aaa tca gat gat gag aac ggt 713
   Thr Leu Thr Asp Ser Asp Asn Glu Asn Lys Ser Asp Asp Glu Asn Gly
   155 160 165
   cgt ccc att cca cct aca tcc tcg cct agt ctc ctc cca tct gct cag 761
   Arg Pro Ile Pro Pro Thr Ser Ser Pro Ser Leu Leu Pro Ser Ala Gln
   170 175 180
   ctg cct agc tcc cat aat cct cca cca gtt agc tgc cag atg cca ttg 809
   Leu Pro Ser Ser His Asn Pro Pro Pro Val Ser Cys Gln Met Pro Leu
   185 190 195 200
   cta gac agc aac acc tcc cat caa atc atg gac acc aac cct gat gag 857
   Leu Asp Ser Asn Thr Ser His Gln Ile Met Asp Thr Asn Pro Asp Glu
   205 210 215
   gaa ttc tcc ccc aat tca tac ctg ctc aga gca tgc tca ggg ccc cag 905
   Glu Phe Ser Pro Asn Ser Tyr Leu Leu Arg Ala Cys Ser Gly Pro Gln
   220 225 230
   caa gcc tcc agc agt ggc cct ccg aac cac cac agc cag tcg act ctg 953
   Gln Ala Ser Ser Ser Gly Pro Pro Asn His His Ser Gln Ser Thr Leu
   235 240 245
   agg ccc cct ctc cca ccc cct cac aac cac acg ctg tcc cat cac cac 1001
   Arg Pro Pro Leu Pro Pro Pro His Asn His Thr Leu Ser His His His
   250 255 260
   tcg tcc gcc aac tcc ctc aac agg aac tca ctg acc aat cgg cgg agt 1049
   Ser Ser Ala Asn Ser Leu Asn Arg Asn Ser Leu Thr Asn Arg Arg Ser
   265 270 275 280
   cag atc cac gcc ccg gcc cca gcg ccc aat gac ctg gcc acc aca cca 1097
   Gln Ile His Ala Pro Ala Pro Ala Pro Asn Asp Leu Ala Thr Thr Pro
   285 290 295
   gag tcc gtt cag ctt cag gac agc tgg gtg cta aac agc aac gtg cca 1145
   Glu Ser Val Gln Leu Gln Asp Ser Trp Val Leu Asn Ser Asn Val Pro
   300 305 310
   ctg gag acc cgg cac ttc ctc ttc aag acc tcc tcg ggg agc aca ccc 1193
   Leu Glu Thr Arg His Phe Leu Phe Lys Thr Ser Ser Gly Ser Thr Pro
   315                 320                 325
   ttg ttc agc agc tct tcc ccg gga tac cct ttg acc tca gga acg gtt     1241
   Leu Phe Ser Ser Ser Ser Pro Gly Tyr Pro Leu Thr Ser Gly Thr Val
       330                 335                 340
   tac acg ccc ccg ccc cgc ctg ctg ccc agg aat act ttc tcc agg aag     1289
   Tyr Thr Pro Pro Pro Arg Leu Leu Pro Arg Asn Thr Phe Ser Arg Lys
   345                 350                 355                 360
   gct ttc aag ctg aag aag ccc tcc aaa tac tgc agc tgg aaa tgt gct     1337
   Ala Phe Lys Leu Lys Lys Pro Ser Lys Tyr Cys Ser Trp Lys Cys Ala
                   365                 370                 375
   gcc ctc tcc gcc att gcc gcg gcc ctc ctc ttg gct att ttg ctg gcg     1385
   Ala Leu Ser Ala Ile Ala Ala Ala Leu Leu Leu Ala Ile Leu Leu Ala
               380                 385                 390
   tat ttc ata gtg ccc tgg tcg ttg aaa aac agc agc ata gac agt ggt     1433
   Tyr Phe Ile Val Pro Trp Ser Leu Lys Asn Ser Ser Ile Asp Ser Gly
           395                 400                 405
   gaa gca gaa gtt ggt cgg cgg gta aca caa gaa gtc cca cca ggg gtg     1481
   Glu Ala Glu Val Gly Arg Arg Val Thr Gln Glu Val Pro Pro Gly Val
       410                 415                 420
   ttt tgg agg tca caa att cac atc agt cag ccc cag ttc tta aag ttc     1529
   Phe Trp Arg Ser Gln Ile His Ile Ser Gln Pro Gln Phe Leu Lys Phe
   425                 430                 435                 440
   aac atc tcc ctc ggg aag gac gct ctc ttt ggt gtt tac ata aga aga     1577
   Asn Ile Ser Leu Gly Lys Asp Ala Leu Phe Gly Val Tyr Ile Arg Arg
                   445                 450                 455
   gga ctt cca cca tct cat gcc cag tat gac ttc atg gaa cgt ctg gac     1625
   Gly Leu Pro Pro Ser His Ala Gln Tyr Asp Phe Met Glu Arg Leu Asp
               460                 465                 470
   ggg aag gag aag tgg agt gtg gtt gag tct ccc agg gaa cgc cgg agc     1673
   Gly Lys Glu Lys Trp Ser Val Val Glu Ser Pro Arg Glu Arg Arg Ser
           475                 480                 485
   ata cag acc ttg gtt cag aat gaa gcc gtg ttt gtg cag tac ctg gat     1721
   Ile Gln Thr Leu Val Gln Asn Glu Ala Val Phe Val Gln Tyr Leu Asp
       490                 495                 500
   gtg ggc ctg tgg cat ctg gcc ttc tac aat gat gga aaa gac aaa gag     1769
   Val Gly Leu Trp His Leu Ala Phe Tyr Asn Asp Gly Lys Asp Lys Glu
   505                 510                 515                 520
   atg gtt tcc ttc aat act gtt gtc cta gat tca gtg cag gac tgt cca     1817
   Met Val Ser Phe Asn Thr Val Val Leu Asp Ser Val Gln Asp Cys Pro
                   525                 530                 535
   cgt aac tgc cat ggg aat ggt gaa tgt gtg tcc ggg gtg tgt cac tgt     1865
   Arg Asn Cys His Gly Asn Gly Glu Cys Val Ser Gly Val Cys His Cys
               540                 545                 550
   ttc cca gga ttt cta gga gca gac tgt gct aaa gct gcc tgc cct gtc     1913
   Phe Pro Gly Phe Leu Gly Ala Asp Cys Ala Lys Ala Ala Cys Pro Val
           555                 560                 565
   ctg tgc agt ggg aat gga caa tat tct aaa ggg acg tgc cag tgc tac 1961
   Leu Cys Ser Gly Asn Gly Gln Tyr Ser Lys Gly Thr Cys Gln Cys Tyr
   570 575 580
   agc ggc tgg aaa ggt gca gag tgc gac gtg ccc atg aat cag tgc atc 2009
   Ser Gly Trp Lys Gly Ala Glu Cys Asp Val Pro Met Asn Gln Cys Ile
   585 590 595 600
   gat cct tcc tgc ggg ggc cac ggc tcc tgc att gat ggg aac tgt gtc 2057
   Asp Pro Ser Cys Gly Gly His Gly Ser Cys Ile Asp Gly Asn Cys Val
   605 610 615
   tgc tct gct ggc tac aaa ggc gag cac tgt gag gaa gtt gat tgc ttg 2105
   Cys Ser Ala Gly Tyr Lys Gly Glu His Cys Glu Glu Val Asp Cys Leu
   620 625 630
   gat ccc acc tgc tcc agc cac gga gtc tgt gtg aat gga gaa tgc ctg 2153
   Asp Pro Thr Cys Ser Ser His Gly Val Cys Val Asn Gly Glu Cys Leu
   635 640 645
   tgc agc cct ggc tgg ggt ggt ctg aac tgt gag ctg gcg agg gtc cag 2201
   Cys Ser Pro Gly Trp Gly Gly Leu Asn Cys Glu Leu Ala Arg Val Gln
   650 655 660
   tgc cca gac cag tgc agt ggg cat ggc acg tac ctg cct gac acg ggc 2249
   Cys Pro Asp Gln Cys Ser Gly His Gly Thr Tyr Leu Pro Asp Thr Gly
   665 670 675 680
   ctc tgc agc tgc gat ccc aac tgg atg ggt ccc gac tgc tct gtt gaa 2297
   Leu Cys Ser Cys Asp Pro Asn Trp Met Gly Pro Asp Cys Ser Val Glu
   685 690 695
   gtg tgc tca gta gac tgt ggc act cac ggc gtc tgc atc ggg gga gcc 2345
   Val Cys Ser Val Asp Cys Gly Thr His Gly Val Cys Ile Gly Gly Ala
   700 705 710
   tgc cgc tgt gaa gag ggc tgg aca ggc gca gcg tgt gac cag cgc gtg 2393
   Cys Arg Cys Glu Glu Gly Trp Thr Gly Ala Ala Cys Asp Gln Arg Val
   715 720 725
   tgc cac ccc cgc tgc att gag cat ggg acc tgt aaa gat ggc aaa tgt 2441
   Cys His Pro Arg Cys Ile Glu His Gly Thr Cys Lys Asp Gly Lys Cys
   730 735 740
   gaa tgc cga gag ggc tgg aat ggt gaa cac tgc acc att gat ggc tgc 2489
   Glu Cys Arg Glu Gly Trp Asn Gly Glu His Cys Thr Ile Asp Gly Cys
   745 750 755 760
   cct gac ttg tgc aac ggt aac ggg aga tgc aca ctg ggt cag aac agc 2537
   Pro Asp Leu Cys Asn Gly Asn Gly Arg Cys Thr Leu Gly Gln Asn Ser
   765 770 775
   tgg cag tgt gtc tgc cag acc ggc tgg aga ggg ccc gga tgc aac gtt 2585
   Trp Gln Cys Val Cys Gln Thr Gly Trp Arg Gly Pro Gly Cys Asn Val
   780 785 790
   gcc atg gaa act tcc tgt gct gat aac aag gat aat gag gga gat ggc 2633
   Ala Met Glu Thr Ser Cys Ala Asp Asn Lys Asp Asn Glu Gly Asp Gly
   795 800 805
   ctg gtg gat tgt ttg gac cct gac tgc tgc ctg cag tca gcc tgt cag     2681
   Leu Val Asp Cys Leu Asp Pro Asp Cys Cys Leu Gln Ser Ala Cys Gln
       810                 815                 820
   aac agc ctg ctc tgc cgg ggg tcc cgg gac cca ctg gac atc att cag     2729
   Asn Ser Leu Leu Cys Arg Gly Ser Arg Asp Pro Leu Asp Ile Ile Gln
   825                 830                 835                 840
   cag ggc cag acg gat tgg ccc gca gtg aag tcc ttc tat gac cgt atc     2777
   Gln Gly Gln Thr Asp Trp Pro Ala Val Lys Ser Phe Tyr Asp Arg Ile
                   845                 850                 855
   aag ctc ttg gca ggc aag gat agc acc cac atc att cct gga gag aac     2825
   Lys Leu Leu Ala Gly Lys Asp Ser Thr His Ile Ile Pro Gly Glu Asn
               860                 865                 870
   cct ttc aac agc agc ttg gtt tct ctc atc cga ggc caa gta gta act     2873
   Pro Phe Asn Ser Ser Leu Val Ser Leu Ile Arg Gly Gln Val Val Thr
           875                 880                 885
   aca gat gga act ccc ctg gtc ggt gtg aac gtg tct ttt gtc aag tac     2921
   Thr Asp Gly Thr Pro Leu Val Gly Val Asn Val Ser Phe Val Lys Tyr
       890                 895                 900
   cca aaa tac ggc tac acc atc acc cgc cag gat ggc acg ttc gac ctg     2969
   Pro Lys Tyr Gly Tyr Thr Ile Thr Arg Gln Asp Gly Thr Phe Asp Leu
   905                 910                 915                 920
   atc gca aat gga ggt gct tcc ttg act cta cac ttt gag cga gcc ccg     3017
   Ile Ala Asn Gly Gly Ala Ser Leu Thr Leu His Phe Glu Arg Ala Pro
                   925                 930                 935
   ttc atg agc cag gag cgc act gtg tgg ctg ccg tgg aac agc ttt tac     3065
   Phe Met Ser Gln Glu Arg Thr Val Trp Leu Pro Trp Asn Ser Phe Tyr
               940                 945                 950
   gcc atg gac acc ctg gtg atg aag acc gag gag aac tcc atc ccc agc     3113
   Ala Met Asp Thr Leu Val Met Lys Thr Glu Glu Asn Ser Ile Pro Ser
           955                 960                 965
   tgt gac ctc agt ggc ttt gtc cgg cct gat cca atc atc atc tcc tcc     3161
   Cys Asp Leu Ser Gly Phe Val Arg Pro Asp Pro Ile Ile Ile Ser Ser
       970                 975                 980
   cca ctg tcc acc ttc ttt agt gct gcc cct ggg cag aat ccc atc gtg     3209
   Pro Leu Ser Thr Phe Phe Ser Ala Ala Pro Gly Gln Asn Pro Ile Val
   985                 990                 995                 1000
   cct gag acc cag gtt ctt cat gaa gaa atc gag ctc cct ggt tcc aat     3257
   Pro Glu Thr Gln Val Leu His Glu Glu Ile Glu Leu Pro Gly Ser Asn
                   1005                1010                1015
   gtg aaa ctt cgc tat ctg agc tct aga act gca ggg tac aag tca ctg     3305
   Val Lys Leu Arg Tyr Leu Ser Ser Arg Thr Ala Gly Tyr Lys Ser Leu
               1020                1025                1030
   ctg aag atc acc atg acc cag tcc aca gtg ccc ctg aac ctc att agg     3353
   Leu Lys Ile Thr Met Thr Gln Ser Thr Val Pro Leu Asn Leu Ile Arg
           1035                1040                1045
   gtt cac ctg atg gtg gct gtc gag ggg cat ctc ttc cag aag tca ttc     3401
   Val His Leu Met Val Ala Val Glu Gly His Leu Phe Gln Lys Ser Phe
   1050 1055 1060
   cag gct tct ccc aac ctg gcc tcc acc ttc atc tgg gac aag aca gat 3449
   Gln Ala Ser Pro Asn Leu Ala Ser Thr Phe Ile Trp Asp Lys Thr Asp
   1065 1070 1075 1080
   gcg tat ggc caa agg gtg tat gga ctc tca gat gct gtt gtg tct gtc 3497
   Ala Tyr Gly Gln Arg Val Tyr Gly Leu Ser Asp Ala Val Val Ser Val
   1085 1090 1095
   ggg ttt gaa tat gag acc tgt ccc agt cta att ctc tgg gag aaa agg 3545
   Gly Phe Glu Tyr Glu Thr Cys Pro Ser Leu Ile Leu Trp Glu Lys Arg
   1100 1105 1110
   aca gcc ctc ctt cag gga ttc gag ctg gac ccc tcc aac ctc ggt ggc 3593
   Thr Ala Leu Leu Gln Gly Phe Glu Leu Asp Pro Ser Asn Leu Gly Gly
   1115 1120 1125
   tgg tcc cta gac aaa cac cac atc ctc aat gtt aaa agt gga atc cta 3641
   Trp Ser Leu Asp Lys His His Ile Leu Asn Val Lys Ser Gly Ile Leu
   1130 1135 1140
   cac aaa ggc act ggg gaa aac cag ttc ctg acc cag cag cct gcc atc 3689
   His Lys Gly Thr Gly Glu Asn Gln Phe Leu Thr Gln Gln Pro Ala Ile
   1145 1150 1155 1160
   atc acc agc atc atg ggc aat ggt cgc cgc cgg agc att tcc tgt ccc 3737
   Ile Thr Ser Ile Met Gly Asn Gly Arg Arg Arg Ser Ile Ser Cys Pro
   1165 1170 1175
   agc tgc aac ggc ctt gct gaa ggc aac aag ctg ctg gcc cca gtg gct 3785
   Ser Cys Asn Gly Leu Ala Glu Gly Asn Lys Leu Leu Ala Pro Val Ala
   1180 1185 1190
   ctg gct gtt gga atc gat ggg agc ctc tat gtg ggt gac ttc aat tac 3833
   Leu Ala Val Gly Ile Asp Gly Ser Leu Tyr Val Gly Asp Phe Asn Tyr
   1195 1200 1205
   atc cga cgc atc ttt ccc tct cga aat gtg acc agc atc ttg gag tta 3881
   Ile Arg Arg Ile Phe Pro Ser Arg Asn Val Thr Ser Ile Leu Glu Leu
   1210 1215 1220
   cga aat aaa gag ttt aaa cat agc aac aac cca gca cac aag tac tac 3929
   Arg Asn Lys Glu Phe Lys His Ser Asn Asn Pro Ala His Lys Tyr Tyr
   1225 1230 1235 1240
   ttg gca gtg gac ccc gtg tcc ggc tcg ctc tac gtg tcc gac acc aac 3977
   Leu Ala Val Asp Pro Val Ser Gly Ser Leu Tyr Val Ser Asp Thr Asn
   1245 1250 1255
   agc agg aga atc tac cgc gtc aag tct ctg agt gga acc aaa gac ctg 4025
   Ser Arg Arg Ile Tyr Arg Val Lys Ser Leu Ser Gly Thr Lys Asp Leu
   1260 1265 1270
   gct ggg aat tcg gaa gtt gtg gca ggg acg gga gag cag tgt cta ccc 4073
   Ala Gly Asn Ser Glu Val Val Ala Gly Thr Gly Glu Gln Cys Leu Pro
   1275 1280 1285
   ttt gat gaa gcc cgc tgc ggg gat gga ggg aag gcc ata gat gca acc 4121
   Phe Asp Glu Ala Arg Cys Gly Asp Gly Gly Lys Ala Ile Asp Ala Thr
   1290                1295                1300
   ctg atg agc ccg aga ggt att gca gta gac aag aat ggg ctc atg tac     4169
   Leu Met Ser Pro Arg Gly Ile Ala Val Asp Lys Asn Gly Leu Met Tyr
   1305                1310                1315                1320
   ttt gtc gat gcc acc atg atc cgg aag gtt gac cag aat gga atc atc     4217
   Phe Val Asp Ala Thr Met Ile Arg Lys Val Asp Gln Asn Gly Ile Ile
                   1325                1330                1335
   tcc acc ctg ctg ggc tcc aat gac ctc act gcc gtc cgg ccg ctg agc     4265
   Ser Thr Leu Leu Gly Ser Asn Asp Leu Thr Ala Val Arg Pro Leu Ser
               1340                1345                1350
   tgt gat tcc agc atg gat gta gcc cag gtt cgt ctg gag tgg cca aca     4313
   Cys Asp Ser Ser Met Asp Val Ala Gln Val Arg Leu Glu Trp Pro Thr
           1355                1360                1365
   gac ctt gct gtc aat ccc atg gat aac tcc ttg tat gtt cta gag aac     4361
   Asp Leu Ala Val Asn Pro Met Asp Asn Ser Leu Tyr Val Leu Glu Asn
       1370                1375                1380
   aat gtc atc ctt cga atc acc gag aac cac caa gtc agc atc att gcg     4409
   Asn Val Ile Leu Arg Ile Thr Glu Asn His Gln Val Ser Ile Ile Ala
   1385                1390                1395                1400
   gga cgc ccc atg cac tgc caa gtt cct ggc att gac tac tca ctc agc     4457
   Gly Arg Pro Met His Cys Gln Val Pro Gly Ile Asp Tyr Ser Leu Ser
                   1405                1410                1415
   aaa cta gcc att cac tct gcc ctg gag tca gcc agt gcc att gcc att     4505
   Lys Leu Ala Ile His Ser Ala Leu Glu Ser Ala Ser Ala Ile Ala Ile
               1420                1425                1430
   tct cac act ggg gtc ctc tac atc act gag aca gat gag aag aag att     4553
   Ser His Thr Gly Val Leu Tyr Ile Thr Glu Thr Asp Glu Lys Lys Ile
           1435                1440                1445
   aac cgt cta cgc cag gta aca acc aac ggg gag atc tgc ctt tta gct     4601
   Asn Arg Leu Arg Gln Val Thr Thr Asn Gly Glu Ile Cys Leu Leu Ala
       1450                1455                1460
   ggg gca gcc tcg gac tgc gac tgc aaa aac gat gtc aat tgc aac tgc     4649
   Gly Ala Ala Ser Asp Cys Asp Cys Lys Asn Asp Val Asn Cys Asn Cys
   1465                1470                1475                1480
   tat tca gga gat gat gcc tac gcg act gat gcc atc ttg aat tcc cca     4697
   Tyr Ser Gly Asp Asp Ala Tyr Ala Thr Asp Ala Ile Leu Asn Ser Pro
                   1485                1490                1495
   tca tcc tta gct gta gct cca gat ggt acc att tac att gca gac ctt     4745
   Ser Ser Leu Ala Val Ala Pro Asp Gly Thr Ile Tyr Ile Ala Asp Leu
               1500                1505                1510
   gga aat att cgg atc agg gcg gtc agc aag aac aag cct gtt ctt aat     4793
   Gly Asn Ile Arg Ile Arg Ala Val Ser Lys Asn Lys Pro Val Leu Asn
           1515                1520                1525
   gcc ttc aac cag tat gag gct gca tcc ccc gga gag cag gag tta tat     4841
   Ala Phe Asn Gln Tyr Glu Ala Ala Ser Pro Gly Glu Gln Glu Leu Tyr
       1530                1535                1540
   gtt ttc aac gct gat ggc atc cac caa tac act gtg agc ctg gtg aca     4889
   Val Phe Asn Ala Asp Gly Ile His Gln Tyr Thr Val Ser Leu Val Thr
   1545                1550                1555                1560
   ggg gag tac ttg tac aat ttc aca tat agt act gac aat gat gtc act     4937
   Gly Glu Tyr Leu Tyr Asn Phe Thr Tyr Ser Thr Asp Asn Asp Val Thr
                   1565                1570                1575
   gaa ttg att gac aat aat ggg aat tcc ctg aag atc cgt cgg gac agc     4985
   Glu Leu Ile Asp Asn Asn Gly Asn Ser Leu Lys Ile Arg Arg Asp Ser
               1580                1585                1590
   agt ggc atg ccc cgt cac ctg ctc atg cct gac aac cag atc atc acc     5033
   Ser Gly Met Pro Arg His Leu Leu Met Pro Asp Asn Gln Ile Ile Thr
           1595                1600                1605
   ctc acc gtg ggc acc aat gga ggc ctc aaa gtc gtg tcc aca cag aac     5081
   Leu Thr Val Gly Thr Asn Gly Gly Leu Lys Val Val Ser Thr Gln Asn
       1610                1615                1620
   ctg gag ctt ggt ctc atg acc tat gat ggc aac act ggg ctc ctg gcc     5129
   Leu Glu Leu Gly Leu Met Thr Tyr Asp Gly Asn Thr Gly Leu Leu Ala
   1625                1630                1635                1640
   acc aag agc gat gaa aca gga tgg acg act ttc tat gac tat gac cac     5177
   Thr Lys Ser Asp Glu Thr Gly Trp Thr Thr Phe Tyr Asp Tyr Asp His
                   1645                1650                1655
   gaa ggc cgc ctg acc aac gtg acg cgc ccc acg ggg gtg gta acc agt     5225
   Glu Gly Arg Leu Thr Asn Val Thr Arg Pro Thr Gly Val Val Thr Ser
               1660                1665                1670
   ctg cac cgg gaa atg gag aaa tct att acc att gac att gag aac tcc     5273
   Leu His Arg Glu Met Glu Lys Ser Ile Thr Ile Asp Ile Glu Asn Ser
           1675                1680                1685
   aac cgt gat gat gac gtc act gtc atc acc aac ctc tct tca gta gag     5321
   Asn Arg Asp Asp Asp Val Thr Val Ile Thr Asn Leu Ser Ser Val Glu
       1690                1695                1700
   gcc tcc tac aca gtg gta caa gat caa gtt cgg aac agc tac cag ctc     5369
   Ala Ser Tyr Thr Val Val Gln Asp Gln Val Arg Asn Ser Tyr Gln Leu
   1705                1710                1715                1720
   tgt aat aat ggt acc ctg agg gtg atg tat gct aat ggg atg ggt atc     5417
   Cys Asn Asn Gly Thr Leu Arg Val Met Tyr Ala Asn Gly Met Gly Ile
                   1725                1730                1735
   agc ttc cac agc gag ccc cat gtc cta gcg ggc acc atc acc ccc acc     5465
   Ser Phe His Ser Glu Pro His Val Leu Ala Gly Thr Ile Thr Pro Thr
               1740                1745                1750
   att gga cgc tgc aac atc tcc ctg cct atg gag aat ggc tta aac tcc     5513
   Ile Gly Arg Cys Asn Ile Ser Leu Pro Met Glu Asn Gly Leu Asn Ser
           1755                1760                1765
   att gag tgg cgc cta aga aag gaa cag att aaa ggc aaa gtc acc atc     5561
   Ile Glu Trp Arg Leu Arg Lys Glu Gln Ile Lys Gly Lys Val Thr Ile
       1770                1775                1780
   ttt ggc agg aag ctc cgg gtc cat gga aga aat ctc ttg tcc att gac     5609
   Phe Gly Arg Lys Leu Arg Val His Gly Arg Asn Leu Leu Ser Ile Asp
   1785                1790                1795                1800
   tat gat cga aat att cgg act gaa aag atc tat gat gac cac cgg aag     5657
   Tyr Asp Arg Asn Ile Arg Thr Glu Lys Ile Tyr Asp Asp His Arg Lys
                   1805                1810                1815
   ttc acc ctg agg atc att tat gac cag gtg ggc cgc ccc ttc ctc tgg     5705
   Phe Thr Leu Arg Ile Ile Tyr Asp Gln Val Gly Arg Pro Phe Leu Trp
               1820                1825                1830
   ctg ccc agc agc ggg ctg gca gct gtc aac gtg tca tac ttc ttc aat     5753
   Leu Pro Ser Ser Gly Leu Ala Ala Val Asn Val Ser Tyr Phe Phe Asn
           1835                1840                1845
   ggg cgc ctg gct ggg ctt cag cgt ggg gcc atg agc gag agg aca gac     5801
   Gly Arg Leu Ala Gly Leu Gln Arg Gly Ala Met Ser Glu Arg Thr Asp
       1850                1855                1860
   atc gac aag caa ggc cgc atc gtg tcc cgc atg ttc gct gac ggg aaa     5849
   Ile Asp Lys Gln Gly Arg Ile Val Ser Arg Met Phe Ala Asp Gly Lys
   1865                1870                1875                1880
   gtg tgg agc tac tcc tac ctt gac aag tcc atg gtc ctc ctg ctt cag     5897
   Val Trp Ser Tyr Ser Tyr Leu Asp Lys Ser Met Val Leu Leu Leu Gln
                   1885                1890                1895
   agc caa cgt cag tat ata ttt gag tat gac tcc tct gac cgc ctc ctt     5945
   Ser Gln Arg Gln Tyr Ile Phe Glu Tyr Asp Ser Ser Asp Arg Leu Leu
               1900                1905                1910
   gcc gtc acc atg ccc agc gtg gcc cgg cac agc atg tcc aca cac acc     5993
   Ala Val Thr Met Pro Ser Val Ala Arg His Ser Met Ser Thr His Thr
           1915                1920                1925
   tcc atc ggc tac atc cgt aat att tac aac ccg cct gaa agc aat gct     6041
   Ser Ile Gly Tyr Ile Arg Asn Ile Tyr Asn Pro Pro Glu Ser Asn Ala
       1930                1935                1940
   tcg gtc atc ttt gac tac agt gat gac ggc cgc atc ctg aag acc tcc     6089
   Ser Val Ile Phe Asp Tyr Ser Asp Asp Gly Arg Ile Leu Lys Thr Ser
   1945                1950                1955                1960
   ttt ttg ggc acc gga cgc cag gtg ttc tac aag tat ggg aaa ctc tcc     6137
   Phe Leu Gly Thr Gly Arg Gln Val Phe Tyr Lys Tyr Gly Lys Leu Ser
                   1965                1970                1975
   aag tta tca gag att gtc tac gac agt acc gcc gtc acc ttc ggg tat     6185
   Lys Leu Ser Glu Ile Val Tyr Asp Ser Thr Ala Val Thr Phe Gly Tyr
               1980                1985                1990
   gac gag acc act ggt gtc ttg aag atg gtc aac ctc caa agt ggg ggc     6233
   Asp Glu Thr Thr Gly Val Leu Lys Met Val Asn Leu Gln Ser Gly Gly
           1995                2000                2005
   ttc tcc tgc acc atc agg tac cgg aag att ggc ccc ctg gtg gac aag     6281
   Phe Ser Cys Thr Ile Arg Tyr Arg Lys Ile Gly Pro Leu Val Asp Lys
       2010                2015                2020
   cag atc tac agg ttc tcc gag gaa ggc atg gtc aat gcc agg ttt gac     6329
   Gln Ile Tyr Arg Phe Ser Glu Glu Gly Met Val Asn Ala Arg Phe Asp
   2025                2030                2035                2040
   tac acc tat cat gac aac agc ttc cgc atc gca agc atc aag ccc gtc     6377
   Tyr Thr Tyr His Asp Asn Ser Phe Arg Ile Ala Ser Ile Lys Pro Val
               2045                2050                2055
   ata agt gag act ccc ctc ccc gtt gac ctc tac cgc tat gat gag att     6425
   Ile Ser Glu Thr Pro Leu Pro Val Asp Leu Tyr Arg Tyr Asp Glu Ile
           2060                2065                2070
   tct ggc aag gtg gaa cac ttt ggt aag ttt gga gtc atc tat tat gac     6473
   Ser Gly Lys Val Glu His Phe Gly Lys Phe Gly Val Ile Tyr Tyr Asp
       2075                2080                2085
   atc aac cag atc atc acc act gcc gtg atg acc ctc agc aaa cac ttc     6521
   Ile Asn Gln Ile Ile Thr Thr Ala Val Met Thr Leu Ser Lys His Phe
   2090                2095                2100
   gac acc cat ggg cgg atc aag gag gtc cag tat gag atg ttc cgg tcc     6569
   Asp Thr His Gly Arg Ile Lys Glu Val Gln Tyr Glu Met Phe Arg Ser
   2105                2110                2115                2120
   ctc atg tac tgg atg acg gtg caa tat gac agc atg ggc agg gtg atc     6617
   Leu Met Tyr Trp Met Thr Val Gln Tyr Asp Ser Met Gly Arg Val Ile
               2125                2130                2135
   aag agg gag cta aaa ctg ggg ccc tat gcc aat acc acg aag tac acc     6665
   Lys Arg Glu Leu Lys Leu Gly Pro Tyr Ala Asn Thr Thr Lys Tyr Thr
           2140                2145                2150
   tat gac tac gat ggg gac ggg cag ctc cag agc gtg gcc gtc aat gac     6713
   Tyr Asp Tyr Asp Gly Asp Gly Gln Leu Gln Ser Val Ala Val Asn Asp
       2155                2160                2165
   cgc ccg acc tgg cgc tac agc tat gac ctt aat ggg aat ctc cac tta     6761
   Arg Pro Thr Trp Arg Tyr Ser Tyr Asp Leu Asn Gly Asn Leu His Leu
   2170                2175                2180
   ctg aac cca ggc aac agt gtg cgc ctc atg ccc ttg cgc tat gac ctc     6809
   Leu Asn Pro Gly Asn Ser Val Arg Leu Met Pro Leu Arg Tyr Asp Leu
   2185                2190                2195                2200
   cgg gat cgg ata acc aga ctc ggg gat gtg cag tac aaa att gac gac     6857
   Arg Asp Arg Ile Thr Arg Leu Gly Asp Val Gln Tyr Lys Ile Asp Asp
               2205                2210                2215
   gat ggc tat ctg tgc cag aga ggg tct gac atc ttc gaa tac aat tcc     6905
   Asp Gly Tyr Leu Cys Gln Arg Gly Ser Asp Ile Phe Glu Tyr Asn Ser
           2220                2225                2230
   aag ggc ctc cta aca aga gcc tac aac aag gcc agc ggg tgg agt gtc     6953
   Lys Gly Leu Leu Thr Arg Ala Tyr Asn Lys Ala Ser Gly Trp Ser Val
       2235                2240                2245
   cag tac cgc tat gat ggc gta gga cgg cgg gct tcc tac aag acc aac     7001
   Gln Tyr Arg Tyr Asp Gly Val Gly Arg Arg Ala Ser Tyr Lys Thr Asn
   2250                2255                2260
   ctg ggc cac cac ctg cag tac ttc tac tct gac ctc cac aac ccg acg     7049
   Leu Gly His His Leu Gln Tyr Phe Tyr Ser Asp Leu His Asn Pro Thr
   2265                2270                2275                2280
   cgc atc acc cat gtc tac aat cac tcc aac tcg gag att acc tca ctg     7097
   Arg Ile Thr His Val Tyr Asn His Ser Asn Ser Glu Ile Thr Ser Leu
                   2285                2290                2295
   tac tac gac ctc cag ggc cac ctc ttt gcc atg gag agc agc agt ggg     7145
   Tyr Tyr Asp Leu Gln Gly His Leu Phe Ala Met Glu Ser Ser Ser Gly
               2300                2305                2310
   gag gag tac tat gtt gcc tct gat aac aca ggg act cct ctg gct gtg     7193
   Glu Glu Tyr Tyr Val Ala Ser Asp Asn Thr Gly Thr Pro Leu Ala Val
           2315                2320                2325
   ttc agc atc aac ggc ctc atg atc aaa cag ctg cag tac acg gcc tat     7241
   Phe Ser Ile Asn Gly Leu Met Ile Lys Gln Leu Gln Tyr Thr Ala Tyr
       2330                2335                2340
   ggg gag att tat tat gac tcc aac ccc gac ttc cag atg gtc att ggc     7289
   Gly Glu Ile Tyr Tyr Asp Ser Asn Pro Asp Phe Gln Met Val Ile Gly
   2345                2350                2355                2360
   ttc cat ggg gga ctc tat gac ccc ctg acc aag ctg gtc cac ttc act     7337
   Phe His Gly Gly Leu Tyr Asp Pro Leu Thr Lys Leu Val His Phe Thr
                   2365                2370                2375
   cag cgt gat tat gat gtg ctg gca gga cga tgg acc tcc cca gac tat     7385
   Gln Arg Asp Tyr Asp Val Leu Ala Gly Arg Trp Thr Ser Pro Asp Tyr
               2380                2385                2390
   acc atg tgg aaa aac gtg ggc aag gag ccg gcc ccc ttt aac ctg tat     7433
   Thr Met Trp Lys Asn Val Gly Lys Glu Pro Ala Pro Phe Asn Leu Tyr
           2395                2400                2405
   atg ttc aag agc aac aat cct ctc agc agt gag cta gat ttg aag aac     7481
   Met Phe Lys Ser Asn Asn Pro Leu Ser Ser Glu Leu Asp Leu Lys Asn
       2410                2415                2420
   tac gtg aca gat gtg aaa agc tgg ctt gtg atg ttt gga ttt cag ctt     7529
   Tyr Val Thr Asp Val Lys Ser Trp Leu Val Met Phe Gly Phe Gln Leu
   2425                2430                2435                2440
   agc aac atc att cct ggc ttc ccg aga gcc aaa atg tat ttc gtg cct     7577
   Ser Asn Ile Ile Pro Gly Phe Pro Arg Ala Lys Met Tyr Phe Val Pro
                   2445                2450                2455
   cct ccc tat gaa ttg tca gag agt caa gca agt gag aat gga cag ctc     7625
   Pro Pro Tyr Glu Leu Ser Glu Ser Gln Ala Ser Glu Asn Gly Gln Leu
               2460                2465                2470
   att aca ggt gtc caa cag aca aca gag aga cat aac cag gcc ttc atg     7673
   Ile Thr Gly Val Gln Gln Thr Thr Glu Arg His Asn Gln Ala Phe Met
           2475                2480                2485
   gct ctg gaa gga cag gtc att act aaa aag ctc cac gcc agc atc cga     7721
   Ala Leu Glu Gly Gln Val Ile Thr Lys Lys Leu His Ala Ser Ile Arg
       2490                2495                2500
   gag aaa gca ggt cac tgg ttt gcc acc acc acg ccc atc att ggc aaa     7769
   Glu Lys Ala Gly His Trp Phe Ala Thr Thr Thr Pro Ile Ile Gly Lys
   2505                2510                2515                2520
   ggc atc atg ttt gcc atc aaa gaa ggg cgg gtg acc acg ggc gtg tcc 7817
   Gly Ile Met Phe Ala Ile Lys Glu Gly Arg Val Thr Thr Gly Val Ser
   2525 2530 2535
   agc atc gcc agc gaa gat agc cgc aag gtg gca tct gtg ctg aac aac 7865
   Ser Ile Ala Ser Glu Asp Ser Arg Lys Val Ala Ser Val Leu Asn Asn
   2540 2545 2550
   gcc tac tac ctg gac aag atg cac tac agc atc gag ggc aag gac acc 7913
   Ala Tyr Tyr Leu Asp Lys Met His Tyr Ser Ile Glu Gly Lys Asp Thr
   2555 2560 2565
   cac tac ttt gtg aag att ggc tca gcc gat ggc gac ctg gtc aca cta 7961
   His Tyr Phe Val Lys Ile Gly Ser Ala Asp Gly Asp Leu Val Thr Leu
   2570 2575 2580
   ggc acc acc atc ggc cgc aag gtg cta gag agc ggg gtg aac gtg acc 8009
   Gly Thr Thr Ile Gly Arg Lys Val Leu Glu Ser Gly Val Asn Val Thr
   2585 2590 2595 2600
   gtg tcc cag ccc acg ctg ctg gtc aac ggc agg act cga agg ttc acg 8057
   Val Ser Gln Pro Thr Leu Leu Val Asn Gly Arg Thr Arg Arg Phe Thr
   2605 2610 2615
   aac att gag ttc cag tac tcc acg ctg ctg ctc agc atc cgc tat ggc 8105
   Asn Ile Glu Phe Gln Tyr Ser Thr Leu Leu Leu Ser Ile Arg Tyr Gly
   2620 2625 2630
   ctc acc ccc gac acc ctg gac gaa gag aag gcc cgc gtc ctg gac cag 8153
   Leu Thr Pro Asp Thr Leu Asp Glu Glu Lys Ala Arg Val Leu Asp Gln
   2635 2640 2645
   gcg aga cag agg gcc ctg ggc acg gcc tgg gcc aag gag cag cag aaa 8201
   Ala Arg Gln Arg Ala Leu Gly Thr Ala Trp Ala Lys Glu Gln Gln Lys
   2650 2655 2660
   gcc agg gac ggg aga gag ggg agc cgc ctg tgg act gag ggc gag aag 8249
   Ala Arg Asp Gly Arg Glu Gly Ser Arg Leu Trp Thr Glu Gly Glu Lys
   2665 2670 2675 2680
   cag cag ctt ctg agc acc ggg cgc gtg caa ggg tac gag gga tat tac 8297
   Gln Gln Leu Leu Ser Thr Gly Arg Val Gln Gly Tyr Glu Gly Tyr Tyr
   2685 2690 2695
   gtg ctt ccc gtg gag caa tac cca gag ctt gca gac agt agc agc aac 8345
   Val Leu Pro Val Glu Gln Tyr Pro Glu Leu Ala Asp Ser Ser Ser Asn
   2700 2705 2710
   atc cag ttt tta aga cag aat gag atg gga aag agg taacaaaata 8391
   Ile Gln Phe Leu Arg Gln Asn Glu Met Gly Lys Arg
   2715 2720
   atctgctgcc attccttgtc tgaatggctc agcaggagta actgttatct cctctcctaa 8451
   ggagatgaag acctaacagg ggcactgcgg ctgggctgct ttaggagacc aagtggcaag 8511
   aaagctcaca ttttttgagt tcaaatgcta ctgtccaagc gagaagtccc tcatcctgaa 8571
   gtagactaaa gcccggctga aaattccgag gaaaacaaaa caaacgaatg aatgaacaga 8631
   cacacacaat gttccaagtt cccctaaaat atgacccact tgttctgggt ctacgcagaa 8691
   aagagacgca aagtgtccaa aaggaacaaa agaacaaaaa cgaataagca aagaagaaaa 8751
   caaacaaaaa caaaacaaaa caaacacacg gaccgataaa caaagaagcg aagataagaa 8811
   agaaggcctc atatccaatt acctcactca ttcacatgtg agcgacacgc agacatccgc 8871
   gagggccagc gtcaccagac cagctgcggg acaaaccact cagactgctt gtaggacaaa 8931
   tacttctgac attttcgttt aagcaaatac aggtgcattt aaaacacgac tttgggggtg 8991
   atttgtgtgt agcgcctggg gaggggggat aaaagaggag gagtgagcac tggaaatact 9051
   ttttaaagaa aaaaaaacat gagggaataa aagaaattcc tatcaaaaat caaagtgaaa 9111
   taataccatc cagcacttaa ctctcaggtc ccaactaagt ctggcctgag ctaatttatt 9171
   tgagcgcaga gtgtaaaatt taattcaaaa tggtggctat aatcactaca gataaatttc 9231
   atactctttt gtctttggag attccattgt ggacagtaat acgcagttac agggtgtagt 9291
   ctgtttagat tccgtagttc gtgggtatca gtttcggtag aggtgcagca tcgtgacact 9351
   tttgctaaca ggtaccactt ctgatcaccc tgtacataca tgagccgaaa ggcacaatca 9411
   ctgtttcaga tttaaaatta ttagtgtgtt tgtttggtcc agaaactgag acaatcacat 9471
   gacagtcacc acgaggagag aaaatttaaa aaataaaaat aaaaacaaaa aaaattttaa 9531
   aaattaaaaa aacaaaaata aagtctaata agaactttgg tacaggaact tttttgtaat 9591
   atacatgtat gaattgttca tcgagttttt atattaattt taatttgctg ctaagcaaag 9651
   actagggaca ggcaaagata atttatggca aagtgtttaa attgtttata cataaataaa 9711
   gtctctaaaa ctcctgtg 9729

<210> 13
<211> 2724
<212> PRT
<213> Homo sapiens

<400> 13
Met Asp Val Lys Asp Arg Arg His Arg Ser Leu Thr Arg Gly Arg Cys
1               5                   10                  15

Gly Lys Glu Cys Arg Tyr Thr Ser Ser Ser Leu Asp Ser Glu Asp Cys
            20                  25                  30

Arg Val Pro Thr Gln Lys Ser Tyr Ser Ser Ser Glu Thr Leu Lys Ala
        35                  40                  45

Tyr Asp His Asp Ser Arg Met His Tyr Gly Asn Arg Val Thr Asp Leu
    50                  55                  60

Ile His Arg Glu Ser Asp Glu Phe Pro Arg Gln Gly Thr Asn Phe Thr
65                  70                  75                  80

Leu Ala Glu Leu Gly Ile Cys Glu Pro Ser Pro His Arg Ser Gly Tyr

                85                  90                  95

Cys Ser Asp Met Gly Ile Leu His Gln Gly Tyr Ser Leu Ser Thr Gly
            100                 105                 110

Ser Asp Ala Asp Ser Asp Thr Glu Gly Gly Met Ser Pro Glu His Ala
        115                 120                 125

Ile Arg Leu Trp Gly Arg Gly Ile Lys Ser Arg Arg Ser Ser Gly Leu
    130                 135                 140

Ser Ser Arg Glu Asn Ser Ala Leu Thr Leu Thr Asp Ser Asp Asn Glu
145                 150                 155                 160

Asn Lys Ser Asp Asp Glu Asn Gly Arg Pro Ile Pro Pro Thr Ser Ser
                165                 170                 175

Pro Ser Leu Leu Pro Ser Ala Gln Leu Pro Ser Ser His Asn Pro Pro
            180                 185                 190

Pro Val Ser Cys Gln Met Pro Leu Leu Asp Ser Asn Thr Ser His Gln
        195                 200                 205

Ile Met Asp Thr Asn Pro Asp Glu Glu Phe Ser Pro Asn Ser Tyr Leu
    210                 215                 220

Leu Arg Ala Cys Ser Gly Pro Gln Gln Ala Ser Ser Ser Gly Pro Pro
225                 230                 235                 240

Asn His His Ser Gln Ser Thr Leu Arg Pro Pro Leu Pro Pro Pro His
                245                 250                 255

Asn His Thr Leu Ser His His His Ser Ser Ala Asn Ser Leu Asn Arg
            260                 265                 270

Asn Ser Leu Thr Asn Arg Arg Ser Gln Ile His Ala Pro Ala Pro Ala
        275                 280                 285

Pro Asn Asp Leu Ala Thr Thr Pro Glu Ser Val Gln Leu Gln Asp Ser
    290                 295                 300

Trp Val Leu Asn Ser Asn Val Pro Leu Glu Thr Arg His Phe Leu Phe
305                 310                 315                 320

Lys Thr Ser Ser Gly Ser Thr Pro Leu Phe Ser Ser Ser Ser Pro Gly
                325                 330                 335

Tyr Pro Leu Thr Ser Gly Thr Val Tyr Thr Pro Pro Pro Arg Leu Leu
            340                 345                 350

Pro Arg Asn Thr Phe Ser Arg Lys Ala Phe Lys Leu Lys Lys Pro Ser
        355                 360                 365

Lys Tyr Cys Ser Trp Lys Cys Ala Ala Leu Ser Ala Ile Ala Ala Ala
    370                 375                 380

Leu Leu Leu Ala Ile Leu Leu Ala Tyr Phe Ile Val Pro Trp Ser Leu
385                 390                 395                 400

Lys Asn Ser Ser Ile Asp Ser Gly Glu Ala Glu Val Gly Arg Arg Val
                405                 410                 415

Thr Gln Glu Val Pro Pro Gly Val Phe Trp Arg Ser Gln Ile His Ile
            420                 425                 430

Ser Gln Pro Gln Phe Leu Lys Phe Asn Ile Ser Leu Gly Lys Asp Ala
        435                 440                 445

Leu Phe Gly Val Tyr Ile Arg Arg Gly Leu Pro Pro Ser His Ala Gln
    450                 455                 460

Tyr Asp Phe Met Glu Arg Leu Asp Gly Lys Glu Lys Trp Ser Val Val
465                 470                 475                 480

Glu Ser Pro Arg Glu Arg Arg Ser Ile Gln Thr Leu Val Gln Asn Glu
                485                 490                 495

Ala Val Phe Val Gln Tyr Leu Asp Val Gly Leu Trp His Leu Ala Phe
            500                 505                 510

Tyr Asn Asp Gly Lys Asp Lys Glu Met Val Ser Phe Asn Thr Val Val
        515                 520                 525

Leu Asp Ser Val Gln Asp Cys Pro Arg Asn Cys His Gly Asn Gly Glu
    530                 535                 540

Cys Val Ser Gly Val Cys His Cys Phe Pro Gly Phe Leu Gly Ala Asp
545                 550                 555                 560

Cys Ala Lys Ala Ala Cys Pro Val Leu Cys Ser Gly Asn Gly Gln Tyr
                565                 570                 575

Ser Lys Gly Thr Cys Gln Cys Tyr Ser Gly Trp Lys Gly Ala Glu Cys
            580                 585                 590

Asp Val Pro Met Asn Gln Cys Ile Asp Pro Ser Cys Gly Gly His Gly
        595                 600                 605

Ser Cys Ile Asp Gly Asn Cys Val Cys Ser Ala Gly Tyr Lys Gly Glu
    610                 615                 620

His Cys Glu Glu Val Asp Cys Leu Asp Pro Thr Cys Ser Ser His Gly
625                 630                 635                 640

Val Cys Val Asn Gly Glu Cys Leu Cys Ser Pro Gly Trp Gly Gly Leu
                645                 650                 655

Asn Cys Glu Leu Ala Arg Val Gln Cys Pro Asp Gln Cys Ser Gly His
            660                 665                 670

Gly Thr Tyr Leu Pro Asp Thr Gly Leu Cys Ser Cys Asp Pro Asn Trp
        675                 680                 685

Met Gly Pro Asp Cys Ser Val Glu Val Cys Ser Val Asp Cys Gly Thr
    690                 695                 700

His Gly Val Cys Ile Gly Gly Ala Cys Arg Cys Glu Glu Gly Trp Thr
705                 710                 715                 720

Gly Ala Ala Cys Asp Gln Arg Val Cys His Pro Arg Cys Ile Glu His
                725                 730                 735

Gly Thr Cys Lys Asp Gly Lys Cys Glu Cys Arg Glu Gly Trp Asn Gly
            740                 745                 750

Glu His Cys Thr Ile Asp Gly Cys Pro Asp Leu Cys Asn Gly Asn Gly
        755                 760                 765

Arg Cys Thr Leu Gly Gln Asn Ser Trp Gln Cys Val Cys Gln Thr Gly
    770                 775                 780

Trp Arg Gly Pro Gly Cys Asn Val Ala Met Glu Thr Ser Cys Ala Asp
785                 790                 795                 800

Asn Lys Asp Asn Glu Gly Asp Gly Leu Val Asp Cys Leu Asp Pro Asp
                805                 810                 815

Cys Cys Leu Gln Ser Ala Cys Gln Asn Ser Leu Leu Cys Arg Gly Ser
            820                 825                 830

Arg Asp Pro Leu Asp Ile Ile Gln Gln Gly Gln Thr Asp Trp Pro Ala
        835                 840                 845

Val Lys Ser Phe Tyr Asp Arg Ile Lys Leu Leu Ala Gly Lys Asp Ser
    850                 855                 860

Thr His Ile Ile Pro Gly Glu Asn Pro Phe Asn Ser Ser Leu Val Ser
865                 870                 875                 880

Leu Ile Arg Gly Gln Val Val Thr Thr Asp Gly Thr Pro Leu Val Gly
                885                 890                 895

Val Asn Val Ser Phe Val Lys Tyr Pro Lys Tyr Gly Tyr Thr Ile Thr
            900                 905                 910

Arg Gln Asp Gly Thr Phe Asp Leu Ile Ala Asn Gly Gly Ala Ser Leu
        915                 920                 925

Thr Leu His Phe Glu Arg Ala Pro Phe Met Ser Gln Glu Arg Thr Val
    930                 935                 940

Trp Leu Pro Trp Asn Ser Phe Tyr Ala Met Asp Thr Leu Val Met Lys
945                 950                 955                 960

Thr Glu Glu Asn Ser Ile Pro Ser Cys Asp Leu Ser Gly Phe Val Arg
                965                 970                 975

Pro Asp Pro Ile Ile Ile Ser Ser Pro Leu Ser Thr Phe Phe Ser Ala
            980                 985                 990

Ala Pro Gly Gln Asn Pro Ile Val Pro Glu Thr Gln Val Leu His Glu
        995                 1000                1005

Glu Ile Glu Leu Pro Gly Ser Asn Val Lys Leu Arg Tyr Leu Ser Ser
    1010                1015                1020

Arg Thr Ala Gly Tyr Lys Ser Leu Leu Lys Ile Thr Met Thr Gln Ser
1025                1030                1035                1040

Thr Val Pro Leu Asn Leu Ile Arg Val His Leu Met Val Ala Val Glu
                1045                1050                1055

Gly His Leu Phe Gln Lys Ser Phe Gln Ala Ser Pro Asn Leu Ala Ser

1060                1065                1070

Thr Phe Ile Trp Asp Lys Thr Asp Ala Tyr Gly Gln Arg Val Tyr Gly
        1075                1080                1085

Leu Ser Asp Ala Val Val Ser Val Gly Phe Glu Tyr Glu Thr Cys Pro
    1090                1095                1100

Ser Leu Ile Leu Trp Glu Lys Arg Thr Ala Leu Leu Gln Gly Phe Glu
1105                1110                1115                1120

Leu Asp Pro Ser Asn Leu Gly Gly Trp Ser Leu Asp Lys His His Ile
                1125                1130                1135

Leu Asn Val Lys Ser Gly Ile Leu His Lys Gly Thr Gly Glu Asn Gln
            1140                1145                1150

Phe Leu Thr Gln Gln Pro Ala Ile Ile Thr Ser Ile Met Gly Asn Gly
        1155                1160                1165

Arg Arg Arg Ser Ile Ser Cys Pro Ser Cys Asn Gly Leu Ala Glu Gly
    1170                1175                1180

Asn Lys Leu Leu Ala Pro Val Ala Leu Ala Val Gly Ile Asp Gly Ser
1185                1190                1195                1200

Leu Tyr Val Gly Asp Phe Asn Tyr Ile Arg Arg Ile Phe Pro Ser Arg
                1205                1210                1215

Asn Val Thr Ser Ile Leu Glu Leu Arg Asn Lys Glu Phe Lys His Ser
            1220                1225                1230

Asn Asn Pro Ala His Lys Tyr Tyr Leu Ala Val Asp Pro Val Ser Gly
        1235                1240                1245

Ser Leu Tyr Val Ser Asp Thr Asn Ser Arg Arg Ile Tyr Arg Val Lys
    1250                1255                1260

Ser Leu Ser Gly Thr Lys Asp Leu Ala Gly Asn Ser Glu Val Val Ala
1265                1270                1275                1280

Gly Thr Gly Glu Gln Cys Leu Pro Phe Asp Glu Ala Arg Cys Gly Asp
                1285                1290                1295

Gly Gly Lys Ala Ile Asp Ala Thr Leu Met Ser Pro Arg Gly Ile Ala
            1300                1305                1310

Val Asp Lys Asn Gly Leu Met Tyr Phe Val Asp Ala Thr Met Ile Arg
        1315                1320                1325

Lys Val Asp Gln Asn Gly Ile Ile Ser Thr Leu Leu Gly Ser Asn Asp
    1330                1335                1340

Leu Thr Ala Val Arg Pro Leu Ser Cys Asp Ser Ser Met Asp Val Ala
1345                1350                1355                1360

Gln Val Arg Leu Glu Trp Pro Thr Asp Leu Ala Val Asn Pro Met Asp
                1365                1370                1375

Asn Ser Leu Tyr Val Leu Glu Asn Asn Val Ile Leu Arg Ile Thr Glu
            1380                1385                1390

Asn His Gln Val Ser Ile Ile Ala Gly Arg Pro Met His Cys Gln Val
        1395                1400                1405

Pro Gly Ile Asp Tyr Ser Leu Ser Lys Leu Ala Ile His Ser Ala Leu
    1410                1415                1420

Glu Ser Ala Ser Ala Ile Ala Ile Ser His Thr Gly Val Leu Tyr Ile
1425                1430                1435                1440

Thr Glu Thr Asp Glu Lys Lys Ile Asn Arg Leu Arg Gln Val Thr Thr
                1445                1450                1455

Asn Gly Glu Ile Cys Leu Leu Ala Gly Ala Ala Ser Asp Cys Asp Cys
            1460                1465                1470

Lys Asn Asp Val Asn Cys Asn Cys Tyr Ser Gly Asp Asp Ala Tyr Ala
        1475                1480                1485

Thr Asp Ala Ile Leu Asn Ser Pro Ser Ser Leu Ala Val Ala Pro Asp
    1490                1495                1500

Gly Thr Ile Tyr Ile Ala Asp Leu Gly Asn Ile Arg Ile Arg Ala Val
1505                1510                1515                1520

Ser Lys Asn Lys Pro Val Leu Asn Ala Phe Asn Gln Tyr Glu Ala Ala
                1525                1530                1535

Ser Pro Gly Glu Gln Glu Leu Tyr Val Phe Asn Ala Asp Gly Ile His
            1540                1545                1550

Gln Tyr Thr Val Ser Leu Val Thr Gly Glu Tyr Leu Tyr Asn Phe Thr
        1555                1560                1565

Tyr Ser Thr Asp Asn Asp Val Thr Glu Leu Ile Asp Asn Asn Gly Asn
    1570                1575                1580

Ser Leu Lys Ile Arg Arg Asp Ser Ser Gly Met Pro Arg His Leu Leu
1585                1590                1595                1600

Met Pro Asp Asn Gln Ile Ile Thr Leu Thr Val Gly Thr Asn Gly Gly
                1605                1610                1615

Leu Lys Val Val Ser Thr Gln Asn Leu Glu Leu Gly Leu Met Thr Tyr
            1620                1625                1630

Asp Gly Asn Thr Gly Leu Leu Ala Thr Lys Ser Asp Glu Thr Gly Trp
        1635                1640                1645

Thr Thr Phe Tyr Asp Tyr Asp His Glu Gly Arg Leu Thr Asn Val Thr
    1650                1655                1660

Arg Pro Thr Gly Val Val Thr Ser Leu His Arg Glu Met Glu Lys Ser
1665                1670                1675                1680

Ile Thr Ile Asp Ile Glu Asn Ser Asn Arg Asp Asp Asp Val Thr Val
                1685                1690                1695

Ile Thr Asn Leu Ser Ser Val Glu Ala Ser Tyr Thr Val Val Gln Asp
            1700                1705                1710

Gln Val Arg Asn Ser Tyr Gln Leu Cys Asn Asn Gly Thr Leu Arg Val
        1715                1720                1725

Met Tyr Ala Asn Gly Met Gly Ile Ser Phe His Ser Glu Pro His Val
    1730                1735                1740

Leu Ala Gly Thr Ile Thr Pro Thr Ile Gly Arg Cys Asn Ile Ser Leu
1745                1750                1755                1760

Pro Met Glu Asn Gly Leu Asn Ser Ile Glu Trp Arg Leu Arg Lys Glu
                1765                1770                1775

Gln Ile Lys Gly Lys Val Thr Ile Phe Gly Arg Lys Leu Arg Val His
            1780                1785                1790

Gly Arg Asn Leu Leu Ser Ile Asp Tyr Asp Arg Asn Ile Arg Thr Glu
        1795                1800                1805

Lys Ile Tyr Asp Asp His Arg Lys Phe Thr Leu Arg Ile Ile Tyr Asp
    1810                1815                1820

Gln Val Gly Arg Pro Phe Leu Trp Leu Pro Ser Ser Gly Leu Ala Ala
1825                1830                1835                1840

Val Asn Val Ser Tyr Phe Phe Asn Gly Arg Leu Ala Gly Leu Gln Arg
                1845                1850                1855

Gly Ala Met Ser Glu Arg Thr Asp Ile Asp Lys Gln Gly Arg Ile Val
            1860                1865                1870

Ser Arg Met Phe Ala Asp Gly Lys Val Trp Ser Tyr Ser Tyr Leu Asp
        1875                1880                1885

Lys Ser Met Val Leu Leu Leu Gln Ser Gln Arg Gln Tyr Ile Phe Glu
    1890                1895                1900

Tyr Asp Ser Ser Asp Arg Leu Leu Ala Val Thr Met Pro Ser Val Ala
1905                1910                1915                1920

Arg His Ser Met Ser Thr His Thr Ser Ile Gly Tyr Ile Arg Asn Ile
                1925                1930                1935

Tyr Asn Pro Pro Glu Ser Asn Ala Ser Val Ile Phe Asp Tyr Ser Asp
            1940                1945                1950

Asp Gly Arg Ile Leu Lys Thr Ser Phe Leu Gly Thr Gly Arg Gln Val
        1955                1960                1965

Phe Tyr Lys Tyr Gly Lys Leu Ser Lys Leu Ser Glu Ile Val Tyr Asp
    1970                1975                1980

Ser Thr Ala Val Thr Phe Gly Tyr Asp Glu Thr Thr Gly Val Leu Lys
1985                1990                1995                2000

Met Val Asn Leu Gln Ser Gly Gly Phe Ser Cys Thr Ile Arg Tyr Arg
                2005                2010                2015

Lys Ile Gly Pro Leu Val Asp Lys Gln Ile Tyr Arg Phe Ser Glu Glu
            2020                2025                2030

Gly Met Val Asn Ala Arg Phe Asp Tyr Thr Tyr His Asp Asn Ser Phe

2035                2040                2045

Arg Ile Ala Ser Ile Lys Pro Val Ile Ser Glu Thr Pro Leu Pro Val
    2050                2055                2060

Asp Leu Tyr Arg Tyr Asp Glu Ile Ser Gly Lys Val Glu His Phe Gly
2065                2070                2075                2080

Lys Phe Gly Val Ile Tyr Tyr Asp Ile Asn Gln Ile Ile Thr Thr Ala
                2085                2090                2095

Val Met Thr Leu Ser Lys His Phe Asp Thr His Gly Arg Ile Lys Glu
            2100                2105                2110

Val Gln Tyr Glu Met Phe Arg Ser Leu Met Tyr Trp Met Thr Val Gln
        2115                2120                2125

Tyr Asp Ser Met Gly Arg Val Ile Lys Arg Glu Leu Lys Leu Gly Pro
    2130                2135                2140

Tyr Ala Asn Thr Thr Lys Tyr Thr Tyr Asp Tyr Asp Gly Asp Gly Gln
2145                2150                2155                2160

Leu Gln Ser Val Ala Val Asn Asp Arg Pro Thr Trp Arg Tyr Ser Tyr
                2165                2170                2175

Asp Leu Asn Gly Asn Leu His Leu Leu Asn Pro Gly Asn Ser Val Arg
            2180                2185                2190

Leu Met Pro Leu Arg Tyr Asp Leu Arg Asp Arg Ile Thr Arg Leu Gly
        2195                2200                2205

Asp Val Gln Tyr Lys Ile Asp Asp Asp Gly Tyr Leu Cys Gln Arg Gly
    2210                2215                2220

Ser Asp Ile Phe Glu Tyr Asn Ser Lys Gly Leu Leu Thr Arg Ala Tyr
2225                2230                2235                2240

Asn Lys Ala Ser Gly Trp Ser Val Gln Tyr Arg Tyr Asp Gly Val Gly
                2245                2250                2255

Arg Arg Ala Ser Tyr Lys Thr Asn Leu Gly His His Leu Gln Tyr Phe
            2260                2265                2270

Tyr Ser Asp Leu His Asn Pro Thr Arg Ile Thr His Val Tyr Asn His
        2275                2280                2285

Ser Asn Ser Glu Ile Thr Ser Leu Tyr Tyr Asp Leu Gln Gly His Leu
    2290                2295                2300

Phe Ala Met Glu Ser Ser Ser Gly Glu Glu Tyr Tyr Val Ala Ser Asp
2305                2310                2315                2320

Asn Thr Gly Thr Pro Leu Ala Val Phe Ser Ile Asn Gly Leu Met Ile
                2325                2330                2335

Lys Gln Leu Gln Tyr Thr Ala Tyr Gly Glu Ile Tyr Tyr Asp Ser Asn
            2340                2345                2350

Pro Asp Phe Gln Met Val Ile Gly Phe His Gly Gly Leu Tyr Asp Pro
        2355                2360                2365

Leu Thr Lys Leu Val His Phe Thr Gln Arg Asp Tyr Asp Val Leu Ala
    2370                2375                2380

Gly Arg Trp Thr Ser Pro Asp Tyr Thr Met Trp Lys Asn Val Gly Lys
2385                2390                2395                2400

Glu Pro Ala Pro Phe Asn Leu Tyr Met Phe Lys Ser Asn Asn Pro Leu
                2405                2410                2415

Ser Ser Glu Leu Asp Leu Lys Asn Tyr Val Thr Asp Val Lys Ser Trp
            2420                2425                2430

Leu Val Met Phe Gly Phe Gln Leu Ser Asn Ile Ile Pro Gly Phe Pro
        2435                2440                2445

Arg Ala Lys Met Tyr Phe Val Pro Pro Pro Tyr Glu Leu Ser Glu Ser
    2450                2455                2460

Gln Ala Ser Glu Asn Gly Gln Leu Ile Thr Gly Val Gln Gln Thr Thr
2465                2470                2475                2480

Glu Arg His Asn Gln Ala Phe Met Ala Leu Glu Gly Gln Val Ile Thr
                2485                2490                2495

Lys Lys Leu His Ala Ser Ile Arg Glu Lys Ala Gly His Trp Phe Ala
            2500                2505                2510

Thr Thr Thr Pro Ile Ile Gly Lys Gly Ile Met Phe Ala Ile Lys Glu
        2515                2520                2525

Gly Arg Val Thr Thr Gly Val Ser Ser Ile Ala Ser Glu Asp Ser Arg
    2530                2535                2540

Lys Val Ala Ser Val Leu Asn Asn Ala Tyr Tyr Leu Asp Lys Met His
2545                2550                2555                2560

Tyr Ser Ile Glu Gly Lys Asp Thr His Tyr Phe Val Lys Ile Gly Ser
                2565                2570                2575

Ala Asp Gly Asp Leu Val Thr Leu Gly Thr Thr Ile Gly Arg Lys Val
            2580                2585                2590

Leu Glu Ser Gly Val Asn Val Thr Val Ser Gln Pro Thr Leu Leu Val
        2595                2600                2605

Asn Gly Arg Thr Arg Arg Phe Thr Asn Ile Glu Phe Gln Tyr Ser Thr
    2610                2615                2620

Leu Leu Leu Ser Ile Arg Tyr Gly Leu Thr Pro Asp Thr Leu Asp Glu
2625                2630                2635                2640

Glu Lys Ala Arg Val Leu Asp Gln Ala Arg Gln Arg Ala Leu Gly Thr
                2645                2650                2655

Ala Trp Ala Lys Glu Gln Gln Lys Ala Arg Asp Gly Arg Glu Gly Ser
            2660                2665                2670

Arg Leu Trp Thr Glu Gly Glu Lys Gln Gln Leu Leu Ser Thr Gly Arg
        2675                2680                2685

Val Gln Gly Tyr Glu Gly Tyr Tyr Val Leu Pro Val Glu Gln Tyr Pro
2690                2695                2700

Glu Leu Ala Asp Ser Ser Ser Asn Ile Gln Phe Leu Arg Gln Asn Glu
2705                2710                2715                2720

Met Gly Lys Arg

<210> 14
<211> 609
<212> DNA
<213> Homo sapiens

<220>
<221> CDS
<222> (99)..(521)

<400> 14
ctgacatact atattagttg tttgttcact gtctccactc cagctagaat ataagttcca 60
tagggcagag tttttgttca ctgctatatt ttataagc atg aat gaa tgc atg aac 116
gaa tgg act gat aac cca caa gcc aaa gac ctc cat gac ctg cca ctg   164
ccc tcc ttt cat ttt att ctc acc tct acc aat act aaa tca cct agt   212
tat gta aat acg ata tgc act ttc atg gcc cct tgc ttt gtc ata tgc   260
tgt tcc ctt tgc ctg gaa tat aaa ctc tca aaa tac cat cca cat ttt   308
aaa atc ttc tcc aga aag ctt cct ctg tcc acc ccc acc ctc cca ccc   356
cca tat aga gta agt cag tct ttc ctt tgt gct aca ttt gta cct gta   404
tct aca gtg gct cta atc aaa ctg cac tgt gtc tct cac ttc cta gat   452
tgt gaa ctc ttt gag gct gaa gac tac tta ttc atc tct tta cct cca   500
atg cct agg aca gga cct tca taaagcaact actctataaa tgttgaaaca      551
tatgcatgac tattctgtaa caggaatgaa aatatggcat ttcaagaagt cactactc    609

<210> 15
<211> 141
<212> PRT
<213> Homo sapiens

<400> 15

<210> 16
<211> 1667
<212> DNA
<213> Homo sapiens

<220>
<221> CDS
<222> (34)..(1494)

<400> 16
gttctctcgc aggtcccaga tgtccagttc cag atg cct gga ccc aga gtg tgg    54
                                     Met Pro Gly Pro Arg Val Trp
                                     1               5

ggg aaa tat ctc tgg aga agc cct cac tcc aaa ggc tgt cca ggc gca    102
Gly Lys Tyr Leu Trp Arg Ser Pro His Ser Lys Gly Cys Pro Gly Ala
        10                  15                  20

atg tgg tgg ctg ctt ctc tgg gga gtc ctc cag gct tgc cca acc cgg    150
Met Trp Trp Leu Leu Leu Trp Gly Val Leu Gln Ala Cys Pro Thr Arg
    25                  30                  35

ggc tcc gtc ctc ttg gcc caa gag cta ccc cag cag ctg aca tcc ccc 198
Gly Ser Val Leu Leu Ala Gln Glu Leu Pro Gln Gln Leu Thr Ser Pro
40                  45                  50                  55

ggg tac cca gag ccg tat ggc aaa ggc caa gag agc agc acg gac atc 246
Gly Tyr Pro Glu Pro Tyr Gly Lys Gly Gln Glu Ser Ser Thr Asp Ile
                60                  65                  70

aag gct cca gag ggc ttt gct gtg agg ctc gtc ttc cag gac ttc gac 294
Lys Ala Pro Glu Gly Phe Ala Val Arg Leu Val Phe Gln Asp Phe Asp
            75                  80                  85

ctg gag ccg tcc cag gac tgt gca ggg gac tct gtc aca atc tca ttc 342
Leu Glu Pro Ser Gln Asp Cys Ala Gly Asp Ser Val Thr Ile Ser Phe
        90                  95                  100

gtc ggt tcg gat cca agc cag ttc tgt ggt cag caa ggc tcc cct ctg 390
Val Gly Ser Asp Pro Ser Gln Phe Cys Gly Gln Gln Gly Ser Pro Leu
    105                 110                 115

ggc agg ccc cct ggt cag agg gag ttt gta tcc tca ggg agg agt ttg 438
Gly Arg Pro Pro Gly Gln Arg Glu Phe Val Ser Ser Gly Arg Ser Leu
120                 125                 130                 135

cgg ctg acc ttc cgc aca cag cct tcc tcg gag aac aag act gcc cac 486
Arg Leu Thr Phe Arg Thr Gln Pro Ser Ser Glu Asn Lys Thr Ala His
                140                 145                 150

ctc cac aag ggc ttc ctg gcc ctc tac caa acc gtg gct gtg aac tat 534
Leu His Lys Gly Phe Leu Ala Leu Tyr Gln Thr Val Ala Val Asn Tyr
            155                 160                 165

agt cag ccc atc agc gag gcc agc agg ggc tct gag gcc atc aac gca 582
Ser Gln Pro Ile Ser Glu Ala Ser Arg Gly Ser Glu Ala Ile Asn Ala
        170                 175                 180

cct gga gac aac cct gcc aag gtc cag aac cac tgc cag gag ccc tat 630
Pro Gly Asp Asn Pro Ala Lys Val Gln Asn His Cys Gln Glu Pro Tyr
    185                 190                 195

tat cag gcc gcg gca gca ggg gca ctc acc tgt gca acc cca ggg acc 678
Tyr Gln Ala Ala Ala Ala Gly Ala Leu Thr Cys Ala Thr Pro Gly Thr
200                 205                 210                 215

tgg aaa gac aga cag gat ggg gag gag gtt ctt cag tgt atg cct gtc 726
Trp Lys Asp Arg Gln Asp Gly Glu Glu Val Leu Gln Cys Met Pro Val
                220                 225                 230

tgc gga cgg cca gtc acc ccc att gcc cag aat cag acg acc ctc ggt 774
Cys Gly Arg Pro Val Thr Pro Ile Ala Gln Asn Gln Thr Thr Leu Gly
            235                 240                 245

tct tcc aga gcc aag ctg ggc aac ttc ccc tgg caa gcc ttc acc agt 822
Ser Ser Arg Ala Lys Leu Gly Asn Phe Pro Trp Gln Ala Phe Thr Ser
        250                 255                 260

atc cac ggc cgt ggg ggc ggg gcc ctg ctg ggg gac aga tgg atc ctc 870
Ile His Gly Arg Gly Gly Gly Ala Leu Leu Gly Asp Arg Trp Ile Leu
    265                 270                 275

act gct gcc cac acc atc tac ccc aag gac agt gtt tct ctc agg aag 918
Thr Ala Ala His Thr Ile Tyr Pro Lys Asp Ser Val Ser Leu Arg Lys
280                 285                 290                 295

aac cag agt gtg aat gtg ttc ttg ggc cac aca gcc ata gat gag atg 966
Asn Gln Ser Val Asn Val Phe Leu Gly His Thr Ala Ile Asp Glu Met
                300                 305                 310

ctg aaa ctg ggg aac cac cct gtc cac cgt gtc gtt gtg cac ccc gac 1014
Leu Lys Leu Gly Asn His Pro Val His Arg Val Val Val His Pro Asp
            315                 320                 325

tac cgt cag aat gag tcc cat aac ttt agc ggg gac atc gcc ctc ctg 1062
Tyr Arg Gln Asn Glu Ser His Asn Phe Ser Gly Asp Ile Ala Leu Leu
        330                 335                 340

gag ctg cag cac agc atc ccc ctg ggc ccc aac gtc ctc ccg gtc tgt 1110
Glu Leu Gln His Ser Ile Pro Leu Gly Pro Asn Val Leu Pro Val Cys
    345                 350                 355

ctg ccc gat aat gag acc ctc tac cgc agc ggc ttg ttg ggc tac gtc 1158
Leu Pro Asp Asn Glu Thr Leu Tyr Arg Ser Gly Leu Leu Gly Tyr Val
360                 365                 370                 375

agt ggg ttt ggc atg gag atg ggc tgg cta act act gag ctg aag tac 1206
Ser Gly Phe Gly Met Glu Met Gly Trp Leu Thr Thr Glu Leu Lys Tyr
                380                 385                 390

tcg agg ctg cct gta gct ccc agg gag gcc tgc aac gcc tgg ctc caa 1254
Ser Arg Leu Pro Val Ala Pro Arg Glu Ala Cys Asn Ala Trp Leu Gln
            395                 400                 405

aag aga cag aga ccc gag gtg ttt tct gac aat atg ttc tgt gtt ggg 1302
Lys Arg Gln Arg Pro Glu Val Phe Ser Asp Asn Met Phe Cys Val Gly
        410                 415                 420

gat gag acg caa agg cac agt gtc tgc cag ggg gac agt ggc agc ctc 1350
Asp Glu Thr Gln Arg His Ser Val Cys Gln Gly Asp Ser Gly Ser Leu
    425                 430                 435

tat gtg gta tgg gac aat cat gcc cat cac tgg gtg gcc acg ggc att 1398
Tyr Val Val Trp Asp Asn His Ala His His Trp Val Ala Thr Gly Ile
440                 445                 450                 455

gtg tcc tgg ggc ata ggg tgt ggc gaa ggg tat gac ttc tac acc aag 1446
Val Ser Trp Gly Ile Gly Cys Gly Glu Gly Tyr Asp Phe Tyr Thr Lys
                460                 465                 470

gtg ctc agc tat gtg gac tgg atc aag gga gtg atg aat ggc aag aat 1494
Val Leu Ser Tyr Val Asp Trp Ile Lys Gly Val Met Asn Gly Lys Asn
            475                 480                 485

tgaccctggg ggcttgaaca gggactgacc agcacagtgg aggccccagg caacagaggg 1554

cctggagtga ggactgaaca ctggggtagg gggtgggggt ttctcttgca gtggcttggt 1614

gcaacagtga tgtgaatagg atttcccttt tttttttttt ttttaaaaaa aaa 1667

<210> 17
<211> 487

<212> PRT
<213> Homo sapiens

<400> 17
Met Pro Gly Pro Arg Val Trp Gly Lys Tyr Leu Trp Arg Ser Pro His
1               5                   10                  15

Ser Lys Gly Cys Pro Gly Ala Met Trp Trp Leu Leu Leu Trp Gly Val
            20                  25                  30

Leu Gln Ala Cys Pro Thr Arg Gly Ser Val Leu Leu Ala Gln Glu Leu
        35                  40                  45

Pro Gln Gln Leu Thr Ser Pro Gly Tyr Pro Glu Pro Tyr Gly Lys Gly
    50                  55                  60

Gln Glu Ser Ser Thr Asp Ile Lys Ala Pro Glu Gly Phe Ala Val Arg
65                  70                  75                  80

Leu Val Phe Gln Asp Phe Asp Leu Glu Pro Ser Gln Asp Cys Ala Gly
                85                  90                  95

Asp Ser Val Thr Ile Ser Phe Val Gly Ser Asp Pro Ser Gln Phe Cys
            100                 105                 110

Gly Gln Gln Gly Ser Pro Leu Gly Arg Pro Pro Gly Gln Arg Glu Phe
        115                 120                 125

Val Ser Ser Gly Arg Ser Leu Arg Leu Thr Phe Arg Thr Gln Pro Ser
    130                 135                 140

Ser Glu Asn Lys Thr Ala His Leu His Lys Gly Phe Leu Ala Leu Tyr
145                 150                 155                 160

Gln Thr Val Ala Val Asn Tyr Ser Gln Pro Ile Ser Glu Ala Ser Arg
                165                 170                 175

Gly Ser Glu Ala Ile Asn Ala Pro Gly Asp Asn Pro Ala Lys Val Gln
            180                 185                 190

Asn His Cys Gln Glu Pro Tyr Tyr Gln Ala Ala Ala Ala Gly Ala Leu
        195                 200                 205

Thr Cys Ala Thr Pro Gly Thr Trp Lys Asp Arg Gln Asp Gly Glu Glu
    210                 215                 220

Val Leu Gln Cys Met Pro Val Cys Gly Arg Pro Val Thr Pro Ile Ala
225                 230                 235                 240

Gln Asn Gln Thr Thr Leu Gly Ser Ser Arg Ala Lys Leu Gly Asn Phe
                245                 250                 255

Pro Trp Gln Ala Phe Thr Ser Ile His Gly Arg Gly Gly Gly Ala Leu
            260                 265                 270

Leu Gly Asp Arg Trp Ile Leu Thr Ala Ala His Thr Ile Tyr Pro Lys
        275                 280                 285

Asp Ser Val Ser Leu Arg Lys Asn Gln Ser Val Asn Val Phe Leu Gly
    290                 295                 300

His Thr Ala Ile Asp Glu Met Leu Lys Leu Gly Asn His Pro Val His
305                 310                 315                 320

Arg Val Val Val His Pro Asp Tyr Arg Gln Asn Glu Ser His Asn Phe
                325                 330                 335

Ser Gly Asp Ile Ala Leu Leu Glu Leu Gln His Ser Ile Pro Leu Gly
            340                 345                 350

Pro Asn Val Leu Pro Val Cys Leu Pro Asp Asn Glu Thr Leu Tyr Arg
        355                 360                 365

Ser Gly Leu Leu Gly Tyr Val Ser Gly Phe Gly Met Glu Met Gly Trp
    370                 375                 380

Leu Thr Thr Glu Leu Lys Tyr Ser Arg Leu Pro Val Ala Pro Arg Glu
385                 390                 395                 400

Ala Cys Asn Ala Trp Leu Gln Lys Arg Gln Arg Pro Glu Val Phe Ser
                405                 410                 415

Asp Asn Met Phe Cys Val Gly Asp Glu Thr Gln Arg His Ser Val Cys
            420                 425                 430

Gln Gly Asp Ser Gly Ser Leu Tyr Val Val Trp Asp Asn His Ala His
        435                 440                 445

His Trp Val Ala Thr Gly Ile Val Ser Trp Gly Ile Gly Cys Gly Glu
    450                 455                 460

Gly Tyr Asp Phe Tyr Thr Lys Val Leu Ser Tyr Val Asp Trp Ile Lys
465                 470                 475                 480

Gly Val Met Asn Gly Lys Asn
                485

<210> 18
<211> 1691
<212> DNA
<213> Homo sapiens

<400> 18
ttttttttta aaaaaaaaaa aaaaaaggga aatcctattc acatcactgt tgcaccaagc 60
cactgcaaga gaaaccccca ccccctaccc cagtgttcag tcctcactcc aggccctctg 120
ttgcctgggg cctccactgt gctggtcagt ccctgttcaa gcccccaggg tcaattcttg 180
ccattcatca ctcccttgat ccagtccaca tagctgagca ccttggtgta gaagtcatac 240
ccttcgccac accctatgcc ccaggacaca atgcccgtgg ccacccagtg atgggcatga 300
ttgtcccata ccacatagag gctgccactg tccccctggc agacactgtg cctttgcgtc 360
tcatccccaa cacagaacat attgtcagaa aacacctcgg gtctctgtct cttttggagc 420
caggcgttgc aggcctccct gggagctaca ggcagcctcg agtacttcag ctcagtagtt 480
agccagccca tctccatgcc aaacccactg acgtagccca acaagccgct gcggtagagg 540
gtctcattat cgggcagaca gaccgggagg acgttggggc ccagggggat gctgtgctgc 600
agctccagga gggcgatgtc cccgctaaag ttatgggact cattctgacg gtagtcgggg 660
tgcacaacga cacggtggac agggtggttc cccagtttca gcatctcatc tatggctgtg 720
tggcccaaga acacattcac actctggttc ttcctgagag aaacactgtc cttggggtag 780
atggtgtggg cagcagtgag gatccatctg tcccccagca gggccccgcc cccacggccg 840
tggatactgg tgaaggcttg ccaggggaag ttgcccagct tggctctgga agaaccgagg 900
gtcgtctgat tctgggcaat gggggtgact ggccgtccgc agacaggcat acactgaaga 960
acctcctccc catcctgtct gtctttccag gtccctgggg ttgcacaggt gagtgcccct 1020

gctgccgcgg cctgataata gggctcctgg cagtggttct ggaccttggc agggttgtct 1080
ccaggtgcgt tgatggcctc agagcccctg ctggcctcgc tgatgggctg actatagttc 1140
acagccacgg tttggtagag ggccaggaag cccttgtgga ggtgggcagt cttgttctcc 1200
gaggaaggct gtgtgcggaa ggtcagccgc aaactcctcc ctgaggatac aaactccctc 1260
tgaccagggg gcctgcccag aggggagcct tgctgaccac agaactggct tggatccgaa 1320
ccgacgaatg agattgtgac agagtcccct gcacagtcct gggacggctc caggtcgaag 1380
tcctggaaga cgagcctcac agcaaagccc tctggagcct tgatgtccgt gctgctctct 1440
tggcctttgc catacggctc tgggtacccg ggggatgtca gctgctgggg tagctcttgg 1500
gccaagagga cggagccccg ggttgggcaa gcctggagga ctccccagag aagcagccac 1560
cacattgcgc ctggacagcc tttggagtga gggcttctcc agagatattt cccccacact 1620
ctgggtccag gcatctggaa ctggacatct gggacctgcg agagaactgg cccaggatag 1680
ggaacaaaag g 1691

<210> 19
<211> 487
<212> PRT
<213> Homo sapiens

<400> 19
Met Pro Gly Pro Arg Val Trp Gly Lys Tyr Leu Trp Arg Ser Pro His
1               5                   10                  15

Ser Lys Gly Cys Pro Gly Ala Met Trp Trp Leu Leu Leu Trp Gly Val
            20                  25                  30

Leu Gln Ala Cys Pro Thr Arg Gly Ser Val Leu Leu Ala Gln Gln Leu
        35                  40                  45

Pro Gln Gln Leu Thr Ser Pro Gly Tyr Pro Glu Pro Tyr Gly Lys Gly
    50                  55                  60

Gln Glu Ser Ser Thr Asp Ile Lys Ala Pro Glu Gly Phe Ala Val Arg
65                  70                  75                  80

Leu Val Phe Gln Asp Phe Asp Leu Glu Pro Ser Gln Asp Cys Ala Gly
                85                  90                  95

Asp Ser Val Thr Ile Ser Phe Val Gly Ser Asp Pro Ser Gln Phe Cys
            100                 105                 110

Gly Gln Gln Gly Ser Pro Leu Gly Arg Pro Pro Gly Gln Arg Glu Phe
        115                 120                 125

Val Ser Ser Gly Arg Ser Leu Arg Leu Thr Phe Arg Thr Gln Pro Ser
    130                 135                 140

Ser Glu Asn Lys Thr Ala His Leu His Lys Gly Phe Leu Ala Leu Tyr
145                 150                 155                 160

Gln Thr Val Ala Val Asn Tyr Ser Gln Pro Ile Ser Glu Ala Ser Arg
                165                 170                 175

Gly Ser Glu Ala Ile Asn Ala Pro Gly Asp Asn Pro Ala Lys Val Gln
            180                 185                 190

Asn His Cys Gln Glu Pro Tyr Tyr Gln Ala Ala Ala Ala Gly Ala Leu
        195                 200                 205

Thr Cys Ala Thr Pro Gly Thr Trp Lys Asp Arg Gln Asp Gly Glu Glu
    210                 215                 220

Val Leu Gln Cys Met Pro Val Cys Gly Arg Pro Val Thr Pro Ile Ala
225                 230                 235                 240

Gln Asn Gln Thr Thr Leu Gly Ser Ser Arg Ala Lys Leu Gly Asn Phe
                245                 250                 255

Pro Trp Gln Ala Phe Thr Ser Ile His Gly Arg Gly Gly Gly Ala Leu
            260                 265                 270

Leu Gly Asp Arg Trp Ile Leu Thr Ala Ala His Thr Ile Tyr Pro Lys
        275                 280                 285

Asp Ser Val Ser Leu Arg Lys Asn Gln Ser Val Asn Val Phe Leu Gly
    290                 295                 300

His Thr Ala Ile Asp Glu Met Leu Lys Leu Gly Asn His Pro Val His
305                 310                 315                 320

Arg Val Val Val His Pro Asp Tyr Arg Gln Asn Glu Ser His Asn Phe
                325                 330                 335

Ser Gly Asp Ile Ala Leu Leu Glu Leu Gln His Ser Ile Pro Leu Gly
            340                 345                 350

Pro Asn Val Leu Pro Val Cys Leu Pro Asp Asn Glu Thr Leu Tyr Arg
        355                 360                 365

Ser Gly Leu Leu Gly Tyr Val Ser Gly Phe Gly Met Glu Met Gly Trp
    370                 375                 380

Leu Thr Thr Glu Leu Lys Tyr Ser Arg Leu Pro Val Ala Pro Arg Glu
385                 390                 395                 400

Ala Cys Asn Ala Trp Leu Gln Lys Arg Gln Arg Pro Glu Val Phe Ser
                405                 410                 415

Asp Asn Met Phe Cys Val Gly Asp Glu Thr Gln Arg His Ser Val Cys
            420                 425                 430

Gln Gly Asp Ser Gly Ser Leu Tyr Val Val Trp Asp Asn His Ala His
        435                 440                 445

His Trp Val Ala Thr Gly Ile Val Ser Trp Gly Ile Gly Cys Gly Glu
    450                 455                 460

Gly Tyr Asp Phe Tyr Thr Lys Val Leu Ser Tyr Val Asp Trp Ile Lys
465                 470                 475                 480

Gly Val Met Asn Gly Lys Asn
                485


<210> 20
<211> 1078
<212> DNA
<213> Homo sapiens

<220>
<221> CDS
<222> (243)..(1043)

<400> 20
ttgatccgtg ccaagtggct tttgtgggc tctgtagagt gctctaaacc cagctcggcc 60

tttgctgtat tagacagaag cacctcattc atatccctgg ggcccctgat ggtgcagtgg 120

tctggctgtg gtctgcacac cagctattct gttttgtttt gttttgtttt tttcctacct 180

ttttccaatc ctcacacctt ctgatcaaca gccccagtag ggtttaaagg tcctagagct 240

ac atg gga ttt agg ttt ctg ggc aca gcc aat tct gcc act ttt gag 287
   Met Gly Phe Arg Phe Leu Gly Thr Ala Asn Ser Ala Thr Phe Glu
   1               5                   10                  15

act tcc ctt ccc ctt cca ctt gcc cct ctc tgg ttc tct gcc acc agt 335
Thr Ser Leu Pro Leu Pro Leu Ala Pro Leu Trp Phe Ser Ala Thr Ser
                20                  25                  30

cca gaa gaa ctg agt gtc gtg ctg ggg acc aac gac tta act agc cca 383
Pro Glu Glu Leu Ser Val Val Leu Gly Thr Asn Asp Leu Thr Ser Pro
            35                  40                  45

tcc atg gaa ata aag gag gtc gcc agc atc att ctt cac aaa gac ttt 431
Ser Met Glu Ile Lys Glu Val Ala Ser Ile Ile Leu His Lys Asp Phe
        50                  55                  60

aag aga gcc aac atg gac aat gac att gcc ttg ctg ctg ctg gct tcg 479
Lys Arg Ala Asn Met Asp Asn Asp Ile Ala Leu Leu Leu Leu Ala Ser
    65                  70                  75

ccc atc aag ctc gat gac ctg aag gtg ccc atc tgc ctc ccc acg cag 527
Pro Ile Lys Leu Asp Asp Leu Lys Val Pro Ile Cys Leu Pro Thr Gln
80                  85                  90                  95

ccc ggc cct gcc aca tgg cgc gaa tgc tgg gtg gca ggt tgg ggc cag 575
Pro Gly Pro Ala Thr Trp Arg Glu Cys Trp Val Ala Gly Trp Gly Gln
                100                 105                 110

acc aat gct gct gac aaa aac tct gtg aaa acg gat ctg atg aaa gtg 623
Thr Asn Ala Ala Asp Lys Asn Ser Val Lys Thr Asp Leu Met Lys Val
            115                 120                 125

cca atg gtc atc atg gac tgg gag gag tgt tca aag atg ttt cca aaa 671
Pro Met Val Ile Met Asp Trp Glu Glu Cys Ser Lys Met Phe Pro Lys
        130                 135                 140

ctt acc aaa aat atg ctg tgt gcc gga tac aag aat gag agc tat gat 719
Leu Thr Lys Asn Met Leu Cys Ala Gly Tyr Lys Asn Glu Ser Tyr Asp
    145                 150                 155

gcc tgc aag ggt gac agt ggg ggg cct ctg gtc tgc acc cca gag cct 767
Ala Cys Lys Gly Asp Ser Gly Gly Pro Leu Val Cys Thr Pro Glu Pro
160                 165                 170                 175

ggt gag aag tgg tac cag gtg ggc atc atc agc tgg gga aag agc tgt 815
Gly Glu Lys Trp Tyr Gln Val Gly Ile Ile Ser Trp Gly Lys Ser Cys
                180                 185                 190

gga gat aag aac acc cca ggg ata tac acc tcg ttg gtg aac tac aac 863
Gly Asp Lys Asn Thr Pro Gly Ile Tyr Thr Ser Leu Val Asn Tyr Asn
            195                 200                 205

ctc tgg atc gag aaa gtg acc cag cta gga ggc agg ccc ttc aat gca     911
Leu Trp Ile Glu Lys Val Thr Gln Leu Gly Gly Arg Pro Phe Asn Ala
        210                 215                 220

gag aaa agg agg act tct gtc aaa cag aaa cct atg ggc tcc cca gtc     959
Glu Lys Arg Arg Thr Ser Val Lys Gln Lys Pro Met Gly Ser Pro Val
    225                 230                 235

tcg gga gtc cca gag cca ggc agc ccc aga tcc tgg ctc ctg ctc tgt     1007
Ser Gly Val Pro Glu Pro Gly Ser Pro Arg Ser Trp Leu Leu Leu Cys
240                 245                 250                 255

ccc ctg tcc cat gtg ttg ttc aga gct att ttg tac tgataataaa          1053
Pro Leu Ser His Val Leu Phe Arg Ala Ile Leu Tyr
                260                 265

atagaggcta ttctttcaac cgaaa                                         1078


<210> 21
<211> 267
<212> PRT
<213> Homo sapiens

<400> 21
Met Gly Phe Arg Phe Leu Gly Thr Ala Asn Ser Ala Thr Phe Glu Thr
1               5                   10                  15

Ser Leu Pro Leu Pro Leu Ala Pro Leu Trp Phe Ser Ala Thr Ser Pro
            20                  25                  30

Glu Glu Leu Ser Val Val Leu Gly Thr Asn Asp Leu Thr Ser Pro Ser
        35                  40                  45

Met Glu Ile Lys Glu Val Ala Ser Ile Ile Leu His Lys Asp Phe Lys
    50                  55                  60

Arg Ala Asn Met Asp Asn Asp Ile Ala Leu Leu Leu Leu Ala Ser Pro
65                  70                  75                  80

Ile Lys Leu Asp Asp Leu Lys Val Pro Ile Cys Leu Pro Thr Gln Pro
                85                  90                  95

Gly Pro Ala Thr Trp Arg Glu Cys Trp Val Ala Gly Trp Gly Gln Thr
            100                 105                 110

Asn Ala Ala Asp Lys Asn Ser Val Lys Thr Asp Leu Met Lys Val Pro
        115                 120                 125

Met Val Ile Met Asp Trp Glu Glu Cys Ser Lys Met Phe Pro Lys Leu
    130                 135                 140

Thr Lys Asn Met Leu Cys Ala Gly Tyr Lys Asn Glu Ser Tyr Asp Ala
145                 150                 155                 160

Cys Lys Gly Asp Ser Gly Gly Pro Leu Val Cys Thr Pro Glu Pro Gly
                165                 170                 175

Glu Lys Trp Tyr Gln Val Gly Ile Ile Ser Trp Gly Lys Ser Cys Gly
            180                 185                 190

Asp Lys Asn Thr Pro Gly Ile Tyr Thr Ser Leu Val Asn Tyr Asn Leu
        195                 200                 205

Trp Ile Glu Lys Val Thr Gln Leu Gly Gly Arg Pro Phe Asn Ala Glu
    210                 215                 220

Lys Arg Arg Thr Ser Val Lys Gln Lys Pro Met Gly Ser Pro Val Ser
225                 230                 235                 240

Gly Val Pro Glu Pro Gly Ser Pro Arg Ser Trp Leu Leu Leu Cys Pro
                245                 250                 255

Leu Ser His Val Leu Phe Arg Ala Ile Leu Tyr
            260                 265

<210> 22
<211> 1334
<212> DNA
<213> Homo sapiens

<220>
<221> CDS
<222> (499)..(1299)

<400> 22
gattttagaa ggttaatcaa aaacccgggg acagtttctt catggcataa ccacagacct 60

ttgtggcacc cgctgtcgtg ggatatcaaa tatcctctgg ggttcggaat gtgggcttat 120

tactgaagat cctgtctgct tggtcagtgg caggtctaga ctaacttctg gtcctgagtt 180

tctaaagtgc tggtagacca gttgatacaa aacagatata ataatgaatg ccttatctat 240

ctgaaggtca gtttgatccg tgccaagtgg ctttttgtgg gctgtgtaga gtgctctaaa 300

cccagctcgg cctttgctgt attagacaga agcacctcat tcatatccct ggggcccctg 360

atggtgcagt ggtctggctg tggtctgcac accagctatt ctgttttgtt ttgttttgtt 420

ttgtttttcc ctaccttttt ccaatcctca caccttctga tcaacagccc cagtagggtt 480

taaaggtcct agagctac atg gga ttt agg ttt ctg ggc aca gcc aat tct 531
Met Gly Phe Arg Phe Leu Gly Thr Ala Asn Ser
1               5                   10

gcc act ttt gag act tcc ctt ccc ctt cca ctt gcc cct ctc tgg ttc 579
Ala Thr Phe Glu Thr Ser Leu Pro Leu Pro Leu Ala Pro Leu Trp Phe
            15                  20                  25

tct gcc acc agt cca gaa gaa ctg agt gtc gtg ctg ggg acc aac gac 627
Ser Ala Thr Ser Pro Glu Glu Leu Ser Val Val Leu Gly Thr Asn Asp
        30                  35                  40

tta act agc cca tcc atg gaa ata aag gag gtc gcc agc atc att ctt 675
Leu Thr Ser Pro Ser Met Glu Ile Lys Glu Val Ala Ser Ile Ile Leu
    45                  50                  55

cac aaa gac ttt aag aga gcc aac atg gac aat gac att gcc ttg ctg 723

His Lys Asp Phe Lys Arg Ala Asn Met Asp Asn Asp Ile Ala Leu Leu
60                  65                  70                  75

ctg ctg gct tcg ccc atc aag ctc gat gac ctg aag gtg ccc atc tgc     771
Leu Leu Ala Ser Pro Ile Lys Leu Asp Asp Leu Lys Val Pro Ile Cys
                80                  85                  90

ctc ccc acg cag ccc ggc cct gcc aca tgg cgc gaa tgc tgg gtg gca     819
Leu Pro Thr Gln Pro Gly Pro Ala Thr Trp Arg Glu Cys Trp Val Ala
            95                  100                 105

ggt tgg ggc cag acc aat gct gct gac aaa aac tct gtg aaa acg gat     867
Gly Trp Gly Gln Thr Asn Ala Ala Asp Lys Asn Ser Val Lys Thr Asp
        110                 115                 120

ctg atg aaa gtg cca atg gtc atc atg gac tgg gag gag tgt tca aag     915
Leu Met Lys Val Pro Met Val Ile Met Asp Trp Glu Glu Cys Ser Lys
    125                 130                 135

atg ttt cca aaa ctt acc aaa aat atg ctg tgt gcc gga tac aag aat     963
Met Phe Pro Lys Leu Thr Lys Asn Met Leu Cys Ala Gly Tyr Lys Asn
140                 145                 150                 155

gag agc tat gat gcc tgc aag ggt gac agt ggg ggg cct ctg gtc tgc     1011
Glu Ser Tyr Asp Ala Cys Lys Gly Asp Ser Gly Gly Pro Leu Val Cys
                160                 165                 170

acc cca gag cct ggt gag aag tgg tac cag gtg ggc atc atc agc tgg     1059
Thr Pro Glu Pro Gly Glu Lys Trp Tyr Gln Val Gly Ile Ile Ser Trp
            175                 180                 185

gga aag agc tgt gga gag aag aac acc cca ggg ata tac acc tcg ttg     1107
Gly Lys Ser Cys Gly Glu Lys Asn Thr Pro Gly Ile Tyr Thr Ser Leu
        190                 195                 200

gtg aac tac aac ctc tgg atc gag aaa gtg acc cag cta gag ggc agg     1155
Val Asn Tyr Asn Leu Trp Ile Glu Lys Val Thr Gln Leu Glu Gly Arg
    205                 210                 215

ccc ttc aat gca gag aaa agg agg act tct gtc aaa cag aaa cct atg     1203
Pro Phe Asn Ala Glu Lys Arg Arg Thr Ser Val Lys Gln Lys Pro Met
220                 225                 230                 235

ggc tcc cca gtc tcg gga gtc cca gag cca ggc agc ccc aga tcc tgg     1251
Gly Ser Pro Val Ser Gly Val Pro Glu Pro Gly Ser Pro Arg Ser Trp
                240                 245                 250

ctc ctg ctc tgt ccc ctg tcc cat gtg ttg ttc aga gct att ttg tac     1299
Leu Leu Leu Cys Pro Leu Ser His Val Leu Phe Arg Ala Ile Leu Tyr
            255                 260                 265

tgataataaa atagaggcta ttctttcaac cgaaa                              1334

<210> 23
<211> 267
<212> PRT
<213> Homo sapiens

<400> 23
Met Gly Phe Arg Phe Leu Gly Thr Ala Asn Ser Ala Thr Phe Glu Thr

1                 5                   10                  15

Ser Leu Pro Leu Pro Leu Ala Pro Leu Trp Phe Ser Ala Thr Ser Pro
            20                  25                  30

Glu Glu Leu Ser Val Val Leu Gly Thr Asn Asp Leu Thr Ser Pro Ser
        35                  40                  45

Met Glu Ile Lys Glu Val Ala Ser Ile Ile Leu His Lys Asp Phe Lys
    50                  55                  60

Arg Ala Asn Met Asp Asn Asp Ile Ala Leu Leu Leu Leu Ala Ser Pro
65                  70                  75                  80

Ile Lys Leu Asp Asp Leu Lys Val Pro Ile Cys Leu Pro Thr Gln Pro
                85                  90                  95

Gly Pro Ala Thr Trp Arg Glu Cys Trp Val Ala Gly Trp Gly Gln Thr
            100                 105                 110

Asn Ala Ala Asp Lys Asn Ser Val Lys Thr Asp Leu Met Lys Val Pro
        115                 120                 125

Met Val Ile Met Asp Trp Glu Glu Cys Ser Lys Met Phe Pro Lys Leu
    130                 135                 140

Thr Lys Asn Met Leu Cys Ala Gly Tyr Lys Asn Glu Ser Tyr Asp Ala
145                 150                 155                 160

Cys Lys Gly Asp Ser Gly Gly Pro Leu Val Cys Thr Pro Glu Pro Gly
                165                 170                 175

Glu Lys Trp Tyr Gln Val Gly Ile Ile Ser Trp Gly Lys Ser Cys Gly
            180                 185                 190

Glu Lys Asn Thr Pro Gly Ile Tyr Thr Ser Leu Val Asn Tyr Asn Leu
        195                 200                 205

Trp Ile Glu Lys Val Thr Gln Leu Glu Gly Arg Pro Phe Asn Ala Glu
    210                 215                 220

Lys Arg Arg Thr Ser Val Lys Gln Lys Pro Met Gly Ser Pro Val Ser
225                 230                 235                 240

Gly Val Pro Glu Pro Gly Ser Pro Arg Ser Trp Leu Leu Leu Cys Pro
                245                 250                 255

Leu Ser His Val Leu Phe Arg Ala Ile Leu Tyr
            260                 265

<210> 24
<211> 1498
<212> DNA
<213> Homo sapiens

<400> 24
aggcgcctgg ttctgcgcgt actggctgta cggagcagga gcaagaggtc gccgccagcc 60
tccgccgccg agcctcgttc gtgtccccgc ccctcgctcc tgcagctact gctcagaaac 120
gctggggcgc ccaccctggc agactaacga agcagctccc ttcccacccc aactgcaggt 180

ctaattttgg acgctttgcc tgccatttct tccaggttga gggagccgca gaggcggagg 240
ctcgcgtatt cctgcagtca gcacccacgt cgcccccgga cgctcggtgc tcaggccctt 300
cgcgagcggg gctctccgtc tgcggtccct tgtgaaggct ctgggcggct gcagaggccg 360
gccgtccggt ttggctcacc tctcccagga aacttcacac tggagagcca aaaggagtgg 420
aagagcctgt cttggagatt ttcctgggga aatcctgagg tcattcatta tgaagtgtac 480
cgcgcgggag tggctcagag taaccacagt gctgttcatg gctagagcaa ttccagccat 540
ggtggttccc aatgccactt tattggagaa acttttggaa aaatacatgg atgaggatgg 600
tgagtggtgg atagccaaac aacgagggaa aagggccatc acagacaatg acatgcagag 660
tattttggac cttcataata aattacgaag tcaggtgtat ccaacagcct ctaatatgga 720
gtatatgaca tgggatgtag agctggaaag atctgcagaa tccagggctg aaattgcttg 780
tgggaacatg gacctgcaag cttgcttcca tcaattggac agaatttggg agcacactgg 840
ggaagatata ggcccccgac gtttcatgta caatcgtggt atgatgaagt gaaagacttt 900
agctacccat atgaacatga atgcaaccca tattgtccat tcaggtgttc tggccctgta 960
tgtacacatt atacacaggt cgtgtgggca actagtaaca gaatcggttg tgccattaat 1020
ttgtgtcata acatgaacat ctgggggcag atatggccca aagctgtcta cctggtgtgc 1080
aattactccc caaagggaaa ctggtggggc catgcccctt acaaacatgg gcggccctgt 1140
tctgcttgcc cacctagttt tggagggggc tgtagagaaa atctgtgcta caaagaaggg 1200
tcagacaggt attatccccc tcgagaagag gaaacaaatg aaatagaacg gcagcagtca 1260
caagtccatg acacccatgt ccggacaaga tcagatgata gtagcagaaa tgaagtcatt 1320
agctttggga aaagtaatga aaatataatg gttttagaaa tcctgtgtta aatattgcta 1380
tattttctta gcagttattt ctacagttaa ttacatagtc atgattgttc tacgtttcat 1440
atattatatg gtgctttgta tatgccccta ataaaatgaa tctaaacatt gaaaaaaa 1498

<210> 25
<211> 300
<212> PRT
<213> Homo sapiens

<400> 25
Met Lys Cys Thr Ala Arg Glu Trp Leu Arg Val Thr Thr Val Leu Phe
1               5                   10                  15

Met Ala Arg Ala Ile Pro Ala Met Val Val Pro Asn Ala Thr Leu Leu
            20                  25                  30

Glu Lys Leu Leu Glu Lys Tyr Met Asp Glu Asp Gly Glu Trp Trp Ile
        35                  40                  45

Ala Lys Gln Arg Gly Lys Arg Ala Ile Thr Asp Asn Asp Met Gln Ser
    50                  55                  60

Ile Leu Asp Leu His Asn Lys Leu Arg Ser Gln Val Tyr Pro Thr Ala
65                  70                  75                  80

Ser Asn Met Glu Tyr Met Thr Trp Asp Val Glu Leu Glu Arg Ser Ala
                85                  90                  95

Glu Ser Arg Ala Glu Ser Cys Leu Trp Glu His Gly Pro Ala Ser Leu
            100                 105                 110

Leu Pro Ser Ile Gly Gln Asn Leu Gly Ala His Trp Gly Arg Tyr Arg
        115                 120                 125

Pro Pro Thr Phe His Val Gln Ser Trp Tyr Asp Glu Val Lys Asp Phe
    130                 135                 140

Ser Tyr Pro Tyr Glu His Glu Cys Asn Pro Tyr Cys Pro Phe Arg Cys
145                 150                 155                 160

Ser Gly Pro Val Cys Thr His Tyr Thr Gln Val Val Trp Ala Thr Ser

165                 170                 175

Asn Arg Ile Gly Cys Ala Ile Asn Leu Cys His Asn Met Asn Ile Trp
            180                 185                 190

Gly Gln Ile Trp Pro Lys Ala Val Tyr Leu Val Cys Asn Tyr Ser Pro
        195                 200                 205

Lys Gly Asn Trp Trp Gly His Ala Pro Tyr Lys His Gly Arg Pro Cys
    210                 215                 220

Ser Ala Cys Pro Pro Ser Phe Gly Gly Gly Cys Arg Glu Asn Leu Cys
225                 230                 235                 240

Tyr Lys Glu Gly Ser Asp Arg Tyr Tyr Pro Pro Arg Glu Glu Glu Thr
                245                 250                 255

Asn Glu Ile Glu Arg Gln Gln Ser Gln Val His Asp Thr His Val Arg
            260                 265                 270

Thr Arg Ser Asp Asp Ser Ser Arg Asn Glu Val Ile Ser Phe Gly Lys
        275                 280                 285

Ser Asn Glu Asn Ile Met Val Leu Glu Ile Leu Cys
    290                 295                 300

<210> 26
<211> 22
<212> DNA
<213> Artificial Sequence

<220>
<223> Description of Artificial Sequence:Ag809 Forward
      Primer

<400> 26
atgtgatctt tggctgtgaa gt                                          22

<210> 27
<211> 23
<212> DNA
<213> Artificial Sequence

<220>
<223> Description of Artificial Sequence:Ag809 Probe
      Primer

<400> 27
ctaccccatg gcctccatcg agt                                         23

<210> 28
<211> 19
<212> DNA
<213> Artificial Sequence

<220>
<223> Description of Artificial Sequence:Ag809 Reverse
      Primer

<400> 28
ggatgtccaa gccatcctt                                                    19

<210> 29
<211> 22
<212> DNA
<213> Artificial Sequence

<220>
<223> Description of Artificial Sequence:Ag2773 Forward
      Primer

<400> 29
ccttgctttg tcatatgctg tt                                                22

<210> 30
<211> 26
<212> DNA
<213> Artificial Sequence

<220>
<223> Description of Artificial Sequence:Ag2773 Probe
      Primer

<400> 30
ccctttgcct ggaatataaa ctctca                                            26

<210> 31
<211> 22
<212> DNA
<213> Artificial Sequence

<220>
<223> Description of Artificial Sequence:Ag2773 Reverse
      Primer

<400> 31
agaggaagct ttctggagaa ga                                                22

<210> 32
<211> 21
<212> DNA
<213> Artificial Sequence

<220>
<223> Description of Artificial Sequence:Ag427 Forward
      Primer

<400> 32
gagctacagg cagcctcgag t                                                 21

<210> 33
<211> 21
<212> DNA
<213> Artificial Sequence

<220>
<223> Description of Artificial Sequence:Ag427 Probe
      Primer

<400> 33
tggcccagct gaccctgctc a                                                21


<210> 34
<211> 20
<212> DNA
<213> Artificial Sequence

<220>
<223> Description of Artificial Sequence:Ag427 Reverse
      Primer

<400> 34
ggctacgtca gtgggtttgg                                                  20


<210> 35
<211> 22
<212> DNA
<213> Artificial Sequence

<220>
<223> Description of Artificial Sequence:Ag1541 Forward
      Primer

<400> 35
agaagaacac cccagggata ta                                               22


<210> 36
<211> 26
<212> DNA
<213> Artificial Sequence

<220>
<223> Description of Artificial Sequence:Ag1541 Probe
      Primer

<400> 36
cctcgttggt gaactacaac ctctgg                                           26


<210> 37
<211> 22
<212> DNA
<213> Artificial Sequence

<220>
<223> Description of Artificial Sequence:Ag1541 Reverse
      Primer

<400> 37
cctctagctg ggtcactttc tc                                               22

<210> 38
<211> 270
<212> PRT
<213> Mus musculus

<400> 38
Met Pro Arg Leu Pro Leu Leu Leu Leu Leu Leu Pro Ser Leu Ala Arg
1               5                   10                  15

Gly Leu Gly Leu Arg Asp Ala Gly Arg Arg His Pro Glu Cys Ser Pro
            20                  25                  30

Cys Gln Gln Asp Arg Cys Pro Ala Pro Ser Pro Cys Pro Ala Pro Trp
        35                  40                  45

Ile Ser Ala Arg Asp Glu Cys Gly Cys Cys Ala Arg Cys Leu Gly Ala
    50                  55                  60

Glu Gly Ala Ser Cys Gly Gly Pro Val Gly Ser Arg Cys Gly Pro Gly
65                  70                  75                  80

Leu Val Cys Ala Ser Arg Ala Ser Gly Thr Ala Pro Glu Gly Thr Gly
                85                  90                  95

Leu Cys Val Cys Ala Gln Arg Gly Ala Val Cys Gly Ser Asp Gly Arg
            100                 105                 110

Ser Tyr Ser Ser Ile Cys Ala Leu Arg Leu Arg Ala Arg His Ala Pro
        115                 120                 125

Arg Ala His His Gly His Leu His Lys Ala Arg Asp Gly Pro Cys Glu
    130                 135                 140

Phe Ala Pro Val Val Leu Met Pro Pro Arg Asp Ile His Asn Val Thr
145                 150                 155                 160

Gly Thr Gln Val Phe Leu Ser Cys Glu Val Lys Ala Val Pro Thr Pro
                165                 170                 175

Val Ile Thr Trp Lys Lys Val Lys His Ser Pro Glu Gly Thr Glu Gly
            180                 185                 190

Leu Glu Glu Leu Pro Gly Asp His Val Asn Ile Ala Val Gln Val Arg
        195                 200                 205

Gly Gly Pro Ser Asp His Glu Thr Thr Ser Trp Ile Leu Ile Asn Pro
    210                 215                 220

Leu Arg Lys Glu Asp Glu Gly Val Tyr His Cys His Ala Ala Asn Ala
225                 230                 235                 240

Ile Gly Glu Ala Gln Ser His Gly Thr Val Thr Val Leu Asp Leu Asn
                245                 250                 255

Arg Tyr Lys Ser Leu Tyr Ser Ser Val Pro Gly Asp Leu Leu
            260                 265                 270

<210> 39
<211> 281
<212> PRT

<213> Mus musculus

<400> 39
Met Glu Arg Pro Pro Arg Ala Leu Leu Leu Gly Ala Ala Gly Leu Leu
1               5                   10                  15

Leu Leu Leu Leu Pro Leu Ser Ser Ser Ser Ser Ser Asp Ala Cys Gly
            20                  25                  30

Pro Cys Val Pro Ala Ser Cys Pro Ala Leu Pro Arg Leu Gly Cys Pro
        35                  40                  45

Leu Gly Glu Thr Arg Asp Ala Cys Gly Cys Cys Pro Val Cys Ala Arg
    50                  55                  60

Gly Glu Gly Glu Pro Cys Gly Gly Gly Ala Ala Gly Arg Gly His Cys
65                  70                  75                  80

Ala Pro Gly Met Glu Cys Val Lys Ser Arg Lys Arg Arg Arg Gly Lys
                85                  90                  95

Ala Gly Ala Ala Ala Gly Gly Pro Ala Thr Leu Ala Val Cys Val Cys
            100                 105                 110

Lys Ser Arg Tyr Pro Val Cys Gly Ser Asn Gly Ile Thr Tyr Pro Ser
        115                 120                 125

Gly Cys Gln Leu Arg Ala Ala Ser Leu Arg Ala Glu Ser Arg Gly Glu
    130                 135                 140

Lys Pro Ile Thr Gln Val Ser Lys Gly Thr Cys Glu Gln Gly Pro Ser
145                 150                 155                 160

Ile Val Thr Pro Pro Lys Asp Ile Trp Asn Val Thr Gly Ala Lys Val
                165                 170                 175

Phe Leu Ser Cys Glu Val Ile Gly Ile Pro Thr Pro Val Leu Ile Trp
            180                 185                 190

Asn Lys Val Lys Arg Asp His Ser Gly Val Gln Arg Thr Glu Leu Leu
        195                 200                 205

Pro Gly Asp Arg Glu Asn Leu Ala Ile Gln Thr Arg Gly Gly Pro Glu
    210                 215                 220

Lys His Glu Val Thr Gly Trp Val Leu Val Ser Pro Leu Ser Lys Glu
225                 230                 235                 240

Asp Ala Gly Glu Tyr Glu Cys His Ala Ser Asn Ser Gln Gly Gln Ala
                245                 250                 255

Ser Ala Ala Ala Lys Ile Thr Val Val Asp Ala Leu His Glu Ile Pro
            260                 265                 270

Leu Lys Lys Gly Glu Gly Ala Gln Leu
        275                 280


<210> 40
<211> 277
<212> PRT

<213> Homo sapiens

<400> 40
Met Glu Arg Ala Ser Leu Arg Ala Leu Leu Phe Gly Pro Ala Gly Leu
1               5                   10                  15

Leu Leu Leu Leu Leu Pro Leu Ser Ser Ser Ser Ser Ser Asp Thr Cys
            20                  25                  30

Gly Pro Cys Glu Pro Ala Ser Cys Pro Pro Leu Pro Pro Leu Gly Cys
        35                  40                  45

Leu Leu Gly Glu Thr Arg Asp Ala Cys Gly Cys Cys Pro Met Cys Ala
    50                  55                  60

Arg Gly Glu Gly Glu Pro Cys Gly Gly Gly Gly Ala Gly Arg Gly Tyr
65                  70                  75                  80

Cys Ala Pro Gly Met Glu Cys Val Lys Ser Arg Lys Arg Arg Arg Gly
                85                  90                  95

Lys Ala Gly Ala Ala Ala Gly Gly Pro Gly Val Ser Gly Val Cys Val
            100                 105                 110

Cys Lys Ser Arg Val Pro Val Cys Gly Ser Asp Gly Thr Thr Tyr Pro
        115                 120                 125

Ser Gly Cys Gln Leu Arg Ala Ala Ser Gln Arg Ala Glu Ser Arg Gly
    130                 135                 140

Glu Lys Ala Ile Thr Gln Val Ser Lys Gly Thr Cys Glu Gln Gly Pro
145                 150                 155                 160

Ser Ile Val Thr Pro Pro Lys Asp Ile Trp Asn Val Thr Gly Ala Gln
                165                 170                 175

Val Tyr Leu Ser Cys Glu Val Ile Gly Ile Pro Thr Pro Val Leu Ile
            180                 185                 190

Trp Asn Lys Val Lys Arg Gly His Tyr Gly Val Gln Arg Thr Glu Leu
        195                 200                 205

Leu Pro Gly Asp Arg Asp Asn Leu Ala Ile Gln Thr Arg Gly Gly Pro
    210                 215                 220

Glu Lys His Glu Val Thr Gly Trp Val Leu Val Ser Pro Leu Ser Lys
225                 230                 235                 240

Glu Asp Ala Gly Glu Tyr Glu Cys His Ala Ser Asn Ser Gln Gly Gln
                245                 250                 255

Ala Ser Ala Ser Ala Lys Ile Thr Val Val Asp Ala Leu His Glu Ile
            260                 265                 270

Ala Ser Glu Lys Arg
        275


<210> 41
<211> 281
<212> PRT

<213> Mus musculus

<400> 41
Met Glu Arg Pro Pro Arg Ala Leu Leu Leu Gly Ala Ala Gly Leu Leu
1               5                   10                  15

Leu Leu Leu Leu Pro Leu Ser Ser Ser Ser Ser Ser Asp Ala Cys Gly
            20                  25                  30

Pro Cys Val Pro Ala Ser Cys Pro Ala Leu Pro Arg Leu Gly Cys Pro
        35                  40                  45

Leu Gly Glu Thr Arg Asp Ala Cys Gly Cys Cys Pro Val Cys Ala Arg
    50                  55                  60

Gly Glu Gly Glu Pro Cys Gly Gly Gly Ala Ala Gly Arg Gly His Cys
65                  70                  75                  80

Ala Pro Gly Met Glu Cys Val Lys Ser Arg Lys Arg Arg Lys Gly Lys
                85                  90                  95

Ala Gly Ala Ala Ala Gly Gly Pro Ala Thr Leu Ala Val Cys Val Cys
            100                 105                 110

Lys Ser Arg Tyr Pro Val Cys Gly Ser Asn Gly Ile Thr Tyr Pro Ser
        115                 120                 125

Gly Cys Gln Leu Arg Ala Ala Ser Leu Arg Ala Glu Ser Arg Gly Glu
    130                 135                 140

Lys Ala Ile Thr Gln Val Ser Lys Gly Thr Cys Glu Gln Gly Pro Ser
145                 150                 155                 160

Ile Val Thr Pro Pro Lys Asp Ile Trp Asn Val Thr Gly Ala Lys Val
                165                 170                 175

Phe Leu Ser Cys Glu Val Ile Gly Ile Pro Thr Pro Val Leu Ile Trp
            180                 185                 190

Asn Lys Val Lys Arg Asp His Ser Gly Val Gln Arg Thr Glu Leu Leu
        195                 200                 205

Pro Gly Asp Arg Glu Asn Leu Ala Ile Gln Thr Arg Gly Gly Pro Glu
    210                 215                 220

Lys His Glu Val Thr Gly Trp Val Leu Val Ser Pro Leu Ser Lys Glu
225                 230                 235                 240

Asp Ala Gly Glu Tyr Glu Cys His Ala Ser Asn Ser Gln Gly Gln Ala
                245                 250                 255

Ser Ala Ala Ala Lys Ile Thr Val Val Asp Ala Leu His Glu Ile Pro
            260                 265                 270

Leu Lys Lys Gly Glu Gly Ala Gln Leu
        275                 280


<210> 42
<211> 282
<212> PRT

<213> Homo sapiens

<400> 42
Met Glu Arg Pro Ser Leu Arg Ala Leu Leu Leu Gly Ala Ala Gly Leu
1               5                   10                  15

Leu Leu Leu Leu Leu Pro Leu Ser Ser Ser Ser Ser Ser Asp Thr Cys
            20                  25                  30

Gly Pro Cys Glu Pro Ala Ser Cys Pro Pro Leu Pro Pro Leu Gly Cys
        35                  40                  45

Leu Leu Gly Glu Thr Arg Asp Ala Cys Gly Cys Cys Pro Met Cys Ala
    50                  55                  60

Arg Gly Glu Gly Glu Pro Cys Gly Gly Gly Gly Ala Gly Arg Gly Tyr
65                  70                  75                  80

Cys Ala Pro Gly Met Glu Cys Val Lys Ser Arg Lys Arg Arg Lys Gly
                85                  90                  95

Lys Ala Gly Ala Ala Ala Gly Gly Pro Gly Val Ser Gly Val Cys Val
            100                 105                 110

Cys Lys Ser Arg Tyr Pro Val Cys Gly Ser Asp Gly Thr Thr Tyr Pro
        115                 120                 125

Ser Gly Cys Gln Leu Arg Ala Ala Ser Gln Arg Ala Glu Ser Arg Gly
    130                 135                 140

Glu Lys Ala Ile Thr Gln Val Ser Lys Gly Thr Cys Glu Gln Gly Pro
145                 150                 155                 160

Ser Ile Val Thr Pro Pro Lys Asp Ile Trp Asn Val Thr Gly Ala Gln
                165                 170                 175

Val Tyr Leu Ser Cys Glu Val Ile Gly Ile Pro Thr Pro Val Leu Ile
            180                 185                 190

Trp Asn Lys Val Lys Arg Gly His Tyr Gly Val Gln Arg Thr Glu Leu
        195                 200                 205

Leu Pro Gly Asp Arg Asp Asn Leu Ala Ile Gln Thr Arg Gly Gly Pro
    210                 215                 220

Glu Lys His Glu Val Thr Gly Trp Val Leu Val Ser Pro Leu Ser Lys
225                 230                 235                 240

Glu Asp Ala Gly Glu Tyr Glu Cys His Ala Ser Asn Ser Gln Gly Gln
                245                 250                 255

Ala Ser Ala Ser Ala Lys Ile Thr Val Val Asp Ala Leu His Glu Ile
            260                 265                 270

Pro Val Lys Lys Gly Glu Gly Ala Glu Leu
        275                 280


<210> 43
<211> 144
<212> PRT

<213> Rattus norvegicus

<400> 43
Pro Leu Arg Phe Leu Ser Gln Thr Glu Ser Ile Thr Ala Phe Met Gly
1               5                   10                  15

Asp Thr Val Leu Leu Lys Cys Glu Val Ile Gly Asp Pro Met Pro Thr
            20                  25                  30

Ile His Trp Gln Lys Asn Gln Gln Asp Leu Thr Pro Asn Pro Gly Asp
        35                  40                  45

Ser Arg Val Val Val Pro Pro Trp Phe Leu Asn His Pro Ser Asn Leu
    50                  55                  60

Tyr Ala Tyr Glu Ser Met Asp Ile Glu Phe Glu Cys Ala Val Ser Gly
65                  70                  75                  80

Lys Pro Val Pro Thr Val Asn Trp Met Lys Asn Gly Asp Val Val Val
                85                  90                  95

Ile Ser Asp Tyr Phe Gln Ile Val Gly Gly Ser Asn Leu Arg Ile Leu
            100                 105                 110

Gly Val Val Lys Ser Asp Glu Gly Phe Tyr Gln Cys Val Ala Glu Asn
        115                 120                 125

Glu Ala Gly Asn Ala Gln Ser Ser Ala Gln Leu Ile Val Pro Lys Pro
    130                 135                 140

<210> 44
<211> 1502
<212> PRT
<213> Homo sapiens

<400> 44


<210> 45
<211> 1948
<212> PRT
<213> Homo sapiens

<400> 45


<210> 46
<211> 4719
<212> DNA
<213> Homo sapiens

<400> 46
agaatgtcct tctggcactc cagacccgtc tgcagccact ccaagaagga gacagcagac 60
aagaccctgc ctcccagaag cgcctcctgg tggaatctct gttcagggac ttagatgcag 120
atggcaatgg ccacctcagc agctccgaac tggctcagca tgtgctgaag aagcaggacc 180
tggatgaaga cttacttggt tgctcaccag gtgacctcct ccgatttgac gattacaaca 240
gtgacagctc cctgaccctc cgcgagttct acatggcctt ccaagtggtt cagctcagcc 300
tcgcccccga ggacagggtc agtgtgacca cagtgaccgt ggggctgagc acagtgctga 360
cctgcgccgt ccatggagac ctgaggccac caatcatctg gaagcgcaac gggctcaccc 420
tgaacttcct ggacttggaa gacatcaatg actttggaga ggatgattcc ctgtacatca 480
ccaaggtgac caccatccac atgggcaatt acacctgcca tgcttccggc cacgagcagc 540
tgttccagac ccacgtcctg caggtgaatg tgccgccagt catccgtgtc tatccagaga 600
gccaggcaca ggagcctgga gtggcagcca gcctaagatg ccatgctgag ggcattccca 660
tgcccagaat cacttggctg aaaaacggcg tggatgtctc aactcagatg tccaaacagc 720
tctccctttt agccaatggg agcgaactcc acatcagcag tgttcggtat gaagacacag 780
gggcatacac ctgcattgcc aaaaatgaag tgggtgtgga tgaagatatc tcctcgctct 840
tcattgaaga ctcagctaga aagacccttg caaacatcct gtggcgagag gaaggcctca 900
gcgtgggaaa catgttctat gtcttctccg acgacggtat catcgtcatc catcctgtgg 960
actgtgagat ccagaggcac ctcaaaccca cggaaaagat tttcatgagc tatgaagaaa 1020
tctgtcctca aagagaaaaa aatgcaaccc agccctgcca gtgggtatct gcagtcaatg 1080
tccggaaccg gtacatctat gtggcccagc cagcactgag cagagtcctt gtggtcgaca 1140
tccaagccca gaaagtccta cagtccatag gtgtggaccc tctgccggct aagctgtcct 1200
atgacaagtc acatgaccaa gtgtgggtcc tgagctgggg ggacgtgcac aagtcccgac 1260
caagtctcca ggtgatcaca gaagccagca ccggccagag ccagcacctc atccgcacac 1320
cctttgcagg agtggatgat ttcttcattc ccccaacaaa cctcatcatc aaccacatca 1380
ggtttggctt catcttcaac aagtctgatc ctgcagtcca caaggtggac ctggaaacaa 1440
tgatgcccct caagaccatc ggcctgcacc accatggctg cgtgccccag gccatggcac 1500
acacccacct gggcggctac ttcttcatcc agtgccgaca ggacagcccc gcctctgctg 1560
cccgacagct gctcgttgac agtgtcacag actctgtgct tggccccaat ggtgatgtaa 1620
caggcacccc acacacatcc cccgacgggc gcttcatagt cagtgctgca gctgacagcc 1680
cctggctgca cgtgcaggag atcacagtgc ggggcgagat ccagaccctg tatgacctgc 1740
aaataaactc gggcatctca gacttggcct tccagcgctc cttcactgaa agcaatcaat 1800

acaacatcta cgcggctctg cacacggagc cggacctgct gttcctggag ctgtccacgg 1860
ggaaggtggg catgctgaag aacttaaagg agccacccgc agggccagct cagccctggg 1920
ggggtaccca cagaatcatg agggacagtg ggctgtttgg acagtacctc ctcacaccag 1980
cccgagagtc actgttcctc atcaatggga gacaaaacac gctgcggtgt gaggtgtcag 2040
gtataaaggg ggggaccaca gtggtgtggg tgggtgaggt atgaagggcc cagagcagag 2100
ccctgggcca aggaacaccc cctagtcctg acactgcagc ctcaagcagg tacgctgtac 2160
atttttacag acaaaagcaa aaacctgtac tcgctttgtg gttcaacact ggtctccttg 2220
caagtttcct agtataaggt atgcgctgct accaagattg gggttttttc gttaggaagt 2280
atgatttatg ccttgagcta cgatgagaac atatgctgct gtgtaaaggg atcatttctg 2340
tgccaagctg cacaccgagt gacctgggga catcatggaa ccaaggatcc ctgctctcca 2400
agcagacacc tctgtcagtt gccttcacat agtcattgtc ccttactgcc agacccagcc 2460
agactttgcc ctgacggagt ggcccggaag cagaggccga ccaggagcag gggcctccct 2520
cccgaactga aagcccatcc gtcctcgcgt gggaccgcat cttctccctc gcagctgctt 2580
cttgcttttc tttccatttg acttgctgta agcctgaggg agagccaaca agacttactg 2640
catcttgggg gatggggaaa tcactcactt tattttggaa atttttgatt aaaaaaaaat 2700
tttataatct caaatgctag taagcagaaa gatgctctcc gaggtccaac tatatccttc 2760
cctgccttag gccgagtctc gggggtggtc acaaccccac atcccacagc cagaaagaac 2820
aatggtcatc tgagaatact ggccctgtcg actattgcca ccctgcttct ccaagagcag 2880
accaggccac ctcatccgta aggactcggt tctgtgttgg gaccccaaaa aaccagaaca 2940
agttctgtgt gcctcctttc agcacagaag ggagacatct cattagtcag gtctggtacc 3000
ccagattcag ggcagactgg gcttgcctgg caaggtatgg gtggcctcca ggctcaatgc 3060
agaaacccca aggacacgag tggggccagg tgagttcctg aagctatacc ttttcaaaac 3120
agattttgtt ttcctacctg tggcccatcc actcctctct ggtaccccat ccccgcatca 3180
gcactgcaga gagaacacat ttcggcgagg gttttcttac ccacattccc caatcaatac 3240
acacacactg cagaacccag aacagaaggc cacaggctgg cactactgca ttctccttat 3300
gtgtctcagg ctgtggtgac tctcacatgg gcatcgaaga agtacaaccc acatagccct 3360
ctggagaccg cctagatcag agactcagca aaaacaggct cgccttccct ctcccacata 3420
tgagtggaac ttacatgtgt cctggtttga atgatcattt tgcaagccac acgggttggg 3480
agaggtggtc tcaccacaga cgtctttgct aatttggcca ccttcaccta ctgacatgac 3540
caggattttc ctttgccatt aaggaatgaa ctctttcaag gagaggaaac cctagactct 3600
gtgtcactct caacacacac agctcctttc actcctgcct gactgccaag ccacctgcat 3660
cccccgcccc agatctcatg agatcaatca cttgtatgtc tcacgcaact tggtccacca 3720
aacgcctgtc ccctgtaact cctaggggtg cgcctagaca ggtacgtctg ttttttattt 3780
taaaagatat gctatgtaga tataagttga ggaagctcac ctcaaaagcc tagaatgcag 3840
tttcacagta gctgggatgc atggatgacc catctcaccc cttttttttt cctgcctcaa 3900
tatcttgata tgttatgttt actcccaatc tcccattttt accactaaaa ttctccaact 3960
ttcataaact tttttttgga aaaatttcca ttgtatcagc ccctgacaga aaaaggatct 4020
ctgagcctaa aggaggaaaa gtcccaccaa ctaccagacc agaacacgag cccctctggg 4080
cagcaggatt cctaagtcaa agaccagttt gacccaaact ggccttttaa aataatcagg 4140
agtgacagag tcaacttctg cagcacctgc ttctccccca ctgtcccttc catcttggaa 4200
tgtgtctaaa aaagcatagc tgccctttgc tgtcctcaga gtgcatttcc tggagacggc 4260
aggcttaggt ctcactgaca gcatgccaga cacaactgaa tcgaagcagg cctgaagcct 4320
aggtcagggt ttcaggagtc cagccccagg aggcaaagtc accaatgcag ggaggtaaat 4380
gccttttggc aggaaaacca atagagttgg ttgggtgggg agtcaggggt gggaggagaa 4440
ggaggaagag gaggaaggcc agactggcct gccctttctc ccatacttca ccccagcaga 4500
ggttcatggg acacagttgg aaagccactg ggaggaaatg cctcactaca ggggggcctc 4560
ctgtagcaag cccagccggt aatcctccta atgaacccac aaggtcaatt cacaactgat 4620
atcttagcta ttaaagaagt actgacttta ccaaaagaat catcaagaaa gctatttata 4680
taaacccccct cagtcatttt gaaataaaat taattttac 4719

<210> 47
<211> 850
<212> PRT
<213> Homo sapiens

<400> 47
Lys Ala Ile Arg Met Phe Lys Cys Trp Ser Val Val Leu Val Leu Gly
1               5                   10                  15

Phe Ile Phe Leu Glu Ser Glu Gly Arg Pro Thr Lys Glu Gly Gly Tyr

            20                  25                  30

Gly Leu Lys Ser Tyr Gln Pro Leu Met Arg Leu Arg His Lys Glu Lys
        35                  40                  45

Asn Gln Glu Ser Ser Arg Val Lys Gly Phe Met Ile Gln Asp Gly Pro
    50                  55                  60

Phe Gly Ser Cys Glu Asn Lys Tyr Cys Gly Leu Gly Arg His Cys Val
65                  70                  75                  80

Thr Ser Arg Glu Thr Gly Gln Ala Glu Cys Ala Cys Met Asp Leu Cys
                85                  90                  95

Lys Arg His Tyr Lys Pro Val Cys Gly Ser Asp Gly Glu Phe Tyr Glu
            100                 105                 110

Asn His Cys Glu Val His Arg Ala Ala Cys Leu Lys Lys Gln Lys Ile
        115                 120                 125

Thr Ile Val His Asn Glu Asp Cys Phe Phe Lys Gly Asp Lys Cys Lys
    130                 135                 140

Thr Thr Glu Tyr Ser Lys Met Lys Asn Met Leu Leu Asp Leu Gln Asn
145                 150                 155                 160

Gln Lys Tyr Ile Met Gln Glu Asn Glu Asn Pro Asn Gly Asp Asp Ile
                165                 170                 175

Ser Arg Lys Lys Leu Leu Val Asp Gln Met Phe Lys Tyr Phe Asp Ala
            180                 185                 190

Asp Ser Asn Gly Leu Val Asp Ile Asn Glu Leu Thr Gln Val Ile Lys
        195                 200                 205

Gln Glu Glu Leu Gly Lys Asp Leu Phe Asp Cys Thr Leu Tyr Val Leu
    210                 215                 220

Leu Lys Tyr Asp Asp Phe Asn Ala Asp Lys His Leu Ala Leu Glu Glu
225                 230                 235                 240

Phe Tyr Arg Ala Phe Gln Val Ile Gln Leu Ser Leu Pro Glu Asp Gln
                245                 250                 255

Lys Leu Ser Ile Thr Ala Ala Thr Val Gly Gln Ser Ala Val Leu Ser
            260                 265                 270

Cys Ala Ile Gln Gly Thr Leu Arg Pro Pro Ile Ile Trp Lys Arg Asn
        275                 280                 285

Asn Ile Ile Leu Asn Asn Leu Asp Leu Glu Asp Ile Asn Asp Phe Gly
    290                 295                 300

Asp Asp Gly Ser Leu Tyr Ile Thr Lys Val Thr Thr Thr His Val Gly
305                 310                 315                 320

Asn Tyr Thr Cys Tyr Ala Asp Gly Tyr Glu Gln Val Tyr Gln Thr His
                325                 330                 335

Ile Phe Gln Val Asn Val Pro Pro Val Ile Arg Val Tyr Pro Glu Ser
            340                 345                 350

Gln Ala Arg Glu Pro Gly Val Thr Ala Ser Leu Arg Cys His Ala Glu
        355                 360                 365

Gly Ile Pro Lys Pro Gln Leu Gly Trp Leu Lys Asn Gly Ile Asp Ile
    370                 375                 380

Thr Pro Lys Leu Ser Lys Gln Leu Thr Leu Gln Ala Asn Gly Ser Glu
385                 390                 395                 400

Val His Ile Ser Asn Val Arg Tyr Glu Asp Thr Gly Ala Tyr Thr Cys
                405                 410                 415

Ile Ala Lys Asn Glu Ala Gly Val Asp Glu Asp Ile Ser Ser Leu Phe
            420                 425                 430

Val Glu Asp Ser Ala Arg Lys Thr Leu Ala Asn Ile Leu Trp Arg Glu
        435                 440                 445

Glu Gly Leu Gly Ile Gly Asn Met Phe Tyr Val Phe Tyr Glu Asp Gly
    450                 455                 460

Ile Lys Val Ile Gln Pro Ile Glu Cys Glu Phe Gln Arg His Ile Lys
465                 470                 475                 480

Pro Ser Glu Lys Leu Leu Gly Phe Gln Asp Glu Val Cys Pro Lys Ala
                485                 490                 495

Glu Gly Asp Glu Val Gln Arg Cys Val Trp Ala Ser Ala Val Asn Val
            500                 505                 510

Lys Asp Lys Phe Ile Tyr Val Ala Gln Pro Thr Leu Asp Arg Val Leu
        515                 520                 525

Ile Val Asp Val Gln Ser Gln Lys Val Val Gln Ala Val Ser Thr Asp
    530                 535                 540

Pro Val Pro Val Lys Leu His Tyr Asp Lys Ser His Asp Gln Val Trp
545                 550                 555                 560

Val Leu Ser Trp Gly Thr Leu Glu Lys Thr Ser Pro Thr Leu Gln Val
                565                 570                 575

Ile Thr Leu Ala Ser Gly Asn Val Pro His His Thr Ile His Thr Gln
            580                 585                 590

Pro Val Gly Lys Gln Phe Asp Arg Val Asp Asp Phe Phe Ile Pro Thr
        595                 600                 605

Thr Thr Leu Ile Ile Thr His Met Arg Phe Gly Phe Ile Leu His Lys
    610                 615                 620

Asp Glu Ala Ala Leu Gln Lys Ile Asp Leu Glu Thr Met Ser Tyr Ile
625                 630                 635                 640

Lys Thr Ile Asn Leu Lys Asp Tyr Lys Cys Val Pro Gln Ser Leu Ala
                645                 650                 655

Tyr Thr His Leu Gly Gly Tyr Tyr Phe Ile Gly Cys Lys Pro Asp Ser
            660                 665                 670

Thr Gly Ala Val Ser Pro Gln Val Met Val Asp Gly Val Thr Asp Ser
        675                 680                 685

Val Ile Gly Phe Asn Ser Asp Val Thr Gly Thr Pro Tyr Val Ser Pro
    690                 695                 700

Asp Gly His Tyr Leu Val Ser Ile Asn Asp Val Lys Gly Leu Val Arg
705                 710                 715                 720

Val Gln Tyr Ile Thr Ile Arg Gly Glu Ile Gln Glu Ala Phe Asp Ile
                725                 730                 735

Tyr Thr Asn Leu His Ile Ser Asp Leu Ala Phe Gln Pro Ser Phe Thr
            740                 745                 750

Glu Ala His Gln Tyr Asn Ile Tyr Gly Ser Ser Ser Thr Gln Thr Asp
        755                 760                 765

Val Leu Phe Val Glu Leu Ser Ser Gly Lys Val Lys Met Ile Lys Ser
    770                 775                 780

Leu Lys Glu Pro Leu Lys Ala Glu Glu Trp Pro Trp Asn Arg Lys Asn
785                 790                 795                 800

Arg Gln Ile Gln Asp Ser Gly Leu Phe Gly Gln Tyr Leu Met Thr Pro
                805                 810                 815

Ser Lys Asp Ser Leu Phe Ile Leu Asp Gly Arg Leu Asn Lys Leu Asn
            820                 825                 830

Cys Glu Ile Thr Glu Val Glu Lys Gly Asn Thr Val Ile Trp Val Gly
        835                 840                 845

Asp Ala
    850


<210> 48
<211> 693
<212> PRT
<213> Homo sapiens

<400> 48
Asn Val Leu Leu Ala Leu Gln Thr Arg Leu Gln Pro Leu Gln Glu Gly
1               5                   10                  15

Asp Ser Arg Gln Asp Pro Ala Ser Gln Lys Arg Leu Leu Val Glu Ser
            20                  25                  30

Leu Phe Arg Asp Leu Asp Ala Asp Gly Asn Gly His Leu Ser Ser Ser
        35                  40                  45

Glu Leu Ala Gln His Val Leu Lys Lys Gln Asp Leu Asp Glu Asp Leu
    50                  55                  60

Leu Gly Cys Ser Pro Gly Asp Leu Leu Arg Phe Asp Asp Tyr Asn Ser
65                  70                  75                  80

Asp Ser Ser Leu Thr Leu Arg Glu Phe Tyr Met Ala Phe Gln Val Val
                85                  90                  95

Gln Leu Ser Leu Ala Pro Glu Asp Arg Val Ser Val Thr Thr Val Thr
            100                 105                 110

Val Gly Leu Ser Thr Val Leu Thr Cys Ala Val His Gly Asp Leu Arg
        115                 120                 125

Pro Pro Ile Ile Trp Lys Arg Asn Gly Leu Thr Leu Asn Phe Leu Asp
    130                 135                 140

Leu Glu Asp Ile Asn Asp Phe Gly Glu Asp Asp Ser Leu Tyr Ile Thr
145                 150                 155                 160

Lys Val Thr Thr Ile His Met Gly Asn Tyr Thr Cys His Ala Ser Gly
                165                 170                 175

His Glu Gln Leu Phe Gln Thr His Val Leu Gln Val Asn Val Pro Pro
            180                 185                 190

Val Ile Arg Val Tyr Pro Glu Ser Gln Ala Gln Glu Pro Gly Val Ala
        195                 200                 205

Ala Ser Leu Arg Cys His Ala Glu Gly Ile Pro Met Pro Arg Ile Thr
    210                 215                 220

Trp Leu Lys Asn Gly Val Asp Val Ser Thr Gln Met Ser Lys Gln Leu
225                 230                 235                 240

Ser Leu Leu Ala Asn Gly Ser Glu Leu His Ile Ser Ser Val Arg Tyr
                245                 250                 255

Glu Asp Thr Gly Ala Tyr Thr Cys Ile Ala Lys Asn Glu Val Gly Val
            260                 265                 270

Asp Glu Asp Ile Ser Ser Leu Phe Ile Glu Asp Ser Ala Arg Lys Thr
        275                 280                 285

Leu Ala Asn Ile Leu Trp Arg Glu Glu Gly Leu Ser Val Gly Asn Met
    290                 295                 300

Phe Tyr Val Phe Ser Asp Asp Gly Ile Ile Val Ile His Pro Val Asp
305                 310                 315                 320

Cys Glu Ile Gln Arg His Leu Lys Pro Thr Glu Lys Ile Phe Met Ser
                325                 330                 335

Tyr Glu Glu Ile Cys Pro Gln Arg Glu Lys Asn Ala Thr Gln Pro Cys
            340                 345                 350

Gln Trp Val Ser Ala Val Asn Val Arg Asn Arg Tyr Ile Tyr Val Ala
        355                 360                 365

Gln Pro Ala Leu Ser Arg Val Leu Val Val Asp Ile Gln Ala Gln Lys
    370                 375                 380

Val Leu Gln Ser Ile Gly Val Asp Pro Leu Pro Ala Lys Leu Ser Tyr
385                 390                 395                 400

Asp Lys Ser His Asp Gln Val Trp Val Leu Ser Trp Gly Asp Val His
                405                 410                 415

Lys Ser Arg Pro Ser Leu Gln Val Ile Thr Glu Ala Ser Thr Gly Gln

            420                 425                 430

Ser Gln His Leu Ile Arg Thr Pro Phe Ala Gly Val Asp Asp Phe Phe
        435                 440                 445

Ile Pro Pro Thr Asn Leu Ile Ile Asn His Ile Arg Phe Gly Phe Ile
    450                 455                 460

Phe Asn Lys Ser Asp Pro Ala Val His Lys Val Asp Leu Glu Thr Met
465                 470                 475                 480

Met Pro Leu Lys Thr Ile Gly Leu His His His Gly Cys Val Pro Gln
                485                 490                 495

Ala Met Ala His Thr His Leu Gly Gly Tyr Phe Phe Ile Gln Cys Arg
            500                 505                 510

Gln Asp Ser Pro Ala Ser Ala Ala Arg Gln Leu Leu Val Asp Ser Val
        515                 520                 525

Thr Asp Ser Val Leu Gly Pro Asn Gly Asp Val Thr Gly Thr Pro His
    530                 535                 540

Thr Ser Pro Asp Gly Arg Phe Ile Val Ser Ala Ala Ala Asp Ser Pro
545                 550                 555                 560

Trp Leu His Val Gln Glu Ile Thr Val Arg Gly Glu Ile Gln Thr Leu
                565                 570                 575

Tyr Asp Leu Gln Ile Asn Ser Gly Ile Ser Asp Leu Ala Phe Gln Arg
            580                 585                 590

Ser Phe Thr Glu Ser Asn Gln Tyr Asn Ile Tyr Ala Ala Leu His Thr
        595                 600                 605

Glu Pro Asp Leu Leu Phe Leu Glu Leu Ser Thr Gly Lys Val Gly Met
    610                 615                 620

Leu Lys Asn Leu Lys Glu Pro Pro Ala Gly Pro Ala Gln Pro Trp Gly
625                 630                 635                 640

Gly Thr His Arg Ile Met Arg Asp Ser Gly Leu Phe Gly Gln Tyr Leu
                645                 650                 655

Leu Thr Pro Ala Arg Glu Ser Leu Phe Leu Ile Asn Gly Arg Gln Asn
            660                 665                 670

Thr Leu Arg Cys Glu Val Ser Gly Ile Lys Gly Gly Thr Thr Val Val
        675                 680                 685

Trp Val Gly Glu Val
    690

<210> 49
<211> 773
<212> PRT
<213> Homo sapiens

<400> 49
His Cys Val Thr Ser Arg Glu Thr Gly Gln Ala Glu Cys Ala Cys Met

1               5                   10                  15

Asp Leu Cys Lys Arg His Tyr Lys Pro Val Cys Gly Ser Asp Gly Glu
                20                  25                  30

Phe Tyr Glu Asn His Cys Glu Val His Arg Ala Ala Cys Leu Lys Lys
            35                  40                  45

Gln Lys Ile Thr Ile Val His Asn Glu Asp Cys Phe Phe Lys Gly Asp
    50                  55                  60

Lys Cys Lys Thr Thr Glu Cys Ser Lys Met Lys Asn Met Leu Leu Asp
65                  70                  75                  80

Leu Gln Asn Gln Arg Tyr Ile Met Gln Glu Asn Glu Asn Pro Asn Gly
                85                  90                  95

Asp Asp Ile Ser Arg Lys Lys Leu Leu Val Asp Gln Met Phe Lys Tyr
            100                 105                 110

Phe Asp Ala Asp Ser Asn Asp Leu Val Asp Ile Asn Glu Leu Thr Gln
        115                 120                 125

Val Ile Lys Gln Glu Glu Leu Gly Lys Asp Leu Phe Asp Cys Thr Leu
    130                 135                 140

Tyr Val Leu Leu Lys Tyr Asp Asp Phe Asn Ala Asp Lys His Leu Ala
145                 150                 155                 160

Leu Glu Glu Phe Tyr Arg Ala Phe Gln Val Ile Gln Leu Ser Leu Pro
                165                 170                 175

Glu Asp Gln Lys Leu Ser Ile Thr Ala Ala Thr Val Gly Gln Ser Ala
            180                 185                 190

Val Leu Ser Cys Ala Ile Gln Gly Thr Leu Arg Pro Pro Ile Ile Trp
        195                 200                 205

Lys Arg Asn Asn Ile Ile Leu Asn Asn Leu Gly Leu Glu Asp Ile Asn
    210                 215                 220

Asp Phe Gly Asp Asp Gly Ser Leu Tyr Ile Thr Lys Val Thr Thr Thr
225                 230                 235                 240

His Val Gly Asn Tyr Thr Cys Tyr Ala Asp Gly Tyr Glu Gln Val Tyr
                245                 250                 255

Gln Thr His Ile Phe Gln Val Asn Val Pro Pro Val Ile Arg Val Tyr
            260                 265                 270

Pro Glu Ser Gln Ala Arg Glu Pro Gly Val Thr Ala Ser Leu Arg Cys
        275                 280                 285

His Ala Glu Gly Ile Pro Lys Pro Gln Leu Gly Trp Leu Lys Asn Gly
    290                 295                 300

Ile Asp Ile Thr Pro Lys Leu Ser Lys Gln Leu Thr Leu Gln Ala Asn
305                 310                 315                 320

Gly Ser Glu Val His Ile Ser Asn Val Arg Tyr Glu Asp Thr Gly Ala
                325                 330                 335

Tyr Thr Cys Ile Ala Lys Asn Glu Ala Gly Val Asp Glu Asp Ile Ser
            340                 345                 350

Ser Leu Phe Val Glu Asp Ser Ala Arg Lys Thr Leu Ala Asn Ile Leu
        355                 360                 365

Trp Arg Glu Glu Gly Leu Gly Ile Gly Asn Met Phe Tyr Val Phe Tyr
    370                 375                 380

Glu Asp Gly Ile Lys Val Ile Gln Pro Ile Glu Cys Glu Phe Gln Arg
385                 390                 395                 400

His Ile Lys Pro Ser Glu Lys Leu Leu Gly Phe Gln Asp Glu Val Cys
                405                 410                 415

Pro Ile Ala Glu Gly Asp Glu Val Gln Arg Cys Val Trp Ala Ser Ala
            420                 425                 430

Val Asn Val Lys Asp Lys Phe Ile Tyr Val Ala Gln Pro Thr Leu Asp
        435                 440                 445

Arg Val Leu Ile Val Asp Val Gln Ser Gln Lys Val Val Gln Ala Val
    450                 455                 460

Ser Thr Asp Pro Val Pro Val Lys Leu His Tyr Asp Lys Ser His Asp
465                 470                 475                 480

Gln Val Trp Val Leu Ser Trp Gly Thr Leu Glu Lys Thr Ser Pro Thr
                485                 490                 495

Leu Gln Val Ile Thr Leu Ala Ser Gly Asn Val Pro His His Thr Ile
            500                 505                 510

His Thr Gln Pro Val Gly Lys Gln Phe Asp Arg Val Asp Asp Phe Phe
        515                 520                 525

Ile Pro Thr Thr Thr Leu Ile Ile Thr His Met Arg Phe Gly Phe Ile
    530                 535                 540

Leu His Lys Asp Glu Ala Ala Leu Gln Lys Ile Asp Leu Glu Thr Met
545                 550                 555                 560

Ser Tyr Ile Lys Thr Ile Asn Leu Lys Asp Tyr Lys Cys Val Pro Gln
                565                 570                 575

Ser Leu Ala Tyr Thr His Leu Gly Gly Tyr Tyr Phe Ile Gly Cys Lys
            580                 585                 590

Pro Asp Ser Thr Gly Ala Val Ser Pro Gln Val Met Val Asp Gly Val
        595                 600                 605

Thr Asp Ser Val Ile Gly Phe Asn Ser Asp Val Thr Gly Thr Pro Tyr
    610                 615                 620

Val Ser Pro Asp Gly His Tyr Leu Val Ser Ile Asn Asp Val Lys Gly
625                 630                 635                 640

Leu Val Arg Val Gln Tyr Ile Thr Ile Arg Gly Glu Ile Gln Glu Ala
                645                 650                 655

Phe Asp Ile Tyr Thr Asn Leu His Ile Ser Asp Leu Ala Phe Gln Pro
            660                 665                 670

Ser Phe Thr Glu Ala His Gln Tyr Asn Ile Tyr Gly Ser Ser Ser Thr
        675                 680                 685

Gln Thr Asp Val Leu Phe Val Glu Leu Ser Ser Gly Lys Val Lys Met
    690                 695                 700

Ile Lys Ser Leu Lys Glu Pro Leu Lys Ala Glu Glu Trp Pro Trp Asn
705                 710                 715                 720

Arg Lys Asn Arg Gln Ile Gln Asp Ser Gly Leu Phe Gly Gln Tyr Leu
                725                 730                 735

Met Thr Pro Ser Lys Asp Ser Leu Phe Ile Leu Asp Gly Arg Leu Asn
            740                 745                 750

Lys Leu Asn Cys Glu Ile Thr Glu Val Glu Lys Gly Asn Thr Val Ile
        755                 760                 765

Trp Val Gly Asp Ala
    770


<210> 50
<211> 306
<212> PRT
<213> Rattus norvegicus

<400> 50
Met Trp Lys Arg Trp Leu Ala Leu Ala Leu Val Thr Ile Ala Leu Val
1               5                   10                  15

His Gly Glu Glu Glu Gln Arg Ser Lys Ser Lys Ile Cys Ala Asn Val
            20                  25                  30

Phe Cys Gly Ala Gly Arg Glu Cys Ala Val Thr Glu Lys Gly Glu Pro
        35                  40                  45

Thr Cys Leu Cys Ile Glu Gln Cys Lys Pro His Lys Arg Pro Val Cys
    50                  55                  60

Gly Ser Asn Gly Lys Thr Tyr Leu Asn His Cys Glu Leu His Arg Asp
65                  70                  75                  80

Ala Cys Leu Thr Gly Ser Lys Ile Gln Val Asp Tyr Asp Gly His Cys
                85                  90                  95

Lys Glu Lys Lys Ser Val Ser Pro Ser Ala Ser Pro Val Val Cys Tyr
            100                 105                 110

Gln Ala Asn Arg Asp Glu Leu Arg Arg Arg Ile Ile Gln Trp Leu Glu
        115                 120                 125

Ala Glu Ile Ile Pro Asp Gly Trp Phe Ser Lys Gly Ser Asn Tyr Ser
    130                 135                 140

Glu Ile Leu Asp Lys Tyr Phe Lys Ser Phe Asp Asn Gly Asp Ser His
145                 150                 155                 160

Leu Asp Ser Ser Glu Phe Leu Lys Phe Val Glu Gln Asn Glu Thr Ala
                165                 170                 175

Val Asn Ile Thr Ala Tyr Pro Asn Gln Glu Asn Asn Lys Leu Leu Arg
            180                 185                 190

Gly Leu Cys Val Asp Ala Leu Ile Glu Leu Ser Asp Glu Asn Ala Asp
        195                 200                 205

Trp Lys Leu Ser Phe Gln Glu Phe Leu Lys Cys Leu Asn Pro Ser Phe
    210                 215                 220

Asn Pro Pro Glu Lys Lys Cys Ala Leu Glu Asp Glu Thr Tyr Ala Asp
225                 230                 235                 240

Gly Ala Glu Thr Glu Val Asp Cys Asn Arg Cys Val Cys Ser Cys Gly
                245                 250                 255

His Trp Val Cys Thr Ala Met Thr Cys Asp Gly Lys Asn Gln Lys Gly
            260                 265                 270

Val Gln Thr His Thr Glu Glu Glu Met Thr Arg Tyr Ala Gln Glu Leu
        275                 280                 285

Gln Lys His Gln Gly Thr Ala Glu Lys Thr Lys Lys Val Asn Thr Lys
    290                 295                 300

Glu Ile
305


<210> 51
<211> 306
<212> PRT
<213> Mus musculus

<400> 51
Met Trp Lys Arg Trp Leu Ala Leu Ser Leu Val Thr Ile Ala Leu Val
1               5                   10                  15

His Gly Glu Glu Glu Pro Arg Ser Lys Ser Lys Ile Cys Ala Asn Val
            20                  25                  30

Phe Cys Gly Ala Gly Arg Glu Cys Ala Val Thr Glu Lys Gly Glu Pro
        35                  40                  45

Thr Cys Leu Cys Ile Glu Gln Cys Lys Pro His Lys Arg Pro Val Cys
    50                  55                  60

Gly Ser Asn Gly Lys Thr Tyr Leu Asn His Cys Glu Leu His Arg Asp
65                  70                  75                  80

Ala Cys Leu Thr Gly Ser Lys Ile Gln Val Asp Tyr Asp Gly His Cys
                85                  90                  95

Lys Glu Lys Lys Ser Ala Ser Pro Ser Ala Ser Pro Val Val Cys Tyr
            100                 105                 110

Gln Ala Asn Arg Asp Glu Leu Arg Arg Arg Leu Ile Gln Trp Leu Glu
        115                 120                 125

Ala Glu Ile Ile Pro Asp Gly Trp Phe Ser Lys Gly Ser Asn Tyr Ser
    130                 135                 140

Glu Ile Leu Asp Lys Tyr Phe Lys Ser Phe Asp Asn Gly Asp Ser His
145                 150                 155                 160

Leu Asp Ser Ser Glu Phe Leu Lys Phe Val Glu Gln Asn Glu Thr Ala
                165                 170                 175

Ile Asn Ile Thr Thr Tyr Ala Asp Gln Glu Asn Asn Lys Leu Leu Arg
            180                 185                 190

Ser Leu Cys Val Asp Ala Leu Ile Glu Leu Ser Asp Glu Asn Ala Asp
        195                 200                 205

Trp Lys Leu Ser Phe Gln Glu Phe Leu Lys Cys Leu Asn Pro Ser Phe
    210                 215                 220

Asn Pro Pro Glu Lys Lys Cys Ala Leu Glu Val Glu Thr Tyr Ala Asp
225                 230                 235                 240

Gly Ala Glu Thr Glu Val Asp Cys Asn Arg Cys Val Cys Ser Cys Gly
                245                 250                 255

His Trp Val Cys Thr Ala Met Thr Cys Asp Gly Lys Asn Gln Lys Gly
            260                 265                 270

Val Gln Thr His Thr Glu Glu Glu Lys Thr Gly Tyr Val Gln Glu Leu
        275                 280                 285

Gln Lys His Gln Gly Thr Ala Glu Lys Thr Lys Lys Val Asn Thr Lys
    290                 295                 300

Glu Ile
305

<210> 52
<211> 299
<212> PRT
<213> Xenopus laevis

<400> 52
Met Tyr Leu Arg Cys Val Pro Leu Leu Ala Leu Leu Val Leu Cys Ser
1               5                   10                  15

Ala Leu Glu Glu Pro Lys Ser Lys Ser Lys Val Cys Ala Asn Val Phe
            20                  25                  30

Cys Gly Ala Gly Arg Glu Cys Ala Val Thr Glu Lys Gly Asp Pro Thr
        35                  40                  45

Cys Asp Cys Ile Glu Lys Cys Lys Ser His Lys Arg Pro Val Cys Gly
    50                  55                  60

Ser Asn Gly Lys Thr Tyr Leu Asn His Cys Glu Leu His Arg Asp Ala
65                  70                  75                  80

Cys Leu Thr Gly Ser Lys Ile Gln Val Asp Tyr Asp Gly His Cys Lys
                85                  90                  95

Glu Lys Thr Ser Asp Thr Pro Ala Ala Val Pro Val Ala Cys Tyr Gln
            100                 105                 110

Ser Asp Arg Asp Glu Met Arg Arg Arg Val Ile His Trp Leu Gln Thr
        115                 120                 125

Glu Ile Thr Pro Asp Gly Trp Phe Ser Lys Gly Ser Asp Tyr Ser Glu
    130                 135                 140

Ile Leu Asp Arg Tyr Phe Lys Lys Phe Asp Asp Gly Asp Ser His Leu
145                 150                 155                 160

Asp Ser Ala Glu Leu Gln Ser Phe Leu Glu Gln Ser Gln Ser Thr Asn
                165                 170                 175

Ile Thr Thr Tyr Lys Asp Glu Glu Thr Asn Arg Met Leu Lys Ser Leu
            180                 185                 190

Cys Val Glu Ala Leu Ile Glu Leu Ser Asp Glu Asn Ala Asp Trp Lys
        195                 200                 205

Leu Asn Lys Asn Glu Phe Leu Lys Cys Leu Asn Pro Asp Phe Gln Pro
    210                 215                 220

Ser Glu Lys Lys Cys Ala Leu Glu Asp Glu Thr Tyr Glu Asp Gly Ala
225                 230                 235                 240

Glu Thr Gln Val Gln Cys Asn Arg Cys Val Cys Ala Cys Gly Asn Trp
                245                 250                 255

Val Cys Thr Ala Met Ala Cys Glu Gly Lys Asp Gly Asp His Gly Glu
            260                 265                 270

Asp Met Gly Arg Tyr Val Glu Glu Ile Arg Lys Gln Gln Glu Thr Ile
        275                 280                 285

Glu Asn Ser Lys Ser Ser Ser Asp Lys Asp Ala
    290                 295


<210> 53
<211> 308
<212> PRT
<213> Homo sapiens

<400> 53
Met Trp Lys Arg Trp Leu Ala Leu Ala Leu Ala Leu Val Ala Val Ala
1               5                   10                  15

Trp Val Arg Ala Glu Glu Glu Leu Arg Ser Lys Ser Lys Ile Cys Ala
            20                  25                  30

Asn Val Phe Cys Gly Ala Gly Arg Glu Cys Ala Val Thr Glu Lys Gly
        35                  40                  45

Glu Pro Thr Cys Leu Cys Ile Glu Gln Cys Lys Pro His Lys Arg Pro
    50                  55                  60

Val Cys Gly Ser Asn Gly Lys Thr Tyr Leu Asn His Cys Glu Leu His
65                  70                  75                  80

Arg Asp Ala Cys Leu Thr Gly Ser Lys Ile Gln Val Asp Tyr Asp Gly
                85                  90                  95

His Cys Lys Glu Lys Lys Ser Val Ser Pro Ser Ala Ser Pro Val Val
            100                 105                 110

Cys Tyr Gln Ser Asn Arg Asp Glu Leu Arg Arg Arg Ile Ile Gln Trp
        115                 120                 125

Leu Glu Ala Glu Ile Ile Pro Asp Gly Trp Phe Ser Lys Gly Ser Asn
    130                 135                 140

Tyr Ser Glu Ile Leu Asp Lys Tyr Phe Lys Asn Phe Asp Asn Gly Asp
145                 150                 155                 160

Ser Arg Leu Asp Ser Ser Glu Phe Leu Lys Phe Val Glu Gln Asn Glu
                165                 170                 175

Thr Ala Ile Asn Ile Thr Thr Tyr Pro Asp Gln Glu Asn Asn Lys Leu
            180                 185                 190

Leu Arg Gly Leu Cys Val Asp Ala Leu Ile Glu Leu Ser Asp Glu Asn
        195                 200                 205

Ala Asp Trp Lys Leu Ser Phe Gln Glu Phe Leu Lys Cys Leu Asn Pro
    210                 215                 220

Ser Phe Asn Pro Pro Glu Lys Lys Cys Ala Leu Glu Asp Glu Thr Tyr
225                 230                 235                 240

Ala Asp Gly Ala Glu Thr Glu Val Asp Cys Asn Arg Cys Val Cys Ala
                245                 250                 255

Cys Gly Asn Trp Val Cys Thr Ala Met Thr Cys Asp Gly Lys Asn Gln
            260                 265                 270

Lys Gly Ala Gln Thr Gln Thr Glu Glu Glu Met Thr Arg Tyr Val Gln
        275                 280                 285

Glu Leu Gln Lys His Gln Glu Thr Ala Glu Lys Thr Lys Arg Val Ser
    290                 295                 300

Thr Lys Glu Ile
305


<210> 54
<211> 315
<212> PRT
<213> Gallus gallus

<400> 54
Met Ile Trp Lys Thr Leu Pro Leu Leu Cys Ala Leu Leu Ala Val Ala
  1               5                  10                  15

Arg Leu Arg Ala Glu Glu Glu Pro Arg Ser Lys Ser Lys Ile Cys Ala
            20                  25                  30

Asn Val Phe Cys Gly Arg Gly Ala Glu Cys Ala Val Thr Glu Lys Gly
        35                  40                  45

Glu Pro Thr Cys Leu Cys Ile Glu Gln Cys Lys Pro His Gly Arg Pro
    50                  55                  60

Val Cys Gly Ser Asn Gly Lys Thr Tyr Leu Asn His Cys Glu Leu His
65                  70                  75                  80

Arg Asp Ala Cys Leu Thr Gly Ser Lys Ile Gln Val Asp Tyr Asp Gly
                85                  90                  95

His Cys Lys Glu Lys Lys Ser Glu Asn Pro Ala Ala Ser Pro Val Val
            100                 105                 110

Cys Tyr Gln Ser Asp Arg Asp Glu Leu His Arg Arg Val Ile Arg Trp
        115                 120                 125

Leu Glu Gly Glu Ile Ile Pro Asp Gly Trp Phe Ser Lys Gly Ser Asn
    130                 135                 140

Tyr Ser Asp Val Leu Glu Lys Tyr Phe Lys Asn Phe Asp Asp Asp Ser
145                 150                 155                 160

Arg Leu Asp Ser Thr Glu Phe Leu Lys Phe Val Glu Gln Asn Glu Thr
                165                 170                 175

Ala Val Pro Thr Ile Thr Thr Tyr Val Asp Gln Glu Thr Asn Lys Leu
            180                 185                 190

Leu Arg Gly Leu Cys Val Val Ala Leu Ile Glu Leu Ser Val Gln Asn
        195                 200                 205

Ala Asp Trp Lys Leu Ser Phe Asn Glu Phe Leu Lys Cys Leu Ser Pro
    210                 215                 220

Ser Phe Asn Pro Pro Glu Lys Lys Cys Ala Leu Glu Asp Glu Thr Tyr
225                 230                 235                 240

Glu Asp Gly Ala Glu Thr Gln Val Glu Cys Asn Arg Cys Val Tyr Ala
                245                 250                 255

Cys Gly Asn Trp Val Cys Thr Ala Met Thr Cys Glu Gly Lys Asn Glu
            260                 265                 270

Lys Met Thr Ala His Arg Gln Gln Pro Gly Gln Asp Leu Thr Glu Glu
        275                 280                 285

Glu Leu Ala Arg Tyr Val Gln Glu Leu Gln Lys His Gln Glu Gln Ala
    290                 295                 300

Glu Lys Ile Lys Lys Met Ser Thr Lys Glu Met
305                 310                 315


<210> 55
<211> 1375
<212> PRT
<213> Drosophila melanogaster

<400> 55
   Met Ala Ile Thr Thr Asn Arg Ser Ser Arg Thr Leu Trp Asn Trp Leu
   1               5                   10                  15
   Leu Ser Ser Cys Leu Ile Phe Gln Leu Ile Gly Ser Ser Leu Ala Ser
               20                  25                  30
   Gln Ala Leu Ser Phe Thr Leu Glu Pro Gln Asp Ala Val Val Pro Glu
           35                  40                  45
   Gly His Ser Val Leu Leu Gln Cys Ala Gly Thr Ala Ser Ile Gly Arg
       50                  55                  60
   Gly Gly Lys Ser Lys Ser Asn Leu Pro Ser Ser Val Ser Ile Arg Trp
   65                  70                  75                  80
   Arg Gly Pro Asp Gly Gln Asp Leu Val Ile Val Gly Asp Thr Phe Arg
                   85                  90                  95
   Thr Gln Leu Lys Asn Gly Ser Leu Tyr Ile Ser Ser Val Glu Glu Asn
               100                 105                 110
   Arg Gly Leu Thr Gly Ala Tyr Gln Cys Leu Leu Thr Ala Glu Gly Val
           115                 120                 125
   Gly Ser Ile Leu Ser Arg Pro Ala Leu Val Ala Ile Val Arg Gln Pro
       130                 135                 140
   Asp Leu Asn Gln Asp Phe Leu Glu Thr Tyr Leu Leu Pro Gly Gln Thr
   145                 150                 155                 160
   Ala Tyr Phe Arg Cys Met Leu Gly Glu Ala Asn Trp Gln Glu Gly Val
                   165                 170                 175
   Lys His Ser Val Gln Trp Leu Lys Asp Asp Leu Pro Leu Pro Leu Asp
               180                 185                 190
   Lys Leu Arg Met Val Val Leu Pro Asn Gly Ala Leu Glu Ile Asp Glu
           195                 200                 205
   Val Gly Pro Ser Asp Arg Gly Ser Tyr Gln Cys Asn Val Thr Ser Gly
       210                 215                 220
   Ser Ser Ser Arg Leu Ser Ser Lys Thr Asn Leu Asn Ile Lys Lys Pro
   225                 230                 235                 240
   Ser Asp Pro Gly Val Glu Asn Ser Val Ala Pro Ser Phe Leu Val Gly
                   245                 250                 255
   Pro Ser Pro Lys Thr Val Arg Glu Gly Asp Thr Val Thr Leu Asp Cys
               260                 265                 270
   Val Ala Asn Gly Val Pro Lys Pro Gln Ile Lys Trp Leu Arg Asn Gly
           275                 280                 285
   Met Asp Leu Asp Phe Asn Asp Leu Asp Ser Arg Phe Ser Ile Val Gly
       290                 295                 300
   Thr Gly Ser Leu Gln Ile Ser Ser Ala Glu Asp Ile Asp Ser Gly Asn
   305                 310                 315                 320
   Tyr Gln Cys Arg Ala Ser Asn Thr Val Asp Ser Leu Asp Ala Gln Ala
                   325                 330                 335
   Thr Val Gln Val Gln Glu Pro Pro Lys Phe Ile Lys Ala Pro Lys Asp
   340                 345                 350
   Thr Thr Ala His Glu Lys Asp Glu Pro Glu Leu Lys Cys Asp Ile Trp
           355                 360                 365
   Gly Lys Pro Lys Pro Val Ile Arg Trp Leu Lys Asn Gly Asp Leu Ile
       370                 375                 380
   Thr Pro Asn Asp Tyr Met Gln Leu Val Asp Gly His Asn Leu Lys Ile
   385                 390                 395                 400
   Leu Gly Leu Leu Asn Ser Asp Ala Gly Met Phe Gln Cys Val Gly Thr
                   405                 410                 415
   Asn Ala Ala Gly Ser Val His Ala Ala Ala Arg Leu Arg Val Val Pro
               420                 425                 430
   Gln Gly Asp Ser Pro Glu Gln Asp Pro Ser Val Pro His Pro Gly Gly
           435                 440                 445
   Lys Pro Leu Asp Ser Gly Leu Gln Ala Arg Leu Pro Ser Gln Pro Arg
       450                 455                 460
   Asp Leu Val Ala Gln Ile Val Lys Ser Arg Phe Val Thr Leu Ser Trp
   465                 470                 475                 480
   Val Glu Pro Leu Gln Asn Ala Gly Asp Val Val Tyr Tyr Thr Val Tyr
                   485                 490                 495
   Tyr Lys Met Asn Asn Ser Glu Arg Glu Gln Lys Met Val Thr Lys Ser
               500                 505                 510
   His Asp Asp Gln Gln Val Asn Ile Gln Ser Leu Leu Pro Gly Arg Thr
           515                 520                 525
   Tyr Gln Phe Arg Val Glu Ala Asn Thr Asn Phe Gly Ser Gly Ala Ser
       530                 535                 540
   Ser Ala Pro Leu Glu Val Ser Thr Gln Pro Glu Val Asn Ile Ala Gly
   545                 550                 555                 560
   Pro Pro Arg Asn Phe Glu Gly Tyr Ala Arg Ser His Lys Glu Ile Tyr
                   565                 570                 575
   Val Lys Trp Glu Glu Pro Thr Val Thr Asn Gly Glu Ile Leu Lys Tyr
               580                 585                 590
   Arg Val Tyr Tyr Ser Glu Asn Asp Ser Gly Ala Asp Leu Tyr His Asp
           595                 600                 605
   Ser Thr Ala Leu Glu Ala Val Leu Thr Glu Leu Arg Pro His Thr Asp
       610                 615                 620
   Tyr Val Ile Ser Val Val Pro Phe Asn Arg Asn Gly Met Gly Asp Ser
   625                 630                 635                 640
   Ser Ala Glu Ile Arg Val Lys Thr Phe Ser Ser Thr Pro Ser Glu Pro
                   645                 650                 655
   Pro Asn Asn Val Thr Leu Glu Val Thr Ser Ser Ser Ser Ile Thr Val
               660                 665                 670
   His Trp Glu Pro Pro Ala Glu Glu Asp Arg Asn Gly Gln Ile Thr Gly
           675                 680                 685
   Tyr Lys Ile Arg Tyr Arg Lys Phe Lys Asp Ala Pro Gln Val Lys Ser
       690                 695                 700
   Thr Pro Ala Asn Ile Arg Tyr Phe Glu Leu Ser Asn Leu Asp Arg Asn
   705                 710                 715                 720
   Ala Glu Tyr Gln Val Lys Ile Ala Ala Met Thr Val Asn Gly Ser Gly
                   725                 730                 735
   Pro Phe Thr Glu Trp Asn Arg Ala Asn Thr Leu Glu Asn Asp Leu Asp
           740                 745                 750
   Glu Thr Gln Val Pro Gly Lys Pro Ile Trp Ile Ser Ile His Pro Gly
           755                 760                 765
   Ala Asn Asn Ile Ala Leu His Trp Gly Pro Pro Gln His Pro Glu Ile
       770                 775                 780
   Lys Ile Arg Asn Tyr Val Leu Gly Trp Gly Arg Gly Ile Pro Asp Glu
   785                 790                 795                 800
   Asn Thr Ile Glu Leu Lys Glu Thr Glu Arg Tyr His Ile Leu Lys Asn
                   805                 810                 815
   Leu Glu Ser Asn Met Asp Tyr Val Val Ser Leu Arg Ala Arg Asn Val
               820                 825                 830
   Lys Gly Asp Gly Pro Pro Ile Tyr Asp Asn Ile Lys Thr Arg Asp Glu
           835                 840                 845
   Glu Pro Val Asp Ala Pro Thr Pro Leu Glu Val Pro Val Gly Leu Arg
       850                 855                 860
   Ala Ile Thr Met Ser Ser Ser Ser Ile Val Val Tyr Trp Ile Asp Thr
   865                 870                 875                 880
   Met Leu Asn Lys Asn Gln His Val Thr Asp Asn Arg His Tyr Thr Val
                   885                 890                 895
   Ser Tyr Gly Ile Thr Gly Ser Asn Arg Tyr Arg Tyr His Asn Thr Thr
               900                 905                 910
   Asp Leu Asn Cys Met Ile Asn Asp Leu Arg Pro Asn Thr Gln Tyr Glu
           915                 920                 925
   Phe Ala Val Lys Val Val Lys Gly Arg Arg Glu Ser Ser Trp Ser Met
       930                 935                 940
   Ser Val Leu Asn Ser Thr Tyr Gln Asn Val Pro Val Thr Pro Pro Arg
   945                 950                 955                 960
   Glu Val Thr Val Arg Leu Asp Glu Met Asn Pro Pro Thr Val Ile Val
                   965                 970                 975
   Gln Trp Ile Pro Pro Lys His Thr Leu Gly Gln Ile Thr Gly Tyr Asn
               980                 985                 990
   Ile Tyr Tyr Thr Thr Asp Thr Thr Lys Arg Asp Arg Asp Trp Ser Val
           995                 1000                1005
   Glu Ala Phe Ala Gly Glu Glu Thr Met Leu Met Leu Pro Asn Leu Lys
      1010                1015                1020
   Pro Tyr Thr Thr Tyr Tyr Phe Lys Val Gln Ala Arg Thr Thr Lys Gly
   1025                1030                1035                1040
   Ala Asn Asn Ala Pro Phe Ser Ala Leu Val Ser Tyr Thr Thr Ser Ala
                  1045                1050                1055
   Ala Val Thr Met Gln Glu Pro Asp Thr Ile Ala Lys Gly Ile Asp Asn
              1060                1065                1070
   Glu Lys Leu Leu Tyr Ile Ile Ile Ala Ala Thr Ala Val Val Leu Leu
          1075                1080                1085
   Val Val Leu Leu Gly Val Leu Leu Leu Cys Arg Arg Lys Pro Gln Ser
      1090                1095                1100
   Ser Pro Glu His Thr Lys Lys Ser Tyr Gln Lys Asn Asn Val Gly Val
   1105                1110                1115                1120
   Pro Lys Pro Pro Asp Leu Trp Ile His His Asp Gln Met Glu Leu Lys
                  1125                1130                1135
   Asn Ile Asp Lys Gly Leu His Thr Val Thr Pro Val Cys Ser Asp Gly
              1140                1145                1150
   Ala Ser Ser Ser Gly Ala Leu Thr Leu Pro Arg Ser Val Val His Ser
          1155                1160                1165
   Glu Tyr Glu Val Glu Thr Pro Val Pro Gly His Val Thr Asn Ser Leu
      1170                1175                1180
   Asp Lys Arg Ser Tyr Val Pro Gly Tyr Met Thr Thr Ser Met Asn Gly
   1185                1190                1195                1200
   Thr Met Glu Arg Pro Gln Tyr Pro Arg Thr Gln Tyr Ser His Gln Asn
                  1205                1210                1215
   Arg Ser His Met Thr Met Glu Ala Gly Leu Ser Gln Gln Ser Leu Thr
              1220                1225                1230
   Gln Pro Gln Ser Asn Ser Met Ala Gln Thr Pro Glu His Pro Tyr Gly
          1235                1240                1245
   Gly Tyr Asp Ala Asn Phe Cys Asn Ala Gly Asn Ala Ala Ala Gly Asn
      1250                1255                1260
   Gly Cys Val Ser Thr Ile Glu Ser Ser Lys Arg Gly His Pro Leu Lys
   1265                1270                1275                1280
   Ser Phe Ser Val Pro Gly Pro Pro Pro Thr Gly Gly Ala Thr Pro Val
                  1285                1290                1295
   Thr Lys His Thr Pro Ala Val Thr Ile Arg Pro Gln Asn Gln Ser Pro
              1300                1305                1310
   Tyr Lys Lys Pro Ser Phe Ser Ala Ala Thr Pro Asn Arg Leu Gln Gly
   1315                1320                1325
   Gly Gly Ser Val Val His Ser Thr Asp Glu Ile Gln Arg Leu Ala Pro
       1330                1335                1340
   Ser Thr Ser Thr Glu Glu Leu Asn Gln Glu Met Ala Asn Leu Glu Gly
   1345                1350                1355                1360
   Leu Met Lys Asp Leu Ser Ala Ile Thr Ala Asn Glu Phe Glu Cys
                   1365                1370                1375


<210> 56
<211> 1395
<212> PRT
<213> Drosophila melanogaster

<400> 56
Met His Pro Met His Pro Glu Asn His Ala Ile Ala Arg Ser Thr Ser
1               5                   10                  15

Thr Thr Asn Asn Pro Ser Arg Ser Arg Ser Ser Arg Met Trp Leu Leu
            20                  25                  30

Pro Ala Trp Leu Leu Leu Val Leu Val Ala Ser Asn Gly Leu Pro Ala
        35                  40                  45

Val Arg Gly Gln Tyr Gln Ser Pro Arg Ile Ile Glu His Pro Thr Asp
    50                  55                  60

Leu Val Val Lys Lys Asn Glu Pro Ala Thr Leu Asn Cys Lys Val Glu
65                  70                  75                  80

Gly Lys Pro Glu Pro Thr Ile Glu Trp Phe Lys Asp Gly Glu Pro Val
                85                  90                  95

Ser Thr Asn Glu Lys Lys Ser His Arg Val Gln Phe Lys Asp Gly Ala
            100                 105                 110

Leu Phe Phe Tyr Arg Thr Met Gln Gly Lys Lys Glu Gln Asp Gly Gly
        115                 120                 125

Glu Tyr Trp Cys Val Ala Lys Asn Arg Val Gly Gln Ala Val Ser Arg
    130                 135                 140

His Ala Ser Leu Gln Ile Ala Val Leu Arg Asp Asp Phe Arg Val Glu
145                 150                 155                 160

Pro Lys Asp Thr Arg Val Ala Lys Gly Glu Thr Ala Leu Leu Glu Cys
                165                 170                 175

Gly Pro Pro Lys Gly Ile Pro Glu Pro Thr Leu Ile Trp Ile Lys Asp
            180                 185                 190

Gly Val Pro Leu Asp Asp Leu Lys Ala Met Ser Phe Gly Ala Ser Ser
        195                 200                 205

Arg Val Arg Ile Val Asp Gly Gly Asn Leu Leu Ile Ser Asn Val Glu
    210                 215                 220

Pro Ile Asp Glu Gly Asn Tyr Lys Cys Ile Ala Gln Asn Leu Val Gly

225                 230                 235                 240

Thr Arg Glu Ser Ser Tyr Ala Lys Leu Ile Val Gln Val Lys Pro Tyr
                245                 250                 255

Phe Met Lys Glu Pro Lys Asp Gln Val Met Leu Tyr Gly Gln Thr Ala
            260                 265                 270

Thr Phe His Cys Ser Val Gly Gly Asp Pro Pro Pro Lys Val Leu Trp
        275                 280                 285

Lys Lys Glu Glu Gly Asn Ile Pro Val Ser Arg Ala Arg Ile Leu His
    290                 295                 300

Asp Glu Lys Ser Leu Glu Ile Ser Asn Ile Thr Pro Thr Asp Glu Gly
305                 310                 315                 320

Thr Tyr Val Cys Glu Ala His Asn Asn Val Gly Gln Ile Ser Ala Arg
                325                 330                 335

Ala Ser Leu Ile Val His Ala Pro Pro Asn Phe Thr Lys Arg Pro Ser
            340                 345                 350

Asn Lys Lys Val Gly Leu Asn Gly Val Val Gln Leu Pro Cys Met Ala
        355                 360                 365

Ser Gly Asn Pro Pro Pro Ser Val Phe Trp Thr Lys Glu Gly Val Ser
    370                 375                 380

Thr Leu Met Phe Pro Asn Ser Ser His Gly Arg Gln Tyr Val Ala Ala
385                 390                 395                 400

Asp Gly Thr Leu Gln Ile Thr Asp Val Arg Gln Glu Asp Glu Gly Tyr
                405                 410                 415

Tyr Val Cys Ser Ala Phe Ser Val Val Asp Ser Ser Thr Val Arg Val
            420                 425                 430

Phe Leu Gln Val Ser Ser Val Asp Glu Arg Pro Pro Pro Ile Ile Gln
        435                 440                 445

Ile Gly Pro Ala Asn Gln Thr Leu Pro Lys Gly Ser Val Ala Thr Leu
    450                 455                 460

Pro Cys Arg Ala Thr Gly Asn Pro Ser Pro Arg Ile Lys Trp Phe His
465                 470                 475                 480

Asp Gly His Ala Val Gln Ala Gly Asn Arg Tyr Ser Ile Ile Gln Gly
                485                 490                 495

Ser Ser Leu Arg Val Asp Asp Leu Gln Leu Ser Asp Ser Gly Thr Tyr
            500                 505                 510

Thr Cys Thr Ala Ser Gly Glu Arg Gly Glu Thr Ser Trp Ala Ala Thr
        515                 520                 525

Leu Thr Val Glu Lys Pro Gly Ser Thr Ser Leu His Arg Ala Ala Asp
    530                 535                 540

Pro Ser Thr Tyr Pro Ala Pro Pro Gly Thr Pro Lys Val Leu Asn Val
545                 550                 555                 560

Ser Arg Thr Ser Ile Ser Leu Arg Trp Ala Lys Ser Gln Glu Lys Pro
                565                 570                 575

Gly Ala Val Gly Pro Ile Ile Gly Tyr Thr Val Glu Tyr Phe Ser Pro
            580                 585                 590

Asp Leu Gln Thr Gly Trp Ile Val Ala Ala His Arg Val Gly Asp Thr
        595                 600                 605

Gln Val Thr Ile Ser Gly Leu Thr Pro Gly Thr Ser Tyr Val Phe Leu
    610                 615                 620

Val Arg Ala Glu Asn Thr Gln Gly Ile Ser Val Pro Ser Gly Leu Ser
625                 630                 635                 640

Asn Val Ile Lys Thr Ile Glu Ala Asp Phe Asp Ala Ala Ser Ala Asn
                645                 650                 655

Asp Leu Ser Ala Ala Arg Thr Leu Leu Thr Gly Lys Ser Val Glu Leu
            660                 665                 670

Ile Asp Ala Ser Ala Ile Asn Ala Ser Ala Val Arg Leu Glu Trp Met
        675                 680                 685

Leu His Val Ser Ala Asp Glu Lys Tyr Val Glu Gly Leu Arg Ile His
    690                 695                 700

Tyr Lys Asp Ala Ser Val Pro Ser Ala Gln Tyr His Ser Ile Thr Val
705                 710                 715                 720

Met Asp Ala Ser Ala Glu Ser Phe Val Val Gly Asn Leu Lys Lys Tyr
                725                 730                 735

Thr Lys Tyr Glu Phe Phe Leu Thr Pro Phe Phe Glu Thr Ile Glu Gly
            740                 745                 750

Gln Pro Ser Asn Ser Lys Thr Ala Leu Thr Tyr Glu Asp Val Pro Ser
        755                 760                 765

Ala Pro Pro Asp Asn Ile Gln Ile Gly Met Tyr Asn Gln Thr Ala Gly
    770                 775                 780

Trp Val Arg Trp Thr Pro Pro Pro Ser Gln His His Asn Gly Asn Leu
785                 790                 795                 800

Tyr Gly Tyr Lys Ile Glu Val Ser Ala Gly Asn Thr Met Lys Val Leu
                805                 810                 815

Ala Asn Met Thr Leu Asn Ala Thr Thr Thr Ser Val Leu Leu Asn Asn
            820                 825                 830

Leu Thr Thr Gly Ala Val Tyr Ser Val Arg Leu Asn Ser Phe Thr Lys
        835                 840                 845

Ala Gly Asp Gly Pro Tyr Ser Lys Pro Ile Ser Leu Phe Met Asp Pro
    850                 855                 860

Thr His His Val His Pro Pro Arg Ala His Pro Ser Gly Thr His Asp
865                 870                 875                 880

Gly Arg His Glu Gly Gln Asp Leu Thr Tyr His Asn Asn Gly Asn Ile
                885                 890                 895

Pro Pro Gly Asp Ile Asn Pro Thr Thr His Lys Lys Thr Thr Asp Tyr
            900                 905                 910

Leu Ser Gly Pro Trp Leu Met Val Leu Val Cys Ile Val Leu Leu Val
        915                 920                 925

Leu Val Ile Ser Ala Ala Ile Ser Met Val Tyr Phe Lys Arg Lys His
    930                 935                 940

Gln Met Thr Lys Glu Leu Gly His Leu Ser Val Val Ser Asp Asn Glu
945                 950                 955                 960

Ile Thr Ala Leu Asn Ile Asn Ser Lys Glu Ser Leu Trp Ile Asp His
                965                 970                 975

His Arg Gly Trp Arg Thr Ala Asp Thr Asp Lys Asp Ser Gly Leu Ser
            980                 985                 990

Glu Ser Lys Leu Leu Ser His Val Asn Ser Ser Gln Ser Asn Tyr Asn
        995                 1000                1005

Asn Ser Asp Gly Gly Thr Asp Tyr Ala Glu Val Asp Thr Arg Asn Leu
    1010                1015                1020

Thr Thr Phe Tyr Asn Cys Arg Lys Ser Pro Asp Asn Pro Thr Pro Tyr
1025                1030                1035                1040

Ala Thr Thr Met Ile Ile Gly Thr Ser Ser Ser Glu Thr Cys Thr Lys
                1045                1050                1055

Thr Thr Ser Ile Ser Ala Asp Lys Asp Ser Gly Thr His Ser Pro Tyr
            1060                1065                1070

Ser Asp Ala Phe Ala Gly Gln Val Pro Ala Val Pro Val Val Lys Ser
        1075                1080                1085

Asn Tyr Leu Gln Tyr Pro Val Glu Pro Ile Asn Trp Ser Glu Phe Leu
    1090                1095                1100

Pro Pro Pro Pro Glu His Pro Pro Pro Ser Ser Thr Tyr Gly Tyr Ala
1105                1110                1115                1120

Gln Gly Ser Pro Glu Ser Ser Arg Lys Ser Ser Lys Ser Ala Gly Ser
                1125                1130                1135

Gly Ile Ser Thr Asn Gln Ser Ile Leu Asn Ala Ser Ile His Ser Ser
            1140                1145                1150

Ser Ser Gly Gly Phe Ser Ala Trp Gly Val Ser Pro Gln Tyr Ala Val
        1155                1160                1165

Ala Cys Pro Pro Glu Asn Val Tyr Ser Asn Pro Leu Ser Ala Val Ala
    1170                1175                1180

Gly Gly Thr Gln Asn Arg Tyr Gln Ile Thr Pro Thr Asn Gln His Pro
1185                1190                1195                1200

Pro Gln Leu Pro Ala Tyr Phe Ala Thr Thr Gly Pro Gly Gly Ala Val

1205                1210                1215

Pro Pro Asn His Leu Pro Phe Ala Thr Gln Arg His Ala Ala Ser Glu
        1220                1225                1230

Tyr Gln Ala Gly Leu Asn Ala Ala Arg Cys Ala Gln Ser Arg Ala Cys
    1235                1240                1245

Asn Ser Cys Asp Ala Leu Ala Thr Pro Ser Pro Met Gln Pro Pro Pro
   1250                1255                1260

Pro Val Pro Val Pro Glu Gly Trp Tyr Gln Pro Val His Pro Asn Ser
1265                1270                1275                1280

His Pro Met His Pro Thr Ser Ser Asn His Gln Ile Tyr Gln Cys Ser
                1285                1290                1295

Ser Glu Cys Ser Asp His Ser Arg Ser Ser Gln Ser His Lys Arg Gln
            1300                1305                1310

Leu Gln Leu Glu Glu His Gly Ser Ser Ala Lys Gln Arg Gly Gly His
        1315                1320                1325

His Arg Arg Arg Ala Pro Val Val Gln Pro Cys Met Glu Ser Glu Asn
   1330                1335                1340

Glu Asn Met Leu Ala Glu Tyr Glu Gln Arg Gln Tyr Thr Ser Asp Cys
1345                1350                1355                1360

Cys Asn Ser Ser Arg Glu Gly Asp Thr Cys Ser Cys Ser Glu Gly Ser
                1365                1370                1375

Cys Leu Tyr Ala Glu Ala Gly Glu Pro Ala Pro Arg Gln Met Thr Ala
            1380                1385                1390

Lys Asn Thr
        1395


<210> 57
<211> 2012
<212> PRT
<213> Homo sapiens

<400> 57

<210> 58
<211> 338
<212> PRT

<213> Homo sapiens

<400> 58
Met Val Gly Arg Val Gln Pro Asp Arg Lys Gln Leu Pro Leu Val Leu
1               5                   10                  15

Leu Arg Leu Leu Cys Leu Leu Pro Thr Gly Leu Pro Val Arg Ser Val
            20                  25                  30

Asp Phe Asn Arg Gly Thr Asp Asn Ile Thr Val Arg Gln Gly Asp Thr
        35                  40                  45

Ala Ile Leu Arg Cys Val Leu Glu Asp Lys Asn Ser Lys Val Ala Trp
    50                  55                  60

Leu Asn Arg Ser Gly Ile Ile Phe Ala Gly His Asp Lys Trp Ser Leu
65                  70                  75                  80

Asp Pro Arg Val Glu Leu Glu Lys Arg His Ser Leu Glu Tyr Ser Leu
                85                  90                  95

Arg Ile Gln Lys Val Asp Val Tyr Asp Glu Gly Ser Tyr Thr Cys Ser
            100                 105                 110

Val Gln Thr Gln His Glu Pro Lys Thr Ser Gln Val Tyr Leu Ile Val
        115                 120                 125

Gln Val Pro Pro Lys Ile Ser Asn Ile Ser Ser Asp Val Thr Val Asn
    130                 135                 140

Glu Gly Ser Asn Val Thr Leu Val Cys Met Ala Asn Gly Arg Pro Glu
145                 150                 155                 160

Pro Val Ile Thr Trp Arg His Leu Thr Pro Thr Gly Arg Glu Phe Glu
                165                 170                 175

Gly Glu Glu Glu Tyr Leu Glu Ile Leu Gly Ile Thr Arg Glu Gln Ser
            180                 185                 190

Gly Lys Tyr Glu Cys Lys Ala Ala Asn Glu Val Ser Ser Ala Asp Val
        195                 200                 205

Lys Gln Val Lys Val Thr Val Asn Tyr Pro Pro Thr Ile Thr Glu Ser
    210                 215                 220

Lys Ser Asn Glu Ala Thr Thr Gly Arg Gln Ala Ser Leu Lys Cys Glu
225                 230                 235                 240

Ala Ser Ala Val Pro Ala Pro Asp Phe Glu Trp Tyr Arg Asp Asp Thr
                245                 250                 255

Arg Ile Asn Ser Ala Asn Gly Leu Glu Ile Lys Ser Thr Glu Gly Gln
            260                 265                 270

Ser Ser Leu Thr Val Thr Asn Val Thr Glu Glu His Tyr Gly Asn Tyr
        275                 280                 285

Thr Cys Val Ala Ala Asn Lys Leu Gly Val Thr Asn Ala Ser Leu Val
    290                 295                 300

Leu Phe Arg Pro Gly Ser Val Arg Gly Ile Asn Gly Ser Ile Ser Leu

305                 310                 315                 320

Ala Val Pro Leu Trp Leu Leu Ala Ala Ser Leu Leu Cys Leu Leu Ser
                325                 330                 335

Lys Cys



<210> 59
<211> 793
<212> PRT
<213> Mus musculus

<400> 59
Met Ala Glu Pro Arg Thr Ala Ser Pro Arg Arg Leu Pro Ala Leu Arg
1               5                   10                  15

Arg Pro Gly Phe Leu Pro Pro Leu Leu Pro Pro Pro Pro Pro Pro Leu
            20                  25                  30

Leu Leu Leu Leu Leu Leu Leu Pro Leu Pro Ala Pro Ser Leu Gly Leu
        35                  40                  45

Gly His Ser Ala Glu Leu Ala Phe Ser Val Glu Pro Asn Asp Asp Ile
    50                  55                  60

Ala Asn Pro Gly Gln Pro Ile Val Leu Gly Cys Lys Val Glu Gly Thr
65                  70                  75                  80

Pro Pro Val Gln Val Ser Trp Arg Lys Asn Gly Ala Glu Leu Pro Glu
                85                  90                  95

Gly Thr His Thr Thr Leu Leu Ala Asn Gly Ser Leu Leu Ile His His
            100                 105                 110

Phe Arg Leu Glu Gln Gly Gly Ser Pro Ser Asp Glu Gly Asp Tyr Glu
        115                 120                 125

Cys Val Ala Gln Asn Arg Phe Gly Leu Leu Val Ser Arg Lys Ala Arg
    130                 135                 140

Leu Gln Ala Ala Thr Met Ser Asp Phe His Val His Pro Gln Ala Val
145                 150                 155                 160

Thr Gly Glu Glu Gly Gly Val Ala Arg Phe Gln Cys Gln Ile His Gly
                165                 170                 175

Leu Pro Lys Pro Leu Ile Thr Trp Glu Lys Asn Arg Val Pro Ile Asp
            180                 185                 190

Thr Asp Asp Glu Arg Tyr Thr Leu Leu Pro Lys Gly Val Leu Gln Ile
        195                 200                 205

Thr Gly Leu Arg Ala Glu Asp Ser Gly Ile Phe His Cys Val Ala Ser
    210                 215                 220

Asn Ile Ala Ser Val Arg Val Ser His Gly Ala Arg Leu Thr Val Ser
225                 230                 235                 240

Gly Ser Gly Ser Gly Thr Tyr Lys Glu Pro Thr Ile Leu Val Gly Pro

                245                 250                 255

Glu Asn Leu Thr Leu Thr Val His Gln Thr Ala Val Leu Glu Cys Val
            260                 265                 270

Ala Thr Gly Asn Pro Arg Pro Ile Val Ser Trp Ser Arg Leu Asp Gly
        275                 280                 285

Arg Pro Ile Gly Val Glu Gly Ile Gln Val Leu Gly Thr Gly Asn Leu
    290                 295                 300

Ile Ile Ser Asp Val Thr Val Gln His Ser Gly Val Tyr Val Cys Ala
305                 310                 315                 320

Ala Asn Arg Pro Gly Thr Arg Val Arg Arg Thr Ala Gln Gly Arg Leu
                325                 330                 335

Val Val Gln Ala Pro Ala Glu Phe Val Gln His Pro Gln Ser Ile Ser
            340                 345                 350

Arg Pro Ala Gly Thr Thr Ala Met Phe Thr Cys Gln Ala Gln Gly Glu
        355                 360                 365

Pro Pro Pro His Val Thr Trp Leu Lys Asn Gly Gln Val Leu Gly Ala
    370                 375                 380

Gly Gly His Val Arg Leu Lys Asn Asn Asn Ser Thr Leu Ser Ile Ser
385                 390                 395                 400

Gly Val Gly Pro Glu Asp Glu Ala Ile Tyr Gln Cys Val Ala Glu Asn
                405                 410                 415

Ile Ala Gly Ser Ser Gln Ala Ser Ala Arg Leu Thr Val Leu Trp Ala
            420                 425                 430

Glu Gly Leu Pro Gly Pro Pro Arg Asn Val Arg Ala Val Ser Val Ser
        435                 440                 445

Ser Thr Glu Val Arg Val Ser Trp Ser Glu Pro Leu Ala His Thr Lys
    450                 455                 460

Glu Ile Ile Gly Tyr Val Leu His Ile Arg Lys Ala Ala Asp Ser Pro
465                 470                 475                 480

Lys Leu Glu Tyr Gln Glu Ala Val Ser Lys Ser Thr Phe Gln His Leu
                485                 490                 495

Val Arg Asp Leu Glu Pro Ser Thr Ala Tyr Ser Phe Tyr Ile Lys Ala
            500                 505                 510

Tyr Thr Pro Arg Gly Ala Ser Leu Ala Ser Val Pro Thr Leu Ala Ser
        515                 520                 525

Thr Leu Gly Glu Ala Pro Val Pro Pro Pro Leu Ser Val Arg Leu Leu
    530                 535                 540

Gly Ser Ser Ser Leu Gln Leu Leu Trp Lys Pro Trp Pro Arg Leu Ala
545                 550                 555                 560

Gln His Asn Gly Gly Phe Lys Leu Phe Tyr Arg Pro Val Ser Ala Thr
                565                 570                 575

Ser Phe Thr Gly Pro Ile Leu Leu Pro Gly Thr Val Ser Ser Tyr Asn
                580                 585                 590

Leu Ser Gln Leu Asp Pro Ser Thr Val Tyr Glu Val Lys Leu Leu Ala
        595                 600                 605

Tyr Asn Gln His Gly Asp Gly Asn Ala Thr Val Arg Phe Val Ser Leu
    610                 615                 620

Lys Gly Ala Ser Glu Arg Thr Gly Ile Val Ile Gly Ile His Ile Gly
625                 630                 635                 640

Val Thr Cys Ile Ile Phe Cys Val Leu Phe Leu Leu Phe Gly Gln Arg
                645                 650                 655

Gly Arg Val Leu Leu Cys Lys Asp Val Glu Asn Gln Leu Ser Pro Pro
            660                 665                 670

Gln Gly Pro Arg Ser Gln Arg Asp Pro Gly Ile Leu Ala Leu Asn Gly
        675                 680                 685

Leu Ser Arg Gly Glu Gly Gly Gln Leu Ser Arg Asp Glu Lys Pro Val
    690                 695                 700

Asp Ala Lys Glu Leu Glu Gln Leu Phe Pro Thr Ala Gly Ser Ala Ala
705                 710                 715                 720

Gln Pro Gly Ser Thr Pro Thr Asp Pro Ala Ala Pro Ala Pro Cys Glu
                725                 730                 735

Glu Thr Gln Leu Ser Met Val Gln Leu Gln Gly Phe Asn Leu Val Ala
            740                 745                 750

Gly Arg Thr Thr Glu Ala Thr Ser Pro Cys Ala Gly Pro Gly Pro Val
        755                 760                 765

Pro Ala Pro Gln Asp Ile Gly Pro Val Pro Leu Ser Glu Gly Gln Thr
    770                 775                 780

Gln Pro Pro Ala Val Ala Ala Pro Gln
785                 790


<210> 60
<211> 350
<212> PRT
<213> Gallus gallus

<400> 60
Met Val Ala Arg Ala Gln Pro Asp Arg Lys Gln Leu Pro Leu Val Leu
1               5                   10                  15

Leu Arg Leu Leu Cys Leu Leu Pro Thr Gly Leu Pro Val Arg Ser Val
            20                  25                  30

Asp Phe Thr Arg Gly Thr Asp Asn Ile Thr Val Arg Gln Gly Asp Thr
        35                  40                  45

Ala Ile Leu Arg Cys Phe Val Glu Asp Arg Ser Ser Lys Val Ala Trp
    50                  55                  60

Leu Asn Arg Ser Gly Ile Ile Phe Ala Gly Glu Asp Lys Trp Ser Leu
65                  70                  75                  80

Asp Pro Arg Val Glu Leu Glu Lys Arg Ser Pro Leu Glu Tyr Ser Leu
                85                  90                  95

Arg Ile Gln Lys Val Asp Val Tyr Asp Glu Gly Ser Tyr Thr Cys Ser
            100                 105                 110

Val Gln Thr Gln His His Pro Lys Thr Ser Gln Val Tyr Leu Ile Val
        115                 120                 125

Gln Val Pro Pro Lys Ile Ser Asn Ile Ser Ser Asp Ile Thr Val Asn
    130                 135                 140

Glu Gly Ser Asn Val Thr Leu Val Cys Met Ala Asn Gly Arg Pro Glu
145                 150                 155                 160

Pro Val Ile Thr Trp Arg His Leu Thr Pro Thr Gly Lys Glu Phe Glu
                165                 170                 175

Gly Glu Glu Glu Tyr Leu Glu Ile Leu Gly Ile Thr Arg Glu Gln Ser
            180                 185                 190

Gly Lys Tyr Glu Cys Lys Ala Ala Asn Glu Val Ala Ser Ala Asp Val
        195                 200                 205

Lys Gln Val Arg Val Thr Val Asn Tyr Pro Pro Thr Ile Thr Glu Ser
    210                 215                 220

Lys Ser Asn Glu Ala Ala Thr Gly Arg Gln Ala Leu Leu Arg Cys Glu
225                 230                 235                 240

Ala Ser Ala Val Pro Thr Pro Asp Phe Glu Trp Tyr Arg Asp Asp Thr
                245                 250                 255

Arg Ile Asn Ser Ala Asn Gly Leu Glu Ile Lys Ser Thr Gly Ser Gln
            260                 265                 270

Ser Leu Leu Met Val Ala Asn Val Thr Glu Glu His Tyr Gly Asn Tyr
        275                 280                 285

Thr Cys Val Ala Ala Asn Lys Leu Gly Val Thr Asn Ala Ser Leu Tyr
    290                 295                 300

Leu Tyr Lys Arg Val Leu Pro Thr Leu Pro Asn Pro Phe Pro Gly Pro
305                 310                 315                 320

Gly Thr Gly Arg Val Asp Asn Gly Ser Val Ser Leu Ala Val Pro Leu
                325                 330                 335

Trp Leu Leu Ala Ala Ser Leu Leu Cys Leu Leu Ser Lys Cys
            340                 345                 350

<210> 61
<211> 338
<212> PRT
<213> Rattus norvegicus

<400> 61
Met Val Gly Arg Val Gln Pro Asp Arg Lys Gln Leu Pro Leu Val Leu
1               5                   10                  15

Leu Arg Leu Leu Cys Leu Leu Pro Thr Gly Leu Pro Val Arg Ser Val
            20                  25                  30

Asp Phe Asn Arg Gly Thr Asp Asn Ile Thr Val Arg Gln Gly Asp Thr
        35                  40                  45

Ala Ile Leu Arg Cys Val Val Glu Asp Lys Asn Ser Lys Val Ala Trp
    50                  55                  60

Leu Asn Arg Ser Gly Ile Ile Phe Ala Gly His Asp Lys Trp Ser Leu
65                  70                  75                  80

Asp Pro Arg Val Glu Leu Glu Lys Arg His Ala Leu Glu Tyr Ser Leu
                85                  90                  95

Arg Ile Gln Lys Val Asp Val Tyr Asp Glu Gly Ser Tyr Thr Cys Ser
            100                 105                 110

Val Gln Thr Gln His Glu Pro Lys Thr Ser Gln Val Tyr Leu Ile Val
        115                 120                 125

Gln Val Pro Pro Lys Ile Ser Asn Ile Ser Ser Asp Val Thr Val Asn
    130                 135                 140

Glu Gly Ser Asn Val Thr Leu Val Cys Met Ala Asn Gly Arg Pro Glu
145                 150                 155                 160

Pro Val Ile Thr Trp Arg His Leu Thr Pro Leu Gly Arg Glu Phe Glu
                165                 170                 175

Gly Glu Glu Glu Tyr Leu Glu Ile Leu Gly Ile Thr Arg Glu Gln Ser
            180                 185                 190

Gly Lys Tyr Glu Cys Lys Ala Ala Asn Glu Val Ser Ser Ala Asp Val
        195                 200                 205

Lys Gln Val Lys Val Thr Val Asn Tyr Pro Pro Thr Ile Thr Glu Ser
    210                 215                 220

Lys Ser Asn Glu Ala Thr Thr Gly Arg Gln Ala Ser Leu Lys Cys Glu
225                 230                 235                 240

Ala Ser Ala Val Pro Ala Pro Asp Phe Glu Trp Tyr Arg Asp Asp Thr
                245                 250                 255

Arg Ile Asn Ser Ala Asn Gly Leu Glu Ile Lys Ser Thr Glu Gly Gln
            260                 265                 270

Ser Ser Leu Thr Val Thr Asn Val Thr Glu Glu His Tyr Gly Asn Tyr
        275                 280                 285

Thr Cys Val Ala Ala Asn Lys Leu Gly Val Thr Asn Ala Ser Leu Val
    290                 295                 300

Leu Phe Arg Pro Gly Ser Val Arg Gly Ile Asn Gly Ser Ile Ser Leu
305                 310                 315                 320

Ala Val Pro Leu Trp Leu Leu Ala Ala Ser Leu Phe Cys Leu Leu Ser
                325                 330                 335

Lys Cys

<210> 62
<211> 8797
<212> DNA
<213> Mus musculus

<400> 62
ctggtgcacc gggagtccga tgagttttct agacaaggtg ggtgagtgac aggtgccaca 60
actccagcct ccccttttct gaaagtcgcg gagttctgtt tcatctggaa taatggatgt 120
aaaggaccgg cgacatcgct ctttgaccag gggacggtgt ggcaaagagt gtcgctacac 180
cagctcctct ctggacagtg aggactgccg tgtgcccact cagaagtcct acagttccag 240
tgagaccttg aaggcttatg accatgacag cagaatgcac tatggaaacc gagtcacaga 300
cctggtgcac cgggagtccg atgagttttc tagacaaggg acaaacttca ccctggcaga 360
attgggaatc tgcgagccct ccccacaccg aagtggttac tgttccgaca tgggtatcct 420
ccaccagggc tactccctga gcactgggtc tgatgcagac tcggacaccg agggagggat 480
gtctccagaa catgccatca gactgtgggg acgagggata aaatccaggc gcagctctgg 540
cttgtccagc cgcgagaact cggcccttac tctgactgac tctgacaatg aaaataaatc 600
ggatgacgac aatggtcgtc ccattccacc tacatcctcg tctagcctcc tcccatctgc 660
tcagctgcct agctcccata atcctccacc agttagctgc cagatgccat tgctagacag 720
caacacctcc catcagatca tggacaccaa ccctgatgag gaattctccc ccaattcata 780
cctgctcaga gcatgctcag ggccccagca agcctccagc agtggccctc caaaccacca 840
cagccagtca acactgaggc cccctctgcc accccctcat aaccacaccc tgtcccacca 900
ccactcctcg gccaactccc tcaacaggaa ctcactgacc aatcggcgga gtcaaatcca 960
cgccccagct cctgcgccca acgacctggc caccacccca gagtctgttc agctccagga 1020
tagctgggtg ctgaacagta acgtcccact ggagactcgg cacttccttt tcaaaacgtc 1080
gtctggaagc acacccctgt tcagcagctc ttctccggga taccctttga cctcagggac 1140
cgtttataca ccaccacccc gcctgctgcc acggaataca ttctccagga aggccttcaa 1200
gctgaagaaa ccctccaaat actgcagttg gaaatgtgct gccctgtctg ccatcgccgc 1260
cgccctcctc ttggccattt tgctggcata tttcatagca atgcatctgc tcggactcaa 1320
ttggcaactc cagccggcag atggacacac ctttaacaat ggcgtaagga ccggcttacc 1380
aggaaacgat gatgtggcaa cagtgccatc tggaggcaaa gtgccctggt cattgaaaaa 1440
cagcagcata gacagtggcg aagcagaagt tggtcggcgg gtgacacagg aagtcccacc 1500
aggggtgttt tggaggtccc agattcacat cagtcagcct caattcttaa agttcaacat 1560
ctccctgggc aaggatgccc tcttcggtgt ctatataagg agaggactac caccgtctca 1620
tgcccagtat gacttcatgg aacgcctgga tggaaaggag aaatggagcg tggtcgagtc 1680
gcccagggaa cgccggagca tccagactct ggtgcagaac gaggctgtgt ttgtgcagta 1740
cttggatgtg ggcctgtggc acctggcctt ctacaatgac ggcaaggaca aggagatggt 1800
ctccttcaac actgttgtct tagattcagt gcaggactgt ccacggaact gtcacgggaa 1860
cggtgaatgc gtgtctggac tgtgtcactg tttcccagga ttcctaggtg cagactgtgc 1920
taaagctgcc tgccctgtac tgtgcagcgg aaatggacag tattctaaag gaacgtgcca 1980
gtgctacagc ggctggaaag gtgcagagtg tgatgtgcct atgaaccaat gtatcgatcc 2040
ttcctgtggg ggccatggct cctgcattga tgggaactgc gtgtgtgctg ctggctacaa 2100
gggcgagcac tgtgaggaag ttgattgctt ggatcctacc tgctccagcc atggtgtctg 2160
tgtgaatgga gagtgtctat gcagccccgg ctggggtggt ctcaactgtg agctggcgag 2220
ggtccagtgc ccagaccagt gtagtgggca tggcacttac ctccctgact ccggcctctg 2280
cagctgtgat ccgaactgga tgggtcccga ctgctctgtt gtgtgctcag tagactgtgg 2340
cactcacggc gtctgcatcg ggggagcctg ccgctgtgaa gagggctgga caggcgcagc 2400
ttgtgaccag cgcgtgtgcc acccccgctg cattgagcac gggacctgta aagatggcaa 2460
atgtgaatgc cgagagggct ggaatggtga acactgcacc attgatggct gccctgattt 2520
gtgcaacggt aacgggagat gcacactggg tcagaacagc tggcagtgtg tctgccagac 2580
cggctggaga gggcctggat gcaacgttgc catggaaacc tcctgcgctg ataacaagga 2640
taatgaggga gatggcctgg tggactgcct ggaccctgac tgctgcctac agtcagcctg 2700
tcagaacagc ctgctctgcc gggggtctcg ggaccccttg gacatcattc agcaaggtca 2760
gacagactgg cctgcagtga agtccttcta tgaccgcatc aagctcttgg caggcaagga 2820
cagcacccac atcattcctg gagacaaccc cttcaatagc agcctggtgt ctctgatccg 2880

aggccaagta gtaaccatgg atgggactcc cttggtgggt gtgaatgtgt cttttgtcaa 2940
gtacccaaaa tatggctaca ccatcactcg ccaggatggc acgtttgacc tgattgccaa 3000
tgggggttct gccttgactc ttcactttga gcgagcccct ttcatgagcc aggagcgcac 3060
agtgtggctg ccatggaaca gcttctatgc catggacacc ctggtaatga agaccgagga 3120
aaactccatc cccagctgtg acctcagtgg ctttgtccgg ccagatccaa tcatcatctc 3180
ctctcctctg tccaccttct tcagcgcttc ccctgcctcg aaccccattg tgcctgagac 3240
ccaggttctt catgaagaaa ttgagctccc tggtaccaat gtgaagctcc gttatctcag 3300
ctctagaact gcagggtata agtcgctgct gaagatcacc atgacgcagt ccacagtgcc 3360
cttgaacctc atcagggttc acttgatggt tgctgtagag gggcatctct tccagaagtc 3420
attccaggct tctcccaacc tagcctacac attcatctgg gacaagacag atgcttatgg 3480
ccaaagggtt tatggcctat cggatgctgt tgtgtctgtt gggtttgaat atgagacctg 3540
ccccagtctc atcctgtggg agaaaaggac agccctgctt cagggattcg agctggaccc 3600
ttccaacctt ggaggctggt ccctggacaa acaccacacc ctcaatgtga aaagcggaat 3660
actacacaaa gggacagggg agaaccagtt cctgacccag cagcctgcca tcatcacgag 3720
catcatgggc aacggtcgcc gcagaagcat ctcctgtccc agctgcaatg gccttgctga 3780
aggcaacaaa ctgttagccc ctgtggccct ggctgtgggg atcgatggga gcctctttgt 3840
tggtgacttc aactatatcc ggcgcatctt tccctctcga aatgtgacca gtatcttgga 3900
gttacgaaat aaagagttta aacatagcaa cagcccagga cacaagtact acttggctgt 3960
ggaccccgtg actggctcac tctacgtctc tgacaccaac agtcgccgaa tctaccgagt 4020
caagtctctg agcggagcca aagacctggc tggaaattcg gaagttgtgg cagggactgg 4080
cgaacaatgt ctaccctttg atgaagcccg ctgtggggat ggagggaagg ctgtggacgc 4140
caccctgatg agccccagag gtattgcagt agacaagaat gggcttatgt actttgttga 4200
tgccaccatg atccggaagg tggaccaaaa cggaatcatc tccaccctgc tgggctccaa 4260
tgacctcaca gctgtccgac cactgagctg tgactcgagc atggacgtgg cccaggtccg 4320
tctagaatgg ccgacagacc tcgccgtcaa ccccatggac aactccctgt acgttctgga 4380
gaacaacgtc atcctgcgga tcacggagaa ccaccaggtc agcatcatcg cgggacggcc 4440
tatgcactgc caggttcccg gcatcgacta ctcgctcagc aaactcgcca tccactctgc 4500
gctggaatca gccagcgcca ttgccatttc tcacactggg gtgctctaca tcactgagac 4560
ggacgagaag aagatcaacc gcctacgcca agtcaccacc aatggagaga tctgcctctt 4620
agccggggcg gcctcagact gtgactgcaa aaacgatgtc aactgcatct gctactcggg 4680
agatgacgct tacgccacgg acgccatcct gaactcgccg tcctccttag ccgtggctcc 4740
ggatggcacc atctacattg cagaccttgg gaatatccgg atcagggcgg tcagcaaaaa 4800
taaacccgtt cttaacgcat tcaaccagta tgaggctgca tctccgggag aacaggaatt 4860
gtacgtgttc aacgctgatg gtatccatca gtacactgtg agtctggtga ctggggagta 4920
cttgtacaat ttcacataca gcgctgacaa tgacgtcacc gagttgattg acaacaacgg 4980
gaattcccta aagatccgcc gggacagcag tggcatgccc cgccacctgc tcatgccgga 5040
taatcagatt atcaccctta ctgtgggcac caatggaggc ctcaaagccg tgtccactca 5100
gaacctggag ctgggcctca tgacttatga tgggaacact ggactcctag ccaccaagag 5160
tgatgaaacc ggatggacaa ctttttatga ctatgaccac gagggccgtc tgaccaatgt 5220
gacccgcccc acgggcgtgg tgaccagtct gcaccgggaa atggagaaat ctatcaccat 5280
tgacattgag aactccaacc gggatgatga cgtcactgtg atcaccaacc tctcctccgt 5340
ggaggcctcc tatacagtgg tacaagatca agtgcgaaac agctaccagc tctgcaataa 5400
tggaaccctg cgggtgatgt acgccaacgg catggctgtc agcttccaca gtgagcccca 5460
cgtcctcgca ggcaccatca cccccaccat cgggcgctgc aacatctctc tgcccatgga 5520
gaatggcctg aactccatcg agtggcgcct gaggaaggaa cagatcaaag gcaaagtcac 5580
catctttggg aggaagcttc gggtccacgg aaggaatctc ctgtccattg attatgaccg 5640
aaatatccgt acggagaaga tctacgatga ccaccggaaa ttcaccctga ggatcatcta 5700
tgaccaggtg ggccgcccct tcctgtggct cccgagcagt gggctggcag ccgtcaatgt 5760
ctcctacttc ttcaatgggc gcttggccgg cctccagcga ggggccatga gcgagaggac 5820
agacattgac aagcaaggcc ggatcgtgtc ccgcatgttc gccgacggga aagtctggag 5880
ttattcctat cttgacaagt ccatggtcct tctgctacag agccaacgtc agtacatatt 5940
tgaatatgac tcctccgatc gcctccacgc agtcactatg cccagtgtcg cccggcacag 6000
catgtccacg cacacctcca ttggttacat ccgaaacatt tacaacccac ccgaaagcaa 6060
tgcatcggtc atctttgact acagtgatga cggccgcatc ctaaagacat ctttcttggg 6120
cactgggcgc caggtgttct acaagtatgg aaaactctcc aagttatcag agatagtcta 6180
cgacagcaca gccgtcacct ttgggtatga cgagaccacc ggtgtcctga agatggtcaa 6240
tctccaaagt gggggcttct cctgtaccat caggtaccga aaggttgggc cccttgtgga 6300
caagcagatt tacaggttct ctgaggaagg aatgatcaac gccaggtttg attataccta 6360
tcacgacaat agcttccgca ttgccagcat caaacccgtc attagcgaga ctccccttcc 6420
tgttgacctc taccgctatg acgagatttc cggcaaggtg gaacacttcg gcaagtttgg 6480
ggtcatctac tacgacatca accagatcat caccactgcc gtcatgacgc ttagcaagca 6540

ctttgacacc catgggcgca tcaaggaagt gcaatatgag atgttccggt ccctcatgta 6600
ctggatgact gtgcaatatg acagtatggg tagggtcatc aagagggaac tgaaactagg 6660
gccctatgcc aacaccacaa agtacaccta tgactatgac ggggacggcc agctccagag 6720
tgtggccgtc aatgaccggc ctacctggcg ctatagctat gacctcaatg ggaacctgca 6780
ccttctaaac ccaggaaaca gtgctcgcct catgccctta cgctatgacc tccgtgaccg 6840
gataaccagg ctaggggacg tgcagtacaa aatcgatgac gatggctatt tgtgccagag 6900
agggtcagac atctttgaat acaactccaa gggccttctg acgagagcat acaacaaggc 6960
cagcggatgg agcgtgcagt accgctatga cggagtgggc cgccgggctt cctacaagac 7020
caacctgggc caccacctac agtacttcta ctccgacctc cacaacccca cacgtatcac 7080
ccatgtttac aaccactcca actctgagat cacctcgctc tactatgacc tccagggcca 7140
cctatttgcc atggagagca gtagtggtga agaatactat gtcgcctcag acaacacggg 7200
gacccctctg gctgtgtaca gtatcaatgg cctcatgatc aagcaactgc agtacacagc 7260
ctatggggag atctactatg actccaatcc agacttccag atggtcattg gcttccacgg 7320
aggcctctat gaccccctca ccaagctcgt ccactttact caacgtgatt atgacgtgct 7380
ggcaggacgg tggacgtccc ccgactacac catgtggagg aacgtgggca aggagccagc 7440
ccccttcaac ctgtacatgt tcaagaacaa caatcctctg agcaatgagc tggacttaaa 7500
gaactacgtg acagacgtga agagctggct tgtgatgttt ggatttcagc tcagcaacat 7560
cattcctgga ttcccgagag ccaaaatgta ttttgtgcct cccccctatg aactgtcaga 7620
gagtcaagca agcgagaacg gacagctcat tacaggtgtc cagcagacaa ctgagaggca 7680
taaccaggcc ttcctggctc tggaaggaca ggtcatcact aaaaagctcc atgccagcat 7740
ccgagagaaa gcaggccact ggtttgctac caccacaccc atcatcggca aaggcatcat 7800
gtttgccatc aaagaagggc gggtgaccac aggagtgtct agcatcgcca gtgaggacag 7860
ccgcaaggta gcatccgtgt tgaacaatgc ctactactta gacaagatgc actacagcat 7920
cgagggcaag gacacacact actttgtgaa gatcggcgcc gcggatggtg acctggtcac 7980
gctaggaacc accattgggc gcaaggtgct ggagagtggg gtgaacgtga cggtgtcaca 8040
gcccacgctg ctggtgaatg gcaggactcg aaggttcacc aacattgagt tccagtactc 8100
cacgctgctg ctcagtatcc gctacggcct cacccccgac acgctggacg aagaaaaggc 8160
ccgcgtcctg gaccaagcgg gacagagagc cctgggtact gcctgggcca aggagcagca 8220
gaaagccagg gacgggagag agggcagccg cctgtggacg gagggcgaga agcagcaact 8280
cctgagcacg ggacgggtac aaggttatga gggctattac gtacttccgg tggaacagta 8340
cccggagctg gcagacagta gcagcaacat ccagttctta agacagaatg agatgggaaa 8400
gaggtaacaa aataacctgc tgccacctct tctctgggtg gctcagcagg agcaactgtg 8460
acctcctctc ctaaggagac gaagacctaa cggggcactg aggccgggct gctttaggat 8520
cccaagtggc aagaaagctc acattttttg agttcaaatg ctactgtcta agcgcaaagt 8580
ccctcatcct gaagtagact agagcccggc cacaaatttc tgaggaaaaa caaaaactaa 8640
aggatgaacg aacgaacgaa cgaatgaaaa cacacacaaa atgtttcaag ttcccctaaa 8700
atatgaccca cttgttccgg gtctaaggca gaaaagagac gcagaatagc caaaaggaaa 8760
ggaacagaaa agaaacaaat taaaaaaaaa aaaaaaa 8797

<210> 63
<211> 2496
<212> DNA
<213> Gallus gallus

<400> 63
atggatataa aagatcgaag acaccgctct ttgacgagag gccggtgcgg gaaggagtgt 60
cgctatacta gttcttcact cgacagtgaa gactgcagag taccaactca gaagtcctac 120
agctccagtg agactctgaa agcatatgac catgacacga ggatgcacta cggaaatcga 180
gtttcagacc tggttcacag ggagtcggat gagtttccaa ggcaaggaac gaacttcacc 240
cttgcagaac tgggaatctg tgagccctct ccccatcgaa gtggctactg ctcggacata 300
ggaatactcc atcaaggcta ttccttgagc actggctctg atgctgactc agacacggag 360
ggcgggatgt ctccagagca cgcgatcagg ctgtggggaa gagggatcaa atccaggcga 420
agttctggcc tgtcaagtcg tgaaaactcg gctctcacgc tcactgactc cgacaatgag 480
aacaagtcag atgaggaaaa cggtcgtccc attccaccta catcctcgtc tagccttctc 540
ccatctgctc agctgcccag ttctcataat cctccaccag ttagctgcca gatgccattg 600
ctagacagca atacgtccca tcaaatcatg gacaccaatc ctgacgagga gttctctcct 660
aattcatacc tactaagagc atgttcaggg ccacagcagg catccagcag tggcccttca 720
aaccatcaca gccagtcaac gctgaggcca cctctccccc ctcctcacaa ccactcgctg 780
tcccatcatc actcgtctgc caactccctc aacaggaact cgctcaccaa ccgccgcaac 840
cagatccacg cgcctgctcc cgctcccaat gacctggcga ccacgcctga gtctgtgcag 900

ctgcaggaca gctgggtgct caacagcaac gtgccgctgg agaccaggca tttcttgttt 960
aagacatctt ctggaacgac tccgctgttc agtagctctt cccctggcta cccactgacc 1020
tcaggaacag tttatactcc acctcccagg ctgttaccta gaaatacatt ttccaggaat 1080
gcattcaagc tgaaaaagcc ctccaagtat tgtagctgga aatgtgctgc tttatctgca 1140
attgctgctg cagtcctgct tgccatcctg ctagcatatt tcatagcgat gcacctcctg 1200
gggctgaact ggcagctgca gcccgcggac ggacacacct tcagcaacgg gctgcggccg 1260
ggcgcggcgg gcgcggagga cggagcggcg gcgccacctg caggcagagg accgtgggtc 1320
actaggaata gcagcataga tagtggagaa acagaagttg gccgcaaggt cacccaagag 1380
gtgccccctg gagtgttctg gcggtctcag atccatatca gccagccaca gttcctgaag 1440
ttcaacatat ccctagggaa ggatgctctt ttcggtgttt atataagaag aggactccca 1500
ccatcacatg cacagtatga tttcatggaa cgcttggatg ggaaagagaa atggagtgtg 1560
gtggaatccc cacgggaacg gcgaagtatt cagactcttg ttcagaatga ggctgtgttt 1620
gttcagtact tggatgtggg tttgtggcac ctggcgtttt acaatgatgg caaggacaaa 1680
gaagtggtct ccttcagtac agttattttg gattcagtgc aagactgtcc acgtaattgt 1740
catggcaatg gcgagtgtgt ttctggtgtc tgccactgtt ttcccggatt tcatggagca 1800
gattgtgcta aagctgcctg cccggtgctg tgcagtggca atggtcagta ctccaaagga 1860
acctgcttgt gctacagtgg ctggaaaggt ccggaatgtg atgtacccat cagccagtgt 1920
attgatccct cgtgtggagg tcatggttcc tgcatcgaag ggaactgtgt ctgttccatt 1980
ggctataaag gagaaaactg tgaggaagtt gattgcttag atccaacatg ctccaatcac 2040
ggggtctgtg tgaacggaga atgtctctgc agcccaggct ggggtggaat aaactgtgag 2100
cttcccagag cccagtgccc agaccagtgc agtgggcatg gcacatacct gtctgacacc 2160
ggtctctgta gctgcgatcc caactggatg ggtcccgact gctccgttga agtgtgctct 2220
gtagactgtg gcacccatgg ggtgtgcatt ggcggagcgt gtcgctgtga agaagggtgg 2280
acaggagtgg cgtgtgacca gcgtgtgtgt catccccggt gtacagagca cggaacttgt 2340
aaagatggga aatgtgaatg cagagagggc tggaatgggg agcactgcac cattggtagg 2400
caaacgactg gcaccgaaac aggctcatat tgctttcttt tcataaatcg tagagacata 2460
gttactgtgg tgtactgtga taggctgttc ctttaa 2496

<210> 64
<211> 6560
<212> DNA
<213> Homo sapiens

<400> 64
caccttcttt agtgctgccc ctgggcagaa tcccatcgtg cctgagaccc aggttcttca 60
tgaagaaatc gagctccctg gttccaatgt gaaacttcgc tatctgagct ctagaactgc 120
agggtacaag tcactgctga agatcaccat gacccagtcc acagtgcccc tgaacctcat 180
tagggttcac ctgatggtgg ctgtcgaggg gcatctcttc cagaagtcat tccaggcttc 240
tcccaacctg gcctacacct tcatctggga caagacagat gcgtatggcc aaagggtgta 300
tggactctca gatgctgttg tgtctgtcgg gtttgaatat gagacctgtc ccagtctaat 360
tctctgggag aaaaggacag ccctccttca gggattcgag ctggacccct ccaacctcgg 420
tggctggtcc ctagacaaac accacatcct caatgttaaa agtggaatcc tacacaaagg 480
cactggggaa aaccagttcc tgacccagca gcctgccatc atcaccagca tcatgggcaa 540
tggtcgccgc cggagcattt cctgtcccag ctgcaacggc cttgctgaag gcaacaagct 600
gctggcccca gtggctctgg ctgttggaat cgatggagc ctctatgtgg gtgacttcaa 660
ttacatccga cgcatctttc cctctcgaaa tgtgaccagc atcttggagt tacgaaataa 720
agagtttaaa catagcaaca acccagcaca caagtactac ttggcagtgg accccgtgtc 780
cggctcgctc tacgtgtccg acaccaacag caggagaatc taccgcgtca agtctctgag 840
tggaaccaaa gacctggctg ggaattcgga agttgtggca gggacgggag agcagtgtct 900
accctttgat gaagcccgct gcggggatgg agggaaggcc atagatgcaa ccctgatgag 960
cccgagaggt attgcagtag acaagaatgg gctcatgtac tttgtcgatg ccaccatgat 1020
ccggaaggtt gaccagaatg gaatcatctc caccctgctg ggctccaatg acctcactgc 1080
cgtccggccg ctgagctgtg attccagcat ggatgtagcc caggttcgtc tggagtggcc 1140
aacagacctt gctgtcaatc ccatggataa ctccttgtat gttctagaga acaatgtcat 1200
ccttcgaatc accgagaacc accaagtcag catcattgcg ggacgcccca tgcactgcca 1260
agttcctggc attgactact cactcagcaa actagccatt cactctgccc tggagtcagc 1320
cagtgccatt gccatttctc acactggggt cctctacatc actgagacag atgagaagaa 1380
gattaaccgt ctacgccagg taacaaccaa cggggagatc tgccttttag ctggggcagc 1440
ctcggactgc gactgcaaaa acgatgtcaa ttgcaactgc tattcaggag atgatgccta 1500
cgcgactgat gccatcttga attccccatc atccttagct gtagctccag atggtaccat 1560

ttacattgca gaccttggaa atattcggat cagggcggtc agcaagaaca agcctgttct 1620
taatgccttc aaccagtatg aggctgcatc ccccggagag caggagttat atgttttcaa 1680
cgctgatggc atccaccaat acactgtgag cctggtgaca ggggagtact tgtacaattt 1740
cacatatagt actgacaatg atgtcactga attgattgac aataatggga attccctgaa 1800
gatccgtcgg gacagcagtg gcatgccccg tcacctgctc atgcctgaca accagatcat 1860
caccctcacc gtgggcacca atggaggcct caaagtcgtg tccacacaga acctggagct 1920
tggtctcatg acctatgatg gcaacactgg gctcctggcc accaagagcg atgaaacagg 1980
atggacgact ttctatgact atgaccacga aggccgcctg accaacgtga cgcgccccac 2040
gggggtggta accagtctgc accgggaaat ggagaaatct attaccattg acattgagaa 2100
ctccaaccgt gatgatgacg tcactgtcat caccaacctc tcttcagtag aggcctccta 2160
cacagtggta caagatcaag ttcggaacag ctaccagctc tgtaataatg gtaccctgag 2220
ggtgatgtat gctaatggga tgggtatcag cttccacagc gagccccatg tcctagcggg 2280
caccatcacc cccaccattg gacgctgcaa catctccctg cctatggaga atggcttaaa 2340
ctccattgag tggcgcctaa gaaaggaaca gattaaaggc aaagtcacca tctttggcag 2400
gaagctccgg gtccatggaa gaaatctctt gtccattgac tatgatcgaa atattcggac 2460
tgaaaagatc tatgatgacc accggaagtt caccctgagg atcatttatg accaggtggg 2520
ccgccccttc ctctggctgc ccagcagcgg gctggcagct gtcaacgtgt catacttctt 2580
caatgggcgc ctggctgggc ttcagcgtgg ggccatgagc gagaggacag acatcgacaa 2640
gcaaggccgc atcgtgtccc gcatgttcgc tgacgggaaa gtgtggagct actcctacct 2700
tgacaagtcc atggtcctcc tgcttcagag ccaacgtcag tatatatttg agtatgactc 2760
ctctgaccgc ctccttgccg tcaccatgcc cagcgtggcc cggcacagca tgtccacaca 2820
cacctccatc ggctacatcc gtaatattta caacccgcct gaaagcaatg cttcggtcat 2880
ctttgactac agtgatgacg gccgcatcct gaagacctcc tttttgggca ccggacgcca 2940
ggtgttctac aagtatggga aactctccaa gttatcagag attgtctacg acagtaccgc 3000
cgtcaccttc gggtatgacg agaccactgg tgtcttgaag atggtcaacc tccaaagtgg 3060
gggcttctcc tgcaccatca ggtaccggaa gattggcccc ctggtggaca agcagatcta 3120
caggttctcc gaggaaggca tggtcaatgc caggtttgac tacacctatc atgacaacag 3180
cttccgcatc gcaagcatca agcccgtcat aagtgagact cccctccccg ttgacctcta 3240
ccgctatgat gagatttctg gcaaggtgga acactttggt aagtttggag tcatctatta 3300
tgacatcaac cagatcatca ccactgccgt gatgaccctc agcaaacact tcgacaccca 3360
tgggcggatc aaggaggtcc agtatgagat gttccggtcc ctcatgtact ggatgacggt 3420
gcaatatgac agcatgggca gggtgatcaa gagggagcta aaactggggc cctatgccaa 3480
taccacgaag tacacctatg actacgatgg ggacgggcag ctccagagcg tggccgtcaa 3540
tgaccgcccg acctggcgct acagctatga ccttaatggg aatctccact tactgaaccc 3600
aggcaacagt gtgcgcctca tgcccttgcg ctatgacctc cgggatcgga taaccagact 3660
cggggatgtg cagtacaaaa ttgacgacga tggctatctg tgccagagag ggtctgacat 3720
cttcgaatac aattccaagg gcctcctaac aagagcctac aacaaggcca gcgggtggag 3780
tgtccagtac cgctatgatg gcgtaggacg gcgggcttcc tacaagacca acctgggcca 3840
ccacctgcag tacttctact ctgacctcca caacccgacg cgcatcaccc atgtctacaa 3900
tcactccaac tcggagatta cctcactgta ctacgacctc cagggccacc tctttgccat 3960
ggagagcagc agtggggagg agtactatgt tgcctctgat aacacaggga ctcctctggc 4020
tgtgttcagc atcaacggcc tcatgatcaa acagctgcag tacacggcct atggggagat 4080
ttattatgac tccaaccccg acttccagat ggtcattggc ttccatgggg gactctatga 4140
ccccctgacc aagctggtcc acttcactca gcgtgattat gatgtgctgg caggacgatg 4200
gacctcccca gactatacca tgtggaaaaa cgtgggcaag gagccggccc cctttaacct 4260
gtatatgttc aagagcaaca atcctctcag cagtgagcta gatttgaaga actacgtgac 4320
agatgtgaaa agctggcttg tgatgtttgg atttcagctt agcaacatca ttcctggctt 4380
cccgagagcc aaaatgtatt tcgtgcctcc tccctatgaa ttgtcagaga gtcaagcaag 4440
tgagaatgga cagctcatta caggtgtcca acagacaaca gagagacata accaggcctt 4500
catggctctg gaaggacagg tcattactaa aaagctccac gccagcatcc gagagaaagc 4560
aggtcactgg tttgccacca ccacgcccat cattggcaaa ggcatcatgt ttgccatcaa 4620
agaagggcgg gtgaccacgg gcgtgtccag catcgccagc gaagatagcc gcaaggtggc 4680
atctgtgctg aacaacgcct actacctgga caagatgcac tacagcatcg agggcaagga 4740
cacccactac tttgtgaaga ttggctcagc cgatggcgac ctggtcacac taggcaccac 4800
catcggccgc aaggtgctag agagcggggt gaacgtgacc gtgtcccagc ccacgctgct 4860
ggtcaacggc aggactcgaa ggttcacgaa cattgagttc cagtactcca cgctgctgct 4920
cagcatccgc tatggcctca cccccgacac cctggacgaa gagaaggccc gcgtcctgga 4980
ccaggcgaga cagagggccc tgggcacggc ctgggccaag gagcagcaga aagccaggga 5040
cgggagagag gggagccgcc tgtggactga gggcgagaag cagcagcttc tgagcaccgg 5100
gcgcgtgcaa gggtacgagg gatattacgt gcttcccgtg gagcaatacc cagagcttgc 5160
agacagtagc agcaacatcc agtttttaag acagaatgag atgggaaaga ggtaacaaaa 5220

taatctgctg ccattccttg tctgaatggc tcagcaggag taactgttat ctcctctcct 5280
aaggagatga agacctaaca ggggcactgc ggctgggctg ctttaggaga ccaagtggca 5340
agaaagctca cattttttga gttcaaatgc tactgtccaa gcgagaagtc cctcatcctg 5400
aagtagacta aagcccggct gaaaattccg aggaaaacaa aacaaacgaa tgaatgaaca 5460
gacacacaca atgttccaag ttcccctaaa atatgaccca cttgttctgg gtctacgcag 5520
aaaagagacg caaagtgtcc aaaaggaaca aaagaacaaa aacgaataag caaagaagaa 5580
aacaaacaaa aacaaaacaa aacaaacaca cggaccgata aacaaagaag cgaagataag 5640
aaagaaggcc tcatatccaa ttacctcact cattcacatg tgagcgacac gcagacatcc 5700
gcgagggcca gcgtcaccag accagctgcg ggacaaacca ctcagactgc ttgtaggaca 5760
aatacttctg acattttcgt ttaagcaaat acaggtgcat ttaaaacacg actttggggg 5820
tgatttgtgt gtagcgcctg ggaggggggg ataaaagagg aggagtgagc actggaaata 5880
ctttttaaag aaaaaaaaac atgagggaat aaaagaaatt cctatcaaaa atcaaagtga 5940
aataatacca tccagcactt aactctcagg tcccaactaa gtctggcctg agctaattta 6000
tttgagcgca gagtgtaaaa tttaattcaa aatggtggct ataatcacta cagataaatt 6060
tcatactctt ttgtctttgg agattccatt gtggacagta atacgcagtt acagggtgta 6120
gtctgtttag attccgtagt tcgtgggtat cagtttcggt agaggtgcag catcgtgaca 6180
cttttgctaa caggtaccac ttctgatcac cctgtacata catgagccga aaggcacaat 6240
cactgtttca gatttaaaat tattagtgtg tttgtttggt ccagaaactg agacaatcac 6300
atgacagtca ccacgaggag agaaaattta aaaaataaaa ataaaaacaa aaaaaatttt 6360
aaaaattaaa aaaacaaaaa taaagtctaa taagaacttt ggtacaggaa cttttttgta 6420
atatacatgt atgaattgtt catcgagttt ttatattaat tttaatttgc tgctaagcaa 6480
agactaggga caggcaaaga taatttatgg caaagtgttt aaattgttta tacataaata 6540
aagtctctaa aactcctgtg 6560

<210> 65
<211> 8797
<212> DNA
<213> Mus musculus

<400> 65
ctggtgcacc gggagtccga tgagttttct agacaaggtg ggtgagtgac aggtgccaca 60
actccagcct ccccttttct gaaagtcgcg gagttctgtt tcatctggaa taatggatgt 120
aaaggaccgg cgacatcgct ctttgaccag gggacggtgt ggcaaagagt gtcgctacac 180
cagctcctct ctggacagtg aggactgccg tgtgcccact cagaagtcct acagttccag 240
tgagaccttg aaggcttatg accatgacag cagaatgcac tatggaaacc gagtcacaga 300
cctggtgcac cgggagtccg atgagttttc tagacaaggg acaaacttca ccctggcaga 360
attgggaatc tgcgagccct ccccacaccg aagtggttac tgttccgaca tgggtatcct 420
ccaccagggc tactccctga gcactgggtc tgatgcagac tcggacaccg agggagggat 480
gtctccagaa catgccatca gactgtgggg acgagggata aaatccaggc gcagctctgg 540
cttgtccagc cgcgagaact cggcccttac tctgactgac tctgacaatg aaaataaatc 600
ggatgacgac aatggtcgtc ccattccacc tacatcctcg tctagcctcc tcccatctgc 660
tcagctgcct agctcccata atcctccacc agttagctgc cagatgccat tgctagacag 720
caacacctcc catcagatca tggacaccaa ccctgatgag gaattctccc ccaattcata 780
cctgctcaga gcatgctcag ggccccagca agcctccagc agtggccctc caaaccacca 840
cagccagtca acactgaggc cccctctgcc accccctcat aaccacaccc tgtcccacca 900
ccactcctcg gccaactccc tcaacaggaa ctcactgacc aatcggcgga gtcaaatcca 960
cgccccagct cctgcgccca acgacctggc caccacccca gagtctgttc agctccagga 1020
tagctgggtg ctgaacagta acgtcccact ggagactcgg cacttccttt tcaaaacgtc 1080
gtctggaagc acacccctgt tcagcagctc ttctccggga taccctttga cctcagggac 1140
cgtttataca ccaccacccc gcctgctgcc acggaataca ttctccagga aggccttcaa 1200
gctgaagaaa ccctccaaat actgcagttg gaaatgtgct gccctgtctg ccatcgccgc 1260
cgccctcctc ttggccattt tgctggcata tttcatagca atgcatctgc tcggactcaa 1320
ttggcaactc cagccggcag atggacacac ctttaacaat ggcgtaagga ccggcttacc 1380
aggaaacgat gatgtggcaa cagtgccatc tggaggcaaa gtgccctggt cattgaaaaa 1440
cagcagcata gacagtggcg aagcagaagt tggtcggcgg gtgacacagg aagtcccacc 1500
aggggtgttt tggaggtccc agattcacat cagtcagcct caattcttaa agttcaacat 1560
ctccctgggc aaggatgccc tcttcggtgt ctatataagg agaggactac caccgtctca 1620
tgcccagtat gacttcatgg aacgcctgga tggaaaggag aaatggagcg tggtcgagtc 1680
gcccagggaa cgccggagca tccagactct ggtgcagaac gaggctgtgt ttgtgcagta 1740
cttggatgtg ggcctgtggc acctggcctt ctacaatgac ggcaaggaca aggagatggt 1800

ctccttcaac actgttgtct tagattcagt gcaggactgt ccacggaact gtcacgggaa 1860
cggtgaatgc gtgtctggac tgtgtcactg tttcccagga ttcctaggtg cagactgtgc 1920
taaagctgcc tgccctgtac tgtgcagcgg aaatggacag tattctaaag gaacgtgcca 1980
gtgctacagc ggctggaaag gtgcagagtg tgatgtgcct atgaaccaat gtatcgatcc 2040
ttcctgtggg ggccatggct cctgcattga tgggaactgc gtgtgtgctg ctggctacaa 2100
gggcgagcac tgtgaggaag ttgattgctt ggatcctacc tgctccagcc atggtgtctg 2160
tgtgaatgga gagtgtctat gcagccccgg ctggggtggt ctcaactgtg agctggcgag 2220
ggtccagtgc ccagaccagt gtagtgggca tggcacttac ctccctgact ccggcctctg 2280
cagctgtgat ccgaactgga tgggtcccga ctgctctgtt gtgtgctcag tagactgtgg 2340
cactcacggc gtctgcatcg gggagcctg ccgctgtgaa gagggctgga caggcgcagc 2400
ttgtgaccag cgcgtgtgcc acccccgctg cattgagcac gggacctgta aagatggcaa 2460
atgtgaatgc cgagagggct ggaatggtga acactgcacc attgatggct gccctgattt 2520
gtgcaacggt aacgggagat gcacactggg tcagaacagc tggcagtgtg tctgccagac 2580
cggctggaga gggcctggat gcaacgttgc catggaaacc tcctgcgctg ataacaagga 2640
taatgaggga gatggcctgg tggactgcct ggaccctgac tgctgcctac agtcagcctg 2700
tcagaacagc ctgctctgcc gggggtctcg ggaccccttg gacatcattc agcaaggtca 2760
gacagactgg cctgcagtga agtccttcta tgaccgcatc aagctcttgg caggcaagga 2820
cagcacccac atcattcctg gagacaaccc cttcaatagc agcctggtgt ctctgatccg 2880
aggccaagta gtaaccatgg atgggactcc cttggtgggt gtgaatgtgt cttttgtcaa 2940
gtacccaaaa tatggctaca ccatcactcg ccaggatggc acgtttgacc tgattgccaa 3000
tgggggttct gccttgactc ttcactttga gcgagcccct ttcatgagcc aggagcgcac 3060
agtgtggctg ccatggaaca gcttctatgc catggacacc ctggtaatga agaccgagga 3120
aaactccatc cccagctgtg acctcagtgg ctttgtccgg ccagatccaa tcatcatctc 3180
ctctcctctg tccaccttct tcagcgcttc ccctgcctcg aaccccattg tgcctgagac 3240
ccaggttctt catgaagaaa ttgagctccc tggtaccaat gtgaagctcc gttatctcag 3300
ctctagaact gcagggtata agtcgctgct gaagatcacc atgacgcagt ccacagtgcc 3360
cttgaacctc atcagggttc acttgatggt tgctgtagag gggcatctct tccagaagtc 3420
attccaggct tctcccaacc tagcctacac attcatctgg gacaagacag atgcttatgg 3480
ccaaagggtt tatggcctat cggatgctgt tgtgtctgtt gggtttgaat atgagacctg 3540
ccccagtctc atcctgtggg agaaaaggac agccctgctt cagggattcg agctggaccc 3600
ttccaacctt ggaggctggt ccctggacaa acaccacacc ctcaatgtga aaagcggaat 3660
actacacaaa gggacagggg agaaccagtt cctgacccag cagcctgcca tcatcacgag 3720
catcatgggc aacggtcgcc gcagaagcat ctcctgtccc agctgcaatg gccttgctga 3780
aggcaacaaa ctgttagccc ctgtggccct ggctgtgggg atcgatggga gcctctttgt 3840
tggtgacttc aactatatcc ggcgcatctt tccctctcga aatgtgacca gtatcttgga 3900
gttacgaaat aaagagttta aacatagcaa cagcccagga cacaagtact acttggctgt 3960
ggaccccgtg actggctcac tctacgtctc tgacaccaac agtcgccgaa tctaccgagt 4020
caagtctctg agcggagcca aagacctggc tggaaattcg gaagttgtgg cagggactgg 4080
cgaacaatgt ctaccctttg atgaagcccg ctgtggggat ggagggaagg ctgtggacgc 4140
caccctgatg agccccagag gtattgcagt agacaagaat gggcttatgt actttgttga 4200
tgccaccatg atccggaagg tggaccaaaa cggaatcatc tccaccctgc tgggctccaa 4260
tgacctcaca gctgtccgac cactgagctg tgactcgagc atggacgtgg cccaggtccg 4320
tctagaatgg ccgacagacc tcgccgtcaa ccccatggac aactccctgt acgttctgga 4380
gaacaacgtc atcctgcgga tcacggagaa ccaccaggtc agcatcatcg cgggacggcc 4440
tatgcactgc caggttcccg gcatcgacta ctcgctcagc aaactcgcca tccactctgc 4500
gctggaatca gccagcgcca ttgccatttc tcacactggg gtgctctaca tcactgagac 4560
ggacgagaag aagatcaacc gcctacgcca agtcaccacc aatggagaga tctgcctctt 4620
agccggggcg gcctcagact gtgactgcaa aaacgatgtc aactgcatct gctactcggg 4680
agatgacgct tacgccacgg acgccatcct gaactcgccg tcctccttag ccgtggctcc 4740
ggatggcacc atctacattg cagaccttgg gaatatccgg atcagggcgg tcagcaaaaa 4800
taaacccgtt cttaacgcat tcaaccagta tgaggctgca tctccgggag aacaggaatt 4860
gtacgtgttc aacgctgatg gtatccatca gtacactgtg agtctggtga ctggggagta 4920
cttgtacaat ttcacataca gcgctgacaa tgacgtcacc gagttgattg acaacaacgg 4980
gaattcccta aagatccgcc gggacagcag tggcatgccc cgccacctgc tcatgccgga 5040
taatcagatt atcaccctta ctgtgggcac caatggaggc ctcaaagccg tgtccactca 5100
gaacctggag ctgggcctca tgacttatga tgggaacact ggactcctag ccaccaagag 5160
tgatgaaacc ggatggacaa ctttttatga ctatgaccac gagggccgtc tgaccaatgt 5220
gacccgcccc acgggcgtgg tgaccagtct gcaccgggaa atggagaaat ctatcaccat 5280
tgacattgag aactccaacc gggatgatga cgtcactgtg atcaccaacc tctcctccgt 5340
ggaggcctcc tatacagtgg tacaagatca agtgcgaaac agctaccagc tctgcaataa 5400
tggaaccctg cgggtgatgt acgccaacgg catggctgtc agcttccaca gtgagcccca 5460

cgtcctcgca ggcaccatca cccccaccat cgggcgctgc aacatctctc tgcccatgga 5520
gaatggcctg aactccatcg agtggcgcct gaggaaggaa cagatcaaag gcaaagtcac 5580
catctttggg aggaagcttc gggtccacgg aaggaatctc ctgtccattg attatgaccg 5640
aaatatccgt acggagaaga tctacgatga ccaccggaaa ttcaccctga ggatcatcta 5700
tgaccaggtg ggccgcccct tcctgtggct cccgagcagt gggctggcag ccgtcaatgt 5760
ctcctacttc ttcaatgggc gcttggccgg cctccagcga ggggccatga gcgagaggac 5820
agacattgac aagcaaggcc ggatcgtgtc ccgcatgttc gccgacggga aagtctggag 5880
ttattcctat cttgacaagt ccatggtcct tctgctacag agccaacgtc agtacatatt 5940
tgaatatgac tcctccgatc gcctccacgc agtcactatg cccagtgtcg cccggcacag 6000
catgtccacg cacacctcca ttggttacat ccgaaacatt tacaacccac ccgaaagcaa 6060
tgcatcggtc atctttgact acagtgatga cggccgcatc ctaaagacat ctttcttggg 6120
cactgggcgc caggtgttct acaagtatgg aaaactctcc aagttatcag agatagtcta 6180
cgacagcaca gccgtcacct ttgggtatga cgagaccacc ggtgtcctga agatggtcaa 6240
tctccaaagt gggggcttct cctgtaccat caggtaccga aaggttgggc cccttgtgga 6300
caagcagatt tacaggttct ctgaggaagg aatgatcaac gccaggtttg attataccta 6360
tcacgacaat agcttccgca ttgccagcat caaacccgtc attagcgaga ctccccttcc 6420
tgttgacctc taccgctatg acgagatttc cggcaaggtg gaacacttcg gcaagtttgg 6480
ggtcatctac tacgacatca accagatcat caccactgcc gtcatgacgc ttagcaagca 6540
ctttgacacc catgggcgca tcaaggaagt gcaatatgag atgttccggt ccctcatgta 6600
ctggatgact gtgcaatatg acagtatggg tagggtcatc aagagggaac tgaaactagg 6660
gccctatgcc aacaccacaa agtacaccta tgactatgac ggggacggcc agctccagag 6720
tgtggccgtc aatgaccggc ctacctggcg ctatagctat gacctcaatg ggaacctgca 6780
ccttctaaac ccaggaaaca gtgctcgcct catgccctta cgctatgacc tccgtgaccg 6840
gataaccagg ctaggggacg tgcagtacaa aatcgatgac gatggctatt tgtgccagag 6900
agggtcagac atctttgaat acaactccaa gggccttctg acgagagcat acaacaaggc 6960
cagcggatgg agcgtgcagt accgctatga cggagtgggc cgccgggctt cctacaagac 7020
caacctgggc caccacctac agtacttcta ctccgacctc cacaacccca cacgtatcac 7080
ccatgtttac aaccactcca actctgagat cacctcgctc tactatgacc tccagggcca 7140
cctatttgcc atggagagca gtagtggtga agaatactat gtcgcctcag acaacacggg 7200
gacccctctg gctgtgtaca gtatcaatgg cctcatgatc aagcaactgc agtacacagc 7260
ctatggggag atctactatg actccaatcc agacttccag atggtcattg gcttccacgg 7320
aggcctctat gaccccctca ccaagctcgt ccactttact caacgtgatt atgacgtgct 7380
ggcaggacgg tggacgtccc ccgactacac catgtggagg aacgtgggca aggagccagc 7440
ccccttcaac ctgtacatgt tcaagaacaa caatcctctg agcaatgagc tggacttaaa 7500
gaactacgtg acagacgtga agagctggct tgtgatgttt ggatttcagc tcagcaacat 7560
cattcctgga ttcccgagag ccaaaatgta ttttgtgcct ccccccctatg aactgtcaga 7620
gagtcaagca agcgagaacg gacagctcat tacaggtgtc cagcagacaa ctgagaggca 7680
taaccaggcc ttcctggctc tggaaggaca ggtcatcact aaaaagctcc atgccagcat 7740
ccgagagaaa gcaggccact ggtttgctac caccacaccc atcatcggca aaggcatcat 7800
gtttgccatc aaagaagggc gggtgaccac aggagtgtct agcatcgcca gtgaggacag 7860
ccgcaaggta gcatccgtgt tgaacaatgc ctactactta gacaagatgc actacagcat 7920
cgagggcaag gacacacact actttgtgaa gatcggcgcc gcggatggtg acctggtcac 7980
gctaggaacc accattgggc gcaaggtgct ggagagtggg gtgaacgtga cggtgtcaca 8040
gcccacgctg ctggtgaatg gcaggactcg aaggttcacc aacattgagt tccagtactc 8100
cacgctgctg ctcagtatcc gctacggcct cacccccgac acgctggacg aagaaaaggc 8160
ccgcgtcctg gaccaagcgg gacagagagc cctgggtact gcctgggcca aggagcagca 8220
gaaagccagg gacgggagag agggcagccg cctgtggacg gagggcgaga agcagcaact 8280
cctgagcacg ggacgggtac aaggttatga gggctattac gtacttccgg tggaacagta 8340
cccggagctg gcagacagta gcagcaacat ccagttctta agacagaatg agatgggaaa 8400
gaggtaacaa aataacctgc tgccacctct tctctgggtg gctcagcagg agcaactgtg 8460
acctcctctc ctaaggagac gaagacctaa cggggcactg aggccgggct gctttaggat 8520
cccaagtggc aagaaagctc acatttttgg agttcaaatg ctactgtcta agcgcaaagt 8580
ccctcatcct gaagtagact agagcccggc cacaaatttc tgaggaaaaa caaaaactaa 8640
aggatgaacg aacgaacgaa cgaatgaaaa cacacacaaa atgtttcaag ttcccctaaa 8700
atatgaccca cttgttccgg gtctaaggca gaaaagagac gcagaatagc caaaaggaaa 8760
ggaacagaaa agaaacaaat taaaaaaaaa aaaaaaa 8797

<210> 66
<211> 8689
<212> DNA

<213> Rattus norvegicus

<400> 66
gtgagcattc ccggaaacgc agctgagttg ttggacaaca gacgtccacc cagtcggtgg 60
gtaaagtgac aggtgccaca actccagcct ccccttttct gaaagtcgcg gagttccatt 120
tcatctgcaa taatggatgt gaaggatcgg cgacatcgct ctttgaccag gggacggtgt 180
ggcaaggagt gtcgctacac cagctcctct ctggacagtg aggactgccg tgtgcccacg 240
cagaagtcct acagttccag tgagaccctg aaggcttatg accatgacag cagaatgcac 300
tatggaaacc gagtcacaga cctggtgcac cgggagtccg atgagttttc tagacaaggg 360
gctaatttca ccctggcaga attgggaatc tgcgagccct ccccacaccg aagtggttac 420
tgttccgaca tggggatcct ccaccagggc tactccctga gcactgggtc tgatgcggac 480
tcggacaccg agggagggat gtctccagaa catgccatca gactgtgggg acgagggata 540
aaatcgaggc gcagctctgg cttgtccagc cgcgagaact cagcccttac tctgactgat 600
tctgacaatg aaaataaatc ggatgacgac aatggtcgac ccattccacc tacatcctcg 660
tctagcctcc tcccatctgc tcagctgcct agctcccata atcctccacc agttagctgc 720
cagatgccat tgctagacag caacacctcc catcagatca tggacaccaa ccccgatgag 780
gaattctccc ctaattcata cctgctcaga gcatgctcag ggccccagca agcctccagt 840
agtggccctc cgaaccacca cagccagtca acgctgaggc cccctctgcc acctcctcat 900
aaccacaccc tgtcccacca ccactcctct gccaactccc tcaacagaaa ctcactgacc 960
aatcggcgga gtcaaatcca cgccccagct cctgcaccca atgacctggc caccacgccg 1020
gagtccgttc agctccagga cagctgggtg ctgaacagta acgtgccgct ggagacgcgg 1080
cacttcctct tcaagacgtc ctccggaagc acacccctgt tcagcagctc ttctccagga 1140
taccccttga cctcagggac cgtttataca ccaccacccc gcctgctgcc acggaataca 1200
ttctctagga aggccttcaa gctgaagaaa ccctccaaat actgcagttg gaaatgcgcc 1260
gccctgtctg ccattgccgc tgccctcctt ctggccattt tgctggccta tttcatagca 1320
atgcatctgc tcggactcaa ttggcaactc cagccggcag atggacacac ctttaacaat 1380
ggcgtaagga ccggcttacc aggaaacgat gatgtggcaa cagtgccatc tggaggcaaa 1440
gtgccctggt cgttgaaaaa cagcagcata gacagcggcg aggcagaagt cggtcgacgg 1500
gtgacacagg aagtcccacc aggggtgttt tggaggtccc agattcacat cagtcagcct 1560
cagttcttaa agttcaacat ctccctgggg aaggatgccc tcttcggcgt ctacataaga 1620
agaggactgc caccatctca tgcacagtat gacttcatgg aacgcctgga cggaaaggag 1680
aagtggagtg tggtcgagtc acccagggaa cgccggagca tccagaccct ggtgcagaac 1740
gaggctgtgt tcgtgcagta cttggatgtg ggcctgtggc acctcgcctt ctacaatgac 1800
ggcaaggaca aggagatggt ctccttcaat acggttgtct tagattcagt gcaggactgt 1860
ccacgaaact gccacgggaa cggcgaatgc gtgtctggac tgtgtcactg tttcccagga 1920
ttcctaggtg cagactgcgc taaagctgcc tgccctgttc tgtgcagtgg gaatggacag 1980
tattccaaag ggacatgcca gtgctacagt ggctggaaag gagcagaatg cgatgtgccc 2040
atgaaccagt gcatcgatcc ttcctgtggg ggccacggct cctgcattga tgggaactgc 2100
gtgtgtgcag ctggctacaa gggcgagcac tgcgaagaag tggattgctt ggatccaacc 2160
tgctccagcc atggtgtctg tgtgaacgga gagtgtctat gcagccccgg ctggggcggg 2220
ctcaactgcg agctggcgag ggtccagtgc ccagaccagt gtagtgggca tggcacttac 2280
ctccctgact ctggcctctg caactgtgat ccgaattgga tgggtcccga ctgctctgtt 2340
gaagtgtgct cagtagactg tggcactcac ggcgtctgca tcgggggagc ctgccgctgt 2400
gaagagggct ggacaggcgc ggcttgtgac cagcgcgtgt gccacccccg ctgcattgag 2460
cacgggacct gtaaagatgg caaatgtgaa tgccgagagg gctggaatgg tgaacactgc 2520
accattgatg gctgccctga tttgtgcaac ggtaacggga gatgcacact gggtcagaac 2580
agctggcagt gtgtctgcca gaccggctgg agagggcccg gatgcaacgt tgccatggaa 2640
acctcctgcg ctgataacaa ggataatgag ggagatggcc tggtggactg cctggaccct 2700
gactgctgcc tccagtcagc ctgtcagaac agcctgctct gtcggggggtc tcgggacccc 2760
ttggacatca ttcagcaagg ccagacagac tggcctgcgg tgaagtcctt ctatgatcgt 2820
atcaagctct tggcaggcaa ggacagcacc cacatcattc ctggagacaa ccccttcaat 2880
agcagcctgg tgtctctgat ccgaggccaa gtagtaacca cggatgggac ccccctggtg 2940
ggtgtgaatg tgtcttttgt caagtaccca aaatatggct acaccatcac tcgccaggac 3000
ggcacgtttg acctgattgc caatgggggc tctgccttga ctcttcactt tgagcgagcc 3060
cctttcatga gccgggagcg cacagtatgg ccgccgtgga acagcttcta tgccatggac 3120
accctggtaa tgaagacgga ggagaactcc atccccagct gtgacctcag tggctttgtc 3180
cggcctgatc cgatcatcat ctcctctcct ctgtccacct tcttcagcgc ttcccctgcg 3240
gcgaacccca ttgtgcctga gacccaggtt cttcatgagg agatcgagct ccctggcacc 3300
aacgtgaagc tccgttacct cagctccaga acagcagggt acaagtcact gctgaagatc 3360
accatgaccc agtccacggt gcccttgaac ctcatccggg ttcacttgat ggttgccgtg 3420
gaggggcatc tcttccagaa gtcgttccag gcttctccca acctggccta cacattcatc 3480

tgggacaaga cagacgctta tggccaaagg gtttatggcc tatcggatgc tgttgtgtct 3540
gttggatttg aatatgagac ctgccccagt ctcatcctgt gggaaaaaag gacagcccta 3600
cttcaaggat tcgagctgga cccttccaac cttggtggct ggtccctgga taagcaccac 3660
accctcaatg tgaaaagcgg aatactactc aaaggcacag gggagaacca gttcctgacc 3720
cagcagcccg ccatcatcac cagcatcatg ggtaacggtc gccgcagaag catctcctgt 3780
cccagctgca atggccttgc tgaaggcaac aaactgttgg cccccgtggc cctggctgtg 3840
gggatcgatg ggagcctctt tgtcggtgac ttcaattata tccggcgcat cttcccttct 3900
cgaaacgtga ccagtatctt ggagttacga aataaagagt ttaaacatag caacagccca 3960
ggacacaagt actacttggc tgtggaccct gtgactggct cgctctatgt ctctgacacc 4020
aacagtcgcc ggatctaccg agtcaagtct ctaagcggag ccaaagacct ggctgggaat 4080
tcggaagttg tggccgggac tggcgaacaa tgtctaccct ttgatgaagc ccgctgtggg 4140
gatggcggga aggctgtgga tgccaccctg atgagcccta gaggtattgc agtagacaag 4200
aacgggctta tgtattttgt tgatgccacc atgatccgga aggtcgacca aaatggaatc 4260
atctccaccc tgctgggctc caatgacctc acagctgtcc gaccactgag ctgtgactct 4320
agcatggacg tggcccaggt ccgtctagaa tggccgacag accttgcggt caaccccatg 4380
gacaattccc tgtacgtcct ggagaacaac gtcatcctgc ggatcaccga gaatcaccag 4440
gtcagcatca tcgcgggacg gcccatgcac tgccaggttc ccggcatcga ctactcgctc 4500
agcaagctcg ccatccactc tgctctggag tcagccagcg ccatcgccat ttctcacacc 4560
ggggtgctct acatcaccga gacggacgag aagaagatca accgcctacg ccaggtcacc 4620
accaacggag agatctgcct cttagccggg gcagcctcag actgtgactg caaaaatgac 4680
gtcaactgca tctgctattc gggagatgac gcatacgcca cggatgccat cttgaactcc 4740
ccgtcctcct tagctgtggc tccggatggc accatctaca tcgcagacct cgggaatatc 4800
cggatcaggg cggtcagcaa aaacaaacct gttcttaacg cgttcaacca gtatgaggct 4860
gcgtctccgg gagaacagga actgtacgtg ttcaacgccg atggtatcca tcagtacacc 4920
gtgagcctgg tgaccgggga gtacttatac aatttcacct acagcgctga caatgatgtc 4980
accgagttga ttgacaacaa cgggaattcc ctaaagatcc gccgggacag cagtggcatg 5040
ccccgacacc tgctcatgcc tgataatcag atcatcaccc ttacggtggg caccaacgga 5100
ggcctcaaag ccgtgtcaac gcagaacctg gagctgggcc tcatgactta tgatgggaac 5160
actggactcc tagccaccaa gagcgatgaa accggatgga caacttttta tgactatgac 5220
cacgagggcc gtctgaccaa tgtgactcgc ccccacgggg tggtgaccag cctgcaccgg 5280
gaaatggaga aatccatcac cgttgacatt gagaactcca accgtgataa cgatgtcact 5340
gtgattacca acctctcttc agtggaggcc tcctacaccg tggtacaaga tcaagtgcgg 5400
aacagctacc agctctgcag caacggaccc ctgcgcgtca tgtacgccaa cggcatgggc 5460
gtcagcttcc acagcgagcc ccacgtcctc gcaggcaccc tcacccccac catcgggcgc 5520
tgtaacatct ccctgcccat ggagaacggc ctgaactcca tcgagtggcg cctgaggaag 5580
gaacagatta aaggcaaagt caccatcttt gggaggaagc ttcggtccac ggaaggaac 5640
ctcctgtcca ttgattatga ccgaaatatc cgcactgaga agatctatga cgaccaccgg 5700
aagttcaccc tgaggatcat ttatgaccag gtgggccgcc ccttcctgtg gctccccagc 5760
agtggactgg cggccgtcaa tgtctcctac ttcttcaacg ggcgcctggc cggcctccag 5820
cgcggggcca tgagcgagag gacagacatt gacaagcaag gccggattgt gtcccgaatg 5880
ttcgccgacg ggaaagtctg gagctattcc taccttgaca agtccatggt cctcctgctg 5940
cagagccagc gtcagtacat atttgaatat gactcctctg accgcctcca cgcagtcacc 6000
atgcccagtg tcgcccggca cagcatgtcc acgcacacct ccattggcta catccggaac 6060
atttacaacc caccggaaag caacgcctcg gtcatctttg actacagtga tgacggccgc 6120
atcctgaaga cgtctttcct gggcaccggg cgccaggtgt tctataagta cggaaaactg 6180
tccaagttat cggagatcgt ctacgacagc actgccgtca ccttcggcta tgacgagacc 6240
actggcgtcc tgaagatggt gaatctccaa agcgggggct tctcctgtac catcaggtac 6300
cgaaaggtcg ggcccctcgt ggacaagcag atttacaggt tctctgagga aggcatgatc 6360
aacgccaggt tcgattacac ctaccacgac aacagcttcc gcatcgccag catcaagccc 6420
gtcatcagtg agactcccct tcccgttgac ctctaccgct acgatgagat ttctggcaag 6480
gtggaacact tcggcaagtt cggggtcatc tactacgaca tcaaccagat catcaccact 6540
gccgtcatga cactcagcaa gcactttgac acccatgggc gcatcaagga agtgcagtat 6600
gagatgttcc ggtccctcat gtactggatg acggtgcaat atgacagtat gggcagggtc 6660
atcaagaggg aactgaaact ggggccctat gccaacacca caaagtacac ctatgactac 6720
gacggggacg gccagctcca gagtgtggcc gtcaatgacc ggcctacctg gcgttatagc 6780
tatgacctca atgggaacct gcacctgcta aacccaggaa acagtgctcg cctcatgccg 6840
ttacgctatg acctccgtga ccggataacc aggctagggg acgtgcagta caaaatcgat 6900
gatgatggct atttatgcca gagaggatct gacatctttg aatacaactc caaggccttt 6960
ctaacgagag cgtacaacaa ggccagcggg tggagtgtgc agtaccgcta tgatggcgtg 7020
agccgccggg cttcctacaa gaccaacctg ggccaccacc tacagtactt ctattccgac 7080
ctccaccacc ccacacgtat cacccatgtt tacaaccact ccaactctga gatcacctca 7140

ctctactatg acctccaggg ccacctcttt gccatggaga gcagtagtgg ggaagagtac 7200
tatgttgcct cagataacac cgggactcct ctggctgttt ttagtatcaa tggcctcatg 7260
atcaagcaac tccaatacac agcctatggg gagatttact atgactccaa tccagacttt 7320
cagatggtca tcggcttcca cggaggcctc tacgaccccc tcaccaagct cgttcacttt 7380
acgcagcgtg attatgacgt gctggcagga cggtggacgt cccccgacta caccatgtgg 7440
aggaatgtgg gcaaggagcc agccccttc aacctgtaca tgttcaagaa caacaatcca 7500
ctcagtaatg agctggattt aaagaactac gtgacagacg tgaagagctg gctcgtgatg 7560
tttggatttc agctcagcaa catcattcct ggattcccaa gagccaaaat gtattttgtg 7620
cctcccccct atgaactgtc agagagccaa gcaagtgaga atggacagct cattacaggt 7680
gtccagcaga caacagagag gcataaccag gcctttctgg ctctagaagg acaggtcatc 7740
tctaaaaagc tccatgcagg catccgagag aaagcaggcc actggtttgc tacgaccacg 7800
cccatcatcg gcaaaggcat catgttcgcc atcaaagaag ggcgggtgac cacaggcgtg 7860
tctagcatcg ccagtgagga cagccgcaag gtagcatccg tgttgaacaa cgcctactac 7920
ttggacaaga tgcactacag catcgagggc aaggacacac actacttcgt gaagatcggt 7980
gcagcggacg gtgacctggt tacgctgggg accaccattg ggcgcaaggt gctggagagc 8040
ggggtgaacg tgaccgtgtc acagcccacg ctgctggtga acggcaggac tcgaaggttc 8100
accaacattg aattccagta ctccacgctg ctgctcagca tacgctacgg cctcaccccc 8160
gacacactgg atgaagagaa ggcccgcgtc ctggaccaag cgcgacagag ggccctgggt 8220
actgcctggg ccaaggagca gcagaaagcc agggacggga gagagggcag ccgtctgtgg 8280
acggagggcg agaagcagca actcctgagc acgggacggg tgcaaggtta tgagggctat 8340
tacgtgcttc cggtggaaca gtacccagag ctggcagaca gtagcagcaa catccagttc 8400
ttaagacaga atgagatggg aaagaggtaa caaaataacc tgctgccacc tcttctctgg 8460
gtggctcagc aggagcaact gtgacctcct ctcctaagga gacgaagacc taacaggggc 8520
actgaggccg ggctgcttta ggaccccaag tggcaagaaa gctcacattt tttgagttca 8580
aatgctactg tccaagcgca aagtccctca tcctgaagta gactagagct cggccacaaa 8640
ttctgaggaa aacaaaacta aaggatgaac gaatgaacca acgaacgaa 8689

<210> 67
<211> 8409
<212> DNA
<213> Gallus gallus

<400> 67
atggatataa aagatcgaag acaccgctct ttgacgagag gccggtgcgg gaaggagtgt 60
cgctatacta gttcttcact cgacagtgaa gactgcagag taccagctca gaagtcctac 120
agctccagtg agaccctgaa agcatatggc catgacacga ggatgcacta cggaaatcga 180
gtttcagacc tggttcacag ggagtcggat gagtttccaa ggcaaggaac gaacttcacc 240
cttgcagaac tgggaatctg tgagccctct ccccatcgaa gtggctactg ctcggacata 300
ggaatactcc atcaaggcta ttccttgagc actggctctg atgctgactc agacacggag 360
ggcgggatgt ctccagagca cgcgatcagg ctgtggggaa gagggatcaa atccagccga 420
agttctggcc tgtcaagtcg tgaaaactcg gctctcacgc tcactgactc cgacaatgag 480
aacaagtcag atgaggaaaa cgattttcac acacaccttt ctgagaaatt gaaagacaga 540
cagacaagct ggcagcagct ggctgagaca aagaactctc taatacgtcg tcccattcca 600
cctacatcct cgtctagcct tctcccatct gctcagctgc ccagttctca taatcctcca 660
ccagttagct gccagatgcc attgctagac agcaatacgt cccatcaaat catggacacc 720
aatcctgacg aggagttctc tcctaattca tacctactaa gagcatgttc agggccacag 780
caggcatcca gcagtggccc ttcaaaccat cacagccagt caacgctgag gccacctctc 840
ccccctcctc acaaccactc gctgtcccat catcactcgt ctgccaactc cctcaacagg 900
aactcgctca ccaaccgccg caaccagatc cacgcgcctg ctcccgctcc caatgacctg 960
gcgaccacgc ctgagtctgt gcagctgcag gacagctggg tgctcaacag caacgtgccg 1020
ctggagacca ggcatttctt gtttaagaca tcttctggaa cgactccgct gttcagtagc 1080
tcttcccctg gctacccact gacctcagga acagtttata ctccacctcc caggctgtta 1140
cctagaaata cattttccag gaatgcattc aagctgaaaa agccctccaa gtattgtagc 1200
tggaaatgtg ctgctttatc tgcaattgct gctgcagtcc tgcttgccat cctgctagca 1260
tatttcatag cgatgcacct cctggggctg aactggcagc tgcagcccgc ggacggacac 1320
accttcagca acgggctgcg gccgggcgcg gcgggcgcgg aggacggagc ggcggcgcca 1380
cctgcaggca gaggaccgtg ggtcactagg aatagcagca tagatagtgg agaaacagaa 1440
gttggccgca aggtcaccca agaggtgccc cctggagtgt tctggcggtc tcagatccat 1500
atcagccagc cacagttcct gaagttcaac atatccctag ggaaggatgc tcttttcggt 1560
gtttatataa gaagaggact cccaccatca catgcacagt atgatttcat ggaacgcttg 1620

gatgggaaag agaaatggag tgtggtggaa tccccacggg aacggcgaag tattcagact 1680
cttgttcaga atgaggctgt gtttgttcag tacttggatg tgggtttgtg gcacctggcg 1740
ttttacaatg atggcaagga caaagaagtg gtctccttca gtacagttat tttggattca 1800
gtgcaagact gtccacgtaa ttgtcatggc aatggcgagt gtgtttctgg tgtctgccac 1860
tgttttcccg gatttcatgg agcagattgt gctaaagctg cctgcccggt gctgtgcagt 1920
ggcaatggtc agtactccaa aggaacctgc ttgtgctaca gtggctggaa aggtccggaa 1980
tgtgatgtac ccatcagcca gtgtattgat ccctcgtgtg gaggtcatgg ttcctgcatc 2040
gaagggaact gtgtctgttc cattggctat aaaggagaaa actgtgagga agttgattgc 2100
ttagatccaa catgctccaa tcacggggtc tgtgtgaacg gagaatgtct ctgcagccca 2160
ggctggggtg gaataaactg tgagcttccc agagcccagt gcccagacca gtgcagtggg 2220
catggcacat acctgtctga caccggtctc tgtagctgcg atcccaactg gatgggtccc 2280
gactgctccg ttgaagtgtg ctctgtagac tgtggcaccc atggggtgtg cattggcgga 2340
gcgtgtcgct gtgaagaagg gtggacagga gtggcgtgtg accagcgtgt gtgtcatccc 2400
cggtgtacag agcacggaac ttgtaaagat gggaaatgtg aatgcagaga gggctggaat 2460
ggggagcact gcaccattgg taggcaaacg acaggcaccg aaacagatgg ctgccctgac 2520
ttgtgcaatg gcaacggagg gtgcacgctg ggccagaaca gctggcagtg tgtctgccag 2580
accggctgga gagggcctgg atgcaacgtt gccatggaaa cctcctgtgc cgataacaag 2640
gataacgagg gagatggctt ggttgactgc ctagtcccag attgctgcct ccagtccact 2700
tgtcaaaaca gcctgctgtg ccggggttcc cgcgatcctc ttgacatcat acaacagagc 2760
cattctggtt caccagctgt gaagtcattc tatgatcgaa tcaagctctt agtggggaag 2820
gacagcactc atatcattcc aggagaaaat cccttcaaca gcagccttgt gtctcttata 2880
agaggccaag tggtgactac agatggaacg cctctagttg gggtcaacgt gtcatttgtc 2940
aagtatccaa agtatggcta taccatcact cgtcaggatg gcatgtttga cttggttgct 3000
aacggtggat catccctaac tttgcacttt gaacggcccc catttatgag tcaggaaagg 3060
acagtatggc tgccgtggaa cagcttctat gccatggaca cgcttgtaat gaaaacagag 3120
gagaactcca ttcccagctg tgatctcagt ggctttgtca gacctgatcc agtcatcatt 3180
tcatcaccac tgtcaacttt cttcagtgat gctcctggcc gaaatcctat tgtaccagaa 3240
acccaggttc ttcatgaaga aattgaggtc cctggctcaa gtataaagct gatctacctg 3300
agttcccgta ctgctggata taagtcctta cttaagatca tcatgactca gtcacttgtg 3360
ccactgaatc taatcaaagt tcatttgatg gtagcagtag aagggcatct atttcaaaaa 3420
tcatttctgg catctcccaa cttggcttat acattcatct gggacaaaac agatgcatat 3480
ggtcagaagg tttatgggtt gtcagatgct gtagtttctg tgggttttga atatgagact 3540
tgtcccagtt tgattctgtg ggagaaaagg actgcgctgc tgcaaggatt tgagctagat 3600
ccttccaatc taggaggatg gtctttggat aaacatcatg tactgaatgt caagagtggt 3660
atattgcaca aaggcaatgg agaaaatcag tttctaactc agcagccagc tgtgataacc 3720
agcattatgg ggaatgggcg ccgaagaagc atatcctgtc ctagctgcaa tggtcttgca 3780
gaaggaaata agcttttggc ccctgtagca ctggcagtgg gaattgatgg aagcctcttt 3840
gttggagatt ttaattacat tcggcgtatc ttcccatcca ggaatgtgac tagcatattg 3900
gagctgagaa ataaagagtt taaacatagc aacaatcctg ctcacaaata ctatctggcc 3960
gtggaccccg tttcgggctc cctgtacgta tcagacacca acagccgacg gatatacaaa 4020
gtcaaatctc ttactggcac gaaagacctg gctggtaatt ctgaagtggt agcggggact 4080
ggagagcaat gcctgccctt tgatgaagcc agatgtggag atggagggaa agcagtggac 4140
gcaaccctaa tgagtcctcg aggaattgca gtggataagt atggactcat gtattttgtt 4200
gatgccacta tgattcgaaa agtggatcag aatggaatta tatcaactct gctgggctcc 4260
aatgacctaa ctgccgtccg acctctaagc tgtgattcca gcatggatgt cagccaggta 4320
cggctggagt ggcctactga tctcgctgtc gatcccatgg acaactcact ttatgtccta 4380
gagaacaatg ttattttacg gatcacagaa aaccatcaag ttagcattat tgctggacgc 4440
cccatgcact gccaggttcc tggtatagac tactctctta gcaaactggc tattcattcc 4500
gcacttgaat cagccagtgc cattgccatc tcacacacag gagttcttta catcagtgag 4560
acagatgaaa aaaaaattaa tcggctacgc caggtaacta ccaatggaga aatatgcctt 4620
cttgcagggg cagcttcaga ctgtgattgc aaaaatgatg tcaactgtaa ttgctattct 4680
ggggatgatg ggtatgccac tgatgccatc ttaaattcac catcttcctt agctgtggcc 4740
ccagatggta ccatctacat agctgatctc ggaaatatcc gcattagggc tgtcagtaaa 4800
aacaggccca ttcttaattc ttttaaccaa tatgaagctg catctccagg agaacaggag 4860
ctgtatgtct tcaatgctga tgggattcac cagtacactc tcagccttgt taccggggag 4920
tacttgtaca atttcaccta tagcagtgat aacgatgtca ccgaggtgat ggacagcaat 4980
ggcaactcct tgaaggtccg tcgggatgcc agcggaatgc cccgccattt actgatgcct 5040
gataatcaga ttgtcacgct ggccgttggc actaatggtg gactcaaact agtctcaacg 5100
cagaccctgg aacttggatt aatgacttat aacggaaaca gtggtctctt agcaacgaag 5160
agtgatgaaa caggatggac aacattttat gactatgatc atgaaggcgc cctgaccaat 5220
gtaacacgtc ccactggagt ggtaactagc cttcatcgag aaatggaaaa gtctattacc 5280

atcgacattg agaattctaa tcgggatgat gatgtcacgg tcatcacaaa tctctcctct 5340
gtggaggctt cctatacagt tgttcaagat caagtgagga acagctacca gctctgtaat 5400
aatggtactt tgagagtgat gtatgccaat ggcatgagta ttagctttca cagcgaacct 5460
catgtcctgg ctgggacagt aactcccacc ataggacgat gtaatatttc tctaccaatg 5520
gagaatggtt tgaactcaat tgaatggcgt ctgaggaaag aacagattaa aggcaaagtg 5580
actgtgtttg gaagaaagct cagggttcat ggaaggaatt tgctgtccat tgattacgac 5640
cggaatatac gcacagaaaa aatctacgat gatcaccgca agttcaccct gaggataatt 5700
tacgatcagc tgggacggcc cttcctctgg ctgcccagca gcggcctggc tgccgtcaac 5760
gtgtcctatt tcttcaacgg gcgcctggct gggcttcagc gcggagccat gagcgaaagg 5820
acagacatcg acaagcaagg caggatcata tcgcgcatgt ttgcagatgg gaaggtttgg 5880
agttacacct acctagaaaa atccatggta ctactgcttc agagccagcg gcagtacatc 5940
tttgagtatg attcttcaga ccggctccat gctgttacta tgcctagtgt tgctcggcat 6000
agcatgtcaa ctcacacgtc tgttggctac attaggaata tttataatcc tcctgaaagc 6060
aacgcatcag tgatttttga ttacagtgat gatgggagga ttttgaaaac atcattttta 6120
ggtactggtc gacaagtctt ttacaagtat ggaaagctat ccaaattatc tgaaattgtt 6180
tatgacagta ctgcggttac ttttggatat gatgaaacta caggtgtcct aaaaatggtg 6240
aatttgcaaa gtggaggatt ttcttgtaca atccgctatc gtaaaattgg ccctcttgtt 6300
gacaaacaaa tctacagatt ctctgaagaa ggtatggtca atgcaaggtt tgattataca 6360
tatcacgaca atagttttcg cattgcaagc atcaaaccca tcataagtga gactcctctt 6420
ccagttgatc tttaccgtta tgatgagatt tctggcaaag ttgagcattt tggcaaattt 6480
ggagttattt attatgatat aaatcaaatt attactacag cagttatgac actgagtaag 6540
cactttgata cccacggacg cattaaagaa gttcaatatg agatgttccg atccctgatg 6600
tactggatga ctgtgcaata tgacagcatg ggaagagtaa ctaaaagaga actgaaactt 6660
gggccgtatg ccaacacaac caagtatacc tatgattatg atggagatgg gcaattgcaa 6720
agcgtagcag taaatgatag gcctacctgg cgttacagtt atgacctgaa tggaaatctt 6780
cacctcctga atcctggaaa cagtgttcga ttgatgcccc tgcgctacga cctcagagac 6840
aggattacgc gcttaggtga cataccgtac aaaatcgatg atgacggatt cctgtgtcaa 6900
cgaggctcag atgtatttga gtacaattcc aaaggacttt taacaagagc ttacaacaaa 6960
gcaaatggat ggaacgttca gtaccgttac gacggacttg gccgaagggc ttcctgtaag 7020
actaacctag gacatcatct acagtacttt tatgctgatc ttcacaatcc aacaagagta 7080
acacatgtct acaatcattc caattcagaa attacctctc tgtattatga tctgcaaggc 7140
cacctctttg caatggagag tagcagtggg gaagaatatt atgtcgcctc cgataacacg 7200
ggcactccgc tagccgtatt cagcatcaat ggcctcatga tcaaacagct tcagtacact 7260
gcatacggag agatttatta tgactcaaac cctgatttcc agctggttat tgggttccat 7320
ggagggctgt atgatccttt aaccaaactc gtccatttta cccaaaggga ctacgatgtc 7380
cttgctggac gctggacatc tcctgattac acaatgtgga aaaacattgg tagagaacct 7440
gctcccttca atctgtacat gttcaagagt aacaaccctc tcagcaatga actggatcta 7500
aagaattatg taacagatgt caaaagctgg ctggtgatgt tcggatttca gcttagcaac 7560
attattcctg gcttccctag agcaaaaatg tactttgtgt cacctccata cgagctgact 7620
gagagtcaag cgtgtgaaaa tggacagcta attacaggag tccagcagac aacagaaaga 7680
cacaatcaag ctttcatggc tcttgaggga caggtcatat ctaaaagatt acatgccagt 7740
attagagaaa aagcaggcca ctggtttgca acaagcactc ctattattgg gaaaggaatc 7800
atgtttgctg tgaagaaagg ccgtgtaacc actggcattt ccagtatagc cacagacgat 7860
agcagaaaaa ttgcctctgt ccttaacagt gctcactacc tggaaaaaat gcactacagc 7920
atcgagggga aggatactca ctactttgtc aagataggct cagccgatag cgacctcgtc 7980
accctcgcga tgaccagcgg gaggaaggtc ctggacagcg gagtaaacgt gaccgtctcc 8040
cagccaaccc tccttatcaa cggaaggact cgacggttca caaacatcga gtttcagtat 8100
tccaccctgc tgatcaacat ccgctacggg ctcaccgccg acacgctgga tgaggagaag 8160
gcacgagtgc tagaccaggc tcggcagcga gccctggggt cggcctgggc caaagagcag 8220
cagaaggcac gggatggccg cgagggcagc cgcgtatgga cagacggaga gaagcaacag 8280
cttctgaaca cgggaagggt tcaaggttac gagggatatt atgtcttgcc tgtggagcag 8340
tacccagagc tagcagacag tagcagcaac atccagtttt taagacagaa tgaaatggga 8400
aagaggtaa 8409

<210> 68
<211> 2764
<212> PRT
<213> Drosophila melanogaster

<400> 68

Met Asp Val Lys Asp Arg Arg His Arg Ser Leu Thr Arg Gly Arg Cys
1               5                   10                  15

Gly Lys Glu Cys Arg Tyr Thr Ser Ser Ser Leu Asp Ser Glu Asp Cys
            20                  25                  30

Arg Val Pro Thr Gln Lys Ser Tyr Ser Ser Ser Glu Thr Leu Lys Ala
        35                  40                  45

Tyr Asp His Asp Ser Arg Met His Tyr Gly Asn Arg Val Thr Asp Leu
    50                  55                  60

Val His Arg Glu Ser Asp Glu Phe Ser Arg Gln Gly Thr Asn Phe Thr
65                  70                  75                  80

Leu Ala Glu Leu Gly Ile Cys Glu Pro Ser Pro His Arg Ser Gly Tyr
                85                  90                  95

Cys Ser Asp Met Gly Ile Leu His Gln Gly Tyr Ser Leu Ser Thr Gly
            100                 105                 110

Ser Asp Ala Asp Ser Asp Thr Glu Gly Gly Met Ser Pro Glu His Ala
        115                 120                 125

Ile Arg Leu Trp Gly Arg Gly Ile Lys Ser Arg Arg Ser Ser Gly Leu
    130                 135                 140

Ser Ser Arg Glu Asn Ser Ala Leu Thr Leu Thr Asp Ser Asp Asn Glu
145                 150                 155                 160

Asn Lys Ser Asp Asp Asp Asn Gly Arg Pro Ile Pro Pro Thr Ser Ser
                165                 170                 175

Ser Ser Leu Leu Pro Ser Ala Gln Leu Pro Ser Ser His Asn Pro Pro
            180                 185                 190

Pro Val Ser Cys Gln Met Pro Leu Leu Asp Ser Asn Thr Ser His Gln
        195                 200                 205

Ile Met Asp Thr Asn Pro Asp Glu Glu Phe Ser Pro Asn Ser Tyr Leu
    210                 215                 220

Leu Arg Ala Cys Ser Gly Pro Gln Gln Ala Ser Ser Ser Gly Pro Pro
225                 230                 235                 240

Asn His His Ser Gln Ser Thr Leu Arg Pro Pro Leu Pro Pro Pro His
                245                 250                 255

Asn His Thr Leu Ser His His His Ser Ser Ala Asn Ser Leu Asn Arg
            260                 265                 270

Asn Ser Leu Thr Asn Arg Arg Ser Gln Ile His Ala Pro Ala Pro Ala
        275                 280                 285

Pro Asn Asp Leu Ala Thr Thr Pro Glu Ser Val Gln Leu Gln Asp Ser
    290                 295                 300

Trp Val Leu Asn Ser Asn Val Pro Leu Glu Thr Arg His Phe Leu Phe
305                 310                 315                 320

Lys Thr Ser Ser Gly Ser Thr Pro Leu Phe Ser Ser Ser Ser Pro Gly

                325                 330                 335

Tyr Pro Leu Thr Ser Gly Thr Val Tyr Thr Pro Pro Pro Arg Leu Leu
            340                 345                 350

Pro Arg Asn Thr Phe Ser Arg Lys Ala Phe Lys Leu Lys Lys Pro Ser
        355                 360                 365

Lys Tyr Cys Ser Trp Lys Cys Ala Ala Leu Ser Ala Ile Ala Ala Ala
    370                 375                 380

Leu Leu Leu Ala Ile Leu Leu Ala Tyr Phe Ile Ala Met His Leu Leu
385                 390                 395                 400

Gly Leu Asn Trp Gln Leu Gln Pro Ala Asp Gly His Thr Phe Asn Asn
                405                 410                 415

Gly Val Arg Thr Gly Leu Pro Gly Asn Asp Asp Val Ala Thr Val Pro
            420                 425                 430

Ser Gly Gly Lys Val Pro Trp Ser Leu Lys Asn Ser Ser Ile Asp Ser
        435                 440                 445

Gly Glu Ala Glu Val Gly Arg Arg Val Thr Gln Glu Val Pro Pro Gly
    450                 455                 460

Val Phe Trp Arg Ser Gln Ile His Ile Ser Gln Pro Gln Phe Leu Lys
465                 470                 475                 480

Phe Asn Ile Ser Leu Gly Lys Asp Ala Leu Phe Gly Val Tyr Ile Arg
                485                 490                 495

Arg Gly Leu Pro Pro Ser His Ala Gln Tyr Asp Phe Met Glu Arg Leu
            500                 505                 510

Asp Gly Lys Glu Lys Trp Ser Val Val Glu Ser Pro Arg Glu Arg Arg
        515                 520                 525

Ser Ile Gln Thr Leu Val Gln Asn Glu Ala Val Phe Val Gln Tyr Leu
    530                 535                 540

Asp Val Gly Leu Trp His Leu Ala Phe Tyr Asn Asp Gly Lys Asp Lys
545                 550                 555                 560

Glu Met Val Ser Phe Asn Thr Val Val Leu Asp Ser Val Gln Asp Cys
                565                 570                 575

Pro Arg Asn Cys His Gly Asn Gly Glu Cys Val Ser Gly Leu Cys His
            580                 585                 590

Cys Phe Pro Gly Phe Leu Gly Ala Asp Cys Ala Lys Ala Ala Cys Pro
        595                 600                 605

Val Leu Cys Ser Gly Asn Gly Gln Tyr Ser Lys Gly Thr Cys Gln Cys
    610                 615                 620

Tyr Ser Gly Trp Lys Gly Ala Glu Cys Asp Val Pro Met Asn Gln Cys
625                 630                 635                 640

Ile Asp Pro Ser Cys Gly Gly His Gly Ser Cys Ile Asp Gly Asn Cys
                645                 650                 655

Val Cys Ala Ala Gly Tyr Lys Gly Glu His Cys Glu Glu Val Asp Cys
            660                 665                 670

Leu Asp Pro Thr Cys Ser Ser His Gly Val Cys Val Asn Gly Glu Cys
        675                 680                 685

Leu Cys Ser Pro Gly Trp Gly Gly Leu Asn Cys Glu Leu Ala Arg Val
    690                 695                 700

Gln Cys Pro Asp Gln Cys Ser Gly His Gly Thr Tyr Leu Pro Asp Ser
705                 710                 715                 720

Gly Leu Cys Ser Cys Asp Pro Asn Trp Met Gly Pro Asp Cys Ser Val
                725                 730                 735

Val Cys Ser Val Asp Cys Gly Thr His Gly Val Cys Ile Gly Gly Ala
            740                 745                 750

Cys Arg Cys Glu Glu Gly Trp Thr Gly Ala Ala Cys Asp Gln Arg Val
        755                 760                 765

Cys His Pro Arg Cys Ile Glu His Gly Thr Cys Lys Asp Gly Lys Cys
    770                 775                 780

Glu Cys Arg Glu Gly Trp Asn Gly Glu His Cys Thr Ile Asp Gly Cys
785                 790                 795                 800

Pro Asp Leu Cys Asn Gly Asn Gly Arg Cys Thr Leu Gly Gln Asn Ser
                805                 810                 815

Trp Gln Cys Val Cys Gln Thr Gly Trp Arg Gly Pro Gly Cys Asn Val
            820                 825                 830

Ala Met Glu Thr Ser Cys Ala Asp Asn Lys Asp Asn Glu Gly Asp Gly
        835                 840                 845

Leu Val Asp Cys Leu Asp Pro Asp Cys Cys Leu Gln Ser Ala Cys Gln
    850                 855                 860

Asn Ser Leu Leu Cys Arg Gly Ser Arg Asp Pro Leu Asp Ile Ile Gln
865                 870                 875                 880

Gln Gly Gln Thr Asp Trp Pro Ala Val Lys Ser Phe Tyr Asp Arg Ile
                885                 890                 895

Lys Leu Leu Ala Gly Lys Asp Ser Thr His Ile Ile Pro Gly Asp Asn
            900                 905                 910

Pro Phe Asn Ser Ser Leu Val Ser Leu Ile Arg Gly Gln Val Val Thr
        915                 920                 925

Met Asp Gly Thr Pro Leu Val Gly Val Asn Val Ser Phe Val Lys Tyr
    930                 935                 940

Pro Lys Tyr Gly Tyr Thr Ile Thr Arg Gln Asp Gly Thr Phe Asp Leu
945                 950                 955                 960

Ile Ala Asn Gly Gly Ser Ala Leu Thr Leu His Phe Glu Arg Ala Pro
                965                 970                 975

Phe Met Ser Gln Glu Arg Thr Val Trp Leu Pro Trp Asn Ser Phe Tyr
            980                 985                 990

Ala Met Asp Thr Leu Val Met Lys Thr Glu Glu Asn Ser Ile Pro Ser
        995                 1000                1005

Cys Asp Leu Ser Gly Phe Val Arg Pro Asp Pro Ile Ile Ile Ser Ser
    1010                1015                1020

Pro Leu Ser Thr Phe Phe Ser Ala Ser Pro Ala Ser Asn Pro Ile Val
1025                1030                1035                1040

Pro Glu Thr Gln Val Leu His Glu Glu Ile Glu Leu Pro Gly Thr Asn
                1045                1050                1055

Val Lys Leu Arg Tyr Leu Ser Ser Arg Thr Ala Gly Tyr Lys Ser Leu
            1060                1065                1070

Leu Lys Ile Thr Met Thr Gln Ser Thr Val Pro Leu Asn Leu Ile Arg
        1075                1080                1085

Val His Leu Met Val Ala Val Glu Gly His Leu Phe Gln Lys Ser Phe
    1090                1095                1100

Gln Ala Ser Pro Asn Leu Ala Tyr Thr Phe Ile Trp Asp Lys Thr Asp
1105                1110                1115                1120

Ala Tyr Gly Gln Arg Val Tyr Gly Leu Ser Asp Ala Val Val Ser Val
                1125                1130                1135

Gly Phe Glu Tyr Glu Thr Cys Pro Ser Leu Ile Leu Trp Glu Lys Arg
            1140                1145                1150

Thr Ala Leu Leu Gln Gly Phe Glu Leu Asp Pro Ser Asn Leu Gly Gly
        1155                1160                1165

Trp Ser Leu Asp Lys His His Thr Leu Asn Val Lys Ser Gly Ile Leu
    1170                1175                1180

His Lys Gly Thr Gly Glu Asn Gln Phe Leu Thr Gln Gln Pro Ala Ile
1185                1190                1195                1200

Ile Thr Ser Ile Met Gly Asn Gly Arg Arg Arg Ser Ile Ser Cys Pro
                1205                1210                1215

Ser Cys Asn Gly Leu Ala Glu Gly Asn Lys Leu Leu Ala Pro Val Ala
            1220                1225                1230

Leu Ala Val Gly Ile Asp Gly Ser Leu Phe Val Gly Asp Phe Asn Tyr
        1235                1240                1245

Ile Arg Arg Ile Phe Pro Ser Arg Asn Val Thr Ser Ile Leu Glu Leu
    1250                1255                1260

Arg Asn Lys Glu Phe Lys His Ser Asn Ser Pro Gly His Lys Tyr Tyr
1265                1270                1275                1280

Leu Ala Val Asp Pro Val Thr Gly Ser Leu Tyr Val Ser Asp Thr Asn
                1285                1290                1295

Ser Arg Arg Ile Tyr Arg Val Lys Ser Leu Ser Gly Ala Lys Asp Leu

1300                1305                1310

Ala Gly Asn Ser Glu Val Val Ala Gly Thr Gly Glu Gln Cys Leu Pro
        1315                1320                1325

Phe Asp Glu Ala Arg Cys Gly Asp Gly Gly Lys Ala Val Asp Ala Thr
    1330                1335                1340

Leu Met Ser Pro Arg Gly Ile Ala Val Asp Lys Asn Gly Leu Met Tyr
1345                1350                1355                1360

Phe Val Asp Ala Thr Met Ile Arg Lys Val Asp Gln Asn Gly Ile Ile
                1365                1370                1375

Ser Thr Leu Leu Gly Ser Asn Asp Leu Thr Ala Val Arg Pro Leu Ser
            1380                1385                1390

Cys Asp Ser Ser Met Asp Val Ala Gln Val Arg Leu Glu Trp Pro Thr
        1395                1400                1405

Asp Leu Ala Val Asn Pro Met Asp Asn Ser Leu Tyr Val Leu Glu Asn
    1410                1415                1420

Asn Val Ile Leu Arg Ile Thr Glu Asn His Gln Val Ser Ile Ile Ala
1425                1430                1435                1440

Gly Arg Pro Met His Cys Gln Val Pro Gly Ile Asp Tyr Ser Leu Ser
                1445                1450                1455

Lys Leu Ala Ile His Ser Ala Leu Glu Ser Ala Ser Ala Ile Ala Ile
            1460                1465                1470

Ser His Thr Gly Val Leu Tyr Ile Thr Glu Thr Asp Glu Lys Lys Ile
        1475                1480                1485

Asn Arg Leu Arg Gln Val Thr Thr Asn Gly Glu Ile Cys Leu Leu Ala
    1490                1495                1500

Gly Ala Ala Ser Asp Cys Asp Cys Lys Asn Asp Val Asn Cys Ile Cys
1505                1510                1515                1520

Tyr Ser Gly Asp Asp Ala Tyr Ala Thr Asp Ala Ile Leu Asn Ser Pro
                1525                1530                1535

Ser Ser Leu Ala Val Ala Pro Asp Gly Thr Ile Tyr Ile Ala Asp Leu
            1540                1545                1550

Gly Asn Ile Arg Ile Arg Ala Val Ser Lys Asn Lys Pro Val Leu Asn
        1555                1560                1565

Ala Phe Asn Gln Tyr Glu Ala Ala Ser Pro Gly Glu Gln Glu Leu Tyr
    1570                1575                1580

Val Phe Asn Ala Asp Gly Ile His Gln Tyr Thr Val Ser Leu Val Thr
1585                1590                1595                1600

Gly Glu Tyr Leu Tyr Asn Phe Thr Tyr Ser Ala Asp Asn Asp Val Thr
                1605                1610                1615

Glu Leu Ile Asp Asn Asn Gly Asn Ser Leu Lys Ile Arg Arg Asp Ser
            1620                1625                1630

Ser Gly Met Pro Arg His Leu Leu Met Pro Asp Asn Gln Ile Ile Thr
        1635                1640                1645

Leu Thr Val Gly Thr Asn Gly Gly Leu Lys Ala Val Ser Thr Gln Asn
    1650                1655                1660

Leu Glu Leu Gly Leu Met Thr Tyr Asp Gly Asn Thr Gly Leu Leu Ala
1665                1670                1675                1680

Thr Lys Ser Asp Glu Thr Gly Trp Thr Thr Phe Tyr Asp Tyr Asp His
                1685                1690                1695

Glu Gly Arg Leu Thr Asn Val Thr Arg Pro Thr Gly Val Val Thr Ser
            1700                1705                1710

Leu His Arg Glu Met Glu Lys Ser Ile Thr Ile Asp Ile Glu Asn Ser
        1715                1720                1725

Asn Arg Asp Asp Asp Val Thr Val Ile Thr Asn Leu Ser Ser Val Glu
    1730                1735                1740

Ala Ser Tyr Thr Val Val Gln Asp Gln Val Arg Asn Ser Tyr Gln Leu
1745                1750                1755                1760

Cys Asn Asn Gly Thr Leu Arg Val Met Tyr Ala Asn Gly Met Ala Val
                1765                1770                1775

Ser Phe His Ser Glu Pro His Val Leu Ala Gly Thr Ile Thr Pro Thr
            1780                1785                1790

Ile Gly Arg Cys Asn Ile Ser Leu Pro Met Glu Asn Gly Leu Asn Ser
        1795                1800                1805

Ile Glu Trp Arg Leu Arg Lys Glu Gln Ile Lys Gly Lys Val Thr Ile
    1810                1815                1820

Phe Gly Arg Lys Leu Arg Val His Gly Arg Asn Leu Leu Ser Ile Asp
1825                1830                1835                1840

Tyr Asp Arg Asn Ile Arg Thr Glu Lys Ile Tyr Asp Asp His Arg Lys
                1845                1850                1855

Phe Thr Leu Arg Ile Ile Tyr Asp Gln Val Gly Arg Pro Phe Leu Trp
            1860                1865                1870

Leu Pro Ser Ser Gly Leu Ala Ala Val Asn Val Ser Tyr Phe Phe Asn
        1875                1880                1885

Gly Arg Leu Ala Gly Leu Gln Arg Gly Ala Met Ser Glu Arg Thr Asp
    1890                1895                1900

Ile Asp Lys Gln Gly Arg Ile Val Ser Arg Met Phe Ala Asp Gly Lys
1905                1910                1915                1920

Val Trp Ser Tyr Ser Tyr Leu Asp Lys Ser Met Val Leu Leu Leu Gln
                1925                1930                1935

Ser Gln Arg Gln Tyr Ile Phe Glu Tyr Asp Ser Ser Asp Arg Leu His
            1940                1945                1950

Ala Val Thr Met Pro Ser Val Ala Arg His Ser Met Ser Thr His Thr
        1955                1960                1965

Ser Ile Gly Tyr Ile Arg Asn Ile Tyr Asn Pro Pro Glu Ser Asn Ala
    1970                1975                1980

Ser Val Ile Phe Asp Tyr Ser Asp Asp Gly Arg Ile Leu Lys Thr Ser
1985                1990                1995                2000

Phe Leu Gly Thr Gly Arg Gln Val Phe Tyr Lys Tyr Gly Lys Leu Ser
                2005                2010                2015

Lys Leu Ser Glu Ile Val Tyr Asp Ser Thr Ala Val Thr Phe Gly Tyr
            2020                2025                2030

Asp Glu Thr Thr Gly Val Leu Lys Met Val Asn Leu Gln Ser Gly Gly
        2035                2040                2045

Phe Ser Cys Thr Ile Arg Tyr Arg Lys Val Gly Pro Leu Val Asp Lys
    2050                2055                2060

Gln Ile Tyr Arg Phe Ser Glu Glu Gly Met Ile Asn Ala Arg Phe Asp
2065                2070                2075                2080

Tyr Thr Tyr His Asp Asn Ser Phe Arg Ile Ala Ser Ile Lys Pro Val
                2085                2090                2095

Ile Ser Glu Thr Pro Leu Pro Val Asp Leu Tyr Arg Tyr Asp Glu Ile
            2100                2105                2110

Ser Gly Lys Val Glu His Phe Gly Lys Phe Gly Val Ile Tyr Tyr Asp
        2115                2120                2125

Ile Asn Gln Ile Ile Thr Thr Ala Val Met Thr Leu Ser Lys His Phe
    2130                2135                2140

Asp Thr His Gly Arg Ile Lys Glu Val Gln Tyr Glu Met Phe Arg Ser
2145                2150                2155                2160

Leu Met Tyr Trp Met Thr Val Gln Tyr Asp Ser Met Gly Arg Val Ile
                2165                2170                2175

Lys Arg Glu Leu Lys Leu Gly Pro Tyr Ala Asn Thr Thr Lys Tyr Thr
            2180                2185                2190

Tyr Asp Tyr Asp Gly Asp Gly Gln Leu Gln Ser Val Ala Val Asn Asp
        2195                2200                2205

Arg Pro Thr Trp Arg Tyr Ser Tyr Asp Leu Asn Gly Asn Leu His Leu
    2210                2215                2220

Leu Asn Pro Gly Asn Ser Ala Arg Leu Met Pro Leu Arg Tyr Asp Leu
2225                2230                2235                2240

Arg Asp Arg Ile Thr Arg Leu Gly Asp Val Gln Tyr Lys Ile Asp Asp
                2245                2250                2255

Asp Gly Tyr Leu Cys Gln Arg Gly Ser Asp Ile Phe Glu Tyr Asn Ser
            2260                2265                2270

Lys Gly Leu Leu Thr Arg Ala Tyr Asn Lys Ala Ser Gly Trp Ser Val

2275                2280                2285

Gln Tyr Arg Tyr Asp Gly Val Gly Arg Arg Ala Ser Tyr Lys Thr Asn
    2290                2295                2300

Leu Gly His His Leu Gln Tyr Phe Tyr Ser Asp Leu His Asn Pro Thr
2305                2310                2315                2320

Arg Ile Thr His Val Tyr Asn His Ser Asn Ser Glu Ile Thr Ser Leu
                2325                2330                2335

Tyr Tyr Asp Leu Gln Gly His Leu Phe Ala Met Glu Ser Ser Ser Gly
            2340                2345                2350

Glu Glu Tyr Tyr Val Ala Ser Asp Asn Thr Gly Thr Pro Leu Ala Val
        2355                2360                2365

Tyr Ser Ile Asn Gly Leu Met Ile Lys Gln Leu Gln Tyr Thr Ala Tyr
    2370                2375                2380

Gly Glu Ile Tyr Tyr Asp Ser Asn Pro Asp Phe Gln Met Val Ile Gly
2385                2390                2395                2400

Phe His Gly Gly Leu Tyr Asp Pro Leu Thr Lys Leu Val His Phe Thr
                2405                2410                2415

Gln Arg Asp Tyr Asp Val Leu Ala Gly Arg Trp Thr Ser Pro Asp Tyr
            2420                2425                2430

Thr Met Trp Arg Asn Val Gly Lys Glu Pro Ala Pro Phe Asn Leu Tyr
        2435                2440                2445

Met Phe Lys Asn Asn Asn Pro Leu Ser Asn Glu Leu Asp Leu Lys Asn
    2450                2455                2460

Tyr Val Thr Asp Val Lys Ser Trp Leu Val Met Phe Gly Phe Gln Leu
2465                2470                2475                2480

Ser Asn Ile Ile Pro Gly Phe Pro Arg Ala Lys Met Tyr Phe Val Pro
                2485                2490                2495

Pro Pro Tyr Glu Leu Ser Glu Ser Gln Ala Ser Glu Asn Gly Gln Leu
            2500                2505                2510

Ile Thr Gly Val Gln Gln Thr Thr Glu Arg His Asn Gln Ala Phe Leu
        2515                2520                2525

Ala Leu Glu Gly Gln Val Ile Thr Lys Lys Leu His Ala Ser Ile Arg
    2530                2535                2540

Glu Lys Ala Gly His Trp Phe Ala Thr Thr Thr Pro Ile Ile Gly Lys
2545                2550                2555                2560

Gly Ile Met Phe Ala Ile Lys Glu Gly Arg Val Thr Thr Gly Val Ser
                2565                2570                2575

Ser Ile Ala Ser Glu Asp Ser Arg Lys Val Ala Ser Val Leu Asn Asn
            2580                2585                2590

Ala Tyr Tyr Leu Asp Lys Met His Tyr Ser Ile Glu Gly Lys Asp Thr
        2595                2600                2605

His Tyr Phe Val Lys Ile Gly Ala Ala Asp Gly Asp Leu Val Thr Leu
    2610                2615                2620

Gly Thr Thr Ile Gly Arg Lys Val Leu Glu Ser Gly Val Asn Val Thr
2625                2630                2635                2640

Val Ser Gln Pro Thr Leu Leu Val Asn Gly Arg Thr Arg Arg Phe Thr
                2645                2650                2655

Asn Ile Glu Phe Gln Tyr Ser Thr Leu Leu Leu Ser Ile Arg Tyr Gly
            2660                2665                2670

Leu Thr Pro Asp Thr Leu Asp Glu Glu Lys Ala Arg Val Leu Asp Gln
        2675                2680                2685

Ala Gly Gln Arg Ala Leu Gly Thr Ala Trp Ala Lys Glu Gln Gln Lys
    2690                2695                2700

Ala Arg Asp Gly Arg Glu Gly Ser Arg Leu Trp Thr Glu Gly Glu Lys
2705                2710                2715                2720

Gln Gln Leu Leu Ser Thr Gly Arg Val Gln Gly Tyr Glu Gly Tyr Tyr
                2725                2730                2735

Val Leu Pro Val Glu Gln Tyr Pro Glu Leu Ala Asp Ser Ser Ser Asn
            2740                2745                2750

Ile Gln Phe Leu Arg Gln Asn Glu Met Gly Lys Arg
        2755                2760

<210> 69
<211> 2802
<212> PRT
<213> Gallus gallus

<400> 69
Met Asp Ile Lys Asp Arg Arg His Arg Ser Leu Thr Arg Gly Arg Cys
1               5                   10                  15

Gly Lys Glu Cys Arg Tyr Thr Ser Ser Ser Leu Asp Ser Glu Asp Cys
            20                  25                  30

Arg Val Pro Ala Gln Lys Ser Tyr Ser Ser Ser Glu Thr Leu Lys Ala
        35                  40                  45

Tyr Gly His Asp Thr Arg Met His Tyr Gly Asn Arg Val Ser Asp Leu
    50                  55                  60

Val His Arg Glu Ser Asp Glu Phe Pro Arg Gln Gly Thr Asn Phe Thr
65                  70                  75                  80

Leu Ala Glu Leu Gly Ile Cys Glu Pro Ser Pro His Arg Ser Gly Tyr
                85                  90                  95

Cys Ser Asp Ile Gly Ile Leu His Gln Gly Tyr Ser Leu Ser Thr Gly
            100                 105                 110

Ser Asp Ala Asp Ser Asp Thr Glu Gly Gly Met Ser Pro Glu His Ala
        115                 120                 125

Ile Arg Leu Trp Gly Arg Gly Ile Lys Ser Ser Arg Ser Ser Gly Leu
    130                 135                 140

Ser Ser Arg Glu Asn Ser Ala Leu Thr Leu Thr Asp Ser Asp Asn Glu
145                 150                 155                 160

Asn Lys Ser Asp Glu Glu Asn Asp Phe His Thr His Leu Ser Glu Lys
                165                 170                 175

Leu Lys Asp Arg Gln Thr Ser Trp Gln Gln Leu Ala Glu Thr Lys Asn
            180                 185                 190

Ser Leu Ile Arg Arg Pro Ile Pro Pro Thr Ser Ser Ser Ser Leu Leu
        195                 200                 205

Pro Ser Ala Gln Leu Pro Ser Ser His Asn Pro Pro Pro Val Ser Cys
    210                 215                 220

Gln Met Pro Leu Leu Asp Ser Asn Thr Ser His Gln Ile Met Asp Thr
225                 230                 235                 240

Asn Pro Asp Glu Glu Phe Ser Pro Asn Ser Tyr Leu Leu Arg Ala Cys
                245                 250                 255

Ser Gly Pro Gln Gln Ala Ser Ser Ser Gly Pro Ser Asn His His Ser
            260                 265                 270

Gln Ser Thr Leu Arg Pro Pro Leu Pro Pro Pro His Asn His Ser Leu
        275                 280                 285

Ser His His His Ser Ser Ala Asn Ser Leu Asn Arg Asn Ser Leu Thr
    290                 295                 300

Asn Arg Arg Asn Gln Ile His Ala Pro Ala Pro Ala Pro Asn Asp Leu
305                 310                 315                 320

Ala Thr Thr Pro Glu Ser Val Gln Leu Gln Asp Ser Trp Val Leu Asn
                325                 330                 335

Ser Asn Val Pro Leu Glu Thr Arg His Phe Leu Phe Lys Thr Ser Ser
            340                 345                 350

Gly Thr Thr Pro Leu Phe Ser Ser Ser Ser Pro Gly Tyr Pro Leu Thr
        355                 360                 365

Ser Gly Thr Val Tyr Thr Pro Pro Pro Arg Leu Leu Pro Arg Asn Thr
    370                 375                 380

Phe Ser Arg Asn Ala Phe Lys Leu Lys Lys Pro Ser Lys Tyr Cys Ser
385                 390                 395                 400

Trp Lys Cys Ala Ala Leu Ser Ala Ile Ala Ala Ala Val Leu Leu Ala
                405                 410                 415

Ile Leu Leu Ala Tyr Phe Ile Ala Met His Leu Leu Gly Leu Asn Trp
            420                 425                 430

Gln Leu Gln Pro Ala Asp Gly His Thr Phe Ser Asn Gly Leu Arg Pro
        435                 440                 445

Gly Ala Ala Gly Ala Glu Asp Gly Ala Ala Ala Pro Pro Ala Gly Arg
    450                 455                 460

Gly Pro Trp Val Thr Arg Asn Ser Ser Ile Asp Ser Gly Glu Thr Glu
465                 470                 475                 480

Val Gly Arg Lys Val Thr Gln Glu Val Pro Pro Gly Val Phe Trp Arg
                485                 490                 495

Ser Gln Ile His Ile Ser Gln Pro Gln Phe Leu Lys Phe Asn Ile Ser
            500                 505                 510

Leu Gly Lys Asp Ala Leu Phe Gly Val Tyr Ile Arg Arg Gly Leu Pro
        515                 520                 525

Pro Ser His Ala Gln Tyr Asp Phe Met Glu Arg Leu Asp Gly Lys Glu
    530                 535                 540

Lys Trp Ser Val Val Glu Ser Pro Arg Glu Arg Arg Ser Ile Gln Thr
545                 550                 555                 560

Leu Val Gln Asn Glu Ala Val Phe Val Gln Tyr Leu Asp Val Gly Leu
                565                 570                 575

Trp His Leu Ala Phe Tyr Asn Asp Gly Lys Asp Lys Glu Val Val Ser
            580                 585                 590

Phe Ser Thr Val Ile Leu Asp Ser Val Gln Asp Cys Pro Arg Asn Cys
        595                 600                 605

His Gly Asn Gly Glu Cys Val Ser Gly Val Cys His Cys Phe Pro Gly
    610                 615                 620

Phe His Gly Ala Asp Cys Ala Lys Ala Ala Cys Pro Val Leu Cys Ser
625                 630                 635                 640

Gly Asn Gly Gln Tyr Ser Lys Gly Thr Cys Leu Cys Tyr Ser Gly Trp
                645                 650                 655

Lys Gly Pro Glu Cys Asp Val Pro Ile Ser Gln Cys Ile Asp Pro Ser
            660                 665                 670

Cys Gly Gly His Gly Ser Cys Ile Glu Gly Asn Cys Val Cys Ser Ile
        675                 680                 685

Gly Tyr Lys Gly Glu Asn Cys Glu Glu Val Asp Cys Leu Asp Pro Thr
    690                 695                 700

Cys Ser Asn His Gly Val Cys Val Asn Gly Glu Cys Leu Cys Ser Pro
705                 710                 715                 720

Gly Trp Gly Gly Ile Asn Cys Glu Leu Pro Arg Ala Gln Cys Pro Asp
                725                 730                 735

Gln Cys Ser Gly His Gly Thr Tyr Leu Ser Asp Thr Gly Leu Cys Ser
            740                 745                 750

Cys Asp Pro Asn Trp Met Gly Pro Asp Cys Ser Val Glu Val Cys Ser
        755                 760                 765

Val Asp Cys Gly Thr His Gly Val Cys Ile Gly Gly Ala Cys Arg Cys

770                 775                 780

Glu Glu Gly Trp Thr Gly Val Ala Cys Asp Gln Arg Val Cys His Pro
785                 790                 795                 800

Arg Cys Thr Glu His Gly Thr Cys Lys Asp Gly Lys Cys Glu Cys Arg
                805                 810                 815

Glu Gly Trp Asn Gly Glu His Cys Thr Ile Gly Arg Gln Thr Thr Gly
            820                 825                 830

Thr Glu Thr Asp Gly Cys Pro Asp Leu Cys Asn Gly Asn Gly Arg Cys
        835                 840                 845

Thr Leu Gly Gln Asn Ser Trp Gln Cys Val Cys Gln Thr Gly Trp Arg
    850                 855                 860

Gly Pro Gly Cys Asn Val Ala Met Glu Thr Ser Cys Ala Asp Asn Lys
865                 870                 875                 880

Asp Asn Glu Gly Asp Gly Leu Val Asp Cys Leu Val Pro Asp Cys Cys
                885                 890                 895

Leu Gln Ser Thr Cys Gln Asn Ser Leu Leu Cys Arg Gly Ser Arg Asp
            900                 905                 910

Pro Leu Asp Ile Ile Gln Gln Ser His Ser Gly Ser Pro Ala Val Lys
        915                 920                 925

Ser Phe Tyr Asp Arg Ile Lys Leu Leu Val Gly Lys Asp Ser Thr His
    930                 935                 940

Ile Ile Pro Gly Glu Asn Pro Phe Asn Ser Ser Leu Val Ser Leu Ile
945                 950                 955                 960

Arg Gly Gln Val Val Thr Thr Asp Gly Thr Pro Leu Val Gly Val Asn
                965                 970                 975

Val Ser Phe Val Lys Tyr Pro Lys Tyr Gly Tyr Thr Ile Thr Arg Gln
            980                 985                 990

Asp Gly Met Phe Asp Leu Val Ala Asn Gly Gly Ser Ser Leu Thr Leu
        995                 1000                1005

His Phe Glu Arg Ala Pro Phe Met Ser Gln Glu Arg Thr Val Trp Leu
    1010                1015                1020

Pro Trp Asn Ser Phe Tyr Ala Met Asp Thr Leu Val Met Lys Thr Glu
1025                1030                1035                1040

Glu Asn Ser Ile Pro Ser Cys Asp Leu Ser Gly Phe Val Arg Pro Asp
                1045                1050                1055

Pro Val Ile Ile Ser Ser Pro Leu Ser Thr Phe Phe Ser Asp Ala Pro
            1060                1065                1070

Gly Arg Asn Pro Ile Val Pro Glu Thr Gln Val Leu His Glu Glu Ile
        1075                1080                1085

Glu Val Pro Gly Ser Ser Ile Lys Leu Ile Tyr Leu Ser Ser Arg Thr
    1090                1095                1100

Ala Gly Tyr Lys Ser Leu Leu Lys Ile Ile Met Thr Gln Ser Leu Val
1105                1110                1115                1120

Pro Leu Asn Leu Ile Lys Val His Leu Met Val Ala Val Glu Gly His
                1125                1130                1135

Leu Phe Gln Lys Ser Phe Leu Ala Ser Pro Asn Leu Ala Tyr Thr Phe
            1140                1145                1150

Ile Trp Asp Lys Thr Asp Ala Tyr Gly Gln Lys Val Tyr Gly Leu Ser
        1155                1160                1165

Asp Ala Val Val Ser Val Gly Phe Glu Tyr Glu Thr Cys Pro Ser Leu
    1170                1175                1180

Ile Leu Trp Glu Lys Arg Thr Ala Leu Leu Gln Gly Phe Glu Leu Asp
1185                1190                1195                1200

Pro Ser Asn Leu Gly Gly Trp Ser Leu Asp Lys His His Val Leu Asn
                1205                1210                1215

Val Lys Ser Gly Ile Leu His Lys Gly Asn Gly Glu Asn Gln Phe Leu
            1220                1225                1230

Thr Gln Gln Pro Ala Val Ile Thr Ser Ile Met Gly Asn Gly Arg Arg
        1235                1240                1245

Arg Ser Ile Ser Cys Pro Ser Cys Asn Gly Leu Ala Glu Gly Asn Lys
    1250                1255                1260

Leu Leu Ala Pro Val Ala Leu Ala Val Gly Ile Asp Gly Ser Leu Phe
1265                1270                1275                1280

Val Gly Asp Phe Asn Tyr Ile Arg Arg Ile Phe Pro Ser Arg Asn Val
                1285                1290                1295

Thr Ser Ile Leu Glu Leu Arg Asn Lys Glu Phe Lys His Ser Asn Asn
            1300                1305                1310

Pro Ala His Lys Tyr Tyr Leu Ala Val Asp Pro Val Ser Gly Ser Leu
        1315                1320                1325

Tyr Val Ser Asp Thr Asn Ser Arg Arg Ile Tyr Lys Val Lys Ser Leu
    1330                1335                1340

Thr Gly Thr Lys Asp Leu Ala Gly Asn Ser Glu Val Val Ala Gly Thr
1345                1350                1355                1360

Gly Glu Gln Cys Leu Pro Phe Asp Glu Ala Arg Cys Gly Asp Gly Gly
                1365                1370                1375

Lys Ala Val Asp Ala Thr Leu Met Ser Pro Arg Gly Ile Ala Val Asp
            1380                1385                1390

Lys Tyr Gly Leu Met Tyr Phe Val Asp Ala Thr Met Ile Arg Lys Val
        1395                1400                1405

Asp Gln Asn Gly Ile Ile Ser Thr Leu Leu Gly Ser Asn Asp Leu Thr
    1410                1415                1420

Ala Val Arg Pro Leu Ser Cys Asp Ser Ser Met Asp Val Ser Gln Val
1425                1430                1435                1440

Arg Leu Glu Trp Pro Thr Asp Leu Ala Val Asp Pro Met Asp Asn Ser
                1445                1450                1455

Leu Tyr Val Leu Glu Asn Asn Val Ile Leu Arg Ile Thr Glu Asn His
            1460                1465                1470

Gln Val Ser Ile Ile Ala Gly Arg Pro Met His Cys Gln Val Pro Gly
        1475                1480                1485

Ile Asp Tyr Ser Leu Ser Lys Leu Ala Ile His Ser Ala Leu Glu Ser
    1490                1495                1500

Ala Ser Ala Ile Ala Ile Ser His Thr Gly Val Leu Tyr Ile Ser Glu
1505                1510                1515                1520

Thr Asp Glu Lys Lys Ile Asn Arg Leu Arg Gln Val Thr Thr Asn Gly
                1525                1530                1535

Glu Ile Cys Leu Leu Ala Gly Ala Ala Ser Asp Cys Asp Cys Lys Asn
            1540                1545                1550

Asp Val Asn Cys Asn Cys Tyr Ser Gly Asp Asp Gly Tyr Ala Thr Asp
        1555                1560                1565

Ala Ile Leu Asn Ser Pro Ser Ser Leu Ala Val Ala Pro Asp Gly Thr
    1570                1575                1580

Ile Tyr Ile Ala Asp Leu Gly Asn Ile Arg Ile Arg Ala Val Ser Lys
1585                1590                1595                1600

Asn Arg Pro Ile Leu Asn Ser Phe Asn Gln Tyr Glu Ala Ala Ser Pro
                1605                1610                1615

Gly Glu Gln Glu Leu Tyr Val Phe Asn Ala Asp Gly Ile His Gln Tyr
            1620                1625                1630

Thr Leu Ser Leu Val Thr Gly Glu Tyr Leu Tyr Asn Phe Thr Tyr Ser
        1635                1640                1645

Ser Asp Asn Asp Val Thr Glu Val Met Asp Ser Asn Gly Asn Ser Leu
    1650                1655                1660

Lys Val Arg Arg Asp Ala Ser Gly Met Pro Arg His Leu Leu Met Pro
1665                1670                1675                1680

Asp Asn Gln Ile Val Thr Leu Ala Val Gly Thr Asn Gly Gly Leu Lys
                1685                1690                1695

Leu Val Ser Thr Gln Thr Leu Glu Leu Gly Leu Met Thr Tyr Asn Gly
            1700                1705                1710

Asn Ser Gly Leu Leu Ala Thr Lys Ser Asp Glu Thr Gly Trp Thr Thr
        1715                1720                1725

Phe Tyr Asp Tyr Asp His Glu Gly Arg Leu Thr Asn Val Thr Arg Pro
    1730                1735                1740

Thr Gly Val Val Thr Ser Leu His Arg Glu Met Glu Lys Ser Ile Thr

1745                1750                1755                1760

Ile Asp Ile Glu Asn Ser Asn Arg Asp Asp Asp Val Thr Val Ile Thr
                1765                1770                1775

Asn Leu Ser Ser Val Glu Ala Ser Tyr Thr Val Val Gln Asp Gln Val
            1780                1785                1790

Arg Asn Ser Tyr Gln Leu Cys Asn Asn Gly Thr Leu Arg Val Met Tyr
        1795                1800                1805

Ala Asn Gly Met Ser Ile Ser Phe His Ser Glu Pro His Val Leu Ala
    1810                1815                1820

Gly Thr Val Thr Pro Thr Ile Gly Arg Cys Asn Ile Ser Leu Pro Met
1825                1830                1835                1840

Glu Asn Gly Leu Asn Ser Ile Glu Trp Arg Leu Arg Lys Glu Gln Ile
                1845                1850                1855

Lys Gly Lys Val Thr Val Phe Gly Arg Lys Leu Arg Val His Gly Arg
            1860                1865                1870

Asn Leu Leu Ser Ile Asp Tyr Asp Arg Asn Ile Arg Thr Glu Lys Ile
        1875                1880                1885

Tyr Asp Asp His Arg Lys Phe Thr Leu Arg Ile Ile Tyr Asp Gln Leu
    1890                1895                1900

Gly Arg Pro Phe Leu Trp Leu Pro Ser Ser Gly Leu Ala Ala Val Asn
1905                1910                1915                1920

Val Ser Tyr Phe Phe Asn Gly Arg Leu Ala Gly Leu Gln Arg Gly Ala
                1925                1930                1935

Met Ser Glu Arg Thr Asp Ile Asp Lys Gln Gly Arg Ile Ile Ser Arg
            1940                1945                1950

Met Phe Ala Asp Gly Lys Val Trp Ser Tyr Thr Tyr Leu Glu Lys Ser
        1955                1960                1965

Met Val Leu Leu Leu Gln Ser Gln Arg Gln Tyr Ile Phe Glu Tyr Asp
    1970                1975                1980

Ser Ser Asp Arg Leu His Ala Val Thr Met Pro Ser Val Ala Arg His
1985                1990                1995                2000

Ser Met Ser Thr His Thr Ser Val Gly Tyr Ile Arg Asn Ile Tyr Asn
                2005                2010                2015

Pro Pro Glu Ser Asn Ala Ser Val Ile Phe Asp Tyr Ser Asp Asp Gly
            2020                2025                2030

Arg Ile Leu Lys Thr Ser Phe Leu Gly Thr Gly Arg Gln Val Phe Tyr
        2035                2040                2045

Lys Tyr Gly Lys Leu Ser Lys Leu Ser Glu Ile Val Tyr Asp Ser Thr
    2050                2055                2060

Ala Val Thr Phe Gly Tyr Asp Glu Thr Thr Gly Val Leu Lys Met Val
2065                2070                2075                2080

Asn Leu Gln Ser Gly Gly Phe Ser Cys Thr Ile Arg Tyr Arg Lys Ile
                2085                2090                2095

Gly Pro Leu Val Asp Lys Gln Ile Tyr Arg Phe Ser Glu Glu Gly Met
            2100                2105                2110

Val Asn Ala Arg Phe Asp Tyr Thr Tyr His Asp Asn Ser Phe Arg Ile
        2115                2120                2125

Ala Ser Ile Lys Pro Ile Ile Ser Glu Thr Pro Leu Pro Val Asp Leu
    2130                2135                2140

Tyr Arg Tyr Asp Glu Ile Ser Gly Lys Val Glu His Phe Gly Lys Phe
2145                2150                2155                2160

Gly Val Ile Tyr Tyr Asp Ile Asn Gln Ile Ile Thr Thr Ala Val Met
                2165                2170                2175

Thr Leu Ser Lys His Phe Asp Thr His Gly Arg Ile Lys Glu Val Gln
            2180                2185                2190

Tyr Glu Met Phe Arg Ser Leu Met Tyr Trp Met Thr Val Gln Tyr Asp
        2195                2200                2205

Ser Met Gly Arg Val Thr Lys Arg Glu Leu Lys Leu Gly Pro Tyr Ala
    2210                2215                2220

Asn Thr Thr Lys Tyr Thr Tyr Asp Tyr Asp Gly Asp Gly Gln Leu Gln
2225                2230                2235                2240

Ser Val Ala Val Asn Asp Arg Pro Thr Trp Arg Tyr Ser Tyr Asp Leu
                2245                2250                2255

Asn Gly Asn Leu His Leu Leu Asn Pro Gly Asn Ser Val Arg Leu Met
            2260                2265                2270

Pro Leu Arg Tyr Asp Leu Arg Asp Arg Ile Thr Arg Leu Gly Asp Ile
        2275                2280                2285

Pro Tyr Lys Ile Asp Asp Asp Gly Phe Leu Cys Gln Arg Gly Ser Asp
    2290                2295                2300

Val Phe Glu Tyr Asn Ser Lys Gly Leu Leu Thr Arg Ala Tyr Asn Lys
2305                2310                2315                2320

Ala Asn Gly Trp Asn Val Gln Tyr Arg Tyr Asp Gly Leu Gly Arg Arg
                2325                2330                2335

Ala Ser Cys Lys Thr Asn Leu Gly His His Leu Gln Tyr Phe Tyr Ala
            2340                2345                2350

Asp Leu His Asn Pro Thr Arg Val Thr His Val Tyr Asn His Ser Asn
        2355                2360                2365

Ser Glu Ile Thr Ser Leu Tyr Tyr Asp Leu Gln Gly His Leu Phe Ala
    2370                2375                2380

Met Glu Ser Ser Ser Gly Glu Glu Tyr Tyr Val Ala Ser Asp Asn Thr
2385                2390                2395                2400

Gly Thr Pro Leu Ala Val Phe Ser Ile Asn Gly Leu Met Ile Lys Gln
                2405                2410                2415

Leu Gln Tyr Thr Ala Tyr Gly Glu Ile Tyr Tyr Asp Ser Asn Pro Asp
            2420                2425                2430

Phe Gln Leu Val Ile Gly Phe His Gly Gly Leu Tyr Asp Pro Leu Thr
        2435                2440                2445

Lys Leu Val His Phe Thr Gln Arg Asp Tyr Asp Val Leu Ala Gly Arg
    2450                2455                2460

Trp Thr Ser Pro Asp Tyr Thr Met Trp Lys Asn Ile Gly Arg Glu Pro
2465                2470                2475                2480

Ala Pro Phe Asn Leu Tyr Met Phe Lys Ser Asn Asn Pro Leu Ser Asn
                2485                2490                2495

Glu Leu Asp Leu Lys Asn Tyr Val Thr Asp Val Lys Ser Trp Leu Val
            2500                2505                2510

Met Phe Gly Phe Gln Leu Ser Asn Ile Ile Pro Gly Phe Pro Arg Ala
        2515                2520                2525

Lys Met Tyr Phe Val Ser Pro Pro Tyr Glu Leu Thr Glu Ser Gln Ala
    2530                2535                2540

Cys Glu Asn Gly Gln Leu Ile Thr Gly Val Gln Gln Thr Thr Glu Arg
2545                2550                2555                2560

His Asn Gln Ala Phe Met Ala Leu Glu Gly Gln Val Ile Ser Lys Arg
                2565                2570                2575

Leu His Ala Ser Ile Arg Glu Lys Ala Gly His Trp Phe Ala Thr Ser
            2580                2585                2590

Thr Pro Ile Ile Gly Lys Gly Ile Met Phe Ala Val Lys Lys Gly Arg
        2595                2600                2605

Val Thr Thr Gly Ile Ser Ser Ile Ala Thr Asp Asp Ser Arg Lys Ile
    2610                2615                2620

Ala Ser Val Leu Asn Ser Ala His Tyr Leu Glu Lys Met His Tyr Ser
2625                2630                2635                2640

Ile Glu Gly Lys Asp Thr His Tyr Phe Val Lys Ile Gly Ser Ala Asp
                2645                2650                2655

Ser Asp Leu Val Thr Leu Ala Met Thr Ser Gly Arg Lys Val Leu Asp
            2660                2665                2670

Ser Gly Val Asn Val Thr Val Ser Gln Pro Thr Leu Leu Ile Asn Gly
        2675                2680                2685

Arg Thr Arg Arg Phe Thr Asn Ile Glu Phe Gln Tyr Ser Thr Leu Leu
    2690                2695                2700

Ile Asn Ile Arg Tyr Gly Leu Thr Ala Asp Thr Leu Asp Glu Glu Lys
2705                2710                2715                2720

Ala Arg Val Leu Asp Gln Ala Arg Gln Arg Ala Leu Gly Ser Ala Trp

2725                2730                2735

Ala Lys Glu Gln Gln Lys Ala Arg Asp Gly Arg Glu Gly Ser Arg Val
        2740                2745                2750

Trp Thr Asp Gly Glu Lys Gln Gln Leu Leu Asn Thr Gly Arg Val Gln
    2755                2760                2765

Gly Tyr Glu Gly Tyr Tyr Val Leu Pro Val Glu Gln Tyr Pro Glu Leu
  2770                2775                2780

Ala Asp Ser Ser Ser Asn Ile Gln Phe Leu Arg Gln Asn Glu Met Gly
2785                2790                2795                2800

Lys Arg



<210> 70
<211> 2771
<212> PRT
<213> Mus musculus

<400> 70
Met Asp Val Lys Glu Arg Lys Pro Tyr Arg Ser Leu Thr Arg Arg Arg
 1               5                  10                  15

Asp Ala Glu Arg Arg Tyr Thr Ser Ser Ser Ala Asp Ser Glu Glu Gly
            20                  25                  30

Lys Gly Pro Gln Lys Ser Tyr Ser Ser Ser Glu Thr Leu Lys Ala Tyr
        35                  40                  45

Asp Gln Asp Ala Arg Leu Ala Tyr Gly Ser Arg Val Lys Asp Met Val
    50                  55                  60

Pro Gln Glu Ala Glu Glu Phe Cys Arg Thr Gly Thr Asn Phe Thr Leu
65                  70                  75                  80

Arg Glu Leu Gly Leu Gly Glu Met Thr Pro Pro His Gly Thr Leu Tyr
                85                  90                  95

Arg Thr Asp Ile Gly Leu Pro His Cys Gly Tyr Ser Met Gly Ala Ser
            100                 105                 110

Ser Asp Ala Asp Leu Glu Ala Asp Thr Val Leu Ser Pro Glu His Pro
        115                 120                 125

Val Arg Leu Trp Gly Arg Ser Thr Arg Ser Gly Arg Ser Ser Cys Leu
    130                 135                 140

Ser Ser Arg Ala Asn Ser Asn Leu Thr Leu Thr Asp Thr Glu His Glu
145                 150                 155                 160

Asn Thr Glu Thr Asp His Pro Ser Ser Leu Gln Asn His Pro Arg Leu
                165                 170                 175

Arg Thr Pro Pro Pro Pro Leu Pro His Ala His Thr Pro Asn Gln His
            180                 185                 190

His Ala Ala Ser Ile Asn Ser Leu Asn Arg Gly Asn Phe Thr Pro Arg

195                 200                 205

Ser Asn Pro Ser Pro Ala Pro Thr Asp His Ser Leu Ser Gly Glu Pro
    210                 215                 220

Pro Ala Gly Ser Ala Gln Glu Pro Thr His Ala Gln Asp Asn Trp Leu
225                 230                 235                 240

Leu Asn Ser Asn Ile Pro Leu Glu Thr Arg Asn Leu Gly Lys Gln Pro
                245                 250                 255

Phe Leu Gly Thr Leu Gln Asp Asn Leu Ile Glu Met Asp Ile Leu Ser
            260                 265                 270

Ala Ser Arg His Asp Gly Ala Tyr Ser Asp Gly His Phe Leu Phe Lys
        275                 280                 285

Pro Gly Gly Thr Ser Pro Leu Phe Cys Thr Thr Ser Pro Gly Tyr Pro
    290                 295                 300

Leu Thr Ser Ser Thr Val Tyr Ser Pro Pro Pro Arg Pro Leu Pro Arg
305                 310                 315                 320

Ser Thr Phe Ser Arg Pro Ala Phe Asn Leu Lys Lys Pro Ser Lys Tyr
                325                 330                 335

Cys Asn Trp Lys Cys Ala Ala Leu Ser Ala Ile Leu Ile Ser Ala Thr
            340                 345                 350

Leu Val Ile Leu Leu Ala Tyr Phe Val Ala Met His Leu Phe Gly Leu
        355                 360                 365

Asn Trp His Leu Gln Pro Met Glu Gly Gln Met Gln Met Tyr Glu Ile
    370                 375                 380

Thr Glu Asp Thr Ala Ser Ser Trp Pro Val Pro Thr Asp Val Ser Leu
385                 390                 395                 400

Tyr Pro Ser Gly Gly Thr Gly Leu Glu Thr Pro Asp Arg Lys Gly Lys
                405                 410                 415

Gly Ala Ala Glu Gly Lys Pro Ser Ser Leu Phe Pro Glu Asp Ser Phe
            420                 425                 430

Ile Asp Ser Gly Glu Ile Asp Val Gly Arg Arg Ala Ser Gln Lys Ile
        435                 440                 445

Pro Pro Gly Thr Phe Trp Arg Ser Gln Val Phe Ile Asp His Pro Val
    450                 455                 460

His Leu Lys Phe Asn Val Ser Leu Gly Lys Ala Ala Leu Val Gly Ile
465                 470                 475                 480

Tyr Gly Arg Lys Gly Leu Pro Pro Ser His Thr Gln Phe Asp Phe Val
                485                 490                 495

Glu Leu Leu Asp Gly Arg Arg Leu Leu Thr Gln Glu Ala Arg Ser Leu
            500                 505                 510

Glu Gly Pro Gln Arg Gln Ser Arg Gly Pro Val Pro Pro Ser Ser His
        515                 520                 525

Glu Thr Gly Phe Ile Gln Tyr Leu Asp Ser Gly Ile Trp His Leu Ala
    530                 535                 540

Phe Tyr Asn Asp Gly Lys Glu Ser Glu Val Val Ser Phe Leu Thr Thr
545                 550                 555                 560

Ala Ile Glu Ser Val Asp Asn Cys Pro Ser Asn Cys Tyr Gly Asn Gly
                565                 570                 575

Asp Cys Ile Ser Gly Thr Cys His Cys Phe Leu Gly Phe Leu Gly Pro
            580                 585                 590

Asp Cys Gly Arg Ala Ser Cys Pro Val Leu Cys Ser Gly Asn Gly Gln
        595                 600                 605

Tyr Met Lys Gly Arg Cys Leu Cys His Ser Gly Trp Lys Gly Ala Glu
    610                 615                 620

Cys Asp Val Pro Thr Asn Gln Cys Ile Asp Val Ala Cys Ser Ser His
625                 630                 635                 640

Gly Thr Cys Ile Met Gly Thr Cys Ile Cys Asn Pro Gly Tyr Lys Gly
                645                 650                 655

Glu Ser Cys Glu Glu Val Asp Cys Met Asp Pro Thr Cys Ser Ser Arg
            660                 665                 670

Gly Val Cys Val Arg Gly Glu Cys His Cys Ser Val Gly Trp Gly Gly
        675                 680                 685

Thr Asn Cys Glu Thr Pro Arg Ala Thr Cys Leu Asp Gln Cys Ser Gly
    690                 695                 700

His Gly Thr Phe Leu Pro Asp Thr Gly Leu Cys Asn Cys Asp Pro Ser
705                 710                 715                 720

Trp Thr Gly His Asp Cys Ser Ile Glu Ile Cys Ala Ala Asp Cys Gly
                725                 730                 735

Gly His Gly Val Cys Val Gly Gly Thr Cys Arg Cys Glu Asp Gly Trp
            740                 745                 750

Met Gly Ala Ala Cys Asp Gln Arg Ala Cys His Pro Arg Cys Ala Glu
        755                 760                 765

His Gly Thr Cys Arg Asp Gly Lys Cys Glu Cys Ser Pro Gly Trp Asn
    770                 775                 780

Gly Glu His Cys Thr Ile Ala His Tyr Leu Asp Arg Val Val Lys Glu
785                 790                 795                 800

Gly Cys Pro Gly Leu Cys Asn Gly Asn Gly Arg Cys Thr Leu Asp Leu
                805                 810                 815

Asn Gly Trp His Cys Val Cys Gln Leu Gly Trp Arg Gly Thr Gly Cys
            820                 825                 830

Asp Thr Ser Met Glu Thr Gly Cys Gly Asp Gly Lys Asp Asn Asp Gly
        835                 840                 845

Asp Gly Leu Val Asp Cys Met Asp Pro Asp Cys Cys Leu Gln Pro Leu
    850                 855                 860

Cys His Val Asn Pro Leu Cys Leu Gly Ser Pro Asp Pro Leu Asp Ile
865                 870                 875                 880

Ile Gln Glu Thr Gln Ala Pro Val Ser Gln Gln Asn Leu Asn Pro Phe
                885                 890                 895

Tyr Asp Arg Ile Lys Phe Leu Val Gly Arg Asp Ser Thr His Ser Ile
            900                 905                 910

Pro Gly Glu Asn Pro Phe Asp Gly Gly His Ala Cys Val Ile Arg Gly
        915                 920                 925

Gln Val Met Thr Ser Asp Gly Thr Pro Leu Val Gly Val Asn Ile Ser
    930                 935                 940

Phe Ile Asn Asn Pro Leu Phe Gly Tyr Thr Ile Ser Arg Gln Asp Gly
945                 950                 955                 960

Ser Phe Asp Leu Val Thr Asn Gly Gly Ile Ser Ile Ile Leu Arg Phe
                965                 970                 975

Glu Arg Ala Pro Phe Ile Thr Gln Glu His Thr Leu Trp Leu Pro Trp
            980                 985                 990

Asp Arg Phe Phe Val Met Glu Thr Ile Val Met Arg His Glu Glu Asn
        995                 1000                1005

Glu Ile Pro Ser Cys Asp Leu Ser Asn Phe Ala Arg Pro Asn Pro Val
    1010                1015                1020

Val Ser Pro Ser Pro Leu Thr Ser Phe Ala Ser Ser Cys Ala Glu Lys
1025                1030                1035                1040

Gly Pro Ile Val Pro Glu Ile Gln Ala Leu Gln Glu Glu Ile Val Ile
                1045                1050                1055

Ala Gly Cys Lys Met Arg Leu Ser Tyr Leu Ser Ser Arg Thr Pro Gly
            1060                1065                1070

Tyr Lys Ser Val Leu Arg Ile Ser Leu Thr His Pro Thr Ile Pro Phe
        1075                1080                1085

Asn Leu Met Lys Val His Leu Met Val Ala Val Glu Gly Arg Leu Phe
    1090                1095                1100

Arg Lys Trp Phe Ala Ala Ala Pro Asp Leu Ser Tyr Tyr Phe Ile Trp
1105                1110                1115                1120

Asp Lys Thr Asp Val Tyr Asn Gln Lys Val Phe Gly Phe Ser Glu Ala
                1125                1130                1135

Phe Val Ser Val Gly Tyr Glu Tyr Glu Ser Cys Pro Asp Leu Ile Leu
            1140                1145                1150

Trp Glu Lys Arg Thr Ala Val Leu Gln Gly Tyr Glu Ile Asp Ala Ser
        1155                1160                1165

Lys Leu Gly Gly Trp Ser Leu Asp Lys His His Ala Leu Asn Ile Gln

1170                 1175                1180

Ser Gly Ile Leu His Lys Gly Asn Gly Glu Asn Gln Phe Val Ser Gln
1185                1190                1195                1200

Gln Pro Pro Val Ile Gly Ser Ile Met Gly Asn Gly Arg Arg Arg Ser
                1205                1210                1215

Ile Ser Cys Pro Ser Cys Asn Gly Leu Ala Asp Gly Asn Lys Leu Leu
            1220                1225                1230

Ala Pro Val Ala Leu Thr Cys Gly Ser Asp Gly Ser Leu Tyr Val Gly
        1235                1240                1245

Asp Phe Asn Tyr Ile Arg Arg Ile Phe Pro Ser Gly Asn Val Thr Asn
    1250                1255                1260

Ile Leu Glu Met Arg Asn Lys Asp Phe Arg His Ser His Ser Pro Ala
1265                1270                1275                1280

His Lys Tyr Tyr Leu Ala Thr Asp Pro Met Ser Gly Ala Val Phe Leu
                1285                1290                1295

Ser Asp Thr Asn Ser Arg Arg Val Phe Lys Val Lys Ser Thr Thr Val
            1300                1305                1310

Val Lys Asp Leu Val Lys Asn Ser Glu Val Val Ala Gly Thr Gly Asp
        1315                1320                1325

Gln Cys Leu Pro Phe Asp Asp Thr Arg Cys Gly Asp Gly Gly Lys Ala
    1330                1335                1340

Thr Glu Ala Thr Leu Thr Asn Pro Arg Gly Ile Thr Val Asp Lys Phe
1345                1350                1355                1360

Gly Leu Ile Tyr Phe Val Asp Gly Thr Met Ile Arg Arg Val Asp Gln
                1365                1370                1375

Asn Gly Ile Ile Ser Thr Leu Leu Gly Ser Asn Asp Leu Thr Ser Ala
            1380                1385                1390

Arg Pro Leu Ser Cys Asp Ser Val Met Glu Ile Ser Gln Val Arg Leu
        1395                1400                1405

Glu Trp Pro Thr Asp Leu Ala Ile Asn Pro Met Asp Asn Ser Leu Tyr
    1410                1415                1420

Val Leu Asp Asn Asn Val Val Leu Gln Ile Ser Glu Asn His Gln Val
1425                1430                1435                1440

Arg Ile Val Ala Gly Arg Pro Met His Cys Gln Val Pro Gly Ile Asp
                1445                1450                1455

His Phe Leu Leu Ser Lys Val Ala Ile His Ala Thr Leu Glu Ser Ala
            1460                1465                1470

Thr Ala Leu Ala Val Ser His Asn Gly Val Leu Tyr Ile Ala Glu Thr
        1475                1480                1485

Asp Glu Lys Lys Ile Asn Arg Ile Arg Gln Val Thr Thr Ser Gly Glu
    1490                1495                1500

Ile Ser Leu Val Ala Gly Ala Pro Ser Gly Cys Asp Cys Lys Asn Asp
1505                1510                1515                1520

Ala Asn Cys Asp Cys Phe Ser Gly Asp Asp Gly Tyr Ala Lys Asp Ala
                1525                1530                1535

Lys Leu Asn Thr Pro Ser Ser Leu Ala Val Cys Ala Asp Gly Glu Leu
            1540                1545                1550

Tyr Val Ala Asp Leu Gly Asn Ile Arg Ile Arg Phe Ile Arg Lys Asn
        1555                1560                1565

Lys Pro Phe Leu Asn Thr Gln Asn Met Tyr Glu Leu Ser Ser Pro Ile
    1570                1575                1580

Asp Gln Glu Leu Tyr Leu Phe Asp Thr Ser Gly Lys His Leu Tyr Thr
1585                1590                1595                1600

Gln Ser Leu Pro Thr Gly Asp Tyr Leu Tyr Asn Phe Thr Tyr Thr Gly
                1605                1610                1615

Asp Gly Asp Ile Thr His Ile Thr Asp Asn Asn Gly Asn Met Val Asn
            1620                1625                1630

Val Arg Arg Asp Ser Thr Gly Met Pro Leu Trp Leu Val Val Pro Asp
        1635                1640                1645

Gly Gln Val Tyr Trp Val Thr Met Gly Thr Asn Ser Ala Leu Arg Ser
    1650                1655                1660

Val Thr Thr Gln Gly His Glu Leu Ala Met Met Thr Tyr His Gly Asn
1665                1670                1675                1680

Ser Gly Leu Leu Ala Thr Lys Ser Asn Glu Asn Gly Trp Thr Thr Phe
                1685                1690                1695

Tyr Glu Tyr Asp Ser Phe Gly Arg Leu Thr Asn Val Thr Phe Pro Thr
            1700                1705                1710

Gly Gln Val Ser Ser Phe Arg Ser Asp Thr Asp Ser Ser Val His Val
        1715                1720                1725

Gln Val Glu Thr Ser Ser Lys Asp Asp Val Thr Ile Thr Thr Asn Leu
    1730                1735                1740

Ser Ala Ser Gly Ala Phe Tyr Thr Leu Leu Gln Asp Gln Val Arg Asn
1745                1750                1755                1760

Ser Tyr Tyr Ile Gly Ala Asp Gly Ser Leu Arg Leu Leu Leu Ala Asn
                1765                1770                1775

Gly Met Glu Val Ala Leu Gln Thr Glu Pro His Leu Leu Ala Gly Thr
            1780                1785                1790

Val Asn Pro Thr Val Gly Lys Arg Asn Val Thr Leu Pro Ile Asp Asn
        1795                1800                1805

Gly Leu Asn Leu Val Glu Trp Arg Gln Arg Lys Glu Gln Ala Arg Gly
    1810                1815                1820

Gln Val Thr Val Phe Gly Arg Arg Leu Arg Val His Asn Arg Asn Leu
1825                1830                1835                1840

Leu Ser Leu Asp Phe Asp Arg Val Thr Arg Thr Glu Lys Ile Tyr Asp
                1845                1850                1855

Asp His Arg Lys Phe Thr Leu Arg Ile Leu Tyr Asp Gln Ala Gly Arg
            1860                1865                1870

Pro Ser Leu Trp Ser Pro Ser Ser Arg Leu Asn Gly Val Asn Val Thr
        1875                1880                1885

Tyr Ser Pro Gly Gly His Ile Ala Gly Ile Gln Arg Gly Ile Met Ser
    1890                1895                1900

Glu Arg Met Glu Tyr Asp Gln Ala Gly Arg Ile Thr Ser Arg Ile Phe
1905                1910                1915                1920

Ala Asp Gly Lys Met Trp Ser Tyr Thr Tyr Leu Glu Lys Ser Met Val
                1925                1930                1935

Leu His Leu His Ser Gln Arg Gln Tyr Ile Phe Glu Phe Asp Lys Asn
            1940                1945                1950

Asp Arg Leu Ser Ser Val Thr Met Pro Asn Val Ala Arg Gln Thr Leu
        1955                1960                1965

Glu Thr Ile Arg Ser Val Gly Tyr Tyr Arg Asn Ile Tyr Gln Pro Pro
    1970                1975                1980

Glu Gly Asn Ala Ser Val Ile Gln Asp Phe Thr Glu Asp Gly His Leu
1985                1990                1995                2000

Leu His Thr Phe Tyr Leu Gly Thr Gly Arg Arg Val Ile Tyr Lys Tyr
                2005                2010                2015

Gly Lys Leu Ser Lys Leu Ala Glu Thr Leu Tyr Asp Thr Thr Lys Val
            2020                2025                2030

Ser Phe Thr Tyr Asp Glu Thr Ala Gly Met Leu Lys Thr Val Asn Leu
        2035                2040                2045

Gln Asn Glu Gly Phe Thr Cys Thr Ile Arg Tyr Arg Gln Ile Gly Pro
    2050                2055                2060

Leu Ile Asp Arg Gln Ile Phe Arg Phe Thr Glu Glu Gly Met Val Asn
2065                2070                2075                2080

Ala Arg Phe Asp Tyr Asn Tyr Asp Asn Ser Phe Arg Val Thr Ser Met
                2085                2090                2095

Gln Ala Val Ile Asn Glu Thr Pro Leu Pro Ile Asp Leu Tyr Arg Tyr
            2100                2105                2110

Asp Asp Val Ser Gly Lys Thr Glu Gln Phe Gly Lys Phe Gly Val Ile
        2115                2120                2125

Tyr Tyr Asp Ile Asn Gln Ile Ile Thr Thr Ala Val Met Thr His Thr
    2130                2135                2140

Lys His Phe Asp Ala Tyr Gly Arg Met Lys Glu Val Gln Tyr Glu Ile

2145                2150                2155                2160

Phe Arg Ser Leu Met Tyr Trp Met Thr Val Gln Tyr Asp Asn Met Gly
                2165                2170                2175

Arg Val Val Lys Lys Glu Leu Lys Val Gly Pro Tyr Ala Asn Thr Thr
            2180                2185                2190

Arg Tyr Ser Tyr Glu Tyr Asp Ala Asp Gly Gln Leu Gln Thr Val Ser
        2195                2200                2205

Ile Asn Asp Lys Pro Leu Trp Arg Tyr Ser Tyr Asp Leu Asn Gly Asn
    2210                2215                2220

Leu His Leu Leu Ser Pro Gly Asn Ser Ala Arg Leu Thr Pro Leu Arg
2225                2230                2235                2240

Tyr Asp Leu Arg Asp Arg Ile Thr Arg Leu Gly Asp Val Gln Tyr Lys
                2245                2250                2255

Met Asp Glu Asp Gly Phe Leu Arg Gln Arg Gly Gly Asp Val Phe Glu
            2260                2265                2270

Tyr Asn Ser Ala Gly Leu Leu Ile Lys Ala Tyr Asn Arg Ala Ser Gly
        2275                2280                2285

Trp Ser Val Arg Tyr Arg Tyr Asp Gly Leu Gly Arg Arg Val Ser Ser
    2290                2295                2300

Lys Ser Ser His Ser His His Leu Gln Phe Phe Tyr Ala Asp Leu Thr
2305                2310                2315                2320

Asn Pro Thr Lys Val Thr His Leu Tyr Asn His Ser Ser Ser Glu Ile
                2325                2330                2335

Thr Ser Leu Tyr Tyr Asp Leu Gln Gly His Leu Phe Ala Met Glu Leu
            2340                2345                2350

Ser Ser Gly Asp Glu Phe Tyr Ile Ala Cys Asp Asn Ile Gly Thr Pro
        2355                2360                2365

Leu Ala Val Phe Ser Gly Thr Gly Leu Met Ile Lys Gln Ile Leu Tyr
    2370                2375                2380

Thr Ala Tyr Gly Glu Ile Tyr Met Asp Thr Asn Pro Asn Phe Gln Ile
2385                2390                2395                2400

Ile Ile Gly Tyr His Gly Gly Leu Tyr Asp Pro Leu Thr Lys Leu Val
                2405                2410                2415

His Met Gly Arg Arg Asp Tyr Asp Val Leu Ala Gly Arg Trp Thr Ser
            2420                2425                2430

Pro Asp His Glu Leu Trp Lys Arg Leu Ser Ser Asn Ser Ile Val Pro
        2435                2440                2445

Phe His Leu Tyr Met Phe Lys Asn Asn Asn Pro Ile Ser Asn Ser Gln
    2450                2455                2460

Asp Ile Lys Cys Phe Met Thr Asp Val Asn Ser Trp Leu Leu Thr Phe
2465                2470                2475                2480

Gly Phe Gln Leu His Asn Val Ile Pro Gly Tyr Pro Lys Pro Asp Thr
                2485                2490                2495

Asp Ala Met Glu Pro Ser Tyr Glu Leu Val His Thr Gln Met Lys Thr
            2500                2505                2510

Gln Glu Trp Asp Asn Ser Lys Ser Ile Leu Gly Val Gln Cys Glu Val
        2515                2520                2525

Gln Lys Gln Leu Lys Ala Phe Val Thr Leu Glu Arg Phe Asp Gln Leu
    2530                2535                2540

Tyr Gly Ser Thr Ile Thr Ser Cys Gln Gln Ala Pro Glu Thr Lys Lys
2545                2550                2555                2560

Phe Ala Ser Ser Gly Ser Ile Phe Gly Lys Gly Val Lys Phe Ala Leu
                2565                2570                2575

Lys Asp Gly Arg Val Thr Thr Asp Ile Ile Ser Val Ala Asn Glu Asp
            2580                2585                2590

Gly Arg Arg Ile Ala Ala Ile Leu Asn Asn Ala His Tyr Leu Glu Asn
        2595                2600                2605

Leu His Phe Thr Ile Asp Gly Val Asp Thr His Tyr Phe Val Lys Pro
    2610                2615                2620

Gly Pro Ser Glu Gly Asp Leu Ala Ile Leu Gly Leu Ser Gly Gly Arg
2625                2630                2635                2640

Arg Thr Leu Glu Asn Gly Val Asn Val Thr Val Ser Gln Ile Asn Thr
                2645                2650                2655

Met Leu Ser Gly Arg Thr Arg Arg Tyr Thr Asp Ile Gln Leu Gln Tyr
            2660                2665                2670

Arg Ala Leu Cys Leu Asn Thr Arg Tyr Gly Thr Thr Val Asp Glu Glu
        2675                2680                2685

Lys Val Arg Val Leu Glu Leu Ala Arg Gln Arg Ala Val Arg Gln Ala
    2690                2695                2700

Trp Ala Arg Glu Gln Gln Arg Leu Arg Glu Gly Glu Glu Gly Leu Arg
2705                2710                2715                2720

Ala Trp Thr Asp Gly Glu Lys Gln Gln Val Leu Asn Thr Gly Arg Val
                2725                2730                2735

Gln Gly Tyr Asp Gly Phe Phe Val Thr Ser Val Glu Gln Tyr Pro Glu
            2740                2745                2750

Leu Ser Asp Ser Ala Asn Asn Ile His Phe Met Arg Gln Ser Glu Met
        2755                2760                2765

Gly Arg Arg
    2770

<210> 71
<211> 1737

<212> PRT
<213> Homo sapiens

<400> 71
Thr Phe Phe Ser Ala Ala Pro Gly Gln Asn Pro Ile Val Pro Glu Thr
1               5                   10                  15

Gln Val Leu His Glu Glu Ile Glu Leu Pro Gly Ser Asn Val Lys Leu
            20                  25                  30

Arg Tyr Leu Ser Ser Arg Thr Ala Gly Tyr Lys Ser Leu Leu Lys Ile
        35                  40                  45

Thr Met Thr Gln Ser Thr Val Pro Leu Asn Leu Ile Arg Val His Leu
    50                  55                  60

Met Val Ala Val Glu Gly His Leu Phe Gln Lys Ser Phe Gln Ala Ser
65                  70                  75                  80

Pro Asn Leu Ala Tyr Thr Phe Ile Trp Asp Lys Thr Asp Ala Tyr Gly
                85                  90                  95

Gln Arg Val Tyr Gly Leu Ser Asp Ala Val Val Ser Val Gly Phe Glu
            100                 105                 110

Tyr Glu Thr Cys Pro Ser Leu Ile Leu Trp Glu Lys Arg Thr Ala Leu
        115                 120                 125

Leu Gln Gly Phe Glu Leu Asp Pro Ser Asn Leu Gly Gly Trp Ser Leu
    130                 135                 140

Asp Lys His His Ile Leu Asn Val Lys Ser Gly Ile Leu His Lys Gly
145                 150                 155                 160

Thr Gly Glu Asn Gln Phe Leu Thr Gln Gln Pro Ala Ile Ile Thr Ser
                165                 170                 175

Ile Met Gly Asn Gly Arg Arg Arg Ser Ile Ser Cys Pro Ser Cys Asn
            180                 185                 190

Gly Leu Ala Glu Gly Asn Lys Leu Leu Ala Pro Val Ala Leu Ala Val
        195                 200                 205

Gly Ile Asp Gly Ser Leu Tyr Val Gly Asp Phe Asn Tyr Ile Arg Arg
    210                 215                 220

Ile Phe Pro Ser Arg Asn Val Thr Ser Ile Leu Glu Leu Arg Asn Lys
225                 230                 235                 240

Glu Phe Lys His Ser Asn Asn Pro Ala His Lys Tyr Tyr Leu Ala Val
                245                 250                 255

Asp Pro Val Ser Gly Ser Leu Tyr Val Ser Asp Thr Asn Ser Arg Arg
            260                 265                 270

Ile Tyr Arg Val Lys Ser Leu Ser Gly Thr Lys Asp Leu Ala Gly Asn
        275                 280                 285

Ser Glu Val Val Ala Gly Thr Gly Glu Gln Cys Leu Pro Phe Asp Glu
    290                 295                 300

Ala Arg Cys Gly Asp Gly Gly Lys Ala Ile Asp Ala Thr Leu Met Ser
305                 310                 315                 320

Pro Arg Gly Ile Ala Val Asp Lys Asn Gly Leu Met Tyr Phe Val Asp
                325                 330                 335

Ala Thr Met Ile Arg Lys Val Asp Gln Asn Gly Ile Ile Ser Thr Leu
            340                 345                 350

Leu Gly Ser Asn Asp Leu Thr Ala Val Arg Pro Leu Ser Cys Asp Ser
        355                 360                 365

Ser Met Asp Val Ala Gln Val Arg Leu Glu Trp Pro Thr Asp Leu Ala
    370                 375                 380

Val Asn Pro Met Asp Asn Ser Leu Tyr Val Leu Glu Asn Asn Val Ile
385                 390                 395                 400

Leu Arg Ile Thr Glu Asn His Gln Val Ser Ile Ile Ala Gly Arg Pro
                405                 410                 415

Met His Cys Gln Val Pro Gly Ile Asp Tyr Ser Leu Ser Lys Leu Ala
            420                 425                 430

Ile His Ser Ala Leu Glu Ser Ala Ser Ala Ile Ala Ile Ser His Thr
        435                 440                 445

Gly Val Leu Tyr Ile Thr Glu Thr Asp Glu Lys Lys Ile Asn Arg Leu
    450                 455                 460

Arg Gln Val Thr Thr Asn Gly Glu Ile Cys Leu Leu Ala Gly Ala Ala
465                 470                 475                 480

Ser Asp Cys Asp Cys Lys Asn Asp Val Asn Cys Asn Cys Tyr Ser Gly
                485                 490                 495

Asp Asp Ala Tyr Ala Thr Asp Ala Ile Leu Asn Ser Pro Ser Ser Leu
            500                 505                 510

Ala Val Ala Pro Asp Gly Thr Ile Tyr Ile Ala Asp Leu Gly Asn Ile
        515                 520                 525

Arg Ile Arg Ala Val Ser Lys Asn Lys Pro Val Leu Asn Ala Phe Asn
    530                 535                 540

Gln Tyr Glu Ala Ala Ser Pro Gly Glu Gln Glu Leu Tyr Val Phe Asn
545                 550                 555                 560

Ala Asp Gly Ile His Gln Tyr Thr Val Ser Leu Val Thr Gly Glu Tyr
                565                 570                 575

Leu Tyr Asn Phe Thr Tyr Ser Thr Asp Asn Asp Val Thr Glu Leu Ile
            580                 585                 590

Asp Asn Asn Gly Asn Ser Leu Lys Ile Arg Arg Asp Ser Ser Gly Met
        595                 600                 605

Pro Arg His Leu Leu Met Pro Asp Asn Gln Ile Ile Thr Leu Thr Val
    610                 615                 620

Gly Thr Asn Gly Gly Leu Lys Val Val Ser Thr Gln Asn Leu Glu Leu

625                 630                 635                 640

Gly Leu Met Thr Tyr Asp Gly Asn Thr Gly Leu Leu Ala Thr Lys Ser
                645                 650                 655

Asp Glu Thr Gly Trp Thr Thr Phe Tyr Asp Tyr Asp His Glu Gly Arg
            660                 665                 670

Leu Thr Asn Val Thr Arg Pro Thr Gly Val Val Thr Ser Leu His Arg
        675                 680                 685

Glu Met Glu Lys Ser Ile Thr Ile Asp Ile Glu Asn Ser Asn Arg Asp
    690                 695                 700

Asp Asp Val Thr Val Ile Thr Asn Leu Ser Ser Val Glu Ala Ser Tyr
705                 710                 715                 720

Thr Val Val Gln Asp Gln Val Arg Asn Ser Tyr Gln Leu Cys Asn Asn
                725                 730                 735

Gly Thr Leu Arg Val Met Tyr Ala Asn Gly Met Gly Ile Ser Phe His
            740                 745                 750

Ser Glu Pro His Val Leu Ala Gly Thr Ile Thr Pro Thr Ile Gly Arg
        755                 760                 765

Cys Asn Ile Ser Leu Pro Met Glu Asn Gly Leu Asn Ser Ile Glu Trp
    770                 775                 780

Arg Leu Arg Lys Glu Gln Ile Lys Gly Lys Val Thr Ile Phe Gly Arg
785                 790                 795                 800

Lys Leu Arg Val His Gly Arg Asn Leu Leu Ser Ile Asp Tyr Asp Arg
                805                 810                 815

Asn Ile Arg Thr Glu Lys Ile Tyr Asp Asp His Arg Lys Phe Thr Leu
            820                 825                 830

Arg Ile Ile Tyr Asp Gln Val Gly Arg Pro Phe Leu Trp Leu Pro Ser
        835                 840                 845

Ser Gly Leu Ala Ala Val Asn Val Ser Tyr Phe Phe Asn Gly Arg Leu
    850                 855                 860

Ala Gly Leu Gln Arg Gly Ala Met Ser Glu Arg Thr Asp Ile Asp Lys
865                 870                 875                 880

Gln Gly Arg Ile Val Ser Arg Met Phe Ala Asp Gly Lys Val Trp Ser
                885                 890                 895

Tyr Ser Tyr Leu Asp Lys Ser Met Val Leu Leu Leu Gln Ser Gln Arg
            900                 905                 910

Gln Tyr Ile Phe Glu Tyr Asp Ser Ser Asp Arg Leu Leu Ala Val Thr
        915                 920                 925

Met Pro Ser Val Ala Arg His Ser Met Ser Thr His Thr Ser Ile Gly
    930                 935                 940

Tyr Ile Arg Asn Ile Tyr Asn Pro Pro Glu Ser Asn Ala Ser Val Ile
945                 950                 955                 960

Phe Asp Tyr Ser Asp Asp Gly Arg Ile Leu Lys Thr Ser Phe Leu Gly
                965                 970                 975

Thr Gly Arg Gln Val Phe Tyr Lys Tyr Gly Lys Leu Ser Lys Leu Ser
            980                 985                 990

Glu Ile Val Tyr Asp Ser Thr Ala Val Thr Phe Gly Tyr Asp Glu Thr
        995                 1000                1005

Thr Gly Val Leu Lys Met Val Asn Leu Gln Ser Gly Gly Phe Ser Cys
    1010                1015                1020

Thr Ile Arg Tyr Arg Lys Ile Gly Pro Leu Val Asp Lys Gln Ile Tyr
1025                1030                1035                1040

Arg Phe Ser Glu Glu Gly Met Val Asn Ala Arg Phe Asp Tyr Thr Tyr
                1045                1050                1055

His Asp Asn Ser Phe Arg Ile Ala Ser Ile Lys Pro Val Ile Ser Glu
            1060                1065                1070

Thr Pro Leu Pro Val Asp Leu Tyr Arg Tyr Asp Glu Ile Ser Gly Lys
        1075                1080                1085

Val Glu His Phe Gly Lys Phe Gly Val Ile Tyr Tyr Asp Ile Asn Gln
    1090                1095                1100

Ile Ile Thr Thr Ala Val Met Thr Leu Ser Lys His Phe Asp Thr His
1105                1110                1115                1120

Gly Arg Ile Lys Glu Val Gln Tyr Glu Met Phe Arg Ser Leu Met Tyr
                1125                1130                1135

Trp Met Thr Val Gln Tyr Asp Ser Met Gly Arg Val Ile Lys Arg Glu
            1140                1145                1150

Leu Lys Leu Gly Pro Tyr Ala Asn Thr Thr Lys Tyr Thr Tyr Asp Tyr
        1155                1160                1165

Asp Gly Asp Gly Gln Leu Gln Ser Val Ala Val Asn Asp Arg Pro Thr
    1170                1175                1180

Trp Arg Tyr Ser Tyr Asp Leu Asn Gly Asn Leu His Leu Leu Asn Pro
1185                1190                1195                1200

Gly Asn Ser Val Arg Leu Met Pro Leu Arg Tyr Asp Leu Arg Asp Arg
                1205                1210                1215

Ile Thr Arg Leu Gly Asp Val Gln Tyr Lys Ile Asp Asp Asp Gly Tyr
            1220                1225                1230

Leu Cys Gln Arg Gly Ser Asp Ile Phe Glu Tyr Asn Ser Lys Gly Leu
        1235                1240                1245

Leu Thr Arg Ala Tyr Asn Lys Ala Ser Gly Trp Ser Val Gln Tyr Arg
    1250                1255                1260

Tyr Asp Gly Val Gly Arg Arg Ala Ser Tyr Lys Thr Asn Leu Gly His
1265                1270                1275                1280

His Leu Gln Tyr Phe Tyr Ser Asp Leu His Asn Pro Thr Arg Ile Thr
                1285                1290                1295

His Val Tyr Asn His Ser Asn Ser Glu Ile Thr Ser Leu Tyr Tyr Asp
            1300                1305                1310

Leu Gln Gly His Leu Phe Ala Met Glu Ser Ser Ser Gly Glu Glu Tyr
        1315                1320                1325

Tyr Val Ala Ser Asp Asn Thr Gly Thr Pro Leu Ala Val Phe Ser Ile
    1330                1335                1340

Asn Gly Leu Met Ile Lys Gln Leu Gln Tyr Thr Ala Tyr Gly Glu Ile
1345                1350                1355                1360

Tyr Tyr Asp Ser Asn Pro Asp Phe Gln Met Val Ile Gly Phe His Gly
                1365                1370                1375

Gly Leu Tyr Asp Pro Leu Thr Lys Leu Val His Phe Thr Gln Arg Asp
            1380                1385                1390

Tyr Asp Val Leu Ala Gly Arg Trp Thr Ser Pro Asp Tyr Thr Met Trp
        1395                1400                1405

Lys Asn Val Gly Lys Glu Pro Ala Pro Phe Asn Leu Tyr Met Phe Lys
    1410                1415                1420

Ser Asn Asn Pro Leu Ser Ser Glu Leu Asp Leu Lys Asn Tyr Val Thr
1425                1430                1435                1440

Asp Val Lys Ser Trp Leu Val Met Phe Gly Phe Gln Leu Ser Asn Ile
                1445                1450                1455

Ile Pro Gly Phe Pro Arg Ala Lys Met Tyr Phe Val Pro Pro Pro Tyr
            1460                1465                1470

Glu Leu Ser Glu Ser Gln Ala Ser Glu Asn Gly Gln Leu Ile Thr Gly
        1475                1480                1485

Val Gln Gln Thr Thr Glu Arg His Asn Gln Ala Phe Met Ala Leu Glu
    1490                1495                1500

Gly Gln Val Ile Thr Lys Lys Leu His Ala Ser Ile Arg Glu Lys Ala
1505                1510                1515                1520

Gly His Trp Phe Ala Thr Thr Thr Pro Ile Ile Gly Lys Gly Ile Met
                1525                1530                1535

Phe Ala Ile Lys Glu Gly Arg Val Thr Thr Gly Val Ser Ser Ile Ala
            1540                1545                1550

Ser Glu Asp Ser Arg Lys Val Ala Ser Val Leu Asn Asn Ala Tyr Tyr
        1555                1560                1565

Leu Asp Lys Met His Tyr Ser Ile Glu Gly Lys Asp Thr His Tyr Phe
    1570                1575                1580

Val Lys Ile Gly Ser Ala Asp Gly Asp Leu Val Thr Leu Gly Thr Thr
1585                1590                1595                1600

Ile Gly Arg Lys Val Leu Glu Ser Gly Val Asn Val Thr Val Ser Gln

                    1605                1610                1615

Pro Thr Leu Leu Val Asn Gly Arg Thr Arg Arg Phe Thr Asn Ile Glu
            1620                1625                1630

Phe Gln Tyr Ser Thr Leu Leu Leu Ser Ile Arg Tyr Gly Leu Thr Pro
        1635                1640                1645

Asp Thr Leu Asp Glu Glu Lys Ala Arg Val Leu Asp Gln Ala Arg Gln
    1650                1655                1660

Arg Ala Leu Gly Thr Ala Trp Ala Lys Glu Gln Gln Lys Ala Arg Asp
1665                1670                1675                1680

Gly Arg Glu Gly Ser Arg Leu Trp Thr Glu Gly Glu Lys Gln Gln Leu
                1685                1690                1695

Leu Ser Thr Gly Arg Val Gln Gly Tyr Glu Gly Tyr Tyr Val Leu Pro
            1700                1705                1710

Val Glu Gln Tyr Pro Glu Leu Ala Asp Ser Ser Ser Asn Ile Gln Phe
        1715                1720                1725

Leu Arg Gln Asn Glu Met Gly Lys Arg
    1730                1735


<210> 72
<211> 2765
<212> PRT
<213> Rattus norvegicus

<400> 72
Met Asp Val Lys Asp Arg Arg His Arg Ser Leu Thr Arg Gly Arg Cys
1               5                   10                  15

Gly Lys Glu Cys Arg Tyr Thr Ser Ser Ser Leu Asp Ser Glu Asp Cys
            20                  25                  30

Arg Val Pro Thr Gln Lys Ser Tyr Ser Ser Ser Glu Thr Leu Lys Ala
        35                  40                  45

Tyr Asp His Asp Ser Arg Met His Tyr Gly Asn Arg Val Thr Asp Leu
    50                  55                  60

Val His Arg Glu Ser Asp Glu Phe Ser Arg Gln Gly Ala Asn Phe Thr
65                  70                  75                  80

Leu Ala Glu Leu Gly Ile Cys Glu Pro Ser Pro His Arg Ser Gly Tyr
                85                  90                  95

Cys Ser Asp Met Gly Ile Leu His Gln Gly Tyr Ser Leu Ser Thr Gly
            100                 105                 110

Ser Asp Ala Asp Ser Asp Thr Glu Gly Gly Met Ser Pro Glu His Ala
        115                 120                 125

Ile Arg Leu Trp Gly Arg Gly Ile Lys Ser Arg Arg Ser Ser Gly Leu
    130                 135                 140

Ser Ser Arg Glu Asn Ser Ala Leu Thr Leu Thr Asp Ser Asp Asn Glu

145                 150                 155                 160

Asn Lys Ser Asp Asp Asp Asn Gly Arg Pro Ile Pro Pro Thr Ser Ser
                165                 170                 175

Ser Ser Leu Leu Pro Ser Ala Gln Leu Pro Ser Ser His Asn Pro Pro
            180                 185                 190

Pro Val Ser Cys Gln Met Pro Leu Leu Asp Ser Asn Thr Ser His Gln
        195                 200                 205

Ile Met Asp Thr Asn Pro Asp Glu Glu Phe Ser Pro Asn Ser Tyr Leu
    210                 215                 220

Leu Arg Ala Cys Ser Gly Pro Gln Gln Ala Ser Ser Ser Gly Pro Pro
225                 230                 235                 240

Asn His His Ser Gln Ser Thr Leu Arg Pro Pro Leu Pro Pro Pro His
                245                 250                 255

Asn His Thr Leu Ser His His His Ser Ser Ala Asn Ser Leu Asn Arg
            260                 265                 270

Asn Ser Leu Thr Asn Arg Arg Ser Gln Ile His Ala Pro Ala Pro Ala
        275                 280                 285

Pro Asn Asp Leu Ala Thr Thr Pro Glu Ser Val Gln Leu Gln Asp Ser
    290                 295                 300

Trp Val Leu Asn Ser Asn Val Pro Leu Glu Thr Arg His Phe Leu Phe
305                 310                 315                 320

Lys Thr Ser Ser Gly Ser Thr Pro Leu Phe Ser Ser Ser Ser Pro Gly
                325                 330                 335

Tyr Pro Leu Thr Ser Gly Thr Val Tyr Thr Pro Pro Pro Arg Leu Leu
            340                 345                 350

Pro Arg Asn Thr Phe Ser Arg Lys Ala Phe Lys Leu Lys Lys Pro Ser
        355                 360                 365

Lys Tyr Cys Ser Trp Lys Cys Ala Ala Leu Ser Ala Ile Ala Ala Ala
    370                 375                 380

Leu Leu Leu Ala Ile Leu Leu Ala Tyr Phe Ile Ala Met His Leu Leu
385                 390                 395                 400

Gly Leu Asn Trp Gln Leu Gln Pro Ala Asp Gly His Thr Phe Asn Asn
                405                 410                 415

Gly Val Arg Thr Gly Leu Pro Gly Asn Asp Asp Val Ala Thr Val Pro
            420                 425                 430

Ser Gly Gly Lys Val Pro Trp Ser Leu Lys Asn Ser Ser Ile Asp Ser
        435                 440                 445

Gly Glu Ala Glu Val Gly Arg Arg Val Thr Gln Glu Val Pro Pro Gly
    450                 455                 460

Val Phe Trp Arg Ser Gln Ile His Ile Ser Gln Pro Gln Phe Leu Lys
465                 470                 475                 480

Phe Asn Ile Ser Leu Gly Lys Asp Ala Leu Phe Gly Val Tyr Ile Arg
                485                 490                 495

Arg Gly Leu Pro Pro Ser His Ala Gln Tyr Asp Phe Met Glu Arg Leu
            500                 505                 510

Asp Gly Lys Glu Lys Trp Ser Val Val Glu Ser Pro Arg Glu Arg Arg
        515                 520                 525

Ser Ile Gln Thr Leu Val Gln Asn Glu Ala Val Phe Val Gln Tyr Leu
    530                 535                 540

Asp Val Gly Leu Trp His Leu Ala Phe Tyr Asn Asp Gly Lys Asp Lys
545                 550                 555                 560

Glu Met Val Ser Phe Asn Thr Val Val Leu Asp Ser Val Gln Asp Cys
                565                 570                 575

Pro Arg Asn Cys His Gly Asn Gly Glu Cys Val Ser Gly Leu Cys His
            580                 585                 590

Cys Phe Pro Gly Phe Leu Gly Ala Asp Cys Ala Lys Ala Ala Cys Pro
        595                 600                 605

Val Leu Cys Ser Gly Asn Gly Gln Tyr Ser Lys Gly Thr Cys Gln Cys
    610                 615                 620

Tyr Ser Gly Trp Lys Gly Ala Glu Cys Asp Val Pro Met Asn Gln Cys
625                 630                 635                 640

Ile Asp Pro Ser Cys Gly Gly His Gly Ser Cys Ile Asp Gly Asn Cys
                645                 650                 655

Val Cys Ala Ala Gly Tyr Lys Gly Glu His Cys Glu Glu Val Asp Cys
            660                 665                 670

Leu Asp Pro Thr Cys Ser Ser His Gly Val Cys Val Asn Gly Glu Cys
        675                 680                 685

Leu Cys Ser Pro Gly Trp Gly Gly Leu Asn Cys Glu Leu Ala Arg Val
    690                 695                 700

Gln Cys Pro Asp Gln Cys Ser Gly His Gly Thr Tyr Leu Pro Asp Ser
705                 710                 715                 720

Gly Leu Cys Asn Cys Asp Pro Asn Trp Met Gly Pro Asp Cys Ser Val
                725                 730                 735

Glu Val Cys Ser Val Asp Cys Gly Thr His Gly Val Cys Ile Gly Gly
            740                 745                 750

Ala Cys Arg Cys Glu Glu Gly Trp Thr Gly Ala Ala Cys Asp Gln Arg
        755                 760                 765

Val Cys His Pro Arg Cys Ile Glu His Gly Thr Cys Lys Asp Gly Lys
    770                 775                 780

Cys Glu Cys Arg Glu Gly Trp Asn Gly Glu His Cys Thr Ile Asp Gly
785                 790                 795                 800

Cys Pro Asp Leu Cys Asn Gly Asn Gly Arg Cys Thr Leu Gly Gln Asn
                805                 810                 815

Ser Trp Gln Cys Val Cys Gln Thr Gly Trp Arg Gly Pro Gly Cys Asn
            820                 825                 830

Val Ala Met Glu Thr Ser Cys Ala Asp Asn Lys Asp Asn Glu Gly Asp
        835                 840                 845

Gly Leu Val Asp Cys Leu Asp Pro Asp Cys Cys Leu Gln Ser Ala Cys
    850                 855                 860

Gln Asn Ser Leu Leu Cys Arg Gly Ser Arg Asp Pro Leu Asp Ile Ile
865                 870                 875                 880

Gln Gln Gly Gln Thr Asp Trp Pro Ala Val Lys Ser Phe Tyr Asp Arg
                885                 890                 895

Ile Lys Leu Leu Ala Gly Lys Asp Ser Thr His Ile Ile Pro Gly Asp
            900                 905                 910

Asn Pro Phe Asn Ser Ser Leu Val Ser Leu Ile Arg Gly Gln Val Val
        915                 920                 925

Thr Thr Asp Gly Thr Pro Leu Val Gly Val Asn Val Ser Phe Val Lys
    930                 935                 940

Tyr Pro Lys Tyr Gly Tyr Thr Ile Thr Arg Gln Asp Gly Thr Phe Asp
945                 950                 955                 960

Leu Ile Ala Asn Gly Gly Ser Ala Leu Thr Leu His Phe Glu Arg Ala
                965                 970                 975

Pro Phe Met Ser Arg Glu Arg Thr Val Trp Pro Pro Trp Asn Ser Phe
            980                 985                 990

Tyr Ala Met Asp Thr Leu Val Met Lys Thr Glu Glu Asn Ser Ile Pro
        995                 1000                1005

Ser Cys Asp Leu Ser Gly Phe Val Arg Pro Asp Pro Ile Ile Ile Ser
    1010                1015                1020

Ser Pro Leu Ser Thr Phe Phe Ser Ala Ser Pro Ala Ala Asn Pro Ile
1025                1030                1035                1040

Val Pro Glu Thr Gln Val Leu His Glu Glu Ile Glu Leu Pro Gly Thr
                1045                1050                1055

Asn Val Lys Leu Arg Tyr Leu Ser Ser Arg Thr Ala Gly Tyr Lys Ser
            1060                1065                1070

Leu Leu Lys Ile Thr Met Thr Gln Ser Thr Val Pro Leu Asn Leu Ile
        1075                1080                1085

Arg Val His Leu Met Val Ala Val Glu Gly His Leu Phe Gln Lys Ser
    1090                1095                1100

Phe Gln Ala Ser Pro Asn Leu Ala Tyr Thr Phe Ile Trp Asp Lys Thr
1105                1110                1115                1120

Asp Ala Tyr Gly Gln Arg Val Tyr Gly Leu Ser Asp Ala Val Val Ser

1125                 1130                 1135

Val Gly Phe Glu Tyr Glu Thr Cys Pro Ser Leu Ile Leu Trp Glu Lys
        1140                 1145                 1150

Arg Thr Ala Leu Leu Gln Gly Phe Glu Leu Asp Pro Ser Asn Leu Gly
    1155                 1160                 1165

Gly Trp Ser Leu Asp Lys His His Thr Leu Asn Val Lys Ser Gly Ile
  1170                 1175                 1180

Leu Leu Lys Gly Thr Gly Glu Asn Gln Phe Leu Thr Gln Gln Pro Ala
1185                 1190                 1195                 1200

Ile Ile Thr Ser Ile Met Gly Asn Gly Arg Arg Arg Ser Ile Ser Cys
                1205                 1210                 1215

Pro Ser Cys Asn Gly Leu Ala Glu Gly Asn Lys Leu Leu Ala Pro Val
        1220                 1225                 1230

Ala Leu Ala Val Gly Ile Asp Gly Ser Leu Phe Val Gly Asp Phe Asn
    1235                 1240                 1245

Tyr Ile Arg Arg Ile Phe Pro Ser Arg Asn Val Thr Ser Ile Leu Glu
  1250                 1255                 1260

Leu Arg Asn Lys Glu Phe Lys His Ser Asn Ser Pro Gly His Lys Tyr
1265                 1270                 1275                 1280

Tyr Leu Ala Val Asp Pro Val Thr Gly Ser Leu Tyr Val Ser Asp Thr
                1285                 1290                 1295

Asn Ser Arg Arg Ile Tyr Arg Val Lys Ser Leu Ser Gly Ala Lys Asp
        1300                 1305                 1310

Leu Ala Gly Asn Ser Glu Val Val Ala Gly Thr Gly Glu Gln Cys Leu
    1315                 1320                 1325

Pro Phe Asp Glu Ala Arg Cys Gly Asp Gly Gly Lys Ala Val Asp Ala
  1330                 1335                 1340

Thr Leu Met Ser Pro Arg Gly Ile Ala Val Asp Lys Asn Gly Leu Met
1345                 1350                 1355                 1360

Tyr Phe Val Asp Ala Thr Met Ile Arg Lys Val Asp Gln Asn Gly Ile
                1365                 1370                 1375

Ile Ser Thr Leu Leu Gly Ser Asn Asp Leu Thr Ala Val Arg Pro Leu
        1380                 1385                 1390

Ser Cys Asp Ser Ser Met Asp Val Ala Gln Val Arg Leu Glu Trp Pro
    1395                 1400                 1405

Thr Asp Leu Ala Val Asn Pro Met Asp Asn Ser Leu Tyr Val Leu Glu
  1410                 1415                 1420

Asn Asn Val Ile Leu Arg Ile Thr Glu Asn His Gln Val Ser Ile Ile
1425                 1430                 1435                 1440

Ala Gly Arg Pro Met His Cys Gln Val Pro Gly Ile Asp Tyr Ser Leu
                1445                 1450                 1455

Ser Lys Leu Ala Ile His Ser Ala Leu Glu Ser Ala Ser Ala Ile Ala
            1460                1465                1470

Ile Ser His Thr Gly Val Leu Tyr Ile Thr Glu Thr Asp Glu Lys Lys
        1475                1480                1485

Ile Asn Arg Leu Arg Gln Val Thr Thr Asn Gly Glu Ile Cys Leu Leu
    1490                1495                1500

Ala Gly Ala Ala Ser Asp Cys Asp Cys Lys Asn Asp Val Asn Cys Ile
1505                1510                1515                1520

Cys Tyr Ser Gly Asp Asp Ala Tyr Ala Thr Asp Ala Ile Leu Asn Ser
                1525                1530                1535

Pro Ser Ser Leu Ala Val Ala Pro Asp Gly Thr Ile Tyr Ile Ala Asp
            1540                1545                1550

Leu Gly Asn Ile Arg Ile Arg Ala Val Ser Lys Asn Lys Pro Val Leu
        1555                1560                1565

Asn Ala Phe Asn Gln Tyr Glu Ala Ala Ser Pro Gly Glu Gln Glu Leu
    1570                1575                1580

Tyr Val Phe Asn Ala Asp Gly Ile His Gln Tyr Thr Val Ser Leu Val
1585                1590                1595                1600

Thr Gly Glu Tyr Leu Tyr Asn Phe Thr Tyr Ser Ala Asp Asn Asp Val
                1605                1610                1615

Thr Glu Leu Ile Asp Asn Asn Gly Asn Ser Leu Lys Ile Arg Arg Asp
            1620                1625                1630

Ser Ser Gly Met Pro Arg His Leu Leu Met Pro Asp Asn Gln Ile Ile
        1635                1640                1645

Thr Leu Thr Val Gly Thr Asn Gly Gly Leu Lys Ala Val Ser Thr Gln
    1650                1655                1660

Asn Leu Glu Leu Gly Leu Met Thr Tyr Asp Gly Asn Thr Gly Leu Leu
1665                1670                1675                1680

Ala Thr Lys Ser Asp Glu Thr Gly Trp Thr Thr Phe Tyr Asp Tyr Asp
                1685                1690                1695

His Glu Gly Arg Leu Thr Asn Val Thr Arg Pro Thr Gly Val Val Thr
            1700                1705                1710

Ser Leu His Arg Glu Met Glu Lys Ser Ile Thr Val Asp Ile Glu Asn
        1715                1720                1725

Ser Asn Arg Asp Asn Asp Val Thr Val Ile Thr Asn Leu Ser Ser Val
    1730                1735                1740

Glu Ala Ser Tyr Thr Val Val Gln Asp Gln Val Arg Asn Ser Tyr Gln
1745                1750                1755                1760

Leu Cys Ser Asn Gly Thr Leu Arg Val Met Tyr Ala Asn Gly Met Gly
                1765                1770                1775

Val Ser Phe His Ser Glu Pro His Val Leu Ala Gly Thr Leu Thr Pro
            1780                1785                1790

Thr Ile Gly Arg Cys Asn Ile Ser Leu Pro Met Glu Asn Gly Leu Asn
        1795                1800                1805

Ser Ile Glu Trp Arg Leu Arg Lys Glu Gln Ile Lys Gly Lys Val Thr
    1810                1815                1820

Ile Phe Gly Arg Lys Leu Arg Val His Gly Arg Asn Leu Leu Ser Ile
1825                1830                1835                1840

Asp Tyr Asp Arg Asn Ile Arg Thr Glu Lys Ile Tyr Asp Asp His Arg
                1845                1850                1855

Lys Phe Thr Leu Arg Ile Ile Tyr Asp Gln Val Gly Arg Pro Phe Leu
            1860                1865                1870

Trp Leu Pro Ser Ser Gly Leu Ala Ala Val Asn Val Ser Tyr Phe Phe
        1875                1880                1885

Asn Gly Arg Leu Ala Gly Leu Gln Arg Gly Ala Met Ser Glu Arg Thr
    1890                1895                1900

Asp Ile Asp Lys Gln Gly Arg Ile Val Ser Arg Met Phe Ala Asp Gly
1905                1910                1915                1920

Lys Val Trp Ser Tyr Ser Tyr Leu Asp Lys Ser Met Val Leu Leu Leu
                1925                1930                1935

Gln Ser Gln Arg Gln Tyr Ile Phe Glu Tyr Asp Ser Ser Asp Arg Leu
            1940                1945                1950

His Ala Val Thr Met Pro Ser Val Ala Arg His Ser Met Ser Thr His
        1955                1960                1965

Thr Ser Ile Gly Tyr Ile Arg Asn Ile Tyr Asn Pro Pro Glu Ser Asn
    1970                1975                1980

Ala Ser Val Ile Phe Asp Tyr Ser Asp Asp Gly Arg Ile Leu Lys Thr
1985                1990                1995                2000

Ser Phe Leu Gly Thr Gly Arg Gln Val Phe Tyr Lys Tyr Gly Lys Leu
                2005                2010                2015

Ser Lys Leu Ser Glu Ile Val Tyr Asp Ser Thr Ala Val Thr Phe Gly
            2020                2025                2030

Tyr Asp Glu Thr Thr Gly Val Leu Lys Met Val Asn Leu Gln Ser Gly
        2035                2040                2045

Gly Phe Ser Cys Thr Ile Arg Tyr Arg Lys Val Gly Pro Leu Val Asp
    2050                2055                2060

Lys Gln Ile Tyr Arg Phe Ser Glu Glu Gly Met Ile Asn Ala Arg Phe
2065                2070                2075                2080

Asp Tyr Thr Tyr His Asp Asn Ser Phe Arg Ile Ala Ser Ile Lys Pro
                2085                2090                2095

Val Ile Ser Glu Thr Pro Leu Pro Val Asp Leu Tyr Arg Tyr Asp Glu

2100                2105                2110

Ile Ser Gly Lys Val Glu His Phe Gly Lys Phe Gly Val Ile Tyr Tyr
        2115                2120                2125

Asp Ile Asn Gln Ile Ile Thr Thr Ala Val Met Thr Leu Ser Lys His
    2130                2135                2140

Phe Asp Thr His Gly Arg Ile Lys Glu Val Gln Tyr Glu Met Phe Arg
2145                2150                2155                2160

Ser Leu Met Tyr Trp Met Thr Val Gln Tyr Asp Ser Met Gly Arg Val
                2165                2170                2175

Ile Lys Arg Glu Leu Lys Leu Gly Pro Tyr Ala Asn Thr Thr Lys Tyr
            2180                2185                2190

Thr Tyr Asp Tyr Asp Gly Asp Gly Gln Leu Gln Ser Val Ala Val Asn
        2195                2200                2205

Asp Arg Pro Thr Trp Arg Tyr Ser Tyr Asp Leu Asn Gly Asn Leu His
    2210                2215                2220

Leu Leu Asn Pro Gly Asn Ser Ala Arg Leu Met Pro Leu Arg Tyr Asp
2225                2230                2235                2240

Leu Arg Asp Arg Ile Thr Arg Leu Gly Asp Val Gln Tyr Lys Ile Asp
                2245                2250                2255

Asp Asp Gly Tyr Leu Cys Gln Arg Gly Ser Asp Ile Phe Glu Tyr Asn
            2260                2265                2270

Ser Lys Gly Leu Leu Thr Arg Ala Tyr Asn Lys Ala Ser Gly Trp Ser
        2275                2280                2285

Val Gln Tyr Arg Tyr Asp Gly Val Ser Arg Arg Ala Ser Tyr Lys Thr
    2290                2295                2300

Asn Leu Gly His His Leu Gln Tyr Phe Tyr Ser Asp Leu His His Pro
2305                2310                2315                2320

Thr Arg Ile Thr His Val Tyr Asn His Ser Asn Ser Glu Ile Thr Ser
                2325                2330                2335

Leu Tyr Tyr Asp Leu Gln Gly His Leu Phe Ala Met Glu Ser Ser Ser
            2340                2345                2350

Gly Glu Glu Tyr Tyr Val Ala Ser Asp Asn Thr Gly Thr Pro Leu Ala
        2355                2360                2365

Val Phe Ser Ile Asn Gly Leu Met Ile Lys Gln Leu Gln Tyr Thr Ala
    2370                2375                2380

Tyr Gly Glu Ile Tyr Tyr Asp Ser Asn Pro Asp Phe Gln Met Val Ile
2385                2390                2395                2400

Gly Phe His Gly Gly Leu Tyr Asp Pro Leu Thr Lys Leu Val His Phe
                2405                2410                2415

Thr Gln Arg Asp Tyr Asp Val Leu Ala Gly Arg Trp Thr Ser Pro Asp
            2420                2425                2430

Tyr Thr Met Trp Arg Asn Val Gly Lys Glu Pro Ala Pro Phe Asn Leu
        2435                2440                2445

Tyr Met Phe Lys Asn Asn Asn Pro Leu Ser Asn Glu Leu Asp Leu Lys
    2450                2455                2460

Asn Tyr Val Thr Asp Val Lys Ser Trp Leu Val Met Phe Gly Phe Gln
2465                2470                2475                2480

Leu Ser Asn Ile Ile Pro Gly Phe Pro Arg Ala Lys Met Tyr Phe Val
                2485                2490                2495

Pro Pro Pro Tyr Glu Leu Ser Glu Ser Gln Ala Ser Glu Asn Gly Gln
            2500                2505                2510

Leu Ile Thr Gly Val Gln Gln Thr Thr Glu Arg His Asn Gln Ala Phe
        2515                2520                2525

Leu Ala Leu Glu Gly Gln Val Ile Ser Lys Lys Leu His Ala Gly Ile
    2530                2535                2540

Arg Glu Lys Ala Gly His Trp Phe Ala Thr Thr Thr Pro Ile Ile Gly
2545                2550                2555                2560

Lys Gly Ile Met Phe Ala Ile Lys Glu Gly Arg Val Thr Thr Gly Val
                2565                2570                2575

Ser Ser Ile Ala Ser Glu Asp Ser Arg Lys Val Ala Ser Val Leu Asn
            2580                2585                2590

Asn Ala Tyr Tyr Leu Asp Lys Met His Tyr Ser Ile Glu Gly Lys Asp
        2595                2600                2605

Thr His Tyr Phe Val Lys Ile Gly Ala Ala Asp Gly Asp Leu Val Thr
    2610                2615                2620

Leu Gly Thr Thr Ile Gly Arg Lys Val Leu Glu Ser Gly Val Asn Val
2625                2630                2635                2640

Thr Val Ser Gln Pro Thr Leu Leu Val Asn Gly Arg Thr Arg Arg Phe
                2645                2650                2655

Thr Asn Ile Glu Phe Gln Tyr Ser Thr Leu Leu Leu Ser Ile Arg Tyr
            2660                2665                2670

Gly Leu Thr Pro Asp Thr Leu Asp Glu Glu Lys Ala Arg Val Leu Asp
        2675                2680                2685

Gln Ala Arg Gln Arg Ala Leu Gly Thr Ala Trp Ala Lys Glu Gln Gln
    2690                2695                2700

Lys Ala Arg Asp Gly Arg Glu Gly Ser Arg Leu Trp Thr Glu Gly Glu
2705                2710                2715                2720

Lys Gln Gln Leu Leu Ser Thr Gly Arg Val Gln Gly Tyr Glu Gly Tyr
                2725                2730                2735

Tyr Val Leu Pro Val Glu Gln Tyr Pro Glu Leu Ala Asp Ser Ser Ser
            2740                2745                2750

Asn Ile Gln Phe Leu Arg Gln Asn Glu Met Gly Lys Arg
        2755                2760                2765

<210> 73
<211> 86
<212> PRT
<213> Trypanosoma cruzi

<400> 73
Met Ile Asn Asn Lys His Gln Thr Ile Pro Thr Thr His Ser Tyr Thr
1               5                   10                  15

Leu Pro Leu Ile His Tyr Ile Lys Thr Tyr Thr Thr Asp Asn Lys His
            20                  25                  30

Asn His Tyr Asn Ser Ile Ser His Tyr Tyr Thr Asn Thr Gln Leu Ser
        35                  40                  45

Asn Ser Arg Cys Thr Ser Tyr Pro Ile Gln Leu His Lys Phe Ile Asn
    50                  55                  60

Thr Tyr Ser Ser Ile Leu His Gln Pro Gln Ser Asn Pro Thr Ser Pro
65                  70                  75                  80

Lys Ile Pro Pro Asn Pro
                85

<210> 74
<211> 533
<212> PRT
<213> Aedes aegypti

<400> 74
Met Ser Leu Glu Ile Glu Val Pro His Val Arg Cys Pro Ser Leu Gly
1               5                   10                  15

Val Leu Ile Leu Thr Leu Asn Leu Ala Leu Phe Leu Pro Gln Thr Ile
            20                  25                  30

Asn Arg Thr Pro Pro Tyr Val Leu Ala Gly Thr Gly Gly Gly Ser Met
        35                  40                  45

Leu Gly Asp Val Asn Ile Ser Ala Ile Leu Asp Ser Phe Ser Val Gly
    50                  55                  60

Tyr Asp Lys Arg Val Arg Pro Asn Tyr Gly Gly Pro Pro Val Glu Val
65                  70                  75                  80

Gly Val Thr Met Tyr Val Leu Ser Ile Ser Ser Val Ser Glu Val Leu
                85                  90                  95

Met Asp Phe Thr Leu Asp Phe Tyr Phe Arg Gln Phe Trp Thr Asp Pro
            100                 105                 110

Arg Leu Ala Tyr Arg Lys Arg Pro Gly Val Glu Thr Leu Ser Val Gly
        115                 120                 125

Ser Glu Phe Ile Lys Asn Ile Trp Val Pro Asp Thr Phe Phe Val Asn
    130                 135                 140

Glu Lys Gln Ser Tyr Phe His Ile Ala Thr Thr Ser Asn Glu Phe Ile
145                 150                 155                 160

Arg Val His His Ser Gly Ser Ile Thr Arg Ser Ile Arg Leu Thr Ile
                165                 170                 175

Thr Ala Ser Cys Pro Met Gly Leu Gln Tyr Phe Pro Met Asp Arg Gln
            180                 185                 190

Leu Cys His Ile Glu Ile Glu Ser Phe Gly Tyr Thr Met Arg Asp Ile
        195                 200                 205

Arg Tyr Phe Trp Lys Asp Gly Leu Ser Ser Val Gly Met Ser Ser Glu
    210                 215                 220

Val Glu Leu Pro Gln Phe Arg Val Leu Gly His Arg Gln Arg Ala Thr
225                 230                 235                 240

Glu Ile Asn Leu Thr Thr Gly Asn Tyr Ser Arg Leu Ala Cys Glu Ile
                245                 250                 255

Gln Phe Val Arg Ser Met Gly Tyr Tyr Leu Ile Gln Ile Tyr Ile Pro
            260                 265                 270

Ser Gly Leu Ile Val Ile Ile Ser Trp Val Ser Phe Trp Leu Asn Arg
        275                 280                 285

Asp Ala Thr Pro Ala Arg Val Ala Leu Gly Val Thr Thr Val Leu Thr
    290                 295                 300

Met Thr Thr Leu Met Ser Ser Thr Asn Ala Ala Leu Pro Lys Ile Ser
305                 310                 315                 320

Tyr Val Lys Ser Ile Asp Val Tyr Leu Gly Thr Cys Phe Val Met Val
                325                 330                 335

Phe Ala Ser Leu Leu Glu Tyr Ala Thr Val Gly Tyr Met Ala Lys Arg
            340                 345                 350

Ile Gln Ile Gly Lys Gln Arg Phe Met Ala Ile Gln Lys Ile Ala Glu
        355                 360                 365

Gln Lys Lys Gln Gln Ala Ala Asp Ala Asn His Pro Pro Pro Pro Pro
    370                 375                 380

Pro Val Ser Asp His Ser His Gly His Gly His Gly His Ser His Gly
385                 390                 395                 400

His Gln His Thr Pro Lys Gln Gln Met Gly Ser Arg Ser Gly Pro Leu
                405                 410                 415

Phe Gln Glu Val Arg Phe Lys Val His Asp Pro Lys Ala His Ser Lys
            420                 425                 430

Gly Gly Thr Leu Glu Asn Thr Ile Asn Gly Gly Arg Gly Gly Gly Gly
        435                 440                 445

Pro Pro Gly Gly Gly Gly Gly Pro Pro Gly Gly Gly Gly Gly Gly Pro
    450                 455                 460

Asp Glu Glu Ser Gly Ala Pro Gln His Leu Ile His Pro Gly Lys Asp
465                 470                 475                 480

Ile Asn Lys Leu Leu Gly Ile Thr Pro Ser Asp Ile Asp Lys Tyr Ser
                485                 490                 495

Arg Ile Val Phe Pro Val Cys Phe Val Cys Phe Asn Leu Met Tyr Trp
            500                 505                 510

Ile Ile Tyr Leu His Val Ser Asp Val Val Ala Asp Asp Leu Val Leu
        515                 520                 525

Leu Gly Glu Glu Lys
    530

<210> 75
<211> 176
<212> PRT
<213> Leptinotarsa decemlineata

<400> 75
Thr Thr Val Leu Thr Met Thr Thr Leu Met Ser Ser Thr Asn Ala Ala
1               5                   10                  15

Leu Pro Lys Ile Ser Tyr Val Lys Ser Ile Asp Val Tyr Leu Gly Thr
            20                  25                  30

Cys Phe Val Met Val Phe Ala Ser Leu Leu Glu Tyr Ala Thr Val Gly
        35                  40                  45

Tyr Met Ala Lys Arg Ile Gln Met Arg Lys Asn Arg Phe Leu Ala Ile
    50                  55                  60

Gln Lys Ile Ala Glu Gln Lys Lys Leu Asn Val Asp Gly Gly Pro Asp
65                  70                  75                  80

Gly Asp His Ala Pro Lys Gln Thr Glu Val Arg Phe Lys Val His Asp
                85                  90                  95

Pro Lys Ala His Ser Lys Gly Gly Thr Leu Glu Ser Thr Val Asn Gly
            100                 105                 110

Gly Arg Gly Gly Asp Arg Gly Gly Gly Gly Pro Asp Glu Glu Ala Ala
        115                 120                 125

Gly Pro Thr Pro Gln His Ile Ile His Pro Asn Lys Asp Val Asn Lys
    130                 135                 140

Leu Tyr Gly Met Thr Pro Ser Asp Ile Asp Lys Tyr Ser Arg Ile Val
145                 150                 155                 160

Phe Pro Val Cys Phe Val Cys Phe Asn Leu Met Tyr Trp Ile Ile Tyr
                165                 170                 175

<210> 76
<211> 83

<212> PRT
<213> Arabidopsis thaliana

<400> 76
Met Tyr Ser Lys Ala Gly Met Leu Leu Leu Leu Leu His Val Leu Gly
1               5                   10                  15

Phe Met Leu Leu Ala Ile Leu Arg Ile Lys Leu Leu Val Cys Met Phe
            20                  25                  30

Leu Ser Leu Cys Leu Leu Phe Cys Ser Leu Cys Trp Phe Cys Leu Asn
        35                  40                  45

Glu Trp Phe Asn Asn Pro Phe Gly Asn Leu Leu Phe Asp Val Cys Leu
    50                  55                  60

Val Thr Leu Gly Met Gln Asn Tyr Leu Glu Ser Trp Phe Gln Asn Leu
65                  70                  75                  80

Val Ser Phe



<210> 77
<211> 71
<212> PRT
<213> Homo sapiens

<400> 77
Gln Val Thr Val Arg Asp Pro Ser Gly Arg Pro Leu Arg Leu Pro Pro
1               5                   10                  15

Val Leu Pro His Pro Ile Phe Asp Asn His Asp Arg His Arg Ile Glu
            20                  25                  30

Glu Lys Arg Lys Arg Thr Tyr Glu Thr Phe Lys Ser Ile Met Lys Lys
        35                  40                  45

Ser Pro Phe Ser Gly Pro Thr Asp Pro Arg Pro Pro Pro Arg Arg Ile
    50                  55                  60

Ala Val Pro Ser Arg Ser Ser
65                  70


<210> 78
<211> 3318
<212> DNA
<213> Homo sapiens

<400> 78
cagatgtcca gttccagatg cctggaccca gagtgtgggg gaaatatctc tggagaagcc 60
ctcactccaa aggctgtcca ggcgcaatgt ggtggctgct tctctgggga gtcctccagg 120
cttgcccaac ccggggctcc gtcctcttgg cccaagagct accccagcag ctgacatccc 180
ccgggtaccc agagccgtat ggcaaaggcc aagagagcag cacggacatc aaggctccag 240
agggctttgc tgtgaggctc gtcttccagg acttcgacct ggagccgtcc caggactgtg 300
caggggactc tgtcacaatc tcattcgtcg gttcggatcc aagccagttc tgtggtcagc 360
aaggctcccc tctgggcagg ccccctggtc agagggagtt tgtatcctca gggaggagtt 420
tgcggctgac cttccgcaca cagccttcct cggagaacaa gactgcccac ctccacaagg 480
gcttcctggc cctctaccaa accgtggctg tgaactatag tcagcccatc agcgaggcca 540
gcaggggctc tgaggccatc aacgcacctg gagacaaccc tgccaaggtc cagaaccact 600

gccaggagcc ctattatcag gccgcggcag caggggcact cacctgtgca accccaggga 660
cctggaaaga cagacaggat ggggaggagg ttcttcagtg tatgcctgtc tgcggacggc 720
cagtcacccc cattgcccag aatcagacga ccctcggttc ttccagagcc aagctgggca 780
acttcccctg gcaagccttc accagtatcc acggccgtgg gggcggggcc ctgctggggg 840
acagatggat cctcactgct gcccacaccg tctaccccaa ggacagtgtt tctctcagga 900
agaaccagag tgtgaatgtg ttcttgggcc acacagccat agatgagatg ctgaaactgg 960
ggaaccaccc tgtccaccgt gtcgttgtgc accccgacta ccgtcagaat gagtcccata 1020
actttagcgg ggacatcgcc ctcctggagc tgcagcacag catccccctg ggccccaacg 1080
tcctcccggt ctgtctgccc gataatgaga ccctctaccg cagcggcttg ttgggctacg 1140
tcagtgggtt tggcatggag atgggctggc taactactga gctgaagtac tcgaggctgc 1200
ctgtagctcc cagggaggcc tgcaacgcct ggctccaaaa gagacagaga cccgaggtgt 1260
tttctgacaa tatgttctgt gttggggatg agacgcaaag gcacagtgtc tgccaggggg 1320
acagtggcag cgtctatgtg gtatgggaca atcatgccca tcactgggtg gccacgggca 1380
ttgtgtcctg gggcataggg tgtggcgaag ggtatgactt ctacaccaag gtgctcagct 1440
atgtggactg gatcaaggga gtgatgaatg gcaagaattg accctggggg cttgaacagg 1500
gactgaccag cacagtggag gccccaggca acagagggcc tggagtgagg actgaacact 1560
ggggtagggg ttgggggtgg ggggttgggg gaggcagggg aaatcctatt cacatcactg 1620
ttgcaccaag ccactgcaag agaaaccccc acccggcaag cccgccccat cccagacagg 1680
aagcagagtc ccacagaccg ctcctcctca ccctctacct ccctgtgctc atgcactagg 1740
ccccgggaag cctgtacatc tcaacaactt tcgccttgaa tgtccttaga accgccttcc 1800
cctacttcat ctgttgacac agcttttata ctcacctgtg gaagagtcag ctactcaccc 1860
gctattagag tatggaggaa ggggttttca ttgcattgca tttctgaaac attcctaaga 1920
ccctttagtt gaccttcaaa tattcaagct attctgcagc tccaagatgc aattatagaa 1980
acagctcctt ttttatttta tgtcctctat atgccaggtg cttcacctgt tatttcactt 2040
aatcctcata ccatatttgc aaaggatgtg ttattatcta tgtgtgacaa atgaggaaac 2100
tgaggctcag gggataaagg gacttgccca agtcccacag ctggtgtgtg actgcagaga 2160
ctgtgctctt cccagtgtgc tgcaatactt ctcaaccctc ctctaacctg ctgtgtcacc 2220
cgctttccct cccagccccc acatccttac cattttccct ccctgggaat tcctgcttct 2280
gcgaaaatgg tatcctctag ctcacacttt cctaatggcc ccatctcctg cagaagccag 2340
gtgagcccag cactggactg aagttcttgc agacacccca cctgtgcccc tatcatcagg 2400
ggaactgctc cacctgagag gaccaactct ttaattttta gtaaaacctg gaggtgatgg 2460
gccgggcgca gtggctcacg cctgtaatcc caacacctta ggagtccgag gtgggtggat 2520
cacgaggtca ggagatccag cccatcctgg ccaacatggt gaaaccccat ctctactaaa 2580
aatacaaaaa ttagccgggc gtggtgacac gtgcctgtag tcccagctac tcgggaggct 2640
gaggcaggag aatcacttga acctgggagg cggaggttgc agtgagctaa gatcacgcca 2700
ctgcactcca gcctgcggac agaccaagac ttcatccccc ccaaaaaaaa aagattggag 2760
gtgatttaca gtgaaagaca caaataaaat acaactgttc aatggaaata gaaaataaac 2820
accataaaag agagaagaga ggtaatttgt tagcatcaag agtcaagttg ctatatggtc 2880
aaaggttaaa tttatctcta aaaaatggca ggattcaaag ttgtacatac atgtgattac 2940
ttctgttttt tacacccaca tacagtacaa aagattatta aaaatattcc caaaaggcag 3000
gtgcaatgat gcacacttat acccccagcc actcaggagg ctgatgcaag aggatcgctt 3060
gagcccagga gttgaagtcc agcctaagca acatagtgaa accccatcgc caaaaatata 3120
ataataattc tctcaaaata ctaaacagag gtggttttat tgataagatt ttggctgttt 3180
ggttttccac tattctctat tggctaaaat ttgtttaatg agcatgaaat gtttttattt 3240
tattttgctt atttttatga ttgcaaaaaa tgatatgagt ttctccctgc caaggcaaaa 3300
aaatatatat atacctat 3318

<210> 79
<211> 2386
<212> DNA
<213> Homo sapiens

<400> 79
tgcacgaaga cgctgtcggg agagcccagg attcaacacg ggccttgaga aatgtggctc 60
ttgtacctcc tggtgccggc cctgttctgc agggcaggag gctccattcc catccctcag 120
aagttatttg gggaggtgac ttcccctctg ttccccaagc cttaccccaa caactttgaa 180
acaaccactg tgatcacagt ccccacggga tacagggtga agctcgtctt ccagcagttt 240
gacctggagc cttctgaagg ctgcttctat gattatgtca agatctctgc tgataagaaa 300
agcctgggga ggttctgtgg gcaactgggt tctccactgg gcaacccccc gggaaagaag 360
gaatttatgt cccaagggaa caagatgctg ctgaccttcc acacagactt ctccaacgag 420

gagaatggga ccatcatgtt ctacaagggc ttcctggcct actaccaagc tgtggacctt 480
gatgaatgtg cttcccggag caaattaggg gaggaggatc cccagcccca gtgccagcac 540
ctgtgtcaca actacgttgg aggctacttc tgttcctgcc gtccaggcta tgagcttcag 600
gaagacaggc attcctgcca ggctgagtgc agcagcgagc tgtacacgga ggcatcaggc 660
tacatctcca gcctggagta ccctcggtcc tacccccctg acctgcgctg caactacagc 720
atccgggtgg agcggggcct caccctgcac ctcaagttcc tggagccttt tgatattgat 780
gaccaccagc aagtacactg cccctatgac cagctacaga tctatgccaa cgggaagaac 840
attggcgagt tctgtgggaa gcaaaggccc cccgacctcg acaccagcag caatgctgtg 900
gatctgctgt tcttcacaga tgagtcgggg gacagccggg gctggaagct gcgctacacc 960
accgagatca tcaagtgccc ccagcccaag accctagacg agttcaccat catccagaac 1020
ctgcagcctc agtaccagtt ccgtgactac ttcattgcta cctgcaagca aggctaccag 1080
ctcatagagg ggaaccaggt gctgcattcc ttcacagctg tctgccagga tgatggcacg 1140
tggcatcgtg ccatgcccag atgcaagatc aaggactgtg ggcagccccg aaacctgcct 1200
aatggtgact tccgttacac caccacaatg ggagtgaaca cctacaaggc ccgtatccag 1260
tactactgcc atgagccata ttacaagatg cagaccagag ctggcagcag ggagtctgag 1320
caaggggtgt acacctgcac agcacagggc atttggaaga atgaacagaa gggagagaag 1380
attcctcggt gcttgccagt gtgtgggaag cccgtgaacc ccgtggaaca gaggcagcgc 1440
atcatcggag ggcaaaaagc caagatgggc aacttcccct ggcaggtgtt caccaacatc 1500
cacgggcgcg ggggcggggc cctgctgggc gaccgctgga tcctcacagc tgcccacacc 1560
ctgtatccca aggaacacga agcgcaaagc aacgcctctt tggatgtgtt cctgggccac 1620
acaaatgtgg aagagctcat gaagctagga aatcacccca tccgcagggt cagcgtccac 1680
ccggactacc gtcaggatga gtcctacaat tttgaggggg acatcgccct gctggagctg 1740
gaaaatagtg tcaccctggg tcccaacctc ctccccatct gcctccctga caacgatacc 1800
ttctacgacc tgggcttgat gggctatgtc agtggcttcg gggtcatgga ggagaagatt 1860
gctcatgacc tcaggtttgt ccgtctgccc gtagctaatc cacaggcctg tgagaactgg 1920
ctccggggaa agaataggat ggatgtgttc tctcaaaaca tgttctgtgc tggacaccca 1980
tctctaaagc aggacgcctg ccagggggat agtgggggcg tttttgcagt aagggacccg 2040
aacactgatc gctgggtggc cacgggcatc gtgtcctggg gcatcgggtg cagcaggggc 2100
tatggcttct acaccaaagt gctcaactac gtggactgga tcaagaaaga gatggaggag 2160
gaggactgag cccagaattc actaggttcg aatccagaga gcagtgtgga aaaaaaaaaa 2220
caaaaaacaa ctgaccagtt gttgataacc actaagagtc tctattaaaa ttactgatgc 2280
agaaagaccg tgtgtgaaat tctctttcct gtagtcccat tgatgtactt tacctgaaac 2340
aaccaaaggg cccctttctt tcttctgagg attgcagagg atatag 2386

<210> 80
<211> 487
<212> PRT
<213> Homo sapiens

<400> 80
Met Pro Gly Pro Arg Val Trp Gly Lys Tyr Leu Trp Arg Ser Pro His
1               5                   10                  15

Ser Lys Gly Cys Pro Gly Ala Met Trp Trp Leu Leu Leu Trp Gly Val
            20                  25                  30

Leu Gln Ala Cys Pro Thr Arg Gly Ser Val Leu Leu Ala Gln Glu Leu
        35                  40                  45

Pro Gln Gln Leu Thr Ser Pro Gly Tyr Pro Glu Pro Tyr Gly Lys Gly
    50                  55                  60

Gln Glu Ser Ser Thr Asp Ile Lys Ala Pro Glu Gly Phe Ala Val Arg
65                  70                  75                  80

Leu Val Phe Gln Asp Phe Asp Leu Glu Pro Ser Gln Asp Cys Ala Gly
                85                  90                  95

Asp Ser Val Thr Ile Ser Phe Val Gly Ser Asp Pro Ser Gln Phe Cys
            100                 105                 110

Gly Gln Gln Gly Ser Pro Leu Gly Arg Pro Pro Gly Gln Arg Glu Phe
        115                 120                 125

Val Ser Ser Gly Arg Ser Leu Arg Leu Thr Phe Arg Thr Gln Pro Ser
    130                 135                 140

Ser Glu Asn Lys Thr Ala His Leu His Lys Gly Phe Leu Ala Leu Tyr
145                 150                 155                 160

Gln Thr Val Ala Val Asn Tyr Ser Gln Pro Ile Ser Glu Ala Ser Arg
                165                 170                 175

Gly Ser Glu Ala Ile Asn Ala Pro Gly Asp Asn Pro Ala Lys Val Gln
            180                 185                 190

Asn His Cys Gln Glu Pro Tyr Tyr Gln Ala Ala Ala Ala Gly Ala Leu
        195                 200                 205

Thr Cys Ala Thr Pro Gly Thr Trp Lys Asp Arg Gln Asp Gly Glu Glu
    210                 215                 220

Val Leu Gln Cys Met Pro Val Cys Gly Arg Pro Val Thr Pro Ile Ala
225                 230                 235                 240

Gln Asn Gln Thr Thr Leu Gly Ser Ser Arg Ala Lys Leu Gly Asn Phe
                245                 250                 255

Pro Trp Gln Ala Phe Thr Ser Ile His Gly Arg Gly Gly Gly Ala Leu
            260                 265                 270

Leu Gly Asp Arg Trp Ile Leu Thr Ala Ala His Thr Val Tyr Pro Lys
        275                 280                 285

Asp Ser Val Ser Leu Arg Lys Asn Gln Ser Val Asn Val Phe Leu Gly
    290                 295                 300

His Thr Ala Ile Asp Glu Met Leu Lys Leu Gly Asn His Pro Val His
305                 310                 315                 320

Arg Val Val Val His Pro Asp Tyr Arg Gln Asn Glu Ser His Asn Phe
                325                 330                 335

Ser Gly Asp Ile Ala Leu Leu Glu Leu Gln His Ser Ile Pro Leu Gly
            340                 345                 350

Pro Asn Val Leu Pro Val Cys Leu Pro Asp Asn Glu Thr Leu Tyr Arg
        355                 360                 365

Ser Gly Leu Leu Gly Tyr Val Ser Gly Phe Gly Met Glu Met Gly Trp
    370                 375                 380

Leu Thr Thr Glu Leu Lys Tyr Ser Arg Leu Pro Val Ala Pro Arg Glu
385                 390                 395                 400

Ala Cys Asn Ala Trp Leu Gln Lys Arg Gln Arg Pro Glu Val Phe Ser
                405                 410                 415

Asp Asn Met Phe Cys Val Gly Asp Glu Thr Gln Arg His Ser Val Cys
            420                 425                 430

Gln Gly Asp Ser Gly Ser Val Tyr Val Val Trp Asp Asn His Ala His
        435                 440                 445

His Trp Val Ala Thr Gly Ile Val Ser Trp Gly Ile Gly Cys Gly Glu
    450                 455                 460

Gly Tyr Asp Phe Tyr Thr Lys Val Leu Ser Tyr Val Asp Trp Ile Lys
465                 470                 475                 480

Gly Val Met Asn Gly Lys Asn
                485

<210> 81
<211> 705
<212> PRT
<213> Homo sapiens

<400> 81
Met Trp Leu Leu Tyr Leu Leu Val Pro Ala Leu Phe Cys Arg Ala Gly
  1               5                  10                  15

Gly Ser Ile Pro Ile Pro Gln Lys Leu Phe Gly Glu Val Thr Ser Pro
             20                  25                  30

Leu Phe Pro Lys Pro Tyr Pro Asn Asn Phe Glu Thr Thr Thr Val Ile
         35                  40                  45

Thr Val Pro Thr Gly Tyr Arg Val Lys Leu Val Phe Gln Gln Phe Asp
     50                  55                  60

Leu Glu Pro Ser Glu Gly Cys Phe Tyr Asp Tyr Val Lys Ile Ser Ala
 65                  70                  75                  80

Asp Lys Lys Ser Leu Gly Arg Phe Cys Gly Gln Leu Gly Ser Pro Leu
                 85                  90                  95

Gly Asn Pro Pro Gly Lys Lys Glu Phe Met Ser Gln Gly Asn Lys Met
            100                 105                 110

Leu Leu Thr Phe His Thr Asp Phe Ser Asn Glu Glu Asn Gly Thr Ile
        115                 120                 125

Met Phe Tyr Lys Gly Phe Leu Ala Tyr Tyr Gln Ala Val Asp Leu Asp
    130                 135                 140

Glu Cys Ala Ser Arg Ser Lys Ser Gly Glu Glu Asp Pro Gln Pro Gln
145                 150                 155                 160

Cys Gln His Leu Cys His Asn Tyr Val Gly Gly Tyr Phe Cys Ser Cys
                165                 170                 175

Arg Pro Gly Tyr Glu Leu Gln Glu Asp Arg His Ser Cys Gln Ala Glu
            180                 185                 190

Cys Ser Ser Glu Leu Tyr Thr Glu Ala Ser Gly Tyr Ile Ser Ser Leu
        195                 200                 205

Glu Tyr Pro Arg Ser Tyr Pro Pro Asp Leu Arg Cys Asn Tyr Ser Ile
    210                 215                 220

Arg Val Glu Arg Gly Leu Thr Leu His Leu Lys Phe Leu Glu Pro Phe
225                 230                 235                 240

Asp Ile Asp Asp His Gln Gln Val His Cys Pro Tyr Asp Gln Leu Gln
                245                 250                 255

Ile Tyr Ala Asn Gly Lys Asn Ile Gly Glu Phe Cys Gly Lys Gln Arg
            260                 265                 270

Pro Pro Asp Leu Asp Thr Ser Ser Asn Ala Val Asp Leu Leu Phe Phe
        275                 280                 285

Thr Asp Glu Ser Gly Asp Ser Arg Gly Trp Lys Leu Arg Tyr Thr Thr
    290                 295                 300

Glu Ile Ile Lys Cys Pro Gln Pro Lys Thr Leu Asp Glu Phe Thr Ile
305                 310                 315                 320

Ile Gln Asn Leu Gln Pro Gln Tyr Gln Phe Arg Asp Tyr Phe Ile Ala
                325                 330                 335

Thr Cys Lys Gln Gly Tyr Gln Leu Ile Glu Gly Asn Gln Val Leu His
            340                 345                 350

Ser Phe Thr Ala Val Cys Gln Asp Asp Gly Thr Trp His Arg Ala Met
        355                 360                 365

Pro Arg Cys Lys Ile Lys Asp Cys Gly Gln Pro Arg Asn Leu Pro Asn
    370                 375                 380

Gly Asp Phe Arg Tyr Thr Thr Thr Met Gly Val Asn Thr Tyr Lys Ala
385                 390                 395                 400

Arg Ile Gln Tyr Tyr Cys His Glu Pro Tyr Tyr Lys Met Gln Thr Arg
                405                 410                 415

Ala Gly Ser Arg Glu Ser Glu Gln Gly Val Tyr Thr Cys Thr Ala Gln
            420                 425                 430

Gly Ile Trp Lys Asn Glu Gln Lys Gly Glu Lys Ile Pro Arg Cys Leu
        435                 440                 445

Pro Val Cys Gly Lys Pro Val Asn Pro Val Glu Gln Arg Gln Arg Ile
    450                 455                 460

Ile Gly Gly Gln Lys Ala Lys Met Gly Asn Phe Pro Trp Gln Val Phe
465                 470                 475                 480

Thr Asn Ile His Gly Arg Gly Gly Gly Ala Leu Leu Gly Asp Arg Trp
                485                 490                 495

Ile Leu Thr Ala Ala His Thr Leu Tyr Pro Lys Glu His Glu Ala Gln
            500                 505                 510

Ser Asn Ala Ser Leu Asp Val Phe Leu Gly His Thr Asn Val Glu Glu
        515                 520                 525

Leu Met Lys Leu Gly Asn His Pro Ile Arg Arg Val Ser Val His Pro
    530                 535                 540

Asp Tyr Arg Gln Asp Glu Ser Tyr Asn Phe Glu Gly Asp Ile Ala Leu

545                 550                 555                 560

Leu Glu Leu Glu Asn Ser Val Thr Leu Gly Pro Asn Leu Leu Pro Ile
                565                 570                 575

Cys Leu Pro Asp Asn Asp Thr Phe Tyr Asp Leu Gly Leu Met Gly Tyr
            580                 585                 590

Val Ser Gly Phe Gly Val Met Glu Glu Lys Ile Ala His Asp Leu Arg
        595                 600                 605

Phe Val Arg Leu Pro Val Ala Asn Pro Gln Ala Cys Glu Asn Trp Leu
    610                 615                 620

Arg Gly Lys Asn Arg Met Asp Val Phe Ser Gln Asn Met Phe Cys Ala
625                 630                 635                 640

Gly His Pro Ser Leu Lys Gln Asp Ala Cys Gln Gly Asp Ser Gly Gly
                645                 650                 655

Val Phe Ala Val Arg Asp Pro Asn Thr Asp Arg Trp Val Ala Thr Gly
            660                 665                 670

Ile Val Ser Trp Gly Ile Gly Cys Ser Arg Gly Tyr Gly Phe Tyr Thr
        675                 680                 685

Lys Val Leu Asn Tyr Val Asp Trp Ile Lys Lys Glu Met Glu Glu Glu
    690                 695                 700

Asp
705

<210> 82
<211> 1746
<212> DNA
<213> Macaca fascicularis

<400> 82
aataaattga ggccgctcac ccacggtacc cacctagtat atagagacag aattcaaact 60
ctggctctag cacttgtgct ttctgctaca ccagctcaag gaagtttgaa gacctacaga 120
agggctgatt ttagaaggtt aatcaaaaac ccaaggacag tttcatcatg tcataaccaa 180
agacccttgt ggcacctgct gtcatgggat aacaaatatc ttgtggggtt ctgaatgtgg 240
acttattact gaagctcctg tctgcttggt cagtggtggt ctagactaac ttctggtcct 300
gagattctaa agtgttggta gaccggttga gataaaagat atataataat gaatgcctta 360
cctatctgaa aaccagtttg atccgtgcca aggggctttt tgtgggctct gtagagtgcc 420
ctaaacccag ctctgccttt gctgtgttag acagaagcac gccattcaca tctctggggc 480
ccccaatggt gccatggtgt ggttgtggtc tgctcactgg ctcttctgtt ttttgttttt 540
gtttttcctg cctttttcca atcctcacac cttctgagct acagccccag tagggtctaa 600
atgtcctaga gctatatgag atttaggttt ctgagcacag ccaattctcc cacttttgag 660
gcttcccttc ccctttcact cgcccctctc tggttctctg ccaccagtcc agaagaactg 720
aatgtcgtgc tggggaccaa cgacttaact agctcatcca tggaaataaa ggaggtcgcc 780
agcatcattc ttcacaagga ctttaagaga gccaacatgg acaatgacat tgccttgctg 840
ctgctggcct cgcccatcac actcgatgac ctgaaggtgc ccatctgcct ccctacgcag 900
cacggccccg ccacatggca cgaatgctgg gtggcaggtt ggggccagac caatgctgct 960
gacaaaaact ctgtgaaaac ggatctgatg aaagcgccga tggtcatcat ggactgggag 1020
gagtgttcaa aggcgtttcc aaaactcacc aaaaatatgc tgtgtgctgg atacaataat 1080
gagagctatg acgcctgcca gggtgacagc gggggacctc tggtctgcac cccagagcct 1140
ggtgagaagt ggtaccaggt gggtatcatc agctggggaa agagctgtgg agagaagaac 1200
accccaggga tatacacctc gttggtgaac tacaacctct ggatcgagaa ggtgacccag 1260
ctagagggca ggcccttcag tgcggagaaa atgaggacct ctgtcaaaca gaaacctatg 1320

ggctcccgag tctcgggggt cccagagcca ggcggcctca gatcctggct cctgctctgt 1380
cccctgtccc atgtgttgtt cagagctatt ttgtactgat aataaaatag aggctatttt 1440
tttaaccaag ggagggtgca tgaaaatgtg tctccagcag aggctctggc tgcagctcag 1500
ggctcaagga tggaaactga gactggaacc aggagaacca gaaagtcagg ctggggccct 1560
ggtttgggga ctgcactttg ggtctgtgga ttagtcagga ctctctccat tctaggtgac 1620
agtcacctaa gtctgactga attcagccaa aatgaggcat ttatggatac atataacagg 1680
aaaataaaaa taaaaataaa aaaccgacac acaaataaaa aacaaaaaaa aaaaaaaaag 1740
gccaca 1746

<210> 83
<211> 1047
<212> DNA
<213> Mus musculus

<400> 83
gactattcct gtcagccgtg gcctccaaca caccagcaca gccgagagcc gatgatccgt 60
gccctcgcat ccctgctact tgttggccca caccctgtga agcaaatgtt gtagtgtggt 120
gtgagacccc tgctatgata gcagaattca atactccagg atcatagaag ggcaggaggc 180
tgagctgggt gagtttccat ggcaggtgag cattcaggaa agtgaccacc atttctgcgg 240
cggctccatt ctcagtgagt ggtggatcct caccgtggcc cactgcttct atgctcagga 300
gctttcccca acagatctca gagtcagagt gggaaccaat gacttaacta cttcacccgt 360
ggaactagag gtcaccacca taatccggca caaaggcttt aaacggctga acatggacaa 420
cgacattgcc ttgttgctgc tagccaagcc cttgacgttc aatgagctga cggtgcccat 480
ctgccttcct ctctggcccg cccctcccag ctggcacgaa tgctgggtgg caggatgggg 540
cgtaaccaac tcaactgaca aggaatctat gtcaacggat ctgatgaagg tgcccatgcg 600
tatcatagag tgggaggaat gcttacagat gtttcccagc ctcaccacaa acatgctgtg 660
tgcctcatat ggtaatgaga gctacgatgc ttgccagtgg gggaccgctt gtctgcacca 720
cagatcctgg cagtaggtgg taccaggtgg gcatcatcag ctggggcaag agctgtggaa 780
aaaaaggctt cccagggata tatactgtat tggcaaagta taccctgtgg attgagaaaa 840
tagcccagac agaggggaag cccctggatt ttagaggtca gagctcctct aacaagaaga 900
aaaacagaca gaacaatcag ctctccaaat ccccagccct gaactgcccc caaagctggc 960
tcctgccctg tctgctgtcc tttgcactgc ttagagcctt gtccaactgg aaataaaaca 1020
atgcagtctc tgatccaccc taacccg 1047

<210> 84
<211> 267
<212> PRT
<213> Macaca fascicularis

<400> 84
Met Arg Phe Arg Phe Leu Ser Thr Ala Asn Ser Pro Thr Phe Glu Ala
1               5                   10                  15

Ser Leu Pro Leu Ser Leu Ala Pro Leu Trp Phe Ser Ala Thr Ser Pro
            20                  25                  30

Glu Glu Leu Asn Val Val Leu Gly Thr Asn Asp Leu Thr Ser Ser Ser
        35                  40                  45

Met Glu Ile Lys Glu Val Ala Ser Ile Ile Leu His Lys Asp Phe Lys
    50                  55                  60

Arg Ala Asn Met Asp Asn Asp Ile Ala Leu Leu Leu Leu Ala Ser Pro
65                  70                  75                  80

Ile Thr Leu Asp Asp Leu Lys Val Pro Ile Cys Leu Pro Thr Gln His
                85                  90                  95

Gly Pro Ala Thr Trp His Glu Cys Trp Val Ala Gly Trp Gly Gln Thr

            100                 105                 110

Asn Ala Ala Asp Lys Asn Ser Val Lys Thr Asp Leu Met Lys Ala Pro
        115                 120                 125

Met Val Ile Met Asp Trp Glu Glu Cys Ser Lys Ala Phe Pro Lys Leu
    130                 135                 140

Thr Lys Asn Met Leu Cys Ala Gly Tyr Asn Asn Glu Ser Tyr Asp Ala
145                 150                 155                 160

Cys Gln Gly Asp Ser Gly Gly Pro Leu Val Cys Thr Pro Glu Pro Gly
                165                 170                 175

Glu Lys Trp Tyr Gln Val Gly Ile Ile Ser Trp Gly Lys Ser Cys Gly
            180                 185                 190

Glu Lys Asn Thr Pro Gly Ile Tyr Thr Ser Leu Val Asn Tyr Asn Leu
        195                 200                 205

Trp Ile Glu Lys Val Thr Gln Leu Glu Gly Arg Pro Phe Ser Ala Glu
    210                 215                 220

Lys Met Arg Thr Ser Val Lys Gln Lys Pro Met Gly Ser Arg Val Ser
225                 230                 235                 240

Gly Val Pro Glu Pro Gly Gly Leu Arg Ser Trp Leu Leu Leu Cys Pro
                245                 250                 255

Leu Ser His Val Leu Phe Arg Ala Ile Leu Tyr
            260                 265

<210> 85
<211> 638
<212> PRT
<213> Homo sapiens

<400> 85
Met Ile Leu Phe Lys Gln Ala Thr Tyr Phe Ile Ser Leu Phe Ala Thr
1               5                   10                  15

Val Ser Cys Gly Cys Leu Thr Gln Leu Tyr Glu Asn Ala Phe Phe Arg
            20                  25                  30

Gly Gly Asp Val Ala Ser Met Tyr Thr Pro Asn Ala Gln Tyr Cys Gln
        35                  40                  45

Met Arg Cys Thr Phe His Pro Arg Cys Leu Leu Phe Ser Phe Leu Pro
    50                  55                  60

Ala Ser Ser Ile Asn Asp Met Glu Lys Arg Phe Gly Cys Phe Leu Lys
65                  70                  75                  80

Asp Ser Val Thr Gly Thr Leu Pro Lys Val His Arg Thr Gly Ala Val
                85                  90                  95

Ser Gly His Ser Leu Lys Gln Cys Gly His Gln Ile Ser Ala Cys His
            100                 105                 110

Arg Asp Ile Tyr Lys Gly Val Asp Met Arg Gly Val Asn Phe Asn Val

115                 120                 125

Ser Lys Val Ser Ser Val Glu Glu Cys Gln Lys Arg Cys Thr Asn Asn
    130                 135                 140

Ile Arg Cys Gln Phe Phe Ser Tyr Ala Thr Gln Thr Phe His Lys Ala
145                 150                 155                 160

Glu Tyr Arg Asn Asn Cys Leu Leu Lys Tyr Ser Pro Gly Gly Thr Pro
                165                 170                 175

Thr Ala Ile Lys Val Leu Ser Asn Val Glu Ser Gly Phe Ser Leu Lys
            180                 185                 190

Pro Cys Ala Leu Ser Glu Ile Gly Cys His Met Asn Ile Phe Gln His
        195                 200                 205

Leu Ala Phe Ser Asp Val Asp Val Ala Arg Val Leu Thr Pro Asp Ala
    210                 215                 220

Phe Val Cys Arg Thr Ile Cys Thr Tyr His Pro Asn Cys Leu Phe Phe
225                 230                 235                 240

Thr Phe Tyr Thr Asn Val Trp Lys Ile Glu Ser Gln Arg Asn Val Cys
                245                 250                 255

Leu Leu Lys Thr Ser Glu Ser Gly Thr Pro Ser Ser Ser Thr Pro Gln
            260                 265                 270

Glu Asn Thr Ile Ser Gly Tyr Ser Leu Leu Thr Cys Lys Arg Thr Leu
        275                 280                 285

Pro Glu Pro Cys His Ser Lys Ile Tyr Pro Gly Val Asp Phe Gly Gly
    290                 295                 300

Glu Glu Leu Asn Val Thr Phe Val Lys Gly Val Asn Val Cys Gln Glu
305                 310                 315                 320

Thr Cys Thr Lys Met Ile Arg Cys Gln Phe Phe Thr Tyr Ser Leu Leu
                325                 330                 335

Pro Glu Asp Cys Lys Glu Glu Lys Cys Lys Cys Phe Leu Arg Leu Ser
            340                 345                 350

Met Asp Gly Ser Pro Thr Arg Ile Ala Tyr Gly Thr Gln Gly Ser Ser
        355                 360                 365

Gly Tyr Ser Leu Arg Leu Cys Asn Thr Gly Asp Asn Ser Val Cys Thr
    370                 375                 380

Thr Lys Thr Ser Thr Arg Ile Val Gly Gly Thr Asn Ser Ser Trp Gly
385                 390                 395                 400

Glu Trp Pro Trp Gln Val Ser Leu Gln Val Lys Leu Thr Ala Gln Arg
                405                 410                 415

His Leu Cys Gly Gly Ser Leu Ile Gly His Gln Trp Val Leu Thr Ala
            420                 425                 430

Ala His Cys Phe Asp Gly Leu Pro Leu Gln Asp Val Trp Arg Ile Tyr
        435                 440                 445

Ser Gly Ile Leu Asn Leu Ser Asp Ile Thr Lys Asp Thr Pro Phe Ser
    450                 455                 460

Gln Ile Lys Glu Ile Ile Ile His Gln Asn Tyr Lys Val Ser Glu Gly
465                 470                 475                 480

Asn His Asp Ile Ala Leu Ile Lys Leu Gln Ala Pro Leu Asn Tyr Thr
                485                 490                 495

Glu Phe Gln Lys Pro Ile Cys Leu Pro Ser Lys Gly Asp Thr Ser Thr
            500                 505                 510

Ile Tyr Thr Asn Cys Trp Val Thr Gly Trp Gly Phe Ser Lys Glu Lys
        515                 520                 525

Gly Glu Ile Gln Asn Ile Leu Gln Lys Val Asn Ile Pro Leu Val Thr
    530                 535                 540

Asn Glu Glu Cys Gln Lys Arg Tyr Gln Asp Tyr Lys Ile Thr Gln Arg
545                 550                 555                 560

Met Val Cys Ala Gly Tyr Lys Glu Gly Gly Lys Asp Ala Cys Lys Gly
                565                 570                 575

Asp Ser Gly Gly Pro Leu Val Cys Lys His Asn Gly Met Trp Arg Leu
            580                 585                 590

Val Gly Ile Thr Ser Trp Gly Glu Gly Cys Ala Arg Arg Glu Gln Pro
        595                 600                 605

Gly Val Tyr Thr Lys Val Ala Glu Tyr Met Asp Trp Ile Leu Glu Lys
    610                 615                 620

Thr Gln Ser Ser Asp Gly Lys Ala Gln Met Gln Ser Pro Ala
625                 630                 635


<210> 86
<211> 643
<212> PRT
<213> Sus scrofa

<400> 86
Met Glu Val Ile Val Leu Phe Arg Ile Ile Ser Phe Arg Gln Ala Val
  1               5                  10                  15

Tyr Phe Met Cys Leu Phe Ala Ala Val Ser Cys Gly Cys Leu Pro Gln
             20                  25                  30

Leu His Lys Asn Thr Phe Phe Arg Gly Gly Asp Val Ser Ala Met Tyr
         35                  40                  45

Thr Pro Ser Ala Arg His Cys Gln Met Met Cys Thr Phe His Pro Arg
     50                  55                  60

Cys Leu Leu Phe Ser Phe Leu Pro Ala Asp Ser Thr Ser Val Thr Asp
 65                  70                  75                  80

Lys Arg Phe Gly Cys Phe Leu Lys Asp Ser Val Thr Gly Met Leu Pro
                 85                  90                  95

Arg Val Leu Arg Glu Asn Ala Ile Ser Gly His Ser Leu Lys Gln Cys
            100                 105                 110

Gly His Gln Ile Arg Ala Cys His Arg Asp Ile Tyr Lys Gly Ile Asp
        115                 120                 125

Met Arg Gly Val Asn Phe Asn Val Ser Lys Val Lys Thr Val Glu Glu
    130                 135                 140

Cys Gln Glu Arg Cys Thr Asn Ser Ile His Cys Leu Phe Phe Thr Tyr
145                 150                 155                 160

Ala Thr Gln Ala Phe Asn Asn Ala Glu Tyr Arg Asn Asn Cys Leu Leu
                165                 170                 175

Lys His Ser Pro Gly Gly Thr Pro Thr Ser Ile Lys Val Leu Ala Asn
            180                 185                 190

Val Glu Ser Gly Phe Ser Leu Lys Pro Cys Ala Asp Ser Glu Ile Gly
        195                 200                 205

Cys His Met Asp Ile Phe Gln His Leu Ala Phe Ser Asp Val Asp Val
    210                 215                 220

Ala Arg Val Ile Ala Pro Asp Ala Phe Val Cys Arg Thr Ile Cys Thr
225                 230                 235                 240

Tyr His Pro Asn Cys Leu Phe Phe Thr Phe Tyr Thr Asn Ala Trp Lys
                245                 250                 255

Ile Glu Ser Gln Arg Asn Val Cys Phe Leu Lys Thr Ser His Ser Gly
            260                 265                 270

Thr Pro Ser Phe Pro Thr Pro Gln Glu Asn Ala Ile Ser Gly Tyr Ser
        275                 280                 285

Leu Leu Thr Cys Lys Gln Thr Leu Pro Glu Pro Cys His Ser Lys Ile
    290                 295                 300

Tyr Ser Glu Val Asp Phe Glu Gly Glu Glu Leu Asn Val Thr Phe Val
305                 310                 315                 320

Gln Gly Ala Asn Leu Cys Gln Glu Thr Cys Thr Lys Thr Ile Arg Cys
                325                 330                 335

Gln Phe Phe Thr Tyr Ser Leu His Pro Glu Asp Cys Arg Gly Glu Lys
            340                 345                 350

Cys Lys Cys Ser Leu Arg Leu Ser Ser Asp Gly Ser Pro Thr Lys Ile
        355                 360                 365

Thr His Gly Met Arg Ala Ser Ser Gly Tyr Ser Leu Arg Leu Cys Arg
    370                 375                 380

Ser Gly Asp His Ser Ala Cys Ala Thr Lys Ala Asn Thr Arg Ile Val
385                 390                 395                 400

Gly Gly Thr Asp Ser Phe Leu Gly Glu Trp Pro Trp Gln Val Ser Leu
                405                 410                 415

Gln Ala Lys Leu Arg Ala Gln Asn His Leu Cys Gly Gly Ser Ile Ile
            420                 425                 430

Gly His Gln Trp Val Leu Thr Ala Ala His Cys Phe Asp Gly Leu Ser
        435                 440                 445

Leu Pro Asp Ile Trp Arg Ile Tyr Gly Gly Ile Leu Asn Ile Ser Glu
    450                 455                 460

Ile Thr Lys Glu Thr Pro Phe Ser Gln Val Lys Glu Ile Ile Ile His
465                 470                 475                 480

Gln Asn Tyr Lys Ile Leu Glu Ser Gly His Asp Ile Ala Leu Leu Lys
                485                 490                 495

Leu Glu Thr Pro Leu Asn Tyr Thr Asp Phe Gln Lys Pro Ile Cys Leu
            500                 505                 510

Pro Ser Arg Asp Asp Thr Asn Val Val Tyr Thr Asn Cys Trp Val Thr
        515                 520                 525

Gly Trp Gly Phe Thr Glu Glu Lys Gly Glu Ile Gln Asn Ile Leu Gln
    530                 535                 540

Lys Val Asn Ile Pro Leu Val Ser Asn Glu Glu Cys Gln Lys Ser Tyr
545                 550                 555                 560

Arg Asp His Lys Ile Ser Lys Gln Met Ile Cys Ala Gly Tyr Lys Glu
                565                 570                 575

Gly Gly Lys Asp Ala Cys Lys Gly Glu Ser Gly Gly Pro Leu Val Cys
            580                 585                 590

Lys Tyr Asn Gly Ile Trp His Leu Val Gly Thr Thr Ser Trp Gly Glu
        595                 600                 605

Gly Cys Ala Arg Arg Glu Gln Pro Gly Val Tyr Thr Lys Val Ile Glu
    610                 615                 620

Tyr Met Asp Trp Ile Leu Glu Lys Thr Gln Asp Asp Asp Gly Gln Ser
625                 630                 635                 640

Trp Met Lys


<210> 87
<211> 625
<212> PRT
<213> Homo sapiens

<400> 87

<210> 88
<211> 257
<212> PRT
<213> Homo sapiens

<400> 88
Met Ile Ala Ile Ser Ala Val Ser Ser Ala Leu Leu Phe Ser Leu Leu
1               5                   10                  15

Cys Glu Ala Ser Thr Val Val Leu Leu Asn Ser Thr Asp Ser Ser Pro

            20                  25                  30

Pro Thr Asn Asn Phe Thr Asp Ile Glu Ala Ala Leu Lys Ala Gln Leu
        35                  40                  45

Asp Ser Ala Asp Ile Pro Lys Ala Arg Arg Lys Arg Tyr Ile Ser Gln
    50                  55                  60

Asn Asp Met Ile Ala Ile Leu Asp Tyr His Asn Gln Val Arg Gly Lys
65                  70                  75                  80

Val Phe Pro Pro Ala Ala Asn Met Glu Tyr Met Val Trp Asp Glu Asn
                85                  90                  95

Leu Ala Lys Ser Ala Glu Ala Trp Ala Ala Thr Cys Ile Trp Asp His
            100                 105                 110

Gly Pro Ser Tyr Leu Leu Arg Phe Leu Gly Gln Asn Leu Ser Val Arg
        115                 120                 125

Thr Gly Arg Tyr Arg Ser Ile Leu Gln Leu Val Lys Pro Trp Tyr Asp
    130                 135                 140

Glu Val Lys Asp Tyr Ala Phe Pro Tyr Pro Gln Asp Cys Asn Pro Arg
145                 150                 155                 160

Cys Pro Met Arg Cys Phe Gly Pro Met Cys Thr His Tyr Thr Gln Met
                165                 170                 175

Val Trp Ala Thr Ser Asn Arg Ile Gly Cys Ala Ile His Thr Cys Gln
            180                 185                 190

Asn Met Asn Val Trp Gly Ser Val Trp Arg Arg Ala Val Tyr Leu Val
        195                 200                 205

Cys Asn Tyr Ala Pro Lys Gly Asn Trp Ile Gly Glu Ala Pro Tyr Lys
    210                 215                 220

Val Gly Val Pro Cys Ser Ser Cys Pro Pro Ser Tyr Gly Gly Ser Cys
225                 230                 235                 240

Thr Asp Asn Leu Cys Phe Pro Gly Val Thr Ser Asn Tyr Leu Tyr Trp
                245                 250                 255

Phe

<210> 89
<211> 415
<212> PRT
<213> Halocynthia roretzi

<400> 89
Met Leu Ile Val Gln Ile Asn Met Lys Leu Ser Val Phe Phe Leu Ala
1               5                   10                  15

Leu Leu Pro Leu Val Ala Arg Thr Ser Phe Ala Ser Asn Pro Asn Val
            20                  25                  30

Leu Ser Ala Glu Glu Asn Trp Ser Asn Leu Val Gly Thr Glu Glu Ile

35                  40                  45

Glu Asn Val Asn Ser Glu Asn Glu Phe Ser Leu Ala Thr Glu Glu Glu
    50                  55                  60

Arg Ser Asn Phe Glu Glu Asn Asn Val Ile Leu Ser Glu Glu Lys Val
65                  70                  75                  80

Val Glu Glu Ile Thr Ala Arg Trp Asp Ile Gly Leu Asp Pro Asp Ala
                85                  90                  95

Asn Glu Thr Phe Ser Val Lys Lys Ala Val Lys Ala Val Lys Ile Ile
            100                 105                 110

Pro Gly Lys Ile Met Asp Lys Ile Val Leu Lys Lys Pro Phe Arg Met
        115                 120                 125

Ala Leu Leu Arg Thr His Asn Ala Arg Arg Ala Ile Ala Gln Pro Lys
    130                 135                 140

Ala Ala Asn Met Arg Arg Met Thr Trp Asp Met Glu Leu Glu Arg Leu
145                 150                 155                 160

Ala Val Ala Tyr Ser Arg Lys Cys Ile Tyr Glu His Asn Pro Arg Thr
                165                 170                 175

Lys His Ser Arg Phe Glu Tyr Val Gly Glu Asn Leu Phe Ile Ser Thr
            180                 185                 190

Gly Tyr Ala Phe Thr Pro Ser Leu Met Lys His Ala Val Glu Ala Trp
        195                 200                 205

Asp Asp Glu Lys Gln Tyr Tyr Asp Tyr Glu Thr Lys Lys Cys Gln Arg
    210                 215                 220

Gly Lys Met Cys Gly His Tyr Thr Gln Val Val Trp Ala Asp Thr Phe
225                 230                 235                 240

Lys Met Gly Cys Gly Val Thr Arg Cys Ser Asp Ile Asp Val Arg Gly
                245                 250                 255

Arg Arg Trp Lys Asn Ala Ile Leu Leu Val Cys Asn Tyr Gly Pro Gly
            260                 265                 270

Gly Asn Tyr Pro Thr His Pro Phe Val Thr Ala Pro Ser Cys Ser Lys
        275                 280                 285

Cys Ala Pro Thr Asp Ile Cys Arg Arg Asn Leu Cys Asn Asn Val Ile
    290                 295                 300

Arg Asp Arg Leu Lys Leu Asp Arg Lys Asp Ile Lys Trp Ser Glu Trp
305                 310                 315                 320

Thr Thr Trp Ser Ser Cys Ser Lys Ser Cys Gly Val Gly Ser Thr Arg
                325                 330                 335

Arg Glu Arg Gln Cys Asn Thr Phe Val Pro Gly Asp Cys Lys Asp Phe
            340                 345                 350

Pro Ser Glu Val Lys Phe Cys Val Lys Lys Pro Cys Lys Ala Ala Met
        355                 360                 365

Phe Gly Asn Gly Gly Ser Phe Ser Tyr Asn Ile Val Met Asn Gln Gly
    370                 375                 380

Asp Lys Leu Leu Lys Gly Ser Leu Gln Gln Ala Leu Gln Lys His Leu
385                 390                 395                 400

Ser Gly Phe Ser Phe Gly Asn Phe Val Lys Arg Arg Gly Arg Lys
                405                 410                 415


<210> 90
<211> 266
<212> PRT
<213> Homo sapiens

<400> 90
Met Arg Val Thr Leu Ala Thr Ile Ala Trp Met Val Ser Phe Val Ser
1               5                   10                  15

Asn Tyr Ser His Thr Ala Asn Ile Leu Pro Asp Ile Glu Asn Glu Asp
            20                  25                  30

Phe Ile Lys Asp Cys Val Arg Ile His Asn Lys Phe Arg Ser Glu Val
        35                  40                  45

Lys Pro Thr Ala Ser Asp Met Leu Tyr Met Thr Trp Asp Pro Ala Leu
    50                  55                  60

Ala Gln Ile Ala Lys Ala Trp Ala Ser Asn Cys Gln Phe Ser His Asn
65                  70                  75                  80

Thr Arg Leu Lys Pro Pro His Lys Leu His Pro Asn Phe Thr Ser Leu
                85                  90                  95

Gly Glu Asn Ile Trp Thr Gly Ser Val Pro Ile Phe Ser Val Ser Ser
            100                 105                 110

Ala Ile Thr Asn Trp Tyr Asp Glu Ile Gln Asp Tyr Asp Phe Lys Thr
        115                 120                 125

Arg Ile Cys Lys Lys Val Cys Gly His Tyr Thr Gln Val Val Trp Ala
    130                 135                 140

Asp Ser Tyr Lys Val Gly Cys Ala Val Gln Phe Cys Pro Lys Val Ser
145                 150                 155                 160

Gly Phe Asp Ala Leu Ser Asn Gly Ala His Phe Ile Cys Asn Tyr Gly
                165                 170                 175

Pro Gly Gly Asn Tyr Pro Thr Trp Pro Tyr Lys Arg Gly Ala Thr Cys
            180                 185                 190

Ser Ala Cys Pro Asn Asn Asp Lys Cys Leu Asp Asn Leu Cys Val Asn
        195                 200                 205

Arg Gln Arg Asp Gln Val Lys Arg Tyr Tyr Ser Val Val Tyr Pro Gly
    210                 215                 220

Trp Pro Ile Tyr Pro Arg Asn Arg Tyr Thr Ser Leu Phe Leu Ile Val
225                 230                 235                 240

Asn Ser Val Ile Leu Ile Leu Ser Val Ile Ile Thr Ile Leu Val Gln
                245                 250                 255

Leu Lys Tyr Pro Asn Leu Val Leu Leu Asp
            260                 265


<210> 91
<211> 219
<212> PRT
<213> Homo sapiens

<400> 91
Met Val Ser Phe Val Ser Asn Tyr Ser His Thr Ala Asn Ile Leu Pro
  1               5                  10                  15

Asp Ile Glu Asn Glu Asp Phe Ile Lys Asp Cys Val Arg Ile His Asn
             20                  25                  30

Lys Phe Arg Ser Glu Val Lys Pro Thr Ala Ser Asp Met Leu Tyr Met
         35                  40                  45

Thr Trp Asp Pro Ala Leu Ala Gln Ile Ala Lys Ala Trp Ala Ser Asn
     50                  55                  60

Cys Gln Phe Ser His Asn Thr Arg Leu Lys Pro Pro His Lys Leu His
 65                  70                  75                  80

Pro Asn Phe Thr Ser Leu Gly Glu Asn Ile Trp Thr Gly Ser Val Pro
                 85                  90                  95

Ile Phe Ser Val Ser Ser Ala Ile Thr Asn Trp Tyr Asp Glu Ile Gln
            100                 105                 110

Asp Tyr Asn Phe Lys Thr Arg Ile Cys Lys Lys Val Cys Gly His Tyr
        115                 120                 125

Thr Gln Val Val Trp Ala Asp Ser Tyr Lys Val Gly Cys Ala Val Gln
    130                 135                 140

Phe Cys Pro Lys Val Ser Gly Phe Asp Ala Leu Ser Asn Gly Ala His
145                 150                 155                 160

Phe Ile Cys Asn Tyr Gly Pro Gly Gly Asn Tyr Pro Thr Trp Pro Tyr
                165                 170                 175

Lys Arg Gly Ala Thr Cys Ser Ala Cys Pro Asn Asn Asp Lys Cys Leu
            180                 185                 190

Asp Asn Leu Cys Val Asn Asp Ser Glu Thr Lys Ser Asn Val Thr Thr
        195                 200                 205

Met Leu Tyr Ile Arg Leu Ala His Ile Ser Thr
    210                 215


<210> 92
<211> 245
<212> PRT
<213> Equus caballus

<400> 92
Met Ala Leu Leu Pro Val Leu Leu Phe Leu Ala Ala Val Leu Leu Pro
1               5                   10                  15

Phe Phe Pro Ala Ser Gly Gln Asp Pro Gly Phe Ala Ala Leu Ser Ile
            20                  25                  30

Thr Lys Ser Glu Val Gln Lys Glu Ile Val Asn Lys His Asn Asp Leu
        35                  40                  45

Arg Arg Thr Val Ser Pro Leu Ala Ser Asn Met Leu Lys Met Gln Trp
    50                  55                  60

Asp Ser Lys Thr Ala Thr Asn Ala Gln Asn Trp Ala Asn Lys Cys Leu
65                  70                  75                  80

Leu Gln His Ser Lys Ala Glu Asp Arg Ala Val Gly Thr Met Lys Cys
                85                  90                  95

Gly Glu Asn Leu Phe Met Ser Ser Ile Pro Asn Ser Trp Ser Asp Ala
            100                 105                 110

Ile Gln Asn Trp His Asp Glu Val His Asp Phe Lys Tyr Gly Val Gly
        115                 120                 125

Pro Lys Thr Pro Asn Ala Val Val Gly His Tyr Thr Gln Val Val Trp
    130                 135                 140

Tyr Ser Ser Tyr Arg Val Gly Cys Gly Ile Ala Tyr Cys Pro Lys Gln
145                 150                 155                 160

Gly Thr Leu Lys Tyr Tyr Tyr Val Cys Gln Tyr Cys Pro Ala Gly Asn
                165                 170                 175

Tyr Val Asn Lys Ile Asn Thr Pro Tyr Glu Gln Gly Thr Pro Cys Ala
            180                 185                 190

Arg Cys Pro Gly Asn Cys Asp Asn Gly Leu Cys Thr Asn Ser Cys Glu
        195                 200                 205

Tyr Glu Asp Leu Val Ser Asn Cys Asp Ser Leu Lys Lys Ile Ala Gly
    210                 215                 220

Cys Glu His Glu Leu Leu Lys Glu Asn Cys Lys Thr Thr Cys Gln Cys
225                 230                 235                 240

Glu Asn Lys Ile Tyr
                245

<210> 93
<211> 2664
<212> DNA
<213> Homo sapiens

<400> 93
gtccggtttg gctcacctct cccaggaaac ttcacactgg agagccaaaa ggagtggaag 60
agcctgtctt ggagattttc ctggggaaat cctgaggtca ttcattatga agtgtaccgc 120
gcgggagtgg ctcagagtaa ccacagtgct gttcatggct agagcaattc cagccatggt 180
ggttcccaat gccactttat tggagaaact tttggaaaaa tacatggatg aggatggtga 240

gtggtggata gccaaacaac gagggaaaag ggccatcaca gacaatgaca tgcagagtat 300
tttggacctt cataataaat tacgaagtca ggtgtatcca acagcctcta atatggagta 360
tatgacatgg gatgtagagc tggaaagatc tgcagaatcc tgggctgaaa gttgcttgtg 420
ggaacatgga cctgcaagct tgcttccatc aattggacag aatttgggag cacactgggg 480
aagatatagg cccccgacgt ttcatgtaca atcgtggtat gatgaagtga aagactttag 540
ctacccatat gaacatgaat gcaacccata ttgtccattc aggtgttctg gccctgtatg 600
tacacattat acacaggtcg tgtgggcaac tagtaacaga atcggttgtg ccattaattt 660
gtgtcataac atgaacatct gggggcagat atggcccaaa gctgtctacc tggtgtgcaa 720
ttactcccca aagggaaact ggtggggcca tgccccttac aaacatgggc ggccctgttc 780
tgcttgccca cctagttttg gagggggctg tagagaaaat ctgtgctaca aagaagggtc 840
agacaggtat tatccccctc gagaagagga aacaaatgaa atagaacgac agcagtcaca 900
agtccatgac acccatgtcc ggacaagatc agatgatagt agcagaaatg aagtcataag 960
cgcacagcaa atgtcccaaa ttgtttcttg tgaagtaaga ttaagagatc agtgcaaagg 1020
aacaacctgc aataggtacg aatgtcctgc tggctgtttg gatagtaaag ctaaagttat 1080
tggcagtgta cattatgaaa tgcaatccag catctgtaga gctgcaattc attatggtat 1140
aatagacaat gatggtggct gggtagatat cactagacaa ggaagaaagc attatttcat 1200
caagtccaat agaaatggta ttcaaacaat tggcaaatat cagtctgcta attccttcac 1260
agtctctaaa gtaacagttc aggctgtgac ttgtgaaaca actgtggaac agctctgtcc 1320
atttcataag cctgcttcac attgcccaag agtatactgt cctcgtaact gtatgcaagc 1380
aaatccacat tatgctcgtg taattggaac tcgagtttat tctgatctgt ccagtatctg 1440
cagagcagca gtacatgctg gagtggttcg aaatcacggt ggttatgttg atgtaatgcc 1500
tgtggacaaa agaaagacct acattgcttc ttttcagaat ggaatcttct cagaaagttt 1560
acagaatcct ccaggaggaa aggcattcag agtgtttgct gttgtgtgaa actgaatact 1620
tggaagagga ccataaagac tattccaaat gcaatatttc tgaattttgt ataaaactgt 1680
aacattactg tacagagtac atcaactatt ttcagcccaa aaaggtgcca aatgcatata 1740
aatcttgata aacaaagtct ataaaataaa acatggaca ttagctttgg gaaaagtaat 1800
gaaaatataa tggttttaga aatcctgtgt taaatattgc tatattttct tagcagttat 1860
ttctacagtt aattacatag tcatgattgt tctacgtttc atatattata tggtgctttg 1920
tatatgccac taataaaatg aatctaaaca ttgaatgtga atggccctca gaaaatcatc 1980
tagtgcattt aaaaataatc gactctaaaa ctgaaagaaa ccttatcaca ttttccccag 2040
ttcaatgcta tgccattacc aactccaaat aatctcaaat aattttccac ttaataactg 2100
taaagttttt ttctgttaat ttaggcatat agaatattaa attctgatat tgcacttctt 2160
attttatata aaataatcct ttaatatcca aatgaatctg ttaaaatgtt tgattccttg 2220
ggaatggcct taaaaataaa tgtaataaag tcagagtggt ggtatgaaaa cattcctagt 2280
gatcatgtag taaatgtagg gttaagcatg gacagccaga gctttctatg tactgttaaa 2340
attgaggtca catattttct tttgtatcct ggcaaatact cctgcaggcc aggaagtata 2400
atagcaaaaa gttgaacaaa gatgaactaa tgtattacat taccattgcc actgattttt 2460
ttttaaatgg taaatgacct tgtatataaa tattgccata tcatggtacc tataatggtg 2520
atatatttgt ttctatgaaa aatgtattgt gctttgatac taaaaatctg taaaatgtta 2580
gttttggtaa ttttttttct gctggtggat ttacatatta aatttttttct gctggtggat 2640
aaacattaaa attaatcatg tttc 2664

<210> 94
<211> 500
<212> PRT
<213> Homo sapiens

<400> 94
Met Lys Cys Thr Ala Arg Glu Trp Leu Arg Val Thr Thr Val Leu Phe
1               5                   10                  15

Met Ala Arg Ala Ile Pro Ala Met Val Val Pro Asn Ala Thr Leu Leu
            20                  25                  30

Glu Lys Leu Leu Glu Lys Tyr Met Asp Glu Asp Gly Glu Trp Trp Ile
        35                  40                  45

Ala Lys Gln Arg Gly Lys Arg Ala Ile Thr Asp Asn Asp Met Gln Ser
    50                  55                  60

Ile Leu Asp Leu His Asn Lys Leu Arg Ser Gln Val Tyr Pro Thr Ala
65                  70                  75                  80

Ser Asn Met Glu Tyr Met Thr Trp Asp Val Glu Leu Glu Arg Ser Ala
                85                  90                  95

Glu Ser Trp Ala Glu Ser Cys Leu Trp Glu His Gly Pro Ala Ser Leu
            100                 105                 110

Leu Pro Ser Ile Gly Gln Asn Leu Gly Ala His Trp Gly Arg Tyr Arg
        115                 120                 125

Pro Pro Thr Phe His Val Gln Ser Trp Tyr Asp Glu Val Lys Asp Phe
    130                 135                 140

Ser Tyr Pro Tyr Glu His Glu Cys Asn Pro Tyr Cys Pro Phe Arg Cys
145                 150                 155                 160

Ser Gly Pro Val Cys Thr His Tyr Thr Gln Val Val Trp Ala Thr Ser
                165                 170                 175

Asn Arg Ile Gly Cys Ala Ile Asn Leu Cys His Asn Met Asn Ile Trp
            180                 185                 190

Gly Gln Ile Trp Pro Lys Ala Val Tyr Leu Val Cys Asn Tyr Ser Pro
        195                 200                 205

Lys Gly Asn Trp Trp Gly His Ala Pro Tyr Lys His Gly Arg Pro Cys
    210                 215                 220

Ser Ala Cys Pro Pro Ser Phe Gly Gly Gly Cys Arg Glu Asn Leu Cys
225                 230                 235                 240

Tyr Lys Glu Gly Ser Asp Arg Tyr Tyr Pro Pro Arg Glu Glu Glu Thr
                245                 250                 255

Asn Glu Ile Glu Arg Gln Gln Ser Gln Val His Asp Thr His Val Arg
            260                 265                 270

Thr Arg Ser Asp Asp Ser Ser Arg Asn Glu Val Ile Ser Ala Gln Gln
        275                 280                 285

Met Ser Gln Ile Val Ser Cys Glu Val Arg Leu Arg Asp Gln Cys Lys
    290                 295                 300

Gly Thr Thr Cys Asn Arg Tyr Glu Cys Pro Ala Gly Cys Leu Asp Ser
305                 310                 315                 320

Lys Ala Lys Val Ile Gly Ser Val His Tyr Glu Met Gln Ser Ser Ile
                325                 330                 335

Cys Arg Ala Ala Ile His Tyr Gly Ile Ile Asp Asn Asp Gly Gly Trp
            340                 345                 350

Val Asp Ile Thr Arg Gln Gly Arg Lys His Tyr Phe Ile Lys Ser Asn
        355                 360                 365

Arg Asn Gly Ile Gln Thr Ile Gly Lys Tyr Gln Ser Ala Asn Ser Phe
    370                 375                 380

Thr Val Ser Lys Val Thr Val Gln Ala Val Thr Cys Glu Thr Thr Val

385                 390                 395                 400

Glu Gln Leu Cys Pro Phe His Lys Pro Ala Ser His Cys Pro Arg Val
                405                 410                 415

Tyr Cys Pro Arg Asn Cys Met Gln Ala Asn Pro His Tyr Ala Arg Val
            420                 425                 430

Ile Gly Thr Arg Val Tyr Ser Asp Leu Ser Ser Ile Cys Arg Ala Ala
        435                 440                 445

Val His Ala Gly Val Val Arg Asn His Gly Gly Tyr Val Asp Val Met
    450                 455                 460

Pro Val Asp Lys Arg Lys Thr Tyr Ile Ala Ser Phe Gln Asn Gly Ile
465                 470                 475                 480

Phe Ser Glu Ser Leu Gln Asn Pro Pro Gly Gly Lys Ala Phe Arg Val
                485                 490                 495

Phe Ala Val Val
            500


<210> 95
<211> 188
<212> PRT
<213> Rattus norvegicus

<400> 95
Met Leu His Asn Lys Leu Arg Gly Gln Val Tyr Pro Pro Ala Ser Asn
 1               5                  10                  15

Met Glu Tyr Met Thr Trp Asp Glu Glu Leu Glu Arg Ser Ala Ala Ala
            20                  25                  30

Trp Ala Gln Arg Cys Leu Trp Glu His Gly Pro Ala Ser Leu Leu Val
        35                  40                  45

Ser Ile Gly Gln Asn Leu Ala Val His Trp Gly Arg Tyr Arg Ser Pro
    50                  55                  60

Gly Phe His Val Gln Ser Trp Tyr Asp Glu Val Lys Asp Tyr Thr Tyr
65                  70                  75                  80

Pro Tyr Pro His Glu Cys Asn Pro Trp Cys Pro Glu Arg Cys Ser Gly
                85                  90                  95

Ala Met Cys Thr His Tyr Thr Gln Met Val Trp Ala Thr Thr Asn Lys
            100                 105                 110

Ile Gly Cys Ala Val His Thr Cys Arg Ser Met Ser Val Trp Gly Asp
        115                 120                 125

Ile Trp Glu Asn Ala Val Tyr Leu Val Cys Asn Tyr Ser Pro Lys Gly
    130                 135                 140

Asn Trp Ile Gly Glu Ala Pro Tyr Lys His Gly Arg Pro Cys Ser Glu
145                 150                 155                 160

Cys Pro Ser Ser Tyr Gly Gly Gly Cys Arg Asn Asn Leu Cys Tyr Arg

165                 170                 175

Glu Glu His Tyr His Gln Lys Pro Glu Trp Met Arg
            180                 185


<210> 96
<211> 258
<212> PRT
<213> Homo sapiens

<400> 96
Met Ile Ala Ile Ser Ala Val Ser Ser Ala Leu Leu Phe Ser Leu Leu
1               5                   10                  15

Cys Glu Ala Ser Thr Val Val Leu Leu Asn Ser Thr Asp Ser Ser Pro
            20                  25                  30

Pro Thr Asn Asn Phe Thr Asp Ile Glu Ala Ala Leu Lys Ala Gln Leu
        35                  40                  45

Asp Ser Ala Asp Ile Pro Lys Ala Arg Arg Lys Arg Tyr Ile Ser Gln
    50                  55                  60

Asn Asp Met Ile Ala Ile Leu Asp Tyr His Asn Gln Val Arg Gly Lys
65                  70                  75                  80

Val Phe Pro Pro Ala Ala Asn Met Glu Tyr Met Val Trp Asp Glu Asn
                85                  90                  95

Leu Ala Lys Ser Ala Glu Ala Trp Ala Ala Thr Cys Ile Trp Asp His
            100                 105                 110

Gly Pro Ser Tyr Leu Leu Arg Phe Leu Gly Gln Asn Leu Ser Val Arg
        115                 120                 125

Thr Gly Arg Tyr Arg Ser Ile Leu Gln Leu Val Lys Pro Trp Tyr Asp
    130                 135                 140

Glu Val Lys Asp Tyr Ala Phe Pro Tyr Pro Gln Asp Cys Asn Pro Arg
145                 150                 155                 160

Cys Pro Met Arg Cys Phe Gly Pro Met Cys Thr His Tyr Thr Gln Met
                165                 170                 175

Val Trp Ala Thr Ser Asn Arg Ile Gly Cys Ala Ile His Thr Cys Gln
            180                 185                 190

Asn Met Asn Val Trp Gly Ser Val Trp Arg Arg Ala Val Tyr Leu Val
        195                 200                 205

Cys Asn Tyr Ala Pro Lys Gly Asn Trp Ile Gly Glu Ala Pro Tyr Lys
    210                 215                 220

Val Gly Val Pro Cys Ser Ser Cys Pro Pro Ser Tyr Gly Gly Ser Cys
225                 230                 235                 240

Thr Asp Asn Leu Cys Phe Pro Gly Val Thr Ser Asn Tyr Leu Tyr Trp
                245                 250                 255

Phe Lys

<210> 97
<211> 253
<212> PRT
<213> Homo sapiens

<400> 97
Met Pro Leu Leu Pro Ser Thr Val Gly Leu Ala Gly Leu Leu Phe Trp
1               5                   10                  15

Ala Gly Gln Ala Val Asn Ala Leu Ile Met Pro Asn Ala Thr Pro Ala
            20                  25                  30

Pro Ala Gln Pro Glu Ser Thr Ala Met Arg Leu Leu Ser Gly Leu Glu
        35                  40                  45

Val Pro Arg Tyr Arg Arg Lys Arg His Ile Ser Val Arg Asp Met Asn
    50                  55                  60

Ala Leu Leu Asp Tyr His Asn His Ile Arg Ala Ser Val Tyr Pro Pro
65                  70                  75                  80

Ala Ala Asn Met Glu Tyr Met Val Trp Asp Lys Arg Leu Ala Arg Ala
                85                  90                  95

Ala Glu Ala Trp Ala Thr Gln Cys Ile Trp Ala His Gly Pro Ser Gln
            100                 105                 110

Leu Met Arg Tyr Val Gly Gln Asn Leu Ser Ile His Ser Gly Gln Tyr
        115                 120                 125

Arg Ser Val Val Asp Leu Met Lys Ser Trp Ser Glu Glu Lys Trp His
    130                 135                 140

Tyr Leu Phe Pro Ala Pro Arg Asp Cys Asn Pro His Cys Pro Trp Arg
145                 150                 155                 160

Cys Asp Gly Pro Thr Cys Ser His Tyr Thr Gln Met Val Trp Ala Ser
                165                 170                 175

Ser Asn Arg Leu Gly Cys Ala Ile His Thr Cys Ser Ser Ile Ser Val
            180                 185                 190

Trp Gly Asn Thr Trp His Arg Ala Ala Tyr Leu Val Cys Asn Tyr Ala
        195                 200                 205

Ile Lys Gly Asn Trp Ile Gly Glu Ser Pro Tyr Lys Met Gly Lys Pro
    210                 215                 220

Cys Ser Ser Cys Pro Pro Ser Tyr Gln Gly Ser Cys Asn Ser Asn Met
225                 230                 235                 240

Cys Phe Lys Gly Leu Lys Ser Asn Lys Phe Thr Trp Phe
                245                 250

<210> 98
<211> 245
<212> PRT

<213> Homo sapiens

<400> 98

## Claims

1. An isolated polypeptide comprising an amino acid sequence selected from the group consisting of:
(a) a mature form of an amino acid sequence selected from the group consisting of SEQ ID NOS: 6, 8, 13, 15, 17, 19, 21, 23, 25, 2, and 4;
(b) a variant of a mature form of an amino acid sequence selected from the group consisting of SEQ ID NOS: 6, 8, 13, 15, 17, 19, 21, 23, 25, 2, and 4, wherein one or more amino acid residues in said variant differs from the amino acid sequence of said mature form, provided that said variant differs in no more than 15% of the amino acid residues from the amino acid sequence of said mature form;
(c) an amino acid sequence selected from the group consisting of SEQ ID NOS: 6, 8, 13, 15, 17, 19, 21, 23, 25, 2, and 4; and
(d) a variant of an amino acid sequence selected from the group consisting of SEQ ID NOS: 6, 8, 13, 15, 17, 19, 21, 23, 25, 2, and 4, wherein one or more amino acid residues in said variant differs from the amino acid sequence of said mature form, provided that said variant differs in no more than 15% of the amino acid residues from said amino acid sequence.

2. The polypeptide of Claim 1, wherein said polypeptide comprises the amino acid sequence of a naturally-occurring allelic variant of an amino acid sequence selected from the group consisting of SEQ ID NOS: 6, 8, 13, 15, 17, 19, 21, 23, 25, 2, and 4.

3. The polypeptide of Claim 2, wherein said allelic variant comprises an amino acid sequence that is the translation of a nucleic acid sequence differing by a single nucleotide from a nucleic acid sequence selected from the group consisting of SEQ ID NOS: 5, 7, 9, 10, 11, 12, 14, 16, 18, 20, 22, 24, 1 and 3.

4. The polypeptide of Claim 1, wherein the amino acid sequence of said variant comprises a conservative amino acid substitution.

5. An isolated nucleic acid molecule comprising a nucleic acid sequence encoding a polypeptide comprising an amino acid sequence selected from the group consisting of:
(a) a mature form of an amino acid sequence selected from the group consisting of SEQ ID NOS: 6, 8, 13, 15, 17, 19, 21, 23, 25, 2, and 4;
(b) a variant of a mature form of an amino acid sequence selected from the group consisting of SEQ ID NOS: 6, 8, 13, 15, 17, 19, 21, 23, 25, 2, and 4, wherein one or more amino acid residues in said variant differs from the amino acid sequence of said mature form, provided that said variant differs in no more than 15% of the amino acid residues from the amino acid sequence of said mature form;
(c) an amino acid sequence selected from the group consisting of SEQ ID NOS: 6, 8, 13, 15, 17, 19, 21, 23, 25, 2, and 4;
(d) a variant of an amino acid sequence selected from the group consisting of SEQ ID NOS: 6, 8, 13, 15, 17, 19, 21, 23, 25, 2, and 4, wherein one or more amino acid residues in said variant differs from the amino acid sequence of said mature form, provided that said variant differs in no more than 15% of amino acid residues from said amino acid sequence;
(e) a nucleic acid fragment encoding at least a portion of a polypeptide comprising an amino acid sequence chosen from the group consisting of SEQ ID NOS: 6, 8, 13, 15, 17, 19, 21, 23, 25, 2, and 4, or a variant of said polypeptide, wherein one or more amino acid residues in said variant differs from the amino acid sequence of said mature form, provided that said variant differs in no more than 15% of amino acid residues from said amino acid sequence; and
(f) a nucleic acid molecule encoding the complement of (a), (b), (c), (d) or (e).

6. The nucleic acid molecule of Claim 5, wherein the nucleic acid molecule comprises the nucleotide sequence of a naturally-occurring allelic nucleic acid variant.

7. The nucleic acid molecule of Claim 5, wherein the nucleic acid molecule encodes a polypeptide comprising the amino acid sequence of a naturally-occurring polypeptide variant.

8. The nucleic acid molecule of Claim 5, wherein the nucleic acid molecule differs by a single nucleotide from a nucleic acid sequence selected from the group consisting of SEQ ID NOS: 5, 7, 9, 10, 11, 12, 14, 16, 18, 20, 22, 24, 1 and 3.

9. The nucleic acid molecule of Claim 5, wherein said nucleic acid molecule comprises a nucleotide sequence selected from the group consisting of:
(a) a nucleotide sequence selected from the group consisting of SEQ ID NOS: 5, 7, 9, 10, 11, 12, 14, 16, 18, 20, 22, 24, 1 and 3; and
(b) a nucleotide sequence differing by one or more nucleotides from a nucleotide sequence selected from the group consisting of SEQ ID NOS: 5, 7, 9, 10, 11, 12, 14, 16, 18, 20, 22, 24, 1 and 3, provided that no more than 20% of the nucleotides differ from said nucleotide sequence.
(c) a nucleic acid fragment of (a); and
(d) a nucleic acid fragment of (b).

10. The nucleic acid molecule of Claim 5, wherein said nucleic acid molecule hybridizes under stringent conditions to a nucleotide sequence chosen from the group consisting of SEQ ID NOS: 5, 7, 9, 10, 11, 12, 14, 16, 18, 20, 22, 24, 1 and 3, or a complement of said nucleotide sequence.

11. The nucleic acid molecule of Claim 5, wherein the nucleic acid molecule comprises a nucleotide sequence selected from the group consisting of:
(a) a first nucleotide sequence comprising a coding sequence differing by one or more nucleotide sequences from a coding sequence encoding said amino acid sequence, provided that no more than 20% of the nucleotides in the coding sequence in said first nucleotide sequence differ from said coding sequence;
(b) an isolated second polynucleotide that is a complement of the first polynucleotide; and
(c) a nucleic acid fragment of (a) or (b).

12. A vector comprising the nucleic acid molecule of Claim 11.

13. The vector of Claim 12, further comprising a promoter operably-linked to said nucleic acid molecule.

14. A cell comprising the vector of Claim 12.

15. An antibody that binds immunospecifically to the polypeptide of Claim 1.

16. The antibody of Claim 15, wherein said antibody is a monoclonal antibody.

17. The antibody of Claim 15, wherein the antibody is a humanized antibody.

18. A method for determining the presence or amount of the polypeptide of Claim 1 in a sample, the method comprising:
(a) providing a sample;
(b) contacting the sample with an antibody that binds immunospecifically to the polypeptide; and
(c) determining the presence or amount of antibody bound to said polypeptide,
thereby determining the presence or amount of polypeptide in said sample.

19. A method for determining the presence or amount of the nucleic acid molecule of Claim 5 in a sample, the method comprising:
(a) providing a sample;
(b) contacting the sample with a probe that binds to said nucleic acid molecule; and
(c) determining the presence or amount of the probe bound to said nucleic acid molecule,
thereby determining the presence or amount of the nucleic acid molecule in said sample.

20. The method of Claim 19 wherein presence or amount of the nucleic acid molecule is used as a marker for cell or tissue type.

21. The method of Claim 20 wherein the cell or tissue type is cancerous.

22. A method of identifying an agent that binds to a polypeptide of Claim 1, the method comprising:
(a) contacting said polypeptide with said agent; and
(b) determining whether said agent binds to said polypeptide.

23. The method of Claim 22 wherein the agent is a cellular receptor or a downstream effector.

24. A method for identifying an agent that modulates the expression or activity of the polypeptide of Claim 1, the method comprising:
(a) providing a cell expressing said polypeptide;
(b) contacting the cell with said agent, and
(c) determining whether the agent modulates expression or activity of said polypeptide,
whereby an alteration in expression or activity of said peptide indicates said agent modulates expression or activity of said polypeptide.

25. A method for modulating the activity of the polypeptide of Claim 1, the method comprising contacting a cell sample expressing the polypeptide of said claim with a compound that binds to said polypeptide in an amount sufficient to modulate the activity of the polypeptide.

26. Use of a polypeptide of Claim 1, or a nucleic acid of Claim 5, or an antibody of Claim 15, for the manufacture of a medicament for treating or preventing a FCTRX-associated disorder in a subject.

27. Use according to Claim 26 wherein the disorder is a neurodegenerative disorder.

28. Use according to Claim 26 wherein the disorder is related to cell signal processing and metabolic pathway modulation.

29. Use according to Claim 26, wherein said subject is a human.

30. Use according to any of Claims 26-29, wherein the disorder is selected from the group consisting of colorectal cancer, adenomatous polyposis coli, myelogenous leukemia, congenital ceonatal alloimmune thrombocytopenia, multiple human solid malignancies, malignant ovarian tumours particularly at the interface between the epithelial and stroma, malignant brain tumours, mammary tumours, human gliomas, astrocytomas, mixed glioma/ astrocytomas, renal cells carcinoma, breast adenocarcinoma, ovarian cancer, melanomas, renal cell carcinoma, clear cell and granular cell carcinomas, autocrine/ paracrine stimulation of tumour cell proliferation, autocrine/ paracrine stimulation of tumour cell survival and tumour cell resistance to cytotoxic therapy, paranechmal and basement membrane invasion and motility of tumour cells thereby contributing to metastasis, tumour-mediated immunosuppression of T-cell mediated immune effector cells and pathways resulting in tumour escape from immune surveillance, neurological disorders, neurodegenerative disorders, nerve trauma, familial myelodysplastic syndrome, Chariot-Marie Tooth neuropathy, demyelinating Gardner syndrome, familial myelodysplastic syndrome, mental health conditions, immunological disorders, allergy and infection, asthma, bronchial asthma, Avellino type eosinophilia, lung diseases, reproductive disorders, male infertility, female reproductive system disorders, male and female reproductive diseases, haemangioma, deafness, glycoprotein la deficiency, desmoid disease, turcot syndrome, liver cirrhosis, hepatitis C, gastric disorders, pancreatic diseases like diabetes, Schistosoma mansoni infection, Spinocerebellar ataxia, Plasmodium falciparum parasitemia, Corneal dystrophy - Groenouw type I, Corneal dystrophy - lattice type I, and Reis-Bucklers corneal dystrophy.

31. A pharmaceutical composition comprising the polypeptide of Claim 1, the nucleic acid molecule of Claim 5, the antibody of Claim 15; and a pharmaceutically-acceptable carrier.

32. A kit comprising, in one or more containers, the pharmaceutical composition of Claim 31.

33. A method for determining the presence of or predisposition to a disease associated with altered levels of the polypeptide of Claim 1 in a first mammalian subject, the method comprising:
(a) measuring the level of expression of the polypeptide *in vitro* in a sample from the first mammalian subject; and
(b) comparing the amount of said polypeptide in the sample of step (a) to the amount of the polypeptide present in a control sample from a second mammalian subject known not to have, or not to be predisposed to, said disease;
wherein an alteration in the expression level of the polypeptide in the first subject as compared to the control sample indicates the presence of or predisposition to said disease.

34. A method for determining the presence of or predisposition to a disease associated with altered levels of the nucleic acid molecule of Claim 5 in a first mammalian subject, the method comprising:
(a) measuring the amount of the nucleic acid *in vitro* in a sample from the first mammalian subject; and
(b) comparing the amount of said nucleic acid in the sample of step (a) to the amount of the nucleic acid present in a control sample from a second mammalian subject known not to have, or not be predisposed to, the disease;
wherein an alteration in the level of the nucleic acid in the first subject as compared to the control sample indicates the presence of or predisposition to the disease.

35. The method of Claim 33 or Claim 34 wherein the predisposition is to cancers.

36. A method of treating a pathological state in a mammal, the method comprising administering to the mammal a polypeptide in an amount that is sufficient to alleviate the pathological state, wherein the polypeptide is a polypeptide having an amino acid sequence at least 95% identical to a polypeptide comprising an amino acid sequence of at least one of SEQ ID NOS: 6, 8, 13, 15, 17, 19, 21, 23, 25, 2, or 4, or a biologically active fragment thereof.

37. Use of the antibody of Claim 15, for the manufacture of a medicament for treating a pathological state in a mammal.
